# EUROPEAN PATENT SPECIFICATION

(11) **EP 4 466 250 B1**
(45) Date of publication and mention of the grant of the patent: **26.03.2025**
(21) Application number: 23704424.3
(22) Date of filing: 31.01.2023
(51) Int. Cl.: C07C 233/36, C07C 237/12, C07C 243/28, C07C 251/76, C07C 251/78, C07C 259/14, C07C 271/22, C07C 311/06, C07D 295/15, A61K 9/00, A61K 47/00

(54) **IONIZABLE CATIONIC LIPIDS FOR LIPID NANOPARTICLES**
IONISIERBARE KATIONISCHE LIPIDE FÜR LIPIDNANOPARTIKEL
LIPIDES CATIONIQUES IONISABLES POUR NANOPARTICULES LIPIDIQUES

(30) Priority: 31.01.2022 US 202263305181 P
(43) Date of publication of application: 27.11.2024
(73) Proprietor: GENEVANT SCIENCES GMBH, 4051 Basel (CH)
(72) Inventor: HOLLAND, Richard J., 4051 Basel (CH); LAM, Kieu Mong, 4051 Basel (CH); LOUIE, Dayna, 4051 Basel (CH); HEYES, James, 4051 Basel (CH); MARTIN, Alan, 4051 Basel (CH); WOOD, Mark, 4051 Basel (CH); ZHANG, Wenxuan, 4051 Basel (CH)
(74) Representative: J A Kemp LLP
(86) International application number: PCT/IB2023/050811
(87) International publication number: WO 2023/144798

(56) References cited:
- WO-A1-2019/036008
- WO-A1-2019/152557
- WO-A1-2020/061367
- US-A1- 2018 273 467

## Description

### BACKGROUND

Ionizable cationic lipids are typically a major lipid component in lipid nanoparticles (LNP). They are designed to be charge-neutral at standard physiological pH (~7) but acquire a positive charge at lower (acidic) pH. This helps with nucleic acid payload encapsulation during the LNP formation, and intracellular delivery as the endosomal fusion step requires a positive charge on the ionizable lipid.

Ionizable lipids such as MC3 (used in the FDA-approved siRNA-LNP product Onpattro) and 3D-P-DMA have demonstrated excellent activity and tolerability in a variety of applications. However, they are not rapidly metabolized, and they take several weeks to clear from target tissues. This makes them less appealing for applications that require multiple doses, especially with short dose intervals and/or chronic indications.

US 2018/273467 and WO 2020/061367 describe nanoparticle compositions comprising lipids, as well as additional lipids such as phospholipids, structural lipids, and PEG lipids, which are said to be useful in the delivery of therapeutic and/or prophylactics to mammalian cells or organs to, for example, regulate polypeptide, protein, or gene expression.

WO 2019/152557 describes immune cell delivery lipid nanoparticle (LNP) compositions that are said to allow for enhanced delivery of agents, e.g., nucleic acids, such as therapeutic and/or prophylactic RNAs, to immune cells, in particular T cells, as well as B cells, dendritic cells and monocytes. The LNPs comprise an amount of an immune cell delivery potentiating lipid such that delivery of an agent by an immune cell delivery LNP is said to be enhanced as compared to an LNP lacking the immune cell delivery potentiating agent. Methods of using the immune cell delivery LNPs for delivery of agents, e.g., nucleic acid delivery, for protein expression, for modulating immune cell activity and modulating immune responses are also described.

Currently, there is a need for ionizable lipids that demonstrate improved clearance while retaining good activity in their ability to deliver nucleic acids. There is also a need for ionizable lipids that have beneficial or improved biodegradability.

### SUMMARY

The invention provides ionizable lipids that have improved biodegradability and/or rates of clearance.

In one embodiment, the invention provides an ionizable lipid, which is a compound of formula (I): 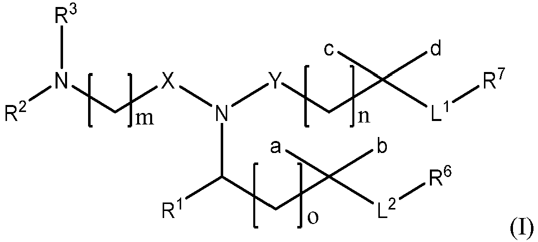 or a salt thereof, wherein:
X is -CH₂-, -O-, -O(C=O); or -C(=O)-
Y is -CH₂-, -O-, -SO₂-, or -C(=O)-;
R¹ is C₂-C₂₅hydrocarbyl;
R² is C₁-C₃alkyl; and R³ is C₁-C₃alkyl; or R² and R³ taken together with the nitrogen to which they are attached form a a aziridino, azetidino, or pyrrolidino ring;
m is 0, 1, 2, 3, 4, 5 or 6;
n is 0-15;
o is 0-15;
L¹ is absent, -(C=O)-O-, -(C=O)N(H)N(R⁴)-, -(C=O)N(H)-N=C(R⁴)-, -(C=O)N(H)N(H)C(R⁴)(R⁵)-, -(C=O)-O-CH₂-R^{z}, or -O(C=O)-R^{w}-;
L² is absent, -(C=O)-O-, -(C=O)N(H)N(R^{4'})-, -(C=O)N(H)-N=C(R^{4'})-, -(C=O)N(H)N(H)C(R^{4'})(R^{5'})-, -(C=O)-O-CH₂-R^{z'}, or -O(C=O)-R^{×}-
a is H or F;
b is H or F;
c is H or F; and d is H or F; or c and d taken together with the carbon to which they are attached form -C(=O)-
R⁴ is H or C₂-C₂₅hydrocarbyl;
R⁵ is H or C₂-C₂₅hydrocarbyl;
R^{4'} is H or C₂-C₂₅hydrocarbyl;
R^{5'} is H or C₂-C₂₅hydrocarbyl;
R⁶ is C₂-C₂₅hydrocarbyl;
R⁷ is C₂-C₂₅hydrocarbyl;
R^{w} is absent, -C(F)H-, or -C(F)₂-;
R^{×} is absent, -C(F)H-, or -C(F)₂-;
R^{z} is phenyl that is substituted with 1, 2, or 3 groups independently selected from C₂-C₁₀hydrocarbyl; and
R^{z'} is phenyl that is substituted with 1, 2, or 3 groups independently selected from C₂-C₁₀hydrocarbyl.

The invention also provides a composition comprising 1) an ionizable lipid of the invention, and 2) an active agent or a therapeutic agent.

The invention also provides a lipid nanoparticle comprising an ionizable lipid of the invention.

The invention also provides a lipid nanoparticle comprising: (a) one or more active agents or therapeutic agents; (b) an ionizable lipid of the invention; (c) a non-cationic lipid; and (d) a conjugated lipid. Typically, the one or more agents are encapsulated within the lipid nanoparticle.

The invention also provides a pharmaceutical composition comprising, 1) an ionizable lipid of the invention, and 2) one or more active agents or therapeutic agents.

The invention also provides a pharmaceutical composition comprising a lipid nanoparticle of the invention and a pharmaceutically acceptable carrier.

The invention also finds use in a method for delivering an active agent or a therapeutic agent (e.g. a nucleic acid) to an animal, comprising administering a nanoparticle of the invention to the animal.

The invention also finds use in a method for introducing a nucleic acid into a cell, the method comprising, contacting the cell *in vivo, ex vivo* or *in vitro* with a nucleic acid-lipid particle described herein.

The invention also finds use in a method for treating a disease or disorder in an animal, comprising administering a therapeutically effective amount of a nucleic acid-lipid particle described herein to the animal.

The invention also provides processes and intermediates disclosed herein that are useful for making the compounds, formulations, or nanoparticles of the invention.

### DETAILED DESCRIPTION

### Definitions

As used herein, the following terms have the meanings ascribed to them unless specified otherwise.

The term "hydrocarbyl" as used herein describes an organic compound or radical consisting of about 4 to about 500 atoms selected from carbon and hydrogen. Certain specific hydrocarbyl moieties have the number of carbon atoms designated, for example, a C₂-C₂₅hydrocarbyl is a hydrocarbon having 2, 3, 4, 5, 6, 7, 8, 9, 10, 11, 12, 13, 14, 15, 16, 17, 18, 19, 20, 21, 22, 23, 24, or 25 carbon atoms and up to 52 hydrogen atoms, depending on the level of unsaturation. Hydrocarbyl moieties include, without limitation, branched or unbranched alkyl, branched or unbranched alkenyl, branched or unbranched alkynyl, cycloalkyl, and aryl moieties. In one embodiment, the hydrocarbyl consists of about 4 to about 400 atoms selected from carbon and hydrogen. In one embodiment, the hydrocarbyl consists of about 4 to about 300 atoms selected from carbon and hydrogen. In one embodiment, the hydrocarbyl consists of about 4 to about 200 atoms selected from carbon and hydrogen. In one embodiment, the hydrocarbyl consists of about 4 to about 100 atoms selected from carbon and hydrogen. In one embodiment, the hydrocarbyl consists of about 4 to about 62 atoms selected from carbon and hydrogen. In one embodiment, the hydrocarbyl consists of about 20 to about 500 atoms selected from carbon and hydrogen. In one embodiment, the hydrocarbyl consists of about 20 to about 400 atoms selected from carbon and hydrogen. In one embodiment, the hydrocarbyl consists of about 20 to about 300 atoms selected from carbon and hydrogen. In one embodiment, the hydrocarbyl consists of about 20 to about 200 atoms selected from carbon and hydrogen. In one embodiment, the hydrocarbyl consists of about 20 to about 100 atoms selected from carbon and hydrogen. In one embodiment, the hydrocarbyl consists of about 20 to about 62 atoms selected from carbon and hydrogen. In one embodiment, the hydrocarbyl consists of about 40 to about 500 atoms selected from carbon and hydrogen. In one embodiment, the hydrocarbyl consists of about 40 to about 400 atoms selected from carbon and hydrogen. In one embodiment, the hydrocarbyl consists of about 40 to about 300 atoms selected from carbon and hydrogen. In one embodiment, the hydrocarbyl consists of about 40 to about 200 atoms selected from carbon and hydrogen. In one embodiment, the hydrocarbyl consists of about 40 to about 100 atoms selected from carbon and hydrogen. In one embodiment, the hydrocarbyl consists of about 40 to about 62 atoms selected from carbon and hydrogen. In one embodiment, the hydrocarbyl consists of at least about 20, at least about 40, at least about 60, or at least about 80 atoms selected from carbon and hydrogen. In one embodiment, the hydrocarbyl consists of less than about 500, less than about 400, less than about 300, less than about 200, or less than about 100 atoms selected from carbon and hydrogen.

The term "alkyl", by itself or as part of another substituent, means, unless otherwise stated, a straight or branched chain hydrocarbon radical, having the number of carbon atoms designated (i.e., C₁₋₈ means one to eight carbons). Examples include (C₁-C₈)alkyl, (C₂-C₈)alkyl, C₁-C₆)alkyl, (C₂-C₆)alkyl and (C₃-C₆)alkyl. Examples of alkyl groups include methyl, ethyl, n-propyl, iso-propyl, n-butyl, t-butyl, iso-butyl, sec-butyl, n-pentyl, n-hexyl, n-heptyl, n-octyl, and and higher homologs and isomers.

The term "alkenyl" refers to an unsaturated alkyl radical having one or more (e.g., 1, 2, 3, or 4) double bonds. Examples of such unsaturated alkyl groups include vinyl, 2-propenyl, crotyl, 2-isopentenyl, 2-(butadienyl), 2,4-pentadienyl, 3-(1,4-pentadienyl) and the higher homologs and isomers.

The term "alkynyl" refers to an unsaturated alkyl radical having one or more (e.g., 1, 2, 3, or 4) triple bonds. Examples of such unsaturated alkyl groups ethynyl, 1- and 3-propynyl, 3-butynyl, and higher homologs and isomers.

The term "alkoxy" refers to an alkyl groups attached to the remainder of the molecule via an oxygen atom ("oxy").

The term "alkylthio" refers to an alkyl groups attached to the remainder of the molecule via a thio group.

The term "cycloalkyl" refers to a saturated or partially unsaturated (non-aromatic) all carbon ring having 3 to 8 carbon atoms (i.e., (C₃-C₈)carbocycle). The term also includes multiple condensed, saturated all carbon ring systems (e.g., ring systems comprising 2, 3 or 4 carbocyclic rings). Accordingly, carbocycle includes multicyclic carbocyles such as a bicyclic carbocycles (e.g., bicyclic carbocycles having about 3 to 15 carbon atoms , about 6 to 15 carbon atoms, or 6 to 12 carbon atoms such as bicyclo[3.1.0]hexane and bicyclo[2.1.1]hexane), and polycyclic carbocycles (e.g tricyclic and tetracyclic carbocycles with up to about 20 carbon atoms). The rings of the multiple condensed ring system can be connected to each other via fused, spiro and bridged bonds when allowed by valency requirements. For example, multicyclic carbocyles can be connected to each other via a single carbon atom to form a spiro connection (e.g., spiropentane, spiro[4,5]decane, etc), via two adjacent carbon atoms to form a fused connection (e.g., carbocycles such as decahydronaphthalene, norsabinane, norcarane) or via two non-adjacent carbon atoms to form a bridged connection (e.g., norbornane, bicyclo[2.2.2]octane, etc). Non-limiting examples of cycloalkyls include cyclopropyl, cyclobutyl, cyclopentyl, cyclohexyl, bicyclo[2.2.1]heptane, pinane, and adamantane.

The term "aryl" as used herein refers to a single all carbon aromatic ring or a multiple condensed all carbon ring system wherein at least one of the rings is aromatic. For example, in certain embodiments, an aryl group has 6 to 20 carbon atoms, 6 to 14 carbon atoms, 6 to 12 carbon atoms, or 6 to 10 carbon atoms. Aryl includes a phenyl radical. Aryl also includes multiple condensed carbon ring systems (e.g., ring systems comprising 2, 3 or 4 rings) having about 9 to 20 carbon atoms in which at least one ring is aromatic and wherein the other rings may be aromatic or not aromatic (i.e., cycloalkyl). The rings of the multiple condensed ring system can be connected to each other via fused, spiro and bridged bonds when allowed by valency requirements. It is to be understood that the point of attachment of a multiple condensed ring system, as defined above, can be at any position of the ring system including an aromatic or a carbocycle portion of the ring. Non-limiting examples of aryl groups include, but are not limited to, phenyl, indenyl, indanyl, naphthyl, 1, 2, 3, 4-tetrahydronaphthyl, anthracenyl, and the like.

The term "interfering RNA" or "RNAi" or "interfering RNA sequence" refers to single-stranded RNA (e.g., mature miRNA) or double-stranded RNA (i.e., duplex RNA such as siRNA, aiRNA, or pre-miRNA) that is capable of reducing or inhibiting the expression of a target gene or sequence (e.g., by mediating the degradation or inhibiting the translation of mRNAs which are complementary to the interfering RNA sequence) when the interfering RNA is in the same cell as the target gene or sequence. Interfering RNA thus refers to the single-stranded RNA that is complementary to a target mRNA sequence or to the double-stranded RNA formed by two complementary strands or by a single, self-complementary strand. Interfering RNA may have substantial or complete identity to the target gene or sequence, or may comprise a region of mismatch (i.e., a mismatch motif). The sequence of the interfering RNA can correspond to the full-length target gene, or a subsequence thereof.

Interfering RNA includes "small-interfering RNA" or "siRNA," e.g., interfering RNA of about 15-60, 15-50, or 15-40 (duplex) nucleotides in length, more typically about 15-30, 15-25, or 19-25 (duplex) nucleotides in length, and is preferably about 20-24, 21-22, or 21-23 (duplex) nucleotides in length (e.g., each complementary sequence of the double-stranded siRNA is 15-60, 15-50, 15-40, 15-30, 15-25, or 19-25 nucleotides in length, preferably about 20-24, 21-22, or 21-23 nucleotides in length, and the double-stranded siRNA is about 15-60, 15-50, 15-40, 15-30, 15-25, or 19-25 base pairs in length, preferably about 18-22, 19-20, or 19-21 base pairs in length). siRNA duplexes may comprise 3' overhangs of about 1 to about 4 nucleotides or about 2 to about 3 nucleotides and 5' phosphate termini. Examples of siRNA include, without limitation, a double-stranded polynucleotide molecule assembled from two separate stranded molecules, wherein one strand is the sense strand and the other is the complementary antisense strand; a double-stranded polynucleotide molecule assembled from a single stranded molecule, where the sense and antisense regions are linked by a nucleic acid-based or non-nucleic acid-based linker; a double-stranded polynucleotide molecule with a hairpin secondary structure having self-complementary sense and antisense regions; and a circular single-stranded polynucleotide molecule with two or more loop structures and a stem having self-complementary sense and antisense regions, where the circular polynucleotide can be processed in vivo or in vitro to generate an active double-stranded siRNA molecule.

Preferably, siRNA are chemically synthesized. siRNA can also be generated by cleavage of longer dsRNA (e.g., dsRNA greater than about 25 nucleotides in length) with the *E. coli* RNase III or Dicer. These enzymes process the dsRNA into biologically active siRNA (see, e.g., Yang et al., Proc. Natl. Acad. Sci. USA, 99:9942-9947 (2002); Calegari et al., Proc. Natl. Acad. Sci. USA, 99:14236 (2002); Byrom et al., Ambion TechNotes, 10(1):4-6 (2003); Kawasaki et al., Nucleic Acids Res., 31:981-987 (2003); Knight et al., Science, 293:2269-2271 (2001); and Robertson et al., J. Biol. Chem., 243:82 (1968)). Preferably, dsRNA are at least 50 nucleotides to about 100, 200, 300, 400, or 500 nucleotides in length. A dsRNA may be as long as 1000, 1500, 2000, 5000 nucleotides in length, or longer. The dsRNA can encode for an entire gene transcript or a partial gene transcript. In certain instances, siRNA may be encoded by a plasmid (e.g., transcribed as sequences that automatically fold into duplexes with hairpin loops).

As used herein, the term "mismatch motif" or "mismatch region" refers to a portion of an interfering RNA (e.g., siRNA, aiRNA, miRNA) sequence that does not have 100% complementarity to its target sequence. An interfering RNA may have at least one, two, three, four, five, six, or more mismatch regions. The mismatch regions may be contiguous or may be separated by 1, 2, 3, 4, 5, 6, 7, 8, 9, 10, 11, 12, or more nucleotides. The mismatch motifs or regions may comprise a single nucleotide or may comprise two, three, four, five, or more nucleotides.

An "effective amount" or "therapeutically effective amount" of an active agent or therapeutic agent such as a nucleic acid (e.g., an interfering RNA or mRNA) is an amount sufficient to produce the desired effect, e.g., an inhibition of expression of a target sequence in comparison to the normal expression level detected in the absence of an interfering RNA; or mRNA-directed expression of an amount of a protein that causes a desirable biological effect in the organism within which the protein is expressed. Inhibition of expression of a target gene or target sequence is achieved when the value obtained with an interfering RNA relative to the control is about 90%, 85%, 80%, 75%, 70%, 65%, 60%, 55%, 50%, 45%, 40%, 35%, 30%, 25%, 20%, 15%, 10%, 5%, or 0%. In other embodiments, the expressed protein is an active form of a protein that is normally expressed in a cell type within the body, and the therapeutically effective amount of the mRNA is an amount that produces an amount of the encoded protein that is at least 50% (e.g., at least 60%, or at least 70%, or at least 80%, or at least 90%) of the amount of the protein that is normally expressed in the cell type of a healthy individual. Suitable assays for measuring expression of a target gene or target sequence include, e.g., examination of protein or RNA levels using techniques known to those of skill in the art such as dot blots, northern blots, in situ hybridization, ELISA, immunoprecipitation, enzyme function, as well as phenotypic assays known to those of skill in the art.

By "decrease," "decreasing," "reduce," or "reducing" of an immune response by an interfering RNA is intended to mean a detectable decrease of an immune response to a given interfering RNA (e.g., a modified interfering RNA). The amount of decrease of an immune response by a modified interfering RNA may be determined relative to the level of an immune response in the presence of an unmodified interfering RNA. A detectable decrease can be about 5%, 10%, 15%, 20%, 25%, 30%, 35%, 40%, 45%, 50%, 55%, 60%, 65%, 70%, 75%, 80%, 85%, 90%, 95%, 100%, or more lower than the immune response detected in the presence of the unmodified interfering RNA. A decrease in the immune response to interfering RNA is typically measured by a decrease in cytokine production (e.g., IFNγ, IFNα, TNFα, IL-6, or IL-12) by a responder cell in vitro or a decrease in cytokine production in the sera of a mammalian subject after administration of the interfering RNA.

By "decrease," "decreasing," "reduce," or "reducing" of an immune response by an mRNA is intended to mean a detectable decrease of an immune response to a given mRNA (e.g., a modified mRNA). The amount of decrease of an immune response by a modified mRNA may be determined relative to the level of an immune response in the presence of an unmodified mRNA. A detectable decrease can be about 5%, 10%, 15%, 20%, 25%, 30%, 35%, 40%, 45%, 50%, 55%, 60%, 65%, 70%, 75%, 80%, 85%, 90%, 95%, 100%, or more lower than the immune response detected in the presence of the unmodified mRNA. A decrease in the immune response to mRNA is typically measured by a decrease in cytokine production (e.g., IFNγ, IFNα, TNFα, IL-6, or IL-12) by a responder cell *in vitro* or a decrease in cytokine production in the sera of a mammalian subject after administration of the mRNA.

As used herein, the term "responder cell" refers to a cell, preferably a mammalian cell, that produces a detectable immune response when contacted with an immunostimulatory interfering RNA such as an unmodified siRNA. Exemplary responder cells include, e.g., dendritic cells, macrophages, peripheral blood mononuclear cells (PBMCs), splenocytes, and the like. Detectable immune responses include, e.g., production of cytokines or growth factors such as TNF-α, IFN-α, IFN-β, IFN-γ, IL-1, IL-2, IL-3, IL-4, IL-5, IL-6, IL-10, IL-12, IL-13, TGF, and combinations thereof.

"Substantial identity" refers to a sequence that hybridizes to a reference sequence under stringent conditions, or to a sequence that has a specified percent identity over a specified region of a reference sequence.

The phrase "stringent hybridization conditions" refers to conditions under which a nucleic acid will hybridize to its target sequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology-Hybridization with Nucleic Probes, "Overview of principles of hybridization and the strategy of nucleic acid assays" (1993). Generally, stringent conditions are selected to be about 5-10° C. lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization.

Exemplary stringent hybridization conditions can be as follows: 50% formamide, 5×SSC, and 1% SDS, incubating at 42° C., or, 5×SSC, 1% SDS, incubating at 65° C., with wash in 0.2×SSC, and 0.1% SDS at 65° C. For PCR, a temperature of about 36° C. is typical for low stringency amplification, although annealing temperatures may vary between about 32° C. and 48° C. depending on primer length. For high stringency PCR amplification, a temperature of about 62° C. is typical, although high stringency annealing temperatures can range from about 50° C. to about 65° C., depending on the primer length and specificity. Typical cycle conditions for both high and low stringency amplifications include a denaturation phase of 90° C.-95° C. for 30 sec.-2 min., an annealing phase lasting 30 sec.-2 min., and an extension phase of about 72° C. for 1-2 min. Protocols and guidelines for low and high stringency amplification reactions are provided, e.g., in Innis et al., PCR Protocols, A Guide to Methods and Applications, Academic Press, Inc. N.Y. (1990).

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37° C., and a wash in 1× SSC at 45° C. A positive hybridization is at least twice background. Those of ordinary skill will readily recognize that alternative hybridization and wash conditions can be utilized to provide conditions of similar stringency. Additional guidelines for determining hybridization parameters are provided in numerous references, e.g., Current Protocols in Molecular Biology, Ausubel et al., eds.

The terms "substantially identical" or "substantial identity," in the context of two or more nucleic acids, refer to two or more sequences or subsequences that are the same or have a specified percentage of nucleotides that are the same (i.e., at least about 60%, preferably at least about 65%, 70%, 75%, 80%, 85%, 90%, or 95% identity over a specified region), when compared and aligned for maximum correspondence over a comparison window, or designated region as measured using one of the following sequence comparison algorithms or by manual alignment and visual inspection. This definition, when the context indicates, also refers analogously to the complement of a sequence. Preferably, the substantial identity exists over a region that is at least about 5, 10, 15, 20, 25, 30, 35, 40, 45, 50, 55, or 60 nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of a number of contiguous positions selected from the group consisting of from about 5 to about 60, usually about 10 to about 45, more usually about 15 to about 30, in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman, Adv. Appl. Math., 2:482 (1981), by the homology alignment algorithm of Needleman and Wunsch, J. Mol. Biol., 48:443 (1970), by the search for similarity method of Pearson and Lipman, Proc. Natl. Acad. Sci. USA, 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Current Protocols in Molecular Biology, Ausubel et al., eds. (1995 supplement)).

A preferred example of algorithms that are suitable for determining percent sequence identity and sequence similarity are the BLAST and BLAST 2.0 algorithms, which are described in Altschul et al., Nuc. Acids Res., 25:3389-3402 (1977) and Altschul et al., J. Mol. Biol., 215:403-410 (1990), respectively. BLAST and BLAST 2.0 are used, with the parameters described herein, to determine percent sequence identity for the nucleic acids of the invention. Software for performing BLAST analyses is publicly available through the National Center for Biotechnology Information (http://www.ncbi.nlm.nih.gov/).

The BLAST algorithm also performs a statistical analysis of the similarity between two sequences (see, e.g., Karlin and Altschul, Proc. Natl. Acad. Sci. USA, 90:5873-5787 (1993)). One measure of similarity provided by the BLAST algorithm is the smallest sum probability (P(N)), which provides an indication of the probability by which a match between two nucleotide sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "nucleic acid" as used herein refers to a polymer containing at least two deoxyribonucleotides or ribonucleotides in either single- or double-stranded form and includes DNA and RNA. DNA may be in the form of, e.g., antisense molecules, plasmid DNA, precondensed DNA, a PCR product, vectors (P1, PAC, BAC, YAC, artificial chromosomes), expression cassettes, chimeric sequences, chromosomal DNA, or derivatives and combinations of these groups. RNA may be in the form of siRNA, asymmetrical interfering RNA (aiRNA), microRNA (miRNA), mRNA, tRNA, rRNA, tRNA, viral RNA (vRNA), self-amplifying RNA, and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

The term "gene" refers to a nucleic acid (e.g., DNA or RNA) sequence that comprises partial length or entire length coding sequences necessary for the production of a polypeptide or precursor polypeptide.

"Gene product," as used herein, refers to a product of a gene such as an RNA transcript or a polypeptide.

The term "lipid" refers to a group of organic compounds that include, but are not limited to, esters of fatty acids and are characterized by being insoluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

As used herein, the term "LNP" refers to a lipid-nucleic acid particle or a nucleic acid-lipid particle (e.g., a stable nucleic acid-lipid particle). A LNP represents a particle made from lipids (e.g., an ionizable lipid of the invention, a non-cationic lipid, and a conjugated lipid that prevents aggregation of the particle), and a nucleic acid, wherein the nucleic acid (e.g., siRNA, aiRNA, miRNA, ssDNA, dsDNA, ssRNA, short hairpin RNA (shRNA), dsRNA, mRNA, self-amplifying RNA, or a plasmid, including plasmids from which an interfering RNA or mRNA is transcribed) is encapsulated within the lipid. In one embodiment, the nucleic acid is at least 50% encapsulated in the lipid; in one embodiment, the nucleic acid is at least 75% encapsulated in the lipid; in one embodiment, the nucleic acid is at least 90% encapsulated in the lipid; and in one embodiment, the nucleic acid is completely encapsulated in the lipid. LNPs typically contain a cationic lipid, a non-cationic lipid, and a lipid conjugate (e.g., a PEG-lipid conjugate). LNP are extremely useful for systemic applications, as they can exhibit extended circulation lifetimes following intravenous (i.v.) injection, they can accumulate at distal sites (e.g., sites physically separated from the administration site), and they can mediate expression of the transfected gene or silencing of target gene expression at these distal sites.

The lipid particles of the invention (e.g., LNP) typically have a mean diameter of from about 40 nm to about 150 nm. In one embodiment, the lipid particles of the invention have a mean diameter of from about 40 nm to about 80 nm. In one embodiment, the lipid particles of the invention have a mean diameter of from about 40 nm to about 70 nm. In one embodiment, the lipid particles of the invention have a mean diameter of from about 40 nm to about 60 nm. In one embodiment, the lipid particles of the invention have a mean diameter of from about 50 nm to about 60 nm. In one embodiment, the lipid particles of the invention have a mean diameter of fromabout 50 nm to about 150 nm. In one embodiment, the lipid particles of the invention have a mean diameter of from about 60 nm to about 130 nm. In one embodiment, the lipid particles of the invention have a mean diameter of from about 70 nm to about 110 nm. In one embodiment, the lipid particles of the invention have a mean diameter of from about 70 to about 90 nm. The lipid particles of the invention are substantially non-toxic. In addition, nucleic acids, when present in the lipid particles of the invention, are resistant in aqueous solution to degradation with a nuclease. Nucleic acid-lipid particles and their method of preparation are disclosed in, e.g., U.S. Patent Publication Nos. 20040142025 and 20070042031.

As used herein, "lipid encapsulated" can refer to a lipid particle that provides an active agent or therapeutic agent, such as a nucleic acid (e.g., an interfering RNA or mRNA), with full encapsulation, partial encapsulation, or both. In a preferred embodiment, the nucleic acid is fully encapsulated in the lipid particle (e.g., to form an SPLP, pSPLP, LNP, or other nucleic acid-lipid particle).

The term "lipid conjugate" refers to a polymer-conjugated lipid that inhibits aggregation of lipid particles. Such lipid conjugates include, but are not limited to, polyamide oligomers (e.g., ATTA-lipid conjugates), polysarcosine-lipid conjugates, polyoxazoline (POZ)-lipid conjugates, PEG-lipid conjugates, such as PEG coupled to dialkyloxypropyls, PEG coupled to diacylglycerols, PEG coupled to cholesterol, PEG coupled to phosphatidylethanolamines, PEG conjugated to ceramides (see, e.g., U.S. Pat. No. 5,885,613), cationic PEG lipids, and mixtures thereof. PEG can be conjugated directly to the lipid or may be linked to the lipid via a linker moiety. Any linker moiety suitable for coupling the PEG to a lipid can be used including, e.g., non-ester containing linker moieties and ester-containing linker moieties. In preferred embodiments, non-ester containing linker moieties are used.

The term "amphipathic lipid" refers, in part, to any suitable material wherein the hydrophobic portion of the lipid material orients into a hydrophobic phase, while the hydrophilic portion orients toward the aqueous phase. Hydrophilic characteristics derive from the presence of polar or charged groups such as carbohydrates, phosphate, carboxylic, sulfato, amino, sulfhydryl, nitro, hydroxyl, and other like groups. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). Examples of amphipathic compounds include, but are not limited to, phospholipids, aminolipids, and sphingolipids.

Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine. Other compounds lacking in phosphorus, such as sphingolipid, glycosphingolipid families, diacylglycerols, and β-acyloxyacids, are also within the group designated as amphipathic lipids. Additionally, the amphipathic lipids described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidylethanolamine, ceramide, sphingomyelin, cephalin, cholesterol, cerebrosides, and diacylglycerols.

The term "non-cationic lipid" refers to any amphipathic lipid as well as any other neutral lipid or anionic lipid.

The term "anionic lipid" refers to any lipid that is negatively charged at physiological pH. These lipids include, but are not limited to, phosphatidylglycerols, cardiolipins, diacylphosphatidylserines, diacylphosphatidic acids, N-dodecanoyl phosphatidylethanolamines, N-succinyl phosphatidylethanolamines, N-glutarylphosphatidylethanolamines, lysylphosphatidylglycerols, palmitoyloleyolphosphatidylglycerol (POPG), and other anionic modifying groups joined to neutral lipids.

The term "cationic lipid" refers to lipids that can be positively charged. These lipids include those with one or more amine groups that bear the positive charge. Certain cationic lipids are ionizable (and termed "ionizable lipids") and may exist in either a charge neutral or positively charged state, depending on the surrounding pH.

In one embodiment, the cationic lipid is a compound of Formula (I) as described herein, which in certain embodiments has a clearance rate such that <10% of the parent ionizable lipid is remaining at seven (7) days post-administration (e.g., in the assay described in Example 33).

The term "hydrophobic lipid" refers to compounds having apolar groups that include, but are not limited to, long-chain saturated and unsaturated aliphatic hydrocarbon groups and such groups optionally substituted by one or more aromatic, cycloaliphatic, or heterocyclic group(s). Suitable examples include, but are not limited to, diacylglycerol, dialkylglycerol, N-N-dialkylamino, 1,2-diacyloxy-3-aminopropane, and 1,2-dialkyl-3-aminopropane.

The term "fusogenic" refers to the ability of a lipid particle, such as a LNP, to fuse with the membranes of a cell. The membranes can be either the plasma membrane or membranes surrounding organelles, e.g., endosome, nucleus, etc.

As used herein, the term "aqueous solution" refers to a composition comprising in whole, or in part, water.

As used herein, the term "organic lipid solution" refers to a composition comprising in whole, or in part, an organic solvent having a lipid.

"Distal site," as used herein, refers to a physically separated site, which is not limited to an adjacent capillary bed, but includes sites broadly distributed throughout an organism.

"Serum-stable" in relation to nucleic acid-lipid particles such as LNP means that the particle is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of lipid particles that leads to a broad biodistribution of an active agent or therapeutic agent, such as an interfering RNA or mRNA, within an organism. Some techniques of administration can lead to the systemic delivery of certain agents, but not others. Systemic delivery means that a useful, preferably therapeutic, amount of an agent is exposed to most parts of the body. To obtain broad biodistribution generally requires a blood lifetime such that the agent is not rapidly degraded or cleared (such as by first pass organs (liver, lung, etc.) or by rapid, nonspecific cell binding) before reaching a disease site distal to the site of administration. Systemic delivery of lipid particles can be by any means known in the art including, for example, intravenous, subcutaneous, and intraperitoneal. In a preferred embodiment, systemic delivery of lipid particles is by intravenous delivery.

"Local delivery," as used herein, refers to delivery of an active agent or therapeutic agent, such as an interfering RNA or mRNA, directly to a target site within an organism. For example, an agent can be locally delivered by direct injection into a disease site such as a tumor or other target site such as a site of inflammation or a target organ such as the liver, heart, pancreas, kidney, and the like.

The term "mammal" refers to any mammalian species such as a human, mouse, rat, dog, cat, hamster, guinea pig, rabbit, livestock, and the like.

The term "cancer" refers to any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, lung cancer, colon cancer, rectal cancer, anal cancer, bile duct cancer, small intestine cancer, stomach (gastric) cancer, esophageal cancer; gallbladder cancer, liver cancer, pancreatic cancer, appendix cancer, breast cancer, ovarian cancer; cervical cancer, prostate cancer, renal cancer (e.g., renal cell carcinoma), cancer of the central nervous system, glioblastoma, skin cancer, lymphomas, choriocarcinomas, head and neck cancers, osteogenic sarcomas, and blood cancers. Non-limiting examples of specific types of liver cancer include hepatocellular carcinoma (HCC), secondary liver cancer (e.g., caused by metastasis of some other non-liver cancer cell type), and hepatoblastoma. As used herein, a "tumor" comprises one or more cancerous cells.

The term "anionic precursor group" includes groups that are capable of forming an ion at physiological pH. For Example, the term includes the groups -CO₂H, -OP(=O)(OH)₂, -OS(=O)₂(OH), -O-S(=O)(OH), and -B(OH)₂. In one embodiment, the anionic precursor is -CO₂H.

In a particular embodiment, PEG-C-DMA has the following structure: 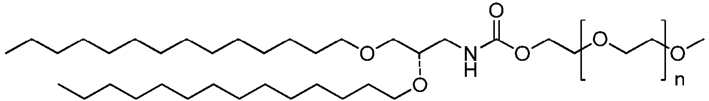 wherein n is selected so that the resulting polymer chain has a molecular weight of from about 500 to about 10,000. In one embodiment, n is selected so that the resulting polymer chain has a molecular weight of from about 500 to about 7500. In one embodiment, n is selected so that the resulting polymer chain has a molecular weight of from about 500 to about 6000. In one embodiment, n is selected so that the resulting polymer chain has a molecular weight of from about 500 to about 5000. In one embodiment, n is selected so that the resulting polymer chain has a molecular weight of from about 500 to about 3000. In one embodiment, n is selected so that the resulting polymer chain has a molecular weight of from about 1000 to about 7500. In one embodiment, n is selected so that the resulting polymer chain has a molecular weight of from about 1000 to about 6000. In one embodiment, n is selected so that the resulting polymer chain has a molecular weight of from about 1000 to about 5000. In one embodiment, n is selected so that the resulting polymer chain has a molecular weight of from about 1000 to about 3000. In another embodiment, n is selected so that the resulting polymer chain has a molecular weight of about 2000. PEG-C-DMA can be prepared as described by Heyes et al, Synthesis and Characterization of Novel Poly (Ethylene Glycol)-lipid Conjugates Suitable for use in Drug Delivery," Journal of Controlled Release, 2006, and in United States Patent Number 8,936,942.

### Description of the Embodiments

The present invention provides novel, serum-stable lipid particles comprising one or more active agents or therapeutic agents. Also described are methods of making the lipid particles, and methods of delivering and/or administering the lipid particles (e.g., for the treatment of a disease or disorder).

In one aspect, the present invention provides lipid particles comprising: (a) one or more active agents or therapeutic agents; (b) one or more ionizable lipids of the invention comprising from about 30 mol % to about 85 mol % of the total lipid present in the particle; (c) one or more non-cationic lipids comprising from about 13 mol % to about 49.5 mol % of the total lipid present in the particle; and (d) one or more conjugated lipids that inhibit aggregation of particles comprising from about 0.1 mol % to about 10 mol % of the total lipid present in the particle.

In one aspect, the present invention provides lipid particles comprising: (a) one or more active agents or therapeutic agents; (b) one or more ionizable lipids of the invention comprising from about 50 mol % to about 85 mol % of the total lipid present in the particle; (c) one or more non-cationic lipids comprising from about 13 mol % to about 49.5 mol % of the total lipid present in the particle; and (d) one or more conjugated lipids that inhibit aggregation of particles comprising from about 0.5 mol % to about 2 mol % of the total lipid present in the particle.

In certain embodiments, the active agent or therapeutic agent is fully encapsulated within the lipid portion of the lipid particle such that the active agent or therapeutic agent in the lipid particle is resistant in aqueous solution to enzymatic degradation, e.g., by a nuclease or protease. In certain other embodiments, the lipid particles are substantially non-toxic to mammals such as humans.

In some embodiments, the active agent or therapeutic agent comprises a nucleic acid. In certain instances, the nucleic acid comprises an interfering RNA molecule such as, e.g., an siRNA, aiRNA, miRNA, or mixtures thereof. In certain other instances, the nucleic acid comprises single-stranded or double-stranded DNA, RNA, or a DNA/RNA hybrid such as, e.g., an antisense oligonucleotide, a ribozyme, a plasmid, an immunostimulatory oligonucleotide, or mixtures thereof. In certain instances, the nucleic acid comprises an mRNA molecule.

In other embodiments, the active agent or therapeutic agent comprises a peptide or polypeptide. In certain instances, the peptide or polypeptide comprises an antibody such as, e.g., a polyclonal antibody, a monoclonal antibody, an antibody fragment; a humanized antibody, a recombinant antibody, a recombinant human antibody, a Primatized^{™} antibody, or mixtures thereof. In certain other instances, the peptide or polypeptide comprises a cytokine, a growth factor, an apoptotic factor, a differentiation-inducing factor, a cell-surface receptor, a ligand, a hormone, a small molecule (e.g., small organic molecule or compound), or mixtures thereof.

In one embodiment, the active agent or therapeutic agent comprises an siRNA. In one embodiment, the siRNA molecule comprises a double-stranded region of about 15 to about 60 nucleotides in length (e.g., about 15-60, 15-50, 15-40, 15-30, 15-25, or 19-25 nucleotides in length, or 15, 16, 17, 18, 19, 20, 21, 22, 23, 24, or 25 nucleotides in length). The siRNA molecules of the invention are capable of silencing the expression of a target sequence in vitro and/or in vivo.

In some embodiments, the siRNA molecule comprises at least one modified nucleotide. In certain preferred embodiments, the siRNA molecule comprises one, two, three, four, five, six, seven, eight, nine, ten, or more modified nucleotides in the double-stranded region. In certain instances, the siRNA comprises from about 1% to about 100% (e.g., about 1%, 5%, 10%, 15%, 20%, 25%, 30%, 35%, 40%, 45%, 50%, 55%, 60%, 65%, 70%, 75%, 80%, 85%, 90%, 95%, or 100%) modified nucleotides in the double-stranded region. In preferred embodiments, less than about 25% (e.g., less than about 25%, 20%, 15%, 10%, or 5%) or from about 1% to about 25% (e.g., from about 1%-25%, 5%-25%, 10%-25%, 15%-25%, 20%-25%, or 10%-20%) of the nucleotides in the double-stranded region comprise modified nucleotides.

In other embodiments, the siRNA molecule comprises modified nucleotides including, but not limited to, 2'-O-methyl (2'OMe) nucleotides, 2'-deoxy-2'-fluoro (2'F) nucleotides, 2'-deoxy nucleotides, 2'-O-(2-methoxyethyl) (MOE) nucleotides, locked nucleic acid (LNA) nucleotides, and mixtures thereof. In preferred embodiments, the siRNA comprises 2'OMe nucleotides (e.g., 2'OMe purine and/or pyrimidine nucleotides) such as, for example, 2'OMe-guanosine nucleotides, 2'OMe-uridine nucleotides, 2'OMe-adenosine nucleotides, 2'OMe-cytosine nucleotides, and mixtures thereof. In certain instances, the siRNA does not comprise 2'OMe-cytosine nucleotides. In other embodiments, the siRNA comprises a hairpin loop structure.

The siRNA may comprise modified nucleotides in one strand (i.e., sense or antisense) or both strands of the double-stranded region of the siRNA molecule. Preferably, uridine and/or guanosine nucleotides are modified at selective positions in the double-stranded region of the siRNA duplex. With regard to uridine nucleotide modifications, at least one, two, three, four, five, six, or more of the uridine nucleotides in the sense and/or antisense strand can be a modified uridine nucleotide such as a 2'OMe-uridine nucleotide. In some embodiments, every uridine nucleotide in the sense and/or antisense strand is a 2'OMe-uridine nucleotide. With regard to guanosine nucleotide modifications, at least one, two, three, four, five, six, or more of the guanosine nucleotides in the sense and/or antisense strand can be a modified guanosine nucleotide such as a 2'OMe-guanosine nucleotide. In some embodiments, every guanosine nucleotide in the sense and/or antisense strand is a 2'OMe-guanosine nucleotide.

In certain embodiments, at least one, two, three, four, five, six, seven, or more 5'-GU-3' motifs in an siRNA sequence may be modified, e.g., by introducing mismatches to eliminate the 5'-GU-3' motifs and/or by introducing modified nucleotides such as 2'OMe nucleotides. The 5'-GU-3' motif can be in the sense strand, the antisense strand, or both strands of the siRNA sequence. The 5'-GU-3' motifs may be adjacent to each other or, alternatively, they may be separated by 1, 2, 3, 4, 5, 6, 7, 8, 9, 10, 11, 12, or more nucleotides.

In some preferred embodiments, a modified siRNA molecule is less immunostimulatory than a corresponding unmodified siRNA sequence. In such embodiments, the modified siRNA molecule with reduced immunostimulatory properties advantageously retains RNAi activity against the target sequence. In another embodiment, the immunostimulatory properties of the modified siRNA molecule and its ability to silence target gene expression can be balanced or optimized by the introduction of minimal and selective 2'OMe modifications within the siRNA sequence such as, e.g., within the double-stranded region of the siRNA duplex. In certain instances, the modified siRNA is at least about 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, or 100% less immunostimulatory than the corresponding unmodified siRNA. It will be readily apparent to those of skill in the art that the immunostimulatory properties of the modified siRNA molecule and the corresponding unmodified siRNA molecule can be determined by, for example, measuring INF-α and/or IL-6 levels from about two to about twelve hours after systemic administration in a mammal or transfection of a mammalian responder cell using an appropriate lipid-based delivery system (such as the LNP delivery system disclosed herein).

In certain embodiments, a modified siRNA molecule has an IC₅₀ (i.e., half-maximal inhibitory concentration) less than or equal to ten-fold that of the corresponding unmodified siRNA (i.e., the modified siRNA has an IC₅₀ that is less than or equal to ten-times the IC₅₀ of the corresponding unmodified siRNA). In other embodiments, the modified siRNA has an IC₅₀ less than or equal to three-fold that of the corresponding unmodified siRNA sequence. In yet other embodiments, the modified siRNA has an IC₅₀ less than or equal to two-fold that of the corresponding unmodified siRNA. It will be readily apparent to those of skill in the art that a dose-response curve can be generated and the IC₅₀ values for the modified siRNA and the corresponding unmodified siRNA can be readily determined using methods known to those of skill in the art.

In yet another embodiment, a modified siRNA molecule is capable of silencing at least about 5%, 10%, 15%, 20%, 25%, 30%, 35%, 40%, 45%, 50%, 55%, 60%, 65%, 70%, 75%, 80%, 85%, 90%, 95%, or 100% of the expression of the target sequence relative to the corresponding unmodified siRNA sequence.

In some embodiments, the siRNA molecule does not comprise phosphate backbone modifications, e.g., in the sense and/or antisense strand of the double-stranded region. In other embodiments, the siRNA comprises one, two, three, four, or more phosphate backbone modifications, e.g., in the sense and/or antisense strand of the double-stranded region. In preferred embodiments, the siRNA does not comprise phosphate backbone modifications.

In further embodiments, the siRNA does not comprise 2'-deoxy nucleotides, e.g., in the sense and/or antisense strand of the double-stranded region. In yet further embodiments, the siRNA comprises one, two, three, four, or more 2'-deoxy nucleotides, e.g., in the sense and/or antisense strand of the double-stranded region. In preferred embodiments, the siRNA does not comprise 2'-deoxy nucleotides.

In certain instances, the nucleotide at the 3'-end of the double-stranded region in the sense and/or antisense strand is not a modified nucleotide. In certain other instances, the nucleotides near the 3'-end (e.g., within one, two, three, or four nucleotides of the 3'-end) of the double-stranded region in the sense and/or antisense strand are not modified nucleotides.

The siRNA molecules described herein may have 3' overhangs of one, two, three, four, or more nucleotides on one or both sides of the double-stranded region, or may lack overhangs (i.e., have blunt ends) on one or both sides of the double-stranded region. Preferably, the siRNA has 3' overhangs of two nucleotides on each side of the double-stranded region. In certain instances, the 3' overhang on the antisense strand has complementarity to the target sequence and the 3' overhang on the sense strand has complementarity to a complementary strand of the target sequence. Alternatively, the 3' overhangs do not have complementarity to the target sequence or the complementary strand thereof. In some embodiments, the 3' overhangs comprise one, two, three, four, or more nucleotides such as 2'-deoxy (2'H) nucleotides. In certain preferred embodiments, the 3' overhangs comprise deoxythymidine (dT) and/or uridine nucleotides. In other embodiments, one or more of the nucleotides in the 3' overhangs on one or both sides of the double-stranded region comprise modified nucleotides. Non-limiting examples of modified nucleotides are described above and include 2'OMe nucleotides, 2'-deoxy-2'F nucleotides, 2'-deoxy nucleotides, 2'-O-2-MOE nucleotides, LNA nucleotides, and mixtures thereof. In preferred embodiments, one, two, three, four, or more nucleotides in the 3' overhangs present on the sense and/or antisense strand of the siRNA comprise 2'OMe nucleotides (e.g., 2'OMe purine and/or pyrimidine nucleotides) such as, for example, 2'OMe-guanosine nucleotides, 2'OMe-uridine nucleotides, 2'OMe-adenosine nucleotides, 2'OMe-cytosine nucleotides, and mixtures thereof.

The siRNA may comprise at least one or a cocktail (e.g., at least two, three, four, five, six, seven, eight, nine, ten, or more) of unmodified and/or modified siRNA sequences that silence target gene expression. The cocktail of siRNA may comprise sequences which are directed to the same region or domain (e.g., a "hot spot") and/or to different regions or domains of one or more target genes. In certain instances, one or more (e.g., at least two, three, four, five, six, seven, eight, nine, ten, or more) modified siRNA that silence target gene expression are present in a cocktail. In certain other instances, one or more (e.g., at least two, three, four, five, six, seven, eight, nine, ten, or more) unmodified siRNA sequences that silence target gene expression are present in a cocktail.

In some embodiments, the antisense strand of the siRNA molecule comprises or consists of a sequence that is at least about 80%, 85%, 90%, 95%, 96%, 97%, 98%, or 99% complementary to the target sequence or a portion thereof. In other embodiments, the antisense strand of the siRNA molecule comprises or consists of a sequence that is 100% complementary to the target sequence or a portion thereof. In further embodiments, the antisense strand of the siRNA molecule comprises or consists of a sequence that specifically hybridizes to the target sequence or a portion thereof.

In further embodiments, the sense strand of the siRNA molecule comprises or consists of a sequence that is at least about 80%, 85%, 90%, 95%, 96%, 97%, 98%, or 99% identical to the target sequence or a portion thereof. In additional embodiments, the sense strand of the siRNA molecule comprises or consists of a sequence that is 100% identical to the target sequence or a portion thereof.

In some embodiments, the ionizable lipid of the invention may comprise from about 30 mol % to about 90 mol %, from about 30 mol % to about 85 mol %, from about 30 mol % to about 80 mol %, from about 30 mol % to about 75 mol %, from about 30 mol % to about 70 mol %, from about 30 mol % to about 65 mol %, or from about 30 mol % to about 60 mol % of the total lipid present in the particle.

In some embodiments, the ionizable lipid of the invention may comprise from about 40 mol % to about 90 mol %, from about 40 mol % to about 85 mol %, from about 40 mol % to about 80 mol %, from about 40 mol % to about 75 mol %, from about 40 mol % to about 70 mol %, from about 40 mol % to about 65 mol %, or from about 40 mol % to about 60 mol % of the total lipid present in the particle.

In other embodiments, the ionizable lipid of the invention may comprise from about 55 mol % to about 90 mol %, from about 55 mol % to about 85 mol %, from about 55 mol % to about 80 mol %, from about 55 mol % to about 75 mol %, from about 55 mol % to about 70 mol %, or from about 55 mol % to about 65 mol % of the total lipid present in the particle.

In yet other embodiments, the ionizable lipid of the invention may comprise from about 60 mol % to about 90 mol %, from about 60 mol % to about 85 mol %, from about 60 mol % to about 80 mol %, from about 60 mol % to about 75 mol %, or from about 60 mol % to about 70 mol % of the total lipid present in the particle.

In still yet other embodiments, the ionizable lipid of the invention may comprise from about 65 mol % to about 90 mol %, from about 65 mol % to about 85 mol %, from about 65 mol % to about 80 mol %, or from about 65 mol % to about 75 mol % of the total lipid present in the particle.

In further embodiments, the ionizable lipid of the invention may comprise from about 70 mol % to about 90 mol %, from about 70 mol % to about 85 mol %, from about 70 mol % to about 80 mol %, from about 75 mol % to about 90 mol %, from about 75 mol % to about 85 mol %, or from about 80 mol % to about 90 mol % of the total lipid present in the particle.

In additional embodiments, the ionizable lipid of the invention may comprise (at least) about 30, 35, 40, 45, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 mol % (or any fraction thereof or range therein) of the total lipid present in the particle.

In the lipid particles of the invention, the non-cationic lipid may comprise, e.g., one or more anionic lipids and/or neutral lipids. In preferred embodiments, the non-cationic lipid comprises one of the following neutral lipid components: (1) cholesterol or a derivative thereof (2) a phospholipid; or (3) a mixture of a phospholipid and cholesterol or a derivative thereof.

Examples of cholesterol derivatives include, but are not limited to, cholestanol, cholestanone, cholestenone, coprostanol, cholesteryl-2'-hydroxyethyl ether, cholesteryl-4'-hydroxybutyl ether, and mixtures thereof. The synthesis of cholesteryl-2'-hydroxyethyl ether is described herein.

The phospholipid may be a neutral lipid including, but not limited to, dipalmitoylphosphatidylcholine (DPPC), distearoylphosphatidylcholine (DSPC), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dipalmitoyl-phosphatidylethanolamine (DPPE), dimyristoyl-phosphatidylethanolamine (DMPE), distearoyl-phosphatidylethanolamine (DSPE), monomethyl-phosphatidylethanolamine, dimethyl-phosphatidylethanolamine, dielaidoyl-phosphatidylethanolamine (DEPE), stearoyloleoyl-phosphatidylethanolamine (SOPE), egg phosphatidylcholine (EPC), and mixtures thereof. In certain preferred embodiments, the phospholipid is DPPC, DSPC, or mixtures thereof.

In some embodiments, the non-cationic lipid (e.g., one or more phospholipids and/or cholesterol) may comprise from about 10 mol % to about 70 mol %, from about 15 mol % to about 70 mol %, from about 20 mol % to about 70 mol %, from about 25 mol % to about 70 mol %, from about 30 mol % to about 70 mol %, from about 10 mol % to about 55 mol %, from about 15 mol % to about 55 mol %, from about 20 mol % to about 55 mol %, from about 25 mol % to about 55 mol %, from about 30 mol % to about 55 mol %, from about 13 mol % to about 50 mol %, from about 15 mol % to about 50 mol % or from about 20 mol % to about 50 mol % of the total lipid present in the particle. When the non-cationic lipid is a mixture of a phospholipid and cholesterol or a cholesterol derivative, the mixture may comprise up to about 40, 50, or 60 mol % of the total lipid present in the particle.

In other embodiments, the non-cationic lipid (e.g., one or more phospholipids and/or cholesterol) may comprise from about 10 mol % to about 49.5 mol %, from about 13 mol % to about 49.5 mol %, from about 15 mol % to about 49.5 mol %, from about 20 mol % to about 49.5 mol %, from about 25 mol % to about 49.5 mol %, from about 30 mol % to about 49.5 mol %, from about 35 mol % to about 49.5 mol %, or from about 40 mol % to about 49.5 mol % of the total lipid present in the particle.

In yet other embodiments, the non-cationic lipid (e.g., one or more phospholipids and/or cholesterol) may comprise from about 10 mol % to about 45 mol %, from about 13 mol % to about 45 mol %, from about 15 mol % to about 45 mol %, from about 20 mol % to about 45 mol %, from about 25 mol % to about 45 mol %, from about 30 mol % to about 45 mol %, or from about 35 mol % to about 45 mol % of the total lipid present in the particle.

In still yet other embodiments, the non-cationic lipid (e.g., one or more phospholipids and/or cholesterol) may comprise from about 10 mol % to about 40 mol %, from about 13 mol % to about 40 mol %, from about 15 mol % to about 40 mol %, from about 20 mol % to about 40 mol %, from about 25 mol % to about 40 mol %, or from about 30 mol % to about 40 mol % of the total lipid present in the particle.

In further embodiments, the non-cationic lipid (e.g., one or more phospholipids and/or cholesterol) may comprise from about 10 mol % to about 35 mol %, from about 13 mol % to about 35 mol %, from about 15 mol % to about 35 mol %, from about 20 mol % to about 35 mol %, or from about 25 mol % to about 35 mol % of the total lipid present in the particle.

In yet further embodiments, the non-cationic lipid (e.g., one or more phospholipids and/or cholesterol) may comprise from about 10 mol % to about 30 mol %, from about 13 mol % to about 30 mol %, from about 15 mol % to about 30 mol %, from about 20 mol % to about 30 mol %, from about 10 mol % to about 25 mol %, from about 13 mol % to about 25 mol %, or from about 15 mol % to about 25 mol % of the total lipid present in the particle.

In additional embodiments, the non-cationic lipid (e.g., one or more phospholipids and/or cholesterol) may comprise (at least) about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 mol % (or any fraction thereof or range therein) of the total lipid present in the particle.

In certain preferred embodiments, the non-cationic lipid comprises cholesterol or a derivative thereof of from about 31.5 mol % to about 42.5 mol % of the total lipid present in the particle. As a non-limiting example, a phospholipid-free lipid particle of the invention may comprise cholesterol or a derivative thereof at about 37 mol % of the total lipid present in the particle. In other preferred embodiments, a phospholipid-free lipid particle of the invention may comprise cholesterol or a derivative thereof of from about 30 mol % to about 45 mol %, from about 30 mol % to about 40 mol %, from about 30 mol % to about 35 mol %, from about 35 mol % to about 45 mol %, from about 40 mol % to about 45 mol %, from about 32 mol % to about 45 mol %, from about 32 mol % to about 42 mol %, from about 32 mol % to about 40 mol %, from about 34 mol % to about 45 mol %, from about 34 mol % to about 42 mol %, from about 34 mol % to about 40 mol %, or about 30, 31, 32, 33, 34, 35, 36, 37, 38, 39, 40, 41, 42, 43, 44, or 45 mol % (or any fraction thereof or range therein) of the total lipid present in the particle.

In certain other preferred embodiments, the non-cationic lipid comprises a mixture of: (i) a phospholipid of from about 4 mol % to about 10 mol % of the total lipid present in the particle; and (ii) cholesterol or a derivative thereof of from about 30 mol % to about 40 mol % of the total lipid present in the particle. As a non-limiting example, a lipid particle comprising a mixture of a phospholipid and cholesterol may comprise DPPC at about 7 mol % and cholesterol at about 34 mol % of the total lipid present in the particle. In other embodiments, the non-cationic lipid comprises a mixture of: (i) a phospholipid of from about 3 mol % to about 15 mol %, from about 4 mol % to about 15 mol %, from about 4 mol % to about 12 mol %, from about 4 mol % to about 10 mol %, from about 4 mol % to about 8 mol %, from about 5 mol % to about 12 mol %, from about 5 mol % to about 9 mol %, from about 6 mol % to about 12 mol %, from about 6 mol % to about 10 mol %, or about 3, 4, 5, 6, 7, 8, 9, 10, 11, 12, 13, 14, or 15 mol % (or any fraction thereof or range therein) of the total lipid present in the particle; and (ii) cholesterol or a derivative thereof of from about 25 mol % to about 45 mol %, from about 30 mol % to about 45 mol %, from about 25 mol % to about 40 mol %, from about 30 mol % to about 40 mol %, from about 25 mol % to about 35 mol %, from about 30 mol % to about 35 mol %, from about 35 mol % to about 45 mol %, from about 40 mol % to about 45 mol %, from about 28 mol % to about 40 mol %, from about 28 mol % to about 38 mol %, from about 30 mol % to about 38 mol %, from about 32 mol % to about 36 mol %, or about 25, 26, 27, 28, 29, 30, 31, 32, 33, 34, 35, 36, 37, 38, 39, 40, 41, 42, 43, 44, or 45 mol % (or any fraction thereof or range therein) of the total lipid present in the particle.

In further preferred embodiments, the non-cationic lipid comprises a mixture of: (i) a phospholipid of from about 10 mol % to about 30 mol % of the total lipid present in the particle; and (ii) cholesterol or a derivative thereof of from about 10 mol % to about 30 mol % of the total lipid present in the particle. As a non-limiting example, a lipid particle comprising a mixture of a phospholipid and cholesterol may comprise DPPC at about 20 mol % and cholesterol at about 20 mol % of the total lipid present in the particle. In other embodiments, the non-cationic lipid comprises a mixture of: (i) a phospholipid of from about 10 mol % to about 30 mol %, from about 10 mol % to about 25 mol %, from about 10 mol % to about 20 mol %, from about 15 mol % to about 30 mol %, from about 20 mol % to about 30 mol %, from about 15 mol % to about 25 mol %, from about 12 mol % to about 28 mol %, from about 14 mol % to about 26 mol %, or about 10, 11, 12, 13, 14, 15, 16, 17, 18, 19, 20, 21, 22, 23, 24, 25, 26, 27, 28, 29, or 30 mol % (or any fraction thereof or range therein) of the total lipid present in the particle; and (ii) cholesterol or a derivative thereof of from about 10 mol % to about 30 mol %, from about 10 mol % to about 25 mol %, from about 10 mol % to about 20 mol %, from about 15 mol % to about 30 mol %, from about 20 mol % to about 30 mol %, from about 15 mol % to about 25 mol %, from about 12 mol % to about 28 mol %, from about 14 mol % to about 26 mol %, or about 10, 11, 12, 13, 14, 15, 16, 17, 18, 19, 20, 21, 22, 23, 24, 25, 26, 27, 28, 29, or 30 mol % (or any fraction thereof or range therein) of the total lipid present in the particle.

### Conjugated Lipid

In the lipid particles of the invention (e.g., LNP comprising, e.g., an interfering RNA such as siRNA, or mRNA), the conjugated lipid may comprise, e.g., one or more of the following: a polyethyleneglycol (PEG)-lipid conjugate, a polyamide (ATTA)-lipid conjugate, or mixtures thereof. In one preferred embodiment, the nucleic acid-lipid particles comprise either a PEG-lipid conjugate or an ATTA-lipid conjugate. The conjugated lipid s may comprise a PEG-lipid including, e.g., a PEG-diacylglycerol (DAG), a PEG dialkyloxypropyl (DAA), a PEG-phospholipid, a PEG-ceramide (Cer), or mixtures thereof. The PEG-DAA conjugate may be PEG-dilauryloxypropyl (C12), a PEG-dimyristyloxypropyl (C14), a PEG-dipalmityloxypropyl (C16), a PEG-distearyloxypropyl (C18), or mixtures thereof.

Additional PEG-lipid conjugates suitable for use in the invention include, but are not limited to, mPEG2000-1,2-di-O-alkyl-sn3-carbomoylglyceride (PEG-C-DOMG). The synthesis of PEG-C-DOMG is described in PCT Application No. PCT/US08/88676, filed Dec. 31, 2008. Yet additional PEG-lipid conjugates suitable for use in the invention include, without limitation, 1-[8'-(1,2-dimyristoyl-3-propanoxy)-carboxamido-3',6'-dioxaoctanyl]carbamoyl-w-methyl-poly(ethylene glycol) (2 KPEG-DMG). The synthesis of 2 KPEG-DMG is described in U.S. Pat. No. 7,404,969.

The PEG moiety of the PEG-lipid conjugates described herein may comprise an average molecular weight ranging from about 550 daltons to about 10,000 daltons. In certain instances, the PEG moiety has an average molecular weight of from about 750 daltons to about 5,000 daltons (e.g., from about 1,000 daltons to about 5,000 daltons, from about 1,500 daltons to about 3,000 daltons, from about 750 daltons to about 3,000 daltons, from about 750 daltons to about 2,000 daltons, etc.). In preferred embodiments, the PEG moiety has an average molecular weight of about 2,000 daltons or about 750 daltons.

In certain instances, the conjugated lipid (e.g., PEG-lipid conjugate) may comprise from about 0.1 to about 10% (or any fraction thereof or range therein) of the total lipid present in the particle. In certain instances, the conjugated lipid (e.g., PEG-lipid conjugate) may comprise from about 0.1 mol % to about 2 mol %, from about 0.5 mol % to about 2 mol %, from about 1 mol % to about 2 mol %, from about 0.6 mol % to about 1.9 mol %, from about 0.7 mol % to about 1.8 mol %, from about 0.8 mol % to about 1.7 mol %, from about 1 mol % to about 1.8 mol %, from about 1.2 mol % to about 1.8 mol %, from about 1.2 mol % to about 1.7 mol %, from about 1.3 mol % to about 1.6 mol %, from about 1.4 mol % to about 1.5 mol %, or about 1, 1.1, 1.2, 1.3, 1.4, 1.5, 1.6, 1.7, 1.8, 1.9, or 2 mol % (or any fraction thereof or range therein) of the total lipid present in the particle.

In the lipid particles of the invention, the active agent or therapeutic agent may be fully encapsulated within the lipid portion of the particle, thereby protecting the active agent or therapeutic agent from enzymatic degradation. In preferred embodiments, a LNP comprising a nucleic acid, such as an interfering RNA (e.g., siRNA) or mRNA, is fully encapsulated within the lipid portion of the particle, thereby protecting the nucleic acid from nuclease degradation. In certain instances, the nucleic acid in the LNP is not substantially degraded after exposure of the particle to a nuclease at 37° C. for at least about 20, 30, 45, or 60 minutes. In certain other instances, the nucleic acid in the LNP is not substantially degraded after incubation of the particle in serum at 37° C. for at least about 30, 45, or 60 minutes or at least about 2, 3, 4, 5, 6, 7, 8, 9, 10, 12, 14, 16, 18, 20, 22, 24, 26, 28, 30, 32, 34, or 36 hours. In other embodiments, the active agent or therapeutic agent (e.g., nucleic acid such as siRNA) is complexed with the lipid portion of the particle. One of the benefits of the formulations of the present invention is that the lipid particle compositions are substantially non-toxic to mammals such as humans.

The term "fully encapsulated" indicates that the active agent or therapeutic agent in the lipid particle is not significantly degraded after exposure to serum or a nuclease or protease assay that would significantly degrade free DNA, RNA, or protein. In a fully encapsulated system, preferably less than about 25% of the active agent or therapeutic agent in the particle is degraded in a treatment that would normally degrade 100% of free active agent or therapeutic agent, more preferably less than about 10%, and most preferably less than about 5% of the active agent or therapeutic agent in the particle is degraded. In the context of nucleic acid therapeutic agents, full encapsulation may be determined by an Oligreen^{®} assay. Oligreen^{®} is an ultra-sensitive fluorescent nucleic acid stain for quantitating oligonucleotides and single-stranded DNA or RNA in solution (available from Invitrogen Corporation; Carlsbad, Calif.). "Fully encapsulated" also indicates that the lipid particles are serum-stable, that is, that they do not rapidly decompose into their component parts upon in vivo administration.

In another aspect, the present invention provides a lipid particle (e.g., LNP) composition comprising a plurality of lipid particles. In preferred embodiments, the active agent or therapeutic agent (e.g., nucleic acid) is fully encapsulated within the lipid portion of the lipid particles (e.g., LNP), such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, %, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the lipid particles (e.g., LNP) have the active agent or therapeutic agent encapsulated therein.

Typically, the lipid particles (e.g., LNP) of the invention have a lipid:active agent (e.g., lipid:nucleic acid) ratio (mass/mass ratio) of from about 1 to about 100. In some instances, the lipid:active agent (e.g., lipid:nucleic acid) ratio (mass/mass ratio) ranges from about 1 to about 50, from about 2 to about 25, from about 3 to about 20, from about 4 to about 15, or from about 5 to about 10. In preferred embodiments, the lipid particles of the invention have a lipid:active agent (e.g., lipid:nucleic acid) ratio (mass/mass ratio) of from about 5 to about 15, e.g., about 5, 6, 7, 8, 9, 10, 11, 12, 13, 14, or 15 (or any fraction thereof or range therein).

Typically, the lipid particles (e.g., LNP) of the invention have a mean diameter of from about 40 nm to about 150 nm. In preferred embodiments, the lipid particles (e.g., LNP) of the invention have a mean diameter of from about 40 nm to about 130 nm, from about 40 nm to about 120 nm, from about 40 nm to about 100 nm, from about 50 nm to about 120 nm, from about 50 nm to about 100 nm, from about 40 nm to about 80 nm, from about 40 nm to about 60 nm, from about 50 nm to about 60 nm, from about 60 nm to about 120 nm, from about 60 nm to about 110 nm, from about 60 nm to about 100 nm, from about 60 nm to about 90 nm, from about 60 nm to about 80 nm, from about 70 nm to about 120 nm, from about 70 nm to about 110 nm, from about 70 nm to about 100 nm, from about 70 nm to about 90 nm, from about 70 nm to about 80 nm, or less than about 150 nm, 120 nm, 110 nm, 100 nm, 90 nm, or 80 nm (or any fraction thereof or range therein).

In one specific embodiment of the invention, the LNP comprises: (a) one or more unmodified and/or modified nucleic acid molecules (e.g., interfering RNA that silence target gene expression, such as siRNA, aiRNA, miRNA; or mRNA that result in target protein expression); (b) an ionizable lipid of the invention comprising from about 56.5 mol % to about 66.5 mol % of the total lipid present in the particle; (c) a non-cationic lipid comprising from about 31.5 mol % to about 42.5 mol % of the total lipid present in the particle; and (d) a conjugated lipid that inhibits aggregation of particles comprising from about 1 mol % to about 2 mol % of the total lipid present in the particle. This specific embodiment of LNP is generally referred to herein as the "1:62" formulation.

In another specific embodiment of the invention, the LNP comprises: (a) one or more unmodified and/or modified nucleic acid molecules (e.g., interfering RNA that silence target gene expression, such as siRNA, aiRNA, miRNA; or mRNA that result in target protein expression); (b) an ionizable lipid of the invention comprising from about 52 mol % to about 62 mol % of the total lipid present in the particle; (c) a non-cationic lipid comprising from about 36 mol % to about 47 mol % of the total lipid present in the particle; and (d) a conjugated lipid that inhibits aggregation of particles comprising from about 1 mol % to about 2 mol % of the total lipid present in the particle. This specific embodiment of LNP is generally referred to herein as the "1:57" formulation.

The present invention also provides a pharmaceutical composition comprising a lipid particle (e.g., LNP) described herein and a pharmaceutically acceptable carrier.

The present invention finds use in a method for introducing one or more active agents or therapeutic agents (e.g., nucleic acid) into a cell, comprising contacting the cell with a lipid particle (e.g., LNP) described herein. In one embodiment, the cell is in a mammal and the mammal is a human. The present invention also finds use in a method for the in vivo delivery of one or more active agents or therapeutic agents (e.g., nucleic acid), comprising administering to a mammalian subject a lipid particle (e.g., LNP) described herein. In a preferred embodiment, the mode of administration includes, but is not limited to, oral, intranasal, intravenous, intraperitoneal, intramuscular, intra-articular, intralesional, intratracheal, subcutaneous, and intradermal. Preferably, the mammalian subject is a human.

In one embodiment, at least about 5%, 10%, 15%, 20%, or 25% of the total injected dose of the lipid particles (e.g., LNP) is present in plasma about 8, 12, 24, 36, or 48 hours after injection. In other embodiments, more than about 20%, 30%, 40% and as much as about 60%, 70% or 80% of the total injected dose of the lipid particles (e.g., LNP) is present in plasma about 8, 12, 24, 36, or 48 hours after injection. In certain instances, more than about 10% of a plurality of the particles is present in the plasma of a mammal about 1 hour after administration. In certain other instances, the presence of the lipid particles (e.g., LNP) is detectable at least about 1 hour after administration of the particle. In certain embodiments, the presence of an active agent or therapeutic agent, such as an interfering RNA (e.g., siRNA) or mRNA is detectable in cells of the at about 8, 12, 24, 36, 48, 60, 72 or 96 hours after administration (e.g., lung, liver, tumor, or at a site of inflammation). In other embodiments, downregulation of expression of a target sequence by an active agent or therapeutic agent, such as an interfering RNA (e.g., siRNA) is detectable at about 8, 12, 24, 36, 48, 60, 72 or 96 hours after administration. In yet other embodiments, downregulation of expression of a target sequence by an active agent or therapeutic agent such as an interfering RNA (e.g., siRNA) occurs preferentially in tumor cells or in cells at a site of inflammation. In further embodiments, the presence or effect of an active agent or therapeutic agent such as an interfering RNA (e.g., siRNA) in cells at a site proximal or distal to the site of administration or in cells of the lung, liver, or a tumor is detectable at about 12, 24, 48, 72, or 96 hours, or at about 6, 8, 10, 12, 14, 16, 18, 19, 20, 22, 24, 26, or 28 days after administration. In other embodiments, upregulation of expression of a target sequence by an active agent or therapeutic agent, such as an mRNA or self-amplifying RNA is detectable at about 8, 12, 24, 36, 48, 60, 72 or 96 hours after administration. In yet other embodiments, upregulation of expression of a target sequence by an active agent or therapeutic agent such as an mRNA or self-amplifying RNA occurs preferentially in tumor cells or in cells at a site of inflammation. In further embodiments, the presence or effect of an active agent or therapeutic agent such as an mRNA or self-replicating RNA in cells at a site proximal or distal to the site of administration or in cells of the lung, liver, or a tumor is detectable at about 12, 24, 48, 72, or 96 hours, or at about 6, 8, 10, 12, 14, 16, 18, 19, 20, 22, 24, 26, or 28 days after administration. In additional embodiments, the lipid particles (e.g., LNP) of the invention are administered parenterally or intraperitoneally.

In some embodiments, the lipid particles (e.g., LNP) of the invention are particularly useful in methods for the therapeutic delivery of one or more nucleic acids comprising an interfering RNA sequence (e.g., siRNA). In particular, this invention finds use in in vitro and in vivo methods for treatment of a disease or disorder in a mammal (e.g., a rodent such as a mouse or a primate such as a human, chimpanzee, or monkey) by downregulating or silencing the transcription and/or translation of one or more target nucleic acid sequences or genes of interest. As a non-limiting example, the invention is useful for in vivo delivery of interfering RNA (e.g., siRNA) to the liver and/or tumor of a mammalian subject. In certain embodiments, the disease or disorder is associated with expression and/or overexpression of a gene and expression or overexpression of the gene is reduced by the interfering RNA (e.g., siRNA). In certain other embodiments, a therapeutically effective amount of the lipid particle (e.g., LNP) may be administered to the mammal. In some instances, an interfering RNA (e.g., siRNA) is formulated into a LNP, and the particles are administered to patients requiring such treatment. In other instances, cells are removed from a patient, the interfering RNA (e.g., siRNA) is delivered in vitro (e.g., using a LNP described herein), and the cells are reinjected into the patient.

In an additional aspect, the present invention provides lipid particles (e.g., LNP) comprising asymmetrical interfering RNA (aiRNA) molecules that silence the expression of a target gene and methods of using such particles to silence target gene expression.

In one embodiment, the aiRNA molecule comprises a double-stranded (duplex) region of about 10 to about 25 (base paired) nucleotides in length, wherein the aiRNA molecule comprises an antisense strand comprising 5' and 3' overhangs, and wherein the aiRNA molecule is capable of silencing target gene expression.

In certain instances, the aiRNA molecule comprises a double-stranded (duplex) region of about 12-20, 12-19, 12-18, 13-17, or 14-17 (base paired) nucleotides in length, more typically 12, 13, 14, 15, 16, 17, 18, 19, or 20 (base paired) nucleotides in length. In certain other instances, the 5' and 3' overhangs on the antisense strand comprise sequences that are complementary to the target RNA sequence, and may optionally further comprise nontargeting sequences. In some embodiments, each of the 5' and 3' overhangs on the antisense strand comprises or consists of one, two, three, four, five, six, seven, or more nucleotides.

In other embodiments, the aiRNA molecule comprises modified nucleotides selected from the group consisting of 2'OMe nucleotides, 2'F nucleotides, 2'-deoxy nucleotides, 2'-O-MOE nucleotides, LNA nucleotides, and mixtures thereof. In a preferred embodiment, the aiRNA molecule comprises 2'OMe nucleotides. As a non-limiting example, the 2'OMe nucleotides may be selected from the group consisting of 2'OMe-guanosine nucleotides, 2'OMe-uridine nucleotides, and mixtures thereof.

In a related aspect, the present invention provides lipid particles (e.g., LNP) comprising microRNA (miRNA) molecules that silence the expression of a target gene and methods of using such compositions to silence target gene expression.

In one embodiment, the miRNA molecule comprises about 15 to about 60 nucleotides in length, wherein the miRNA molecule is capable of silencing target gene expression.

In certain instances, the miRNA molecule comprises about 15-50, 15-40, or 15-30 nucleotides in length, more typically about 15-25 or 19-25 nucleotides in length, and are preferably about 20-24, 21-22, or 21-23 nucleotides in length. In a preferred embodiment, the miRNA molecule is a mature miRNA molecule targeting an RNA sequence of interest.

In some embodiments, the miRNA molecule comprises modified nucleotides selected from the group consisting of 2'OMe nucleotides, 2'F nucleotides, 2'-deoxy nucleotides, 2'-O-MOE nucleotides, LNA nucleotides, and mixtures thereof. In a preferred embodiment, the miRNA molecule comprises 2'OMe nucleotides. As a non-limiting example, the 2'OMe nucleotides may be selected from the group consisting of 2'OMe-guanosine nucleotides, 2'OMe-uridine nucleotides, and mixtures thereof.

In some embodiments, the lipid particles (e.g., LNP) of the invention are useful in methods for the therapeutic delivery of one or more mRNA molecules. In particular, this invention finds use in in vitro and in vivo methods for treatment of a disease or disorder in a mammal (e.g., a rodent such as a mouse or a primate such as a human, chimpanzee, or monkey) through the expression of one or more target proteins. As a non-limiting example, the invention is useful for in vivo delivery of one or more mRNA molecules to a mammalian subject. In certain other embodiments, a therapeutically effective amount of the lipid particle (e.g., LNP) may be administered to the mammal. In some instances, one or more mRNA molecules are formulated into a LNP, and the particles are administered to patients requiring such treatment. In other instances, cells are removed from a patient, one or more mRNA molecules are delivered in vitro (e.g., using a LNP described herein), and the cells are reinjected into the patient.

As such, the lipid particles of the invention (e.g., LNP) are advantageous and suitable for use in the administration of active agents or therapeutic agents, such as nucleic acid (e.g., interfering RNA such as siRNA, aiRNA, and/or miRNA; or mRNA; guide RNA; self-amplifying RNA; circular RNA; DNA, including plasmid DNA and closed ended DNA to a subject (e.g., a mammal such as a human) because they are stable in circulation, of a size required for pharmacodynamic behavior resulting in access to extravascular sites, and are capable of reaching target cell populations.

### Active Agents

Active agents (e.g., therapeutic agents) include any molecule or compound capable of exerting a desired effect on a cell, tissue, organ, or subject. Such effects may be, e.g., biological, physiological, and/or cosmetic. Active agents may be any type of molecule or compound including, but not limited to, nucleic acids, peptides, polypeptides, small molecules, and mixtures thereof. Non-limiting examples of nucleic acids include interfering RNA molecules (e.g., siRNA, aiRNA, miRNA), antisense oligonucleotides, mRNA, self-amplifying RNA, plasmids, ribozymes, immunostimulatory oligonucleotides, and mixtures thereof. Examples of peptides or polypeptides include, without limitation, antibodies (e.g., polyclonal antibodies, monoclonal antibodies, antibody fragments; humanized antibodies, recombinant antibodies, recombinant human antibodies, Primatized^{™} antibodies), cytokines, growth factors, apoptotic factors, differentiation-inducing factors, cell-surface receptors and their ligands, hormones, and mixtures thereof. Examples of small molecules include, but are not limited to, small organic molecules or compounds such as any conventional agent or drug known to those of skill in the art.

In some embodiments, the active agent is a therapeutic agent, or a salt or derivative thereof. Therapeutic agent derivatives may be therapeutically active themselves or they may be prodrugs, which become active upon further modification. Thus, in one embodiment, a therapeutic agent derivative retains some or all of the therapeutic activity as compared to the unmodified agent, while in another embodiment, a therapeutic agent derivative is a prodrug that lacks therapeutic activity, but becomes active upon further modification.

### Nucleic Acids

In certain embodiments, lipid particles of the present invention are associated with a nucleic acid, resulting in a nucleic acid-lipid particle (e.g., LNP). In some embodiments, the nucleic acid is fully encapsulated in the lipid particle. As used herein, the term "nucleic acid" includes any oligonucleotide or polynucleotide, with fragments containing up to 60 nucleotides generally termed oligonucleotides, and longer fragments termed polynucleotides. In particular embodiments, oligonucletoides of the invention are from about 15 to about 60 nucleotides in length. Nucleic acid may be administered alone in the lipid particles of the invention, or in combination (e.g., co-administered) with lipid particles of the invention comprising peptides, polypeptides, or small molecules such as conventional drugs.

In the context of this invention, the terms "polynucleotide" and "oligonucleotide" refer to a polymer or oligomer of nucleotide or nucleoside monomers consisting of naturally-occurring bases, sugars and intersugar (backbone) linkages. The terms "polynucleotide" and "oligonucleotide" also include polymers or oligomers comprising non-naturally occurring monomers, or portions thereof, which function similarly. Such modified or substituted oligonucleotides are often preferred over native forms because of properties such as, for example, enhanced cellular uptake, reduced immunogenicity, and increased stability in the presence of nucleases.

Oligonucleotides are generally classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose.

The nucleic acid that is present in a lipid-nucleic acid particle according to this invention includes any form of nucleic acid that is known. The nucleic acids used herein can be single-stranded DNA or RNA, or double-stranded DNA or RNA, or DNA-RNA hybrids. Examples of double-stranded DNA are described herein and include, e.g., structural genes, genes including control and termination regions, and self-replicating systems such as viral or plasmid DNA. Examples of double-stranded RNA are described herein and include, e.g., siRNA and other RNAi agents such as aiRNA and pre-miRNA. Single-stranded nucleic acids include, e.g., antisense oligonucleotides, ribozymes, mature miRNA, and triplex-forming oligonucleotides.

Nucleic acids of the invention may be of various lengths, generally dependent upon the particular form of nucleic acid. For example, in particular embodiments, plasmids or genes may be from about 1,000 to about 100,000 nucleotide residues in length. In particular embodiments, oligonucleotides may range from about 10 to about 100 nucleotides in length. In various related embodiments, oligonucleotides, both single-stranded, double-stranded, and triple-stranded, may range in length from about 10 to about 60 nucleotides, from about 15 to about 60 nucleotides, from about 20 to about 50 nucleotides, from about 15 to about 30 nucleotides, or from about 20 to about 30 nucleotides in length.

In particular embodiments, an oligonucleotide (or a strand thereof) of the invention specifically hybridizes to or is complementary to a target polynucleotide sequence. The terms "specifically hybridizable" and "complementary" as used herein indicate a sufficient degree of complementarity such that stable and specific binding occurs between the DNA or RNA target and the oligonucleotide. It is understood that an oligonucleotide need not be 100% complementary to its target nucleic acid sequence to be specifically hybridizable. In preferred embodiments, an oligonucleotide is specifically hybridizable when binding of the oligonucleotide to the target sequence interferes with the normal function of the target sequence to cause a loss of utility or expression therefrom, and there is a sufficient degree of complementarity to avoid non-specific binding of the oligonucleotide to non-target sequences under conditions in which specific binding is desired, i.e., under physiological conditions in the case of in vivo assays or therapeutic treatment, or, in the case of in vitro assays, under conditions in which the assays are conducted. Thus, the oligonucleotide may include 1, 2, 3, or more base substitutions as compared to the region of a gene or mRNA sequence that it is targeting or to which it specifically hybridizes.

### siRNA

The siRNA component of the nucleic acid-lipid particles of the present invention is capable of silencing the expression of a target gene of interest. Each strand of the siRNA duplex is typically about 15 to about 60 nucleotides in length, preferably about 15 to about 30 nucleotides in length. In certain embodiments, the siRNA comprises at least one modified nucleotide. The modified siRNA is generally less immunostimulatory than a corresponding unmodified siRNA sequence and retains RNAi activity against the target gene of interest. In some embodiments, the modified siRNA contains at least one 2'OMe purine or pyrimidine nucleotide such as a 2'OMe-guanosine, 2'OMe-uridine, 2'OMe-adenosine, and/or 2'OMe-cytosine nucleotide. In preferred embodiments, one or more of the uridine and/or guanosine nucleotides are modified. The modified nucleotides can be present in one strand (i.e., sense or antisense) or both strands of the siRNA. The siRNA sequences may have overhangs (e.g., 3' or 5' overhangs as described in Elbashir et al., Genes Dev., 15:188 (2001) or Nykänen et al., Cell, 107:309 (2001)), or may lack overhangs (i.e., have blunt ends).

The modified siRNA generally comprises from about 1% to about 100% (e.g., about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, or 100%) modified nucleotides in the double-stranded region of the siRNA duplex. In certain embodiments, one, two, three, four, five, six, seven, eight, nine, ten, or more of the nucleotides in the double-stranded region of the siRNA comprise modified nucleotides.

In some embodiments, less than about 25% (e.g., less than about 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, or 1%) of the nucleotides in the double-stranded region of the siRNA comprise modified nucleotides.

In other embodiments, from about 1% to about 25% (e.g., from about 1%-25%, 2%-25%, 3%-25%, 4%-25%, 5%-25%, 6%-25%, 7%-25%, 8%-25%, 9%-25%, 10%-25%, 11%-25%, 12%-25%, 13%-25%, 14%-25%, 15%-25%, 16%-25%, 17%-25%, 18%-25%, 19%-25%, 20%-25%, 21%-25%, 22%-25%, 23%-25%, 24%-25%, etc.) or from about 1% to about 20% (e.g., from about 1%-20%, 2%-20%, 3%-20%, 4%-20%, 5%-20%, 6%-20%, 7%-20%, 8%-20%, 9%-20%, 10%-20%, 11%-20%, 12%-20%, 13%-20%, 14%-20%, 15%-20%, 16%-20%, 17%-20%, 18%-20%, 19%-20%, 1%-19%, 2%-19%, 3%-19%, 4%-19%, 5%-19%, 6%-19%, 7%-19%, 8%-19%, 9%-19%, 10%-19%, 11%-19%, 12%-19%, 13%-19%, 14%-19%, 15%-19%, 16%-19%, 17%-19%, 18%-19%, 1%-18%, 2%-18%, 3%-18%, 4%-18%, 5%-18%, 6%-18%, 7%-18%, 8%-18%, 9%-18%, 10%-18%, 11%-18%, 12%-18%, 13%-18%, 14%-18%, 15%-18%, 16%-18%, 17%-18%, 1%-17%, 2%-17%, 3%-17%, 4%-17%, 5%-17%, 6%-17%, 7%-17%, 8%-17%, 9%-17%, 10%-17%, 11%-17%, 12%-17%, 13%-17%, 14%-17%, 15%-17%, 16%-17%, 1%-16%, 2%-16%, 3%-16%, 4%-16%, 5%-16%, 6%-16%, 7%-16%, 8%-16%, 9%-16%, 10%-16%, 11%-16%, 12%-16%, 13%-16%, 14%-16%, 15%-16%, 1%-15%, 2%-15%, 3%-15%, 4%-15%, 5%-15%, 6%-15%, 7%-15%, 8%-15%, 9%-15%, 10%-15%, 11%-15%, 12%-15%, 13%-15%, 14%-15%, etc.) of the nucleotides in the double-stranded region of the siRNA comprise modified nucleotides.

In further embodiments, e.g., when one or both strands of the siRNA are selectively modified at uridine and/or guanosine nucleotides, the resulting modified siRNA can comprise less than about 30% modified nucleotides (e.g., less than about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, or 1% modified nucleotides) or from about 1% to about 30% modified nucleotides (e.g., from about 1%-30%, 2%-30%, 3%-30%, 4%-30%, 5%-30%, 6%-30%, 7%-30%, 8%-30%, 9%-30%, 10%-30%, 11%-30%, 12%-30%, 13%-30%, 14%-30%, 15%-30%, 16%-30%, 17%-30%, 18%-30%, 19%-30%, 20%-30%, 21%-30%, 22%-30%, 23%-30%, 24%-30%, 25%-30%, 26%-30%, 27%-30%, 28%-30%, or 29%-30% modified nucleotides).

### Selection of siRNA Sequences

Suitable siRNA sequences can be identified using any means known in the art. Typically, the methods described in Elbashir et al., Nature, 411:494-498 (2001) and Elbashir et al., EMBO J., 20:6877-6888 (2001) are combined with rational design rules set forth in Reynolds et al., Nature Biotech., 22(3):326-330 (2004).

Generally, the nucleotide sequence 3' of the AUG start codon of a transcript from the target gene of interest is scanned for dinucleotide sequences (e.g., AA, NA, CC, GG, or UU, wherein N=C, G, or U) (see, e.g., Elbashir et al., EMBO J., 20:6877-6888 (2001)). The nucleotides immediately 3' to the dinucleotide sequences are identified as potential siRNA sequences (i.e., a target sequence or a sense strand sequence). Typically, the 19, 21, 23, 25, 27, 29, 31, 33, 35, or more nucleotides immediately 3' to the dinucleotide sequences are identified as potential siRNA sequences. In some embodiments, the dinucleotide sequence is an AA or NA sequence and the 19 nucleotides immediately 3' to the AA or NA dinucleotide are identified as potential siRNA sequences. siRNA sequences are usually spaced at different positions along the length of the target gene. To further enhance silencing efficiency of the siRNA sequences, potential siRNA sequences may be analyzed to identify sites that do not contain regions of homology to other coding sequences, e.g., in the target cell or organism. For example, a suitable siRNA sequence of about 21 base pairs typically will not have more than 16-17 contiguous base pairs of homology to coding sequences in the target cell or organism. If the siRNA sequences are to be expressed from an RNA Pol III promoter, siRNA sequences lacking more than 4 contiguous A's or T's are selected.

Once a potential siRNA sequence has been identified, a complementary sequence (i.e., an antisense strand sequence) can be designed. A potential siRNA sequence can also be analyzed using a variety of criteria known in the art. For example, to enhance their silencing efficiency, the siRNA sequences may be analyzed by a rational design algorithm to identify sequences that have one or more of the following features: (1) G/C content of about 25% to about 60% G/C; (2) at least 3 A/Us at positions 15-19 of the sense strand; (3) no internal repeats; (4) an A at position 19 of the sense strand; (5) an A at position 3 of the sense strand; (6) a U at position 10 of the sense strand; (7) no G/C at position 19 of the sense strand; and (8) no G at position 13 of the sense strand. siRNA design tools that incorporate algorithms that assign suitable values of each of these features and are useful for selection of siRNA can be found at, e.g., http://boz094.ust.hk/RNAi/siRNA. One of skill in the art will appreciate that sequences with one or more of the foregoing characteristics may be selected for further analysis and testing as potential siRNA sequences.

Additionally, potential siRNA sequences with one or more of the following criteria can often be eliminated as siRNA: (1) sequences comprising a stretch of 4 or more of the same base in a row; (2) sequences comprising homopolymers of Gs (i.e., to reduce possible non-specific effects due to structural characteristics of these polymers; (3) sequences comprising triple base motifs (e.g., GGG, CCC, AAA, or TTT); (4) sequences comprising stretches of 7 or more G/Cs in a row; and (5) sequences comprising direct repeats of 4 or more bases within the candidates resulting in internal fold-back structures. However, one of skill in the art will appreciate that sequences with one or more of the foregoing characteristics may still be selected for further analysis and testing as potential siRNA sequences.

In some embodiments, potential siRNA sequences may be further analyzed based on siRNA duplex asymmetry as described in, e.g., Khvorova et al., Cell, 115:209-216 (2003); and Schwarz et al., Cell, 115:199-208 (2003). In other embodiments, potential siRNA sequences may be further analyzed based on secondary structure at the target site as described in, e.g., Luo et al., Biophys. Res. Commun., 318:303-310 (2004). For example, secondary structure at the target site can be modeled using the Mfold algorithm (available at http://www.bioinfo.rpi.edu/applications/mfold/rna/form1.cgi) to select siRNA sequences which favor accessibility at the target site where less secondary structure in the form of base-pairing and stem-loops is present.

Once a potential siRNA sequence has been identified, the sequence can be analyzed for the presence of any immunostimulatory properties, e.g., using an in vitro cytokine assay or an in vivo animal model. Motifs in the sense and/or antisense strand of the siRNA sequence such as GU-rich motifs (e.g., 5'-GU-3',5'-UGU-3',5'-GUGU-3',5'-UGUGU-3', etc.) can also provide an indication of whether the sequence may be immunostimulatory. Once an siRNA molecule is found to be immunostimulatory, it can then be modified to decrease its immunostimulatory properties as described herein. As a non-limiting example, an siRNA sequence can be contacted with a mammalian responder cell under conditions such that the cell produces a detectable immune response to determine whether the siRNA is an immunostimulatory or a non-immunostimulatory siRNA. The mammalian responder cell may be from a naive mammal (i.e., a mammal that has not previously been in contact with the gene product of the siRNA sequence). The mammalian responder cell may be, e.g., a peripheral blood mononuclear cell (PBMC), a macrophage, and the like. The detectable immune response may comprise production of a cytokine or growth factor such as, e.g., TNF-α, IFN-α, IFN-β, IFN-γ, IL-6, IL-12, or a combination thereof. An siRNA molecule identified as being immunostimulatory can then be modified to decrease its immunostimulatory properties by replacing at least one of the nucleotides on the sense and/or antisense strand with modified nucleotides. For example, less than about 30% (e.g., less than about 30%, 25%, 20%, 15%, 10%, or 5%) of the nucleotides in the double-stranded region of the siRNA duplex can be replaced with modified nucleotides such as 2'OMe nucleotides. The modified siRNA can then be contacted with a mammalian responder cell as described above to confirm that its immunostimulatory properties have been reduced or abrogated.

Suitable in vitro assays for detecting an immune response include, but are not limited to, the double monoclonal antibody sandwich immunoassay technique of David et al. (U.S. Pat. No. 4,376,110); monoclonal-polyclonal antibody sandwich assays (Wide et al., in Kirkham and Hunter, eds., Radioimmunoassay Methods, E. and S. Livingstone, Edinburgh (1970)); the "Western blot" method of Gordon et al. (U.S. Pat. No. 4,452,901); immunoprecipitation of labeled ligand (Brown et al., J. Biol. Chem., 255:4980-4983 (1980)); enzyme-linked immunosorbent assays (ELISA) as described, for example, by Raines et al., J. Biol. Chem., 257:5154-5160 (1982); immunocytochemical techniques, including the use of fluorochromes (Brooks et al., Clin. Exp. Immunol., 39:477 (1980)); and neutralization of activity (Bowen-Pope et al., Proc. Natl. Acad. Sci. USA, 81:2396-2400 (1984)). In addition to the immunoassays described above, a number of other immunoassays are available, including those described in U.S. Pat. Nos. 3,817,827; 3,850,752; 3,901,654; 3,935,074; 3,984,533; 3,996,345; 4,034,074; and 4,098,876.

A non-limiting example of an in vivo model for detecting an immune response includes an in vivo mouse cytokine induction assay as described in, e.g., Judge et al., Mol. Ther., 13:494-505 (2006). In certain embodiments, the assay that can be performed as follows: (1) siRNA can be administered by standard intravenous injection in the lateral tail vein; (2) blood can be collected by cardiac puncture about 6 hours after administration and processed as plasma for cytokine analysis; and (3) cytokines can be quantified using sandwich ELISA kits according to the manufacturer's instructions (e.g., mouse and human IFN-α (PBL Biomedical; Piscataway, N.J.); human IL-6 and TNF-α (eBioscience; San Diego, Calif.); and mouse IL-6, TNF-α, and IFN-γ (BD Biosciences; San Diego, Calif.)).

Monoclonal antibodies that specifically bind cytokines and growth factors are commercially available from multiple sources and can be generated using methods known in the art (see, e.g., Kohler et al., Nature, 256: 495-497 (1975) and Harlow and Lane, ANTIBODIES, A LABORATORY MANUAL, Cold Spring Harbor Publication, New York (1999)). Generation of monoclonal antibodies has been previously described and can be accomplished by any means known in the art (Buhring et al., in Hybridoma, Vol. 10, No. 1, pp. 77-78 (1991)). In some methods, the monoclonal antibody is labeled (e.g., with any composition detectable by spectroscopic, photochemical, biochemical, electrical, optical, or chemical means) to facilitate detection.

### Generating siRNA Molecules

siRNA can be provided in several forms including, e.g., as one or more isolated small-interfering RNA (siRNA) duplexes, as longer double-stranded RNA (dsRNA), or as siRNA or dsRNA transcribed from a transcriptional cassette in a DNA plasmid. The siRNA sequences may have overhangs (e.g., 3' or 5' overhangs as described in Elbashir et al., Genes Dev., 15:188 (2001) or Nykänen et al., Cell, 107:309 (2001), or may lack overhangs (i.e., to have blunt ends).

An RNA population can be used to provide long precursor RNAs, or long precursor RNAs that have substantial or complete identity to a selected target sequence can be used to make the siRNA. The RNAs can be isolated from cells or tissue, synthesized, and/or cloned according to methods well known to those of skill in the art. The RNA can be a mixed population (obtained from cells or tissue, transcribed from cDNA, subtracted, selected, etc.), or can represent a single target sequence. RNA can be naturally occurring (e.g., isolated from tissue or cell samples), synthesized in vitro (e.g., using T7 or SP6 polymerase and PCR products or a cloned cDNA), or chemically synthesized.

To form a long dsRNA, for synthetic RNAs, the complement is also transcribed in vitro and hybridized to form a dsRNA. If a naturally occurring RNA population is used, the RNA complements are also provided (e.g., to form dsRNA for digestion by *E. coli* RNAse III or Dicer), e.g., by transcribing cDNAs corresponding to the RNA population, or by using RNA polymerases. The precursor RNAs are then hybridized to form double stranded RNAs for digestion. The dsRNAs can be directly administered to a subject or can be digested in vitro prior to administration.

Methods for isolating RNA, synthesizing RNA, hybridizing nucleic acids, making and screening cDNA libraries, and performing PCR are well known in the art (see, e.g., Gubler and Hoffman, Gene, 25:263-269 (1983); Sambrook et al., supra; Ausubel et al., supra), as are PCR methods (see, U.S. Pat. Nos. 4,683,195 and 4,683,202; PCR Protocols: A Guide to Methods and Applications (Innis et al., eds, 1990)). Expression libraries are also well known to those of skill in the art. Additional basic texts disclosing the general methods of use in this invention include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994).

Preferably, siRNA are chemically synthesized. The oligonucleotides that comprise the siRNA molecules of the invention can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). The synthesis of oligonucleotides makes use of common nucleic acid protecting and coupling groups, such as dimethoxytrityl at the 5'-end and phosphoramidites at the 3'-end. As a non-limiting example, small scale syntheses can be conducted on an Applied Biosystems synthesizer using a 0.2 µmol scale protocol. Alternatively, syntheses at the 0.2 µmol scale can be performed on a 96-well plate synthesizer from Protogene (Palo Alto, Calif.). However, a larger or smaller scale of synthesis is also within the scope of this invention. Suitable reagents for oligonucleotide synthesis, methods for RNA deprotection, and methods for RNA purification are known to those of skill in the art.

siRNA molecules can also be synthesized via a tandem synthesis technique, wherein both strands are synthesized as a single continuous oligonucleotide fragment or strand separated by a cleavable linker that is subsequently cleaved to provide separate fragments or strands that hybridize to form the siRNA duplex. The linker can be a polynucleotide linker or a non-nucleotide linker. The tandem synthesis of siRNA can be readily adapted to both multiwell/multiplate synthesis platforms as well as large scale synthesis platforms employing batch reactors, synthesis columns, and the like. Alternatively, siRNA molecules can be assembled from two distinct oligonucleotides, wherein one oligonucleotide comprises the sense strand and the other comprises the antisense strand of the siRNA. For example, each strand can be synthesized separately and joined together by hybridization or ligation following synthesis and/or deprotection. In certain other instances, siRNA molecules can be synthesized as a single continuous oligonucleotide fragment, where the self-complementary sense and antisense regions hybridize to form an siRNA duplex having hairpin secondary structure.

### Modifying siRNA Sequences

In certain aspects, siRNA molecules comprise a duplex having two strands and at least one modified nucleotide in the double-stranded region, wherein each strand is about 15 to about 60 nucleotides in length. Advantageously, the modified siRNA is less immunostimulatory than a corresponding unmodified siRNA sequence, but retains the capability of silencing the expression of a target sequence. In preferred embodiments, the degree of chemical modifications introduced into the siRNA molecule strikes a balance between reduction or abrogation of the immunostimulatory properties of the siRNA and retention of RNAi activity. As a non-limiting example, an siRNA molecule that targets a gene of interest can be minimally modified (e.g., less than about 30%, 25%, 20%, 15%, 10%, or 5% modified) at selective uridine and/or guanosine nucleotides within the siRNA duplex to eliminate the immune response generated by the siRNA while retaining its capability to silence target gene expression.

Examples of modified nucleotides suitable for use in the invention include, but are not limited to, ribonucleotides having a 2'-O-methyl (2'OMe), 2'-deoxy-2'-fluoro (2'F), 2'-deoxy, 5-C-methyl, 2'-O-(2-methoxyethyl) (MOE), 4'-thio, 2'-amino, or 2'-C-allyl group. Modified nucleotides having a Northern conformation such as those described in, e.g., Saenger, Principles of Nucleic Acid Structure, Springer-Verlag Ed. (1984), are also suitable for use in siRNA molecules. Such modified nucleotides include, without limitation, locked nucleic acid (LNA) nucleotides (e.g., 2'-O, 4'-C-methylene-(D-ribofuranosyl) nucleotides), 2'-O-(2-methoxyethyl) (MOE) nucleotides, 2'-methyl-thio-ethyl nucleotides, 2'-deoxy-2'-fluoro (2'F) nucleotides, 2'-deoxy-2'-chloro (2'Cl) nucleotides, and 2'-azido nucleotides. In certain instances, the siRNA molecules described herein include one or more G-clamp nucleotides. A G-clamp nucleotide refers to a modified cytosine analog wherein the modifications confer the ability to hydrogen bond both Watson-Crick and Hoogsteen faces of a complementary guanine nucleotide within a duplex (see, e.g., Lin et al., J. Am. Chem. Soc., 120:8531-8532 (1998)). In addition, nucleotides having a nucleotide base analog such as, for example, C-phenyl, C-naphthyl, other aromatic derivatives, inosine, azole carboxamides, and nitroazole derivatives such as 3-nitropyrrole, 4-nitroindole, 5-nitroindole, and 6-nitroindole (see, e.g., Loakes, Nucl. Acids Res., 29:2437-2447 (2001)) can be incorporated into siRNA molecules.

In certain embodiments, siRNA molecules may further comprise one or more chemical modifications such as terminal cap moieties, phosphate backbone modifications, and the like. Examples of terminal cap moieties include, without limitation, inverted deoxy abasic residues, glyceryl modifications, 4',5'-methylene nucleotides, 1-(β-D-erythrofuranosyl) nucleotides, 4'-thio nucleotides, carbocyclic nucleotides, 1,5-anhydrohexitol nucleotides, L-nucleotides, α-nucleotides, modified base nucleotides, threo-pentofuranosyl nucleotides, acyclic 3',4'-seco nucleotides, acyclic 3,4-dihydroxybutyl nucleotides, acyclic 3,5-dihydroxypentyl nucleotides, 3'-3'-inverted nucleotide moieties, 3'-3'-inverted abasic moieties, 3'-2'-inverted nucleotide moieties, 3'-2'-inverted abasic moieties, 5'-5'-inverted nucleotide moieties, 5'-5'-inverted abasic moieties, 3'-5'-inverted deoxy abasic moieties, 5'-amino-alkyl phosphate, 1,3-diamino-2-propyl phosphate, 3-aminopropyl phosphate, 6-aminohexyl phosphate, 1,2-aminododecyl phosphate, hydroxypropyl phosphate, 1,4-butanediol phosphate, 3'-phosphoramidate, 5'-phosphoramidate, hexylphosphate, aminohexyl phosphate, 3'-phosphate, 5'-amino, 3'-phosphorothioate, 5'-phosphorothioate, phosphorodithioate, and bridging or non-bridging methylphosphonate or 5'-mercapto moieties (see, e.g., U.S. Pat. No. 5,998,203; Beaucage et al., Tetrahedron 49:1925 (1993)). Non-limiting examples of phosphate backbone modifications (i.e., resulting in modified internucleotide linkages) include phosphorothioate, phosphorodithioate, methylphosphonate, phosphotriester, morpholino, amidate, carbamate, carboxymethyl, acetamidate, polyamide, sulfonate, sulfonamide, sulfamate, formacetal, thioformacetal, and alkylsilyl substitutions (see, e.g., Hunziker et al., Nucleic Acid Analogues: Synthesis and Properties, in Modern Synthetic Methods, VCH, 331-417 (1995); Mesmaeker et al., Novel Backbone Replacements for Oligonucleotides, in Carbohydrate Modifications in Antisense Research, ACS, 24-39 (1994)). Such chemical modifications can occur at the 5'-end and/or 3'-end of the sense strand, antisense strand, or both strands of the siRNA.

In some embodiments, the sense and/or antisense strand of the siRNA molecule can further comprise a 3'-terminal overhang having about 1 to about 4 (e.g., 1, 2, 3, or 4) 2'-deoxy ribonucleotides and/or any combination of modified and unmodified nucleotides. Additional examples of modified nucleotides and types of chemical modifications that can be introduced into siRNA molecules are described, e.g., in UK Patent No. GB 2,397,818 B and U.S. Patent Publication Nos. 20040192626, 20050282188, and 20070135372.

The siRNA molecules described herein can optionally comprise one or more non-nucleotides in one or both strands of the siRNA. As used herein, the term "non-nucleotide" refers to any group or compound that can be incorporated into a nucleic acid chain in the place of one or more nucleotide units, including sugar and/or phosphate substitutions, and allows the remaining bases to exhibit their activity. The group or compound is abasic in that it does not contain a commonly recognized nucleotide base such as adenosine, guanine, cytosine, uracil, or thymine and therefore lacks a base at the l'-position.

In other embodiments, chemical modification of the siRNA comprises attaching a conjugate to the siRNA molecule. The conjugate can be attached at the 5' and/or 3'-end of the sense and/or antisense strand of the siRNA via a covalent attachment such as, e.g., a biodegradable linker. The conjugate can also be attached to the siRNA, e.g., through a carbamate group or other linking group (see, e.g., U.S. Patent Publication Nos. 20050074771, 20050043219, and 20050158727). In certain instances, the conjugate is a molecule that facilitates the delivery of the siRNA into a cell. Examples of conjugate molecules suitable for attachment to siRNA include, without limitation, steroids such as cholesterol, glycols such as polyethylene glycol (PEG), human serum albumin (HSA), fatty acids, carotenoids, terpenes, bile acids, folates (e.g., folic acid, folate analogs and derivatives thereof), sugars (e.g., galactose, galactosamine, N-acetyl galactosamine, glucose, mannose, fructose, fucose, etc.), phospholipids, peptides, ligands for cellular receptors capable of mediating cellular uptake, and combinations thereof (see, e.g., U.S. Patent Publication Nos. 20030130186, 20040110296, and 20040249178; U.S. Pat. No. 6,753,423). Other examples include the lipophilic moiety, vitamin, polymer, peptide, protein, nucleic acid, small molecule, oligosaccharide, carbohydrate cluster, intercalator, minor groove binder, cleaving agent, and cross-linking agent conjugate molecules described in U.S. Patent Publication Nos. 20050119470 and 20050107325. Yet other examples include the 2'-O-alkyl amine, 2'-β-alkoxyalkyl amine, polyamine, C5-cationic modified pyrimidine, cationic peptide, guanidinium group, amidininium group, cationic amino acid conjugate molecules described in U.S. Patent Publication No. 20050153337. Additional examples include the hydrophobic group, membrane active compound, cell penetrating compound, cell targeting signal, interaction modifier, and steric stabilizer conjugate molecules described in U.S. Patent Publication No. 20040167090. Further examples include the conjugate molecules described in U.S. Patent Publication No. 20050239739. The type of conjugate used and the extent of conjugation to the siRNA molecule can be evaluated for improved pharmacokinetic profiles, bioavailability, and/or stability of the siRNA while retaining RNAi activity. As such, one skilled in the art can screen siRNA molecules having various conjugates attached thereto to identify ones having improved properties and full RNAi activity using any of a variety of well-known in vitro cell culture or in vivo animal models.

### Target Genes

In certain embodiments, the nucleic acid component (e.g., siRNA) of the nucleic acid-lipid particles described herein can be used to downregulate or silence the translation (i.e., expression) of a gene of interest. Genes of interest include, but are not limited to, genes associated with viral infection and survival, genes associated with metabolic diseases and disorders (e.g., liver diseases and disorders), genes associated with tumorigenesis and cell transformation (e.g., cancer), angiogenic genes, immunomodulator genes such as those associated with inflammatory and autoimmune responses, ligand receptor genes, and genes associated with neurodegenerative disorders. In certain embodiments, the gene of interest is expressed in hepatocytes.

Genes associated with viral infection and survival include those expressed by a virus in order to bind, enter, and replicate in a cell. Of particular interest are viral sequences associated with chronic viral diseases. Viral sequences of particular interest include sequences of Filoviruses such as Ebola virus and Marburg virus (see, e.g., Geisbert et al., J. Infect. Dis., 193:1650-1657 (2006)); Arenaviruses such as Lassa virus, Junin virus, Machupo virus, Guanarito virus, and Sabia virus (Buchmeier et al., Arenaviridae: the viruses and their replication, In: FIELDS VIROLOGY, Knipe et al. (eds.), 4th ed., Lippincott-Raven, Philadelphia, (2001)); Influenza viruses such as Influenza A, B, and C viruses, (see, e.g., Steinhauer et al., Annu Rev Genet., 36:305-332 (2002); and Neumann et al., J Gen Virol., 83:2635-2662 (2002)); Hepatitis viruses (see, e.g., Hamasaki et al., FEBS Lett., 543:51 (2003); Yokota et al., EMBO Rep., 4:602 (2003); Schlomai et al., Hepatology, 37:764 (2003); Wilson et al., Proc. Natl. Acad. Sci. USA, 100:2783 (2003); Kapadia et al., Proc. Natl. Acad. Sci. USA, 100:2014 (2003); and FIELDS VIROLOGY, Knipe et al. (eds.), 4th ed., Lippincott-Raven, Philadelphia (2001)); Human Immunodeficiency Virus (HIV) (Banerjea et al., Mol. Ther., 8:62 (2003); Song et al., J. Virol., 77:7174 (2003); Stephenson, JAMA, 289:1494 (2003); Qin et al., Proc. Natl. Acad. Sci. USA, 100:183 (2003)); Herpes viruses (Jia et al., J. Virol., 77:3301 (2003)); and Human Papilloma Viruses (HPV) (Hall et al., J. Virol., 77:6066 (2003); Jiang et al., Oncogene, 21:6041 (2002)).

Exemplary Filovirus nucleic acid sequences that can be silenced include, but are not limited to, nucleic acid sequences encoding structural proteins (e.g., VP30, VP35, nucleoprotein (NP), polymerase protein (L-pol)) and membrane-associated proteins (e.g., VP40, glycoprotein (GP), VP24). Complete genome sequences for Ebola virus are set forth in, e.g., Genbank Accession Nos. NC_002549; AY769362; NC_006432; NC_004161; AY729654; AY354458; AY142960; AB050936; AF522874; AF499101; AF272001; and AF086833. Ebola virus VP24 sequences are set forth in, e.g., Genbank Accession Nos. U77385 and AY058897. Ebola virus L-pol sequences are set forth in, e.g., Genbank Accession No. X67110. Ebola virus VP40 sequences are set forth in, e.g., Genbank Accession No. AY058896. Ebola virus NP sequences are set forth in, e.g., Genbank Accession No. AY058895. Ebola virus GP sequences are set forth in, e.g., Genbank Accession No. AY058898; Sanchez et al., Virus Res., 29:215-240 (1993); Will et al., J. Virol., 67:1203-1210 (1993); Volchkov et al., FEBS Lett., 305:181-184 (1992); and U.S. Pat. No. 6,713,069. Additional Ebola virus sequences are set forth in, e.g., Genbank Accession Nos. L11365 and X61274. Complete genome sequences for Marburg virus are set forth in, e.g., Genbank Accession Nos. NC_001608; AY430365; AY430366; and AY358025. Marburg virus GP sequences are set forth in, e.g., Genbank Accession Nos. AF005734; AF005733; and AF005732. Marburg virus VP35 sequences are set forth in, e.g., Genbank Accession Nos. AF005731 and AF005730. Additional Marburg virus sequences are set forth in, e.g., Genbank Accession Nos. X64406; Z29337; AF005735; and Z12132. Non-limiting examples of siRNA molecules targeting Ebola virus and Marburg virus nucleic acid sequences include those described in U.S. Patent Publication No. 20070135370.

Exemplary Influenza virus nucleic acid sequences that can be silenced include, but are not limited to, nucleic acid sequences encoding nucleoprotein (NP), matrix proteins (M1 and M2), nonstructural proteins (NS1 and NS2), RNA polymerase (PA, PB1, PB2), neuraminidase (NA), and haemagglutinin (HA). Influenza A NP sequences are set forth in, e.g., Genbank Accession Nos. NC_004522; AY818138; AB166863; AB188817; AB189046; AB189054; AB189062; AY646169; AY646177; AY651486; AY651493; AY651494; AY651495; AY651496; AY651497; AY651498; AY651499; AY651500; AY651501; AY651502; AY651503; AY651504; AY651505; AY651506; AY651507; AY651509; AY651528; AY770996; AY790308; AY818138; and AY818140. Influenza A PA sequences are set forth in, e.g., Genbank Accession Nos. AY818132; AY790280; AY646171; AY818132; AY818133; AY646179; AY818134; AY551934; AY651613; AY651610; AY651620; AY651617; AY651600; AY651611; AY651606; AY651618; AY651608; AY651607; AY651605; AY651609; AY651615; AY651616; AY651640; AY651614; AY651612; AY651621; AY651619; AY770995; and AY724786. Non-limiting examples of siRNA molecules targeting Influenza virus nucleic acid sequences include those described in U.S. Patent Publication No. 20070218122.

Exemplary hepatitis virus nucleic acid sequences that can be silenced include, but are not limited to, nucleic acid sequences involved in transcription and translation (e.g., En1, En2, X, P) and nucleic acid sequences encoding structural proteins (e.g., core proteins including C and C-related proteins, capsid and envelope proteins including S, M, and/or L proteins, or fragments thereof) (see, e.g., FIELDS VIROLOGY, supra). Exemplary Hepatitis C virus (HCV) nucleic acid sequences that can be silenced include, but are not limited to, the 5'-untranslated region (5'-UTR), the 3'-untranslated region (3'-UTR), the polyprotein translation initiation codon region, the internal ribosome entry site (IRES) sequence, and/or nucleic acid sequences encoding the core protein, the E1 protein, the E2 protein, the p7 protein, the NS2 protein, the NS3 protease/helicase, the NS4A protein, the NS4B protein, the NS5A protein, and/or the NS5B RNA-dependent RNA polymerase. HCV genome sequences are set forth in, e.g., Genbank Accession Nos. NC_004102 (HCV genotype 1a), AJ238799 (HCV genotype 1b), NC_009823 (HCV genotype 2), NC_009824 (HCV genotype 3), NC_009825 (HCV genotype 4), NC_ 009826 (HCV genotype 5), and NC_009827 (HCV genotype 6). Hepatitis A virus nucleic acid sequences are set forth in, e.g., Genbank Accession No. NC_001489; Hepatitis B virus nucleic acid sequences are set forth in, e.g., Genbank Accession No. NC_003977; Hepatitis D virus nucleic acid sequence are set forth in, e.g., Genbank Accession No. NC_001653; Hepatitis E virus nucleic acid sequences are set forth in, e.g., Genbank Accession No. NC_001434; and Hepatitis G virus nucleic acid sequences are set forth in, e.g., Genbank Accession No. NC_ 001710. Silencing of sequences that encode genes associated with viral infection and survival can conveniently be used in combination with the administration of conventional agents used to treat the viral condition. Non-limiting examples of siRNA molecules targeting hepatitis virus nucleic acid sequences include those described in U.S. Patent Publication Nos. 20060281175, 20050058982, and 20070149470; U.S. Pat. No. 7,348,314; and U.S. Provisional Application No. 61/162,127, filed Mar. 20, 2009.

Genes associated with metabolic diseases and disorders (e.g., disorders in which the liver is the target and liver diseases and disorders) include, for example, genes expressed in dyslipidemia (e.g., liver X receptors such as LXRα and LXRβ (Genback Accession No. NM_ 007121), farnesoid X receptors (FXR) (Genbank Accession No. NM_005123), sterol-regulatory element binding protein (SREBP), site-1 protease (SIP), 3-hydroxy-3-methylglutaryl coenzyme-A reductase (HMG coenzyme-A reductase), apolipoprotein B (ApoB) (Genbank Accession No. NM_000384), apolipoprotein CIII (ApoC3) (Genbank Accession Nos. NM_000040 and NG-008949 REGION: 5001.8164), and apolipoprotein E (ApoE) (Genbank Accession Nos. NM_ 000041 and NG_007084 REGION: 5001.8612)); and diabetes (e.g., glucose 6-phosphatase) (see, e.g., Forman et al., Cell, 81:687 (1995); Seol et al., Mol. Endocrinol., 9:72 (1995), Zavacki et al., Proc. Natl. Acad. Sci. USA, 94:7909 (1997); Sakai et al., Cell, 85:1037-1046 (1996); Duncan et al., J. Biol. Chem., 272:12778-12785 (1997); Willy et al., Genes Dev., 9:1033-1045 (1995); Lehmann et al., J. Biol. Chem., 272:3137-3140 (1997); Janowski et al., Nature, 383:728-731 (1996); and Peet et al., Cell, 93:693-704 (1998)). One of skill in the art will appreciate that genes associated with metabolic diseases and disorders (e.g., diseases and disorders in which the liver is a target and liver diseases and disorders) include genes that are expressed in the liver itself as well as and genes expressed in other organs and tissues. Silencing of sequences that encode genes associated with metabolic diseases and disorders can conveniently be used in combination with the administration of conventional agents used to treat the disease or disorder. Non-limiting examples of siRNA molecules targeting the ApoB gene include those described in U.S. Patent Publication No. 20060134189. Non-limiting examples of siRNA molecules targeting the ApoC3 gene include those described in U.S. Provisional Application No. 61/147,235, filed Jan. 26, 2009.

Examples of gene sequences associated with tumorigenesis and cell transformation (e.g., cancer or other neoplasia) include mitotic kinesins such as Eg5 (KSP, KIF11; Genbank Accession No. NM_004523); serine/threonine kinases such as polo-like kinase 1 (PLK-1) (Genbank Accession No. NM_005030; Barr et al., Nat. Rev. Mol. Cell. Biol., 5:429-440 (2004)); tyrosine kinases such as WEE1 (Genbank Accession Nos. NM_003390 and NM_ 001143976); inhibitors of apoptosis such as XIAP (Genbank Accession No. NM_001167); COP9 signalosome subunits such as CSN1, CSN2, CSN3, CSN4, CSN5 (JAB1; Genbank Accession No. NM_006837); CSN6, CSN7A, CSN7B, and CSN8; ubiquitin ligases such as COP1 (RFWD2; Genbank Accession Nos. NM_022457 and NM_001001740); and histone deacetylases such as HDAC1, HDAC2 (Genbank Accession No. NM_001527), HDAC3, HDAC4, HDAC5, HDAC6, HDAC7, HDAC8, HDAC9, etc. Non-limiting examples of siRNA molecules targeting the Eg5 and XIAP genes include those described in U.S. patent application Ser. No. 11/807,872, filed May 29, 2007. Non-limiting examples of siRNA molecules targeting the PLK-1 gene include those described in U.S. Patent Publication Nos. 20050107316 and 20070265438; and U.S. patent application Ser. No. 12/343,342, filed Dec. 23, 2008. Non-limiting examples of siRNA molecules targeting the CSN5 gene include those described in U.S. Provisional Application No. 61/045,251, filed Apr. 15, 2008.

Additional examples of gene sequences associated with tumorigenesis and cell transformation include translocation sequences such as MLL fusion genes, BCR-ABL (Wilda et al., Oncogene, 21:5716 (2002); Scherr et al., Blood, 101:1566 (2003)), TEL-AML1, EWS-FLI1, TLS-FUS, PAX3-FKHR, BCL-2, AML1-ETO, and AML1-MTG8 (Heidenreich et al., Blood, 101:3157 (2003)); overexpressed sequences such as multidrug resistance genes (Nieth et al., FEBS Lett., 545:144 (2003); Wu et al, Cancer Res. 63:1515 (2003)), cyclins (Li et al., Cancer Res., 63:3593 (2003); Zou et al., Genes Dev., 16:2923 (2002)), beta-catenin (Verma et al., Clin Cancer Res., 9:1291 (2003)), telomerase genes (Kosciolek et al., Mol Cancer Ther., 2:209 (2003)), c-MYC, N-MYC, BCL-2, growth factor receptors (e.g., EGFR/ErbB1 (Genbank Accession Nos. NM_005228, NM_201282, NM_201283, and NM_201284; see also, Nagy et al. Exp. Cell Res., 285:39-49 (2003), ErbB2/HER-2 (Genbank Accession Nos. NM_004448 and NM_001005862), ErbB3 (Genbank Accession Nos. NM_001982 and NM_001005915), and ErbB4 (Genbank Accession Nos. NM_005235 and NM_001042599); and mutated sequences such as RAS (reviewed in Tuschl and Borkhardt, Mol. Interventions, 2:158 (2002)). Non-limiting examples of siRNA molecules targeting the EGFR gene include those described in U.S. patent application Ser. No. 11/807,872, filed May 29, 2007.

Silencing of sequences that encode DNA repair enzymes find use in combination with the administration of chemotherapeutic agents (Collis et al., Cancer Res., 63:1550 (2003)). Genes encoding proteins associated with tumor migration are also target sequences of interest, for example, integrins, selectins, and metalloproteinases. The foregoing examples are not exclusive. Those of skill in the art will understand that any whole or partial gene sequence that facilitates or promotes tumorigenesis or cell transformation, tumor growth, or tumor migration can be included as a template sequence.

Angiogenic genes are able to promote the formation of new vessels. Of particular interest is vascular endothelial growth factor (VEGF) (Reich et al., Mol. Vis., 9:210 (2003)) or VEGFR. siRNA sequences that target VEGFR are set forth in, e.g., GB 2396864; U.S. Patent Publication No. 20040142895; and CA 2456444.

Anti-angiogenic genes are able to inhibit neovascularization. These genes are particularly useful for treating those cancers in which angiogenesis plays a role in the pathological development of the disease. Examples of anti-angiogenic genes include, but are not limited to, endostatin (see, e.g., U.S. Pat. No. 6,174,861), angiostatin (see, e.g., U U.S. Pat. No. 5,639,725), and VEGFR2 (see, e.g., Decaussin et al., J. Pathol., 188: 369-377 (1999)). Immunomodulator genes are genes that modulate one or more immune responses. Examples of immunomodulator genes include, without limitation, cytokines such as growth factors (e.g., TGF-α, TGF-β, EGF, FGF, IGF, NGF, PDGF, CGF, GM-CSF, SCF, etc.), interleukins (e.g., IL-2, IL-4, IL-12 (Hill et al., J. Immunol., 171:691 (2003)), IL-15, IL-18, IL-20, etc.), interferons (e.g., IFN-α, IFN-β, IFN-γ, etc.) and TNF. Fas and Fas ligand genes are also immunomodulator target sequences of interest (Song et al., Nat. Med., 9:347 (2003)). Genes encoding secondary signaling molecules in hematopoietic and lymphoid cells are also included in the present invention, for example, Tec family kinases such as Bruton's tyrosine kinase (Btk) (Heinonen et al., FEBS Lett., 527:274 (2002)).

Cell receptor ligands include ligands that are able to bind to cell surface receptors (e.g., insulin receptor, EPO receptor, G-protein coupled receptors, receptors with tyrosine kinase activity, cytokine receptors, growth factor receptors, etc.), to modulate (e.g., inhibit, activate, etc.) the physiological pathway that the receptor is involved in (e.g., glucose level modulation, blood cell development, mitogenesis, etc.). Examples of cell receptor ligands include, but are not limited to, cytokines, growth factors, interleukins, interferons, erythropoietin (EPO), insulin, glucagon, G-protein coupled receptor ligands, etc. Templates coding for an expansion of trinucleotide repeats (e.g., CAG repeats) find use in silencing pathogenic sequences in neurodegenerative disorders caused by the expansion of trinucleotide repeats, such as spinobulbular muscular atrophy and Huntington's Disease (Caplen et al., Hum. Mol. Genet., 11:175 (2002)).

Certain other target genes, which may be targeted by a nucleic acid (e.g., by siRNA) to downregulate or silence the expression of the gene, include but are not limited to, Actin, Alpha 2, Smooth Muscle, Aorta (ACTA2), Alcohol dehydrogenase 1A (ADH1A), Alcohol dehydrogenase 4 (ADH4), Alcohol dehydrogenase 6 (ADH6), Afamin (AFM), Angiotensinogen (AGT), Serine-pyruvate aminotransferase (AGXT), Alpha-2-HS-glycoprotein (AHSG), Aldo-keto reductase family 1 member C4 (AKR1C4), Serum albumin (ALB), alpha-1-microglobulin/bikunin precursor (AMBP), Angiopoietin-related protein 3 (ANGPTL3), Serum amyloid P-component (APCS), Apolipoprotein A-II (APOA2), Apolipoprotein B-100 (APOB), Apolipoprotein C3 (APOC3), Apolipoprotein C-IV (APOC4), Apolipoprotein F (APOF), Beta-2-glycoprotein 1 (APOH), Aquaporin-9 (AQP9), Bile acid-CoA:amino acid N-acyltransferase (BAAT), C4b-binding protein beta chain (C4BPB), Putative uncharacterized protein encoded by LINC01554 (C5orf27), Complement factor 3 (C3), Complement Factor 5 (C5), Complement component C6 (C6), Complement component C8 alpha chain (C8A), Complement component C8 beta chain (C8B), Complement component C8 gamma chain (C8G), Complement component C9 (C9), Calmodulin Binding Transcription Activator 1 (CAMTA1), CD38 (CD38), Complement Factor B (CFB), Complement factor H-related protein 1 (CFHR1), Complement factor H-related protein 2 (CFHR2), Complement factor H-related protein 3 (CFHR3), Cannabinoid receptor 1 (CNR1), ceruloplasmin (CP), carboxypeptidase B2 (CPB2), Connective tissue growth factor (CTGF), C-X-C motif chemokine 2 (CXCL2), Cytochrome P450 1A2 (CYP1A2), Cytochrome P450 2A6 (CYP2A6), Cytochrome P450 2C8 (CYP2C8), Cytochrome P450 2C9 (CYP2C9), Cytochrome P450 Family 2 Subfamily D Member 6 (CYP2D6), Cytochrome P450 2E1 (CYP2E1), Phylloquinone omega-hydroxylase CYP4F2 (CYP4F2), 7-alpha-hydroxycholest-4-en-3-one 12-alpha-hydroxylase (CYP8B1), Dipeptidyl peptidase 4 (DPP4), coagulation factor 12 (F12), coagulation factor II (thrombin) (F2), coagulation factor IX (F9), fibrinogen alpha chain (FGA), fibrinogen beta chain (FGB), fibrinogen gamma chain (FGG), fibrinogen-like 1 (FGL1), flavin containing monooxygenase 3 (FMO3), flavin containing monooxygenase 5 (FMO5), group-specific component (vitamin D binding protein) (GC), Growth hormone receptor (GHR), glycine N-methyltransferase (GNMT), hyaluronan binding protein 2 (HABP2), hepcidin antimicrobial peptide (HAMP), hydroxyacid oxidase (glycolate oxidase) 1 (HAO1), HGF activator (HGFAC), haptoglobin-related protein; haptoglobin (HPR), hemopexin (HPX), histidine-rich glycoprotein (HRG), hydroxysteroid (11-beta) dehydrogenase 1 (HSD11B1), hydroxysteroid (17-beta) dehydrogenase 13 (HSD17B13), Inter-alpha-trypsin inhibitor heavy chain H1 (ITIH1), Inter-alpha-trypsin inhibitor heavy chain H2 (ITIH2), Inter-alpha-trypsin inhibitor heavy chain H3 (ITIH3), Inter-alpha-trypsin inhibitor heavy chain H4 (ITIH4), Prekallikrein (KLKB1), Lactate dehydrogenase A (LDHA), liver expressed antimicrobial peptide 2 (LEAP2), leukocyte cell-derived chemotaxin 2 (LECT2), Lipoprotein (a) (LPA), mannan-binding lectin serine peptidase 2 (MASP2), S-adenosylmethionine synthase isoform type-1 (MAT1A), NADPH Oxidase 4 (NOX4), Poly [ADP-ribose] polymerase 1 (PARP1), paraoxonase 1 (PON1), paraoxonase 3 (PON3), Vitamin K-dependent protein C (PROC), Retinol dehydrogenase 16 (RDH16), serum amyloid A4, constitutive (SAA4), serine dehydratase (SDS), Serpin Family A Member 1 (SERPINA1), Serpin A11 (SERPINA11), Kallistatin (SERPINA4), Corticosteroid-binding globulin (SERPINA6), Antithrombin-III (SERPINC1), Heparin cofactor 2 (SERPIND1), Serpin Family H Member 1 (SERPINH1), Solute Carrier Family 5 Member 2 (SLC5A2), Sodium/bile acid cotransporter (SLC10A1), Solute carrier family 13 member 5 (SLC13A5), Solute carrier family 22 member 1 (SLC22A1), Solute carrier family 25 member 47 (SLC25A47), Solute carrier family 2, facilitated glucose transporter member 2 (SLC2A2), Sodium-coupled neutral amino acid transporter 4 (SLC38A4), Solute carrier organic anion transporter family member 1B1 (SLCO1B1), Sphingomyelin Phosphodiesterase 1 (SMPD1), Bile salt sulfotransferase (SULT2A1), tyrosine aminotransferase (TAT), tryptophan 2,3-dioxygenase (TDO2), UDP glucuronosyltransferase 2 family, polypeptide B10 (UGT2B10), UDP glucuronosyltransferase 2 family, polypeptide B15 (UGT2B15), UDP glucuronosyltransferase 2 family, polypeptide B4 (UGT2B4) and vitronectin (VTN).

In addition to its utility in silencing the expression of any of the above-described genes for therapeutic purposes, certain nucleic acids (e.g., siRNA) described herein are also useful in research and development applications as well as diagnostic, prophylactic, prognostic, clinical, and other healthcare applications. As a non-limiting example, certain nucleic acids (e.g., siRNA) can be used in target validation studies directed at testing whether a gene of interest has the potential to be a therapeutic target. Certain nucleic acids (e.g., siRNA) can also be used in target identification studies aimed at discovering genes as potential therapeutic targets.

### CRISPR

Targeted genome editing has progressed from being a niche technology to a method used by many biological researchers. This progression has been largely fueled by the emergence of the clustered, regularly interspaced, short palindromic repeat (CRISPR) technology (*see, e.g.,* Sander et al., Nature Biotechnology, 32(4), 347-355, including Supplementary Information (2014) and International Publication Numbers WO 2016/197132 and WO 2016/197133). Accordingly, provided herein are improvements (e.g., lipid nanoparticles and formulations thereof) that can be used in combination with CRISPR technology to treat diseases, such as HBV. Regarding the targets for using CRISPR, the guide RNA (gRNA) utilized in the CRISPR technology can be designed to target specifically identified sequences, *e.g.,* target genes, *e.g.,* of the HBV genome. Examples of such target sequences are provided in International Publication Number WO 2016/197132. Further, International Publication Number WO 2013/151665 (*e.g., see* Table 6 and the associated Sequence Listing) describes about 35,000 mRNA sequences, claimed in the context of an mRNA expression construct. Certain embodiments of the present invention utilize CRISPR technology to target the expression of any of these sequences. Certain embodiments of the present invention may also utilize CRISPR technology to target the expression of a target gene discussed herein.

### aiRNA

Like siRNA, asymmetrical interfering RNA (aiRNA) can recruit the RNA-induced silencing complex (RISC) and lead to effective silencing of a variety of genes in mammalian cells by mediating sequence-specific cleavage of the target sequence between nucleotide 10 and 11 relative to the 5' end of the antisense strand (Sun et al., Nat. Biotech., 26:1379-1382 (2008)). Typically, an aiRNA molecule comprises a short RNA duplex having a sense strand and an antisense strand, wherein the duplex contains overhangs at the 3' and 5' ends of the antisense strand. The aiRNA is generally asymmetric because the sense strand is shorter on both ends when compared to the complementary antisense strand. In some aspects, aiRNA molecules may be designed, synthesized, and annealed under conditions similar to those used for siRNA molecules. As a non-limiting example, aiRNA sequences may be selected and generated using the methods described above for selecting siRNA sequences.

In another embodiment, aiRNA duplexes of various lengths (e.g., about 10-25, 12-20, 12-19, 12-18, 13-17, or 14-17 base pairs, more typically 12, 13, 14, 15, 16, 17, 18, 19, or base pairs) may be designed with overhangs at the 3' and 5' ends of the antisense strand to target an mRNA of interest. In certain instances, the sense strand of the aiRNA molecule is about 10-25, 12-20, 12-19, 12-18, 13-17, or 14-17 nucleotides in length, more typically 12, 13, 14, 15, 16, 17, 18, 19, or 20 nucleotides in length. In certain other instances, the antisense strand of the aiRNA molecule is about 15-60, 15-50, or 15-40 nucleotides in length, more typically about 15-30, 15-25, or 19-25 nucleotides in length, and is preferably about 20-24, 21-22, or 21-23 nucleotides in length.

In some embodiments, the 5' antisense overhang contains one, two, three, four, or more nontargeting nucleotides (e.g., "AA", "UU", "dTdT", etc.). In other embodiments, the 3' antisense overhang contains one, two, three, four, or more nontargeting nucleotides (e.g., "AA", "UU", "dTdT", etc.). In certain aspects, the aiRNA molecules described herein may comprise one or more modified nucleotides, e.g., in the double-stranded (duplex) region and/or in the antisense overhangs. As a non-limiting example, aiRNA sequences may comprise one or more of the modified nucleotides described above for siRNA sequences. In a preferred embodiment, the aiRNA molecule comprises 2'OMe nucleotides such as, for example, 2'OMe-guanosine nucleotides, 2'OMe-uridine nucleotides, or mixtures thereof.

In certain embodiments, aiRNA molecules may comprise an antisense strand which corresponds to the antisense strand of an siRNA molecule, e.g., one of the siRNA molecules described herein. In other embodiments, aiRNA molecules may be used to silence the expression of any of the target genes set forth above, such as, e.g., genes associated with viral infection and survival, genes associated with metabolic diseases and disorders, genes associated with tumorigenesis and cell transformation, angiogenic genes, immunomodulator genes such as those associated with inflammatory and autoimmune responses, ligand receptor genes, and genes associated with neurodegenerative disorders.

### miRNA

Generally, microRNAs (miRNA) are single-stranded RNA molecules of about 21-23 nucleotides in length which regulate gene expression. miRNAs are encoded by genes from whose DNA they are transcribed, but miRNAs are not translated into protein (non-coding RNA); instead, each primary transcript (a pri-miRNA) is processed into a short stem-loop structure called a pre-miRNA and finally into a functional mature miRNA. Mature miRNA molecules are either partially or completely complementary to one or more messenger RNA (mRNA) molecules, and their main function is to downregulate gene expression. The identification of miRNA molecules is described, e.g., in Lagos-Quintana et al., Science, 294:853-858; Lau et al., Science, 294:858-862; and Lee et al., Science, 294:862-864.

The genes encoding miRNA are much longer than the processed mature miRNA molecule. miRNA are first transcribed as primary transcripts or pri-miRNA with a cap and poly-A tail and processed to short, ~70-nucleotide stem-loop structures known as pre-miRNA in the cell nucleus. This processing is performed in animals by a protein complex known as the Microprocessor complex, consisting of the nuclease Drosha and the double-stranded RNA binding protein Pasha (Denli et al., Nature, 432:231-235 (2004)). These pre-miRNA are then processed to mature miRNA in the cytoplasm by interaction with the endonuclease Dicer, which also initiates the formation of the RNA-induced silencing complex (RISC) (Bernstein et al., Nature, 409:363-366 (2001). Either the sense strand or antisense strand of DNA can function as templates to give rise to miRNA.

When Dicer cleaves the pre-miRNA stem-loop, two complementary short RNA molecules are formed, but only one is integrated into the RISC complex. This strand is known as the guide strand and is selected by the argonaute protein, the catalytically active RNase in the RISC complex, on the basis of the stability of the 5' end (Preall et al., Curr. Biol., 16:530-535 (2006)). The remaining strand, known as the anti-guide or passenger strand, is degraded as a RISC complex substrate (Gregory et al., Cell, 123:631-640 (2005)). After integration into the active RISC complex, miRNAs base pair with their complementary mRNA molecules and induce target mRNA degradation and/or translational silencing.

Mammalian miRNA molecules are usually complementary to a site in the 3' UTR of the target mRNA sequence. In certain instances, the annealing of the miRNA to the target mRNA inhibits protein translation by blocking the protein translation machinery. In certain other instances, the annealing of the miRNA to the target mRNA facilitates the cleavage and degradation of the target mRNA through a process similar to RNA interference (RNAi). miRNA may also target methylation of genomic sites which correspond to targeted mRNA. Generally, miRNA function in association with a complement of proteins collectively termed the miRNP.

In certain aspects, the miRNA molecules described herein are about 15-100, 15-90, 15-80, 15-75, 15-70, 15-60, 15-50, or 15-40 nucleotides in length, more typically about 15-30, 15-25, or 19-25 nucleotides in length, and are preferably about 20-24, 21-22, or 21-23 nucleotides in length. In certain other aspects, miRNA molecules may comprise one or more modified nucleotides. As a non-limiting example, miRNA sequences may comprise one or more of the modified nucleotides described above for siRNA sequences. In a preferred embodiment, the miRNA molecule comprises 2'OMe nucleotides such as, for example, 2'OMe-guanosine nucleotides, 2'OMe-uridine nucleotides, or mixtures thereof.

In some embodiments, miRNA molecules may be used to silence the expression of any of the target genes set forth above, such as, e.g., genes associated with viral infection and survival, genes associated with metabolic diseases and disorders, genes associated with tumorigenesis and cell transformation, angiogenic genes, immunomodulator genes such as those associated with inflammatory and autoimmune responses, ligand receptor genes, and genes associated with neurodegenerative disorders.

In other embodiments, one or more agents that block the activity of a miRNA targeting an mRNA of interest are administered using a lipid particle of the invention (e.g., a nucleic acid-lipid particle). Examples of blocking agents include, but are not limited to, steric blocking oligonucleotides, locked nucleic acid oligonucleotides, and Morpholino oligonucleotides. Such blocking agents may bind directly to the miRNA or to the miRNA binding site on the target mRNA.

### Antisense Oligonucleotides

In one embodiment, the nucleic acid is an antisense oligonucleotide directed to a target gene or sequence of interest. The terms "antisense oligonucleotide" or "antisense" include oligonucleotides that are complementary to a targeted polynucleotide sequence. Antisense oligonucleotides are single strands of DNA or RNA that are complementary to a chosen sequence. Antisense RNA oligonucleotides prevent the translation of complementary RNA strands by binding to the RNA. Antisense DNA oligonucleotides can be used to target a specific, complementary (coding or non-coding) RNA. If binding occurs, this DNA/RNA hybrid can be degraded by the enzyme RNase H. In a particular embodiment, antisense oligonucleotides comprise from about 10 to about 60 nucleotides, more preferably from about 15 to about 30 nucleotides. The term also encompasses antisense oligonucleotides that may not be exactly complementary to the desired target gene. Thus, the invention can be utilized in instances where non-target specific-activities are found with antisense, or where an antisense sequence containing one or more mismatches with the target sequence is the most preferred for a particular use.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (see, U.S. Pat. Nos. 5,739,119 and 5,759,829). Furthermore, examples of antisense inhibition have been demonstrated with the nuclear protein cyclin, the multiple drug resistance gene (MDR1), ICAM-1, E-selectin, STK-1, striatal GABAA receptor, and human EGF (see, Jaskulski et al., Science, 240:1544-6 (1988); Vasanthakumar et al., Cancer Commun., 1:225-32 (1989); Penis et al., Brain Res Mol Brain Res., 15; 57:310-20 (1998); and U.S. Pat. Nos. 5,801,154; 5,789,573; 5,718,709 and 5,610,288). Moreover, antisense constructs have also been described that inhibit and can be used to treat a variety of abnormal cellular proliferations, e.g., cancer (see, U.S. Pat. Nos. 5,747,470; 5,591,317; and 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, Tₘ, binding energy, and relative stability. Antisense oligonucleotides may be selected based upon their relative inability to form dimers, hairpins, or other secondary structures that would reduce or prohibit specific binding to the target mRNA in a host cell. Highly preferred target regions of the mRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al., Nucleic Acids Res., 25:3389-402 (1997)).

### Ribozymes

According to another embodiment of the invention, nucleic acid-lipid particles are associated with ribozymes. Ribozymes are RNA-protein complexes having specific catalytic domains that possess endonuclease activity (see, Kim et al., Proc. Natl. Acad. Sci. USA., 84:8788-92 (1987); and Forster et al., Cell, 49:211-20 (1987)). For example, a large number of ribozymes accelerate phosphoester transfer reactions with a high degree of specificity, often cleaving only one of several phosphoesters in an oligonucleotide substrate (see, Cech et al., Cell, 27:487-96 (1981); Michel et al., J. Mol. Biol., 216:585-610 (1990); Reinhold-Hurek et al., Nature, 357:173-6 (1992)). This specificity has been attributed to the requirement that the substrate bind via specific base-pairing interactions to the internal guide sequence ("IGS") of the ribozyme prior to chemical reaction.

At least six basic varieties of naturally-occurring enzymatic RNA molecules are known presently. Each can catalyze the hydrolysis of RNA phosphodiester bonds in trans (and thus can cleave other RNA molecules) under physiological conditions. In general, enzymatic nucleic acids act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, hepatitis δ virus, group I intron or RNaseP RNA (in association with an RNA guide sequence), or *Neurospora* VS RNA motif, for example. Specific examples of hammerhead motifs are described in, e.g., Rossi et al., Nucleic Acids Res., 20:4559-65 (1992). Examples of hairpin motifs are described in, e.g., EP 0360257, Hampel et al., Biochemistry, 28:4929-33 (1989); Hampel et al., Nucleic Acids Res., 18:299-304 (1990); and U.S. Pat. No. 5,631,359. An example of the hepatitis δ virus motif is described in, e.g., Perrotta et al., Biochemistry, 31:11843-52 (1992). An example of the RNaseP motif is described in, e.g., Guerrier-Takada et al., Cell, 35:849-57 (1983). Examples of the *Neurospora* VS RNA ribozyme motif is described in, e.g., Saville et al., Cell, 61:685-96 (1990); Saville et al., Proc. Natl. Acad. Sci. USA, 88:8826-30 (1991); Collins et al., Biochemistry, 32:2795-9 (1993). An example of the Group I intron is described in, e.g., U.S. Pat. No. 4,987,071. Important characteristics of enzymatic nucleic acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus, the ribozyme constructs need not be limited to specific motifs mentioned herein.

Methods of producing a ribozyme targeted to any polynucleotide sequence are known in the art. Ribozymes may be designed as described in, e.g., PCT Publication Nos. WO 93/23569 and WO 94/02595, and synthesized to be tested in vitro and/or in vivo as described therein.

Ribozyme activity can be optimized by altering the length of the ribozyme binding arms or chemically synthesizing ribozymes with modifications that prevent their degradation by serum ribonucleases (see, e.g., PCT Publication Nos. WO 92/07065, WO 93/15187, WO 91/03162, and WO 94/13688; EP 92110298.4; and U.S. Pat. No. 5,334,711, which describe various chemical modifications that can be made to the sugar moieties of enzymatic RNA molecules), modifications which enhance their efficacy in cells, and removal of stem II bases to shorten RNA synthesis times and reduce chemical requirements.

### Immunostimulatory Oligonucleotides

Nucleic acids associated with lipid particles of the present invention may be immunostimulatory, including immunostimulatory oligonucleotides (ISS; single- or double-stranded) capable of inducing an immune response when administered to a subject, which may be a mammal such as a human. ISS include, e.g., certain palindromes leading to hairpin secondary structures (see, Yamamoto et al., J. Immunol., 148:4072-6 (1992)), or CpG motifs, as well as other known ISS features (such as multi-G domains; see; PCT Publication No. WO 96/11266).

Immunostimulatory nucleic acids are considered to be non-sequence specific when it is not required that they specifically bind to and reduce the expression of a target sequence in order to provoke an immune response. Thus, certain immunostimulatory nucleic acids may comprise a sequence corresponding to a region of a naturally-occurring gene or mRNA, but they may still be considered non-sequence specific immunostimulatory nucleic acids.

In one embodiment, the immunostimulatory nucleic acid or oligonucleotide comprises at least one CpG dinucleotide. The oligonucleotide or CpG dinucleotide may be unmethylated or methylated. In another embodiment, the immunostimulatory nucleic acid comprises at least one CpG dinucleotide having a methylated cytosine. In one embodiment, the nucleic acid comprises a single CpG dinucleotide, wherein the cytosine in the CpG dinucleotide is methylated. In an alternative embodiment, the nucleic acid comprises at least two CpG dinucleotides, wherein at least one cytosine in the CpG dinucleotides is methylated. In a further embodiment, each cytosine in the CpG dinucleotides present in the sequence is methylated. In another embodiment, the nucleic acid comprises a plurality of CpG dinucleotides, wherein at least one of the CpG dinucleotides comprises a methylated cytosine. Examples of immunostimulatory oligonucleotides suitable for use in the compositions and methods of the present invention are described in PCT Application No. PCT/US08/88676, filed Dec. 31, 2008, PCT Publication Nos. WO 02/069369 and WO 01/15726, U.S. Pat. No. 6,406,705, and Raney et al., J. Pharm. Exper. Ther., 298:1185-92 (2001). In certain embodiments, the oligonucleotides used in the compositions and methods of the invention have a phosphodiester ("PO") backbone or a phosphorothioate ("PS") backbone, and/or at least one methylated cytosine residue in a CpG motif.

### mRNA

In certain embodiments, the nucleic acid is one or more mRNA molecules (e.g, a cocktail of mRNA molecules).

### Modifications to mRNA

mRNA used in the practice of the present invention can include one, two, or more than two nucleoside modifications. In some embodiments, the modified mRNA exhibits reduced degradation in a cell into which the mRNA is introduced, relative to a corresponding unmodified mRNA.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1 -carboxymethyl-pseudouridine, 5-propynyl-uridine, 1 -propynyl-pseudouridine, 5-taurinomethyluridine, 1-taurinomethyl-pseudouridine, 5-taurinomethyl-2-thio-uridine, l -taurinomethyl-4-thio-uridine, 5-methyluridine, 1 -methy 1-pseudouridine, 4-thio- 1 -methy 1-pseudouridine, 2-thio- 1 -methy 1-pseudouridine, 1 -methy 1- 1 -deaza-pseudouridine, 2-thio- 1 -methyl- 1 -deaza-pseudouridine, dihy drouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1 -methyl-pseudoisocytidine, pyrrolo-cytidine, pyrrolopseudoisocytidine, 2-thio-cytidine, 2-thio-5-methyl-cytidine, 4-thio-pseudoisocytidine, 4-thio- 1 -methyl-pseudoisocytidine, 4-thio- 1 -methyl- 1 -deaza-pseudoisocytidine, 1 -methyl- 1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxypseudoisocytidine, and 4-methoxy- 1 -methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1 -methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In specific embodiments, a modified nucleoside is 5'-0-(l -Thiophosphate)- Adenosine, 5 '-0-( 1 -Thiophosphate)-Cy tidine, 5 ' -0-( 1 -Thiophosphate)-Guanosine, 5 '-0-( 1 - Thiophosphate)-Uridine or 5'-0-(l -Thiophosphate)-Pseudouridine. The α-thio substituted phosphate moiety is provided to confer stability to RNA polymers through the unnatural phosphorothioate backbone linkages. Phosphorothioate RNA have increased nuclease resistance and subsequently a longer half-life in a cellular environment. Phosphorothioate linked nucleic acids are expected to also reduce the innate immune response through weaker binding/activation of cellular innate immune molecules.

In certain embodiments it is desirable to intracellularly degrade a modified nucleic acid introduced into the cell, for example if precise timing of protein production is desired. Thus, the invention provides a modified nucleic acid containing a degradation domain, which is capable of being acted on in a directed manner within a cell.

In other embodiments, modified nucleosides include inosine, 1 -methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1 -methylguanosine, N2-methylguanosine, N2,N2-dimethylguanosine, 8-oxo-guanosine, 7-methyl-8-oxo-guanosine, 1 -methyl-6-thio-guanosine, N2-methyl-6-thio-guanosine, and N2,N2-dimethyl-6-thio-guanosine.

### Optional Components of the Modified Nucleic Acids

In further embodiments, the modified nucleic acids may include other optional components, which can be beneficial in some embodiments. These optional components include, but are not limited to, untranslated regions, kozak sequences, intronic nucleotide sequences, internal ribosome entry site (IRES), caps and polyA tails. For example, a 5' untranslated region (UTR) and/or a 3 ' UTR may be provided, wherein either or both may independently contain one or more different nucleoside modifications. In such embodiments, nucleoside modifications may also be present in the translatable region. Also provided are nucleic acids containing a Kozak sequence.

Additionally, provided are nucleic acids containing one or more intronic nucleotide sequences capable of being excised from the nucleic acid.

### Untranslated Regions (UTRs)

Untranslated regions (UTRs) of a gene are transcribed but not translated. The 5'UTR starts at the transcription start site and continues to the start codon but does not include the start codon; whereas, the 3'UTR starts immediately following the stop codon and continues until the transcriptional termination signal. There is growing body of evidence about the regulatory roles played by the UTRs in terms of stability of the nucleic acid molecule and translation. The regulatory features of a UTR can be incorporated into the mRNA used in the present invention to increase the stability of the molecule. The specific features can also be incorporated to ensure controlled down-regulation of the transcript in case they are misdirected to undesired organs sites.

### 5' Capping

The 5' cap structure of an mRNA is involved in nuclear export, increasing mRNA stability and binds the mRNA Cap Binding Protein (CBP), which is responsible for mRNA stability in the cell and translation competency through the association of CBP with poly(A) binding protein to form the mature cyclic mRNA species. The cap further assists the removal of 5' proximal introns removal during mRNA splicing.

Endogenous mRNA molecules may be 5'-end capped generating a 5'-ppp-5'-triphosphate linkage between a terminal guanosine cap residue and the 5'-terminal transcribed sense nucleotide of the mRNA molecule. This 5'-guanylate cap may then be methylated to generate an N7-methyl-guanylate residue. The ribose sugars of the terminal and/or anteterminal transcribed nucleotides of the 5' end of the mRNA may optionally also be 2'-0-methylated. 5'-decapping through hydrolysis and cleavage of the guanylate cap structure may target a nucleic acid molecule, such as an mRNA molecule, for degradation.

### IRES Sequences

mRNA containing an internal ribosome entry site (IRES) are also useful in the practice of the present invention. An IRES may act as the sole ribosome binding site, or may serve as one of multiple ribosome binding sites of an mRNA. An mRNA containing more than one functional ribosome binding site may encode several peptides or polypeptides that are translated independently by the ribosomes ("multicistronic mRNA"). When mRNA are provided with an IRES, further optionally provided is a second translatable region. Examples of IRES sequences that can be used according to the invention include without limitation, those from picornaviruses (e.g. FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (S1V) or cricket paralysis viruses (CrPV).

### Poly-A tails

During RNA processing, a long chain of adenine nucleotides (poly-A tail) may be added to a polynucleotide such as an mRNA molecule in order to increase stability. Immediately after transcription, the 3' end of the transcript may be cleaved to free a 3' hydroxyl. Then poly-A polymerase adds a chain of adenine nucleotides to the RNA. The process, called polyadenylation, adds a poly-A tail that can be between 100 and 250 residues long.

Generally, the length of a poly-A tail is greater than 30 nucleotides in length. In another embodiment, the poly-A tail is greater than 35 nucleotides in length (e.g., at least or greater than about 35, 40, 45, 50, 55, 60, 70, 80, 90, 100, 120, 140, 160, 180, 200, 250, 300, 350, 400, 450, 500, 600, 700, 800, 900, 1000, 1100, 1200, 1300, 1400, 1500, 1600, 1700, 1800, 1900, 2,000, 2,500, and 3,000 nucleotides).

In this context the poly-A tail may be 10, 20, 30, 40, 50, 60, 70, 80, 90, or 100% greater in length than the modified mRNA. The poly-A tail may also be designed as a fraction of modified nucleic acids to which it belongs. In this context, the poly-A tail may be 10, 20, 30, 40, 50, 60, 70, 80, or 90% or more of the total length of the modified mRNA or the total length of the modified mRNA minus the poly-A tail.

### Generating mRNA Molecules

Methods for isolating RNA, synthesizing RNA, hybridizing nucleic acids, making and screening cDNA libraries, and performing PCR are well known in the art *(see, e.g.,* Gubler and Hoffman, Gene, 25:263-269 (1983); Sambrook et al., Molecular Cloning, A Laboratory Manual (2nd ed. 1989)); as are PCR methods *(see,* U.S. Patent Nos. 4,683,195 and 4,683,202; PCR Protocols: A Guide to Methods and Applications (Innis et al., eds, 1990)). Expression libraries are also well known to those of skill in the art. Additional basic texts disclosing the general methods of use in this invention include Kriegler, Gene Transfer and Expression: A Laboratory Manual (1990); and Current Protocols in Molecular Biology (Ausubel et al., eds., 1994).

### Encoded Polypeptides

The mRNA component of a nucleic acid-lipid particle described herein can be used to express a polypeptide of interest. Certain diseases in humans are caused by the absence or impairment of a functional protein in a cell type where the protein is normally present and active. The functional protein can be completely or partially absent due, *e.g.,* to transcriptional inactivity of the encoding gene or due to the presence of a mutation in the encoding gene that renders the protein completely or partially non-functional. Examples of human diseases that are caused by complete or partial inactivation of a protein include X-linked severe combined immunodeficiency (X-SCID) and adrenoleukodystrophy (X-ALD). X-SCID is caused by one or more mutations in the gene encoding the common gamma chain protein that is a component of the receptors for several interleukins that are involved in the development and maturation of B and T cells within the immune system. X-ALD is caused by one or more mutations in a peroxisomal membrane transporter protein gene called ABCD1. Individuals afflicted with X-ALD have very high levels of long chain fatty acids in tissues throughout the body, which causes a variety of symptoms that may lead to mental impairment or death.

Attempts have been made to use gene therapy to treat some diseases caused by the absence or impairment of a functional protein in a cell type where the protein is normally present and active. Gene therapy typically involves introduction of a vector that includes a gene encoding a functional form of the affected protein, into a diseased person, and expression of the functional protein to treat the disease. Thus far, gene therapy has met with limited success. Additionally, certain aspects of delivering mRNA using LNPs have been described, e.g., in International Publication Numbers WO 2018/006052 and WO 2015/011633.

As such, there is a continuing need for improvement for expressing a functional form of a protein within a human who suffers from a disease caused by the complete or partial absence of the functional protein, and there is a need for improved delivery of nucleic acids (e.g., mRNA) via a methods and compositions, e.g., that can trigger less of an immune response to the therapy. Certain embodiments of the present invention are useful in this context. Thus, in certain embodiments, expression of the polypeptide ameliorates one or more symptoms of a disease or disorder. Certain compositions and methods of the invention may be useful for treating human diseases caused by the absence, or reduced levels, of a functional polypeptide within the human body. In other embodiments, certain compositions and methods of the invention may be useful for expressing a vaccine antigen for treating cancer.

### Self-Amplifying RNA

In certain embodiments, the nucleic acid is one or more self-amplifying RNA molecules. Self-amplifying RNA (sa-RNA) may also be referred to as self-replicating RNA, replication-competent RNA, replicons or RepRNA. RepRNA, referred to as self-amplifying mRNA when derived from positive-strand viruses, is generated from a viral genome lacking at least one structural gene; it can translate and replicate (hence "self-amplifying") without generating infectious progeny virus. In certain embodiments, the RepRNA technology may be used to insert a gene cassette encoding a desired antigen of interest. For example, the alphaviral genome is divided into two open reading frames (ORFs): the first ORF encodes proteins for the RNA dependent RNA polymerase (replicase), and the second ORF encodes structural proteins. In sa-RNA vaccine constructs, the ORF encoding viral structural proteins may be replaced with any antigen of choice, while the viral replicase remains an integral part of the vaccine and drives intracellular amplification of the RNA after immunization.

### Other Active Agents

In certain embodiments, the active agent associated with the lipid particles of the invention may comprise one or more therapeutic proteins, polypeptides, or small organic molecules or compounds. Non-limiting examples of such therapeutically effective agents or drugs include oncology drugs (e.g., chemotherapy drugs, hormonal therapeutic agents, immunotherapeutic agents, radiotherapeutic agents, etc.), lipid-lowering agents, anti-viral drugs, anti-inflammatory compounds, antidepressants, stimulants, analgesics, antibiotics, birth control medication, antipyretics, vasodilators, anti-angiogenics, cytovascular agents, signal transduction inhibitors, cardiovascular drugs such as anti-arrhythmic agents, hormones, vasoconstrictors, and steroids. These active agents may be administered alone in the lipid particles of the invention, or in combination (e.g., co-administered) with lipid particles of the invention comprising nucleic acid, such as interfering RNA or mRNA.

Non-limiting examples of chemotherapy drugs include platinum-based drugs (e.g., oxaliplatin, cisplatin, carboplatin, spiroplatin, iproplatin, satraplatin, etc.), alkylating agents (e.g., cyclophosphamide, ifosfamide, chlorambucil, busulfan, melphalan, mechlorethamine, uramustine, thiotepa, nitrosoureas, etc.), anti-metabolites (e.g., 5-fluorouracil (5-FU), azathioprine, methotrexate, leucovorin, capecitabine, cytarabine, floxuridine, fludarabine, gemcitabine, pemetrexed, raltitrexed, etc.), plant alkaloids (e.g., vincristine, vinblastine, vinorelbine, vindesine, podophyllotoxin, paclitaxel (taxol), docetaxel, etc.), topoisomerase inhibitors (e.g., irinotecan (CPT-11; Camptosar), topotecan, amsacrine, etoposide (VP16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), tyrosine kinase inhibitors (e.g., gefltinib (Iressa^{®}), sunitinib (Sutent^{®}; SU11248), erlotinib (Tarceva^{®}; OSI-1774), lapatinib (GW572016; GW2016), canertinib (CI 1033), semaxinib (SU5416), vatalanib (PTK787/ZK222584), sorafenib (BAY 43-9006), imatinib (Gleevec^{®}; STI571), dasatinib (BMS-354825), leflunomide (SU101), vandetanib (Zactima^{™}; ZD6474), etc.), pharmaceutically acceptable salts thereof, stereoisomers thereof, derivatives thereof, analogs thereof, and combinations thereof.

Examples of conventional hormonal therapeutic agents include, without limitation, steroids (e.g., dexamethasone), finasteride, aromatase inhibitors, tamoxifen, and goserelin as well as other gonadotropin-releasing hormone agonists (GnRH).

Examples of conventional immunotherapeutic agents include, but are not limited to, immunostimulants (e.g., *Bacillus* Calmette-Guérin (BCG), levamisole, interleukin-2, alpha-interferon, etc.), monoclonal antibodies (e.g., anti-CD20, anti-HER2, anti-CD52, anti-HLA-DR, and anti-VEGF monoclonal antibodies), immunotoxins (e.g., anti-CD33 monoclonal antibody-calicheamicin conjugate, anti-CD22 monoclonal antibody-pseudomonas exotoxin conjugate, etc.), and radioimmunotherapy (e.g., anti-CD20 monoclonal antibody conjugated to ¹¹¹In, ⁹⁰Y, or ¹³¹I, etc.).

Examples of conventional radiotherapeutic agents include, but are not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁹Rh, ¹¹¹Ag, ¹¹¹In, ^{117m} Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

Additional oncology drugs that may be used according to the invention include, but are not limited to, alkeran, allopurinol, altretamine, amifostine, anastrozole, araC, arsenic trioxide, bexarotene, biCNU, carmustine, CCNU, celecoxib, cladribine, cyclosporin A, cytosine arabinoside, cytoxan, dexrazoxane, DTIC, estramustine, exemestane, FK506, gemtuzumab-ozogamicin, hydrea, hydroxyurea, idarubicin, interferon, letrozole, leustatin, leuprolide, litretinoin, megastrol, L-PAM, mesna, methoxsalen, mithramycin, nitrogen mustard, pamidronate, Pegademase, pentostatin, porfimer sodium, prednisone, rituxan, streptozocin, STI-571, taxotere, temozolamide, VM-26, toremifene, tretinoin, ATRA, valrubicin, and velban. Other examples of oncology drugs that may be used according to the invention are ellipticin and ellipticin analogs or derivatives, epothilones, intracellular kinase inhibitors, and camptothecins.

Non-limiting examples of lipid-lowering agents for treating a lipid disease or disorder associated with elevated triglycerides, cholesterol, and/or glucose include statins, fibrates, ezetimibe, thiazolidinediones, niacin, beta-blockers, nitroglycerin, calcium antagonists, fish oil, and mixtures thereof.

Examples of anti-viral drugs include, but are not limited to, abacavir, aciclovir, acyclovir, adefovir, amantadine, amprenavir, arbidol, atazanavir, atripla, cidofovir, combivir, darunavir, delavirdine, didanosine, docosanol, edoxudine, efavirenz, emtricitabine, enfuvirtide, entecavir, entry inhibitors, famciclovir, fixed dose combinations, fomivirsen, fosamprenavir, foscarnet, fosfonet, fusion inhibitors, ganciclovir, ibacitabine, immunovir, idoxuridine, imiquimod, indinavir, inosine, integrase inhibitors, interferon type III (e.g., IFN-λ molecules such as IFN-λ1, IFN-λ2, and IFN-λ3), interferon type II (e.g., IFN-γ), interferon type I (e.g., IFN-α such as PEGylated IFN-α, IFN-β, IFN-κ, IFN-δ, IFN-ε, IFN-τ, IFN-ω, and IFN-ζ), interferon, lamivudine, lopinavir, loviride, MK-0518, maraviroc, moroxydine, nelfinavir, nevirapine, nexavir, nucleoside analogues, oseltamivir, penciclovir, peramivir, pleconaril, podophyllotoxin, protease inhibitors, reverse transcriptase inhibitors, ribavirin, rimantadine, ritonavir, saquinavir, stavudine, synergistic enhancers, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, zidovudine, pharmaceutically acceptable salts thereof, stereoisomers thereof, derivatives thereof, analogs thereof, and mixtures thereof.

### Lipid Particles

The lipid particles of the invention typically comprise an active agent or therapeutic agent, an ionizable lipid of the invention, a non-cationic lipid, and a conjugated lipid that inhibits aggregation of particles. In some embodiments, the active agent or therapeutic agent is fully encapsulated within the lipid portion of the lipid particle such that the active agent or therapeutic agent in the lipid particle is resistant in aqueous solution to enzymatic degradation, e.g., by a nuclease or protease. In other embodiments, the lipid particles described herein are substantially non-toxic to mammals such as humans. The lipid particles of the invention typically have a mean diameter of from about 40 nm to about 150 nm, from about 40 nm to about 80 nm, from about 40 nm to about 60 nm, , from about 50 nm to about 80 nm, from about 50 nm to about 60 nm, from about 50 nm to about 150 nm, from about 60 nm to about 130 nm, from about 70 nm to about 110 nm, or from about 70 to about 90 nm.

In preferred embodiments, the lipid particles of the invention are serum-stable nucleic acid-lipid particles (LNP) which comprise one or more nucleic acid molecules, such as an interfering RNA (e.g., siRNA, aiRNA, and/or miRNA) or mRNA; an ionizable lipid of the invention (e.g., a cationic lipid of Formula I); a non-cationic lipid (e.g., cholesterol alone or mixtures of one or more phospholipids and cholesterol); and a conjugated lipid that inhibits aggregation of the particles (e.g., one or more PEG-lipid conjugates). The LNP may comprise at least 1, 2, 3, 4, 5, 6, 7, 8, 9, 10, or more unmodified and/or modified nucleic acid molecules. Nucleic acid-lipid particles and their method of preparation are described in, e.g., U.S. Pat. Nos. 5,753,613; 5,785,992; 5,705,385; 5,976,567; 5,981,501; 6,110,745; and 6,320,017; and PCT Publication No. WO 96/40964.

### Ionizable Lipids

In one embodiment, the invention provides an ionizable lipid consisting of: an ionizable group of formula -NR²R³, wherein R² is C₁-C₄alkyl and R³ is C₁-C₄alkyl; 15-40 carbon atoms; 30-80 hydrogen atoms; 0-10 oxygen atoms; 0-10 nitrogen atoms; 0 - 4 fluorine; and 0-5 sulfur atoms, wherein the ionizable lipid has a clearance rate such that <10% of the parent ionizable lipid is remaining at seven (7) days post-administration (e.g., in the assay described in Example 32 herein).

In one embodiment, the ionizable lipid comprises at least one group of formula -C(=O)-O-.

In one embodiment, the ionizable lipid comprises at least two groups of formula -C(=O)-O-.

In one embodiment, the ionizable lipid comprises at least one group of formula -(C=O)N(H)N(R⁴)(R⁵)-, wherein R⁴ is C₂-C₃₀hydrocarbyl; and R⁵ is C₂-C₃₀hydrocarbyl.

In one embodiment, the ionizable lipid comprises at least one group of formula -(C=O)N(H)-N=C(R⁴)-, wherein R⁴ is C₂-C₃₀hydrocarbyl; and R⁵ is C₂-C₃₀hydrocarbyl.

In one embodiment, the ionizable lipid comprises at least one group of formula -(C=O)N(H)N(H)(R⁴)(R⁵)-, wherein R⁴ is C₂-C₃₀hydrocarbyl; and R⁵ is C₂-C₃₀hydrocarbyl.

In one embodiment, the ionizable lipid comprises at least one group of formula -CHF-C(=O)-O- or -CF₂-C(=O)-O-.

In one embodiment, X is -CH₂-.

In one embodiment, X is -C(=O)-.

In one embodiment, X is -O-.

In one embodiment, R² is methyl.

In one embodiment, R² is ethyl.

In one embodiment, R² is propyl.

In one embodiment, R³ is methyl.

In one embodiment, R³ is ethyl.

In one embodiment, R³ is propyl.

In one embodiment, m is 1.

In one embodiment, m is 2.

In one embodiment, m is 3.

In one embodiment, the group: 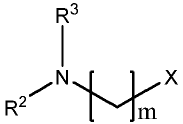 is selected from the group consisting of: (CH₃)₂NCH₂CH₂C(=O)-, (CH₃)₂NCH₂CH₂CH₂C(=O)-, (CH₃)₂NCH₂CH₂CH₂CH₂C(=O)-, (CH₃)₂NCH₂CH₂-, (CH₃)₂NCH₂CH₂CH-, 5-pyrolidin-1-ylpentanoyl, and (CH₃)₂NCH₂CH₂CH₂O-.

In one embodiment, the group: 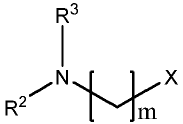 is selected from the group consisting of: (CH₃)₂NCH₂CH₂C(=O)-, (CH₃)₂NCH₂CH₂CH₂C(=O)-, (CH₃)₂NCH₂CH₂CH₂CH₂C(=O)-, (CH₃)₂NCH₂CH₂-, (CH₃)₂NCH₂CH₂CH-, and (CH₃)₂NCH₂CH₂CH₂O-.

In one embodiment, R¹ is C₅-C₂₅hydrocarbyl.

In one embodiment, R¹ is C₂-C₂₅alkyl.

In one embodiment, R¹ is C₅-C₂₅alkyl.

In one embodiment, R¹ is C₅-C₂₅alkenyl.

In one embodiment, R¹ is 1-nonyl or 1-decyl.

In one embodiment, o is 2, 3, 4, 5, 6, 7, 8, 9, 10, 11, 12, 13, 14, or 15.

In one embodiment, o is 10, 11, 12, 13, 14, or 15.

In one embodiment, o is 2, 3, 4, 5, 6, 7, 8, 9, or 10.

In one embodiment, o is 2, 3, 4, or 5.

In one embodiment, L² is -(C=O)-O-, -(C=O)N(H)N(R⁴)-, -(C=O)N(H)-N=C(R⁴)-, -(C=O)N(H)N(H)C(R⁴)(R⁵)-, or -(C=O)-O-CH₂-R^{z}.

In one embodiment, L² is -(C=O)-O-.

In one embodiment, L² is -(C=O)N(H)N(R⁴)-.

In one embodiment, L² is -(C=O)-O-CH₂-R^{z}.

In one embodiment, a is H.

In one embodiment, a is F.

In one embodiment, b is H.

In one embodiment, b is F.

In one embodiment, R⁶ is C₂-C₂₅hydrocarbyl.

In one embodiment, R⁶ is C₅-C₂₅hydrocarbyl.

In one embodiment, R⁶ is C₂-C₂₅alkyl.

In one embodiment, R⁶ is C₅-C₂₅alkyl.

In one embodiment, R⁶ is C₅-C₂₅alkenyl.

In one embodiment, the group: 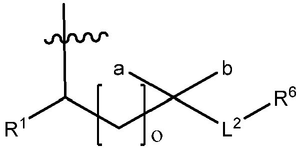 is selected from the group consisting of: 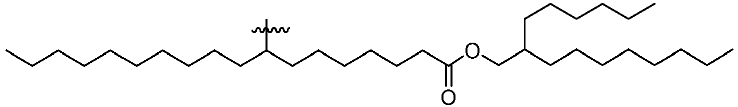 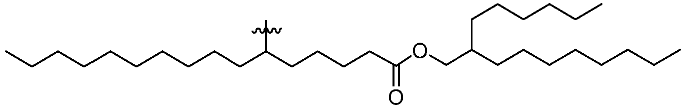 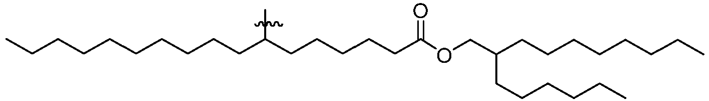 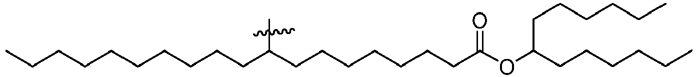 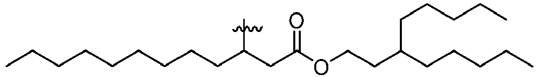 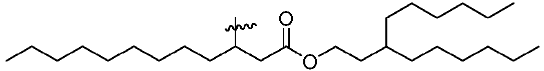 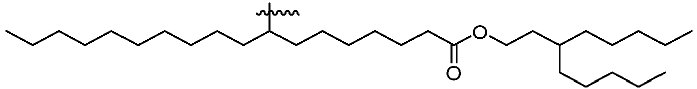 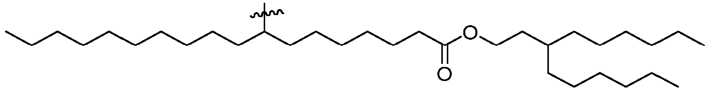 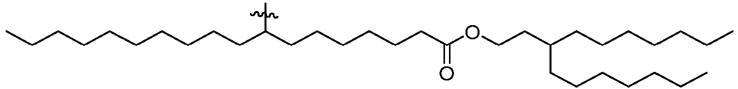 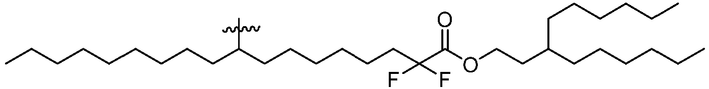 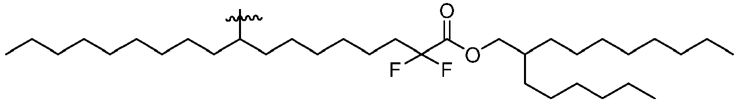 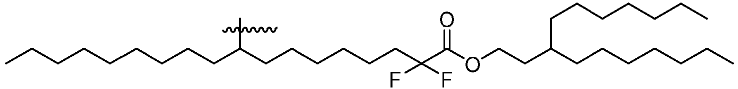 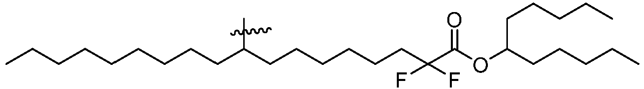 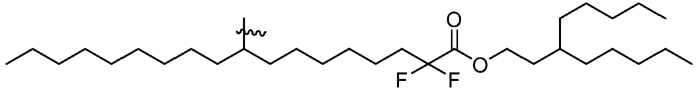 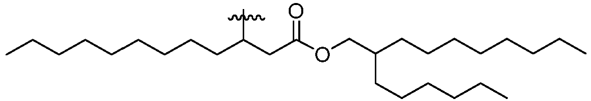 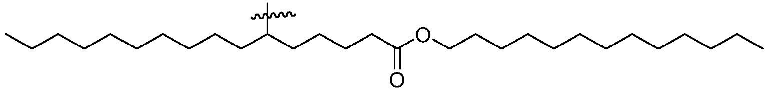 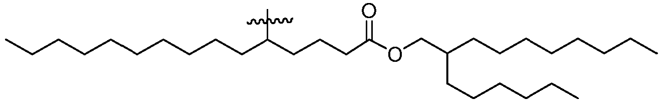 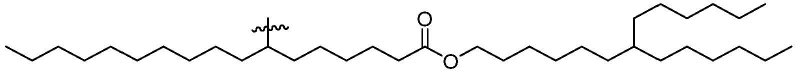 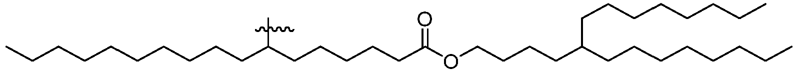 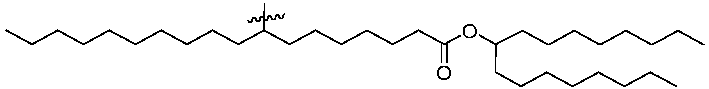 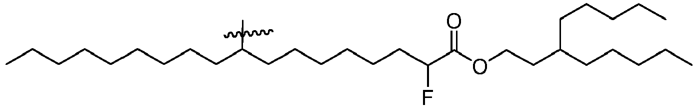 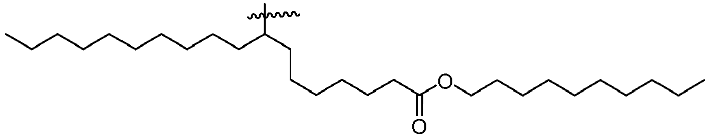 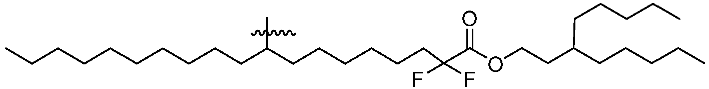 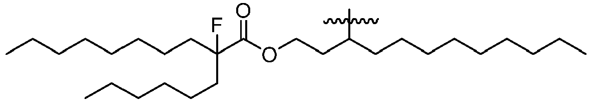 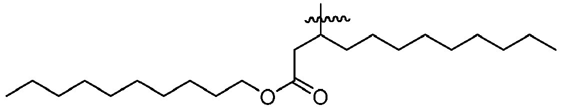 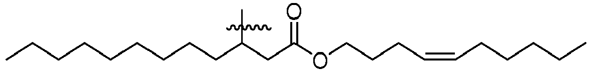 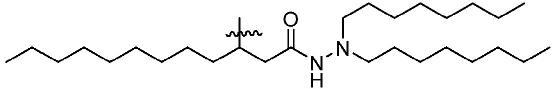 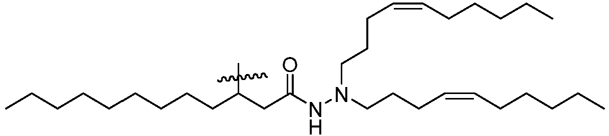 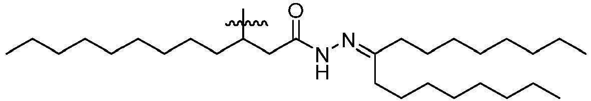 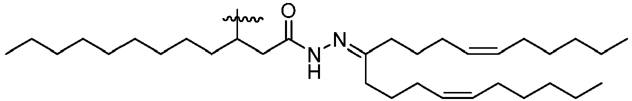 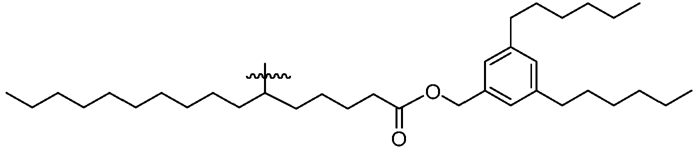 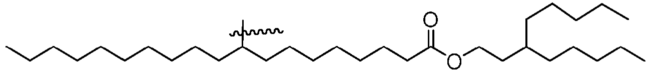 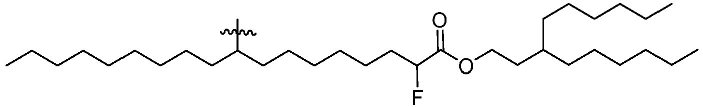 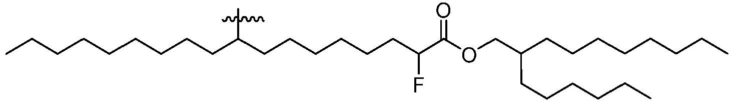 and

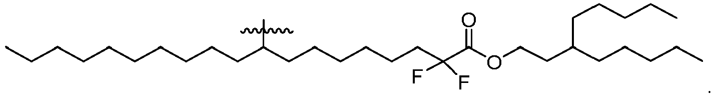

In one embodiment, the group: 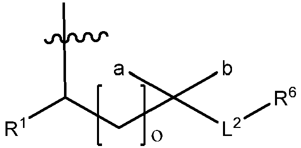 is selected from the group consisting of: 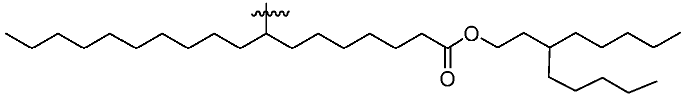 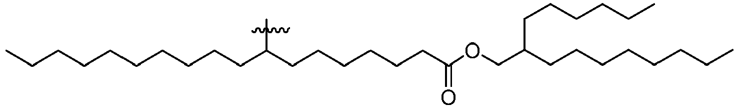 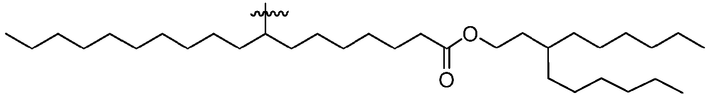 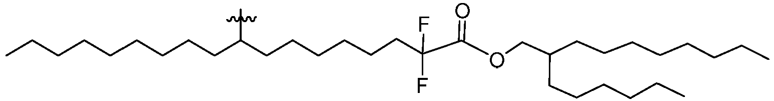 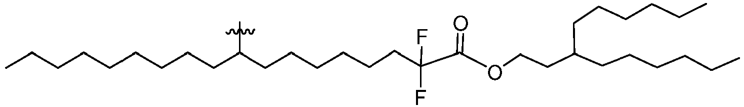 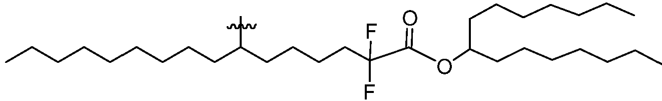 and

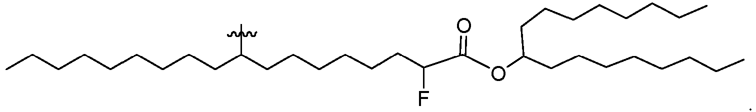

In one embodiment, Y is -CH₂-.

In one embodiment, Y is -C(=O)-.

In one embodiment, Y is -O-.

In one embodiment, n is 2, 3, 4, 5, 6, 7, 8, 9, 10, 11, 12, 13, 14, or 15.

In one embodiment, n is 10, 11, 12, 13, 14, or 15.

In one embodiment, n is 2, 3, 4, 5, 6, 7, 8, 9, or 10.

In one embodiment, n is 2, 3, 4, or 5.

In one embodiment, L¹ is absent; and L² is -(C=O)-O-, -(C=O)N(H)N(R⁴)-, -(C=O)N(H)-N=C(R⁴)-, -(C=O)N(H)N(H)C(R⁴)(R⁵)-, or -(C=O)-O-CH₂-R^{z}.

In one embodiment, L¹ is -(C=O)-O-, -(C=O)N(H)N(R⁴)-, -(C=O)N(H)-N=C(R⁴)-, -(C=O)N(H)N(H)C(R⁴)(R⁵)-, or -(C=O)-O-CH₂-R^{a}; and L² is absent.

In one embodiment, L¹ is -(C=O)-O-, -(C=O)N(H)N(R⁴)-, -(C=O)N(H)-N=C(R⁴)-, -(C=O)N(H)N(H)C(R⁴)(R⁵)-, or -(C=O)-O-CH₂-R^{a}.

In one embodiment, L¹ is -(C=O)-O.

In one embodiment, L¹ is -(C=O)N(H)N(R⁴)-.

In one embodiment, L¹ is -(C=O)-O-CH₂-R^{z}.

In one embodiment, c is H.

In one embodiment, c is F.

In one embodiment, d is H.

In one embodiment, d is F.

In one embodiment, R⁷ is C₂-C₂₅hydrocarbyl.

In one embodiment, R⁷ is C₅-C₂₅hydrocarbyl.

In one embodiment, R⁷ is C₂-C₂₅alkyl.

In one embodiment, R⁷ is C₅-C₂₅alkyl.

In one embodiment, R⁷ is C₅-C₂₅alkenyl.

In one embodiment, the group: 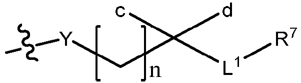 is selected from the group consisting of:

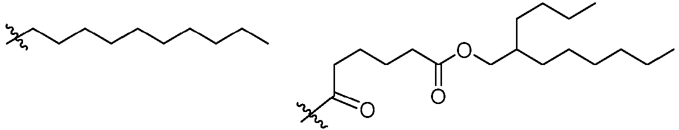

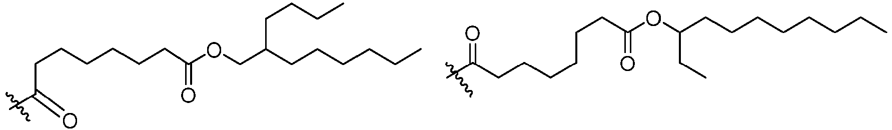

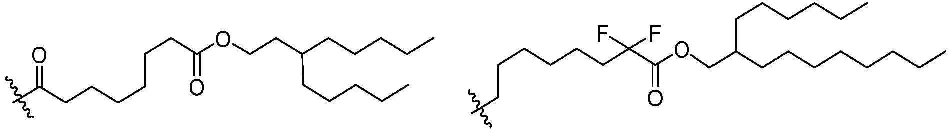

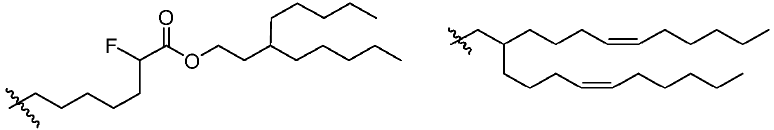

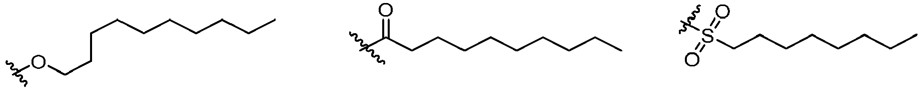

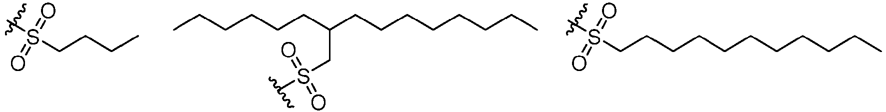

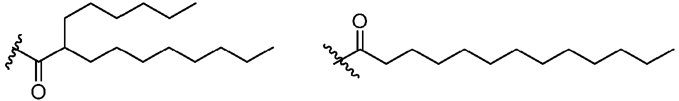

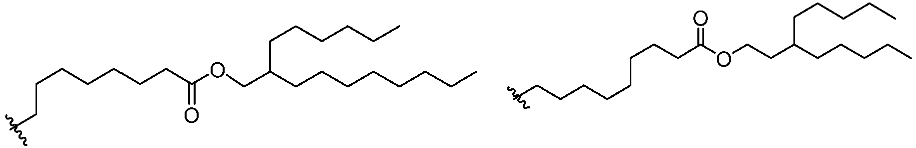

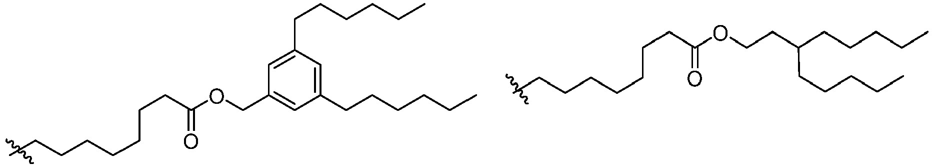

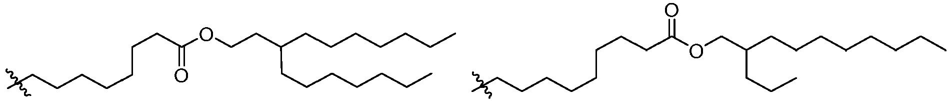

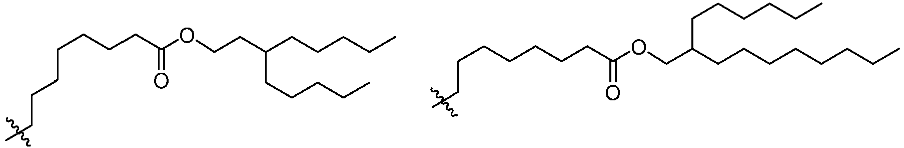

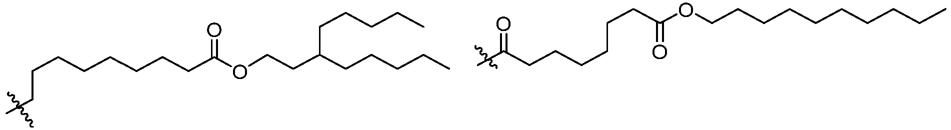

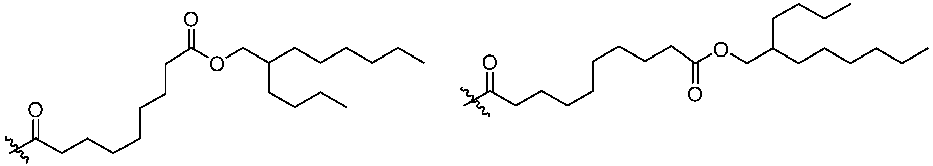

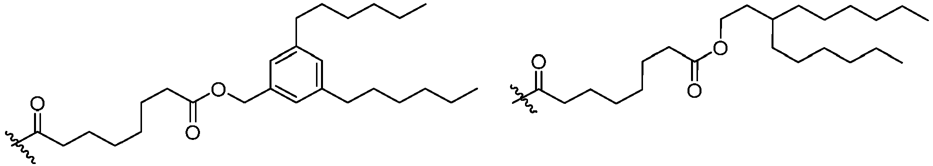

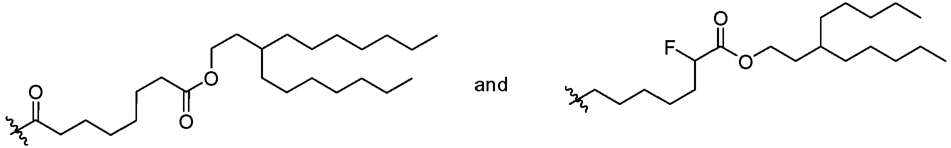

In one embodiment, the group: 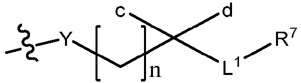 is selected from the group consisting of:

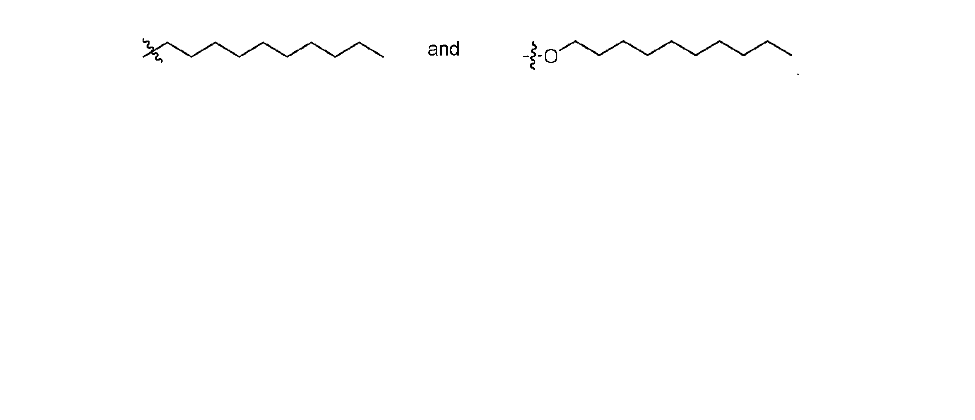

In one embodiment, R⁴ is C₂-C₂₅hydrocarbyl.

In one embodiment, R⁴ is C₅-C₂₅hydrocarbyl.

In one embodiment, R⁴ is C₂-C₂₅alkyl.

In one embodiment, R⁴ is C₅-C₂₅alkyl.

In one embodiment, R⁴ is C₅-C₂₅alkenyl.

In one embodiment, R⁵ is C₂-C₂₅hydrocarbyl.

In one embodiment, R⁵ is C₅-C₂₅hydrocarbyl.

In one embodiment, R⁵ is C₂-C₂₅alkyl.

In one embodiment, R⁵ is C₅-C₂₅alkyl.

In one embodiment, R⁵ is C₅-C₂₅alkenyl.

In one embodiment, R^{4'} is C₂-C₂₅hydrocarbyl.

In one embodiment, R'⁴ is C₅-C₂₅hydrocarbyl.

In one embodiment, R^{4'} is C₂-C₂₅alkyl.

In one embodiment, R^{4'} is C₅-C₂₅alkyl.

In one embodiment, R^{4'} is C₅-C₂₅alkenyl.

In one embodiment, R'⁵ is C₂-C₂₅hydrocarbyl.

In one embodiment, R'⁵*^{'}* is C₅-C₂₅hydrocarbyl.

In one embodiment, R^{5'} is C₂-C₂₅alkyl.

In one embodiment, R⁵ is C₅-C₂₅alkyl.

In one embodiment, R^{5'} is C₅-C₂₅alkenyl.

In one embodiment, R^{z} is phenyl that is substituted with 1 or 2 groups independently selected from C₂-C₁₀hydrocarbyl. In one embodiment, each C₂-C₁₀hydrocarbyl is independently selected from the group consisting of C₅-C₁₀hydrocarbyl. In one embodiment, each C₂-C₁₀hydrocarbyl is independently selected from the group consisting of C₂-C₁₀alkyl. In one embodiment, each C₂-C₁₀hydrocarbyl is independently selected from the group consisting of C₅-C₁₀alkyl. In one embodiment, each C₂-C₁₀hydrocarbyl is independently selected from the group consisting of C₅-C₁₀alkenyl.

In one embodiment, R^{z'} is phenyl that is substituted with 1 or 2 groups independently selected from C₂-C₁₀hydrocarbyl. In one embodiment, each C₂-C₁₀hydrocarbyl is independently selected from the group consisting of C₅-C₁₀hydrocarbyl. In one embodiment, each C₂-C₁₀hydrocarbyl is independently selected from the group consisting of C₂-C₁₀alkyl. In one embodiment, each C₂-C₁₀hydrocarbyl is independently selected from the group consisting of C₅-C₁₀alkyl. In one embodiment, each C₂-C₁₀hydrocarbyl is independently selected from the group consisting of C₅-C₁₀alkenyl.

In one embodiment, the ionizable lipid is selected from the compounds of Examples 1, 16, 17, 43, 46, 90, 101, and 115, and salts thereof.

In one embodiment, X is -C(=O)- and Y is absent or -O-.

In one embodiment, X is -C(=O)- and Y is -O-.

In one embodiment, Y is -CH₂-; L¹ is absent; and c and d are each H.

In one embodiment, a is F or b is H.

In one embodiment, a is F and b is F.

In one embodiment, a is F; b is H; and L¹ is -(C=O)-O-.

In one embodiment, a is F; b is F; and L¹ is -(C=O)-O-.

In one embodiment, R² and R³ are each methyl.

In one embodiment, L¹ is absent; and c and d are each H.

In one embodiment, L¹ is absent; and a, b, c, and d are each H.

In one embodiment, one of L¹ and L² is -(C=O)-O-.

In one embodiment, L¹ is -(C=O)-O- and R⁷ is a branched C₂-C₂₅hydrocarbyl.

In one embodiment, L¹ is -(C=O)-O- and R⁷ is a C₅-C₂₅hydrocarbyl that is branched at a carbon that is within 3 carbons of the point of attachment to L¹.

In one embodiment, L¹ is -(C=O)-O- and R⁷ is a C₄-C₂₅hydrocarbyl that is branched at a carbon that is within 2 carbons of the point of attachment to L¹.

In one embodiment, L¹ is -(C=O)-O- and R⁷ is a C₃-C₂₅hydrocarbyl that is branched at a carbon that is attached to L¹.

In one embodiment, one of L¹ and L² is -(C=O)-O- and R⁷ is a branched C₂-C₂₅hydrocarbyl.

In one embodiment, one of L¹ and L² is -(C=O)-O- and R⁷ is a C₅-C₂₅hydrocarbyl that is branched at a carbon that is within 3 carbons of the point of attachment to L¹.

In one embodiment, one of L¹ and L² is -(C=O)-O- and R⁷ is a C₄-C₂₅hydrocarbyl that is branched at a carbon that is within 2 carbons of the point of attachment to L¹.

In one embodiment, one of L¹ and L² is -(C=O)-O- and R⁷ is a C₃-C₂₅hydrocarbyl that is branched at a carbon that is attached to L¹.

In one embodiment, Y is absent or -O-; N is 0; c is H; d is H; L¹ is absent; and R⁷ is C₂-C₂₅hydrocarbyl.

In one embodiment, Y is absent or -O-; N is 0; c is H; d is H; L¹ is absent; and R⁷ is C₅-C₁₅hydrocarbyl.

In one embodiment, Y is absent or -O-; N is 0; c is H; d is H; L¹ is absent; and R⁷ is C₁₀hydrocarbyl.

In one embodiment, Y is absent or -O-; N is 0; c is H; d is H; L¹ is absent; and R⁷ is C₅-C₁₅hydrocarbyl that is branched at a carbon that is within 3 carbons of the point of attachment to the remainder of the compound of formula (I).

In one embodiment, Y is absent or -O-; N is 0; c is H; d is H; L¹ is absent; and R⁷ is C₅-C₁₅hydrocarbyl that is branched at a carbon that is within 2 carbons of the point of attachment to the remainder of the compound of formula (I).

In one embodiment, Y is absent or -O-; N is 0; c is H; d is H; L¹ is absent; and R⁷ is C₅-C₁₅hydrocarbyl that is branched at a carbon that is attached to the remainder of the compound of formula (I).

In one embodiment, Y is absent or -O-; N is 0; c is H; d is H; L¹ is absent; and R⁷ is C₁₀hydrocarbyl that is branched at a carbon that is within 3 carbons of the point of attachment to the remainder of the compound of formula (I).

In one embodiment, Y is absent or -O-; N is 0; c is H; d is H; L¹ is absent; and R⁷ is C₁₀hydrocarbyl that is branched at a carbon that is within 2 carbons of the point of attachment to the remainder of the compound of formula (I).

In one embodiment, Y is absent or -O-; N is 0; c is H; d is H; L¹ is absent; and R⁷ is C₁₀hydrocarbyl that is branched at a carbon that is attached to the remainder of the compound of formula (I).

In one embodiment, L² is -(C=O)-O- and R⁶ is a branched C₂-C₂₅hydrocarbyl.

In one embodiment, L² is -(C=O)-O- and R⁶ is a C₅-C₂₅hydrocarbyl that is branched at a carbon that is within 3 carbons of the point of attachment to L².

In one embodiment, L² is -(C=O)-O- and R⁶ is a C₄-C₂₅hydrocarbyl that is branched at a carbon that is within 2 carbons of the point of attachment to L².

In one embodiment, L² is -(C=O)-O- and R⁶ is a C₃-C₂₅hydrocarbyl that is branched at a carbon that is attached to L².

In one embodiment, L² is -(C=O)-O-; a is F; b is H; and R⁶ is a branched C₂-C₂₅hydrocarbyl.

In one embodiment, L² is -(C=O)-O-; a is F; b is H; and R⁶ is a C₅-C₂₅hydrocarbyl that is branched at a carbon that is within 3 carbons of the point of attachment to L².

In one embodiment, one of L² is -(C=O)-O-; a is F; b is H; and R⁶ is a C₄-C₂₅hydrocarbyl that is branched at a carbon that is within 2 carbons of the point of attachment to L².

In one embodiment, L² is -(C=O)-O-; a is F; b is H; and R⁶ is a C₃-C₂₅hydrocarbyl that is branched at a carbon that is attached to L².

In one embodiment, L² is -(C=O)-O-; a is F; b is F; and R⁶ is a branched C₂-C₂₅hydrocarbyl.

In one embodiment, L² is -(C=O)-O-; a is F; b is F; and R⁶ is a C₅-C₂₅hydrocarbyl that is branched at a carbon that is within 3 carbons of the point of attachment to L².

In one embodiment, L² is -(C=O)-O-; a is F; b is F; and R⁶ is a C₄-C₂₅hydrocarbyl that is branched at a carbon that is within 2 carbons of the point of attachment to L².

In one embodiment, L² is -(C=O)-O-; a is F; b is F; and R⁶ is a C₃-C₂₅hydrocarbyl that is branched at a carbon that is attached to L².

In one embodiment, the invention provides a compound of formula (I): 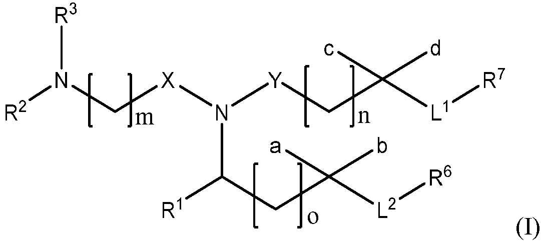 or a salt thereof, wherein:
X is -CH₂-, -O-, -O(C=O); or -C(=O)-
Y is -CH₂-, -O-, -SO₂-, or -C(=O)-;
R¹ is C₂-C₂₅hydrocarbyl;
R² is C₁-C₃alkyl; and R³ is C₁-C₃alkyl; or R² and R³ taken together with the nitrogen to which they are attached form a a aziridino, azetidino, or pyrrolidino ring;
m is 0, 1, 2, 3, 4, 5 or 6;
n is 0-15;
o is 0-15;
L¹ is absent, -(C=O)-O-, -(C=O)N(H)N(R⁴)-, -(C=O)N(H)-N=C(R⁴)-, -(C=O)N(H)N(H)C(R⁴)(R⁵)-, -(C=O)-O-CH₂-R^{z}, or -O(C=O)-R^{w}-;
L² is absent, -(C=O)-O-, -(C=O)N(H)N(R^{4'})-, -(C=O)N(H)-N=C(R^{4'})-, -(C=O)N(H)N(H)C(R^{4'})(R^{5'})-, -(C=O)-O-CH₂-R^{z'}, or -O(C=O)-R^{x}-
a is H or F;
b is H or F;
c is H or F; and d is H or F; or c and d taken together with the carbon to which they are attached form -C(=O)-;
R⁴ is H or C₂-C₂₅hydrocarbyl;
R⁵ is H or C₂-C₂₅hydrocarbyl;
R^{4'} is H or C₂-C₂₅hydrocarbyl;
R^{5'} is H or C₂-C₂₅hydrocarbyl;
R⁶ is C₂-C₂₅hydrocarbyl;
R⁷ is C₂-C₂₅hydrocarbyl;
R^{w} is absent, -C(F)H-, or -C(F)₂-;
R^{x} is absent, -C(F)H-, or -C(F)₂-;
R^{z} is phenyl that is substituted with 1, 2, or 3 groups independently selected from C₂-C₁₀hydrocarbyl; and
R^{z'} is phenyl that is substituted with 1, 2, or 3 groups independently selected from C₂-C₁₀hydrocarbyl.

In one embodiment, the invention provides a compound of formula (I): 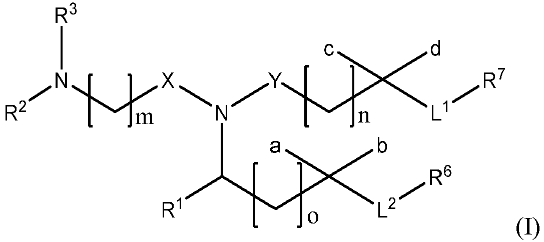 or a salt thereof, wherein:
X is -CH₂-, -O-, -O(C=O); or -C(=O)-
Y is -CH₂-, -O-, -SO₂-, or -C(=O)-;
R¹ is C₂-C₂₅hydrocarbyl;
R² is C₁-C₃alkyl; and R³ is C₁-C₃alkyl; or R² and R³ taken together with the nitrogen to which they are attached form a a aziridino, azetidino, or pyrrolidino ring;
m is 0, 1, 2, 3, 4, 5 or 6;
n is 0-15;
o is 0-15;
L¹ is absent or -(C=O)-O-;
L² is absent or -(C=O)-O-;
a is H or F;
b is H or F;
c is H or F; and d is H or F; or c and d taken together with the carbon to which they are attached form -C(=O)-;
R⁴ is H or C₂-C₂₅hydrocarbyl;
R⁵ is H or C₂-C₂₅hydrocarbyl;
R^{4'} is H or C₂-C₂₅hydrocarbyl;
R^{5'} is H or C₂-C₂₅hydrocarbyl;
R⁶ is C₂-C₂₅hydrocarbyl;
R⁷ is C₂-C₂₅hydrocarbyl;
R^{w} is absent, -C(F)H-, or -C(F)₂-;
R^{x} is absent, -C(F)H-, or -C(F)₂-;
R^{z} is phenyl that is substituted with 1, 2, or 3 groups independently selected from C₂-C₁₀hydrocarbyl; and
R^{z'} is phenyl that is substituted with 1, 2, or 3 groups independently selected from C₂-C₁₀hydrocarbyl.

### Specific Embodiments

In one specific embodiment, the invention provides an ionizable lipid of formula (I), wherein X is -C(=O)-. Such ionizable lipids typically demonstrate high activity.

In one specific embodiment, the invention provides an ionizable lipid of formula (I), wherein X is -C(=O)- and Y is -O-. Such ionizable lipids typically demonstrate high activity and possess desirable pKa properties.

In one specific embodiment, the invention provides an ionizable lipid of formula (I), wherein Y is -CH₂-; L¹ is absent; and c and d are each H. Such ionizable lipids typically demonstrate high activity.

In one specific embodiment, the invention provides an ionizable lipid of formula (I), wherein R² is methyl and R³ is methyl. Such ionizable lipids typically demonstrate high activity.

In one specific embodiment, the invention provides an ionizable lipid of formula (I), wherein L¹ is absent; and c and d are each H. Such ionizable lipids typically demonstrate high activity.

In one specific embodiment, the invention provides an ionizable lipid of formula (I), wherein L² is -(C=O)-O- and R⁶ is a C₂-C₂₅hydrocarbyl that is branched at a carbon atom that is attached to L² or that is branched at a carbon atom that is 2 carbons from the point of attachment to L² or that is branched at a carbon atom that is 3 carbons from the point of attachment to L². Such ionizable lipids typically demonstrate high activity.

When a hydrocarbyl is branched at a carbon atom that is attached to L¹ or L², or is branched at a carbon atom that is 2 carbons from the point of attachment to L¹ or L², or is branched at a carbon atom that is 3 carbons from the point of attachment to L¹ or L², it is understood that the hydrocarbyl is branched at one of the carbons illustrated in the following formula:

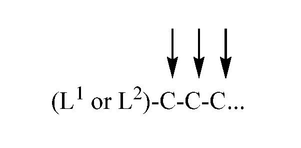

In one embodiment, the invention provides a nucleic acid-lipid particle comprising:
(a) one or more nucleic acid molecules;
(b) a non-cationic lipid;
(c) a conjugated lipid; and
(d) a compound of formula (I) or salt thereof, wherein the one or more nucleic acid molecules are encapsulated within the lipid particle.

In one embodiment, the compound or salt comprises from about 30 mol % to about 85 mol % of the total lipid present in the particle; the non-cationic lipid comprises from about 13 mol % to about 49.5 mol % of the total lipid present in the particle; and the conjugated lipid comprises from about 0.1 mol % to about 10 mol % of the total lipid present in the particle.

In one embodiment, the compound or salt comprises from about 30 mol % to about 85 mol % of the total lipid present in the particle; the non-cationic lipid comprises from about 13 mol % to about 49.5 mol % of the total lipid present in the particle; and the conjugated lipid comprises from about 0.1 mol % to about 10 mol % of the total lipid present in the particle.

In one embodiment, the nucleic acid is selected from the group consisting of small interfering RNA (siRNA), Dicer-substrate dsRNA, small hairpin RNA (shRNA), asymmetrical interfering RNA (aiRNA), microRNA (miRNA), mRNA, tRNA, rRNA, viral RNA (vRNA), self-amplifying RNA, and combinations thereof.

In one embodiment, the nucleic acid is an mRNA molecule.

In one embodiment, the nucleic acid comprises a double stranded siRNA molecule.

In one embodiment, the double stranded siRNA molecule comprises at least one modified nucleotide.

In one embodiment, the siRNA comprises at least one 2'-O-methyl (2'OMe) nucleotide.

In one embodiment, the non-cationic lipid comprises cholesterol or a derivative thereof.

In one embodiment, the cholesterol or derivative thereof comprises from about 31.5 mol % to about 42.5 mol % of the total lipid present in the particle.

In one embodiment, the non-cationic lipid comprises a phospholipid.

In one embodiment, the non-cationic lipid comprises a mixture of a phospholipid and cholesterol or a derivative thereof.

In one embodiment, the phospholipid comprises dipalmitoylphosphatidylcholine (DPPC), distearoylphosphatidylcholine (DSPC), or a mixture thereof.

In one embodiment, the phospholipid comprises from about 4 mol % to about 10 mol % of the total lipid present in the particle and the cholesterol comprises from about 30 mol % to about 40 mol % of the total lipid present in the particle.

In one embodiment, the phospholipid comprises from about 10 mol % to about 30 mol % of the total lipid present in the particle and the cholesterol comprises from about 10 mol % to about 30 mol % of the total lipid present in the particle.

In one embodiment, the conjugated lipid comprises a polyethyleneglycol (PEG)-lipid conjugate.

In one embodiment, the PEG-lipid conjugate comprises a compound of formula (I): 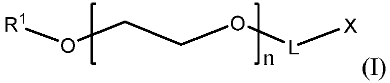 or a salt thereof, wherein:
R¹ is H, (C₁-C₆)alkyl, or (C₁-C₆)alkanoyl;
n is an integer in the range of from about 10 to about 150; and
L is absent and X is -C(=O)NR²R³; or
L is (C₁-C₆)alkyl and X is selected from the group consisting of -N(R⁴)C(=O)CH(R²)(R³), -OCH(R²)(R³), -C(=O)OCH₂CH(R²)(R³), -N(R⁴)C(=O)N(R²)(R³), and -SO₂N(R²)(R³);
R² is (C₁₀-C₂₀)alkyl;
R³ is (C₁₀-C₂₀)alkyl; and
R⁴ is H or (C₁-C₆)alkyl.

In one embodiment, the PEG-lipid conjugate comprises a compound selected from: 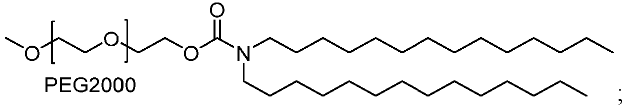 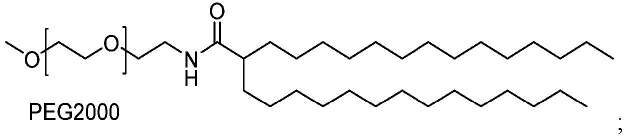 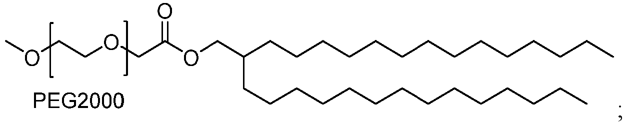 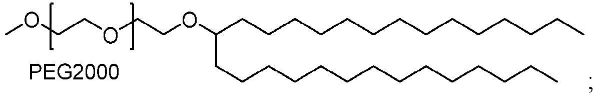 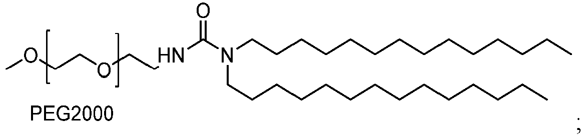 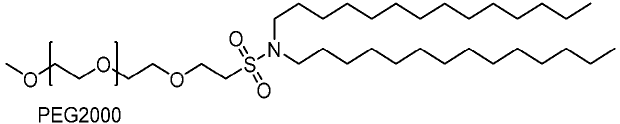 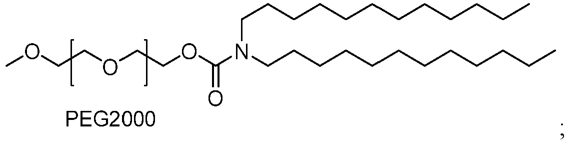 and 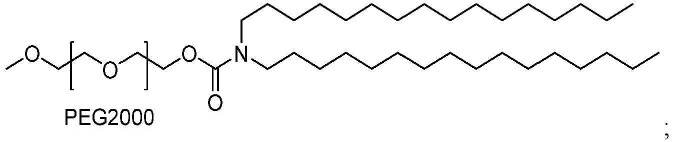 or a salt thereof.

In one embodiment, the PEG-lipid conjugate comprises a compound of the following formula: 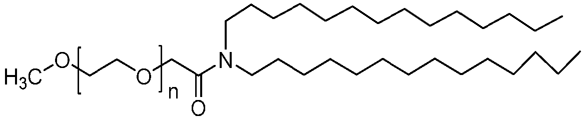 wherein n is 45-50.

In one embodiment, the PEG-lipid conjugate comprises a PEG-diacylglycerol (PEG-DAG) conjugate, a PEG-dialkyloxypropyl (PEG-DAA) conjugate, or a mixture thereof.

In one embodiment, the PEG-DAA conjugate comprises a PEG-dimyristyloxypropyl (PEG-DMA) conjugate, a PEG-distearyloxypropyl (PEG-DSA) conjugate, or a mixture thereof.

In one embodiment, the PEG-DAA conjugate comprises a PEG-dimyristyloxypropyl (PEG-DMA) conjugate.

In one embodiment, the polyethyleneglycol (PEG)-lipid conjugate is a compound of formula:

A-B-C

or a salt thereof, wherein:
A is (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl , (C₁-C₆)alkylthio , or (C₂-C₆)alkanoyloxy, wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is substituted with one or more anionic precursor groups, and wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is optionally substituted with one or more groups independently selected from the group consisting of halo, hydroxyl, (C₁-C₃)alkoxy, (C₁-C₆)alkanoyl, (C₁-C₃)alkoxycarbonyl , (C₁-C₃)alkylthio , or (C₂-C₃)alkanoyloxy;
B is a polyethylene glycol chain having a molecular weight of from about 550 daltons to about 10,000 daltons;
C is -L-R^{a}
L is selected from the group consisting of a direct bond, -C(O)O-, -C(O)NR^{b}-, -NR^{b}-, - C(O)-, -NR^{b}C(O)O-, -NR^{b}C(O)NR^{b}-, -S-S-, -O-, -(O)CCH₂CH₂C(O)-, and -NHC(O)CH₂CH₂C(O)NH-;
R^{a} is a branched (C₁₀-C₅₀)alkyl or branched (C₁₀-C₅₀)alkenyl wherein one or more carbon atoms of the branched (C₁₀-C₅₀)alkyl or branched (C₁₀-C₅₀)alkenyl have been replaced with -O-; and
each R^{b} is independently H or (C₁-C₆)alkyl.

In one embodiment, the PEG has an average molecular weight of about 2,000 daltons.

In one embodiment, the conjugated lipid comprises from about 1 mol % to about 2 mol % of the total lipid present in the particle.

In one embodiment, the nucleic acid in the nucleic acid-lipid particle is not substantially degraded after incubation of the particle in serum at 37°C for 30 minutes.

In one embodiment, the nucleic acid is fully encapsulated in the nucleic acid-lipid particle.

In one embodiment, the nucleic acid-lipid particle has a lipid:nucleic acid mass ratio of from about 5 to about 15.

In one embodiment, the nucleic acid-lipid particle has a lipid:nucleic acid mass ratio of from about 5 to about 30.

In one embodiment, the nucleic acid-lipid particle has a median diameter of from about 40 nm to about 150 nm.

In one embodiment, the compound or salt comprises from about 56.5 mol % to about 66.5 mol % of the total lipid present in the particle; the non-cationic lipid comprises cholesterol or a derivative thereof comprising from about 31.5 mol % to about 42.5 mol % of the total lipid present in the particle; and a PEG-lipid conjugate comprises from about 1 mol % to about 2 mol % of the total lipid present in the particle.

In one embodiment, the nucleic acid-lipid particle comprises about 61.5 mol % compound or salt, about 36.9% cholesterol or a derivative thereof, and about 1.5 mol % PEG-lipid conjugate.

In one embodiment, the compound or salt comprises from about 52 mol % to about 62 mol % of the total lipid present in the particle; the non-cationic lipid comprises mixture of a phospholipid and cholesterol or a derivative thereof comprising from about 36 mol % to about 47 mol % of the total lipid present in the particle; and the PEG-lipid conjugate comprising from about 1 mol % to about 2 mol % of the total lipid present in the particle.

In one embodiment, the nucleic acid-lipid particle comprises about 57.1 mol % compound or salt, about 7.1 mol % phospholipid, about 34.3 mol % cholesterol or a derivative thereof, and about 1.4 mol % PEG-lipid conjugate.

In one embodiment, the nucleic acid-lipid particle comprises about 57.1 mol % compound or salt, about 20 mol % phospholipid, about 20 mol % cholesterol or a derivative thereof, and about 1.4 mol % PEG-lipid conjugate.

In one embodiment, the compound or salt comprises from 50 mol % to 65 mol % of the total lipid present in the particle; the non-cationic lipid comprises a mixture of a phospholipid and cholesterol or a derivative thereof, wherein the phospholipid comprises from 4 mol % to 10 mol % of the total lipid present in the particle and the cholesterol or derivative thereof comprises from 30 mol % to 40 mol % of the total lipid present in the particle; and the conjugated lipid comprises from 0.5 mol % to 2 mol % of the total lipid present in the particle.

In one embodiment, the compound or salt comprises from 50 mol % to 65 mol % of the total lipid present in the particle; the non-cationic lipid comprising a mixture of a phospholipid and cholesterol or a derivative thereof, wherein the phospholipid comprises from 3 mol % to 15 mol % of the total lipid present in the particle and the cholesterol or derivative thereof comprises from 30 mol % to 40 mol % of the total lipid present in the particle; and the conjugated lipid comprises from 0.5 mol % to 2 mol % of the total lipid present in the particle.

In one embodiment, the compound or salt comprises from 50 mol % to 65 mol % of the total lipid present in the particle; the non-cationic lipid comprises up to 49.5 mol % of the total lipid present in the particle; the cholesterol or derivative thereof comprises from 30 mol % to 40 mol % of the total lipid present in the particle; and the conjugated lipid comprises from 0.5 mol % to 2 mol % of the total lipid present in the particle.

In one embodiment, the compound or salt comprises from 50 mol % to 85 mol % of the total lipid present in the particle; the non-cationic lipid comprises from 13 mol % to 49.5 mol % of the total lipid present in the particle; and the conjugated lipid comprises from 0.5 mol % to 2 mol % of the total lipid present in the particle.

In one embodiment, the compound or salt comprises from about 30 mol % to about 50 mol % of the total lipid present in the particle; the non-cationic lipid comprises mixture of a phospholipid and cholesterol or a derivative thereof comprising from about 47 mol % to about 69 mol % of the total lipid present in the particle; and the conjugated lipid comprises from about 1 mol % to about 3 mol % of the total lipid present in the particle.

In one embodiment, the non-cationic lipid comprises a mixture of cholesterol and DSPC; the conjugated lipid is: 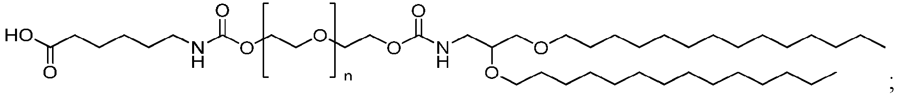 wherein n is selected so that the resulting polymer chain has a molecular weight of about 2000 daltons
wherein the conjugated lipid comprises about 2 mol % of the total lipid present in the particle; DSPC comprises about 10 mol % of the total lipid present in the particle; cholesterol comprises about 48 mol % of the total lipid present in the particle; and the compound or salt comprises about 40 mol % of the total lipid present in the particle.

In one embodiment, the non-cationic lipid comprises a mixture of cholesterol and DSPC; the conjugated lipid is: 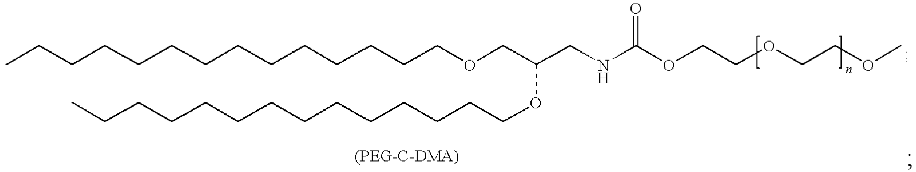 wherein n is selected so that the resulting polymer chain has a molecular weight of about 2000 daltons;
wherein the conjugated lipid comprises about 2 mol % of the total lipid present in the particle; DSPC comprises about 10 mol % of the total lipid present in the particle; cholesterol comprises about 48 mol % of the total lipid present in the particle; and the compound or salt comprises about 40 mol % of the total lipid present in the particle.

In one embodiment, the non-cationic lipid comprises a mixture of cholesterol and DSPC; the conjugated lipid is: 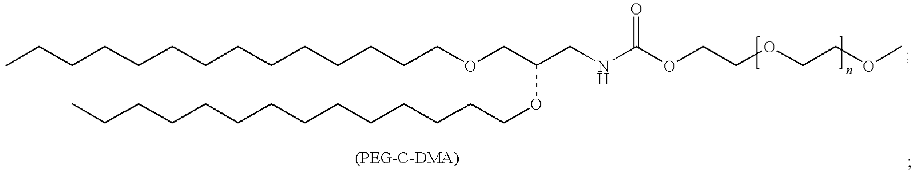 wherein n is selected so that the resulting polymer chain has a molecular weight of about 2000;
wherein the conjugated lipid comprises about 1.6 mol % of the total lipid present in the particle; DSPC comprises about 10.9 mol % of the total lipid present in the particle; cholesterol comprises about 32.8 mol % of the total lipid present in the particle; and the compound or salt comprises about 54.9 mol % of the total lipid present in the particle.

In one embodiment, the lipid to nucleic acid ratio is about 24.

In one embodiment, the nucleic acid-lipid particle comprises two or more different compounds of formula (I) or salts thereof.

### Non-Cationic Lipids

The non-cationic lipids used in the lipid particles of the invention (e.g., LNP) can be any of a variety of neutral uncharged, zwitterionic, or anionic lipids capable of producing a stable complex.

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoylphosphatidylethanolamine 4-(N-maleimidomethyl)-cyclohexane-1-carboxylate (DOPE-mal), dipalmitoyl-phosphatidylethanolamine (DPPE), dimyristoyl-phosphatidylethanolamine (DMPE), distearoyl-phosphatidylethanolamine (DSPE), monomethyl-phosphatidylethanolamine, dimethyl-phosphatidylethanolamine, dielaidoyl-phosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are preferably acyl groups derived from fatty acids having C₁₀-C₂₄ carbon chains, e.g., lauroyl, myristoyl, palmitoyl, stearoyl, or oleoyl.

Additional examples of non-cationic lipids include sterols such as cholesterol and derivatives thereof such as cholestanol, cholestanone, cholestenone, coprostanol, cholesteryl-2'-hydroxyethyl ether, cholesteryl-4'-hydroxybutyl ether, and mixtures thereof.

In some embodiments, the non-cationic lipid present in the lipid particles (e.g., LNP) comprises or consists of cholesterol or a derivative thereof, e.g., a phospholipid-free lipid particle formulation. In other embodiments, the non-cationic lipid present in the lipid particles (e.g., LNP) comprises or consists of one or more phospholipids, e.g., a cholesterol-free lipid particle formulation. In further embodiments, the non-cationic lipid present in the lipid particles (e.g., LNP) comprises or consists of a mixture of one or more phospholipids and cholesterol or a derivative thereof.

Other examples of non-cationic lipids suitable for use in the present invention include nonphosphorous containing lipids such as, e.g., linoleyl alcohol, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, ceramide, sphingomyelin, and the like.

In some embodiments, the non-cationic lipid comprises from about 13 mol % to about 49.5 mol %, from about 20 mol % to about 45 mol %, from about 25 mol % to about 45 mol %, from about 30 mol % to about 45 mol %, from about 35 mol % to about 45 mol %, from about 20 mol % to about 40 mol %, from about 25 mol % to about 40 mol %, or from about 30 mol % to about 40 mol % of the total lipid present in the particle.

In certain embodiments, the cholesterol present in phospholipid-free lipid particles comprises from about 30 mol % to about 45 mol %, from about 30 mol % to about 40 mol %, from about 35 mol % to about 45 mol %, or from about 35 mol % to about 40 mol % of the total lipid present in the particle. As a non-limiting example, a phospholipid-free lipid particle may comprise cholesterol at about 37 mol % of the total lipid present in the particle.

In certain other embodiments, the cholesterol present in lipid particles containing a mixture of phospholipid and cholesterol comprises from about 30 mol % to about 40 mol %, from about 30 mol % to about 35 mol %, or from about 35 mol % to about 40 mol % of the total lipid present in the particle. As a non-limiting example, a lipid particle comprising a mixture of phospholipid and cholesterol may comprise cholesterol at about 34 mol % of the total lipid present in the particle. As other non-limiting examples, the particle may comprise cholesterol at either 33% or 38.5%.

In further embodiments, the cholesterol present in lipid particles containing a mixture of phospholipid and cholesterol comprises from about 10 mol % to about 30 mol %, from about 15 mol % to about 25 mol %, or from about 17 mol % to about 23 mol % of the total lipid present in the particle. As a non-limiting example, a lipid particle comprising a mixture of phospholipid and cholesterol may comprise cholesterol at about 20 mol % of the total lipid present in the particle.

In embodiments where the lipid particles contain a mixture of phospholipid and cholesterol or a cholesterol derivative, the mixture may comprise up to about 40, 45, 50, 55, or 60 mol % of the total lipid present in the particle. In certain instances, the phospholipid component in the mixture may comprise from about 2 mol % to about 20 mol %, from about 2 mol % to about 15 mol %, from about 2 mol % to about 12 mol %, from about 2 mol % to about 10 mol %, from about 2 mol % to about 9 mol %, from about 2 mol % to about 8 mol %, 4 mol % to about 20 mol %, from about 4 mol % to about 15 mol %, from about 4 mol % to about 12 mol %, from about 4 mol % to about 10 mol %, from about 4 mol % to about 9 mol %, from about 4 mol % to about 8 mol %, 6 mol % to about 20 mol %, from about 6 mol % to about 15 mol %, from about 6 mol % to about 12 mol %, from about 6 mol % to about 10 mol %, from about 6 mol % to about 9 mol %, from about 2 mol % to about 8 mol %, 8 mol % to about 20 mol %, from about 8 mol % to about 15 mol %, from about 8 mol % to about 12 mol %, from about 8 mol % to about 10 mol %, from about 8 mol % to about 9 mol % of the total lipid present in the particle. As a non-limiting example, a lipid particle comprising a mixture of phospholipid and cholesterol may comprise a phospholipid such as DPPC or DSPC at about 7 mol % (e.g., in a mixture with about 34 mol % cholesterol) of the total lipid present in the particle. In certain other instances, the phospholipid component in the mixture may comprise from about 10 mol % to about 30 mol %, from about 10 mol % to about 15 mol %, from about 15 mol % to about 20 mol %, from about 15 mol % to about 25 mol %, or from about 17 mol % to about 23 mol % of the total lipid present in the particle. As another non-limiting example, a lipid particle comprising a mixture of phospholipid and cholesterol may comprise a phospholipid such as DPPC or DSPC at about 20 mol % (e.g., in a mixture with about 20 mol % cholesterol) of the total lipid present in the particle.

### Lipid Conjugate

In addition to cationic and non-cationic lipids, the lipid particles of the invention (e.g., LNP) comprise a lipid conjugate. The conjugated lipid is useful in that it prevents the aggregation of particles. Suitable conjugated lipids include, but are not limited to, PEG-lipid conjugates, ATTA-lipid conjugates, cationic-polymer-lipid conjugates (CPLs), POZ polyoxazoline (POZ)-lipids, polysarcosine (pSAR)-lipids and mixtures thereof. In certain embodiments, the particles comprise either a PEG-lipid conjugate or an ATTA-lipid conjugate together with a CPL.

In a preferred embodiment, the lipid conjugate is a PEG-lipid. Examples of PEG-lipids include, but are not limited to, PEG coupled to dialkyloxypropyls (PEG-DAA) as described in, e.g., PCT Publication No. WO 05/026372, PEG coupled to diacylglycerol (PEG-DAG) as described in, e.g., U.S. Patent Publication Nos. 20030077829 and 2005008689, PEG coupled to phospholipids such as phosphatidylethanolamine (PEG-PE), PEG conjugated to ceramides as described in, e.g., U.S. Pat. No. 5,885,613, PEG conjugated to cholesterol or a derivative thereof, and mixtures thereof. Additional PEG-lipids include, without limitation, PEG-C-DOMG, 2 KPEG-DMG, and a mixture thereof.

PEG is a linear, water-soluble polymer of ethylene glycol repeating units. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, for example, the following: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), and monomethoxypolyethylene glycol-imidazolyl-carbonyl (MePEG-IM). Other PEGs such as those described in U.S. Pat. Nos. 6,774,180 and 7,053,150 (e.g., mPEG (20 KDa) amine) are also useful for preparing the PEG-lipid conjugates of the present invention. In addition, monomethoxypolyethyleneglycolacetic acid (MePEG-CH₂COOH) is particularly useful for preparing PEG-lipid conjugates including, e.g., PEG-DAA conjugates.

The PEG moiety of the PEG-lipid conjugates described herein may comprise an average molecular weight ranging from about 550 daltons to about 10,000 daltons. In certain instances, the PEG moiety has an average molecular weight of from about 750 daltons to about 5,000 daltons (e.g., from about 1,000 daltons to about 5,000 daltons, from about 1,500 daltons to about 3,000 daltons, from about 750 daltons to about 3,000 daltons, from about 750 daltons to about 2,000 daltons, etc.). In preferred embodiments, the PEG moiety has an average molecular weight of about 2,000 daltons or about 750 daltons.

In certain instances, the PEG can be optionally substituted by an alkyl, alkoxy, acyl, or aryl group. The PEG can be conjugated directly to the lipid or may be linked to the lipid via a linker moiety. Any linker moiety suitable for coupling the PEG to a lipid can be used including, e.g., non-ester containing linker moieties and ester-containing linker moieties. In a preferred embodiment, the linker moiety is a non-ester containing linker moiety. As used herein, the term "non-ester containing linker moiety" refers to a linker moiety that does not contain a carboxylic ester bond (-OC(O)-). Suitable non-ester containing linker moieties include, but are not limited to, amido (-C(O)NH-), amino (-NR-), carbonyl (-C(O)-), carbamate (-NHC(O)O-), urea (-NHC(O)NH-), disulphide (-S-S-), ether (-O-), succinyl (-(O)CCH₂CH₂C(O)-), succinamidyl (-NHC(O)CH₂CH₂C(O)NH-), ether, disulphide, as well as combinations thereof (such as a linker containing both a carbamate linker moiety and an amido linker moiety). In a preferred embodiment, a carbamate linker is used to couple the PEG to the lipid.

In other embodiments, an ester containing linker moiety is used to couple the PEG to the lipid. Suitable ester containing linker moieties include, e.g., carbonate (-OC(O)O-), succinoyl, phosphate esters (-O-(O)POH-O-), sulfonate esters, and combinations thereof.

Additional PEG-lipid conjugates suitable for use in the invention include, but are not limited to, compounds of formula:

A-B-C

or a salt thereof, wherein:
A is (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl , (C₁-C₆)alkylthio , or (C₂-C₆)alkanoyloxy, wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is substituted with one or more anionic precursor groups, and wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is optionally substituted with one or more groups independently selected from the group consisting of halo, hydroxyl, (C₁-C₃)alkoxy, (C₁-C₆)alkanoyl, (C₁-C₃)alkoxycarbonyl , (C₁-C₃)alkylthio , or (C₂-C₃)alkanoyloxy;
B is a polyethylene glycol chain having a molecular weight of from about 550 daltons to about 10,000 daltons;
C is -L-R^{a};
L is selected from the group consisting of a direct bond, -C(O)O-, -C(O)NR^{b}-, -NR^{b}-, - C(O)-, -NR^{b}C(O)O-, -NR^{b}C(O)NR^{b}-, -S-S-, -O-, -(O)CCH₂CH₂C(O)-, and -NHC(O)CH₂CH₂C(O)NH-;
R^{a} is a branched (C₁₀-C₅₀)alkyl or branched (C₁₀-C₅₀)alkenyl wherein one or more carbon atoms of the branched (C₁₀-C₅₀)alkyl or branched (C₁₀-C₅₀)alkenyl have been replaced with -O-; and
each R^{b} is independently H or (C₁-C₆)alkyl.

The conjugated lipids may comprise a PEG-lipid including, e.g., a compound of formula A-PEG-diacylglycerol (DAG), A-PEG dialkyloxypropyl (DAA), A-PEG-phospholipid, A-PEG-ceramide (Cer), or mixtures thereof, wherein A is (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl , (C₁-C₆)alkylthio , or (C₂-C₆)alkanoyloxy, wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is substituted with one or more anionic precursor groups, and wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is optionally substituted with one or more groups independently selected from the group consisting of halo, hydroxyl, (C₁-C₃)alkoxy, (C₁-C₆)alkanoyl, (C₁-C₃)alkoxycarbonyl , (C₁-C₃)alkylthio, or (C₂-C₃)alkanoyloxy. The A-PEG-DAA conjugate may be A-PEG-dilauryloxypropyl (C12), A-PEG-dimyristyloxypropyl (C14), A-PEG-dipalmityloxypropyl (C16), or A-PEG-distearyloxypropyl (C18), or mixtures thereof.

Phosphatidylethanolamines having a variety of acyl chain groups of varying chain lengths and degrees of saturation can be conjugated to PEG to form the lipid conjugate. Such phosphatidylethanolamines are commercially available, or can be isolated or synthesized using conventional techniques known to those of skilled in the art. Phosphatidylethanolamines containing saturated or unsaturated fatty acids with carbon chain lengths in the range of C₁₀ to C₂₀ are preferred. Phosphatidylethanolamines with mono- or diunsaturated fatty acids and mixtures of saturated and unsaturated fatty acids can also be used. Suitable phosphatidylethanolamines include, but are not limited to, dimyristoyl-phosphatidylethanolamine (DMPE), dipalmitoyl-phosphatidylethanolamine (DPPE), dioleoylphosphatidylethanolamine (DOPE), and distearoyl-phosphatidylethanolamine (DSPE).

The term "ATTR" or "polyamide" refers to, without limitation, compounds described in U.S. Pat. Nos. 6,320,017 and 6,586,559. These compounds include a compound having the formula: 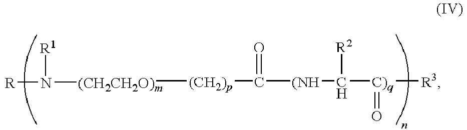 wherein R is a member selected from the group consisting of hydrogen, alkyl and acyl; R¹ is a member selected from the group consisting of hydrogen and alkyl; or optionally, R and R¹ and the nitrogen to which they are bound form an azido moiety; R² is a member of the group selected from hydrogen, optionally substituted alkyl, optionally substituted aryl and a side chain of an amino acid; R³ is a member selected from the group consisting of hydrogen, halogen, hydroxy, alkoxy, mercapto, hydrazino, amino and NR⁴R⁵, wherein R⁴ and R⁵ are independently hydrogen or alkyl; n is 4 to 80; m is 2 to 6; p is 1 to 4; and q is 0 or 1. It will be apparent to those of skill in the art that other polyamides can be used in the compounds of the present invention.

The term "diacylglycerol" refers to a compound having 2 fatty acyl chains, R¹ and R², both of which have independently between 2 and 30 carbons bonded to the 1- and 2-position of glycerol by ester linkages. The acyl groups can be saturated or have varying degrees of unsaturation. Suitable acyl groups include, but are not limited to, lauryl (C₁₂), myristyl (C₁₄), palmityl (C₁₆), stearyl (C₁₈), and icosyl (C₂₀). In preferred embodiments, R¹ and R² are the same, i.e., R¹ and R² are both myristyl (i.e., dimyristyl), R¹ and R² are both stearyl (i.e., distearyl), etc. Diacylglycerols have the following general formula:

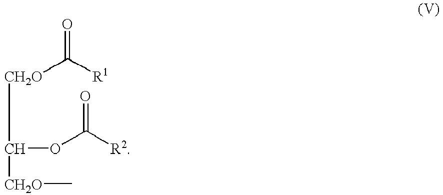

The term "dialkyloxypropyl" refers to a compound having 2 alkyl chains, R¹ and R², both of which have independently between 2 and 30 carbons. The alkyl groups can be saturated or have varying degrees of unsaturation. Dialkyloxypropyls have the following general formula:

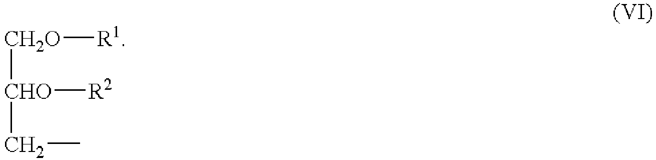

In a preferred embodiment, the PEG-lipid is a PEG-DAA conjugate having the following formula: 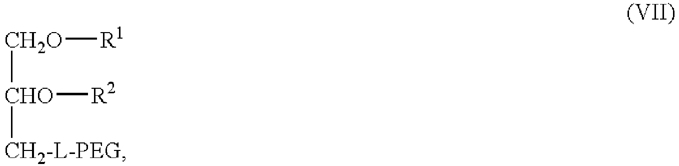 wherein R¹ and R² are independently selected and are long-chain alkyl groups having from about 10 to about 22 carbon atoms; PEG is a polyethyleneglycol; and L is a non-ester containing linker moiety or an ester containing linker moiety as described above. The long-chain alkyl groups can be saturated or unsaturated. Suitable alkyl groups include, but are not limited to, lauryl (C₁₂), myristyl (C₁₄), palmityl (C₁₆), stearyl (C₁₈), and icosyl (C₂₀). In preferred embodiments, R¹ and R² are the same, i.e., R¹ and R² are both myristyl (i.e., dimyristyl), R¹ and R² are both stearyl (i.e., distearyl), etc.

In Formula VII above, the PEG has an average molecular weight ranging from about 550 daltons to about 10,000 daltons. In certain instances, the PEG has an average molecular weight of from about 500 daltons to about 5,000 daltons (e.g., from about 1,000 daltons to about 5,000 daltons, from about 1,500 daltons to about 3,000 daltons, from about 750 daltons to about 3,000 daltons, from about 750 daltons to about 2,000 daltons, etc.). In preferred embodiments, the PEG has an average molecular weight of about 2,000 daltons or about 750 daltons. The PEG can be optionally substituted with alkyl, alkoxy, acyl, or aryl. In certain embodiments, the terminal hydroxyl group is substituted with a methoxy or methyl group.

In a preferred embodiment, "L" is a non-ester containing linker moiety. Suitable non-ester containing linkers include, but are not limited to, an amido linker moiety, an amino linker moiety, a carbonyl linker moiety, a carbamate linker moiety, a urea linker moiety, an ether linker moiety, a disulphide linker moiety, a succinamidyl linker moiety, and combinations thereof. In a preferred embodiment, the non-ester containing linker moiety is a carbamate linker moiety (i.e., a PEG-C-DAA conjugate). In another preferred embodiment, the non-ester containing linker moiety is an amido linker moiety (i.e., a PEG-A-DAA conjugate). In yet another preferred embodiment, the non-ester containing linker moiety is a succinamidyl linker moiety (i.e., a PEGS-DAA conjugate).

In particular embodiments, the PEG-lipid conjugate is selected from:

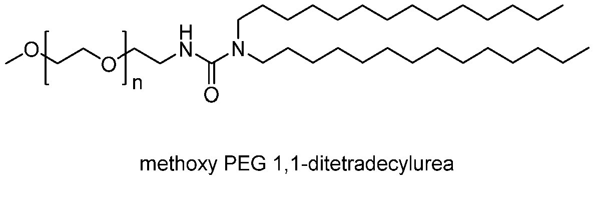

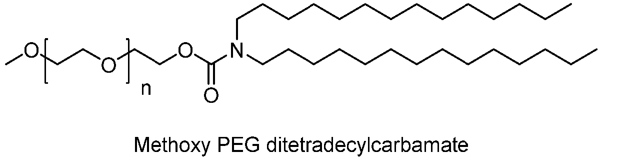

In one embodiment, n is selected so that the resulting polymer chain has a molecular weight of about 2000.

The PEG-DAA conjugates are synthesized using standard techniques and reagents known to those of skill in the art. It will be recognized that the PEG-DAA conjugates will contain various amide, amine, ether, thio, carbamate, and urea linkages. Those of skill in the art will recognize that methods and reagents for forming these bonds are well known and readily available. See, e.g., March, ADVANCED ORGANIC CHEMISTRY (Wiley 1992); Larock, COMPREHENSIVE ORGANIC TRANSFORMATIONS (VCH 1989); and Furniss, VOGEL'S TEXTBOOK OF PRACTICAL ORGANIC CHEMISTRY, 5th ed. (Longman 1989). It will also be appreciated that any functional groups present may require protection and deprotection at different points in the synthesis of the PEG-DAA conjugates. Those of skill in the art will recognize that such techniques are well known. See, e.g., Green and Wuts, PROTECTIVE GROUPS IN ORGANIC SYNTHESIS (Wiley 1991).

Preferably, the PEG-DAA conjugate is a dilauryloxypropyl (C₁₂)-PEG conjugate, dimyristyloxypropyl (C₁₄)-PEG conjugate, a dipalmityloxypropyl (C₁₆)-PEG conjugate, or a distearyloxypropyl (C₁₈)-PEG conjugate. Those of skill in the art will readily appreciate that other dialkyloxypropyls can be used in the PEG-DAA conjugates of the present invention.

In addition to the foregoing, it will be readily apparent to those of skill in the art that other hydrophilic polymers can be used in place of PEG. Examples of suitable polymers that can be used in place of PEG include, but are not limited to, polyvinylpyrrolidone, polymethyloxazoline, polyethyloxazoline, polyhydroxypropyl methacrylamide, polymethacrylamide and polydimethylacrylamide, polylactic acid, polyglycolic acid, and derivatized celluloses such as hydroxymethylcellulose or hydroxyethylcellulose.

The charges on the polycationic moieties can be either distributed around the entire particle moiety, or alternatively, they can be a discrete concentration of charge density in one particular area of the particle moiety e.g., a charge spike. If the charge density is distributed on the particle, the charge density can be equally distributed or unequally distributed. All variations of charge distribution of the polycationic moiety are encompassed by the present invention.

In certain instances, the polycationic moiety can have a ligand attached, such as a targeting ligand or a chelating moiety for complexing calcium. Preferably, after the ligand is attached, the cationic moiety maintains a positive charge. In certain instances, the ligand that is attached has a positive charge. Suitable ligands include, but are not limited to, a compound or device with a reactive functional group and include lipids, amphipathic lipids, carrier compounds, bioaffinity compounds, biomaterials, biopolymers, biomedical devices, analytically detectable compounds, therapeutically active compounds, enzymes, peptides, proteins, antibodies, immune stimulators, radiolabels, fluorogens, biotin, drugs, haptens, DNA, RNA, polysaccharides, liposomes, virosomes, micelles, immunoglobulins, functional groups, other targeting moieties, or toxins.

The lipid conjugate (e.g., PEG-lipid) typically comprises from about 0.1 mol % to about 10 mol %, from about 0.5 mol % to about 10 mol %, from about 1 mol % to about 10 mol %, from about 2 mol % to about 10 mol %, from about 2 mol % to about 8 mol %, from about 2 mol % to about 5 mol %, from about 2 mol % to about 4 mol %, from about 0.6 mol % to about 1.9 mol %, from about 0.7 mol % to about 1.8 mol %, from about 0.8 mol % to about 1.7 mol %, from about 0.9 mol % to about 1.6 mol %, from about 0.9 mol % to about 1.8 mol %, from about 1 mol % to about 1.8 mol %, from about 1 mol % to about 1.7 mol %, from about 1.2 mol % to about 1.8 mol %, from about 1.2 mol % to about 1.7 mol %, from about 1.3 mol % to about 1.6 mol %, or from about 1.4 mol % to about 1.5 mol % of the total lipid present in the particle.

One of ordinary skill in the art will appreciate that the concentration of the lipid conjugate can be varied depending on the lipid conjugate employed and the rate at which the nucleic acid-lipid particle is to become fusogenic.

By controlling the composition and concentration of the lipid conjugate, one can control the rate at which the lipid conjugate exchanges out of the nucleic acid-lipid particle and, in turn, the rate at which the nucleic acid-lipid particle becomes fusogenic. For instance, when a PEG-phosphatidylethanolamine conjugate or a PEG-ceramide conjugate is used as the lipid conjugate, the rate at which the nucleic acid-lipid particle becomes fusogenic can be varied, for example, by varying the concentration of the lipid conjugate, by varying the molecular weight of the PEG, or by varying the chain length and degree of saturation of the acyl chain groups on the phosphatidylethanolamine or the ceramide. In addition, other variables including, for example, pH, temperature, ionic strength, etc. can be used to vary and/or control the rate at which the nucleic acid-lipid particle becomes fusogenic. Other methods which can be used to control the rate at which the nucleic acid-lipid particle becomes fusogenic will become apparent to those of skill in the art upon reading this disclosure.

### Preparation of Lipid Particles

The lipid particles of the present invention, e.g., LNP, in which an active agent or therapeutic agent such as a nucleic acid molecule is encapsulated in a lipid bilayer and is protected from degradation, can be formed by any method known in the art including, but not limited to, a continuous mixing method or a direct dilution process.

In preferred embodiments, the non-cationic lipids are egg sphingomyelin (ESM), distearoylphosphatidylcholine (DSPC), dioleoylphosphatidylcholine (DOPC), 1-palmitoyl-2-oleoyl-phosphatidylcholine (POPC), dipalmitoyl-phosphatidylcholine (DPPC), monomethyl-phosphatidylethanolamine, dimethyl-phosphatidylethanolamine, 14:0 PE (1,2-dimyristoyl-phosphatidylethanolamine (DMPE)), 16:0 PE (1,2-dipalmitoyl-phosphatidylethanolamine (DPPE)), 18:0 PE (1,2-distearoyl-phosphatidylethanolamine (DSPE)), 18:1 PE (1,2-dioleoylphosphatidylethanolamine (DOPE)), 18:1 trans PE (1,2-dielaidoyl-phosphatidylethanolamine (DEPE)), 18:0-18:1 PE (1-stearoyl-2-oleoyl-phosphatidylethanolamine (SOPE)), 16:0-18:1 PE (1-palmitoyl-2-oleoyl-phosphatidylethanolamine (POPE)), polyethylene glycol-based polymers (e.g., PEG 2000, PEG 5000, PEG-modified diacylglycerols, or PEG-modified dialkyloxypropyls), cholesterol, or combinations thereof.

In certain embodiments, the present invention provides for LNP produced via a continuous mixing method, e.g., a process that includes providing an aqueous solution comprising a nucleic acid, such as an interfering RNA or mRNA, in a first reservoir, providing an organic lipid solution in a second reservoir, and mixing the aqueous solution with the organic lipid solution such that the organic lipid solution mixes with the aqueous solution so as to substantially instantaneously produce a liposome encapsulating the nucleic acid (e.g., interfering RNA or mRNA). This process and the apparatus for carrying this process are described in detail in U.S. Patent Publication No. 20040142025.

The action of continuously introducing lipid and buffer solutions into a mixing environment, such as in a mixing chamber, causes a continuous dilution of the lipid solution with the buffer solution, thereby producing a liposome substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution (i.e., aqueous solution) to produce a nucleic acid-lipid particle.

The LNP formed using the continuous mixing method typically have a size of from about 40 nm to about 150 nm, from about 40 nm to about 80 nm, from about 40 nm to about 60 nm, from about 50 nm to about 60 nm, from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

In another embodiment, the present invention provides for LNP produced via a direct dilution process that includes forming a liposome solution and immediately and directly introducing the liposome solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of liposome solution introduced thereto. As a non-limiting example, a liposome solution in 45% ethanol when introduced into the collection vessel containing an equal volume of dilution buffer will advantageously yield smaller particles.

In yet another embodiment, the present invention provides for LNP produced via a direct dilution process in which a third reservoir containing dilution buffer is fluidly coupled to a second mixing region. In this embodiment, the liposome solution formed in a first mixing region is immediately and directly mixed with dilution buffer in the second mixing region. In preferred aspects, the second mixing region includes a T-connector arranged so that the liposome solution and the dilution buffer flows meet as opposing 180° flows; however, connectors providing shallower angles can be used, e.g., from about 27° to about 180°. A pump mechanism delivers a controllable flow of buffer to the second mixing region. In one aspect, the flow rate of dilution buffer provided to the second mixing region is controlled to be substantially equal to the flow rate of liposome solution introduced thereto from the first mixing region. In another aspect, the flow rate of dilution buffer provided to the second mixing region is controlled to be substantially different to the flow rate of liposome solution introduced thereto from the first mixing region. This embodiment advantageously allows for more control of the flow of dilution buffer mixing with the liposome solution in the second mixing region, and therefore also the concentration of liposome solution in buffer throughout the second mixing process. Such control of the dilution buffer flow rate advantageously allows for small particle size formation at reduced concentrations.

These processes and the apparatuses for carrying out these direct dilution processes are described in detail in U.S. Patent Publication No. 20070042031.

The LNP formed using the direct dilution process typically have a size of from about 40 nm to about 150 nm, from about 40 nm to about 80 nm, from about 40 nm to about 60 nm, from about 50 nm to about 60 nm, from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

If needed, the lipid particles of the invention (e.g., LNP) can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles, is described in U.S. Pat. No. 4,737,323. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In some embodiments, the nucleic acids in the LNP are precondensed as described in, e.g., U.S. patent application Ser. No. 09/744,103.

In other embodiments, the methods will further comprise adding non-lipid polycations which are useful to effect the lipofection of cells using the present compositions. Examples of suitable non-lipid polycations include, hexadimethrine bromide (sold under the brandname POLYBRENE^{®}, from Aldrich Chemical Co., Milwaukee, Wis., USA) or other salts of hexadimethrine. Other suitable polycations include, for example, salts of poly-L-ornithine, poly-L-arginine, poly-L-lysine, poly-D-lysine, polyallylamine, and polyethyleneimine. Addition of these salts is preferably after the particles have been formed.

In some embodiments, the nucleic acid to lipid ratios (mass/mass ratios) in a formed LNP will range from about 0.01 to about 0.2, from about 0.02 to about 0.1, from about 0.03 to about 0.1, or from about 0.01 to about 0.08. The ratio of the starting materials also falls within this range. In other embodiments, the LNP preparation uses about 400 µg nucleic acid per 10 mg total lipid or a nucleic acid to lipid mass ratio of about 0.01 to about 0.08 and, more preferably, about 0.04, which corresponds to 1.25 mg of total lipid per 50 µg of nucleic acid. In other preferred embodiments, the particle has a nucleic acid:lipid mass ratio of about 0.08.

In other embodiments, the lipid to nucleic acid ratios (mass/mass ratios) in a formed LNP will range from about 1 (1:1) to about 100 (100:1), from about 5 (5:1) to about 100 (100:1), from about 1 (1:1) to about 50 (50:1), from about 2 (2:1) to about 50 (50:1), from about 3 (3:1) to about 50 (50:1), from about 4 (4:1) to about 50 (50:1), from about 5 (5:1) to about 50 (50:1), from about 1 (1:1) to about 25 (25:1), from about 2 (2:1) to about 25 (25:1), from about 3 (3:1) to about 25 (25:1), from about 4 (4:1) to about 25 (25:1), from about 5 (5:1) to about 25 (25:1), from about 5 (5:1) to about 20 (20:1), from about 5 (5:1) to about 15 (15:1), from about 5 (5:1) to about 10 (10: 1), about 5 (5:1), 6 (6:1), 7 (7:1), 8 (8:1), 9 (9:1), (10:1), 11 (11:1), 12 (12:1), 13 (13:1), 14 (14:1), 15 (15:1), 16 (16:1), 17 (17:1), 18 (18:1), 19 (19:1), 20 (20:1), 21 (21:1), 22 (22:1), 23 (23:1), 24 (24:1), 25 (25:1), 26 (26:1), 27 (27:1), 28 (28:1), 29 (29:1) or 30 (30:1). The ratio of the starting materials also typically falls within this range.

As previously discussed, the conjugated lipid may further include a CPL. A variety of general methods for making LNP -CPLs (CPL-containing LNP) are discussed herein. Two general techniques include "post-insertion" technique, that is, insertion of a CPL into, for example, a pre-formed LNP, and the "standard" technique, wherein the CPL is included in the lipid mixture during, for example, the LNP formation steps. The post-insertion technique results in LNP having CPLs mainly in the external face of the LNP bilayer membrane, whereas standard techniques provide LNP having CPLs on both internal and external faces. The method is especially useful for vesicles made from phospholipids (which can contain cholesterol) and also for vesicles containing PEG-lipids (such as PEG-DAAs and PEG-DAGs). Methods of making LNP -CPL, are taught, for example, in U.S. Pat. Nos. 5,705,385; 6,586,410; 5,981,501; 6,534,484; and 6,852,334; U.S. Patent Publication No. 20020072121; and PCT Publication No. WO 00/62813.

Other methods for generating LNP may be found, for example, in U.S. Patent No. 9,005,654 and PCT Publication No. WO 2007/012191.

### Kits

The present invention also provides lipid particles (e.g., LNP) in kit form. The kit may comprise a container which is compartmentalized for holding the various elements of the lipid particles (e.g., the active agents or therapeutic agents such as nucleic acids and the individual lipid components of the particles). In some embodiments, the kit may further comprise an endosomal membrane destabilizer (e.g., calcium ions). The kit typically contains the lipid particle compositions of the present invention, preferably in dehydrated form, with instructions for their rehydration and administration.

As explained herein, the lipid particles of the invention (e.g., LNP) can be tailored to preferentially target particular tissues, organs, or tumors of interest. In certain instances, preferential targeting of lipid particles such as LNP may be carried out by controlling the composition of the particle itself. For instance, as set forth in Example 11, it has been found that the 1:57 PEG-cDSA LNP formulation can be used to preferentially target tumors outside of the liver, whereas the 1:57 PEG-cDMA LNP formulation can be used to preferentially target the liver (including liver tumors).

In certain other instances, it may be desirable to have a targeting moiety attached to the surface of the lipid particle to further enhance the targeting of the particle. Methods of attaching targeting moieties (e.g., antibodies, proteins, etc.) to lipids (such as those used in the present particles) are known to those of skill in the art.

### Administration of Lipid Particles

Once formed, the lipid particles of the invention (e.g., LNP) are useful for the introduction of active agents or therapeutic agents (e.g., nucleic acids, such as interfering RNA or mRNA) into cells. Accordingly, the present invention also finds use in methods for introducing an active agent or therapeutic agent such as a nucleic acid (e.g., interfering RNA or mRNA) into a cell. The methods are carried out in vitro or in vivo by first forming the particles as described above and then contacting the particles with the cells for a period of time sufficient for delivery of the active agent or therapeutic agent to the cells to occur.

The lipid particles of the invention (e.g., LNP) can be adsorbed to almost any cell type with which they are mixed or contacted. Once adsorbed, the particles can either be endocytosed by a portion of the cells, exchange lipids with cell membranes, or fuse with the cells. Transfer or incorporation of the active agent or therapeutic agent (e.g., nucleic acid) portion of the particle can take place via any one of these pathways. In particular, when fusion takes place, the particle membrane is integrated into the cell membrane and the contents of the particle combine with the intracellular fluid.

The lipid particles of the invention (e.g., LNP) can be administered either alone or in a mixture with a pharmaceutically-acceptable carrier (e.g., physiological saline or phosphate buffer) selected in accordance with the route of administration and standard pharmaceutical practice. Generally, normal buffered saline (e.g., 135-150 mM NaCl) will be employed as the pharmaceutically-acceptable carrier. Other suitable carriers include, e.g., water, buffered water, 0.4% saline, 0.3% glycine, and the like, including glycoproteins for enhanced stability, such as albumin, lipoprotein, globulin, etc. Additional suitable carriers are described in, e.g., REMINGTON'S PHARMACEUTICAL SCIENCES, Mack Publishing Company, Philadelphia, Pa., 17th ed. (1985). As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The phrase "pharmaceutically-acceptable" refers to molecular entities and compositions that do not produce an allergic or similar untoward reaction when administered to a human.

The pharmaceutically-acceptable carrier is generally added following particle formation. Thus, after the particle is formed, the particle can be diluted into pharmaceutically-acceptable carriers such as normal buffered saline.

The concentration of particles in the pharmaceutical formulations can vary widely, i.e., from less than about 0.05%, usually at or at least about 2 to 5%, to as much as about 10 to 90% by weight, and will be selected primarily by fluid volumes, viscosities, etc., in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration.

The pharmaceutical compositions of the present invention may be sterilized by conventional, well-known sterilization techniques. Aqueous solutions can be packaged for use or filtered under aseptic conditions and lyophilized, the lyophilized preparation being combined with a sterile aqueous solution prior to administration. The compositions can contain pharmaceutically-acceptable auxiliary substances as required to approximate physiological conditions, such as pH adjusting and buffering agents, tonicity adjusting agents and the like, for example, sodium acetate, sodium lactate, sodium chloride, potassium chloride, and calcium chloride. Additionally, the particle suspension may include lipid-protective agents which protect lipids against free-radical and lipid-peroxidative damages on storage. Lipophilic free-radical quenchers, such as alphatocopherol and water-soluble iron-specific chelators, such as ferrioxamine, are suitable.

### In Vivo Administration

Systemic delivery for in vivo therapy, e.g., delivery of a therapeutic nucleic acid to a distal target cell via body systems such as the circulation, has been achieved using nucleic acid-lipid particles such as those described in PCT Publication Nos. WO 05/007196, WO 05/121348, WO 05/120152, and WO 04/002453. The present invention also provides fully encapsulated lipid particles that protect the nucleic acid from nuclease degradation in serum, are nonimmunogenic, are small in size, and are suitable for repeat dosing.

For in vivo administration, administration can be in any manner known in the art, e.g., by injection, oral administration, inhalation (e.g., intransal or intratracheal), transdermal application, or rectal administration. Administration can be accomplished via single or divided doses. The pharmaceutical compositions can be administered parenterally, i.e., intraarticularly, intravenously, intraperitoneally, subcutaneously, or intramuscularly. In some embodiments, the pharmaceutical compositions are administered intravenously or intraperitoneally by a bolus injection (see, e.g., U.S. Pat. No. 5,286,634). Intracellular nucleic acid delivery has also been discussed in Straubringer et al., Methods Enzymol., 101:512 (1983); Mannino et al., Biotechniques, 6:682 (1988); Nicolau et al., Crit. Rev. Ther. Drug Carrier Syst., 6:239 (1989); and Behr, Acc. Chem. Res., 26:274 (1993). Still other methods of administering lipid-based therapeutics are described in, for example, U.S. Pat. Nos. 3,993,754; 4,145,410; 4,235,871; 4,224,179; 4,522,803; and 4,588,578. The lipid particles can be administered by direct injection at the site of disease or by injection at a site distal from the site of disease (see, e.g., Culver, HUMAN GENE THERAPY, MaryAnn Liebert, Inc., Publishers, New York. pp. 70-71 (1994)).

The compositions of the present invention, either alone or in combination with other suitable components, can be made into aerosol formulations (i.e., they can be "nebulized") to be administered via inhalation (e.g., intranasally or intratracheally) (see, Brigham et al., Am. J. Sci., 298:278 (1989)). Aerosol formulations can be placed into pressurized acceptable propellants, such as dichlorodifluoromethane, propane, nitrogen, and the like.

In certain embodiments, the pharmaceutical compositions may be delivered by intranasal sprays, inhalation, and/or other aerosol delivery vehicles. Methods for delivering nucleic acid compositions directly to the lungs via nasal aerosol sprays have been described, e.g., in U.S. Pat. Nos. 5,756,353 and 5,804,212. Likewise, the delivery of drugs using intranasal microparticle resins and lysophosphatidyl-glycerol compounds (U.S. Pat. No. 5,725,871) are also well-known in the pharmaceutical arts. Similarly, transmucosal drug delivery in the form of a polytetrafluoroetheylene support matrix is described in U.S. Pat. No. 5,780,045.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions are preferably administered, for example, by intravenous infusion, orally, topically, intraperitoneally, intravesically, or intrathecally.

Generally, when administered intravenously, the lipid particle formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, Pa., 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, etc. Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration.

In certain applications, the lipid particles disclosed herein may be delivered via oral administration to the individual. The particles may be incorporated with excipients and used in the form of ingestible tablets, buccal tablets, troches, capsules, pills, lozenges, elixirs, mouthwash, suspensions, oral sprays, syrups, wafers, and the like (see, e.g., U.S. Pat. Nos. 5,641,515, 5,580,579, and 5,792,451). These oral dosage forms may also contain the following: binders, gelatin; excipients, lubricants, and/or flavoring agents. When the unit dosage form is a capsule, it may contain, in addition to the materials described above, a liquid carrier. Various other materials may be present as coatings or to otherwise modify the physical form of the dosage unit. Of course, any material used in preparing any unit dosage form should be pharmaceutically pure and substantially non-toxic in the amounts employed.

Typically, these oral formulations may contain at least about 0.1% of the lipid particles or more, although the percentage of the particles may, of course, be varied and may conveniently be between about 1% or 2% and about 60% or 70% or more of the weight or volume of the total formulation. Naturally, the amount of particles in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

Formulations suitable for oral administration can consist of: (a) liquid solutions, such as an effective amount of a packaged therapeutic agent such as nucleic acid (e.g., interfering RNA or mRNA) suspended in diluents such as water, saline, or PEG 400; (b) capsules, sachets, or tablets, each containing a predetermined amount of a therapeutic agent such as nucleic acid (e.g., interfering RNA or mRNA), as liquids, solids, granules, or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise a therapeutic agent such as nucleic acid (e.g., interfering RNA or mRNA) in a flavor, e.g., sucrose, as well as pastilles comprising the therapeutic agent in an inert base, such as gelatin and glycerin or sucrose and acacia emulsions, gels, and the like containing, in addition to the therapeutic agent, carriers known in the art.

In another example of their use, lipid particles can be incorporated into a broad range of topical dosage forms. For instance, a suspension containing nucleic acid-lipid particles such as LNP can be formulated and administered as gels, oils, emulsions, topical creams, pastes, ointments, lotions, foams, mousses, and the like.

When preparing pharmaceutical preparations of the lipid particles of the invention, it is preferable to use quantities of the particles which have been purified to reduce or eliminate empty particles or particles with therapeutic agents such as nucleic acid associated with the external surface.

The methods may be practiced in a variety of hosts. Preferred hosts include mammalian species, such as primates (e.g., humans and chimpanzees as well as other nonhuman primates), canines, felines, equines, bovines, ovines, caprines, rodents (e.g., rats and mice), lagomorphs, and swine.

The amount of particles administered will depend upon the ratio of therapeutic agent (e.g., nucleic acid) to lipid, the particular therapeutic agent (e.g., nucleic acid) used, the disease or disorder being treated, the age, weight, and condition of the patient, and the judgment of the clinician, but will generally be between about 0.01 and about 50 mg per kilogram of body weight, preferably between about 0.1 and about 5 mg/kg of body weight, or about 10⁸-10¹⁰ particles per administration (e.g., injection).

### In Vitro Administration

For in vitro applications, the delivery of therapeutic agents such as nucleic acids (e.g., interfering RNA or mRNA) can be to any cell grown in culture, whether of plant or animal origin, vertebrate or invertebrate, and of any tissue or type. In preferred embodiments, the cells are animal cells, more preferably mammalian cells, and most preferably human cells.

Contact between the cells and the lipid particles, when carried out in vitro, takes place in a biologically compatible medium. The concentration of particles varies widely depending on the particular application, but is generally between about 1 µmol and about 10 mmol. Treatment of the cells with the lipid particles is generally carried out at physiological temperatures (about 37° C.) for periods of time of from about 1 to 48 hours, preferably of from about 2 to 4 hours.

In one group of preferred embodiments, a lipid particle suspension is added to 60-80% confluent plated cells having a cell density of from about 10³ to about 10⁵ cells/ml, more preferably about 2×10⁴ cells/ml. The concentration of the suspension added to the cells is preferably of from about 0.01 to 0.2 µg/ml, more preferably about 0.1 µg/ml.

Using an Endosomal Release Parameter (ERP) assay, the delivery efficiency of the LNP or other lipid particle of the invention can be optimized. An ERP assay is described in detail in U.S. Patent Publication No. 20030077829. More particularly, the purpose of an ERP assay is to distinguish the effect of various cationic lipids and helper lipid components of LNP based on their relative effect on binding/uptake or fusion with/destabilization of the endosomal membrane. This assay allows one to determine quantitatively how each component of the LNP or other lipid particle affects delivery efficiency, thereby optimizing the LNP or other lipid particle. Usually, an ERP assay measures expression of a reporter protein (e.g., luciferase, β-galactosidase, green fluorescent protein (GFP), etc.), and in some instances, a LNP formulation optimized for an expression plasmid will also be appropriate for encapsulating an interfering RNA or mRNA. In other instances, an ERP assay can be adapted to measure downregulation of transcription or translation of a target sequence in the presence or absence of an interfering RNA (e.g., siRNA). In other instances, an ERP assay can be adapted to measure the expression of a target protein in the presence or absence of an mRNA. By comparing the ERPs for each of the various LNP or other lipid particles, one can readily determine the optimized system, e.g., the LNP or other lipid particle that has the greatest uptake in the cell.

### Cells for Delivery of Lipid Particles

The compositions of the present invention are used to treat a wide variety of cell types, in vivo and in vitro. Suitable cells include, e.g., hematopoietic precursor (stem) cells, fibroblasts, keratinocytes, hepatocytes, endothelial cells, skeletal and smooth muscle cells, osteoblasts, neurons, quiescent lymphocytes, terminally differentiated cells, slow or noncycling primary cells, parenchymal cells, lymphoid cells, epithelial cells, bone cells, and the like. In preferred embodiments, an active agent or therapeutic agent such as one or more nucleic acid molecules (e.g, an interfering RNA (e.g., siRNA) or mRNA) is delivered to cancer cells such as, e.g., lung cancer cells, colon cancer cells, rectal cancer cells, anal cancer cells, bile duct cancer cells, small intestine cancer cells, stomach (gastric) cancer cells, esophageal cancer cells, gallbladder cancer cells, liver cancer cells, pancreatic cancer cells, appendix cancer cells, breast cancer cells, ovarian cancer cells, cervical cancer cells, prostate cancer cells, renal cancer cells, cancer cells of the central nervous system, glioblastoma tumor cells, skin cancer cells, lymphoma cells, choriocarcinoma tumor cells, head and neck cancer cells, osteogenic sarcoma tumor cells, and blood cancer cells.

In vivo delivery of lipid particles such as LNP encapsulating one or more nucleic acid molecules (e.g., interfering RNA (e.g., siRNA) or mRNA) is suited for targeting cells of any cell type. The methods and compositions can be employed with cells of a wide variety of vertebrates, including mammals, such as, e.g, canines, felines, equines, bovines, ovines, caprines, rodents (e.g., mice, rats, and guinea pigs), lagomorphs, swine, and primates (e.g. monkeys, chimpanzees, and humans).

To the extent that tissue culture of cells may be required, it is well-known in the art. For example, Freshney, Culture of Animal Cells, a Manual of Basic Technique, 3rd Ed., Wiley-Liss, New York (1994), Kuchler et al., Biochemical Methods in Cell Culture and Virology, Dowden, Hutchinson and Ross, Inc. (1977), and the references cited therein provide a general guide to the culture of cells. Cultured cell systems often will be in the form of monolayers of cells, although cell suspensions are also used.

### Detection of Lipid Particles

In some embodiments, the lipid particles of the present invention (e.g., LNP) are detectable in the subject at about 1, 2, 3, 4, 5, 6, 7, 8 or more hours. In other embodiments, the lipid particles of the present invention (e.g., LNP) are detectable in the subject at about 8, 12, 24, 48, 60, 72, or 96 hours, or about 6, 8, 10, 12, 14, 16, 18, 19, 22, 24, 25, or 28 days after administration of the particles. The presence of the particles can be detected in the cells, tissues, or other biological samples from the subject. The particles may be detected, e.g., by direct detection of the particles, detection of a therapeutic nucleic acid, such as an interfering RNA (e.g., siRNA) sequence or mRNA sequence, detection of the target sequence of interest (i.e., by detecting expression or reduced expression of the sequence of interest), or a combination thereof.

### Detection of Particles

Lipid particles of the invention such as LNP can be detected using any method known in the art. For example, a label can be coupled directly or indirectly to a component of the lipid particle using methods well-known in the art. A wide variety of labels can be used, with the choice of label depending on sensitivity required, ease of conjugation with the lipid particle component, stability requirements, and available instrumentation and disposal provisions. Suitable labels include, but are not limited to, spectral labels such as fluorescent dyes (e.g., fluorescein and derivatives, such as fluorescein isothiocyanate (FITC) and Oregon Green^{™}; rhodamine and derivatives such Texas red, tetrarhodimine isothiocynate (TRITC), etc., digoxigenin, biotin, phycoerythrin, AMCA, CyDyes^{™}, and the like; radiolabels such as ³H, ¹²⁵I, ³⁵S, ¹⁴C, ³²P, ³³P, etc.; enzymes such as horse radish peroxidase, alkaline phosphatase, etc.; spectral colorimetric labels such as colloidal gold or colored glass or plastic beads such as polystyrene, polypropylene, latex, etc. The label can be detected using any means known in the art.

### Detection of Nucleic Acids

Nucleic acids (e.g., interfering RNA or mRNA) are detected and quantified herein by any of a number of means well-known to those of skill in the art. The detection of nucleic acids may proceed by well-known methods such as Southern analysis, Northern analysis, gel electrophoresis, PCR, radiolabeling, scintillation counting, and affinity chromatography. Additional analytic biochemical methods such as spectrophotometry, radiography, electrophoresis, capillary electrophoresis, high performance liquid chromatography (HPLC), thin layer chromatography (TLC), and hyperdiffusion chromatography may also be employed.

The selection of a nucleic acid hybridization format is not critical. A variety of nucleic acid hybridization formats are known to those skilled in the art. For example, common formats include sandwich assays and competition or displacement assays. Hybridization techniques are generally described in, e.g., "Nucleic Acid Hybridization, A Practical Approach," Eds. Hames and Higgins, IRL Press (1985).

The sensitivity of the hybridization assays may be enhanced through use of a nucleic acid amplification system which multiplies the target nucleic acid being detected. In vitro amplification techniques suitable for amplifying sequences for use as molecular probes or for generating nucleic acid fragments for subsequent subcloning are known. Examples of techniques sufficient to direct persons of skill through such in vitro amplification methods, including the polymerase chain reaction (PCR) the ligase chain reaction (LCR), Qβ-replicase amplification and other RNA polymerase mediated techniques (e.g., NASBA^{™}) are found in Sambrook et al., In Molecular Cloning: A Laboratory Manual, Cold Spring Harbor Laboratory Press (2000); and Ausubel et al., SHORT PROTOCOLS IN MOLECULAR BIOLOGY, eds., Current Protocols, Greene Publishing Associates, Inc. and John Wiley & Sons, Inc. (2002); as well as U.S. Pat. No. 4,683,202; PCR Protocols, A Guide to Methods and Applications (Innis et al. eds.) Academic Press Inc. San Diego, Calif. (1990); Arnheim & Levinson (Oct. 1, 1990), C&EN 36; The Journal Of NIH Research, 3:81 (1991); Kwoh et al., Proc. Natl. Acad. Sci. USA, 86:1173 (1989); Guatelli et al., Proc. Natl. Acad. Sci. USA, 87:1874 (1990); Lomell et al., J. Clin. Chem., 35:1826 (1989); Landegren et al., Science, 241:1077 (1988); Van Brunt, Biotechnology, 8:291 (1990); Wu and Wallace, Gene, 4:560 (1989); Barringer et al., Gene, 89:117 (1990); and Sooknanan and Malek, Biotechnology, 13:563 (1995). Improved methods of cloning in vitro amplified nucleic acids are described in U.S. Pat. No. 5,426,039. Other methods described in the art are the nucleic acid sequence based amplification (NASBA^{™}, Cangene, Mississauga, Ontario) and Qβ-replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation.

Nucleic acids for use as probes, e.g., in in vitro amplification methods, for use as gene probes, or as inhibitor components are typically synthesized chemically according to the solid phase phosphoramidite triester method described by Beaucage et al., Tetrahedron Letts., 22:1859 1862 (1981), e.g., using an automated synthesizer, as described in Needham VanDevanter et al., Nucleic Acids Res., 12:6159 (1984). Purification of polynucleotides, where necessary, is typically performed by either native acrylamide gel electrophoresis or by anion exchange HPLC as described in Pearson et al., J. Chrom., 255:137 149 (1983). The sequence of the synthetic polynucleotides can be verified using the chemical degradation method of Maxam and Gilbert (1980) in Grossman and Moldave (eds.) Academic Press, New York, Methods in Enzymology, 65:499.

An alternative means for determining the level of transcription is in situ hybridization. In situ hybridization assays are well-known and are generally described in Angerer et al., Methods Enzymol., 152:649 (1987). In an in situ hybridization assay, cells are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters.

### Ionizable Lipids

Ionizable cationic lipids are typically the major lipid component in lipid nanoparticles (LNP). They are designed to be charge-neutral at standard physiological pH (~7) but acquire a positive charge at lower (acidic) pH. This helps with nucleic acid payload encapsulation during the LNP formation, and intracellular delivery (the endosomal fusion step requires a positive charge on the ionizable lipid).

Ionizable lipids such as MC3 (used in the FDA-approved siRNA-LNP product Onpattro) and 3D-P-DMA have demonstrated excellent activity and tolerability in a variety of applications. However, they are not rapidly metabolized, and take several weeks to clear from target tissues. This makes them less appealing for applications that require multiple doses, especially with short dose intervals and/or chronic indications.

One way to achieve a more favorable profile is the inclusion of carboxylic ester groups in the hydrophobic domain of the lipid. Ester groups are subject to enzymatic breakdown in the body, and this has been reported to improve lipid clearance in target tissues (Maier et al 2013). However, the chemical structure immediately surrounding the esters must be considered, as this affects how effective the strategy is. ALC-0315, the ionizable lipid in BNT162b2 (also known as the Pfizer COVID vaccine), has carboxylic esters in its hydrophobic domain, but was found to metabolize at a similar rate to MC3 [ref: Comirnaty Assessment Report, European Medicines Agency]. Further, the esters must be incorporated while maintaining a favorable activity and tolerability profile.

To this end, a series of ionizable cationic lipids incorporating ester functionality has been designed, synthesized, and evaluated in the following ways:
- Formulability (particle size, polydispersity, payload encapsulation, pKa)
- Potency (mouse liver expression model, delivering luciferase-encoding and OTC mRNA)
- Tolerability (mouse immunestimulation model, cytokine readout)
- Lipid clearance (mouse model - clearance from target tissue (liver))

The present invention will be described in greater detail by way of specific examples. The following examples are offered for illustrative purposes and are not intended to limit the invention in any manner. Those of skill in the art will readily recognize a variety of noncritical parameters, which can be changed or modified to yield essentially the same results.

### EXAMPLES

### Preparatory Example 1. Synthesis of heptadecan-9-ylidenehydrazine

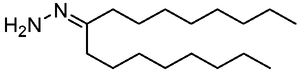

### Step 1. Synthesis of 9-heptadecanol

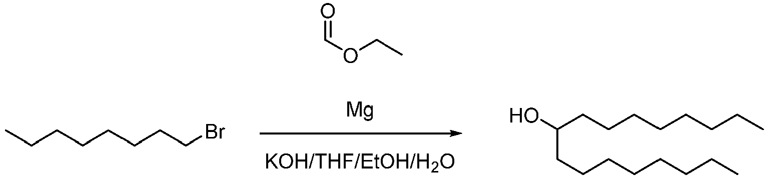

A Grignard reagent was prepared from 1-bromooctane (6 g, 31 mmol) and magnesium (808 mg, 33 mmol) in THF with stirring at 45°C for 2 hr. The reaction was cooled to 10°C and ethyl formate (2.6 mL, 32 mmol) in THF (5 mL) added dropwise. H₂O (3 mL) and 6M HCl (8 mL) were added to dissolve residual magnesium. The reaction was partitioned between hexane and H₂O. The organics were washed with brine, dried (MgSO₄), filtered and concentrated *in-vacuo.* The residue was stirred in EtOH with KOH (1.6 g) solution in water (3 mL). The ethanol was removed *in-vacuo* and the residue partitioned between 6M HCl and hexane. The organics were washed with H₂O and brine, dried (MgSO₄), filtered and concentrated *in-vacuo.* The crude product was purified by automated flash chromatography (0-20% EtOAc/Hex) to give 9-heptadecanol (3.2 g, 81.2 %).

### Step 2. Synthesis of 9-heptadecanone

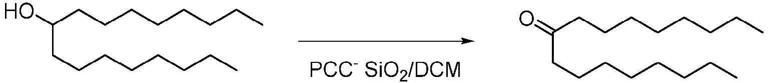

9-Heptadecanol (3.2 g, 12.6 mmol) and PCC-SiO₂ (1:1) (16.3 g, 38 mmol) were stirred in DCM (100 mL) at RT for 16 hr. The reaction was filtered through silica eluting with EtOAc. The organics were concentrated *in-vacuo* and purified by automated flash chromatography (0-10% EtOAc/Hex) to give 9-heptadecanone (3.2 g, 99.6 %).

### Step 3. Synthesis of N'-(heptadecan-9-ylidene)tert-butoxycarbohydrazide

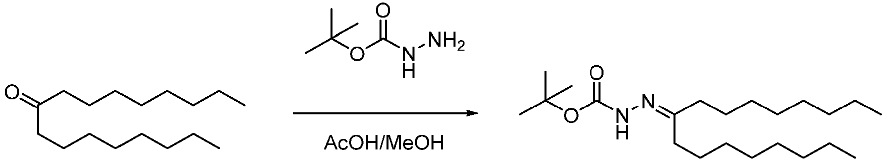

tert-Butoxycarbohydrazide (195 mg, 1.47 mmol), 9-heptadecanone (750 mg, 2.9 mmol) and AcOH (cat) were stirred in MeOH (10 mL) at reflux for 15 hr. The reaction was concentrated *in-vacuo* and purified by automated flash chromatography (0-20% EtOAc/Hex) to give N'-(heptadecan-9-ylidene)tert-butoxycarbohydrazide (320 mg, 58.9 %).

### Step 4. Synthesis of heptadecan-9-ylidenehydrazine

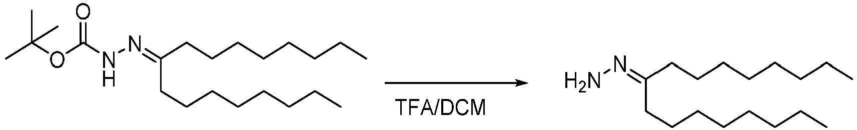

N'-(Heptadecan-9-ylidene)tert-butoxycarbohydrazide (320 mg, 0.9 mmol) was stirred in 25 % TFA in DCM (40 mL) at RT until the reaction was complete. The solvent was removed *in-vacuo* to give heptadecan-9-ylidenehydrazine (231 mg, 99.1 %) which was used without further purification.

### Preparatory Example 2. Synthesis of ((6Z,15Z)-henicosa-6,15-dien-11-ylidene)hydrazine

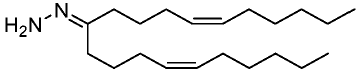

((6Z,15Z)-Henicosa-6,15-dien-11-ylidene)hydrazine was prepared using analogous methodology as described above.

### Preparatory Example 3. Synthesis of Heptadecan-9-ylhydrazine

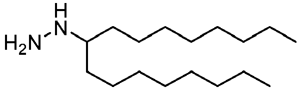

### Step 1. Synthesis of give N'-(heptadecan-9-yl)tert-butoxycarbohydrazide

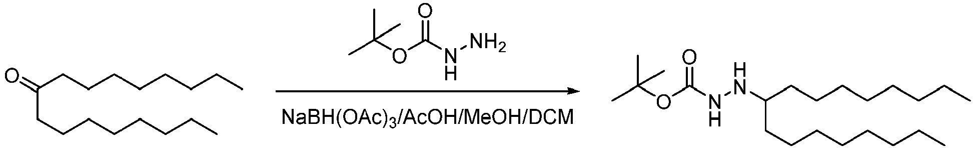

tert-Butoxycarbohydrazide (412 mg, 3.1mmol), 9-heptadecanone (794 mg, 3.1 mmol), AcOH (0.75 mL, 3.2 mmol) and NaBH(OAc)₃ (2.0 g, 9.4 mmol) were stirred in DCM (20 mL) at RT for 2 days and at 40°C for a further 20 hr. The reaction was washed with sat NaHCO₃ and brine, dried (MgSO₄), filtered and concentrated *in-vacuo.* The residue was purified by automated flash chromatography (0-12% EtOAc/Hex) to give N'-(heptadecan-9-yl)tert-butoxycarbohydrazide (700 mg, 60.5 %).

### Step 2 Synthesis of heptadecan-9-ylhydrazine

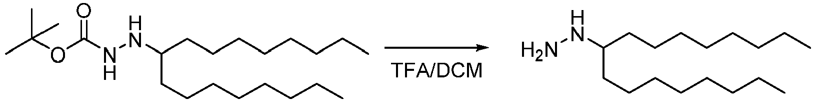

N'-(heptadecan-9-yl)tert-butoxycarbohydrazide (700 mg, 1.9 mmol) was stirred with 25% TFA in DCM (10 mL) at RT for 1.5 hr. The reaction was concentrated *in-vacuo* to give heptadecan-9-ylhydrazine (500 mg, 97.9 %) which was used without purification.

### Preparatory Example 4. Synthesis of ((6Z,15Z)-Henicosa-6,15-dien-11-yl)hydrazine

((6Z,15Z)-Henicosa-6,15-dien-11-yl)hydrazine was prepared using analogous methodology as described above

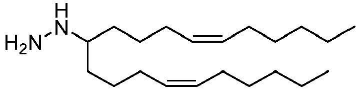

### Preparatory Example 5. Synthesis of 1,1-dioctylhydrazine

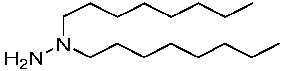

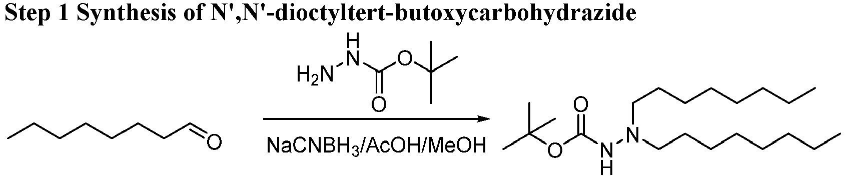

Octanal (2.4 mL, 15.0 mmol), tert-butoxycarbohydrazide (1 g, 7.6 mmol) and AcOH (7.6 mmol) were stirred in dry MeOH under a nitrogen atmosphere at 75°C for 1 hr. After cooling to RT NaBH3CN (1.9 g, 30.3 mmol) was added and the mixture stirred at RT for 15 hr. The solvent was removed in-vacuo, the residue taken up in EtOAc, washed with H2O and brine, dried (MgSO4), filtered and concentrated. The crude material was purified by automated flash chromatography (0-10% EtOAc/Hex) to give N',N'-dioctyltert-butoxycarbohydrazide (2.0 g, 74.3 %).

### Step 2 Synthesis of 1,1-dioctylhydrazine

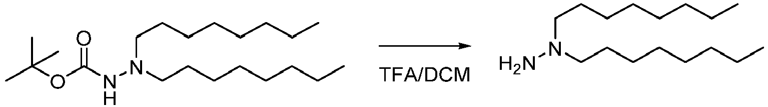

N',N'-Dioctyltert-butoxycarbohydrazide (1.1 g, 3.0 mmol) was stirred in 30% TFA in DCM (20 mL) at 0°C until complete allowing to warm to RT. The solvent was removed *in-vacuo* to give 1,1-dioctylhydrazine (782 mg, Quant) which was used without purification.

### Preparatory Example 6. Synthesis of 1,1-di((Z)-Dec-4-en-1-yl)hydrazine

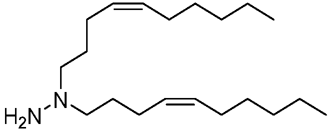

### 1,1-di((Z)-Dec-4-en-1-yl)hydrazine was prepared using analogous methodology

### Preparatory Example 7. General Method (a) For the Synthesis of Branched Alkyl Alcohols - Exemplified by the synthesis of 5-octyltridec-4-en-1-ol

### Step 1. Synthesis of 9-(4-bromobutyl)heptadecan-9-ol

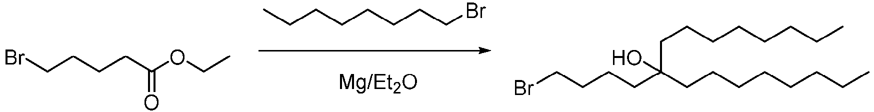

A Grignard reagent was prepared from Mg (2.6 g, 105 mmol) and 1-bromooctane (20.0 g, 105 mmol) in anhydrous Et₂O (35 mL) with refluxing for 1 hr. The reaction was cooled, and a solution of ethyl 5-bromopentanoate (8.0 g, 38.3 mmol) in dry Et₂O (25 mL) was added dropwise followed with refluxing for an additional 1 hr. The reaction was cooled to RT and poured into 100 mL satd. ice/NH₄Cl solution. The aqueous portion was extracted with Et₂O and the organics dried (MgSO₄), filtered and concentrated *in-vacuo.* The crude material was purified by automated flash chromatography (0-20% EtOAc/Hex) to give 9-(4-bromobutyl)heptadecan-9-ol (10.6 g, 70.7%).

### Step 2. Synthesis of 9-(4-bromobutylidene)heptadecane

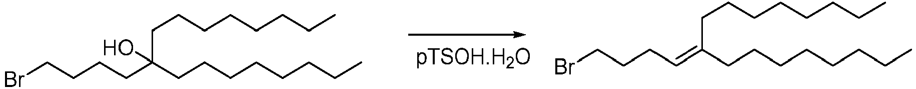

9-(4-Bromobutyl)heptadecan-9-ol (10.6 g, 27.0mmol) and p-TsOH.H₂O (93 mg, 0.54 mmol) were stirred under N₂ at 130°C for 2 hr. The crude material was purified by automated flash chromatography (0-4% EtOAc/Hex) to give 9-(4-bromobutylidene)heptadecane (8.84 g, 87.5%).

### Step 3. Synthesis of 5-octyltridec-4-en-1-ol

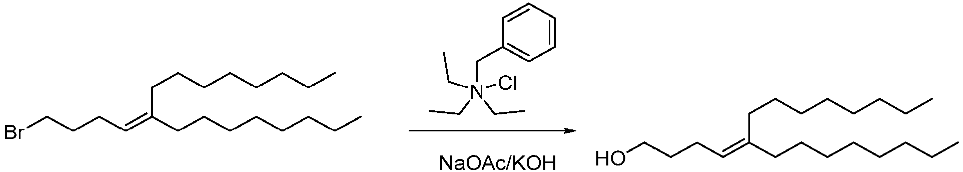

9-(4-Bromobutylidene)heptadecane (8.84 g, 23.7 mmol), benzyl(chloro)triethylamine (540 mg, 2.4 mmol) and NaOAc (7.8 g, 94.7 mmol) were stirred in DMF (60 mL) at 70°C for 20 hr. The reaction was poured into ice/water and extracted with Et₂O. The combined organics were dried (MgSO₄), filtered and concentrated *in-vacuo.* The residue was taken up in MeOH, KOH (18.6 g, 332 mmol) in H₂O added and the reaction stirred for 30 min. MeOH was removed *in-vacuo* and the reaction partitioned between H₂O and Et₂O. The combined organics were dried (MgSO₄), filtered and concentrated *in-vacuo* and the crude material purified by automated flash chromatography (0-15% EtOAc/Hex) to give 5-octyltridec-4-en-1-ol (4.5 g, 60.8%).

### Step 4. Synthesis of 5-octyltridecan-1-ol

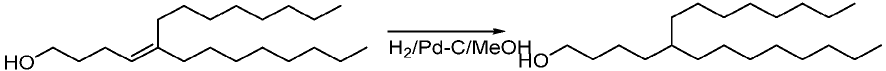

5-Octyltridec-4-en-1-ol (4.469 g, 14.39 mmol) was subjected to catalytic hydrogenation over Pd-C at RT in MeOH (50 mL) for 15 hr. The reaction was filtered and concentrated *in-vacuo* to give 5-octyltridecan-1-ol (3.5 g, 77.8%) which was used without further purification.

The following compounds were prepared using analogous methodology as described above

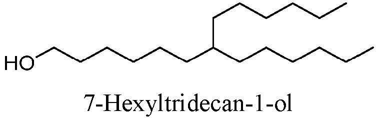

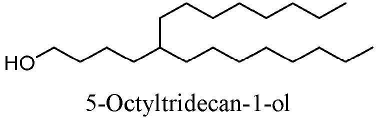

### Preparatory Example 8. General Method (b) for the Synthesis Of Branched Alkyl Alcohols - Exemplified by the synthesis of 3-pentyloctan-1-ol

### Step 1. Synthesis of ethyl 3-pentyloct-2-enoate

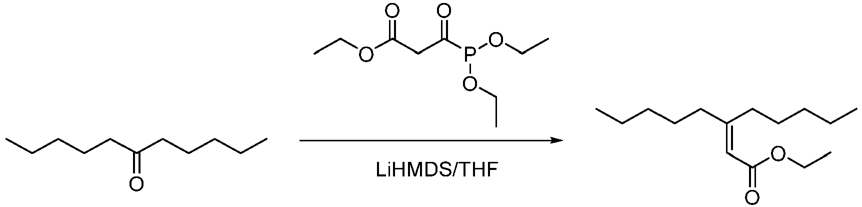

Triethyl phosphonoacetate (65.8 g, 294 mmol) was stirred in THF (220 mL) at -10°C. 1M LiHMDS in THF (294 mL, 294 mmol) was added dropwise and the reaction stirred at -10°C for 1 hr then 0°C for 1 hr. 6-Undecanone (25 g, 147 mmol) was added and the reaction stirred at 45°C for 16 hr. The reaction was quenched with H2O, extracted with Et2O and washed with brine. The organics were dried (MgSO4) filtered and concentrated in-vacuo to give ethyl 3-pentyloct-2-enoate (43.5 g) which was used in the subsequent step without further processing.

### Step 2. Synthesis of ethyl 3-pentyloctanoate

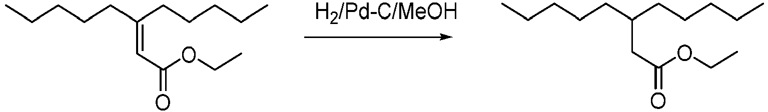

Ethyl 3-pentyloct-2-enoate (35 g, 147 mmol) was subjected to catalytic hydrogenation over Pd-C in MeOH (100 mL) at RT for 20 hr. The reaction was filtered and concentrated in-vacuo to give ethyl 3-pentyloctanoate (33.3 g, 93.6 %) which was used without further purification.

### Step 3. Synthesis of 3-pentyloctan-1-ol

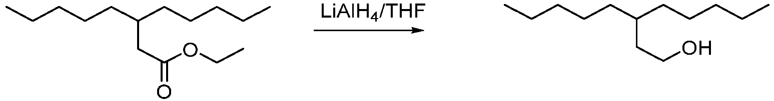

LiAlH₄ (6.8 g, 179 mmol) was stirred in THF (500 mL) at 0°C for 30 min. Ethyl 3-pentyloctanoate (33.3 g, 138 mmol in THF (500 mL) was added dropwise and the reaction stirred at RT until complete. The reaction was cooled to 0°C, diluted with Et₂O (500mL) and quenched with 1M NaOH (16 mL). The reaction was stirred for 30 mins, dried (MgSO₄), filtered through celite and concentrated *in-vacuo.* The crude was purified by automated flash chromatography (0-30% EtOAc/Hex) to give 3-pentyloctan-1-ol (24.7 g, 89.7%).

The following compounds were prepared using analogous methodology as described above:

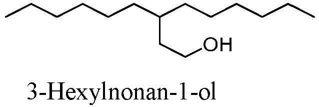

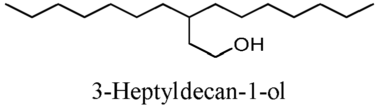

### Preparatory Example 9. Synthesis of (3,5-dihexylphenyl)methanol

### Step 1. Synthesis of methyl 3,5-bis(hex-1-yn-1-yl)benzoate

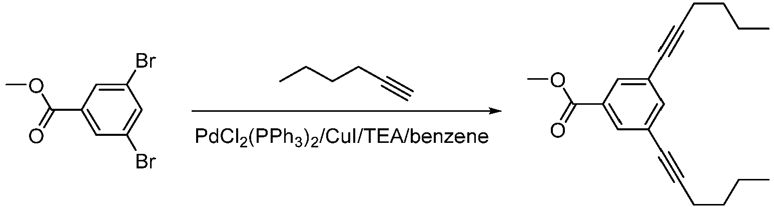

Methyl 3,5-dibromobenzoate (7.4 g, 25.0 mmol), hexyne (8.2 g, 100.0 mmol), dichlorobis[4-(diphenylphosphanyl)phenyl]palladium (1.4 g, 2.0 mmol), CuI (0.2 g, 1.0 mmol) and TEA (21.0 mL, 150 mmol) were stirred in benzene (100 mL) at 80°C for 48 hr. The reaction was filtered through a silica pad eluting with EtOAc. The organics were concentrated *in-vacuo* and the crude purified by automated flash chromatography (0-20% EtOAc/Hex) to give methyl 3,5-bis(hex-1-yn-1-yl)benzoate (7.0 g, 94.4%).

### Step 2. Synthesis of methyl 3,5-dihexylbenzoate

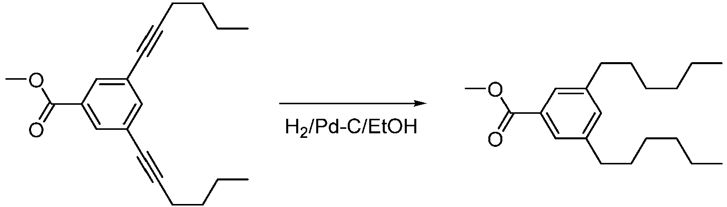

Methyl 3,5-bis(hex-1-yn-1-yl)benzoate (7.0 g, 23.6 mmol) was subjected to catalytic hydrogenation over Pd-C in EtOH (100 mL) at RT for 18 hr. The reaction was filtered through Celite and concentrated *in-vacuo* to give methyl 3,5-dihexylbenzoate (6.3 g, 87.7 %) which was used without further purification.

### Step 3. Synthesis of (3,5-dihexylphenyl)methanol

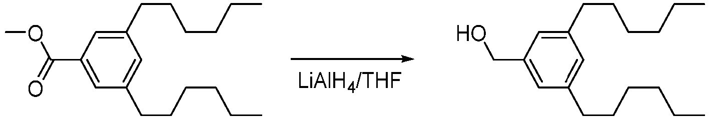

Methyl 3,5-dihexylbenzoate (6.3 g, 20.7 mmol) was stirred in dry THF at 0°C. LiAlH₄ (1.2 g, 31.0 mmol) was added in portions and the reaction stirred for 1hr allowing to warm to RT. After completion the reaction was quenched with H₂O and 1M NaOH and extracted with EtOAc. The organics were dried (MgSO₄), filtered, concentrated *in-vacuo* and purified by automated flash chromatography (10 % EtOAc/Hex) to give (3,5-dihexylphenyl)methanol (3.0 g, 52.4%).

### Preparatory Example 10. Synthesis of 2-fluoro-2-hexyldecanoic acid

### Step 1. Synthesis of ethyl 2-hexyldecanoate

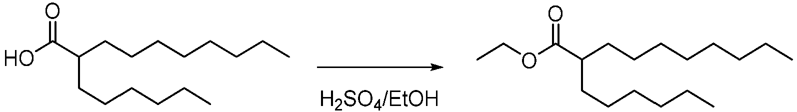

Hexyldecanoic acid (3 g, 11.7 mmol) and H₂SO₄ (cat) were refluxed in EtOH (20 mL) for 15 hr. The reaction was concentrated *in-vacuo* and the residue partitioned between hexane and water. The organics were dried (MgSO₄), filtered, concentrated *in-vacuo* and the residue purified by automated flash chromatography (0-6% EtOAc/Hex) to give ethyl 2-hexyldecanoate (3.0 g, 90.7%).

### Step 2. Synthesis of ethyl 2-fluoro-2-hexyldecanoate

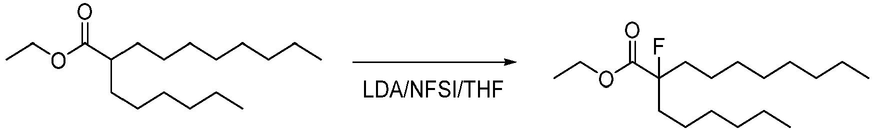

Ethyl 2-hexyldecanoate (1 g, 3.5 mmol) in THF was added dropwise to a solution of LDA (2.6 mL, 2 M, 5.273 mmol) at -78 °C and stirred for 0.5 hr. N-fluorobenzenesulfonimide (1.7 g, 5.3 mmol,) in THF (15 mL) was added dropwise and the reaction was stirred for 18 hr allowing to warm to RT. The reaction was diluted with EtOAc, washed sequentially with aq. satd. NH4Cl, water and brine, dried (MgSO4), concentrated in-vacuo and purified by automated flash chromatography (0-6% EtOAc/Hex). NMR showed 60% conversion. The reaction was repeated a second time to give ethyl 2-fluoro-2-hexyldecanoate (770 mg, 72.4 %).

### Step 3. Synthesis of 2-fluoro-2-hexyldecanoic acid

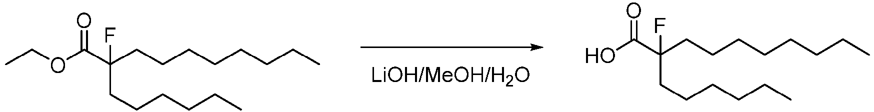

Ethyl 2-fluoro-2-hexyldecanoate (770 mg, 2.5 mmol) and LiOH (125 mg, 5.2 mmol) were stirred in MeOH (10 mL) and H₂O (2 mL) at RT for 15 hr. 6 M HCl (0.87 mL) was added and the MeOH removed *in-vacuo.* The residue was dissolved in DCM, washed with brine, dried (MgSO₄), filtered and concentrated *in-vacuo* to give 2-fluoro-2-hexyldecanoic acid (520 mg, 74.4 %).

### Preparatory Example 11. Synthesis of Amino Alcohol THP Ethers Exemplified by the Synthesis of 5-(Oxan-2-yloxy)pentan-1-amine

### Step 1. Synthesis of benzyl N-(5-hydroxypentyl)carbamate

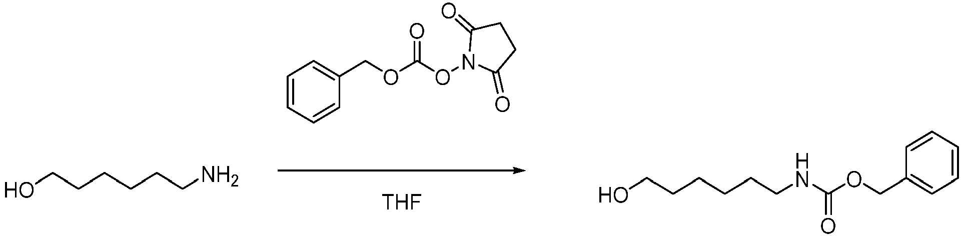

5-Aminopentanol (21.0 g, 203 mmol) and benzyl 2,5-dioxopyrrolidin-1-yl carbonate (50.7 g, 203 mmol) were stirred in THF at RT for 16 hr. The reaction was concentrated *in-vacuo,* the residue taken up in EtOAc (250 mL), washed with 1M HCl and 0.5M NaOH, dried (MgSO₄), filtered and concentrated *in-vacuo.* The resultant oil was agitated in hexane until the product crystalized. The solid was recovered by filtration to give benzyl N-(5-hydroxypentyl)carbamate (44 g, 91.0%).

### Step 2. Synthesis of benzyl N-[5-(oxan-2-yloxy)pentyl]carbamate

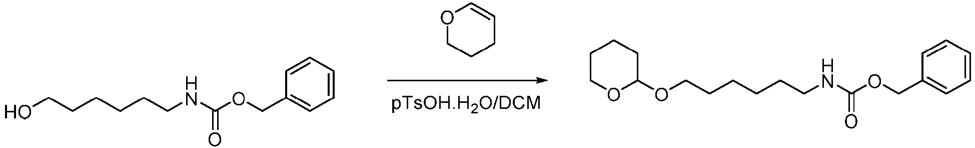

Benzyl N-(5-hydroxypentyl)carbamate (20.0 g, 84 mmol), 2H-pyran,3,4-dihydro- (8.6 g, 101) and pTsOH-H₂O (1.6 g, 8.4 mmol) were stirred in DCM (270 mL) at RT for 2 hr. The reaction was washed with NaHCO₃ (sat. soln) and brine, dried (MgSO₄), filtered and concentrated *in-vacuo.* The crude material was purified by automated flash chromatography (10-50 % EtOAc/Hex) to give benzyl N-[5-(oxan-2-yloxy)pentyl]carbamate (19.5 g, 72.0 %).

### Step 3. Synthesis of 5-(oxan-2-yloxy)pentan-1-amine

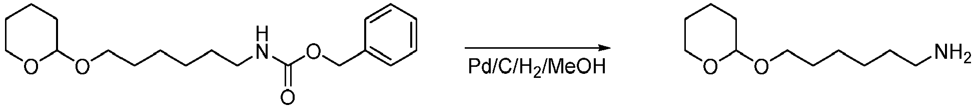

Benzyl N-[5-(oxan-2-yloxy)pentyl]carbamate (19.0 g, 59 mmol) was subjected to catalytic hydrogenation over Pd/C in MeOH at RT for 16 hr. The reaction was filtered through Celite and concentrated *in-vacuo* to give 5-(oxan-2-yloxy)pentan-1-amine (10.9 g, 98.5%) which was used without purification.

The following compound was prepared using analogous methodology as described above:

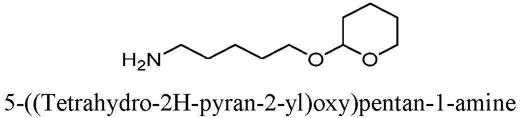

### Preparatory Example 12. Synthesis of Keto-THP Ethers Exemplified by the Synthesis of 1-(Oxan-2-yloxy)hexadecan-7-one

### Step 1. Synthesis of 2-[(6-bromohexyl)oxy]oxane

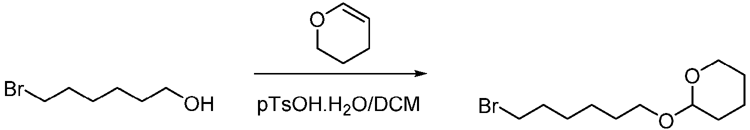

6-Bromo-1-hexanol (40 g, 220 mmol), dihydropyran (23 g, 265 mmol) and pTsOH-H₂O (4.2 g, 22.0 mmol) were stirred in DCM (500 mL) at RT for 2hr. The reaction was washed with NaHCO₃ (sat. soln) and brine, dried (MgSO₄), filtered and concentrated *in-vacuo.* The crude material was purified by flash chromatography (0-20% EtOAc/Hex) to give 2-[(6-bromohexyl)oxy]oxane (49 g, 83.6 %).

### Step 2. Synthesis of 1-(oxan-2-yloxy)hexadecan-7-ol

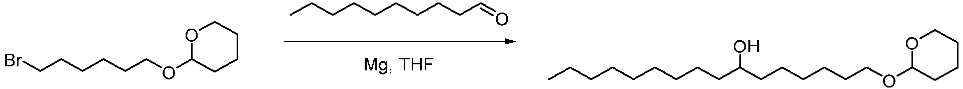

A Grignard reagent was prepared from 2-[(6-bromohexyl)oxy]oxane (49 g, 185 mmol) and magnesium (5.4 g, 222 mmol) in anhydrous THF (175 mL) with refluxing for 30 mins. The reaction was cooled to 0°C and decanal (23 g, 148 mmol) in anhydrous THF (144 mL) added dropwise over 1hr. The reaction was stirred for 2 hr allowing to warm to RT then quenched with sat'd NH₄Cl solution. The organics were separated, washed with water and brine and dried (MgSO₄). The filtrate was concentrated *in-vacuo* and the residue purified by automated flash chromatography (0-15% EtOAc/Hex) to give 1-(oxan-2-yloxy)hexadecan-7-ol (47 g, 92.8 %).

### Step 3. Synthesis of 1-(oxan-2-yloxy)hexadecan-7-one

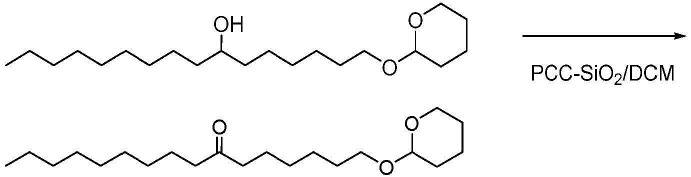

1-(Oxan-2-yloxy)hexadecan-7-ol (47 g, 137 mmol) and PCC/SiO₂ (1:1) (118 g, 274 mmol) were stirred in DCM (294 mL) at RT for 16 hr. The mixture was filtered through a pad of silica and eluted with 50:50 EtOAc/Hex. The filtrate was concentrated *in-vacuo* and the residue purified by automated flash chromatography (0-10% EtOAc/Hex) to give 1-(oxan-2-yloxy)hexadecan-7-one (37.5 g, 80.3 %).

### Preparatory Example 13. Synthesis of Oxo-alkenes Exemplified by the Synthesis of Heptadec-1-en-7-one

### Step 1. Synthesis of heptadec-1-en-7-ol

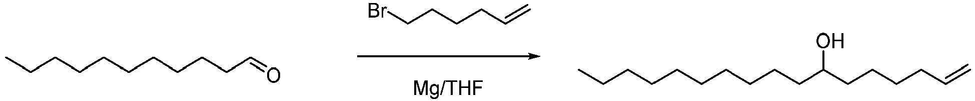

A Grignard reagent was prepared from 6-bromohex-1-ene (35 g, 215 mmol) and magnesium (6.3 g, 258 mmol) in THF (250 mL). After the initial reflux the reaction was stirred for 2 hours then cooled to 0°C Undecanal (29 g, 172 mmol) was added dropwise and the reaction stirred at RT for 16 hr. The reaction was quenched with water (100 mL), cooled to 0°C and 6M HCl (50mL) added to dissolve residual magnesium. The solution was diluted with Et₂O (250 mL), the organics separated, washed with brine, dried (MgSO₄), filtered and concentrated *in-vacuo* to give heptadec-1-en-7-ol (43 g, 98.4 %) which was used without further purification.

### Step 2. Synthesis of heptadec-1-en-7-one

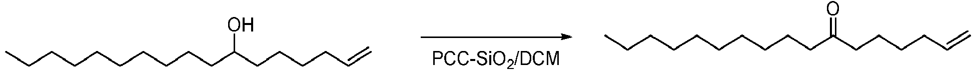

Heptadec-1-en-7-ol (43 g, 169 mmol) and PCC/Silica 1:1 wt (91 g, 211 mmol) were stirred in DCM (300 mL) at RT for 16 hr. The reaction was filtered through silica, eluting with DCM and the filtrate concentrated *in-vacuo.* The residue was purified by automated flash chromatography (0-10% EtOAc/hexane) to give heptadec-1-en-7-one (31 g, 72.7 %).

### Preparatory Example 14. Synthesis of Oxo-carboxylic Acids. Method (a) Exemplified by the Synthesis of 8-Oxooctadecanoic Acid

### Step 1 Synthesis of 2-[(7-bromoheptyl)oxy]oxane

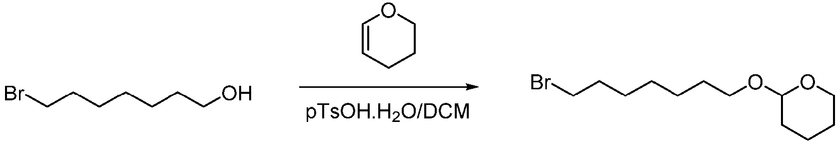

7-Bromoheptan-1-ol (102 g, 523 mmol), dihydropyran (53 g, 627 mmol) and pTsOH-H₂O (10 g, 52 mmol) were stirred in DCM (1370 mL for 2 hr. The reaction was washed with NaHCO₃ (sat soln) and brine, dried (MgSO₄), filtered and concentrated *in-vacuo.* The crude material was passed through silica pad with 25% EtOAc/Hex and the combined organics concentrated *in-vacuo* to give 2-[(7-bromoheptyl)oxy]oxane (135 g, 92.5 %) which was used without further purification.

### Step 2 Synthesis of 1-(oxan-2-yloxy)octadecan-8-ol

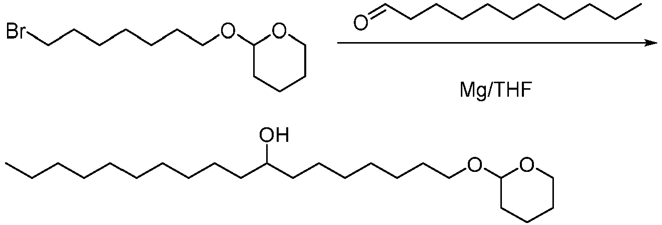

A Grignard reagent was prepared from 2-[(7-bromoheptyl)oxy]oxane (135 g, 483 mmol) and magnesium (14.1 g, 580 mmol) in anhydrous THF (82 mL). After addition of the bromide the reaction was stirred at reflux for 30 min. The reaction was cooled to 0°C and Undecanal (66 g, 387 mmol) in anhydrous THF (412 mL) added dropwise over 1 hr. The reaction was stirred for 16 hr allowing to warm to RT then quenched with H₂O (100 mL) followed by 6M HCl (200 mL). The reaction was extracted with EtOAc, washed with H₂O and brine, dried (MgSO₄) and concentrated *in-vacuo.* The residue was purified by automated flash chromatography (0-50% EtOAc/Hex) to give 1-(oxan-2-yloxy)octadecan-8-ol (68 g, 47.0%) .

### Step 3 Synthesis of 8-oxooctadecanoic acid

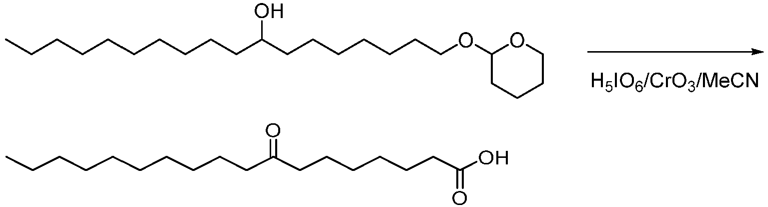

Periodic acid (239 g, 1009 mmol) and CrO₃ (0.5 g, 2.6 mol%) were dissolved in MeCN (3 L, 0.75% H₂O V/V) at RT for 16 hr. 1-(oxan-2-yloxy)octadecan-8-ol (68 g, 183 mmol) in MeCN (1 L, 0.75 v % water) was added over 30-60 mins while maintaining the reaction temp at 0-5°C. The reaction was stirred for 16 hr allowing to warm to RT. Na₂HPO₄ (163 g in 2.7 L H₂O) was added to quench and the reaction extracted with EtOAc. The organics were washed sequentially with brine, NaHSO_{3 aq}. (29.91 g in 680 mL H₂O) and brine. The reaction was dried (MgSO₄), filtered and concentrated *in-vacuo.* The residual solid was triturated in a minimum volume of MeCN to give 8-oxooctadecanoic acid (40.9 g, 74.7 %).

### Preparatory Example 15. Synthesis of Oxo-carboxylic Acids. Method (b) Exemplified by the Synthesis of 8-Oxooctadecanoic Acid

### Step 1. Synthesis of 1-decylcyclooctan-1-ol

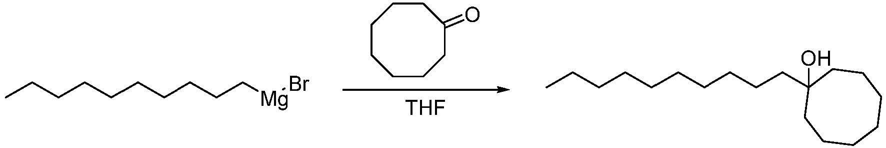

Bromo(decyl)magnesium (22.2 g, 90.4 mmol) was stirred at 10°C in THF (150 mL). Cyclooctanone (9.3 g, 72 mmol) in THF (50mL) was added dropwise. After complete addition the reaction was cooled to 0°C and quenched with water (100mL) and 6M HCl (400mL). The organics were separated, washed with brine, dried (MgSO₄), filtered and concentrated *in-vacuo.* The crude material was purified by automated flash chromatography (0-10% EtOAc-hexane) to give 1-decylcyclooctan-1-ol (14.2 g, 73.1%).

### Step 2. Synthesis of (Z)-1-decylcyclooct-1-ene

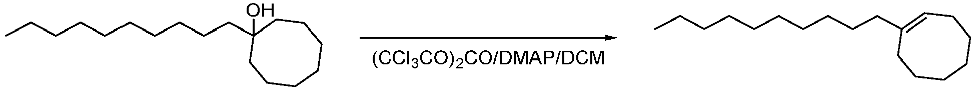

1-Decylcyclooctan-1-ol (7.71 g, 28.8) and DMAP (7.0 g, 57.4 mmol) were stirred in DCM (77.1 mL) at 0°C. Triphosgene (4.3 g, 14.4 mmol) in DCM (40 mL) was added dropwise. The reaction was stirred for 3 hr allowing to warm to RT then quenched with 2M HCl. The organics were separated, washed with brine, dried (MgSO₄), filtered and concentrated *in-vacuo.* The crude material was purified by automated flash chromatography (100% hexane) to give (Z)-1-decylcyclooct-1-ene (7.2 g, 76.5 %).

### Step 3. Synthesis of 8-oxooctadecanoic acid

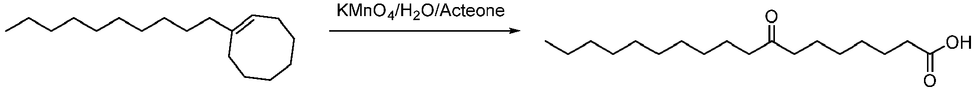

(1Z)-1-Decylcyclooct-1-ene (5.5 g, 22 mmol) was stirred in acetone (55 mL) and H₂O (5.5 mL) at 0°C. KMnO₄ (17.4 g, 110 mmol) was added in portions over 4 hr while maintaining the temperature at 0°C. The reaction was stirred for 18 hr allowing to warm to RT, then quenched with alternate addition of sodium bisulfite and 6 M HCl. The reaction was partitioned between 1M HCl and EtOAc and the combined organics washed with brine, dried (MgSO₄), filtered and concentrated *in-vacuo.* The crude material was purified by automated flash chromatography (5-30% EtOAc-hexane) to give 8-oxooctadecanoic acid (3.7 g, 56.0%).

The following compounds were prepared using analogous methodology as described above: 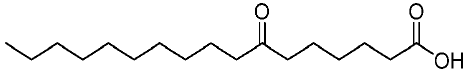 7-Oxoheptadecanoic acid 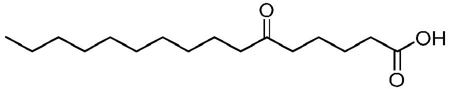 6-Oxohexadecanoic acid 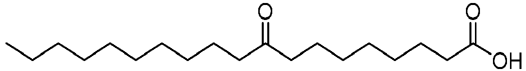 9-Oxononadecanoic acid

### Preparatory Example 16. Synthesis of Oxo Esters Exemplified by the Synthesis of 2-Butyloctyl 8-Oxooctadecanoate

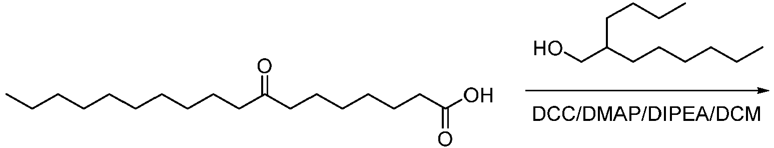

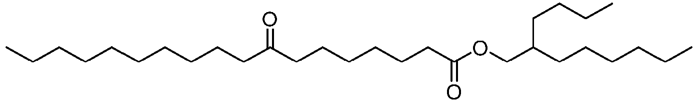

8-Oxooctadecanoic acid (1 g, 3.4 mmol), butyloctanol (687 mg, 3.7 mmol), DCC (1.4 g, 6.7 mmol) and DMAP (20 mg, 0.17 mmol) were stirred in DCM at RT for 1.5 hr. The reaction was concentrated *in-vacuo,* the residue suspended in hexanes and filtered. The filtrate was concentrated *in-vacuo* and the residue purified by automated flash chromatography (0-25 % EtOAc/Hex) to give 2-butyloctyl 8-oxooctadecanoate (1.1 g, 68.9 %).

The following compounds were prepared using analogous methodology described above 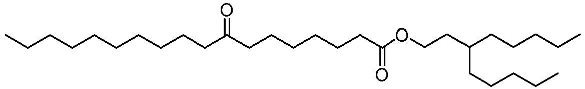 3-Pentyloctyl 8-oxooctadecanoate 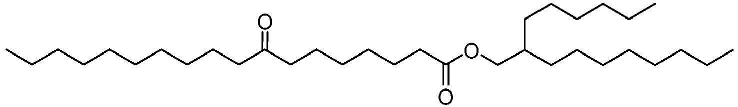 2-Hexyldecyl 8-oxooctadecanoate 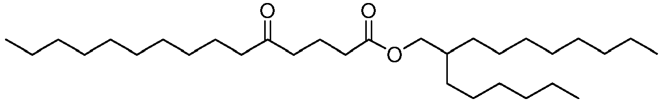 2-Hexyldecyl 5-oxopentadecanoate 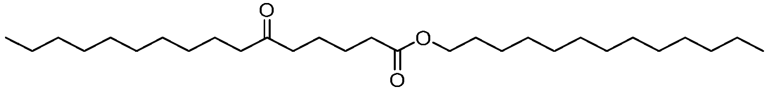 Tridecyl 6-oxohexadecanoate 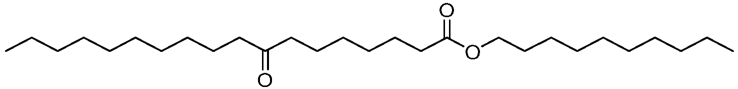 Decyl 8-oxooctadecanoate 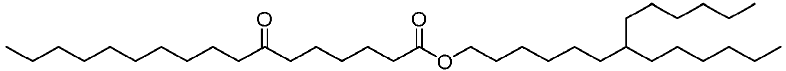 7-Hexyltridecyl 7-oxoheptadecanoate 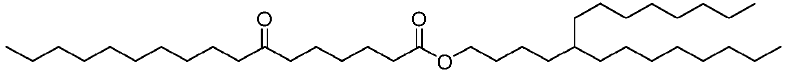 5-Octyltridecyl 7-oxoheptadecanoate 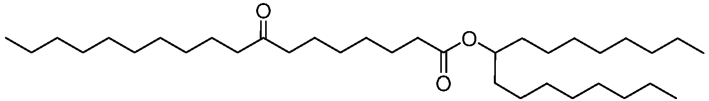 Heptadecan-9-yl 8-oxooctadecanoate 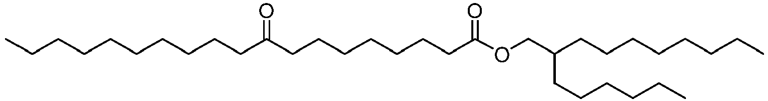 2-Hexyldecyl 9-oxononadecanoate 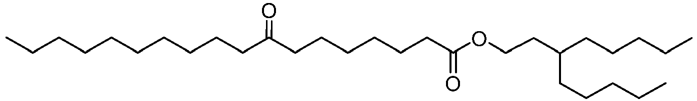 3-Pentyloctyl 8-oxooctadecanoate 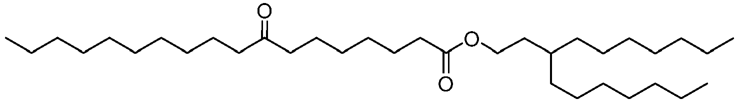 3-Heptyldecyl 8-oxooctadecanoate 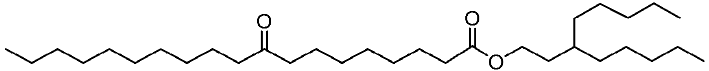 3-Pentyloctyl 9-oxononadecanoate

### Preparatory Example 17. Synthesis of 8-[(2-Butyloctyl)oxy]-8-Oxooctanoic Acid

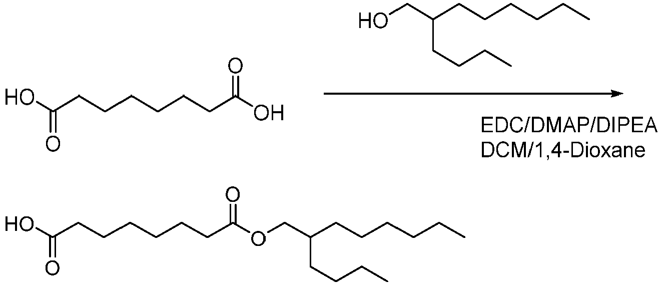

Suberic acid (5 g, 28.7 mmol), butyloctanol (5.4 g, 28.7 mmol), EDC (5.8 g, 30.1 mmol), DIPEA (10 mL, 57.4 mmol) and DMAP (cat.) were stirred DCM (10 mL) and 1,4 dioxane (180 mL) at RT for 18 hr. The reaction was concentrated *in-vacuo,* the residue taken-up in DCM, washed with NaHCO₃ (sat. soln.) and brine, dried (MgSO₄), filtered and concentrated *in-vacuo.* The crude material was purified by automated flash chromatography (5-30% EtOAc-hexane) to give 8-[(2-butyloctyl)oxy]-8-oxooctanoic acid (4.4 g, 45.0 %).

The following compounds were prepared using analogous methods as described above 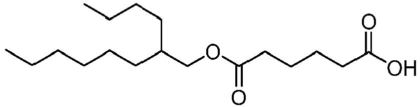 6-((2-Butyloctyl)oxy)-6-oxohexanoic acid 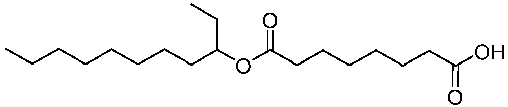 8-Oxo-8-(undecan-3-yloxy)octanoic acid 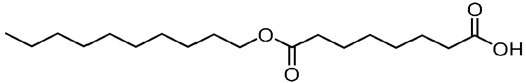 8-(Decyloxy)-8-oxooctanoic acid 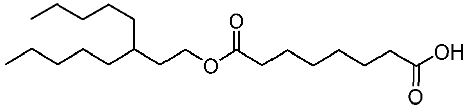 8-Oxo-8-((3-pentyloctyl)oxy)octanoic acid 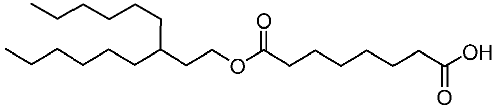 8-((3-Hexylnonyl)oxy)-8-oxooctanoic acid 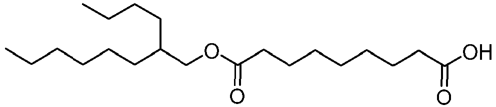 9-((2-Butyloctyl)oxy)-9-oxononanoic acid 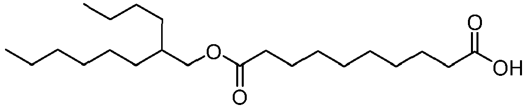 10-((2-Butyloctyl)oxy)-10-oxodecanoic acid 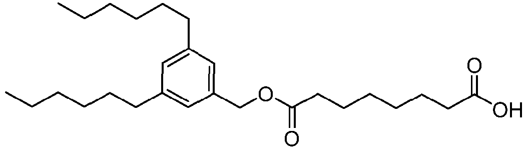 8-((3,5-Dihexylbenzyl)oxy)-8-oxooctanoic acid

### Preparatory Example 18. Synthesis of 3,5-Dihexylbenzyl 8-Aminooctanoate

### Step 1. Synthesis of (3,5-dihexylphenyl)methyl 8-[(tert-butoxycarbonyl)amino]octanoate

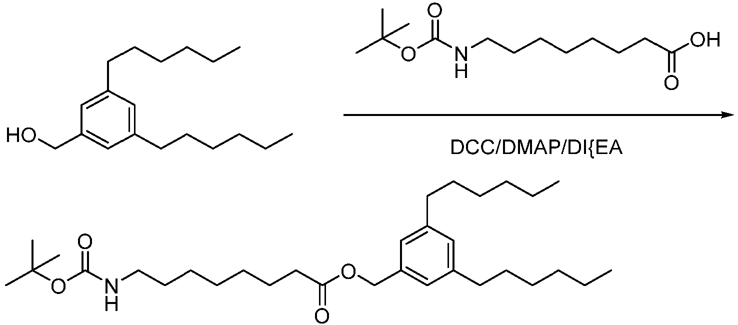

(3,5-Dihexylphenyl)methanol (3.0 g, 10.9 mmol), 8-[(tert-butoxycarbonyl)-amino]octanoic acid (2.8 g, 10.9 mmol), DCC (2.5 g, 11.9 mmol), DIPEA (3.8 mL, 21.7 mmol) and DMAP (133 mg, 1.1 mmol) were stirred in DCM at RT for 16 hr. The resultant precipitate was removed by filtration, the filtrate concentrated *in-vacuo* and the residue purified by automated flash chromatography (10% EtOAc/Hex) to give (3,5-dihexylphenyl)methyl 8-[(tert-butoxycarbonyl)amino]octanoate (1.81 g, 32.2%).

### Step 2. Synthesis of (3,5-dihexylphenyl)methyl 8-aminooctanoate

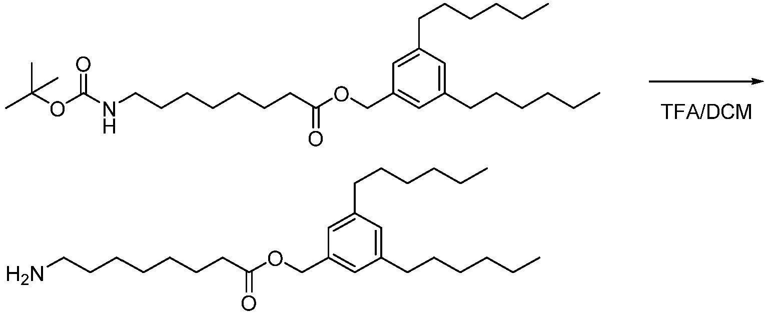

(3,5-Dihexylphenyl)methyl 8-[(tert-butoxycarbonyl)amino]octanoate (1.8 g, 3.5 mmol) was stirred in 20% TFA solution at RT in DCM for 1 hr. The reaction was concentrated *in-vacuo,* the residue taken up in DCM and converted to the free base with 1M NaOH. The organics were separated, dried (MgSO₄) and concentrated *in-vacuo* to give (3,5-dihexylphenyl)methyl 8-aminooctanoate (1.35 g, 93.0 %) which was used without purification.

### Preparatory Example 19. Synthesis of 2-Hexyldecyl 8-Aminooctanoate

### Step 1. Synthesis of 8-{[(benzyloxy)carbonyl]amino}octanoic acid

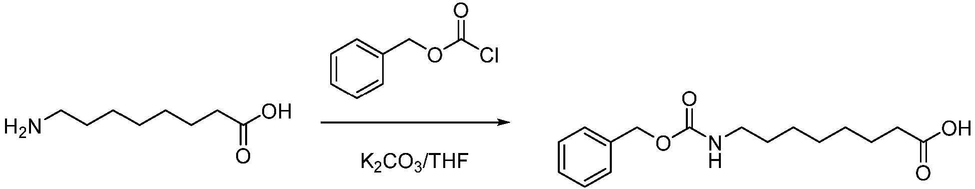

8-Aminooctanoic acid (5 g, 31.4 mmol), K₂CO₃ (8.7 g, 63 mmol) and benzyl chloroformate (7.0 mL, 47.1 mmol) were stirred in THF (150 mL) at RT for 16 hr. The reaction was acidified (6M HCl) and concentrated *in-vacuo.* The residue was partitioned between DCM (100 mL) and H₂O water (100 mL) and the aqueous extracted with DCM. The combined organics were dried (MgSO₄), filtered and concentrated *in-vacuo* to give 8-{[(benzyloxy)carbonyl]amino } octanoic acid (8.4 g, 91.2 %) which was used without purification.

### Step 2. Synthesis of 2-hexyldecyl 8-{[(benzyloxy)carbonyl]amino}octanoate

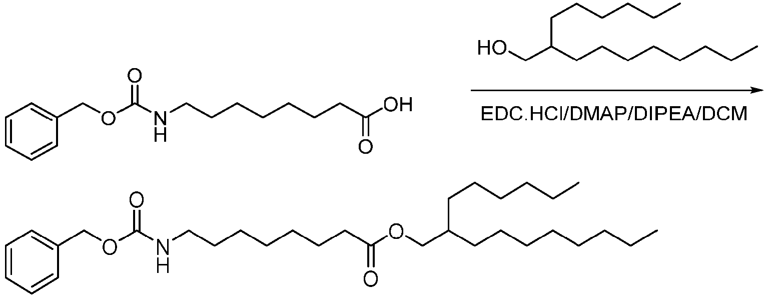

8-{[(Benzyloxy)carbonyl]amino}octanoic acid (1.05 g, 3.6 mmol), EDC.HCl (1.4 g, 7.2 mmol), hexyldecanol (1.0 g, 4.0 mmol), DMAP (22 mg, 0.18 mmol) and DIPEA (1.9 mL, 10.7 mmol) were stirred in DCM (25 mL) at RT for 16 hr. The reaction was concentrated *in-vacuo,* taken up in EtOAc (100 mL), washed with saturated NaHCO₃ and brine, dried (MgSO₄), filtered and concentrated *in-vacuo.* The residue was purified by automated flash chromatography (0-50% EtOAc/hexanes) to give 2-hexyldecyl 8-{[(benzyloxy)carbonyl]amino}octanoate (1.45 g, 64.5 %).

### Step 3. Synthesis of 2-hexyldecyl 8-aminooctanoate

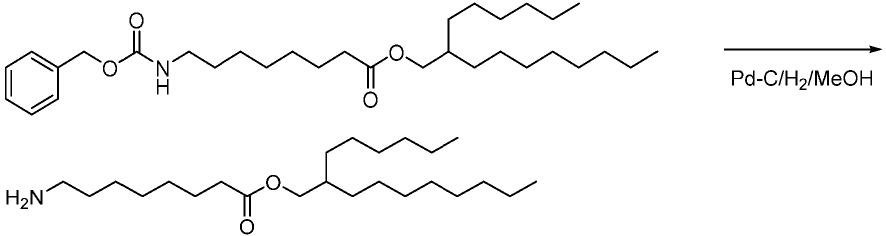

2-Hexyldecyl 8-{[(benzyloxy)carbonyl]amino}octanoate (1 g, 1.9 mmol) was subjected to catalytic hydrogenation over 10 % Palladium on Carbon (100 mg) in MeOH (25 mL) at RT for 16 hr. The reaction was filtered through celite and concentrated *in-vacuo* to give 2-hexyldecyl 8-aminooctanoate (0.71 g, 95.8 %).

The following compounds were prepared using analogous methodology as described above. 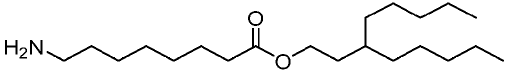 3-Pentyloctyl 8-aminooctanoate 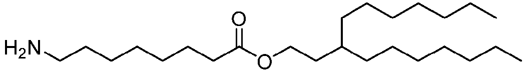 3-Heptyldecyl 8-aminooctanoate 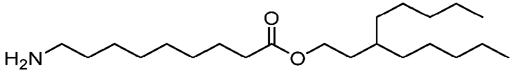 3-Pentyloctyl 9-aminononanoate

### Example 1. Synthesis of 2-Hexyldecyl 8-(N-decyl-4-(dimethylamino)butanamido)-octadecanoate (1)

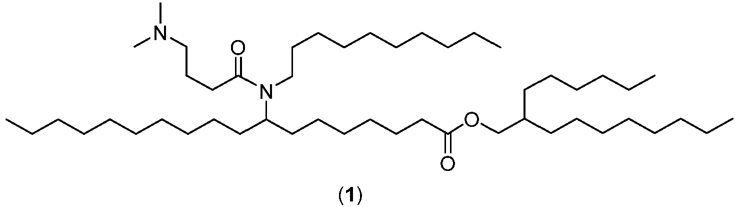

### Step 1. Synthesis of 2-hexyldecyl 8-oxooctadecanoate

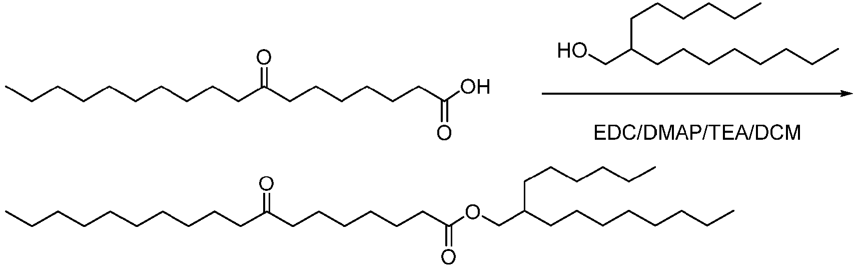

8-Oxooctadecanoic acid (1 g, 3.4 mmol), hexyldecanol (894 mg, 3.7 mmol), EDC (1.29 g, 6.7 mmol), DIPEA (1.8 mL, 10.1 mmol) and DMAP (cat.) were stirred in DCM (30 mL) at RT for 18 hr. The reaction was diluted with DCM, washed with NaHCO₃ (sat) and brine, dried (MgSO₄), filtered and concentrated *in-vacuo.* The crude material was purified by automated flash chromatography (0-10% EtOAc-hexane) to give 2-hexyldecyl 8-oxooctadecanoate (1.33 g, 75.9 %).

### Step 2. Synthesis of 2-hexyldecyl 8-(decylamino)octadecenoate

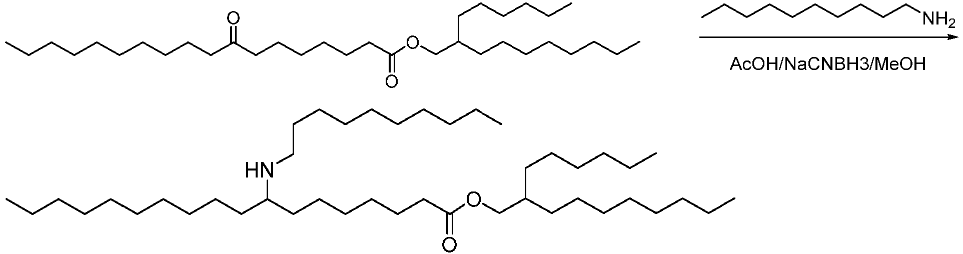

2-Hexyldecyl 8-oxooctadecanoate (933 mg, 1.8 mmol), N-decylamine (0.3 g, 1.9 mmol), NaCNBH₃ (340 mg, 5.4 mmol) and AcOH (0.2 g, 3.6 mmol) were heated in MeOH (10 ml) at 50°C for 18 hr. The reaction was concentrated *in-vacuo,* the residue taken-up in DCM and washed with NaHCO₃ (sat) and brine, dried (MgSO₄), filtered and concentrated *in-vacuo.* The crude material was purified by automated flash chromatography (0-5% MeOH-DCM) to give 2-hexyldecyl 8-(decylamino)octadecenoate (447 mg, 37.7 %).

### Step 3. Synthesis of 2-hexyldecyl 8-[N-decyl-4-(dimethylamino)butanamido]-octadecenoate (1)

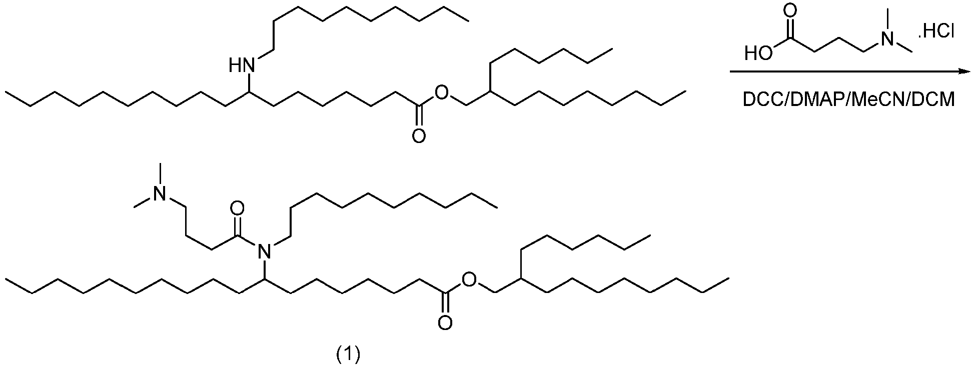

4-Dimethylaminobutanoic acid (HCl) (0.38 g, 2.2 mmol), DCC (0.51 g, 2.5 mmol) and DMAP (0.41 g, 3.4 mmol) were stirred in MeCN (6 mL) at RT for 3 hr. A solution 2-hexyldecyl 8-(decylamino)octadecanoate (744 mg, 1.1 mmol) in DCM (8 mL) was added and the reaction stirred at RT for 18 hr. The reaction was concentrated, dispersed in hexane:EtOAc:TEA (75:25:1), filtered through Celite and the filtrate concentrated *in-vacuo.* The residue was purified by automated chromatography (0-10% MeOH-DCM) to give 2-hexyldecyl 8-[N-decyl-4-(dimethylamino)butanamido]octadecanoate (**1**) (230 mg, 26.4%). ¹H NMR (400 MHz, CDCl₃) δ 4.01 - 3.92 (m, 2H), 3.63 (s, 1H), 3.10 - 2.97 (m, 2H), 2.31 (h, *J =* 6.6 Hz, 6H), 2.22 (s, 6H), 1.82 (q, *J =* 7.7 Hz, 2H), 1.48 (d, *J =* 36.9 Hz, 10H), 1.27 (s, 58H), 0.88 (t, *J =* 6.6 Hz, 12H).

The following compounds were prepared using analogous methods as described above 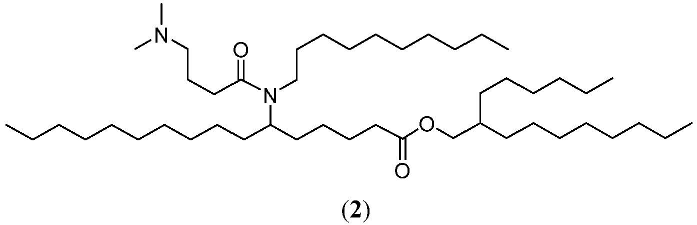 **2-Hexyldecyl 6-(N-decyl-4-(dimethylamino)butanamido)hexadecanoate** (**2**). ¹H NMR (400 MHz, CDCl3) δ 3.95 (d, *J =* 5.7 Hz, 2H), 3.65 (s, 1H), 3.09 - 2.98 (m, 2H), 2.31 (dt, *J =* 15.3, 7.4 Hz, 6H), 2.21 (s, 6H), 1.82 (q, *J* = 7.7 Hz, 2H), 1.48 (d, *J* = 29.4 Hz, 10H), 1.27 (s, 54H), 0.88 (t, *J =* 6.7 Hz, 12H). 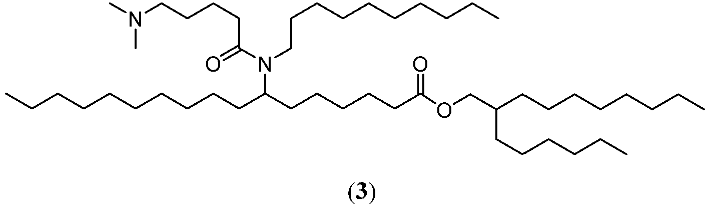 **2-Hexyldecyl 7-(N-decyl-5-(dimethylamino)pentanamido)heptadecanoate** (**3**). ¹H NMR (400 MHz, CDCl₃) δ 3.98 (dd, *J =* 5.8, 3.9 Hz, 2H), 3.70 - 3.57 (m, 1H), 3.04 (s, 2H), 2.31 (dtt, *J =* 7.5, 5.8, 3.0 Hz, 6H), 2.24 (s, 6H), 1.75 - 1.40 (m, 17H), 1.28 (p, *J =* 5.4 Hz, 55H), 0.90 (td, *J =* 6.8, 2.1 Hz, 12H). 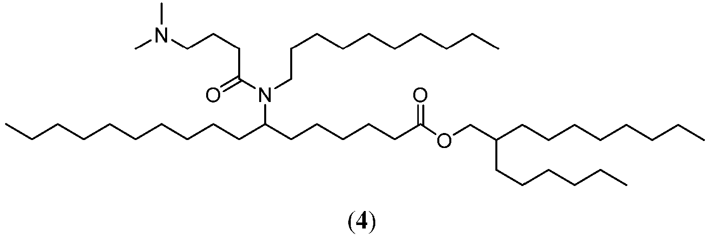 **2-Hexyldecyl 7-(N-decyl-4-(dimethylamino)butanamido)heptadecanoate** (**4**). ¹H NMR (400 MHz, CDCl₃) δ 3.98 (dd, *J =* 5.8, 4.1 Hz, 2H), 3.72 - 3.60 (m, 1H), 3.06 (q, *J =* 7.7 Hz, 2H), 2.39 - 2.27 (m, 6H), 2.24 (d, *J =* 1.1 Hz, 6H), 1.85 (h, *J =* 7.4 Hz, 2H), 1.72 - 1.40 (m, 13H), 1.29 (dq, *J =* 10.3, 6.5 Hz, 55H), 0.90 (td, *J =* 6.8, 2.3 Hz, 12H). 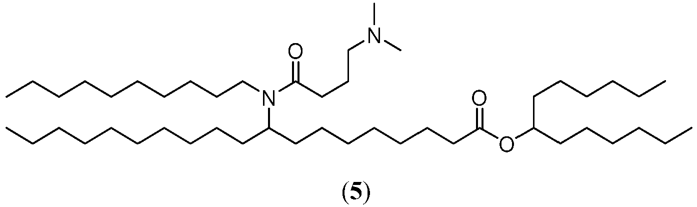 **Tridecan-7-yl 9-(N-decyl-4-(dimethylamino)butanamido)nonadecanoate** (**5**). ¹H NMR (400 MHz, CDCl₃) δ 4.88 (q, *J =* 6.4 Hz, 1H), 3.66 (t, *J =* 7.1 Hz, 1H), 3.06 (q, *J =* 6.7 Hz, 2H), 2.39 - 2.25 (m, 6H), 2.24 (s, 6H), 1.84 (h, *J* = 7.6 Hz, 2H), 1.72 - 1.40 (m, 17H), 1.29 (d, *J =* 8.9 Hz, 57H), 0.90 (t, *J =* 6.4 Hz, 12H). 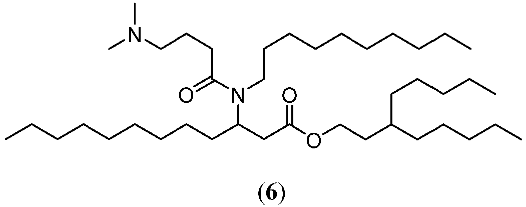 **3-Pentyloctyl 3-(N-decyl-4-(dimethylamino)butanamido)dodecanoate** (**6**). ¹H NMR (400 MHz, CDCl₃) δ 4.37 - 4.16 (m, 1H), 4.08 (q, *J =* 7.1 Hz, 2H), 3.17 (tt, *J =* 8.4, 4.9 Hz, 1H), 2.83 - 2.35 (m, 3H), 2.32 (qd, *J =* 8.0, 1.8 Hz, 3H), 2.24 (m, 6H), 1.83 (qd, *J =* 7.6, 5.3 Hz, 2H), 1.58 (q, *J=* 6.9 Hz, 4H), 1.50 (d, *J* = 5.9 Hz, 2H), 1.29 (dd, *J =* 13.2, 5.6 Hz 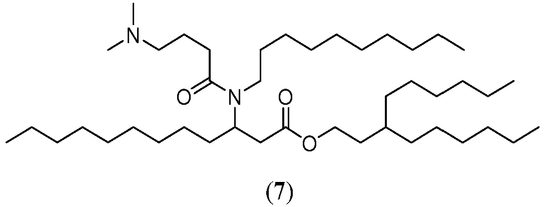 **3-Hexylnonyl 3-(N-decyl-4-(dimethylamino)butanamido)dodecanoate** (**7**). ¹H NMR (400 MHz, CDCl₃) δ 4.36 - 4.19 (m, 1H), 4.08 (q, *J =* 7.2 Hz, 2H), 3.17 (tt, *J =* 8.1, 4.7 Hz, 2H), 2.77 - 2.36 (m, 4H), 2.32 (qd, *J* = 7.7, 2.2 Hz, 3H), 2.24 (m, 6H), 1.83 (qd, *J =* 7.6, 5.1 Hz, 2H), 1.76 (s, 1H), 1.55 (dh, *J =* 24.3, 6.8 Hz, 5H), 1.37 - 1.18 (m, 47H), 0.90 (t, *J =* 6.8 Hz, 12H). 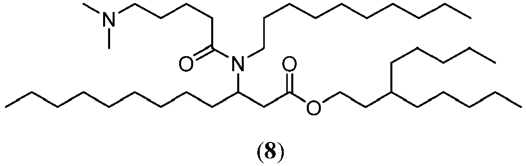 **3-Pentyloctyl 3-(N-decyl-5-(dimethylamino)pentanamido)dodecanoate** (**8**). ¹H NMR (400 MHz, CDCl₃) δ 4.35 - 4.15 (m, 1H), 4.08 (q, *J =* 7.5 Hz, 2H), 3.23 - 3.09 (m, 1H), 3.06 - 2.91 (m, 1H), 2.80 - 2.33 (m, 3H), 2.29 (ddd, *J =* 7.7, 6.1, 3.9 Hz, 3H), 2.23 (d, *J =* 1.2 Hz, 6H), 1.66 (p, *J =* 7.5 Hz, 3H), 1.61 - 1.45 (m, 7H), 1.37 - 1.14 (m, 44H), 0.90 (td, *J* = 6.9, 2.7 Hz, 12H). 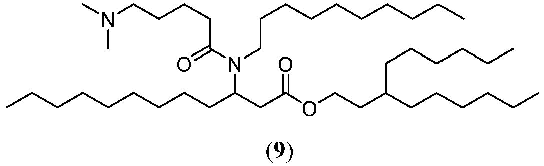 **3-Hexylnonyl 3-(N-decyl-5-(dimethylamino)pentanamido)dodecanoate (9).** ¹H NMR (400 MHz, CDCl₃) δ 4.26 (s, 1H), 4.08 (q, *J = 7.5* Hz, 2H), 3.21 - 3.08 (m, 1H), 2.78 - 2.33 (m, 3H), 2.29 (ddd, *J* = 7.5, 6.1, 3.7 Hz, 3H), 2.23 (d, *JJ* 1.2 Hz, 6H), 1.67 (p, *J =* 7.5 Hz, 3H), 1.61 - 1.45 (m, 7H), 1.28 (t, *J* = 5.7 Hz, 49H), 0.90 (td, *J* = 6.9, 2.5 Hz, 12H). 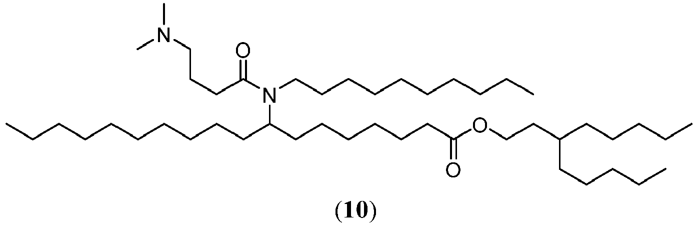 **3-Pentyloctyl 8-(N-decyl-4-(dimethylamino)butanamido)octadecenoate** (**10**) ¹H NMR (400 MHz, CDCl₃) δ 4.10 (td, *J =* 7.1, 2.8 Hz, 2H), 3.05 (t, *J =* 8.3 Hz, 2H), 2.40 (dd, *J =* 20.2, 11.9 Hz, 6H), 2.29 (td, *J* = 7.6, 4.9 Hz, 3H), 1.65 - 1.54 (m, 6H), 1.44 (q, *J* = 6.2 Hz, 5H), 1.29 (d, *J* = 10.3 Hz, 52H), 0.90 (td, *J =* 6.9, 2.7 Hz, 12H), 0.09 (s, 2H). 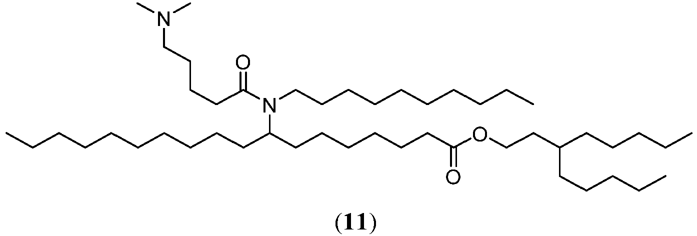 **3-Pentyloctyl 8-(N-decyl-5-(dimethylamino)pentanamido)octadecenoate** (**11**). ¹H NMR (400 MHz, CDCl₃) δ 4.10 (td, *J =* 7.1, 2.8 Hz, 2H), 3.04 (t, *J =* 8.3 Hz, 2H), 2.37 (s, 3H), 2.36 - 2.26 (m, 6H), 1.64 - 1.55 (m, 6H), 1.53 (s, 2H), 1.45 (s, 5H), 1.29 (d, *J =* 10.2 Hz, 48H), 0.90 (td, *J =* 6.9, 2.9 Hz, 12H). 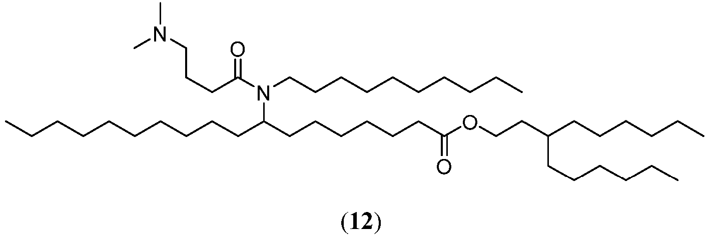 **3-Hexylnonyl 8-(N-decyl-4-(dimethylamino)butanamido)octadecenoate** (**12**). ¹H NMR (400 MHz, CDCl₃) δ 4.10 (td, *J =* 7.1, 3.0 Hz, 2H), 3.06 (q, *J =* 6.7 Hz, 2H), 2.47 (s, 1H), 2.42 - 2.34 (m, 6H), 2.29 (td, *J =* 7.6, 5.1 Hz, 4H), 1.96 - 1.88 (m, 2H), 1.66 - 1.51 (m, 7H), 1.44 (d, *J =* 7.0 Hz, 5H), 1.29 (d, *J =* 9.5 Hz, 56H), 0.90 (td, *J =* 6.9, 2.4 Hz, 12H). 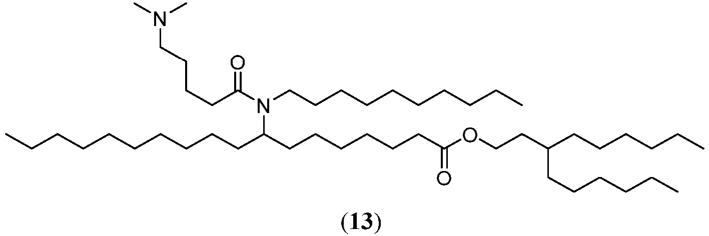 **3-Hexylnonyl 8-(N-decyl-5-(dimethylamino)pentanamido)octadecenoate** (**13**). ¹H NMR (400 MHz, CDCl₃) δ 4.10 (td, *J =* 7.1, 2.7 Hz, 2H), 3.08 - 2.99 (m, 2H), 2.47 (s, 2H), 2.34 (d, *J =* 7.1 Hz, 6H), 2.29 (td, *J =* 7.5, 5.6 Hz, 3H), 1.75 - 1.54 (m, 10H), 1.45 (s, 5H), 1.29 (d, *J* = 9.9 Hz, 56H), 0.90 (td, *J =* 6.8, 2.3 Hz, 12H). 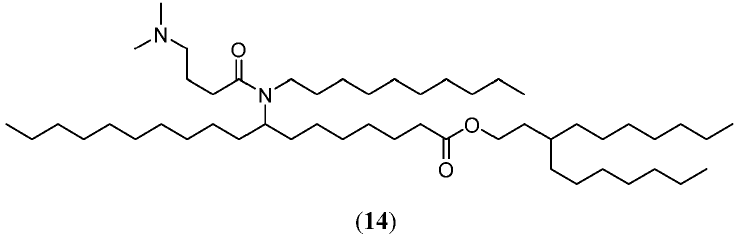 **3-Heptyldecyl 8-(N-decyl-4-(dimethylamino)butanamido)octadecenoate** (**14**). ¹H NMR (400 MHz, CDCl₃) δ 4.09 (td, *J =* 7.1, 2.9 Hz, 2H), 3.06 (q, *J =* 6.8 Hz, 2H), 2.62 (s, 2H), 2.40 (dt, *J* = 9.5, 7.0 Hz, 6H), 2.29 (td, *J =* 7.5, 5.0 Hz, 2H), 1.98 (s, 2H), 1.59 (tq, *J =* 16.8, 8.3 Hz, 6H), 1.44 (d, *J* = 7.2 Hz, 5H), 1.29 (d, *J =* 9.8 Hz, 61H), 0.90 (td, *J =* 6.8, 2.1 Hz, 12H). 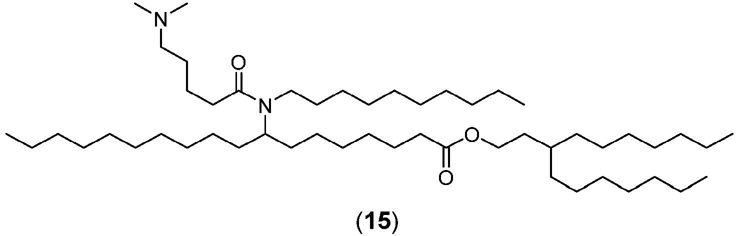 **3-Heptyldecyl 8-(N-decyl-5-(dimethylamino)pentanamido)octadecenoate** (**15**). ¹H NMR (400 MHz, CDCl₃) δ 4.09 (td, *J =* 7.1, 2.6 Hz, 2H), 3.04 (q, *J* = 7.4 Hz, 2H), 2.67 (s, 2H), 2.53 (s, 6H), 2.41 - 2.24 (m, 4H), 1.72 (s, 4H), 1.60 (dq, *J =* 13.8, 7.0 Hz, 7H), 1.49 - 1.38 (m, 5H), 1.29 (d, *J =* 9.3 Hz, 59H), 0.90 (td, *J =* 6.9, 2.2 Hz, 12H). 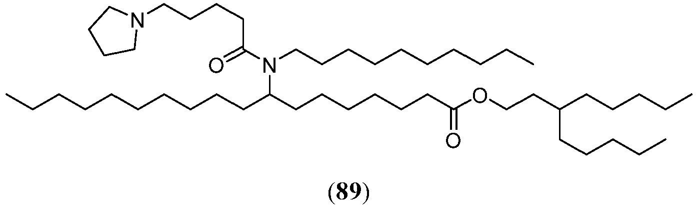 **3-Pentyloctyl 8-(N-decyl-5-(pyrrolidin-1-yl)pentanamido)octadecanoate** (**89**). ¹H NMR (400 MHz, CDCl3) δ 4.10 (td, J = 7.1, 3.1 Hz, 2H), 3.04 (dd, J = 11.3, 7.0 Hz, 2H), 2.54 - 2.42 (m, 6H), 2.30 (ddd, J = 15.2, 7.7, 5.3 Hz, 4H), 1.83 - 1.75 (m, 4H), 1.70 (dq, J = 16.0, 7.7 Hz, 3H), 1.64 - 1.50 (m, 11H), 1.49 - 1.41 (m, 5H), 1.29 (d, J = 11.0 Hz, 53H), 0.90 (td, J = 6.9, 2.8 Hz, 12H). 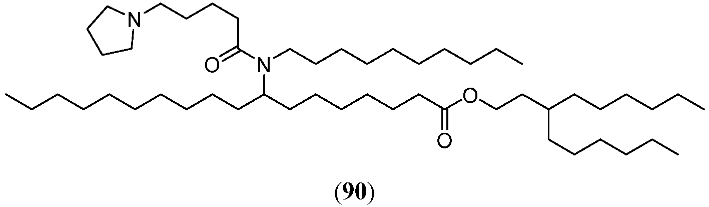 **3-Hexylnonyl 8-(N-decyl-5-(pyrrolidin-1-yl)pentanamido)octadecanoate** (**90**). ¹H NMR (400 MHz, CDCl3) δ 4.10 (td, J = 7.1, 3.1 Hz, 2H), 3.04 (dd, J = 10.9, 6.4 Hz, 2H), 2.48 (ddt, J = 18.2, 7.6, 3.0 Hz, 6H), 2.30 (ddd, J = 15.2, 7.7, 5.3 Hz, 4H), 1.79 (q, J = 3.2 Hz, 4H), 1.71 (dd, J = 10.0, 7.2 Hz, 3H), 1.66 - 1.52 (m, 9H), 1.49 - 1.41 (m, 5H), 1.29 (d, J = 10.0 Hz, 55H), 0.90 (td, J = 6.9, 2.4 Hz, 12H). 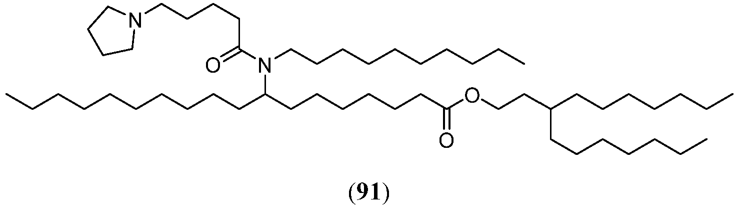 **3-Heptyldecyl 8-(N-decyl-5-(pyrrolidin-1-yl)pentanamido)octadecanoate** (**91**). ¹H NMR (400 MHz, CDCl3) δ 4.10 (td, J = 7.1, 3.1 Hz, 2H), 3.03 (d, J = 12.6 Hz, 2H), 2.54 - 2.42 (m, 6H), 2.30 (ddd, J = 15.3, 7.7, 5.4 Hz, 4H), 1.79 (q, J = 3.6 Hz, 4H), 1.74 - 1.65 (m, 2H), 1.63 - 1.54 (m, 12H), 1.46 (s, 6H), 1.29 (d, J = 10.0 Hz, 54H), 0.90 (td, J = 6.8, 2.3 Hz, 12H). 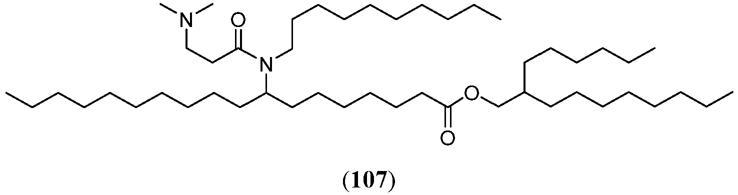 **2-Hexyldecyl 8-(N-decyl-3-(dimethylamino)propanamido)octadecanoate** (**107**). ¹H NMR (400 MHz, CDCl₃) δ 3.98 (dd, *J* = 5.8, 2.7 Hz, 2H), 3.68 - 3.57 (m, 1H), 3.10 - 3.01 (m, 2H), 2.68 (q, *J =* 8.1 Hz, 2H), 2.51 (q, *J* = 7.8 Hz, 2H), 2.35 - 2.26 (m, 8H), 1.79 - 1.40 (m, 12H), 1.28 (d, *J = 6.6* Hz, 64H), 0.90 (q, *J =* 4.2 Hz, 12H). 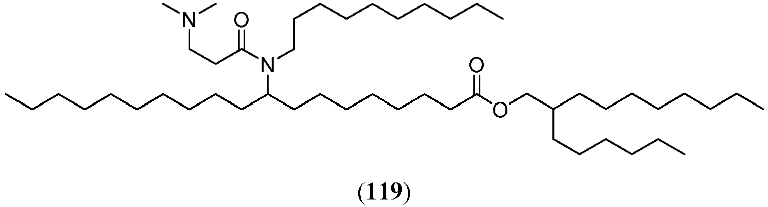 **2-Hexyldecyl 9-(N-decyl-3-(dimethylamino)propanamido)nonadecanoate** (**119**). ¹H NMR (400 MHz, CDCl₃) δ 3.99 (d, *J =* 5.7 Hz, 2H), 3.69 - 3.58 (m, 1H), 3.06 (dd, *J =* 10.8, 5.9 Hz, 2H), 2.70 (dd, *J =* 10.9, 6.4 Hz, 2H), 2.53 (q, *J* = 8.1 Hz, 2H), 2.30 (t, *J* = 4.2 Hz, 8H), 1.71 - 1.11 (m, 77H), 0.90 (t, *J =* 6.6 Hz, 12H).

### Example 2. Synthesis of 3-hexylnonyl 9-(N-decyl-4-(dimethylamino)butanamido)-2,2-difluorooctadecanoate (16)

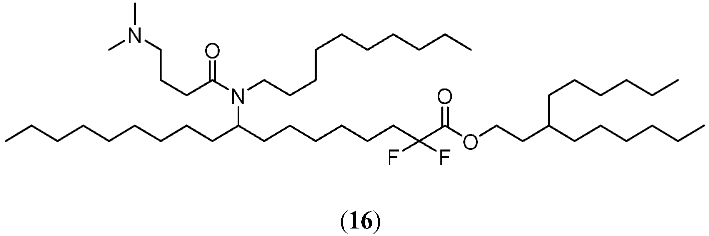

### Step 1. Synthesis of hexadec-1-en-7-ol

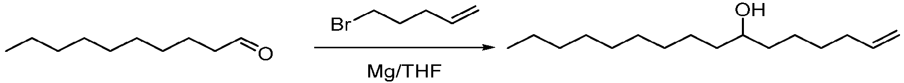

A Grignard reagent was prepared with 6-bromohex-1-ene (70 g, 429 mmol) and magnesium (11.2 g, 459 mmol) in THF (150 mL) and stirred at 45°C for 2 hr. The reaction was cooled to 15°C and decanal (54 g, 343 mmol) in THF (150 mL) added dropwise. After 1 hr the reaction was cooled (0°C, ice bath) and quenched with H₂O followed by 6 M HCl (350 mL). The reaction was extracted with hexane, the organics washed with brine, dried (MgSO₄), filtered and concentrated *in-vacuo.* The residue was purified by automated column chromatography (2-20% EtOAc-hexane) to give hexadec-1-en-7-ol (65 g, Yield 78.7%).

### Step 2. Synthesis of hexadec-1-en-7-one

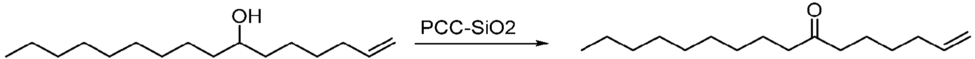

Hexadec-1-en-7-ol (65 g, 270 mmol) and PCC-SiO₂ (1:1) (140 g, 324 mmol) were stirred in DCM (600 mL) at RT for 18 hr. The mixture was partially concentrated *in-vacuo* and eluted through a short bed of silica with EtOAc-hexane (25%, 700 mL). The combined organics were concentrated *in-vacuo* and purified by automated flash chromatography (0-10% EtOAc-hexane) to give hexadec-1-en-7-one (59 g, 91.5%).

### Step 3. Synthesis of decyl(hexadec-1-en-7-yl)amine

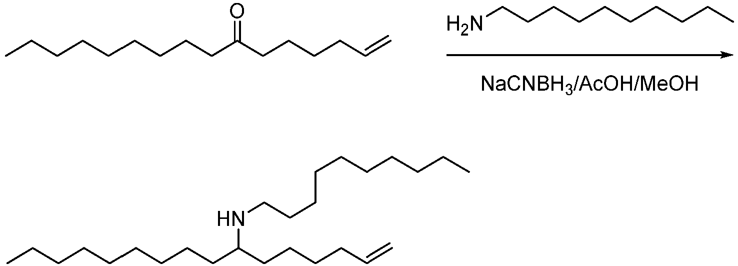

Hexadec-1-en-7-one (59 g, 247 mmol), N-decylamine (40.9 g, 260 mmol), NaCNBH₃ (47 g, 742 mmol) and AcOH (30 g, 495 mmol) were stirred in MeOH at 50°C for 18 hr. The reaction was concentrated *in-vacuo,* the residue taken-up in Et₂O and washed sequentially with NaHCO₃ (sat. soln.) and brine, dried (MgSO₄), filtered and concentrated *in-vacuo.* The crude material was purified by automated flash chromatography (0-10% MeOH-DCM) to give decyl(hexadec-1-en-7-yl)amine (50 g, 53.2%).

### Step 4. Synthesis of N-decyl-4-(dimethylamino)-N-(hexadec-1-en-7-yl)butanamide

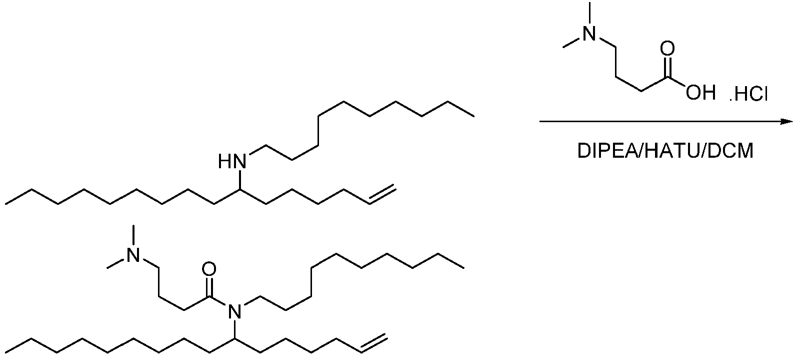

4-Dimethylamino]butanoic acid (HCl salt) (25.4 g, 151 mmol), HATU (60 g, 158 mmol) and DIPEA (46 mL, 263 mmol) were stirred in DCM (500 mL) for 1 hr at RT. Decyl(hexadec-1-en-7-yl)amine (25 g, 66 mmol in DCM (65 mL) was added and the reaction stirred at RT for 18 hr. The reaction was concentrated *in-vacuo,* the residue taken-up in Et₂O and washed sequentially with 1 M NaOH, NaHCO₃ (sat. soln), water and brine, dried (MgSO₄), filtered and concentrated *in-vacuo.* The residue was purified by automated flash chromatography (0-10% MeOH-DCM) to give N-decyl-4-(dimethylamino)-N-(hexadec-1-en-7-yl)butanamide (20 g, 61.6%).

### Step 5. Synthesis of ethyl 9-[N-decyl-4-(dimethylamino)butanamido]-2,2-difluorooctadecanoate

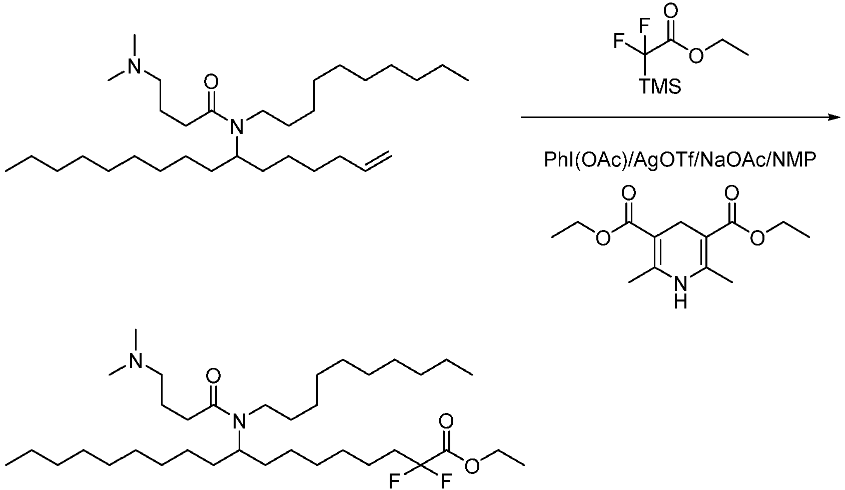

In a vacuum, heat dried flask, Hantsch ester (2.3 g, 9.13 mmol), NaOAc (2.25 g, 27.4 mmol), AgOTf (5.9 g, 22.8 mmol) and iodobenzene diacetate (5.9 g, 18.3 mmo) were added and the flask evacuated and back filled with nitrogen. NMP (15 mL), N-decyl-4-(dimethylamino)-N-(hexadec-1-en-7-yl)butanamide (4.5 g, 9.1 mmol) in NMP and ethyl 2,2-difluoro-2-(trimethylsilyl)acetate (9.0 g, 46 mmol) were added and the reaction stirred at RT for 18 hr. The mixture was filtered through Celite, eluting with Et₂O. The organics were washed with water and brine, dried (MgSO₄), filtered and concentrated *in-vacuo.* The residue was purified by automated flash chromatography (0-10% MeOH-DCM) to give ethyl 9-[N-decyl-4-(dimethylamino)butanamido]-2,2-difluorooctadecanoate (5 g, 80.0 %).

### Step 6. Synthesis of 9-[N-decyl-4-(dimethylamino)butanamido]-2,2-difluorooctadecanoic acid

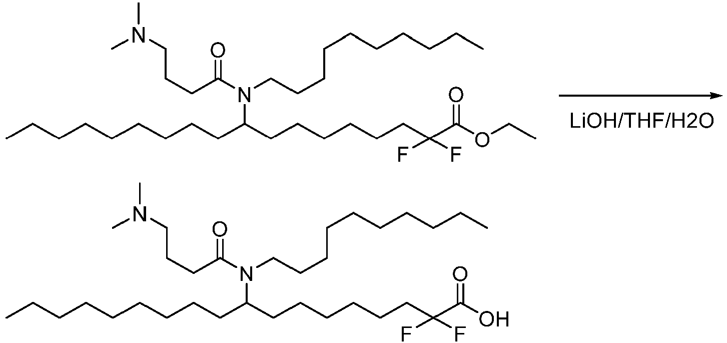

Ethyl 9-[N-decyl-4-(dimethylamino)butanamido]-2,2-difluorooctadecanoate (5 g, 8.1 mmol) and LiOH (0.39 g, 16.2 mmol) were stirred in THF (50 mL) and H₂O (10 mL) at 50°C for 2 hr. The reaction was quenched with 6M HCl (3.0 mL) and partially concentrated. The aqueous residue was diluted with H₂O and extracted with DCM. The combined organics were washed with brine, dried (MgSO₄), filtered and concentrated *in-vacuo.* The residue was azeotroped with toluene (50 mL) to give 9-[N-decyl-4-(dimethylamino)butanamido]-2,2-difluorooctadecanoic acid which was used without purification.

### Step 7. Synthesis of 3-hexylnonyl 9-[N-decyl-4-(dimethylamino)butanamido]-2,2-difluorooctadecanoate

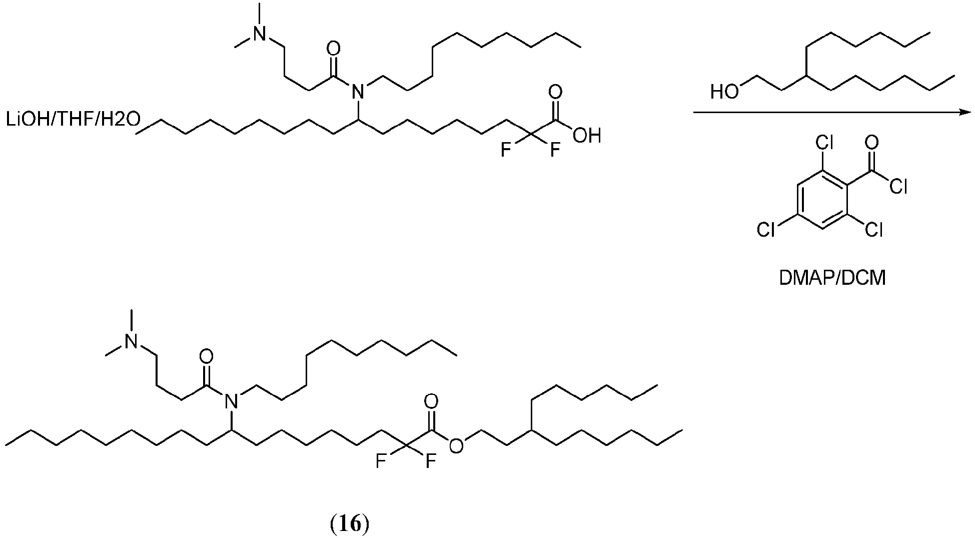

9-[N-Decyl-4-(dimethylamino)butanamido]-2,2-difluorooctadecanoic acid (4 g, 6.8 mmol), 3-hexylnonan-1-ol (1.63 g, 7.1 mmol), 2,4,6 trichlorobenzoyl chloride (1.8 g, 7.5 mmol) and DMAP (1.7 g, 13.6 mmol were stirred in DCM (100 mL) at RT for 3 hr. The reaction was concentrated *in-vacuo* and the residue partitioned between Et₂O and H₂O. The organics were washed with NaHCO₃ (sat. soln.) and brine, dried (MgSO₄), filtered and concentrated *in-vacuo.* The residue was purified by automated flash chromatography (0-12% MeOH-DCM) to give 3-hexylnonyl 9-[N-decyl-4-(dimethylamino)butanamido]-2,2-difluorooctadecanoate (**16**) (2.44 g, 44.9 %).¹H NMR (400 MHz, CDCl3) δ 4.29 (td, J = 7.1, 1.9 Hz, 2H), 3.66 (dd, J = 9.4, 4.8 Hz, 1H), 3.06 (q, J = 7.1 Hz, 2H), 2.41 - 2.31 (m, 4H), 2.27 (s, 6H), 2.13 - 1.96 (m, 2H), 1.87 (h, J = 7.3 Hz, 2H), 1.67 (q, J = 6.9 Hz, 2H), 1.60 - 1.39 (m, 9H), 1.28 (q, J = 6.6 Hz, 56H), 0.95 - 0.84 (m, 12H).¹⁹F NMR (376 MHz, CDCl3) δ -105.9 (2F).

The following compounds were prepared using analogous methodology as described above 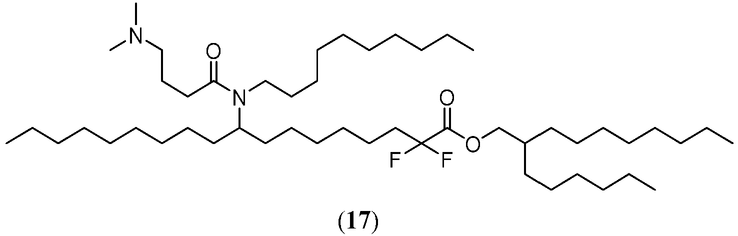 **2-Hexyldecyl 9-(N-decyl-4-(dimethylamino)butanamido)-2,2-difluorooctadecanoate** (**17**). ¹H NMR (400 MHz, CDCl₃) δ 4.18 (dd, *J =* 6.0, 2.0 Hz, 2H), 3.69 - 3.57 (m, 1H), 3.06 (q, *J =* 7.1 Hz, 2H), 2.57 (s, 2H), 2.46 - 2.34 (m, 8H), 2.01 - 1.89 (m, 3H), 1.59 - 1.40 (m, 8H), 1.38 - 1.18 (m, 60H), 0.90 (td, *J =* 6.8, 2.7 Hz, 12H). 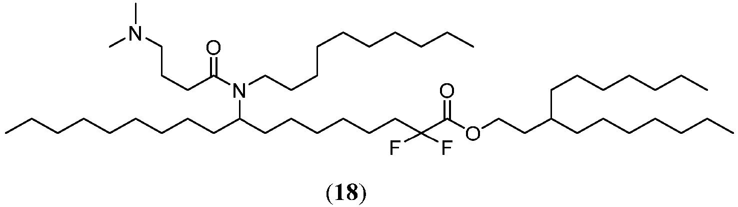 **3-Heptyldecyl 9-(N-decyl-4-(dimethylamino)butanamido)-2,2-difluorooctadecanoate** (**18**). ¹H NMR (400 MHz, CDCl₃) δ 4.29 (td, *J =* 7.1, 1.9 Hz, 2H), 3.71 - 3.56 (m, 1H), 3.06 (q, *J =* 7.0 Hz, 2H), 2.42 - 2.29 (m, 8H), 2.04 (ddt, *J =* 16.8, 11.4, 5.3 Hz, 3H), 1.91 (h, *J* = 7.1 Hz, 3H), 1.67 (q, *J =* 6.9 Hz, 2H), 1.60 - 1.39 (m, 9H), 1.29 (d, *J =* 8.3 Hz, 53H), 0.90 (td, *J =* 6.7, 3.1 Hz, 12H). 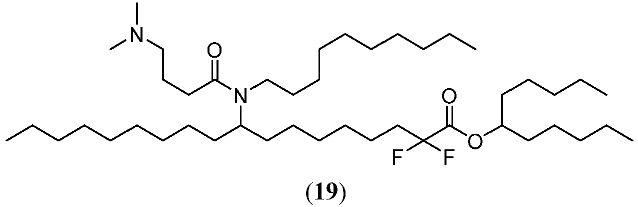 **Undecan-6-yl 9-(N-decyl-4-(dimethylamino)butanamido)-2,2-difluorooctadecanoate** (**19**). ¹H NMR (400 MHz, CDCl₃) δ 3.57 (s, 1H), 3.28 (q, *J =* 7.8 Hz, 2H), 3.06 (dd, *J =* 11.9, 5.3 Hz, 2H), 2.94 (d, *J =* 2.3 Hz, 6H), 2.60 (t, *J =* 5.9 Hz, 1H), 2.53 (t, *J* = 5.9 Hz, 1H), 2.18 - 1.75 (m, 11H), 1.70 - 1.42 (m, 13H), 1.42 - 1.16 (m, 55H), 0.90 (t, *J =* 6.6 Hz, 12H). 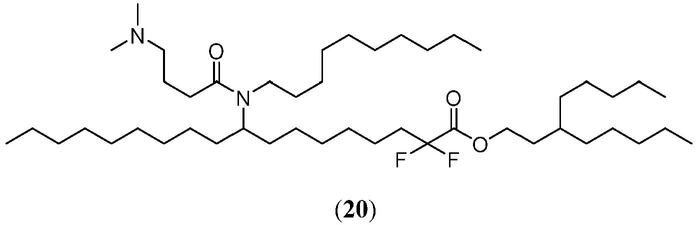 **3-Pentyloctyl 9-(N-decyl-4-(dimethylamino)butanamido)-2,2-difluorooctadecanoate** (**20**). ¹H NMR (400 MHz, CDCl₃) δ 4.29 (td, *J =* 7.1, 1.9 Hz, 2H), 3.71 - 3.60 (m, 1H), 3.06 (q, *J =* 7.4 Hz, 2H), 2.35 (tdd, *J =* 6.6, 4.2, 2.4 Hz, 4H), 2.26 (s, 6H), 2.13 - 1.96 (m, 3H), 1.85 (h, *J =* 7.4 Hz, 2H), 1.68 (q, *J =* 6.8 Hz, 2H), 1.60 - 1.40 (m, 9H), 1.38 - 1.19 (m, 49H), 0.94 - 0.86 (m, 12H). 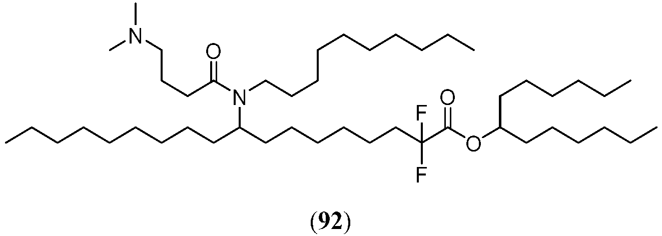 **Tridecan-7-yl 9-(N-decyl-4-(dimethylamino)butanamido)-2,2-difluorooctadecanoate** (**92**). ¹H NMR (400 MHz, CDCl₃) δ 5.02 (p, *J* = 6.8 Hz, 1H), 3.65 (t, *J =* 7.2 Hz, 1H), 3.06 (q, *J* = 7.1 Hz, 2H), 2.40 - 2.31 (m, 4H), 2.29 (s, 6H), 2.16 - 1.94 (m, 2H), 1.68 - 1.10 (m, 65H), 0.90 (t, *J* = 6.7 Hz, 12H). 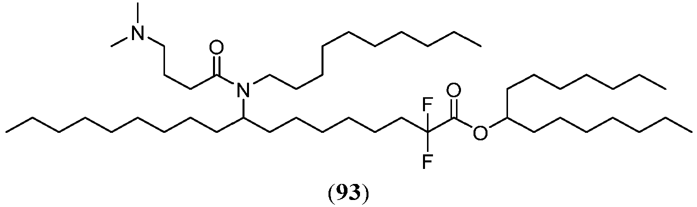 **Pentadecan-8-yl 9-(N-decyl-4-(dimethylamino)butanamido)-2,2-difluorooctadecanoate** (**93**). ¹H NMR (400 MHz, CDCl₃) δ 5.09 - 4.94 (m, 1H), 3.65 (t, *J* = 7.2 Hz, 1H), 3.06 (q, *J =* 7.0 Hz, 2H), 2.37 (dt, *J =* 14.7, 7.7 Hz, 4H), 2.30 (s, 6H), 2.20 - 1.97 (m, 2H), 1.88 (h, *J =* 7.2 Hz, 2H), 1.67 - 1.08 (m, 68H), 0.90 (t, *J =* 6.7 Hz, 12H). 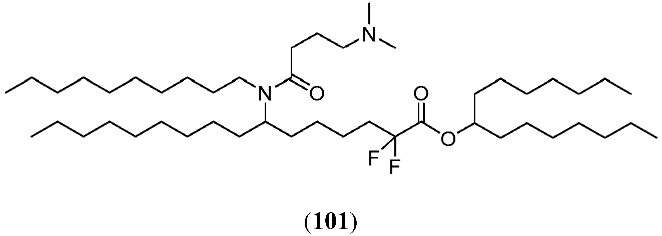 **Pentadecan-8-yl 7-(N-decyl-4-(dimethylamino)butanamido)-2,2-difluorohexadecanoate** (**101**). ¹H NMR (400 MHz, CDCl₃) δ 5.01 (q, *J* = 5.8 Hz, 1H), 3.78 - 3.57 (m, 1H), 3.15 - 2.94 (m, 2H), 2.58 - 2.17 (m, 10H), 2.04 (tq, *J =* 16.1, 7.6 Hz, 2H), 1.88 (h, *J =* 7.5 Hz, 2H), 1.73 - 1.01 (m, 66H), 0.90 (t, *J =* 6.6 Hz, 12H). 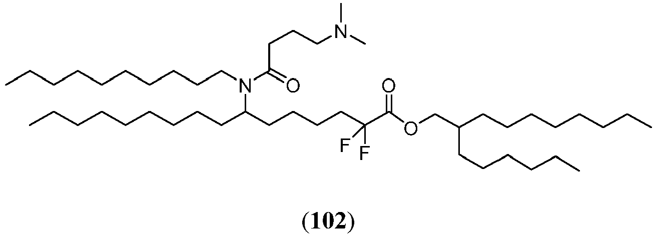 **2-Hexyldecyl 7-(N-decyl-4-(dimethylamino)butanamido)-2,2-difluorohexadecanoate** (**102**). ¹H NMR (400 MHz, CDCl₃) δ 4.18 (dd, *J =* 5.7, 3.2 Hz, 2H), 3.71 - 3.61 (m, 0H), 3.05 (dd, *J =* 10.6, 6.2 Hz, 2H), 2.59 - 2.27 (m, 9H), 2.06 (dp, *J* = 16.6, 8.1 Hz, 1H), 1.92 (q, *J* = 7.2 Hz, 2H), 1.73 (s, 1H), 1.59 - 1.40 (m, 8H), 1.29 (dt, *J =* 10.2, 6.3 Hz, 53H), 0.90 (td, *J =* 6.7, 2.7 Hz, 12H). 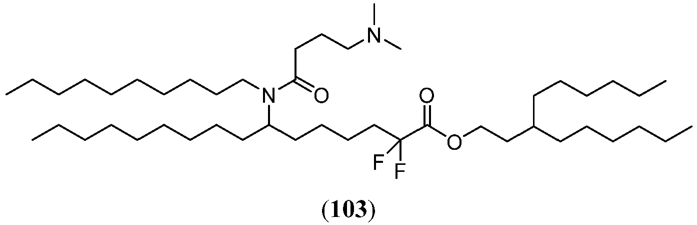 **3-Hexylnonyl 7-(N-decyl-4-(dimethylamino)butanamido)-2,2-difluorohexadecanoate** (**103**). ¹H NMR (400 MHz, CDCl₃) δ 4.29 (td, *J =* 7.1, 2.9 Hz, 2H), 3.65 (d, *J =* 8.4 Hz, 1H), 3.05 (dd, *J =* 10.5, 6.2 Hz, 2H), 2.49 (s, 2H), 2.45 - 2.31 (m, 7H), 2.04 (tt, *J =* 16.5, 8.2 Hz, 2H), 1.91 (p, *J* = 7.0 Hz, 2H), 1.72 - 1.62 (m, 2H), 1.59 - 1.40 (m, 13H), 1.29 (d, *J =* 8.3 Hz, 53H), 0.90 (td, *J =* 6.8, 3.0 Hz, 12H). 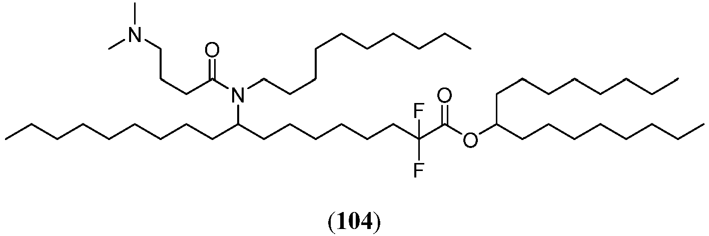 **Heptadecan-9-yl 9-(N-decyl-4-(dimethylamino)butanamido)-2,2-difluorooctadecanoate** (**104**). ¹H NMR (400 MHz, CDCl₃) δ 5.06 - 4.96 (m, 1H), 3.74 - 3.50 (m, 1H), 3.06 (q, *J =* 7.1 Hz, 2H), 2.64 - 2.26 (m, 9H), 2.14 - 1.89 (m, 4H), 1.73 - 1.12 (m, 71H), 0.90 (t, *J =* 6.8 Hz, 12H). 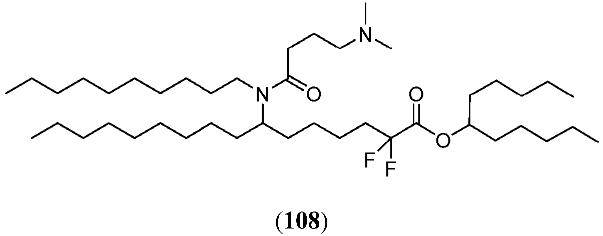 **Undecan-6-yl 7-(N-decyl-4-(dimethylamino)butanamido)-2,2-difluorohexadecanoate** (**108**). ¹H NMR (400 MHz, CDCl₃) δ 5.01 (d, *J =* 8.0 Hz, 1H), 3.67 (s, 0H), 3.05 (d, *J =* 10.4 Hz, 2H), 2.35 (q, *J =* 7.7 Hz, 4H), 2.27 (s, 6H), 2.05 (dt, *J =* 16.2, 8.2 Hz, 2H), 1.86 (h, *J =* 7.7 Hz, 2H), 1.67 - 1.42 (m, 8H), 1.40 - 1.24 (m, 41H), 0.94 - 0.86 (m, 12H). 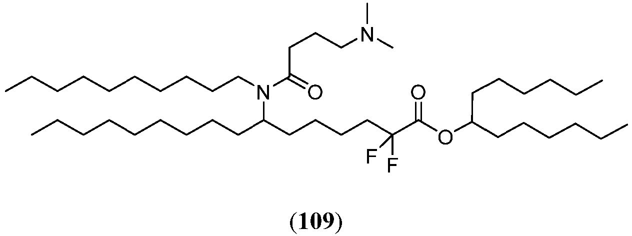 **Tridecan-7-yl 7-(N-decyl-4-(dimethylamino)butanamido)-2,2-difluorohexadecanoate** (**107**). ¹H NMR (400 MHz, CDCl₃) δ 5.14 - 4.93 (m, 1H), 3.05 (dd, *J =* 11.0, 6.0 Hz, 2H), 2.35 (q, *J=* 7.4 Hz, 3H), 2.27 (s, 5H), 2.03 (tt, *J =* 16.2, 8.2 Hz, 2H), 1.87 (h, *J* = 7.5 Hz, 2H), 1.74 - 1.41 (m, 10H), 1.34 - 1.21 (m, 47H), 0.90 (t, *J =* 6.6 Hz, 12H). 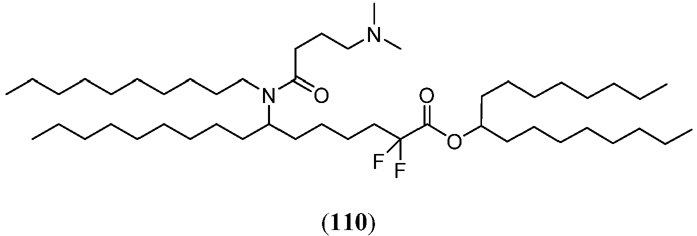 **Heptadecan-9-yl 7-(N-decyl-4-(dimethylamino)butanamido)-2,2-difluorohexadecanoate** (**110**). ¹H NMR (400 MHz, CDCl₃) δ 5.01 (d, *J* = 7.4 Hz, 1H), 3.05 (dd, *J* = 10.9, 6.1 Hz, 2H), 2.52 (s, 2H), 2.39 (d, *J =* 6.2 Hz, 7H), 2.15 - 1.87 (m, 3H), 1.73 - 1.40 (m, 10H), 1.29 (d, *J* = 8.5 Hz, 54H), 0.90 (t, *J =* 6.7 Hz, 12H).

### Example 3. Synthesis of 2-hexyldecyl 3-{6-[(2-butyloctyl)oxy]-N-[3-(dimethylamino)propyl]-6-oxohexanamido}dodecanoate (21)

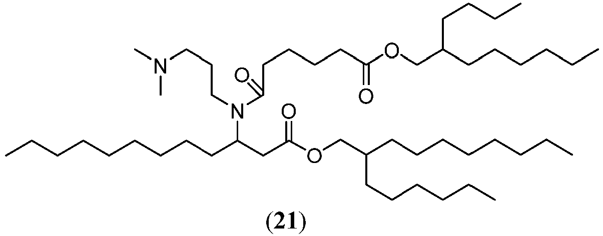

### Step 1. Synthesis of methyl 3-oxododecanoate

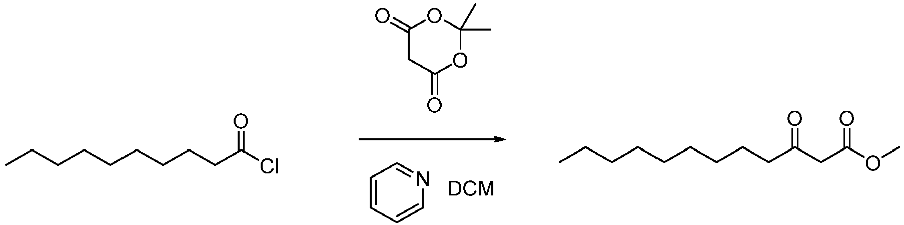

Meldrum's acid (15 g, 104 mmol) and pyridine (16.5 g, 208 mmol) were stirred in DCM (250 mL) at 0°C. Decanoyl chloride (21.6 mL, 104 mmol) was added dropwise and the reaction stirred for 16 hr allowing to warm to RT. The reaction was washed with 1M HCl, dried (MgSO₄), filtered and concentrated *in-vacuo.* The residue was refluxed in MeOH (200 mL) for 3.5 hr then concentrated *in-vacuo.* The residue was purified by automated flash chromatography (2-20% EtOAc-hexane) to give methyl 3-oxododecanoate (17.7 g, 74.4%).

### Step 2. Synthesis of 3-{[3-(dimethylamino)propyl]amino}dodecanoate

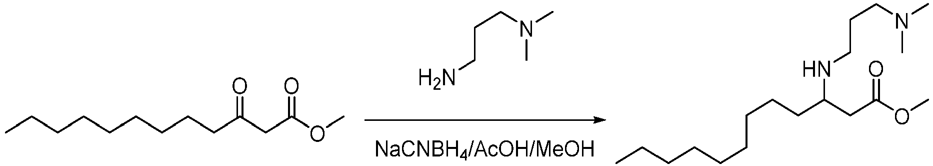

Methyl 3-oxododecanoate (7.92 g, 34.7 mmol), DMAP (4.08 g, 40.0 mmol) and AcOH (7.9 mL, 139 mmol) were heated at reflux in MeOH (285 mL) for 1 hr, then cooled to 0°C and treated portion wise with NaCNBH₃ (4.9 g, 69 mmol). The reaction was stirred at RT for 40 hr, partially concentrated *in-vacuo,* the residue taken up in EtOAc and washed sequentially with NaHCO₃ (sat. soln.) and brine, dried (MgSO₄), filtered and concentrated *in-vacuo.* The crude material was purified by automated flash chromatography (0-12% MeOH-DCM) to give 3-{[3-(dimethylamino)propyl]amino}dodecanoate (1.9 g, 17.4%).

### Step 3. Synthesis of methyl 3-{[(benzyloxy)carbonyl][3-(dimethylamino)-propyl]amino}dodecanoate

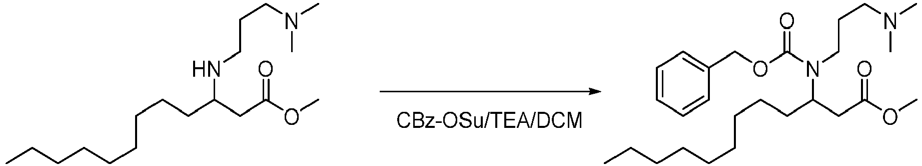

Methyl 3-{[3-(dimethylamino)propyl]amino}dodecanoate (1.2 g, 3.8 mmol), CBz-OSu (1.0 g, 4.2 mmol) and TEA (0.8 mL, 5.7 mmol) were stirred in DCM (35 mL) at RT for 18 hr. The organics were washed with NaHCO₃ (sat. soln.) and brine, dried (MgSO₄), filtered and concentrated *in-vacuo.* The crude material was purified by automated flash chromatography (50:50 EtOAc/Hex) to give methyl 3-{[(benzyloxy)carbonyl][3-(dimethylamino)propyl]amino}dodecanoate (1.6 g, 93.5%).

### Step 4. Synthesis of 3-(((benzyloxy)carbonyl)(3-(dimethylamino)propyl)amino)dodecanoic acid

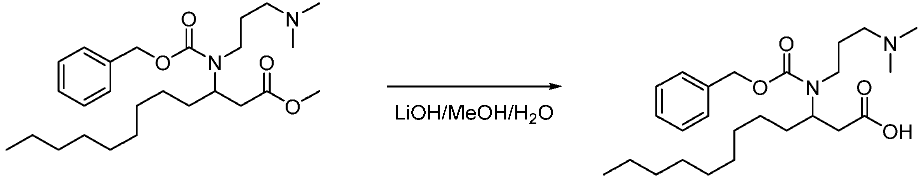

Methyl 3-{[(benzyloxy)carbonyl][3-(dimethylamino)propyl]amino}dodecanoate (1.68 g, 3.7 mmol) and LiOH (0.18 g, 7.5 mmol) were heated at 50°C in MeOH/H₂O (20:3) for 18 hr. The reaction was cooled to RT, quenched with 1M HCl (1.3 mL) and concentrated *in-vacuo.* The residue was taken-up in DCM, washed with brine, dried (MgSO₄), filtered and concentrated *in-vacuo* to give 3-(((benzyloxy)carbonyl)(3-(dimethylamino)propyl)amino)dodecanoic acid which was used without purification.

### Step 5. Synthesis of 2-hexyldecyl 3-{[(benzyloxy)carbonyl][3-(dimethylamino)-propyl]amino}dodecanoate

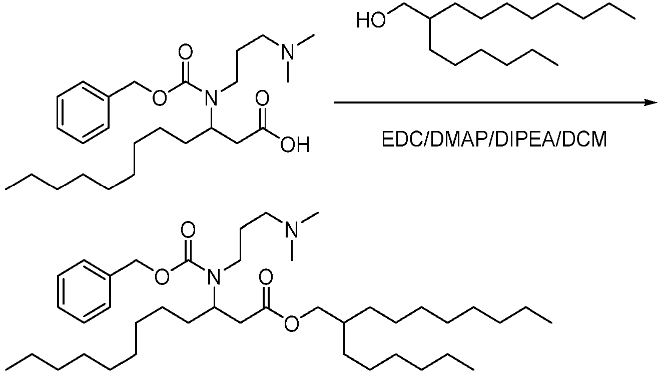

3-{[(Benzyloxy)carbonyl][3-(dimethylamino)propyl]amino } dodecanoic acid (1.2 g, 2.8 mmol), hexyldecanol (0.72 g, 3.0 mmol), EDC (1.04 g, 5.4 mmol), DIPEA (1.4 mL, 8.1 mmol) and DMAP (cat.) were stirred in DCM (30 mL) at RT for 18hr. The reaction was washed with NaHCO₃ (sat. soln.) and brine, dried (MgSO₄), filtered and concentrated *in-vacuo.* The crude material was purified by automated flash chromatography to give 2-hexyldecyl 3-{[(benzyloxy)carbonyl][3-(dimethylamino)propyl]amino}dodecanoate (827 mg, 46.2%).

### Step 6. Synthesis of 2-hexyldecyl 3-{[3-(dimethylamino)propyl]amino}dodecanoate

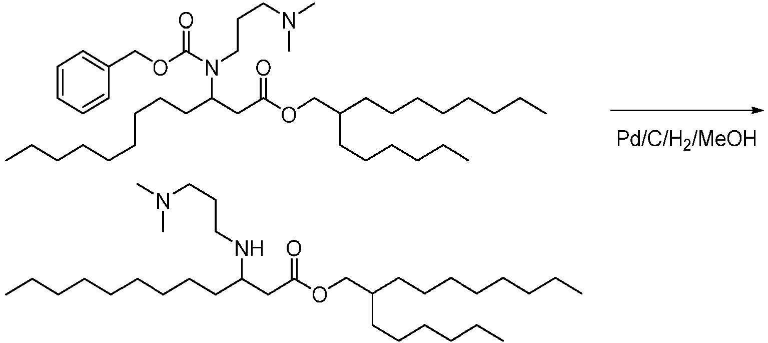

2-Hexyldecyl 3-{[(benzyloxy)carbonyl][3-(dimethylamino)propyl]amino}dodecanoate (874 mg, 1.3 mmol) was subjected to catalytic hydrogenation over 10% Pd/C in MeOH (5 mL) for 18 hr. The reaction was filtered through Celite, concentrated *in-vacuo* and the crude purified by automated flash chromatography (0-15%MeOH/DCM) to give 2-hexyldecyl 3-{[3-(dimethylamino)propyl]amino}dodecanoate (294 mg, 42.2%).

### Step 7. Synthesis of 2-hexyldecyl 3-{6-[(2-butyloctyl)oxy]-N-[3-(dimethylamino)propyl]-6-oxohexanamido}dodecanoate (21)

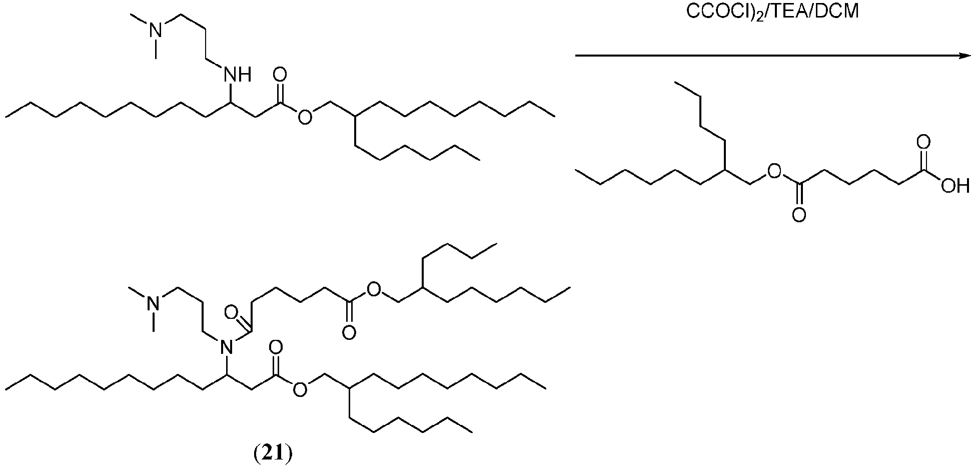

6-[(2-Butyloctyl)oxy]-6-oxohexanoic acid (252 mg, 0.8 mmol) and oxalyl chloride (0.20 g, 1.7 mmol) were stirred at RT for 3 hr. The reaction was concentrated *in-vacuo* and the residue re-dissolved in DCM (5 mL). The acid chloride intermediate was added to 2-hexyldecyl 3-{[3-(dimethylamino)propyl]amino}dodecanoate (295 mg, 0.6 mmol) and TEA (0.14 ml, 1.6 mmol) in DCM (5 mL) and stirred at RT for 2 hr. The reaction was diluted with DCM, washed with NaHCO₃ (sat. soln) and brine, dried (MgSO4), filtered and concentrated *in-vacuo.* The crude material was purified by automated flash chromatography (0-10 %MeOH/DCM) to give 2-hexyldecyl 3-{6-[(2-butyloctyl)oxy]-N-[3-(dimethylamino)propyl]-6-oxohexanamido}dodecanoate (**21**) (231 mg, 50.%). ¹H NMR (400 MHz, CDCl₃) δ 4.23 (s, 1H), 3.95 (q, *J =* 5.3 Hz, 4H), 3.22 (q, *J =* 8.2 Hz, 2H), 3.04 (s, 1H), 2.52 (td, *J =* 15.1, 6.8 Hz, 2H), 2.40 - 2.23 (m, 6H), 2.21 (s, 6H), 1.79 - 1.55 (m, 11H), 1.25 (q, *J =* 4.8 Hz, 53H), 0.88 (td, *J =* 6.7, 3.9 Hz, 15H).

The following compound was prepared using analogous methodology as described above. 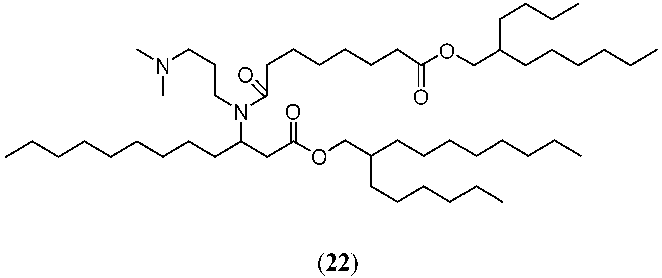 **2-Hexyldecyl 3-(8-((2-butyloctyl)oxy)-N-(3-(dimethylamino)propyl)-8-oxooctanamido)-dodecanoate** (**22**). ¹H NMR (400 MHz, CDCl₃) δ 4.27 (s, 1H), 3.97 (t, *J* = 6.4 Hz, 4H), 3.24 (dq, *J =* 12.2, 6.8 Hz, 2H), 3.07 (d, *J = 4.7* Hz, 1H), 2.62 - 2.45 (m, 2H), 2.36 - 2.25 (m, 5H), 2.24 (s, 6H), 1.71 - 1.59 (m, 8H), 1.29 (q, *J =* 4.6 Hz, 57H), 0.91 (td, *J =* 6.7, 3.9 Hz, 15H).

### Example 4. Synthesis of Synthesis of tridecyl 6-{6-[(2-butyloctyl)oxy]-N-[2-(dimethylamino)ethyl]-6-oxohexanamido}hexadecanoate (23)

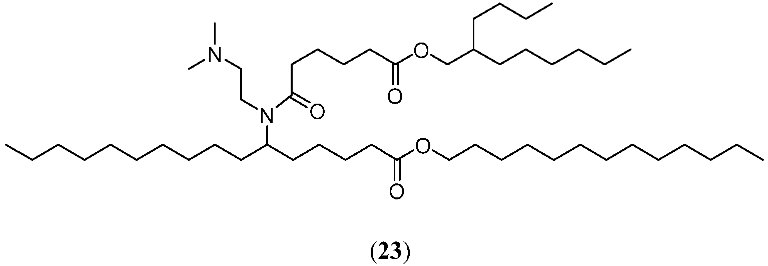

### Step 1. Synthesis of tridecyl 6-{[2-(dimethylamino)ethyl]amino}hexadecanoate

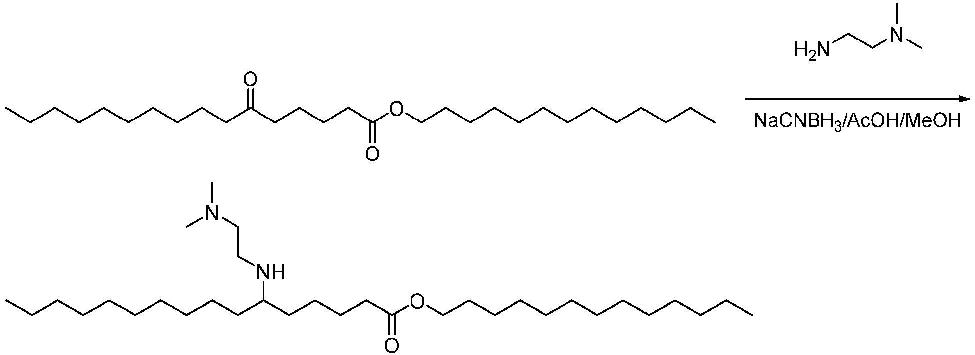

Tridecyl 6-oxohexadecanoate (377 mg, 0.83 mmol) 1,1-dimethylethylenediamine (81 mg, 0.92 mmol) AcOH (0.1 mL, 1.7 mmol) and NaCNBH₃ (157 mg, 2.5 mmol) were stirred in MeOH at 50°C for 2 hr. The reaction was removed from heat and stirring continued for an additional 30 hr. The reaction was partially concentrated *in-vacuo* then dissolved in DCM, washed (NaHCO₃ and brine), dried (MgSO₄), filtered and concentrated *in-vacuo.* The crude material was purified by automated flash chromatography (0-15% MeOH-DCM) to give tridecyl 6-{[2-(dimethylamino)ethyl]amino}hexadecanoate (201 mg, 46.0 %).

### Step 2. Synthesis of 6-{6-[(2-butyloctyl)oxy]-N-[2-(dimethylamino)ethyl]-6-oxohexanamido}hexadecanoate (23)

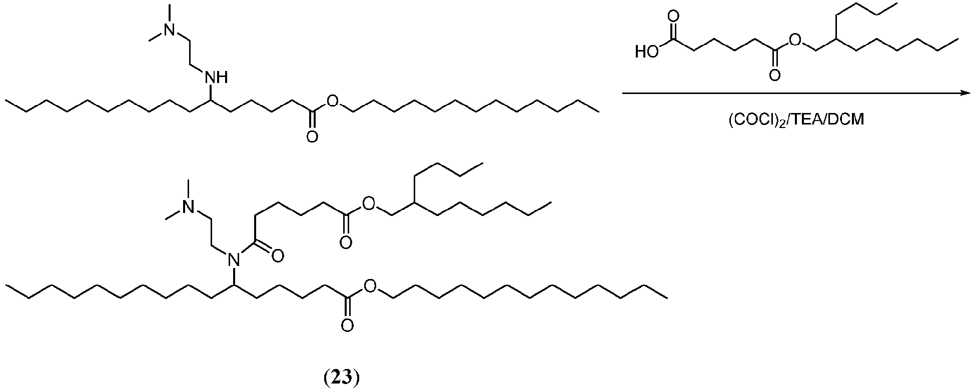

6-[(2-Butyloctyl)oxy]-6-oxohexanoic acid (0.2 g, 0.64 mmol) and oxalyl chloride (0.15 mL, 1.74 mmol) were stirred in DCM (10 mL) with DMF (1 drop) at RT for 3 hr. The reaction was concentrated *in-vacuo,* the residue taken-up in DCM (5 mL) and added to tridecyl 6-{[2-(dimethylamino)ethyl]amino}hexadecanoate (303 mg, 0.6 mmol) and TEA (0.32 mL, 2.3 mmol) in DCM (10 mL). After stirring at RT for 1 hr the reaction was diluted with DCM, washed (NaHCO₃ (sat. aq.) and brine), dried (MgSO₄), filtered and concentrated *in-vacuo.* The crude material was purified by automated flash chromatography (0-12% MeOH-DCM) to give tridecyl 6-{6-[(2-butyloctyl)oxy]-N-[2-(dimethylamino)ethyl]-6-oxohexanamido}hexadecanoate (**23**) (113 mg, 23.8 %). ¹H NMR (400 MHz, CDCl₃) δ 4.07 (td, *J =* 6.8, 4.4 Hz, 2H), 3.99 (d, *J* = 5.8 Hz, 2H), 3.31 (d, *J =* 8.4 Hz, 1H), 2.58 (s, 1H), 2.47 - 2.25 (m, 12H), 1.74 - 1.58 (m, 9H), 1.49 (td, *J =* 11.9, 7.6 Hz, 4H), 1.39 - 1.16 (m, 52H), 0.91 (m, 12H).

The following compounds were prepared using analogous methodology as described above. 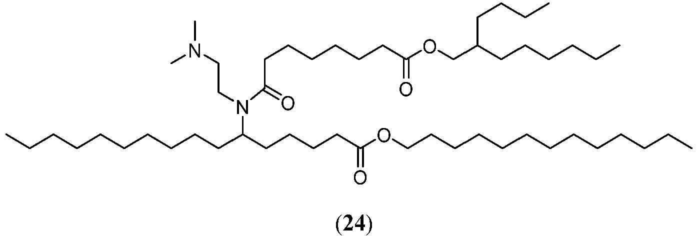 **Tridecyl 6-(8-((2-butyloctyl)oxy)-N-(2-(dimethylamino)ethyl)-8-oxooctanamido)-hexadecanoate** (**24**). ¹H NMR (400 MHz, CDCl₃) δ 4.07 (td, *J* = 6.8, 4.4 Hz, 2H), 3.99 (d, *J* = 5.8 Hz, 2H), 3.31 (d, *J =* 8.4 Hz, 1H), 2.58 (s, 1H), 2.47 - 2.25 (m, 12H), 1.74 - 1.58 (m, 9H), 1.49 (td, *J =* 11.9, 7.6 Hz, 4H), 1.39 - 1.16 (m, 52H), 0.91 (m, 12H). 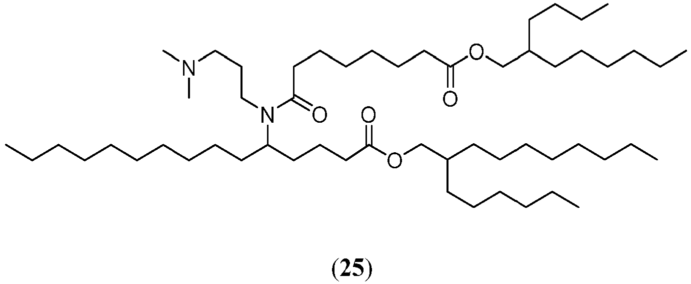 **2-Hexyldecyl 5-(8-((2-butyloctyl)oxy)-N-(3-(dimethylamino)propyl)-8-oxooctanamido)-pentadecanoate** (**25**). ¹H NMR (400 MHz, CDCl₃) δ 3.99 (dd, *J* = 5.8, 2.1 Hz, 4H), 3.14 (dd, *J =* 11.0, 5.8 Hz, 2H), 2.44 - 2.22 (m, 13H), 1.71 - 1.60 (m, 7H), 1.48 (s, 6H), 1.40 - 1.16 (m, 60H), 0.91 (td, *J =* 6.7, 4.0 Hz, 15H). 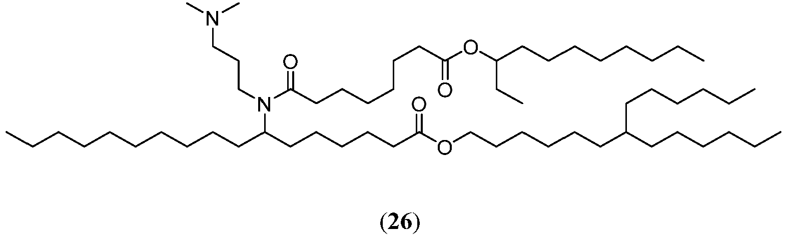 **7-Hexyltridecyl 7-(N-(3-(dimethylamino)propyl)-8-oxo-8-(undecan-3-yloxy)octanamido)-heptadecanoate** (**26**). ¹H NMR (400 MHz, CDCl₃) δ 4.12 - 4.02 (m, 3H), 2.46 - 2.15 (m, 14H), 1.59 (dt, *J =* 34.1, 5.6 Hz, 28H), 1.42 - 1.16 (m, 58H), 0.98 - 0.79 (m, 15H). 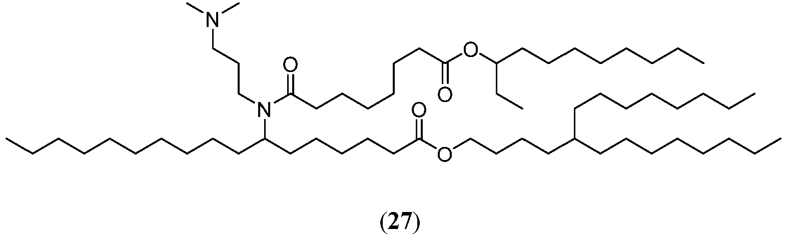 **5-Octyltridecyl 7-(N-(3-(dimethylamino)propyl)-8-oxo-8-(undecan-3-yloxy)octanamido)-heptadecanoate** (**27**). ¹H NMR (400 MHz, CDCl₃) δ 3.69 - 3.63 (m, 1H), 3.36 - 3.29 (m, 2H), 2.36 - 2.27 (m, 2H), 2.15 - 1.96 (m, 14H), 1.85 - 1.48 (m, 44H), 1.46 - 1.16 (m, 44H), 0.89 (t, *J* = 6.7 Hz, 15H). 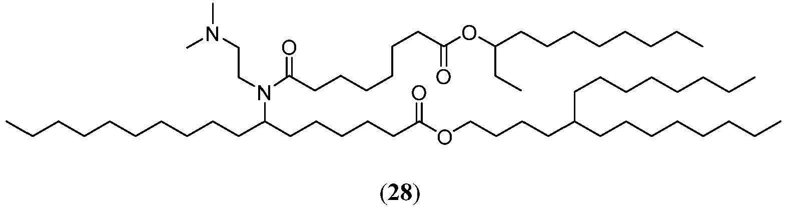 **5-Octyltridecyl 7-(N-(2-(dimethylamino)ethyl)-8-oxo-8-(undecan-3-yloxy)octanamido)-heptadecanoate** (**28**). ¹H NMR (400 MHz, CDCl₃) δ 3.69 - 3.63 (m, 1H), 3.35 - 3.28 (m, 2H), 2.18 - 1.93 (m, 15H), 1.89 - 1.82 (m, 2H), 1.71 - 1.55 (m, 41H), 1.43 - 1.19 (m, 44H), 0.89 (t, *J* = 6.7 Hz, 15H). 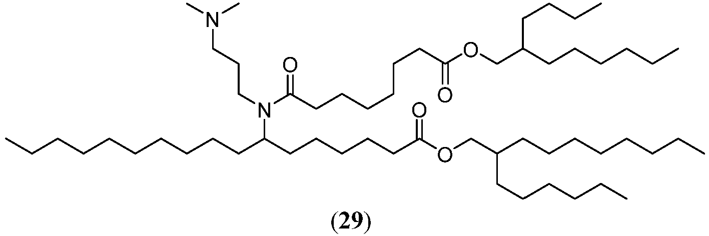 **2-Hexyldecyl 7-(8-((2-butyloctyl)oxy)-N-(3-(dimethylamino)propyl)-8-oxooctanamido)-heptadecanoate** (**29**). ¹H NMR (400 MHz, CDCl₃) δ 4.03 - 3.94 (m, 4H), 3.67 - 3.61 (m, 1H), 3.18 - 3.09 (m, 2H), 2.42 - 2.12 (m, 12H), 1.81 - 1.41 (m, 24H), 1.41 - 1.03 (m, 56H), 0.90 (t, *J* = 3.2 Hz, 15H). 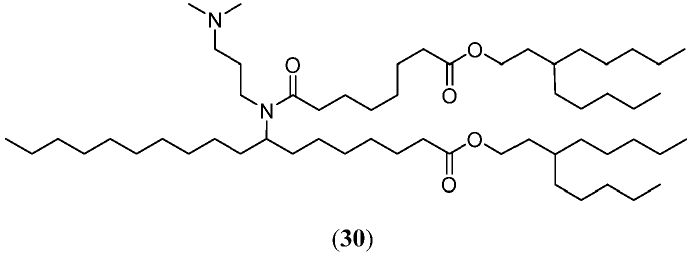 **3-Pentyloctyl 8-(N-(3-(dimethylamino)propyl)-8-oxo-8-((3-pentyloctyl)oxy)-octanamido)octadecenoate** (**30**). ¹H NMR (400 MHz, CDCl₃) δ 4.14 - 4.07 (m, 4H), 3.67 - 3.61 (m, 1H), 3.19 - 3.11 (m, 2H), 2.36 - 2.21 (m, 10H), 1.72 - 1.53 (m, 22H), 1.53 - 1.39 (m, 6H), 1.39 - 1.18 (m, 52H), 0.97 - 0.84 (m, 15H). 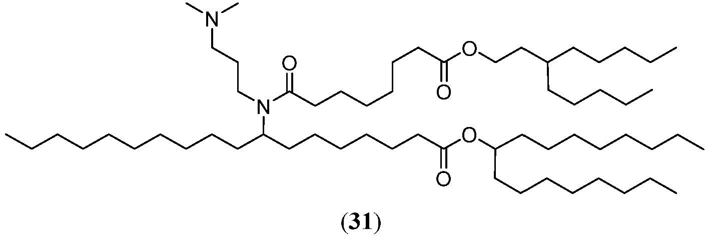 **Heptadecan-9-yl 8-(N-(3-(dimethylamino)propyl)-8-oxo-8-((3-pentyloctyl)oxy)-octanamido)octadecenoate** (**31**). ¹H NMR (400 MHz, CDCl₃) δ 4.91 - 4.85 (m, 1H), 4.10 (t, *J* = 7.1 Hz, 2H), 2.42 - 2.19 (m, 12H), 2.04 - 1.83 (m, 12H), 1.77 - 1.46 (m, 18H), 1.39 - 1.13 (m, 60H), 0.96 - 0.82 (m, 15H).

### Example 5. Synthesis of Synthesis of 2-hexyldecyl 3-[N-decyl-4-(dimethylamino)-butanamido]dodecanoate (32)

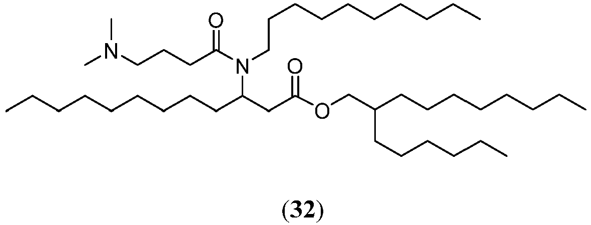

### Step 1. Synthesis of methyl 3-(decylamino)dodecanoate

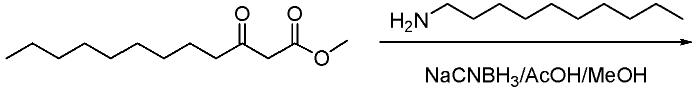

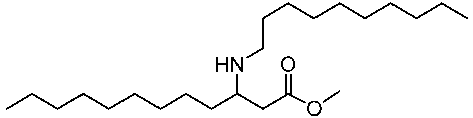

Methyl 3-oxododecanoate (1 g, 4.4 mmol), N-decylamine (723 mg, 5.0 mmol), NaCNBH₃ (826 mg, 13.1 mmol) and AcOH (0.5 mL, 8.8 mmol) were stirred in MeOH in methanol at 50°C for 18 hr. The reaction was cooled to RT and partially concentrated *in-vacuo.* The mixture was taken-up in DCM, washed with NaHCO₃ (sat. aq.) and brine, dried (MgSO₄), filtered and concentrated *in-vacuo.* The crude material was purified by automated flash chromatography (0-12% MeOH-DCM) to give methyl 3-(decylamino)dodecanoate (560 mg, 34.6%).

### Step 2. Synthesis of methyl 3-[N-decyl-4-(dimethylamino)butanamido]dodecanoate

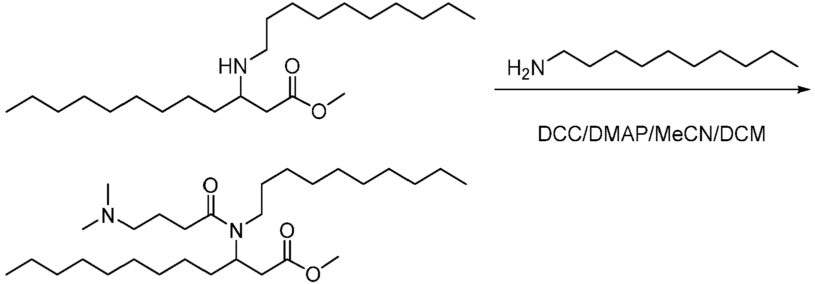

4-Dimethylamino]butanoic acid HCl salt (508 mg, 3.03 mmol), DCC (688 mg, 3.3 mmol) and DMAP (555 mg, 4.5 mmol) were stirred in MeCN (5 mL) at RT for 2 hr. Methyl 3-(decylamino)dodecanoate (560 mg, 1.5 mmol) in DCM (5 mL) was added and stirring continued for 72 hr. The reaction was filtered through Celite, rinsed with DCM and the filtrate washed with NaHCO₃ (sat. aq.) and brine, dried (MgSO₄), filtered and concentrated *in-vacuo.* The crude material was purified by automated flash chromatography (0-10% MeOH-DCM) to give methyl 3-[N-decyl-4-(dimethylamino)butanamido]dodecanoate (498 mg, 68.1 %).

### Step 3. Synthesis of 3-[N-decyl-4-(dimethylamino)butanamido]dodecanoic acid

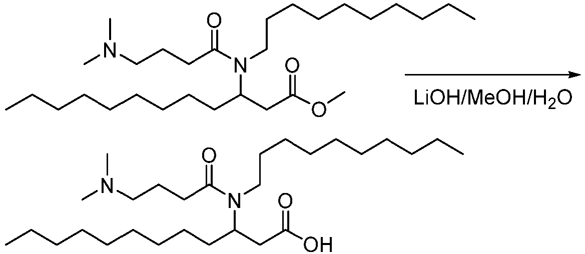

Methyl 3-[N-decyl-4-(dimethylamino)butanamido]dodecanoate (498 mg, 1.0 mmol) and LiOH (49 mg, 2.1 mmol) were stirred in MeOH (3.0 mL) and H₂O water (0.5 mL) at 50°C for 2 hr. The reaction was cooled to RT and quenched with 6 M HCl (0.35 mL). The reaction was partially concentrated *in-vacuo* then partitioned between H₂O water and DCM. The aqueous layer was extracted with DCM and the combined organics washed with brine, dried (MgSO₄), filtered and concentrated *in-vacuo* to give 3-[N-decyl-4-(dimethylamino)butanamido]dodecanoic acid (409 mg, 84.6 %) which was used without further purification.

### Step 4. Synthesis of 2-hexyldecyl 3-[N-decyl-4-(dimethylamino)butanamido]dodecanoate (32)

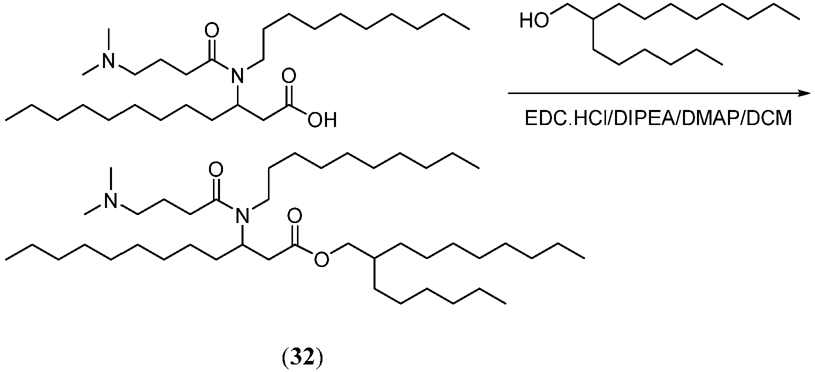

3-[N-Decyl-4-(dimethylamino)butanamido]dodecanoic acid (369 mg, 0.8 mmol), hexyldecanol (229 mg, 0.95 mmol), EDC (302 mg, 1.6 mmol), DIPEA (0.411 mL, 2.4 mmol) and DMAP (cat.) were stirred in DCM (5 mL) at RT for 18 hr. The reaction was washed with NaHCO₃ (sat. aq.) and brine, dried (MgSO₄), filtered and concentrated *in-vacuo.* The crude material was purified by automated flash chromatography (0-12% MeOH-DCM) to give 2-hexyldecyl 3-[N-decyl-4-(dimethylamino)butanamido]dodecanoate (32). (88 mg, 16.1 %). ¹H NMR (400 MHz, CDCl₃) δ 4.26 (s, 1H), 4.03 - 3.93 (m, 2H), 3.89 - 3.76 (m, 1H), 3.16 (ddt, *J* = 15.2, 10.6, 5.7 Hz, 1H), 2.98 (s, 1H), 2.79 - 2.22 (m, 13H), 1.90 (ddd, *J*= 27.0, 14.0, 7.1 Hz, 3H), 1.51 (dq, *J=* 22.6, 6.4 Hz, 5H), 1.28 (q, *J* = 4.2 Hz, 51H), 0.90 (m, 12H).

### Example 6. Synthesis of Synthesis of 2-hexyldecyl 8-(N-{7,7-difluoro-8-[(2-hexyldecyl)oxy]-8-oxooctyl}-4-(dimethylamino)butanamido)octadecenoate (33)

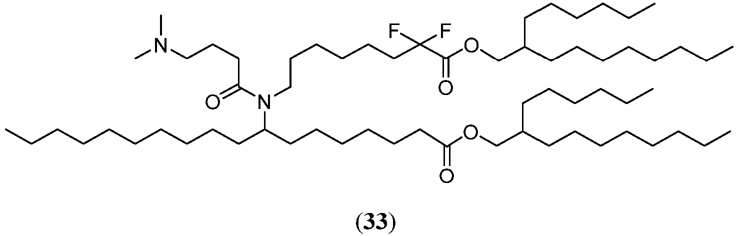

### Step 1. Synthesis of methyl 8-oxooctadecanoate

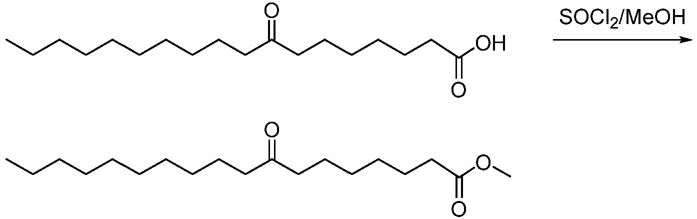

8-Oxooctadecanoic acid (3.3 g, 10.9 mmol was stirred in MeOH (60 mL) at 0°C. Thionyl chloride (1.43 g, 12.0 mmol) was added dropwise and the reaction stirred for 18 hr allowing to warm to RT. The reaction was concentrated *in-vacuo,* the residue taken-up in hexane and washed with NaHCO₃ (sat. soln.) and brine, dried (MgSO4), filtered and concentrated *in-vacuo.* The crude material was purified by automated flash chromatography (2-20% EtOAc-hexane) to give methyl 8-oxooctadecanoate (3.33 g, 97.9%).

### Step 2. Synthesis of methyl 8-(hex-5-en-1-ylamino)octadecenoate

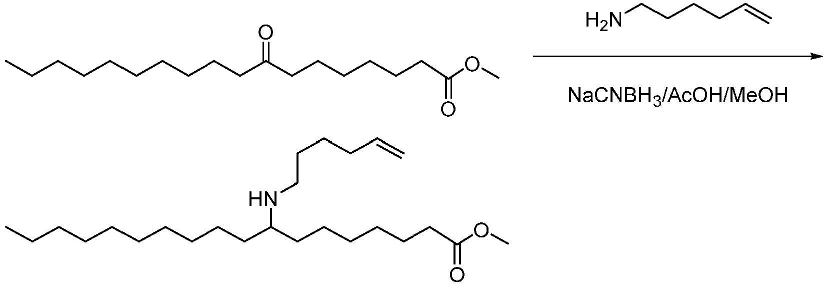

Methyl 8-oxooctadecanoate (2.68 g, 8.6 mmol), hex-5-en-1-amine (0.94 g, 9.4 mmol), AcOH (1.0 mL, 17.2 mmol) and NaCNBH₃ (1.62 g, 25.7 mmol) were stirred in MeOH (32 mL) at 50 °C for 18hr. The reaction was concentrated *in-vacuo,* the residue taken-up in EtOAc and washed with NaHCO₃ (sat. soln.) and brine, dried (MgSO₄), filtered and concentrated. The crude material was purified by automated flash chromatography (0-10% MeOH-DCM) to give methyl 8-(hex-5-en-1-ylamino)octadecanoate (2.1 g, 61.9%).

### Step 3. Synthesis of 8-[4-(dimethylamino)-N-(hex-5-en-1-yl)butanamido]octadecenoate

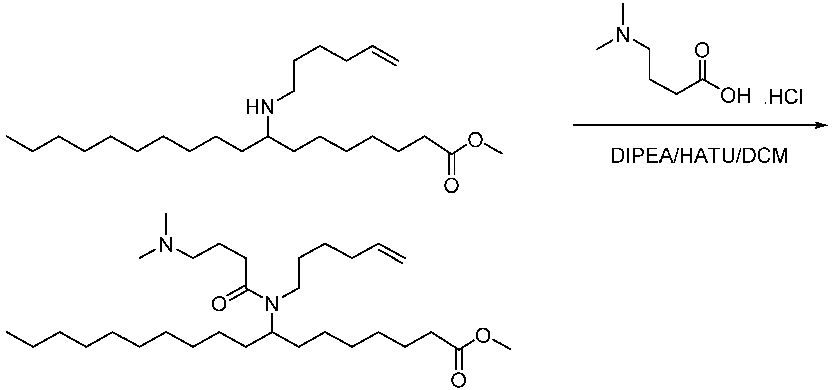

4-Dimethylaminobutanoic acid (HCl salt) (1.8 g, 10.6 mmol), HATU (4.0 g, 10.6 mmol) and DIPEA (3.7 mL, 21.2 mmol) were stirred in DCM (50 mL) at RT for 1 hr. Methyl 8-(hex-5-en-1-ylamino)octadecanoate (2.1 g, 5.3 mmol) in DCM (5 mL) was added and the reaction stirred at RT for 18 hr. The reaction was concentrated *in-vacuo,* the residue taken-up in EtOAc and washed with NaHCO₃ (sat. soln.) and brine, dried (MgSO₄), filtered and concentrated *in-vacuo.* The crude material was purified by automated flash chromatography (1-15% MeOH-DCM) to give methyl 8-[4-(dimethylamino)-N-(hex-5-en-1-yl)butanamido]octadecanoate (2.0 g, 74.1%).

### Step 4. Synthesis of methyl 8-[4-(dimethylamino)-N-[(5E)-8-ethoxy-7,7-difluoro-8-oxooct-5-en-1-yl]butanamido]octadecenoate

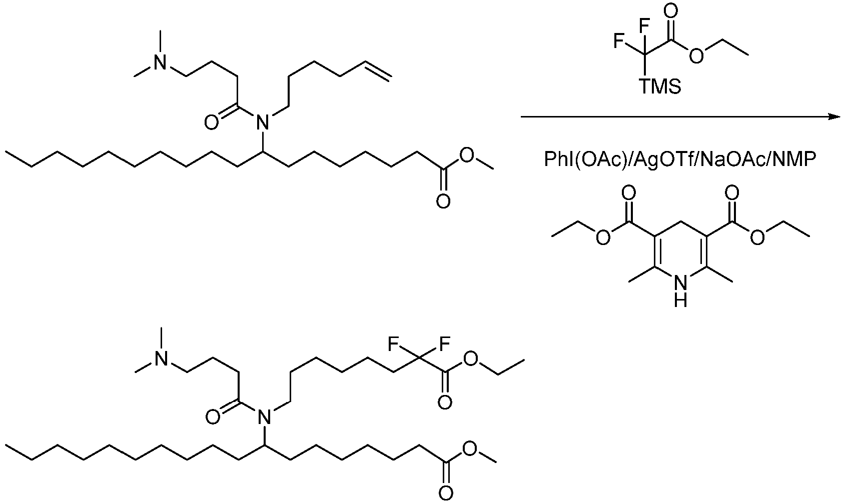

In a vacuum, heat dried flask, Hantzch ester (1.0 g, 3.9 mmol), Iodobenzenediacetate (2.5 g, 7.9 mmol), NaOAc (1.0 g, 11.8 mmol) and AgOTf (2.5 g, 9.8 mmol) were purged with nitrogen and dissolved in NMP (40 mL). Methyl 8-[4-(dimethylamino)-N-(hex-5-en-1-yl)butanamido]octadecanoate (2 g, 3.9 mmol) and ethyl 2,2-difluoro-2-(trimethylsilyl)acetate (3.6 g, 20.0 mmol) were added and the reaction stirred at RT for 18 hr. The reaction was filtered through Celite, the filtrate washed with water and brine, dried (MgSO₄), filtered and concentrated *in-vacuo.* The crude material was purified by automated flash chromatography (0-10% MeOH-DCM) to give methyl 8-[4-(dimethylamino)-N-[(5E)-8-ethoxy-7,7-difluoro-8-oxooct-5-en-1-yl]butanamido]octadecanoate (2.21 g, 66.8%).

### Step 5. Synthesis of 2-hexyldecyl 8-(N-{7,7-difluoro-8-[(2-hexyldecyl)oxy]-8-oxooctyl}-4-(dimethylamino)butanamido)octadecenoate (33).

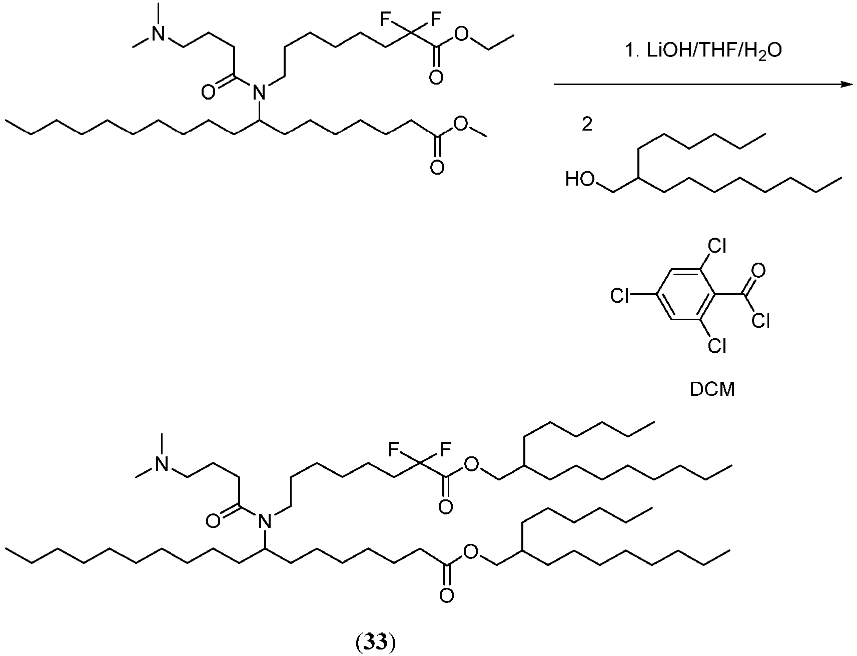

Methyl 8-[4-(dimethylamino)-N-(8-ethoxy-7,7-difluoro-8-oxooctyl)butanamido]-octadecanoate (2.21 g, 3.9 mmol) and LiOH (335 mg, 14.0 mmol) were stirred in THF (22 mL ) and H₂O (6.6 mL) at 50°C for 2 hr. The reaction was quenched with 6M HCl (11.6 mL) and partially concentrated *in-vacuo.* The aqueous was diluted with HCl (1M) and extracted with DCM. The combined organics were washed with brine, dried (MgSO₄), filtered and concentrated *in-vacuo* azeotroping with toluene.

The bis-acid and 2-hexyldecan-1-ol (1.8 g, 7.3 mmol) were dissolved in DCM (22 mL) and 2,4,6 trichlorobenzoyl chloride (1.87 g, 7.76 mmol) and DMAP (1.71 g, 14.0 mmol) added. The reaction was stirred at RT for 18 hr, concentrated *in-vacuo* and the residue taken-up in EtOAc. The organics were washed with NaHCO₃ (sat. soln) and brine, dried (MgSO₄), filtered and concentrated *in-vacuo.* The crude material was purified by flash chromatography (0-12% MeOH-DCM) to give 2-hexyldecyl 8-(N-{7,7-difluoro-8-[(2-hexyldecyl)oxy]-8-oxooctyl}-4-(dimethylamino)butanamido)octadecenoate **(33)** (615 mg, 16.9%). ¹H NMR (400MHz, CDCl₃) δ 4.18 (dd, *J* = 5.8, 4.0 Hz, 2H), 3.98 (dd, *J =* 5.8, 3.2 Hz, 2H), 3.65 (t, *J* = 7.2 Hz, 1H), 3.11 - 3.00 (m, 2H), 2.39 - 2.28 (m, 6H), 2.26 (s, 6H), 2.16 - 1.97 (m, 3H), 1.85 (dt, *J =* 9.2, 6.9 Hz, 3H), 1.73 (s, 1H), 1.68 - 1.38 (m, 13H), 1.29 (m,73H), 0.95 - 0.86 (m, 15H). ¹⁹F NMR (376MHz, CDCl₃) δ -105.78 (m, 2F).

The following compounds were prepared using analogous methodology as described above. 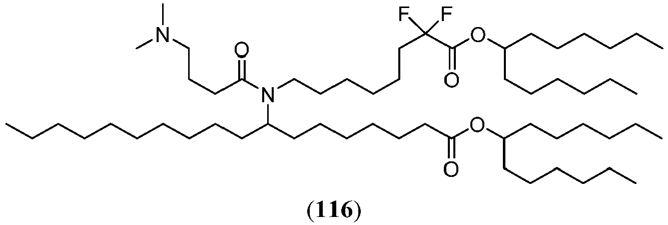 **Tridecan-7-yl 8-(N-(7,7-difluoro-8-oxo-8-(tridecan-7-yloxy)octyl)-4-(dimethylamino)-butanamido)octadecanoate (116).** ¹H NMR (400 MHz, CDCl₃) δ 5.02 (q, *J* = 6.5 Hz, 1H), 4.89 (t, *J =* 6.4 Hz, 1H), 3.67 (d, *J* = 7.8 Hz, 0H), 3.40 (s, 0H), 3.06 (t, *J* = 8.0 Hz, 2H), 2.38 - 2.25 (m, 5H), 2.24 (s, 4H), 2.07 (tq, *J* = 16.8, 9.2 Hz, 2H), 1.84 (q, *J* = 8.1 Hz, 2H), 1.73 - 1.15 (m, 59H), 0.90 (t, *J=* 6.6 Hz, 12H). 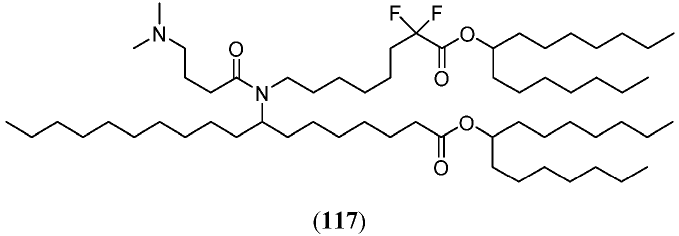 **Pentadecan-8-yl 8-(N-(7,7-difluoro-8-oxo-8-(pentadecan-8-yloxy)octyl)-4-(dimethylamino)butanamido)octadecanoate (117).** ¹H NMR (400 MHz, CDCl₃) δ 5.07 - 4.98 (m, 1H), 4.88 (td, *J* = 6.5, 3.1 Hz, 1H), 3.64 (s, 0H), 2.47 (s, 1H), 2.42 - 2.24 (m, 7H), 2.06 (tt, *J* = 16.7, 8.3 Hz, 2H), 1.96 - 1.88 (m, 2H), 1.72 - 1.15 (m, 65H), 0.90 (t, *J* = 6.7 Hz, 12H). 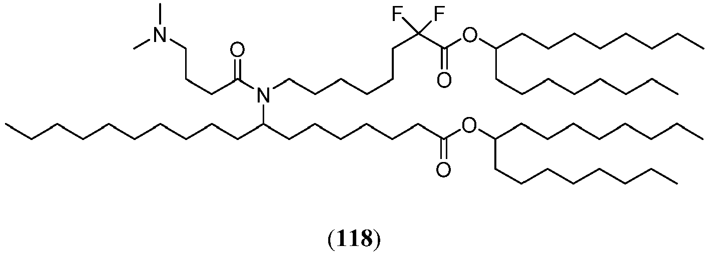 **Heptadecan-9-yl 8-(N-(7,7-difluoro-8-(heptadecan-9-yloxy)-8-oxooctyl)-4-(dimethylamino)butanamido)octadecanoate (118).** ¹H NMR (400 MHz, CDCl₃) δ 5.02 (q, *J* = 5.9 Hz, 1H), 4.88 (t, *J* = 6.1 Hz, 1H), 3.64 (s, 1H), 3.43 - 3.38 (m, 0H), 3.06 (dd, *J =* 11.6, 5.5 Hz, 2H), 2.51 (s, 2H), 2.43 - 2.24 (m, 7H), 2.07 (tq, *J* = 16.7, 9.2 Hz, 2H), 1.93 (q, *J* = 7.2 Hz, 2H), 1.72 - 1.13 (m, 76H), 0.90 (t, *J =* 6.7 Hz, 12H).

### Example 7. Synthesis of Synthesis of 3-pentyloctyl 9-[4-(dimethylamino)-N-{6-fluoro-7-oxo-7-[(3-pentyloctyl)oxy]heptyl}butanamido]-2-fluorooctadecanoate (34)

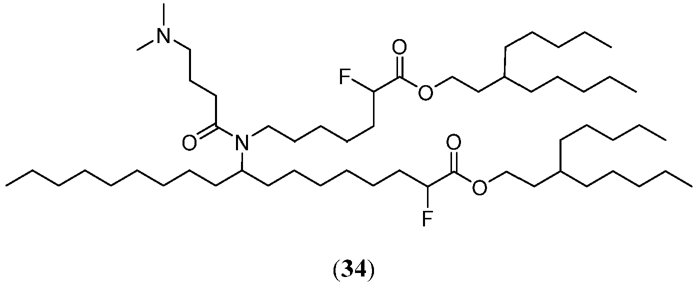

### Step 1. Synthesis of [1-(oxan-2-yloxy)hexadecan-7-yl][5-(oxan-2-yloxy)pentyl]amine

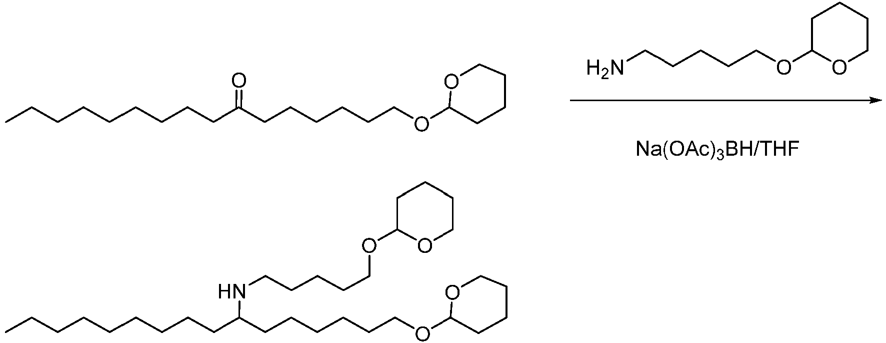

1-(Oxan-2-yloxy)hexadecan-7-one (9.9 g, 29.1 mmol), 5-(oxan-2-yloxy)pentan-1-amine (6 g, 32.0 mmol) and Na(OAc)₃BH (18.5 g, 87.4 mmol) were stirred in THF (100 mL) at 50°C for 18 hr. The reaction was concentrated *in-vacuo,* the residue taken-up in Et₂O, washed with NaHCO₃ (sat. soln) and brine, dried (MgSO₄), filtered and concentrated *in-vacuo.* The crude material was purified by automated flash chromatography (0-10% MeOH-DCM) to give [1-(oxan-2-yloxy)hexadecan-7-yl][5-(oxan-2-yloxy)pentyl]amine (5.2 g, 34.9%).

### Step 2. Synthesis of 4-(dimethylamino)-N-[1-(oxan-2-yloxy)hexadecan-7-yl]-N-[5-(oxan-2-yloxy)pentyl]butanamide

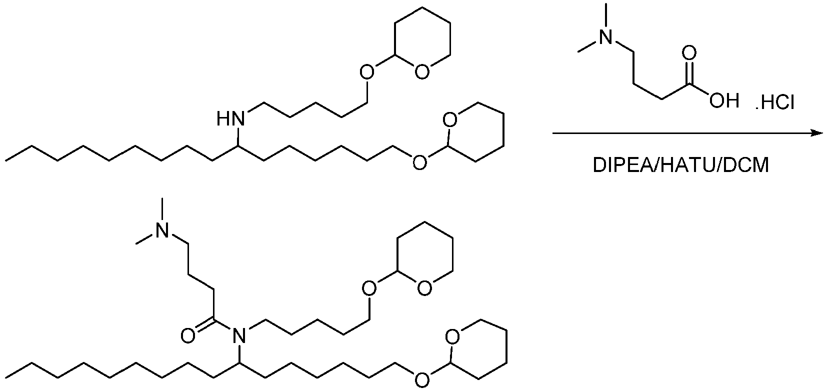

4-Dimethylamino]butanoic acid (HCl salt) (5.2 g, 31.1 mmol), HATU (11.8 g, 31.0 mmol) and DIPEA (10.8 mL, 62.1 mmol) were stirred in DCM (75 mL) at RT for 1hr. [1-(Oxan-2-yloxy)hexadecan-7-yl][5-(oxan-2-yloxy)pentyl]amine (5.3 g, 10.4 mmol) solution in DCM (10mL) was added and stirring continued at RT for 18 hr. The reaction was concentrated *in-vacuo,* the residue taken-up in EtOAc, washed with NaHCO₃ (sat. soln.) and brine, dried (MgSO₄), filtered and concentrated *in-vacuo.* The crude material was purified by automated flash chromatography (1-15% MeOH-DCM) to give 4-(dimethylamino)-N-[1-(oxan-2-yloxy)hexadecan-7-yl]-N-[5-(oxan-2-yloxy)pentyl]butanamide (2.08 g, 32.1%).

### Step 3. Synthesis of 4-(dimethylamino)-N-(1-hydroxyhexadecan-7-yl)-N-(5-hydroxypentyl)butanamide

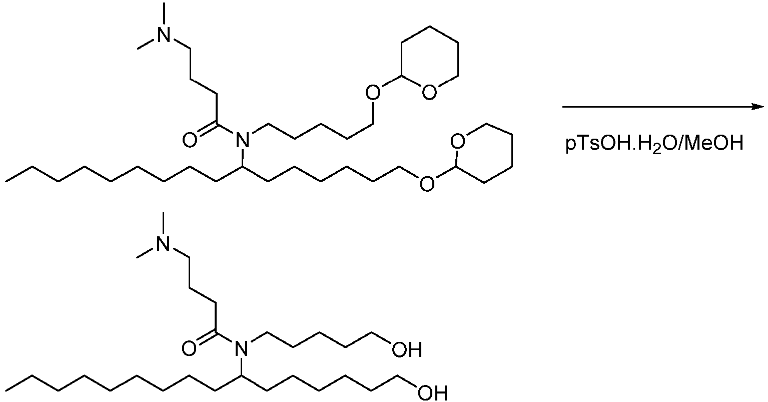

4-(Dimethylamino)-N-[1-(oxan-2-yloxy)hexadecan-7-yl]-N-[5-(oxan-2-yloxy)pentyl]butanamide (2.08 g, 3.3 mmol) and pTsOH-H₂O (127 mg, 0.7 mmol) were stirred in MeOH (25 mL) at RT for 18 hr. The reaction was concentrated *in-vacuo,* the residue taken-up in EtOAc, washed with NaHCO₃ (sat. soln.) and brine, dried (MgSO₄), filtered and concentrated *in-vacuo* to give 4-(dimethylamino)-N-(1-hydroxyhexadecan-7-yl)-N-(5-hydroxypentyl)butanamide (1.5 g, 98.7%) which was used without further processing.

### Step 4. Synthesis of 4-(dimethylamino)-N-(1-oxohexadecan-7-yl)-N-(5-oxopentyl)-butanamide

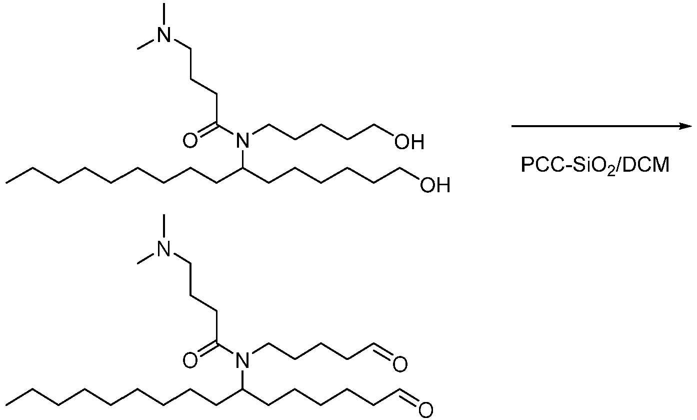

4-(Dimethylamino)-N-(1-hydroxyhexadecan-7-yl)-N-(5-hydroxypentyl)butanamide (1.5 g, 3.3 mmol) and PCC-SiO₂ (1:1) (5.0 g, 11.5 mmol) were stirred in DCM (30 mL) at RT for 18 hr. The mixture with filtered through a bed of silica, eluting with EtOAc. The filtrate was concentrated *in-vacuo* and the crude material purified by automated flash chromatography (0-12% MeOH-DCM) to give 4-(dimethylamino)-N-(1-oxohexadecan-7-yl)-N-(5-oxopentyl)butanamide (486 mg, 32.7%).

### Step 5. Synthesis of ethyl (2Z)-9-[4-(dimethylamino)-N-[(5Z)-7-ethoxy-6-fluoro-7-oxohept-5-en-1-yl]butanamido]-2-fluorooctadec-2-enoate

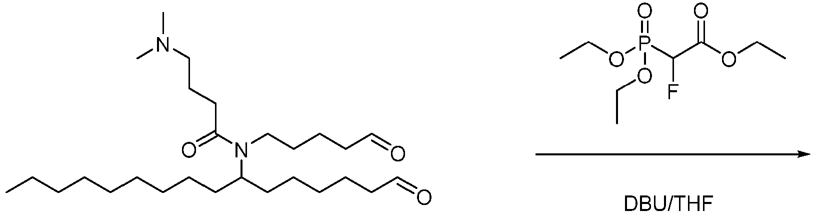

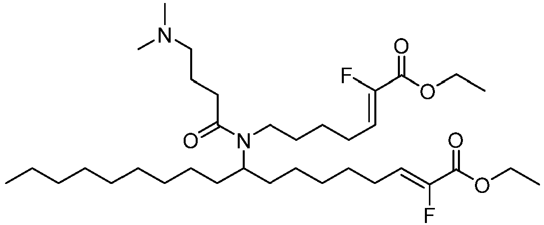

4-(Dimethylamino)-N-(1-oxohexadecan-7-yl)-N-(5-oxopentyl)butanamide (346 mg, 0.76 mmol), ethyl 2-(diethoxyphosphoryl)-2-fluoroacetate (0.62 mL, 3.1 mmol) and DBU (0.46 mL, 3.1 mmol) were stirred in THF (10 mL) at RT for 18 hr. The reaction was concentrated *in-vacuo,* the residue taken-up in EtOAc, washed with NaHCO₃ (sat. soln.) and brine, dried (MgSO₄), filtered and concentrated *in-vacuo.* The crude material was purified by automated flash chromatography (0-10% MeOH-DCM) to give ethyl (2Z)-9-[4-(dimethylamino)-N-[(5Z)-7-ethoxy-6-fluoro-7-oxohept-5-en-1-yl]butanamido]-2-fluorooctadec-2-enoate (116 mg, 24.1%).

### Step 6. Synthesis of ethyl 9-[4-(dimethylamino)-N-(7-ethoxy-6-fluoro-7-oxoheptyl)-butanamido]-2-fluorooctadecanoate

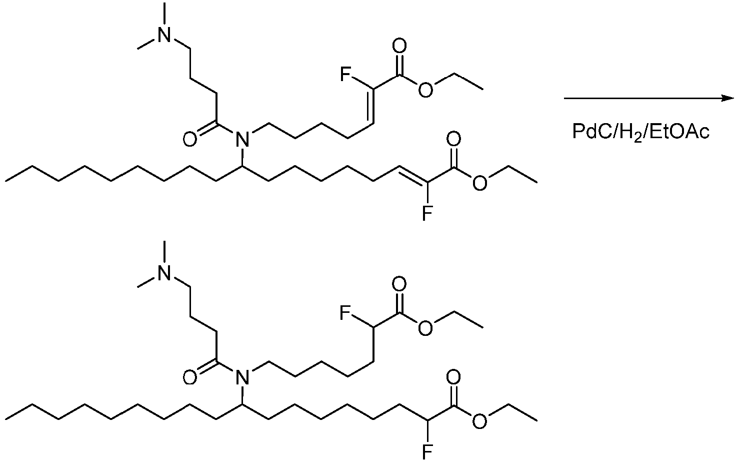

Ethyl (2Z)-9-[4-(dimethylamino)-N-[(5Z)-7-ethoxy-6-fluoro-7-oxohept-5-en-1-yl]butanamido]-2-fluorooctadec-2-enoate (116 mg, 0.18 mmol) was subjected to catalytic hydrogenation over Pd/C (10% w/w) (15 mg) in EtOAc (5 mL) at RT for 18 hr. The mixture was filtered through Celite and the filtrate concentrated *in-vacuo* to give ethyl 9-[4-(dimethylamino)-N-(7-ethoxy-6-fluoro-7-oxoheptyl)butanamido]-2-fluorooctadecanoate (117 mg, 100%) that was used without purification.

### Step 7. Synthesis of 9-[N-(6-carboxy-6-fluorohexyl)-4-(dimethylamino)butanamido]-2-fluorooctadecanoic acid

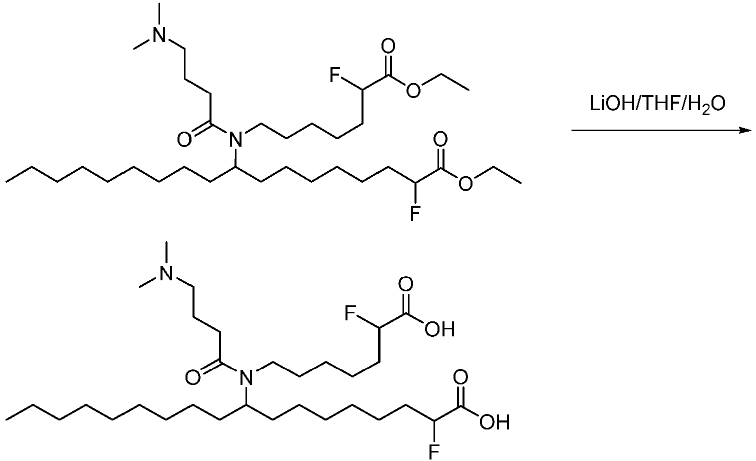

Ethyl 9-[4-(dimethylamino)-N-(7-ethoxy-6-fluoro-7-oxoheptyl)butanamido]-2-fluorooctadecanoate (117 mg, 0.19 mmol) and LiOH (18 mg, 0.74 mmol) were stirred in THF (0.6 mL) and H₂O water (0.18 mL) at 50°C for 2 hr. The reaction was cooled to RT, quenched with 6 M HCl (0.126 mL) and extracted with DCM. The combined organics were washed with brine and dried (MgSO₄), filtered and concentrated *in-vacuo* to give 9-[N-(6-carboxy-6-fluorohexyl)-4-(dimethylamino)butanamido]-2-fluorooctadecanoic acid (75 mg, 70.3%) which was used without purification.

### Step 8. Synthesis of 3-pentyloctyl 9-[4-(dimethylamino)-N-{6-fluoro-7-oxo-7-[(3-pentyloctyl)oxy]heptyl}butanamido]-2-fluorooctadecanoate (34)

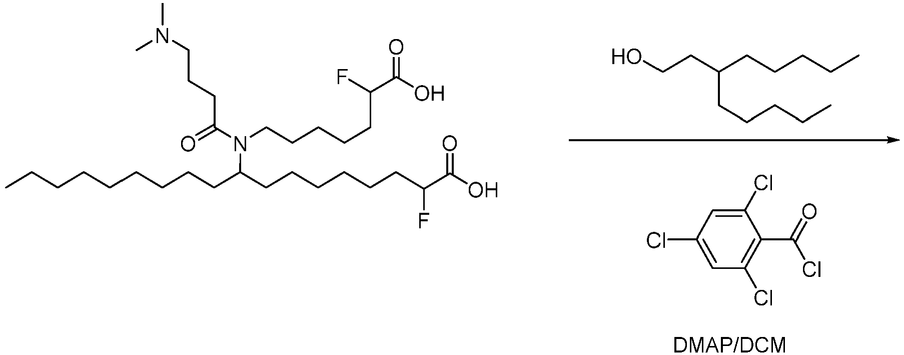

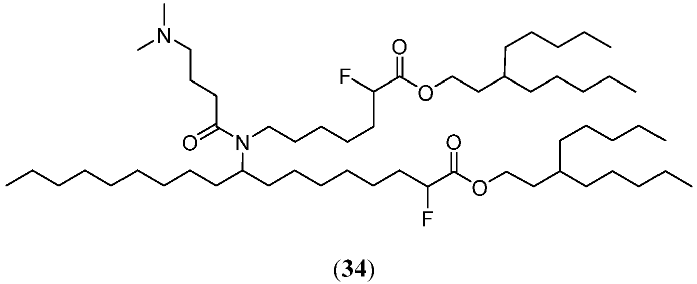

9-[N-(6-Carboxy-6-fluorohexyl)-4-(dimethylamino)butanamido]-2-fluorooctadecanoic acid (75 mg, 0.13 mmol), 3-pentyloctan-1-ol (72 mg, 0.36 mmol), 2,4,6 trichlorobenzoyl chloride (95 g, 0.4 mmol) and DMAP (64 mg, 0.52 mmol) were stirred in in DCM (4 mL) at RT for 18 hr. The reaction was concentrated *in-vacuo,* the residue taken-up in EtOAc, washed with NaHCO₃ (sat. soln.) and brine, dried (MgSO₄), filtered and concentrated *in-vacuo.* The crude material was purified by automated flash chromatography (0-12% MeOH-DCM) to give 3-pentyloctyl 9-[4-(dimethylamino)-N-{6-fluoro-7-oxo-7-[(3-pentyloctyl)oxy]heptyl}butanamido]-2-fluorooctadecanoate **(34)** (42 mg, 34.3%). ¹H NMR (400 MHz, CDCl₃) δ 5.00 - 4.91 (m, 1H), 4.88 - 4.79 (m, 1H), 4.23 (qd, *J =* 6.2, 2.9 Hz, 4H), 3.71 - 3.56 (m, 1H), 3.07 (d, *J* = 8.3 Hz, 2H), 2.62 (s, 2H), 2.45 (m, 8H), 1.99 - 1.80 (m, 7H), 1.69 - 1.40 (m, 18H), 1.39 - 1.15 (m, 56H), 0.95 - 0.86 (m, 15H).

### Example 8. Synthesis of Synthesis of decyl 8-{N-[(6Z)-2-[(4Z)-dec-4-en-1-yl]dodec-6-en-1-yl]-5-(dimethylamino)pentanamido}octadecenoate (35)

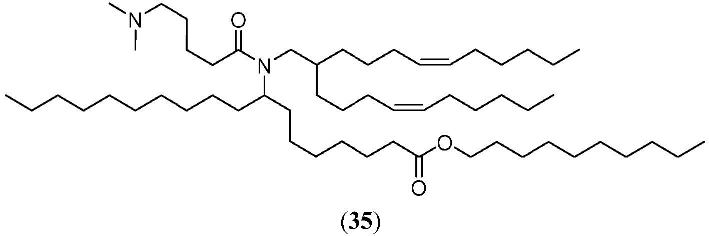

### Step 1. Synthesis of (6Z,15Z)-henicosa-6,15-dien-11-ol

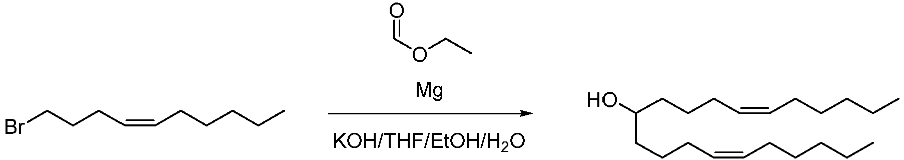

A Grignard reagent was prepared from (4Z)-1-bromodec-4-ene (19.9 g, 91 mmol) and magnesium (2.4 g, 97.0 mmol) in THF. The reaction was heated at 45°C for 3hr. After cooling to 10°C, ethyl formate (7.0 g, 93.5 mmol in THF (35 mL) was added dropwise and the reaction stirred for 1 hr. Water (40 mL) and 6 M HCl (50 mL) were added to quench and the reaction partitioned between water and hexane. The organics were separated, washed with brine, dried (MgSO₄), filtered and concentrated *in-vacuo.* The residue was taken-up in EtOH (30 mL) and treated with a solution of KOH (4.6 g) in water (10 mL). After 5 min the EtOH was removed and the residue partitioned between hexane and 6 M HCl. The organics were washed with water and brine, dried (MgSO₄), filtered and concentrated *in-vacuo.* The crude material was purified by automated flash chromatography (0-10% EtOAc-hexane) to give (6Z,15Z)-henicosa-6,15-dien-11-ol (11.4 g, 81.4%).

### Step 2. Synthesis of (6Z,15Z)-henicosa-6,15-dien-11-yl 4-methylbenzenesulfonate

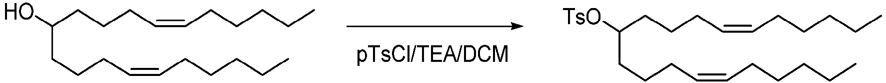

(6Z,15Z)-Henicosa-6,15-dien-11-ol (10 g, 32.4 mmol), TEA (9.0 mL, 64.8 mmol), trimethylammonium HCl (0.31 g, 3.2 mmol) and p toluenesulfonyl chloride (8.0 g, 42.1 mmol) were stirred in DCM (50 mL) at RT for 18 hr. The reaction was quenched slowly with N,N dimethyl propane diamine (1.09 g, 10.7 mmol). After stirring for 15 min the reaction was washed with water and brine, dried (MgSO₄), filtered and concentrated *in-vacuo.* The crude material was purified by automated flash chromatography to give (6Z,15Z)-henicosa-6,15-dien-11-yl 4-methylbenzenesulfonate (0-5% EtOAc-hexane) (8.7 g, 57.9%).

### Step 3. Synthesis of (6Z)-2-[(4Z)-dec-4-en-1-yl]dodec-6-enenitrile

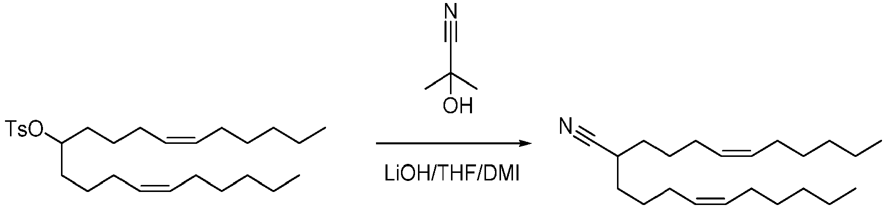

(6Z,15Z)-Henicosa-6,15-dien-11-yl 4-methylbenzenesulfonate (8.7 g, 18.8 mmol), dimethylimidazolidinone (22.6 mL, 0.83 M), acetone cyanohydrin (3.5 g, 41.5 mmol) and LiOH (880 mg, 36.6 mmol) were stirred in THF (52 mL) at 65°C for 18 hr. The reaction was partitioned between EtOAc-hexane (1:1, 500 mL) and water, the organics washed with brine, dried (MgSO₄), filtered and concentrated *in-vacuo.* The crude material was purified by automated flash chromatography (0-10% EtOAc-hexane) to give (6Z)-2-[(4Z)-dec-4-en-1-yl]dodec-6-enenitrile (4.9 g, 81.5 %).

### Step 4. Synthesis of yield (6Z,15Z)-11-(aminomethyl)henicosa-6,15-diene

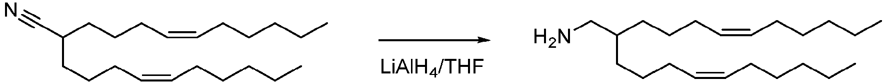

(6Z)-2-[(4Z)-Dec-4-en-1-yl]dodec-6-enenitrile (3.4 g, 9.9 mmol) and LiAlH₄ (1.9 g, 50.0 mmol) were stirred in THF (75 mL) at 0°C for 18 hr allowing to warm to RT. The reaction was cooled to 0°C and quenched with 1M NaOH (1.9 mL). The mixture was dried (MgSO₄), filtered and concentrated *in-vacuo* and the crude material purified by automated flash chromatography (0-10% MeOH-DCM) to give (6Z,15Z)-11-(aminomethyl)henicosa-6,15-diene (2.4 g, 75.4%).

### Step 5. Synthesis of decyl 8-{[(6Z)-2-[(4Z)-dec-4-en-1-yl]dodec-6-en-1-yl]amino}octadecenoate

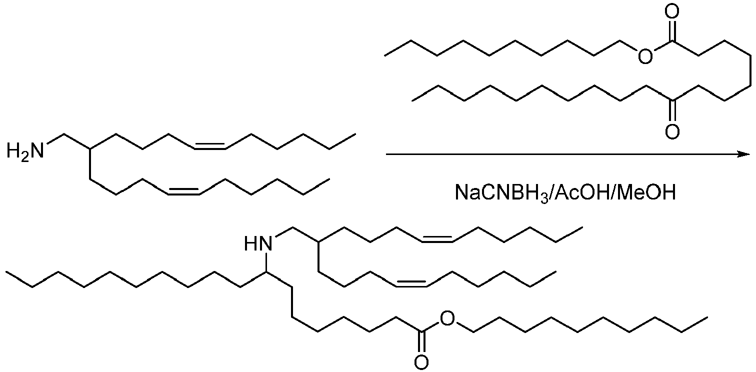

Decyl 8-oxooctadecanoate (368 mg, 0.84 mmol), (6Z,15Z)-11-(aminomethyl)henicosa-6,15-diene (297 mg, 0.9 mmol), AcOH (0.1 mL, 1.7 mmol) and NaCNBH₃ (156 mg, 2.5 mmol) were stirred in MeOH (10 mL) at (50°C) for 18 hr. The reaction was concentrated *in-vacuo,* the residue taken-up in DCM, washed with NaHCO₃ (sat. soln) and brine, dried (MgSO₄), filtered and concentrated *in-vacuo.* The crude material was purified by automated flash chromatography (5-40% EtOAc-hexane) to give decyl 8-{[(6Z)-2-[(4Z)-dec-4-en-1-yl]dodec-6-en-1-yl]amino}octadecanoate (438 mg, 70.2%).

### Step 6. decyl 8-{N-[(6Z)-2-[(4Z)-dec-4-en-1-yl]dodec-6-en-1-yl]-5-(dimethylamino)-pentanamido}octa decenoate

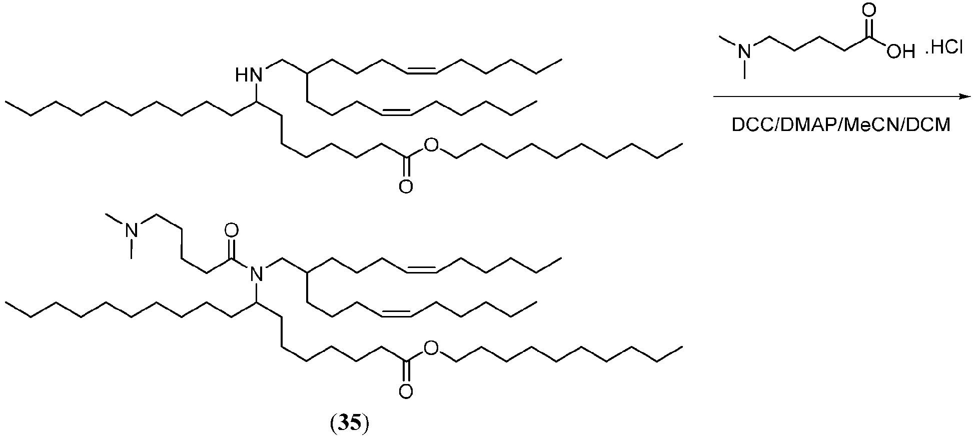

5-Dimethylamino]pentanoic acid, HCl salt (71 mg, 0.4 mmol), DCC (90 mg, 0.43 mmol) and DMAP (72 mg, 0.6 mmol) were stirred in MeCN (5 mL) at RT for 3 hr. Decyl 8-{[(6Z)-2-[(4Z)-dec-4-en-1-yl]dodec-6-en-1-yl]amino}octadecanoate (146 mg, 0.2 mmol) in DCM (2 mL) was added and the reaction stirred at RT for 18 hr. The reaction was concentrated *in-vacuo,* the residue dispersed in hexane:EtOAc:TEA (75:25:1), filtered through Celite and the filtrate concentrated *in-vacuo.* The crude material was purified by automated flash chromatography (0-12% MeOH-DCM) to give decyl 8-{N-[(6Z)-2-[(4Z)-dec-4-en-1-yl]dodec-6-en-1-yl]-5-(dimethylamino)pentanamido}octadecanoate **(35)** (88 mg, 51.5%). ¹H NMR (400 MHz, CDCl₃) δ 5.38 (hd, *J* = 7.9, 5.4 Hz, 4H), 4.07 (td, *J* = 6.8, 2.4 Hz, 2H), 3.63 (s, 1H), 3.09 (dd, *J* = 18.4, 7.5 Hz, 2H), 2.42 - 2.26 (m, 6H), 2.23 (d, *J* = 1.5 Hz, 6H), 2.01 (dd, *J* = 13.6, 6.8 Hz, 8H), 1.76 - 1.57 (m, 11H), 1.51 (dq, *J* = 14.7, 7.2 Hz, 5H), 1.43 - 1.17 (m, 53H), 0.96 - 0.84 (m, 12H).

The following compounds were prepared using analogous methods as described above. 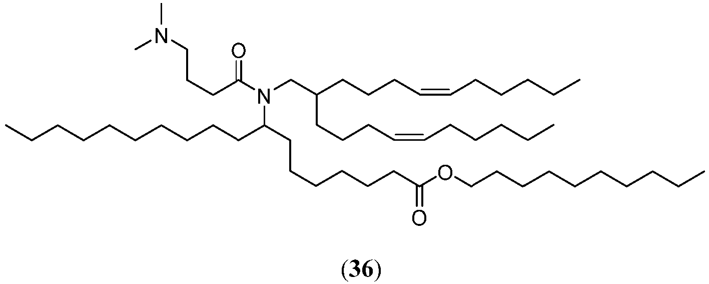 **Decyl 8-(N-((Z)-2-((Z)-dec-4-en-1-yl)dodec-6-en-1-yl)-4-(dimethylamino)butanamido)-octadecenoate (36).** ¹H NMR (400 MHz, CDCl₃) δ 5.48 - 5.25 (m, 4H), 4.07 (td, *J =* 6.8, 2.7 Hz, 2H), 3.66 (s, 1H), 3.09 (d, *J* = 7.5 Hz, 2H), 2.42 - 2.26 (m, 6H), 2.24 (s, 6H), 2.02 (t, *J* = 6.9 Hz, 7H), 1.82 (dt, *J* = 9.7, 7.2 Hz, 2H), 1.63 (d, *J* = 4.1 Hz, 9H), 1.47 (s, 2H), 1.41 - 1.18 (m, 53H), 0.97 - 0.84 (m, 12H). 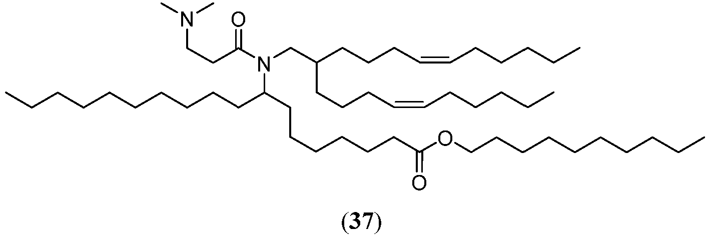 **Decyl 8-(N-((Z)-2-((Z)-dec-4-en-1-yl)dodec-6-en-1-yl)-3-(dimethylamino)propanamido)-**octadecenoate (37). ¹H NMR (400 MHz, CDCl₃) δ 5.46 - 5.30 (m, 4H), 4.96 - 4.83 (m, 1H), 4.07 (td, *J =* 6.8, 2.0 Hz, 3H), 3.09 (d, *J* = 7.5 Hz, 2H), 2.75 - 2.61 (m, 3H), 2.60 - 2.45 (m, 3H), 2.35 - 2.23 (m, 9H), 2.03 (q, *J* = 6.5 Hz, 7H), 1.58 (d, *J* = 53.6 Hz, 20H), 1.43 - 1.19 (m, 57H), 0.91 (ddt, *J* = 6.9, 3.6, 2.3 Hz, 12H).

### Example 9. Synthesis of Synthesis of 3-pentyloctyl 9-[N-(decyloxy)-4-(dimethylamino)butanamido]-2-fluorooctadecanoate (38)

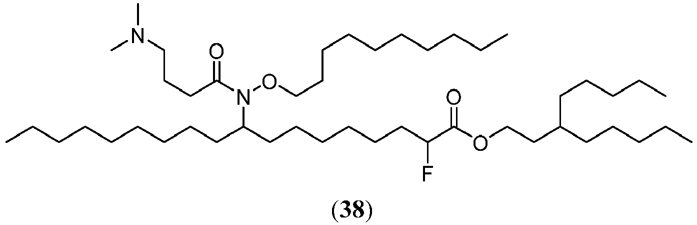

### Step 1. Synthesis of (decyloxy)[1-(oxan-2-yloxy)hexadecan-7-yl]amine

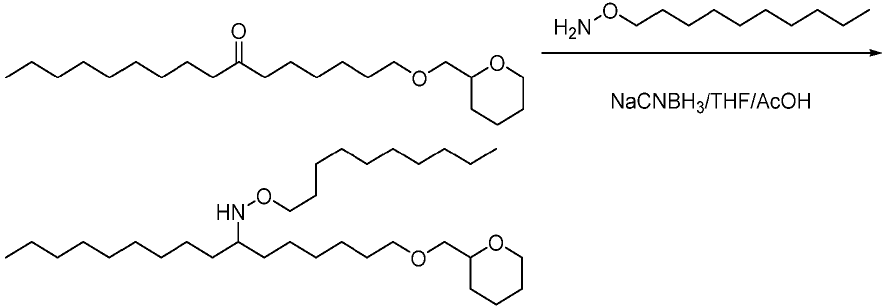

1-(Oxan-2-yloxy)hexadecan-7-one (7 g, 20.6 mmol), O-decylhydroxylamine (3.6 g, 21 mmol) and NaCNBH₃ (3.9 g, 61.7 mmol) were stirred in THF (10 mL) at 50°C for 16 hr. The reaction was concentrated *in-vacuo,* the residue dissolved in EtOAc and washed with water. The aqueous was back extracted with EtOAc and the combined organics washed with brine, dried (MgSO₄), filtered and concentrated *in-vacuo.* The residue was purified by automated flash chromatography (0-10% EtOAc/Hex) to give (decyloxy)[1-(oxan-2-yloxy)hexadecan-7-yl]amine (700 mg, 6.8%).

### Step 2. Synthesis of N-(decyloxy)-4-(dimethylamino)-N-[1-(oxan-2-yloxy)hexadecan-7-yl]butanamide

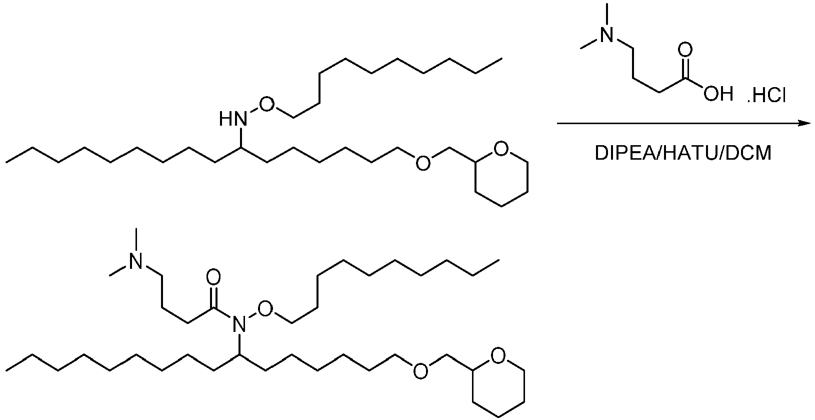

4-(Dimethylamino)butanoic acid HCl salt (1.4 g, 8.0 mmol), HATU (3.1 g, 8.0 mmol) and DIPEA (4.2 mL, 24.1 mmol) were stirred in DCM (40 mL) at RT for 1 hr. (Decyloxy)[1-(oxan-2-yloxy)hexadecan-7-yl]amine (2 g, 4.0 mmol) in DCM (10 mL) was added and the reaction refluxed for 16 hr. After cooling to RT the reaction was washed sequentially with 1M HCl, sat NaHCO₃, and brine, dried (MgSO₄), concentrated *in-vacuo* and the residue purified by automated flash chromatography (0-10% MeOH/DCM) to give N-(decyloxy)-4-(dimethylamino)-N-[1-(oxan-2-yloxy)hexadecan-7-yl]butanamide (2 g, 81.8%).

### Step 3. Synthesis of N-(decyloxy)-4-(dimethylamino)-N-(1-hydroxyhexadecan-7-yl)butanamide

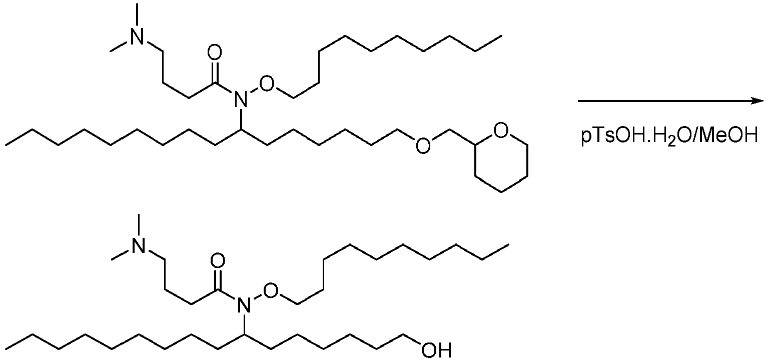

N-(Decyloxy)-4-(dimethylamino)-N-[1-(oxan-2-yloxy)hexadecan-7-yl]butanamide (2.5 g, 4.1 mmol) and pTsOH-H₂O (389 mg, 2.05 mmol) were stirred at RT in MeOH (10 mL) for 16 hr. The reaction was concentrated *in-vacuo,* the residue taken up in DCM, washed with sat NaHCO₃ and brine, dried (MgSO₄) and filtered. The filtrate was concentrated *in-vacuo* and the residue purified by automated flash chromatography (0-20% MeOH/DCM) to give N-(decyloxy)-4-(dimethylamino)-N-(1-hydroxyhexadecan-7-yl)butanamide (1.0 g, 46.4 %).

### Step 4. Synthesis of N-(decyloxy)-4-(dimethylamino)-N-(1-oxohexadecan-7-yl)butanamide

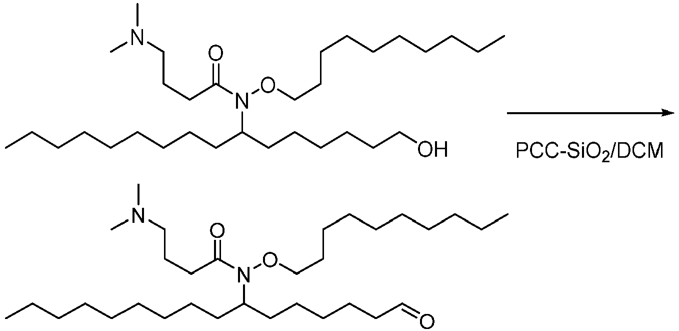

N-(Decyloxy)-4-(dimethylamino)-N-(1-hydroxyhexadecan-7-yl)butanamide (1.0 g, 1.9 mmol) and PCC-SiO₂ (1:1) (3.3 g, 7.6 mmol) were stirred in DCM (10 mL) at RT for 16 hr. The reaction was filtered through silica and concentrated *in-vacuo.* The residue was purified by automated flash chromatography (0-30% ACN/DCM) to give N-(decyloxy)-4-(dimethylamino)-N-(1-oxohexadecan-7-yl)butanamide (438 mg, 44.0 %).

### Step 5. Synthesis of ethyl (2Z)-9-[N-(decyloxy)-4-(dimethylamino)butanamido]-2-fluorooctadec-2-enoate

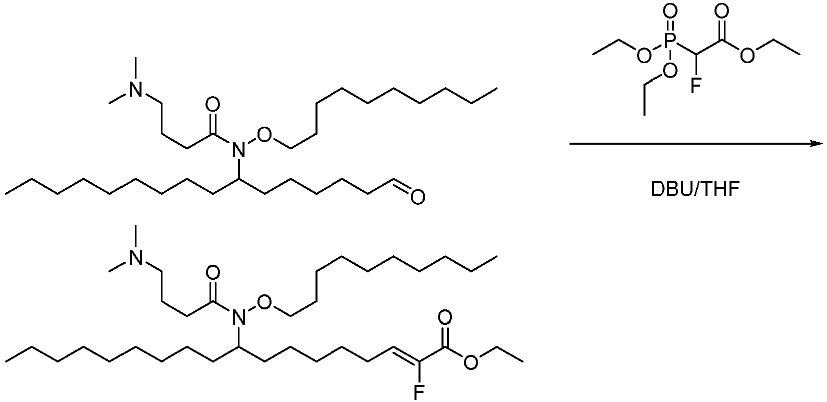

N-(Decyloxy)-4-(dimethylamino)-N-(1-oxohexadecan-7-yl)butanamide (438 mg, 0.8 mmol), ethyl 2-(diethoxyphosphoryl)-2-fluoroacetate (606 mg, 2.5 mmol) and DBU (381 mg, 2.5 mmol) were stirred at RT in THF (10 mL) for 16 hr. The solvent was removed *in-vacuo,* the residue taken up in EtOAc, washed with brine, and dried (MgSO₄). The filtrate was concentrated *in-vacuo* and purified by automated flash chromatography (0-20% ACN/DCM) to give ethyl (2Z)-9-[N-(decyloxy)-4-(dimethylamino)butanamido]-2-fluorooctadec-2-enoate (512 mg, 100.0%).

### Step 6. Synthesis of crude ethyl 9-[N-(decyloxy)-4-(dimethylamino)butanamido]-2-fluorooctadecanoate

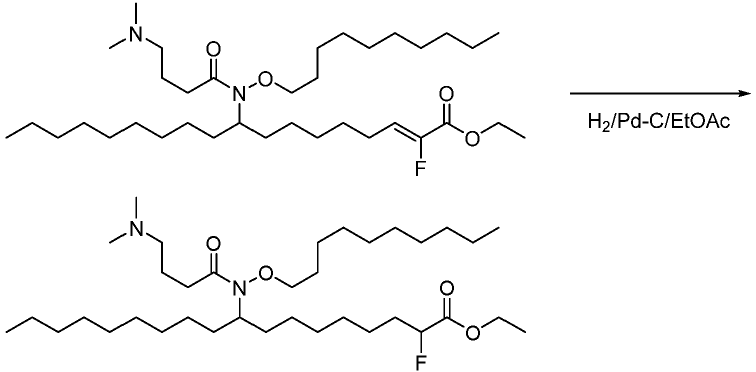

Ethyl (2Z)-9-[N-(decyloxy)-4-(dimethylamino)butanamido]-2-fluorooctadec-2-enoate (546 mg, 0.9 mmol) was subjected to catalytic hydrogenation over Pd-C in EtOAc (10 mL) at RT until complete. The reaction was filtered through Celite and concentrated *in-vacuo* to give ethyl 9-[N-(decyloxy)-4-(dimethylamino)butanamido]-2-fluorooctadecanoate which was used without purification.

### Step 7. Synthesis of 9-[N-(decyloxy)-4-(dimethylamino)butanamido]-2-fluorooctadecanoic acid

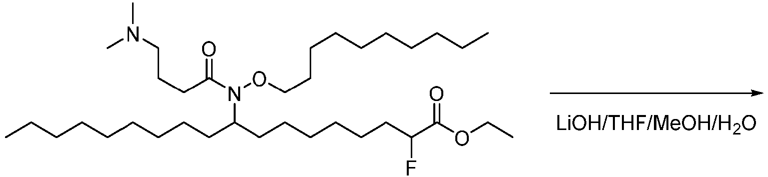

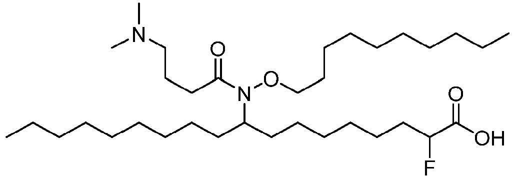

Ethyl 9-[N-(decyloxy)-4-(dimethylamino)butanamido]-2-fluorooctadecanoate (548 mg, 0.9 mmol) and LiOH (85 mg, 3.6 mmol) were stirred in THF (10 mL) and H₂O (1 mL) at RT for 16 hr. 6M HCl (0.9 mL) was added and the THF removed *in-vacuo.* The residue was taken up in DCM, washed with water the aqueous layer back extracted by DCM and the combined organics washed with brine. The organics were dried (MgSO₄), filtered and concentrated *in-vacuo* to give 9-[N-(decyloxy)-4-(dimethylamino)butanamido]-2-fluorooctadecanoic acid (390 mg, 74.6 %) which was used without purification.

### Step 8. Synthesis of 3-pentyloctyl 9-[N-(decyloxy)-4-(dimethylamino)butanamido]-2-fluorooctadecanoate (38)

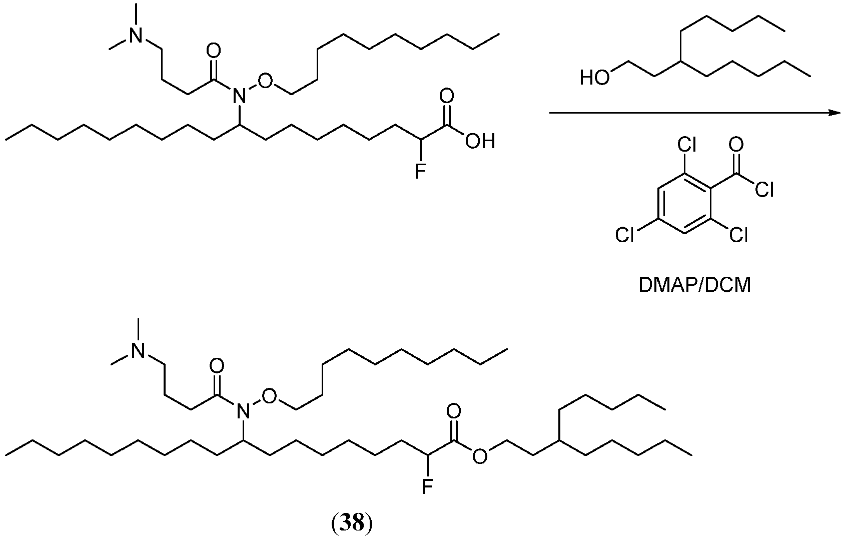

9-[N-(Decyloxy)-4-(dimethylamino)butanamido]-2-fluorooctadecanoic acid (390 mg, 0.66 mmol), 3-pentyloctan-1-ol (0.2 g, 1.0 mmol), 2,4,6-trichlorobenzoyl chloride (324 mg, 1.33 mmol), TEA (81 mg, 0.8 mmol) and DMAP (106 mg, 0.86 mmol) were stirred in THF (6 mL) at RT for 1.5 hr. The reaction was filtered, concentrated *in-vacuo* and the residue purified by automated flash chromatography (0-100% DCM/Hex and 0-30% ACN/DCM) to give 3-pentyloctyl 9-[N-(decyloxy)-4-(dimethylamino)butanamido]-2-fluorooctadecanoate **(38)** (30 mg, 5.9 %). ¹H NMR (400 MHz, CDCl₃) δ 4.30 - 4.20 (m, 3H), 3.98 - 3.89 (m, 2H), 3.30 (d, J = 5.3 Hz, 2H), 2.97 (s, 6H), 2.93 - 2.88 (m, 2H), 2.29 - 2.09 (m, 2H), 2.09 - 1.58 (m, 8H), 1.56 - 0.92 (m, 56H), 0.95 - 0.75 (m, 12H).

The following compounds were prepared using analogous methods as described above. 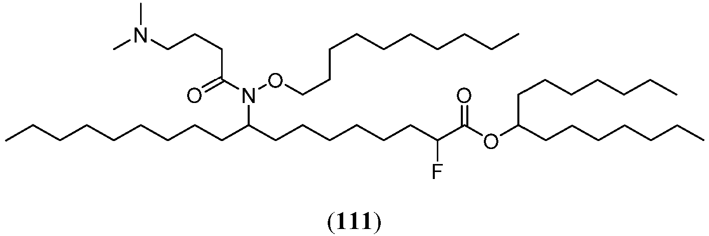 **Pentadecan-8-yl 9-(N-(decyloxy)-4-(dimethylamino)butanamido)-2-fluorooctadecanoate** (111). ¹H NMR (400 MHz, CDCl₃) δ 5.05 - 4.90 (m, 1H), 4.81 (t, *J* = 5.9 Hz, 0H), 4.39 - 4.18 (m, 1H), 3.86 (t, *J* = 6.5 Hz, 2H), 2.48 (t, *J* = 7.5 Hz, 2H), 2.33 (t, *J* = 7.4 Hz, 2H), 2.25 (s, 6H), 1.94 - 1.77 (m, 4H), 1.69 - 1.53 (m, 11H), 1.45 (td, *J =* 13.9, 6.4 Hz, 5H), 1.29 (dd, *J =* 11.9, 6.1 Hz, 56H), 0.90 (t, *J* = 6.7 Hz, 12H).

### Example 10. Synthesis of 3-pentyloctyl 9-[N-(decyloxy)-4-(dimethylamino)butanamido]-2,2-difluorononadecanoate (39)

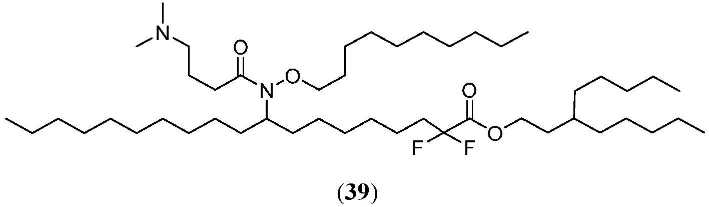

### Step 1. Synthesis of (decyloxy)(heptadec-1-en-7-yl)amine

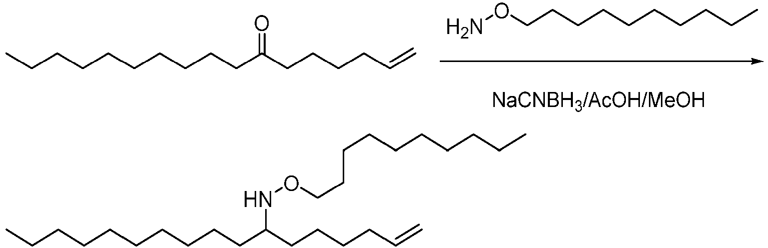

Heptadec-1-en-7-one (5.2 g, 20.8 mmol), AcOH (2.4 mL, 42 mmol) and NaCNBH₃ (4.0 g, 62.0 mmol) were stirred in THF (60 mL) at 50°C for 15 hr. The solvent was removed *in-vacuo* and the residue partitioned between H₂0 (50 mL) and DCM (50 mL). The aqueous layer was further extracted with DCM, the combined organics dried (Na₂SO₄) concentrated *in-vacuo* and the residue purified by automated flash chromatography (0-15% EtOAc/Hex) to give (decyloxy)(heptadec-1-en-7-yl)amine (4.04 g, 47.5 %).

### Step 2. Synthesis of N-(decyloxy)-4-(dimethylamino)-N-(heptadec-1-en-7-yl)butanamide

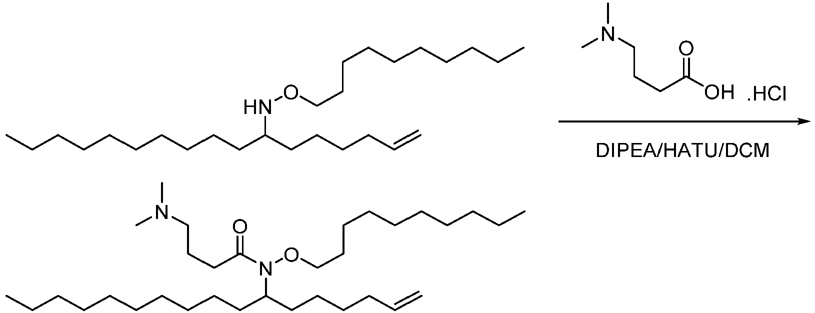

4-(Dimethylamino)butanoic acid HCl salt (3.3 g, 19.5 mmol), HATU (7.4 g, 19.5 mmol) and DIPEA (7.6 g, 58.6 mmol) were stirred in DCM (40 mL) at RT for 1 hr. (Decyloxy)(heptadec-1-en-7-yl)amine (4 g, 9.8 mmol) in DCM (10 mL) was added and the reaction heated at reflux for 16 hr. After cooling the reaction was washed sequentially with 1M HCl, sat NaHCO₃ and brine, dried (MgSO₄), filtered and the filtrate concentrated *in-vacuo.* The residue was purified by automated flash chromatography (0-10% MeOH/DCM) to give N-(decyloxy)-4-(dimethylamino)-N-(heptadec-1-en-7-yl)butanamide (4.1 g, 80.3 %).

### Step 3. Synthesis of ethyl 9-[N-(decyloxy)-4-(dimethylamino)butanamido]-2,2-difluorononadecanoate

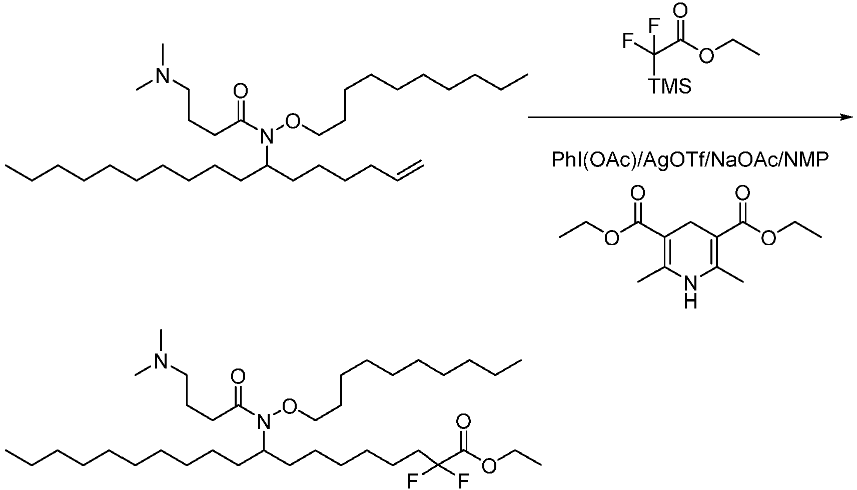

In a vacuum dried flask, Hantsch ester (2.0 g, 7.7 mmol), NaOAc (4.9 g, 15.3 mmol), AgOTf (4.9 g, 19.1 mmol) and iodobenzene diacetate (4.9 g, 15.3 mmol) were stirred under nitrogen in NMP (30 mL). N-(Decyloxy)-4-(dimethylamino)-N-(heptadec-1-en-7-yl)butanamide (4 g, 7.7 mmol) in NMP (30 mL) and ethyl 2,2-difluoro-2-(trimethylsilyl)acetate (7.5 g, 38.3 mmol) were added and the reaction was stirred at RT for 18 hr. The reaction was filtered through Celite and the filtrate washed with brine, dried (MgSO₄), filtered and concentrated *in-vacuo.* The crude material was purified by automated flash chromatography (0-50% ACN/DCM) to give ethyl 9-[N-(decyloxy)-4-(dimethylamino)butanamido]-2,2-difluorononadecanoate (3.2 g, 64.7 %).

### Step 4. Synthesis of 3-pentyloctyl 9-[N-(decyloxy)-4-(dimethylamino)butanamido]-2,2-difluorononadecanoate (39)

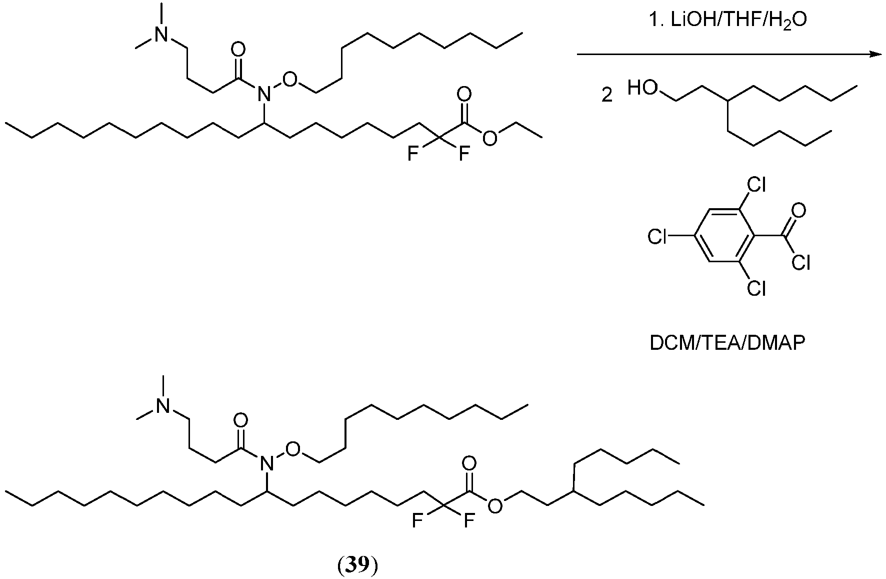

Ethyl 9-[N-(decyloxy)-4-(dimethylamino)butanamido]-2,2-difluorononadecanoate (731 mg, 1.1 mmol) and LiOH (108 mg, 4.5 mmol) were stirred in THF (10 mL) and water (2 mL) at RT until complete. The reaction was quenched with 6M HCl (0.75 mL) and partially concentrated *in-vacuo.* The reaction was diluted with DCM, washed with brine, dried (MgSO₄), filtered and concentrated *in-vacuo* azeotroping with toluene (25 mL). The crude carboxylic acid, 3-pentyloctan-1-ol (0.34 g, 1.7 mmol), 2,4,6-trichlorobenzoyl chloride (0.6 g, 2.3 mmol), TEA (137 mg, 1.4 mmol) and DMAP (179 mg, 1.5 mmol) were stirred in THF (6 mL) RT for 1.5 hr. The reaction was concentrated *in-vacuo* and the crude material purified by automated flash chromatography (0-100% DCM/Hex and 0-15% ACN/DCM) to give a mixture of 3-pentyloctyl 9-[N-(decyloxy)-4-(dimethylamino)butanamido]-2,2-difluorononadecanoate and an unsaturated impurity. The mixture was subjected to catalytic hydrogenation over Pd-C in EtOAc (10 mL) at RT for 1 hr. The reaction was filtered through celite and concentrated *in-vacuo* to give 3-pentyloctyl 9-[N-(decyloxy)-4-(dimethylamino)butanamido]-2,2-difluorononadecanoate **(39)** (371 mg, 41.0 %). ¹H NMR (400 MHz, CDCl3) δ 4.30 (t, J = 7.1 Hz, 2H), 4.24 (dd, J = 9.5, 4.6 Hz, 1H), 3.99 - 3.92 (m, 2H), 3.30 (dd, J = 12.4, 7.1 Hz, 2H), 2.98 (s, 6H), 2.92 (dd, J = 7.3, 4.3 Hz, 2H), 2.04 (dt, J = 11.6, 6.4 Hz, 4H), 1.74 - 1.61 (m, 6H), 1.53 - 1.19 (m, 57H), 0.94 - 0.87 (zm, 12H).

The following compound was prepared using analogous methodology as described above. 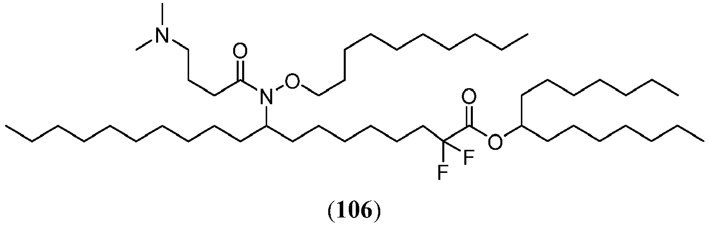 **Pentadecan-8-yl 9-(N-(decyloxy)-4-(dimethylamino)butanamido)-2,2-difluoro-nonadecanoate (106).** ¹H NMR (400 MHz, CDCl₃) δ 7.31, 7.28, 5.32, 5.03, 5.02, 5.00, 4.30, 3.88, 3.86, 3.84, 2.50, 2.48, 2.46, 2.34, 2.32, 2.30, 2.25, 2.07, 2.05, 2.03, 2.01, 1.99, 1.87, 1.85, 1.83, 1.81, 1.64, 1.62, 1.60, 1.58, 1.47, 1.30, 1.29, 1.27, 0.92, 0.91, 0.90, 0.88.

### Example 11. Synthesis of Synthesis of 3-{N-[3-(dimethylamino)propyl]decanamido}-dodecyl 2-fluoro-2-hexyldecanoate (40)

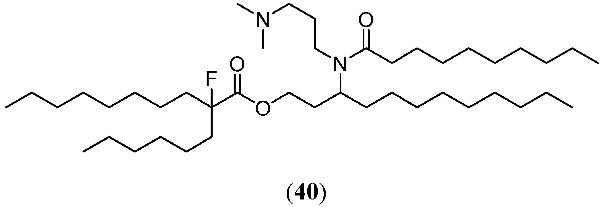

### Step 1. Synthesis of 3-{[3-(dimethylamino)propyl]amino}dodecan-1-ol

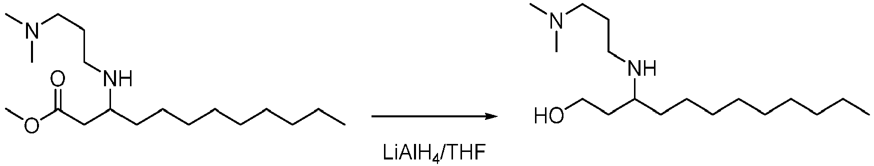

LiAlH₄ (1.7 g, 26.8 mmol) was stirred in THF (100 mL) at 0°C. Methyl 3-{[3-(dimethylamino)propyl]amino}dodecanoate (2.8 g, 8.9 mmol) in THF (100 mL) was added dropwise and the reaction stirred at RT until complete. The reaction was cooled to 0°C, 1M NaOH (20 mL) added and extracted with EtOAc. The organics were dried (MgSO₄), filtered, and the filtrate concentrated *in-vacuo* to give crude 3-{[3-(dimethylamino)propyl]amino}dodecan-1-ol (2.6 g, 100.0 %), which was used without purification.

### Step 2. Synthesis of benzyl N-[3-(dimethylamino)propyl]-N-(1-hydroxydodecan-3-yl)carbamate

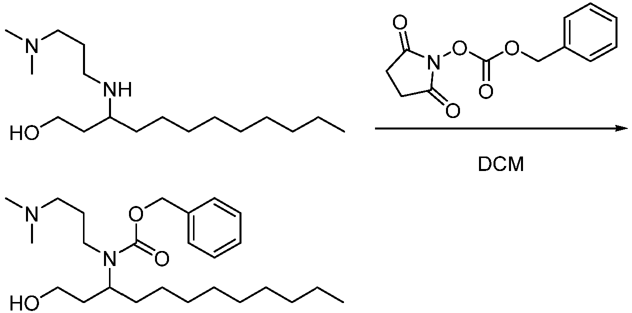

3-{[3-(Dimethylamino)propyl]amino}dodecan-1-ol (2.6 g, 8.9 mmol) and benzyl 2,5-dioxopyrrolidin-1-yl carbonate (2.2 g, 8.9 mmol) were stirred in DCM (50 mL) at 0°C for 16 hr allowing to warm to RT. The reaction was concentrated *in-vacuo* and the residue purified by automated flash chromatography (0-20% MeOH/DCM) to give benzyl N-[3-(dimethylamino)propyl]-N-(1-hydroxydodecan-3-yl)carbamate (2.1 g, 56.0 %)

### Step 3. Synthesis of 3-{[(benzyloxy)carbonyl][3-(dimethylamino)propyl]amino}dodecyl 2-fluoro-2-hexyldecanoate

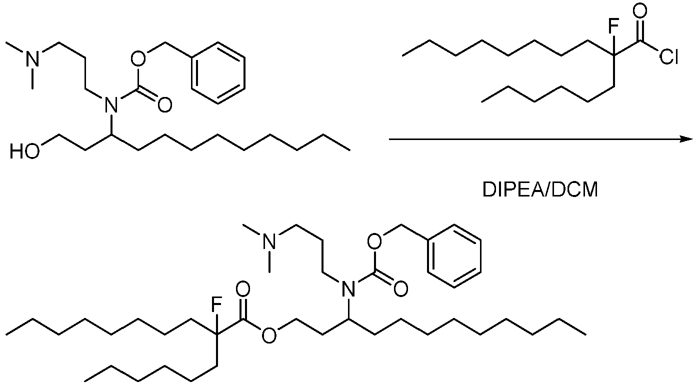

Benzyl N-[3-(dimethylamino)propyl]-N-(1-hydroxydodecan-3-yl)carbamate (230 mg, 0.5 mmol), 2-fluoro-2-hexyldecanoyl chloride (160 mg, 0.5 mmol) and DIPEA (0.2 mL, 1.1 mmol) were heated in DCM 80°C for 10 min under microwave irradiation. The reaction was concentrated *in-vacuo* and the residue purified by automated flash chromatography (0-15% MeOH/DCM) to give 3-{[(benzyloxy)carbonyl][3-(dimethylamino)propyl]amino }dodecyl 2-fluoro-2-hexyldecanoate (196 mg, 53.0 %).

### Step 4. Synthesis of 3-{[3-(dimethylamino)propyl]amino}dodecyl 2-fluoro-2-hexyldecanoate

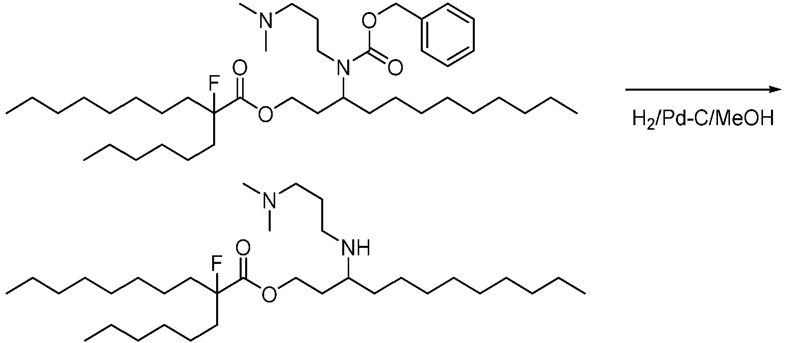

3-{[(Benzyloxy)carbonyl][3-(dimethylamino)propyl]amino}dodecyl 2-fluoro-2-hexyldecanoate (196 mg, 0.3 mmol) was subjected to catalytic hydrogenation over Pd/C in MeOH at RT for 16 h. The reaction was filtered and concentrated *in-vacuo* to give 3-{[3-(dimethylamino)propyl]amino}dodecyl 2-fluoro-2-hexyldecanoate (157 mg, 99.9%) which was used without further purification.

### Step 5. Synthesis of 3-{N-[3-(dimethylamino)propyl]decanamido}dodecyl 2-fluoro-2-hexyldecanoate (40)

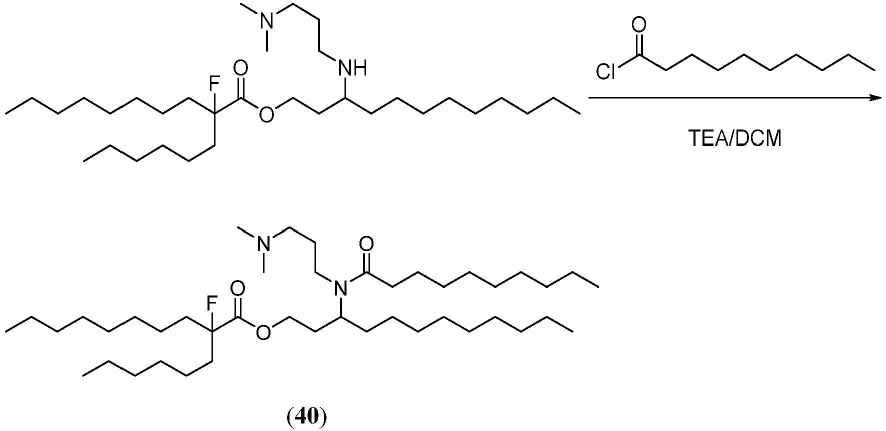

3-{[3-(Dimethylamino)propyl]amino}dodecyl 2-fluoro-2-hexyldecanoate (157 mg, 0.3 mmol), decanoyl chloride (61 mg, 0.32 mmol) and TEA (59 mg, 0.6 mmol) were refluxed in DCM (20 mL) for 24 hr. After cooling the reaction was washed with NaHCO₃, brine, dried (MgSO₄) and filtered. The filtrate was concentrated *in-vacuo* and the residue purified by automated flash chromatography (0-10% MeOH/DCM) to give 3-{N-[3-(dimethylamino)propyl]decanamido}dodecyl 2-fluoro-2-hexyldecanoate **(40)** (64 mg, 31.7%). ¹H NMR (400 MHz, CDCl3) δ 4.41 - 4.34 (m, 1H), 4.23 - 4.16 (m, 1H), 4.12 - 4.05 (m, 1H), 4.01 - 3.94 (m, 1H), 3.93 - 3.85 (m, 1H), 3.78 - 3.71 (m, 1H), 3.24 - 3.15 (m, 2H), 2.39 - 2.23 (m, 7H), 1.96 - 1.72 (m, 8H), 1.51 (d, J = 7.4 Hz, 8H), 1.39 - 1.08 (m, 42H), 0.90 (t, 12H).

### Example 12. Synthesis of Synthesis of 2-hexyldecyl 8-{8-[(2-butyloctyl)oxy]-N-[3-(dimethylamino)propoxy]-8-oxooctanamido}octadecenoate (41)

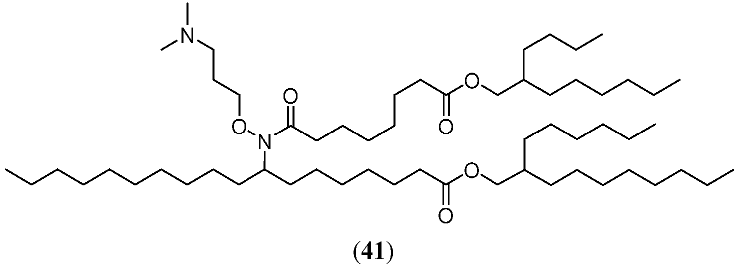

### Step 1. Synthesis of 2-butyloctyl 8-chloro-8-oxooctanoate

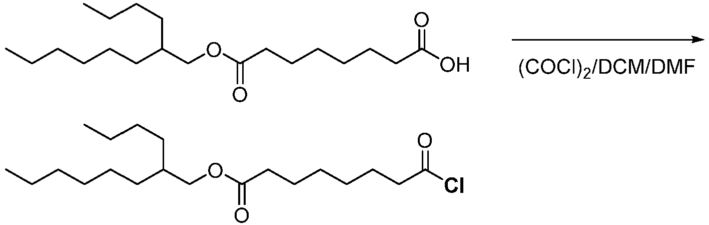

8-[(2-Butyloctyl)oxy]-8-oxooctanoic acid (120 mg, 0.35 mmol), oxalyl chloride (67 mg, 0.53 mmol) and DMF (cat) were stirred in DCM at RT for 3 hr. The reaction was concentrated *in-vacuo* and the residue dried under vacuum to give 2-butyloctyl 8-chloro-8-oxooctanoate (126 mg, 99.6%) which was used without purification.

### Step 2. Synthesis of 2-hexyldecyl 8-{[3-(dimethylamino)propoxy]amino}octadecenoate

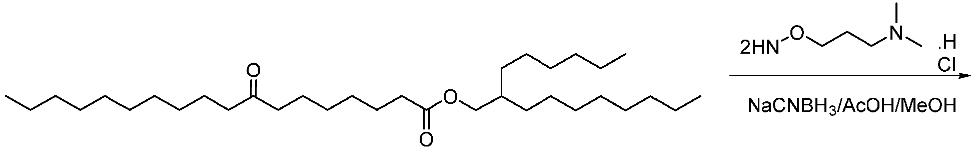

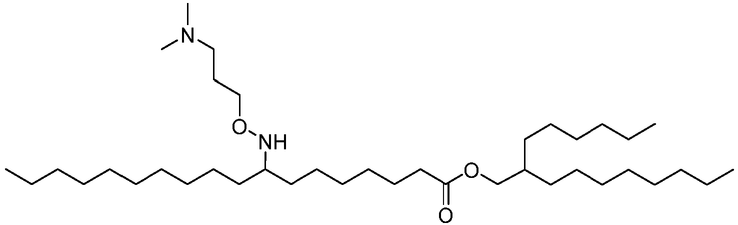

[3-(Dimethylamino)propoxy]amine (HCl salt) (148 mg, 1.0 mmol), 2-hexyldecyl 8-oxooctadecanoate (500 mg, 1.0 mmol), AcOH (115 mg, 1.9 mmol) and NaCNBH₃ (180 mg, 2.9 mmol) were stirred in MeOH (8 mL) at 45°C for 3 days. The reaction was concentrated *in-vacuo* and the residue partitioned between 1:1 DCM/H₂O. The aqueous was further extracted with DCM and the organics washed with brine, dried MgSO₄, filtered and concentrated *in-vacuo.* The residue was purified by automated flash chromatography (0-15% MeOH/DCM) to give 2-hexyldecyl 8-{[3-(dimethylamino)propoxy]amino}octadecanoate (385 mg, 64.4 %).

### Step 3. Synthesis of 2-hexyldecyl 8-{8-[(2-butyloctyl)oxy]-N-[3-(dimethylamino)propoxy]-8-oxooctanamido}octadecenoate (41)

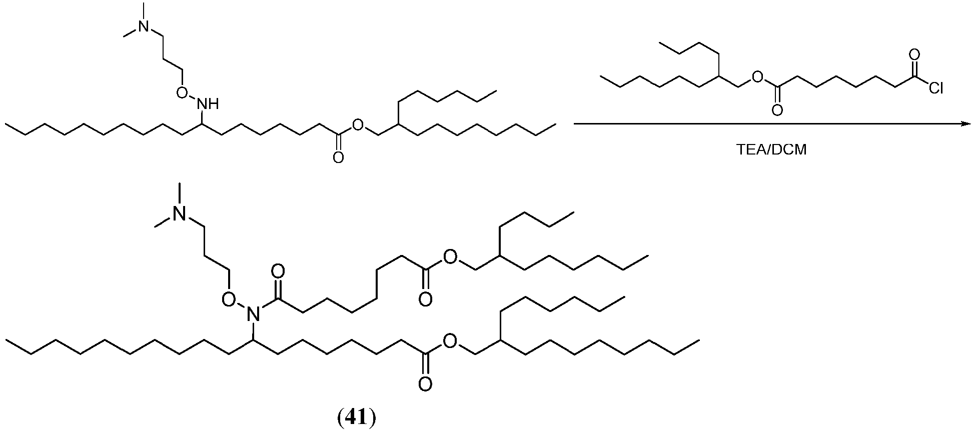

2-Hexyldecyl 8-{[3-(dimethylamino)propoxy]amino}octadecanoate (192 mg, 0.31 mmol) and TEA (62 mg, 0.6 mmol) was stirred in DCM at RT. 2-Butyloctyl 8-chloro-8-oxooctanoate (133 mg, 0.37 mmol,) in DCM was added and the reaction stirred at RT for 18 hr. The organics were washed with sat. NaHCO₃ and brine, dried (MgSO₄), filtered and concentrated *In-vacuo.* The crude material was purified by automated flash chromatography (0-15% MeOH/DCM) to give 2-hexyldecyl 8-{8-[(2-butyloctyl)oxy]-N-[3-(dimethylamino)propoxy]-8-oxooctanamido}octadecanoate **(41)** (88 mg, 30.2%). ¹H NMR (400 MHz, CDCl3) δ 4.32 - 4.24 (m, 1H), 3.99 (m, 4H), 3.93 - 3.89 (m, 2H), 2.53 - 2.13 (m, 14H), 1.60 - 1.42 (m, 14H), 1.43 - 1.02 (m, 66H), 0.94 - 0.85 (m, 15H).

### Example 13. Synthesis of 2-hexyldecyl 7-{N-[3-(dimethylamino)propoxy]decanamido}-heptadecanoate (42)

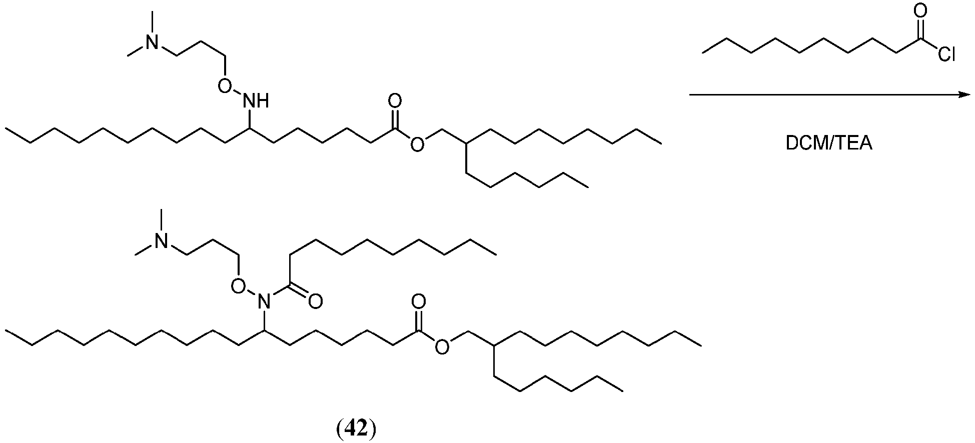

2-Hexyldecyl 7-{[3-(dimethylamino)propoxy]amino}heptadecanoate (133 mg, 0.22 mmol) and TEA (44 mg, 0.44 mmol) were stirred in DCM (3 mL) at RT. Decanoyl chloride (5 mg, 0.26 mmol) in DCM was added dropwise. The reaction was stirred at RT for 18 hr. The organics were washed with sat NaHCO₃ solution and brine, dried (MgSO₄), filtered and concentrated *in-vacuo.* The crude product was purified by automated flash chromatography (0-10% MeOH/DCM) to give 2-hexyldecyl 7-{N-[3-(dimethylamino)propoxy]decanamido}heptadecanoate **(42)** (51 mg, 30.6%). ¹H NMR (400 MHz, CDCl3) δ 4.30 (s, 2H), 3.98 (d, J = 5.8 Hz, 2H), 3.92 (s, 1H), 2.57 - 2.19 (m, 12H), 1.87 - 1.81 (m, 2H), 1.65 - 1.56 (m, 11H), 1.48 - 1.42 (s, 3H), 1.38 - 1.17 (m, 51H), 0.90 (td, J = 6.9, 1.8 Hz, 12H).

### Example 14. Synthesis of 8-[N-(decyloxy)-4-(dimethylamino)butanamido]-octadecenoate (43)

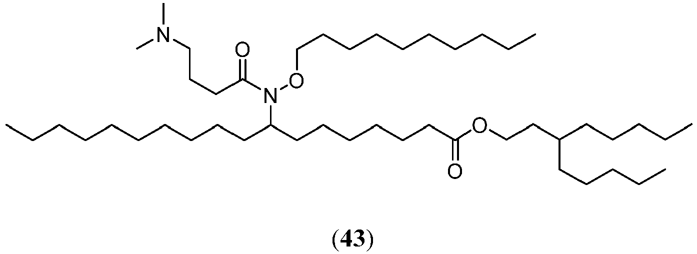

### Step 1. Synthesis of 3-pentyloctyl 8-[(decyloxy)amino]octadecenoate

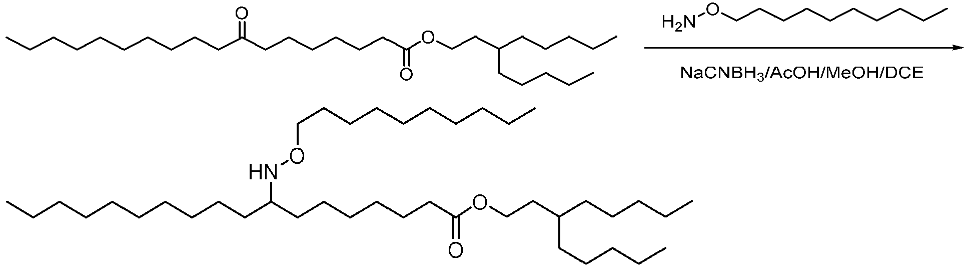

3-Pentyloctyl 8-oxooctadecanoate (13.3 g, 28 mmol), O-decylhydroxylamine (7.2 g, 41.5 mmol) AcOH (3.2 mL, 55 mmol) and NaCNBH₃ (5.2 g, 83 mmol) were stirred in MeOH (100 mL) and DCE (50 mL) at 50°C for 15 hr. Additional NaCNBH₃ (1eq) was added and the reaction stirred at 60°C for 72 hr. The reaction was concentrated *in-vacuo,* and the residue partitioned between H₂O (50 mL) and DCM (50 mL). The aqueous layer extracted with DCM and the combined organics dried MgSO₄, filtered, concentrated *in-vacuo* and the residue purified by automated flash chromatography to give 3-pentyloctyl 8-[(decyloxy)amino]octadecanoate (18 g, 28.2 mmol, Quant).

### Step 2. Synthesis of 3-pentyloctyl 8-[N-(decyloxy)-4-(dimethylamino)butanamido]-octadecenoate (43)

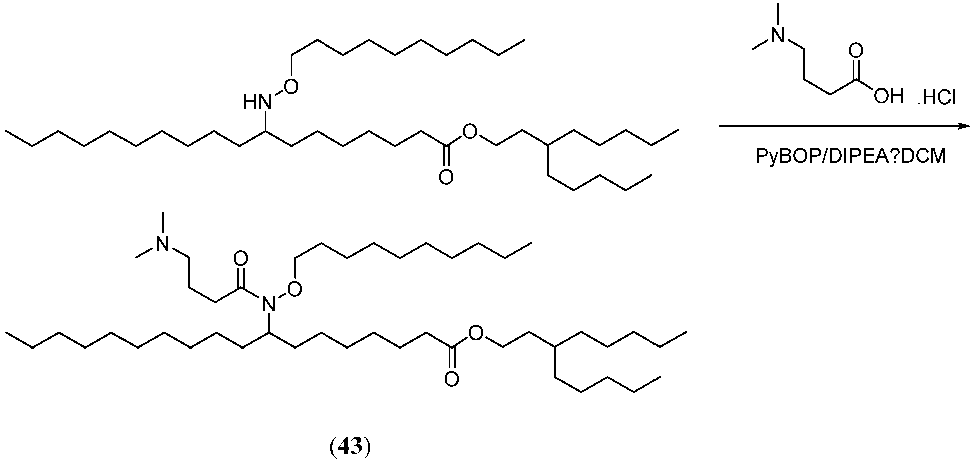

3-Pentyloctyl 8-[(decyloxy)amino]octadecanoate (16.21g, 25.4 mmol), 4-(dimethylamino)butane carboxylic acid HCl salt (4.7 g, 27.9 mmol), DIPEA (10.8 g, 83.8 mmol) and PyBOP (14.5 g, 27.9 mmol) were stirred in DCM (50 mL) at RT for 18 hr. The reaction was washed with 1M HCl, NaHCO₃ (sat. aq.) and brine, dried (MgSO₄), filtered and concentrated *in-vacuo.* The crude material was purified with automated flash chromatography (0-15% MeOH/DCM) to give 3-pentyloctyl 8-[N-(decyloxy)-4-(dimethylamino)butanamido]octadecanoate which was taken up in EtOAc, washed with sat NaHCO₃ (3 ×) and 1M NaOH (2 ×) twice, dried (MgSO₄), filtered and concentrated *in-vacuo* to give 8-[N-(decyloxy)-4-(dimethylamino)butanamido]octadecanoate **(43)** (6.4 g, 33.5 %). ¹H NMR (400 MHz, CDCl3) δ 4.25 (tt, J = 9.9, 5.0 Hz, 1H), 4.05 (t, J = 7.1 Hz, 2H), 3.81 (t, J = 6.5 Hz, 2H), 2.43 (t, J = 7.5 Hz, 2H), 2.26 (dt, J = 17.2, 7.4 Hz, 4H), 2.20 (s, 6H), 1.78 (p, J = 7.4 Hz, 2H), 1.56 (dq, J = 20.5, 6.6 Hz, 8H), 1.47 - 1.33 (m, 5H), 1.24 (dd, J = 13.4, 4.8 Hz, 50H), 0.85 (td, J = 6.9, 3.0 Hz, 12H).

The following compounds were prepared using analogous methodology as described above. 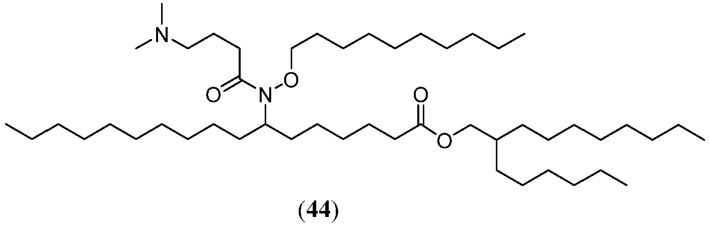 **2-Hexyldecyl 7-(N-(decyloxy)-4-(dimethylamino)butanamido)heptadecanoate (44).** ¹H NMR (400 MHz, CDCl3) δ 4.29 (s, 1H), 3.98 (d, J = 5.8 Hz, 2H), 3.86 (t, J = 6.5 Hz, 2H), 2.55 - 2.21 (m, 10H), 1.61-1.90 (m, 19H), 1.29 (m, 48H), 0.99 - 0.73 (m, 12H). 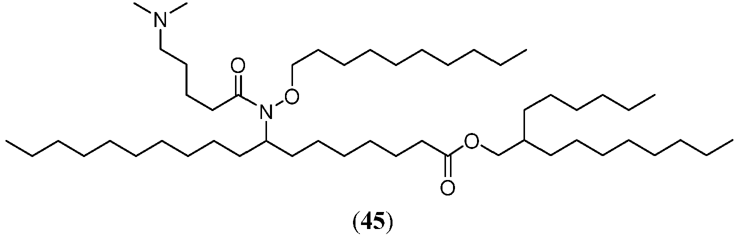 **2-Hexyldecyl 8-(N-(decyloxy)-5-(dimethylamino)pentanamido)octadecenoate (45).** ¹H NMR (400 MHz, CDCl3) δ 4.29 (s, 1H), 3.98 (d, J = 5.8 Hz, 2H), 3.85 (t, J = 6.5 Hz, 2H), 2.47 (t, J = 7.2 Hz, 2H), 2.38 (s, 2H), 2.29 (d, J = 7.8 Hz, 6H), 1.84 - 1.52 (m, 22H), 1.52 - 1.06 (m, 53H), 0.90 (td, J = 6.9, 2.0 Hz, 12H). 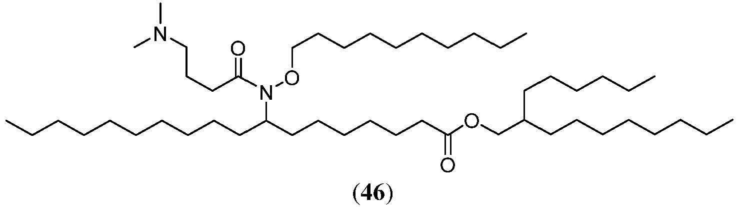 **2-Hexyldecyl 8-(N-(decyloxy)-4-(dimethylamino)butanamido)octadecenoate (46).** ¹H NMR (400 MHz, CDCl3) δ 4.28 (s, 1H), 3.98 (d, J = 5.8 Hz, 2H), 3.86 (t, J = 6.6 Hz, 2H), 2.51 (t, J = 7.3 Hz, 3H), 2.30 (t, J = 7.5 Hz, 4H), 1.96 - 1.83 (m, 2H), 1.63 (d, J = 16.9 Hz, 22H), 1.54 - 1.22 (m, 52H), 0.90 (td, J = 6.8, 2.1 Hz, 12H). 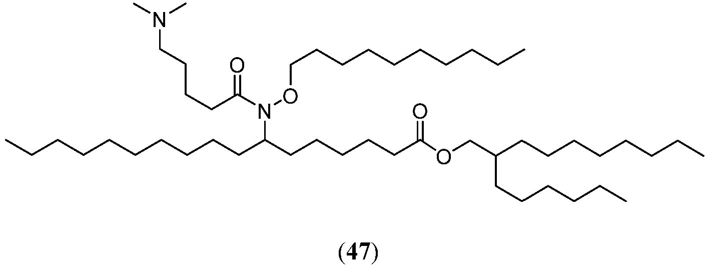 **2-Hexyldecyl 7-(N-(decyloxy)-5-(dimethylamino)pentanamido)heptadecanoate (47).** Product confirmed by MS (ESI +ve) 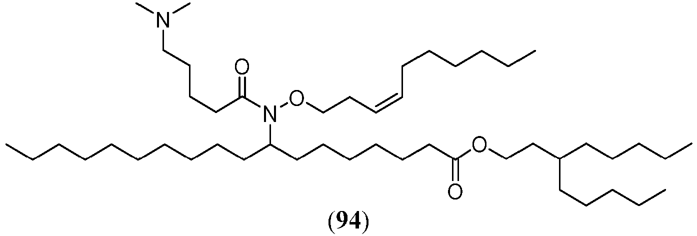 **3-Pentyloctyl (Z)-8-(N-(dec-3-en-1-yloxy)-5-(dimethylamino)pentanamido)octadecanoate (94).** ¹H NMR (400 MHz, CDCl₃) δ 5.51 - 5.30 (m, 2H), 4.39 - 4.19 (m, 1H), 4.10 (t, *J* = 7.1 Hz, 2H), 3.86 (t, *J* = 6.6 Hz, 2H), 2.47 (t, *J* = 7.3 Hz, 2H), 2.40 - 2.24 (m, 10H), 2.05 (dd, *J* = 13.8, 6.7 Hz, 2H), 1.95 (s, 7H), 1.76 - 1.49 (m, 7H), 1.44 - 1.20 (m, 44H), 0.91 (ddt, *J* = 7.0, 5.3, 2.6 Hz, 12H). 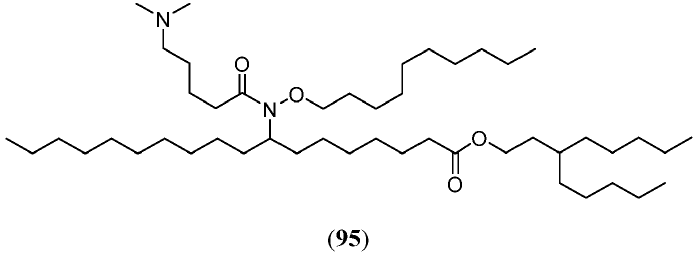 **3-Pentyloctyl 8-(N-(decyloxy)-5-(dimethylamino)pentanamido)octadecanoate (95).** ¹H NMR (400 MHz, CDCl₃) δ 4.29 (d, *J* = 4.9 Hz, 0H), 4.10 (t, *J* = 7.1 Hz, 2H), 3.85 (t, *J* = 6.5 Hz, 2H), 2.47 (t, *J =* 7.4 Hz, 2H), 2.34 (dd, *J* = 16.2, 8.8 Hz, 3H), 2.29 (d, *J* = 5.3 Hz, 7H), 1.74 - 1.51 (m, 20H), 1.51 - 1.14 (m, 57H), 0.90 (td, *J =* 6.9, 2.6 Hz, 12H). 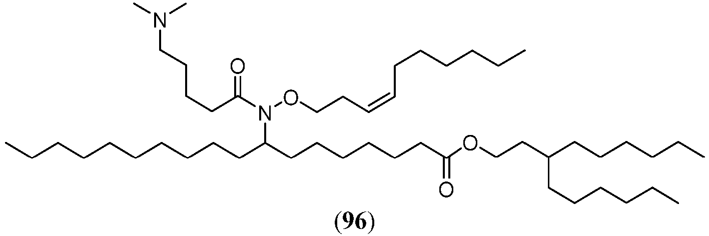 **3-Hexylnonyl (Z)-8-(N-(dec-3-en-1-yloxy)-5-(dimethylamino)pentanamido)octadecanoate (96).** ¹H NMR (400 MHz, CDCl₃) δ 5.50 - 5.31 (m, 2H), 4.29 (s, 1H), 4.10 (t, *J =* 7.1 Hz, 2H), 3.86 (t, *J* = 6.6 Hz, 2H), 2.47 (t, *J* = 7.4 Hz, 2H), 2.37 - 2.23 (m, 10H), 2.16 (q, *J* = 7.4 Hz, 2H), 2.05 (q, *J* = 7.1 Hz, 2H), 1.76 - 1.54 (m, 20H), 1.52 - 1.15 (m, 47H), 0.91 (tt, *J* = 6.8, 2.8 Hz, 12H). 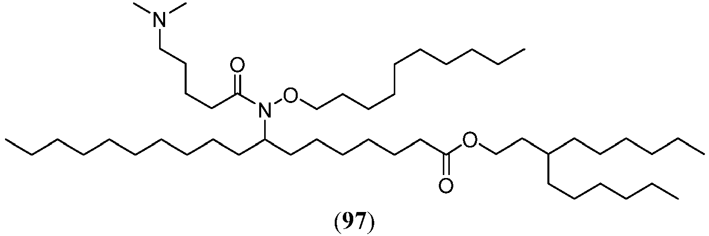 **3-Hexylnonyl 8-(N-(decyloxy)-5-(dimethylamino)pentanamido)octadecanoate (97).** ¹H NMR (400 MHz, CDCl₃) δ 4.29 (d, *J* = 5.1 Hz, 0H), 4.10 (t, *J* = 7.1 Hz, 2H), 3.85 (t, *J* = 6.6 Hz, 2H), 2.47 (t, *J* = 7.4 Hz, 2H), 2.38 - 2.22 (m, 9H), 1.74 - 1.51 (m, 17H), 1.50 - 1.19 (m, 57H), 0.90 (td, *J =* 6.8, 2.2 Hz, 12H). 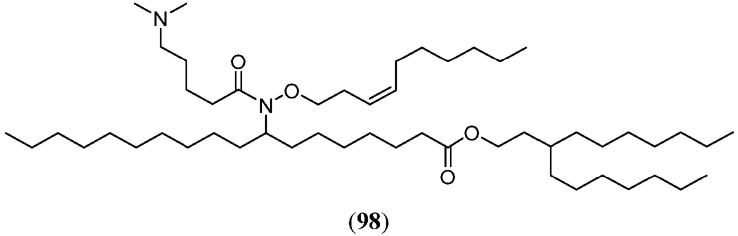 **3-Heptyldecyl (Z)-8-(N-(dec-3-en-1-yloxy)-5-(dimethylamino)pentanamido)octadecanoate (98).** ¹H NMR (400 MHz, CDCl₃) δ 5.51 - 5.31 (m, 2H), 4.29 (s, 1H), 4.09 (t, *J* = 7.1 Hz, 2H), 3.86 (t, *J* = 6.6 Hz, 2H), 2.48 (t, *J=* 7.2 Hz, 2H), 2.44 - 2.23 (m, 9H), 2.17 (q, *J* = 7.5 Hz, 2H), 1.77 - 1.18 (m, 70H), 0.90 (td, *J =* 6.7, 2.3 Hz, 12H). 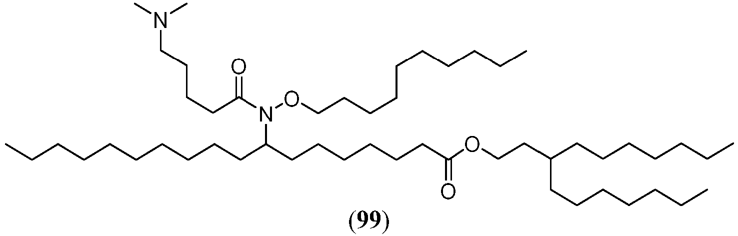 **3-Heptyldecyl 8-(N-(decyloxy)-5-(dimethylamino)pentanamido)octadecanoate. (99).** ¹H NMR (400 MHz, CDCl₃) δ 4.29 (s, 1H), 4.09 (t, *J* = 7.1 Hz, 2H), 3.85 (t, *J* = 6.5 Hz, 2H), 2.68 - 2.14 (m, 12H), 1.63 (tq, *J* = 13.9, 7.3 Hz, 21H), 1.53 - 1.16 (m, 60H), 1.00 - 0.79 (m, 12H). 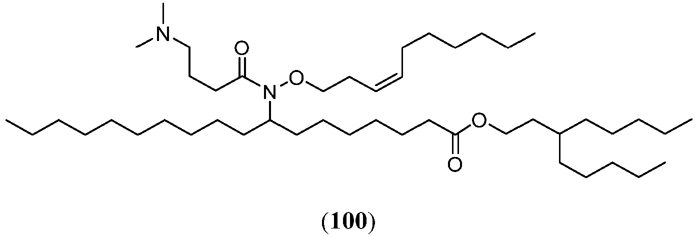 **3-Pentyloctyl (Z)-8-(N-(dec-3-en-1-yloxy)-4-(dimethylamino)butanamido)octadecanoate (100).** ¹H NMR (400 MHz, CDCl₃) δ 5.50 - 5.31 (m, 2H), 4.29 (s, 0H), 4.10 (t, *J* = 7.1 Hz, 2H), 3.87 (t, *J* = 6.6 Hz, 2H), 2.50 (t, *J* = 7.4 Hz, 2H), 2.40 (s, 2H), 2.33 - 2.27 (m, 7H), 2.16 (q, *J* = 7.4 Hz, 2H), 2.05 (q, *J* = 7.0 Hz, 2H), 1.87 (p, *J* = 7.4 Hz, 2H), 1.77 - 1.54 (m, 16H), 1.29 (d, *J* = 13.9 Hz, 42H), 0.91 (tt, *J =* 6.9, 2.8 Hz, 12H).

### Example 15. Synthesis of 2-hexyldecyl 3-{N-[3-(dimethylamino)propyl]octane-1-sulfonamido}dodecanoate (48)

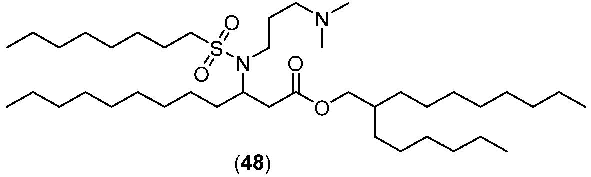

### Step 1. Synthesis of methyl 3-{[3-(dimethylamino)propyl]amino}dodecanoate

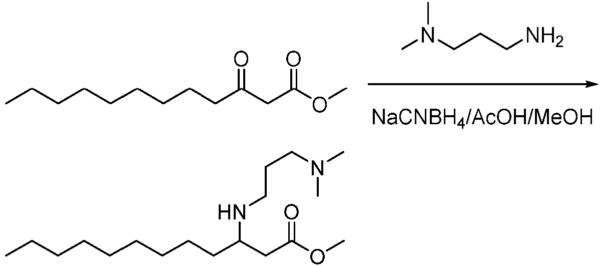

Methyl 3-oxododecanoate (5 g, 21.9 mmol), dimethylaminopropylamine (2.2 g, 21.9 mmol) and AcOH (1.4 g, 22.6 mmol) were stirred in MeOH (100 mL) at 75°C for 1 hr. After cooling NaCNBH₃ (2.8 g, 43.8 mmol was added in portions and the reaction stirred at RT for 18 hr. The reaction was concentrated *in-vacuo,* the residue taken up in EtOAc, washed with H₂O and brine, dried (MgSO₄), filtered and concentrated *in-vacuo.* The crude material was purified with automated flash chromatography (0-10% MeOH/DCM/0.5% TEA) to give methyl 3-{[3-(dimethylamino)propyl]amino}dodecanoate (2.62 g, 38.0 %).

### Step 2. Synthesis of methyl 3-{N-[3-(dimethylamino)propyl]octane-1-sulfonamido}dodecanoate

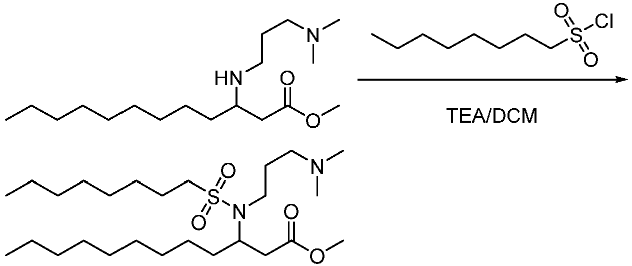

Methyl 3-{[3-(dimethylamino)propyl]amino}dodecanoate (300 mg, 1.0 mmol), TEA (193 mg, 1.9 mmol) and 1-octanesulfonyl chloride (213 mg, 1.0 mmol) were stirred in DCM at RT for 18 hr. The organics were washed with brine, dried (MgSO₄), filtered and concentrated *in-vacuo.* The residue was purified by automated flash chromatography (0-5% MeOH/DCM) to give methyl 3-{N-[3-(dimethylamino)propyl]octane-1-sulfonamido}dodecanoate (276 mg, 59.0%).

### Step 3. Synthesis of 3-{N-[3-(dimethylamino)propyl]octane-1-sulfonamido}dodecanoic acid

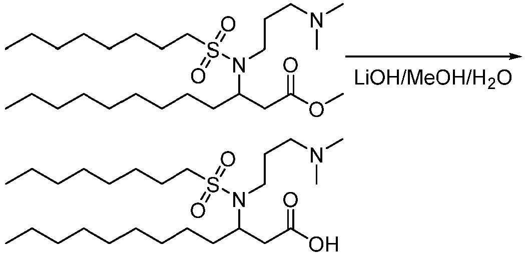

Methyl 3-{N-[3-(dimethylamino)propyl]octane-1-sulfonamido}dodecanoate (278 mg, 0.6 mmol) and LiOH (27 mg, 1.1 mmol) were stirred in MeOH (10 mL) and H₂O (2 mL) at RT for 18 hr. 6M HCl (0.19 mL, 6 M, 1.1 mmol) was added, the reaction diluted with water and extracted with DCM. The organics were dried (MgSO₄), filtered and concentrated *in-vacuo* to give 3-{N-[3-(dimethylamino)propyl]octane-1-sulfonamido}dodecanoic acid (0.27 g, Quant), which was used without purification.

### Step 4. Synthesis of 2-hexyldecyl 3-{N-[3-(dimethylamino)propyl]octane-1-sulfonamido}dodecanoate (48)

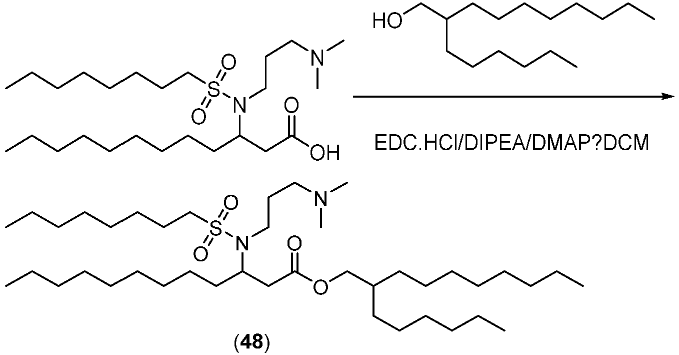

3-{N-[3-(Dimethylamino)propyl]octane-1-sulfonamido}dodecanoic acid (0.27 g, 0.6 mmol), hexyldecanol (151 mg, 0.6 mmol), EDC HCl (217 mg, 1.1 mmol), DIPEA (0.3 mL, 1.7 mmol) and DMAP (cat) were stirred at RT for 18 hr. The organics were washed with sat NaHCO₃ and brine, dried (MgSO₄) filtered and concentrated *in-vacuo.* The residue was purified by automated flash chromatography (0-12% MeOH/DCM) and (0-2% MeOH/EtOAc) to give 2-hexyldecyl 3-{N-[3-(dimethylamino)propyl]octane-1-sulfonamido}dodecanoate **(48)** (61 mg, 15.4%). ¹H NMR (400 MHz, CDCl3) δ 4.05 - 4.00 (m, 3H), 3.25 - 3.14 (m, 2H), 2.95 (td, J = 7.0, 3.2 Hz, 2H), 2.71 - 2.53 (m, 2H), 2.32 - 2.24 (m, 7H), 1.91 - 1.58 (m, 12H), 1.43 - 1.28 (m, 44H), 0.93- 0.75 (m, 12H).

The following compound was prepared using analogous methodology as described above. 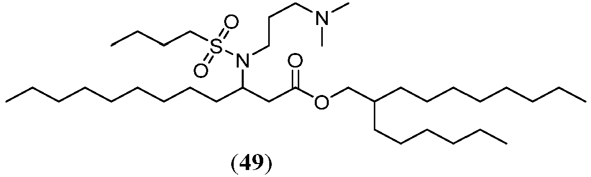 **2-Hexyldecyl 3-(N-(3-(dimethylamino)propyl)butylsulfonamido)dodecanoate (49).** Product confirmed by MS (ESI +ve)

### Example 16. Synthesis of Decyl 3-{N-[3-(dimethylamino)propyl]2-hexyldecanesulfonamido}dodecanoate (50)

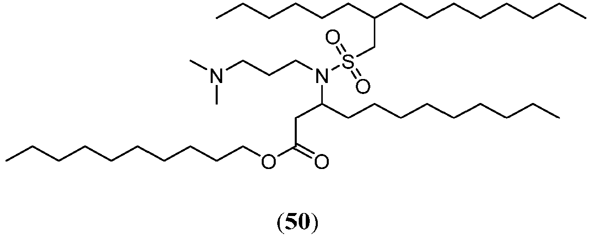

### Step 1. Synthesis of 2-hexyldecyl methanesulfonate

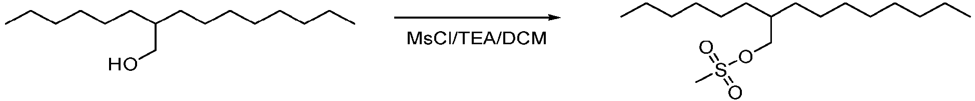

Hexyldecanol (2 g, 8.2 mmol) and TEA (1.44 mL, 10.3 mmol) were stirred in DCM (30 mL) at 0°C. MsCl (0.7 mL, 9.1 mmol) was added dropwise and the reaction stirred at RT for 2 hr. The reaction was quenched with sat NaHCO₃ and diluted with Et₂O.The organics were washed with brine, dried (MgSO₄) and concentrated *in-vacuo* to give crude 2-hexyldecyl methanesulfonate (2.64 g, Quant) which was used without purification.

### Step 2. Synthesis of 7-(bromomethyl)pentadecane

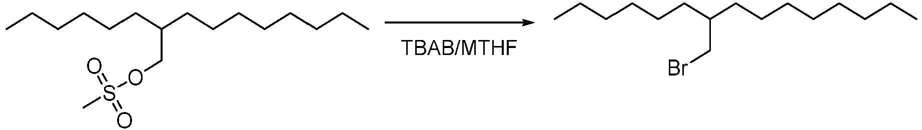

2-Hexyldecyl methanesulfonate (2.64 g, 8.2 mmol) and TBAB (3.5 g, 10.7 mmol) were stirred in methyl THF (50 mL) at 80°C for 2.5 hr. The reaction was poured into ice water and extracted with hexane. The aqueous layer was back extracted with hexane and the combined organics washed with brine, dried (MgSO₄), filtered and concentrated *in-vacuo.* The crude material was purified by automated flash chromatography (100% Hexane) to give 7-(bromomethyl)pentadecane (2.52 g, 95.2%).

### Step 3. Synthesis of 2-hexyldecane-1-sulfonyl chloride

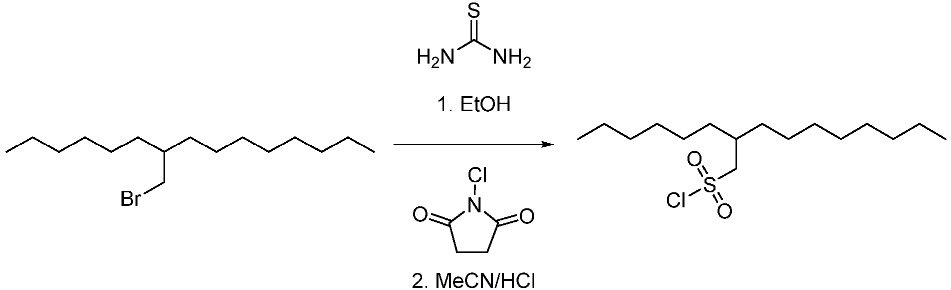

7-(Bromomethyl)pentadecane (500 mg, 1.64 mmol) and thiourea (124 mg, 1.64 mmol) were refluxed in EtOH (10 mL) for 1 hr. The solvent was removed *in-vacuo* and the residue stirred with chlorosuccinimide (1.1 g, 8.2 mmol), 2M HCl (1.1 mL, 1.1 mmol) and MeCN (10 mL) maintaining the temperature between 10 and 20°C for 15 min. The reaction was partitioned between Et₂O (15 mL) and H₂O (15 mL). The organics were separated, dried (Na₂SO₄), concentrated *in-vacuo* and the crude purified by automated flash chromatography (PE-EtOAc, 5:1) to give 2-hexyldecane-1-sulfonyl chloride (436 mg, 82.0 %).

### Step 4. Synthesis of methyl 3-{N-[3-(dimethylamino)propyl]2-hexyldecanesulfonamido}-dodecanoate

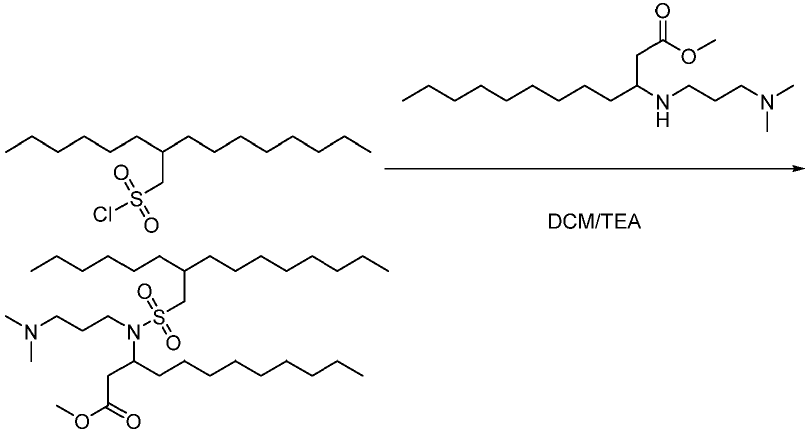

Methyl 3- f [3-(dimethylamino)propyl]amino}dodecanoate (426 mg, 1.4 mmol) TEA (380 µL, 2.7 mmol) and 2-hexyldecane-1-sulfonyl chloride (462 mg, 1.4 mmol) were stirred in DCM (10 mL) at RT for 2 days. The organics were washed with brine, dried (MgSO₄), filtered, the filtrate concentrated *in-vacuo* and the residue purified by automated flash chromatography (0-10% MeOH/DCM) to give methyl 3-{N-[3-(dimethylamino)propyl]2-hexyldecanesulfonamido}dodecanoate (366 mg, 44.8%).

### Step 5. Synthesis of 3-{N-[3-(dimethylamino)propyl]2-hexyldecanesulfonamido}-dodecanoic acid

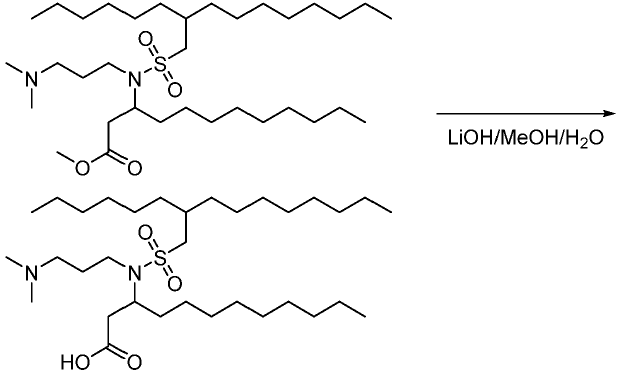

Methyl 3-{N-[3-(dimethylamino)propyl]2-hexyldecanesulfonamido}dodecanoate (366 mg, 0.61 mmol) and LiOH (29 mg, 1.2 mmol) were stirred in MeOH (10 mL) and H₂O (2 mL) at RT for 18 hr. The reaction was quenched with 6 M HCl (0.2 mL), diluted with H₂O and extracted with DCM. The organics were dried (MgSO₄), filtered and concentrated *in-vacuo* to give 3-{N-[3-(dimethylamino)propyl]2-hexyldecanesulfonamido}dodecanoic acid (291 mg, 81.4 %) which was used without further purification.

### Step 6. Synthesis of decyl 3-{N-[3-(dimethylamino)propyl]2-hexyldecane-sulfonamido}dodecanoate (50)

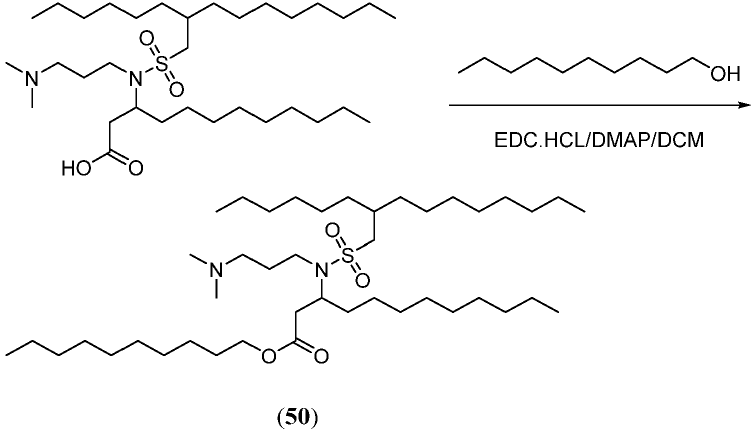

Decanol (86 mg, 0.54 mmol), 3-{N-[3-(dimethylamino)propyl]2-hexyldecane-sulfonamido}dodecanoic acid (291 mg, 0.5 mmol), EDC HCl (189 mg, 1.0 mmol), DIPEA (0.26 mL, 1.5 mmol) and DMAP (6 mg, 0.05 mmol) were stirred in DCM (2 mL) at RT for 18 hr. The organics were washed with sat NaHCO₃ and brine, dried (MgSO₄), filtered and concentrated under *in-vacuo.* The residue was purified by automated flash chromatography (0-2% MeOH/EtOAc) twice, to give decyl 3-{N-[3-(dimethylamino)propyl]2-hexyldecane-sulfonamido}dodecanoate (50) (41mg, 11.4%) . ¹H NMR (400 MHz, CDCl3) δ 4.11 - 4.04 (m, 3H), 3.20 - 3.14 (m, 2H), 2.91 (dd, J = 13.7, 6.2 Hz, 1H), 2.80 (dd, J = 13.7, 6.1 Hz, 1H), 2.65 (dd, J = 15.4, 7.1 Hz, 1H), 2.55 (dd, J = 15.5, 6.9 Hz, 1H), 2.31 - 2.23 (m, 7H), 2.08 - 2.02 (m, 1H), 1.84 - 1.63 (m, 10H), 1.42-1.49 (m, 5H), 1.49 - 1.28 (m, 44H), 0.90 (t, J = 6.6 Hz, 12H).

### Example 17. Synthesis of 3-{N-[3-(dimethylamino)propyl]undecane-1-sulfonamido}dodecanoate (51)

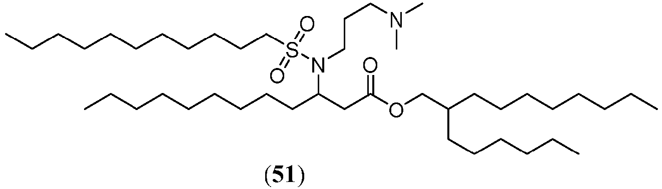

### Step 1. Synthesis of methyl 3-{N-[3-(dimethylamino)propyl]undecane-1-sulfonamido}dodecanoate

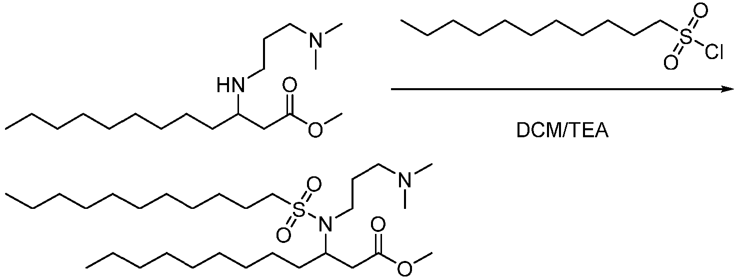

Methyl 3-{[3-(dimethylamino)propyl]amino}dodecanoate (939 mg, 3.0 mmol) TEA (0.8 mL, 6.0 mmol) and undecane-1-sulfonyl chloride (989 mg, 3.9 mmol) were stirred in DCM (30 mL) at RT for 18 hr. The organics were washed with brine, dried (MgSO₄), filtered and the filtrate was concentrated *in-vacuo* The residue was purified by automated flash chromatography (0-10% MeOH/DCM) to give methyl 3-{N-[3-(dimethylamino)propyl]undecane-1-sulfonamido}dodecanoate (564 mg, 35.5%).

### Step 2. Synthesis of 3-{N-[3-(dimethylamino)propyl]undecane-1-sulfonamido}dodecanoic acid

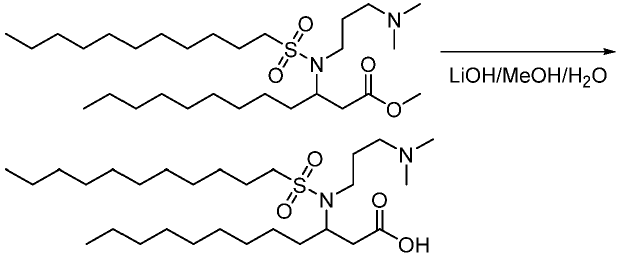

Methyl 3-{N-[3-(dimethylamino)propyl]undecane-1-sulfonamido}dodecanoate (564 mg, 1.1 mmol) and LiOH (76 mg, 3.2 mmol) were stirred in MeOH (10 mL) and H₂O (2 mL) at RT for 18 hr. 6M HCl (0.53 mL,) was added, the reaction diluted with H₂O and extracted with DCM. The organics were dried (MgSO₄), filtered and concentrated *in-vacuo* to give 3-{N-[3-(dimethylamino)propyl]undecane-1-sulfonamido}dodecanoic acid , which was used without purification.

### Step 3. Synthesis of 2-hexyldecyl 3-{N-[3-(dimethylamino)propyl]undecane-1-sulfonamido}dodecanoate (51)

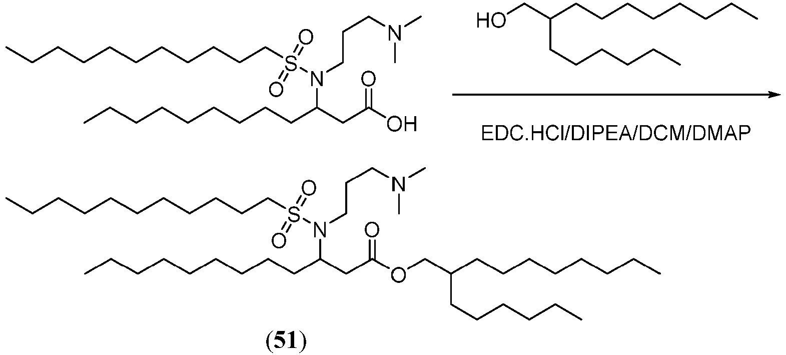

Hexyldecanol (139 mg, 0.6 mmol), 3-{N-[3-(dimethylamino)propyl]undecane-1-sulfonamido}dodecanoic acid (270 mg, 0.52 mmol), EDC HCl (0.2 g, 1.04 mmol), DIPEA (202 mg, 0.3 mL, 1.56 mmol) and DMAP (13 mg, 0.1 mmol) were stirred in DCM (10 mL) at RT for 18 hr. the organics were washed with sat NaHCO₃ and brine, dried (MgSO₄), filtered and concentrated *in-vacuo.* The residue was purified by automated flash chromatography (0-4% MeOH/EtOAc and 0-10% MeOH/DCM) to give 2-hexyldecyl 3-{N-[3-(dimethylamino)propyl]undecane-1-sulfonamido}dodecanoate **(51)** (50 mg, 13.0%) . ¹H NMR (400 MHz, CDCl3) δ 4.08 - 3.89 (m, 3H), 3.29 - 3.08 (m, 2H), 3.01 - 2.83 (m, 2H), 2.72 - 2.47 (m, 2H), 2.33 - 2.09 (m, 7H), 1.86 - 1.45 (m, 10H), 1.44 - 0.94 (m, 52H), 0.87 (t, J = 6.5 Hz, 12H).

The following compound was prepared using analogous methodology as described above 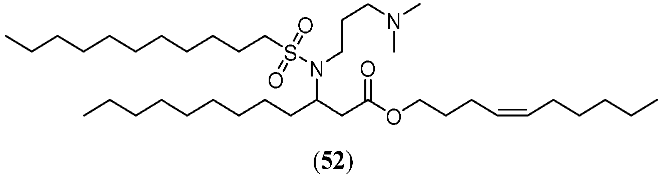 **(Z)-Dec-4-en-1-yl 3-(N-(3-(dimethylamino)propyl)undecylsulfonamido)dodecanoate (52).** ¹H NMR (400 MHz, CDCl3) δ 5.46 - 5.26 (m, 2H), 4.15 - 3.91 (m, 3H), 3.23 - 3.06 (m, 2H), 3.01 - 2.82 (m, 2H), 2.70 - 2.46 (m, 2H), 2.35 - 2.13 (m, 7H), 2.05 (dq, J = 35.5, 7.2 Hz, 4H), 1.89 - 1.45 (m, 10H), 1.44 - 0.99 (m, 35H), 0.94 - 0.80 (m, 9H).

### Example 18. Synthesis of N-[3-(dimethylamino)propyl]-N-[1-(N',N'-dioctylhydrazinecarbonyl)undecan-2-yl]decanamide (53)

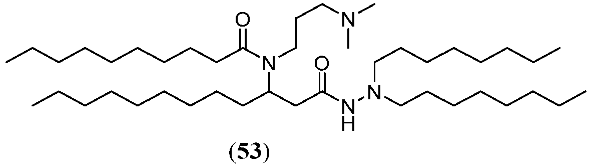

### Step 1. Synthesis of methyl 3-{N-[3-(dimethylamino)propyl]decanamido}dodecanoate

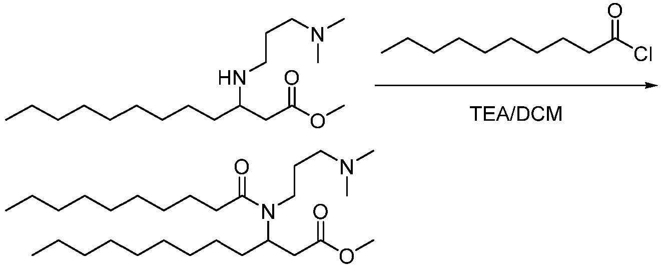

Methyl 3-{[3-(dimethylamino)propyl]amino}dodecanoate (2 g, 6.4 mmol) and TEA (4.6 mL, 12.7 mmol)) were stirred in DCM at RT. Decanoyl chloride (1.3 g, 7.0 mmol) was added dropwise and the reaction stirred at RT for 18 hr. The organics were washed with brine, dried (MgSO₄), filtered and concentrated *in-vacuo.* The residue was purified by (0-10% MeOH/DCM) to give methyl 3-{N-[3-(dimethylamino)propyl]decanamido }dodecanoate (2 g, 67.4%).

### Step 2. Synthesis of 3-{N-[3-(dimethylamino)propyl]decanamido}dodecanoic acid

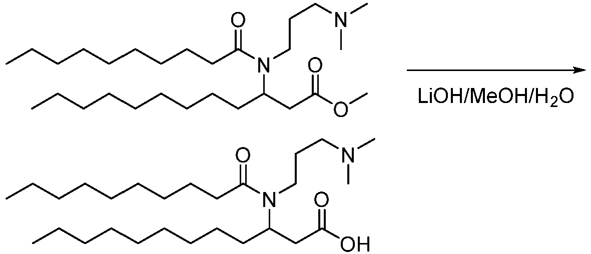

Methyl 3-{N-[3-(dimethylamino)propyl]decanamido }dodecanoate (3.4 g, 7.3 mmol) and LiOH (347 mg, 14.5 mmol) were stirred in MeOH (8 mL) and H₂O (2 mL) at RT for 18 hr. 6 M HCl (2.4 mL) was added and the reaction extracted with DCM. The organics were combined, dried (MgSO₄), filtered and concentrated *in-vacuo* to give 3-{N-[3-(dimethylamino)propyl]decanamido}dodecanoic acid (3.03 g, 91.9%) which was used without purification.

### Step 3. Synthesis of N-[3-(dimethylamino)propyl]-N-[1-(N',N'-dioctylhydrazine-carbonyl)undecan-2-yl]decanamide (53)

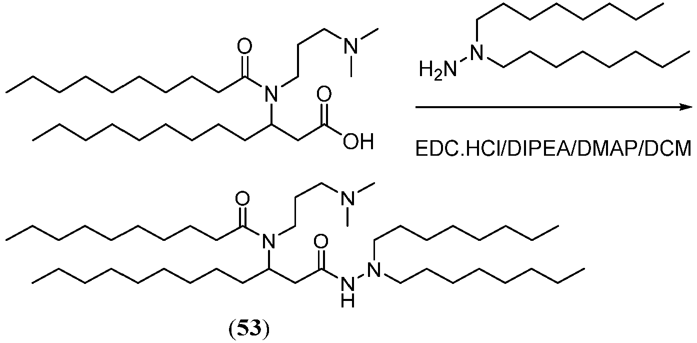

1,1-Dioctylhydrazine (782 mg, 3.0 mmol), 3-{N-[3-(dimethylamino)propyl]-decanamido}dodecanoic acid (1.5 g, 3.35 mmol), EDC HCl (1.17 g, 6.1 mmol) and DMAP (37 mg, 0.31 mmol) were stirred in DCM at RT for 16 hr. The reaction was quenched with sat. NaHCO₃, extracted with DCM, the organics washed with brine, dried (MgSO₄) and concentrated *in-vacuo.* The residue was purified by automated flash chromatography (5% MeOH/DCM) to give N-[3-(dimethylamino)propyl]-N-[1-(N',N'-dioctylhydrazinecarbonyl)undecan-2-yl]decanamide **(53)** (495 mg, 23.4 %). ¹H NMR (400 MHz, CDCl3) δ 5.74 (s, 1H), 3.32 - 3.07 (m, 2H), 2.83 - 2.55 (m, 5H), 2.55 - 2.14 (m, 12H), 1.79 - 1.64 (m, 5H), 1.55 - 1.40 (m, 5H), 1.39 - 0.99 (m, 46H), 0.96 - 0.84 (m, 12H).

The following compound was prepared using analogous methodology as described above. 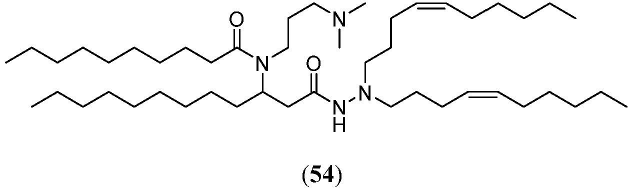 **N-(1-(2,2-di((Z)-Dec-4-en-1-yl)hydrazineyl)-1-oxododecan-3-yl)-N-(3-(dimethylamino)-propyl)decanamide (54).** ¹H NMR (400 MHz, CDCl3) δ 5.72 (d, J = 31.3 Hz, 1H), 5.45 - 5.22 (m, 4H), 4.45 (d, J = 57.4 Hz, 1H), 3.31 - 3.16 (m, 1H), 2.80 - 2.59 (m, 6H), 2.59 - 2.20 (m, 9H), 2.11 - 1.93 (m, 8H), 1.82 - 1.43 (m, 10H), 1.43 - 1.13 (m, 40H), 0.99 - 0.78 (m, 12H).

### Example 19. Synthesis of N-[3-(dimethylamino)propyl]-N-{1-[N'-(heptadecan-9-ylidene)hydrazinecarbonyl]undecan-2-yl}decanamide (55)

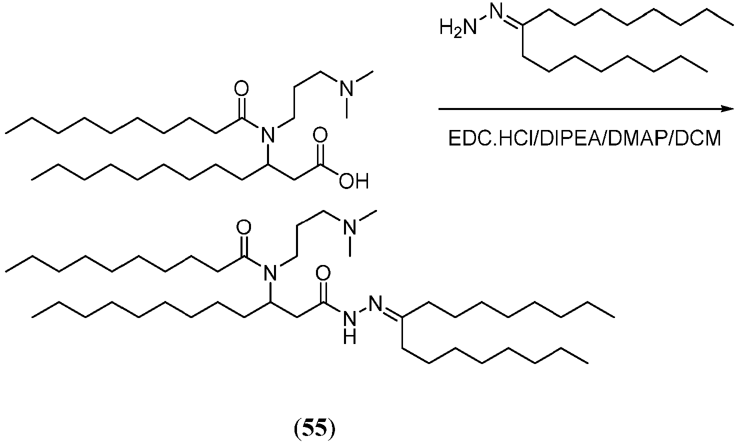

Heptadecan-9-ylidenehydrazine (231 mg, 0.86 mmol), 3-{N-[3-(dimethylamino)-propyl]decanamido} dodecanoic acid (430mg, 0.95 mmol), EDC HCl (330 mg, 1.7 mmol) and DMAP (10 mg, 0.1 mmol) were stirred in DCM (20 mL) at RT for 16 hr. The reaction was quenched with sat. NaHCO₃ solution and extracted with DCM. The organics were washed with brine, dried (MgSO₄) concentrated *in-vacuo* and the residue purified by automated flash chromatography (5% MeOH/DCM) to give N-[3-(dimethylamino)propyl]-N-{1-[N'-(heptadecan-9-ylidene)hydrazinecarbonyl]undecan-2-yl}decanamide **(55)** (176 mg, 29.0 %). ¹H NMR (400 MHz, CDCl3) δ 8.19 (s, 1H), 4.53 - 4.38 (m, 1H), 3.73 - 3.65 (m, 1H), 3.39 - 3.09 (m, 3H), 2.95 - 2.86 (m, 1H), 2.82 - 2.65 (m, 3H), 2.59 - 2.19 (m, 12H), 2.19 - 2.10 (m, 2H), 1.57 - 1.41 (m, 6H), 1.40 - 1.15 (m, 46H), 0.95 - 0.84 (m, 12H).

The following compounds were prepared using analogous methodology as described above. 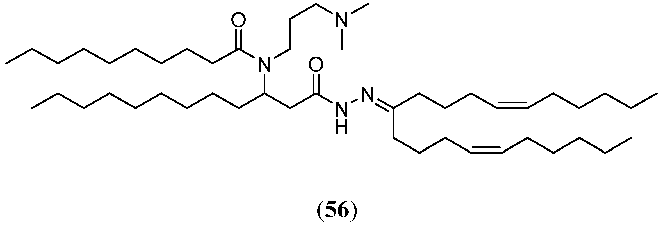 **N-(3-(Dimethylamino)propyl)-N-(1-(2-((6Z,15Z)-henicosa-6,15-dien-11-ylidene)hydrazineyl)-1-oxododecan-3-yl)decanamide (56).** ¹H NMR (400 MHz, CDCl3) δ 8.21 (s, 1H), 5.49 - 5.30 (m, 4H), 4.58 - 4.35 (m, 3H), 3.35 - 3.12 (m, 3H), 3.04 - 2.59 (m, 7H), 2.54 - 2.21 (m, 10H), 2.18 - 1.77 (m, 12H), 1.51 - 1.05 (m, 40H), 0.95 - 0.77 (m, 12H). 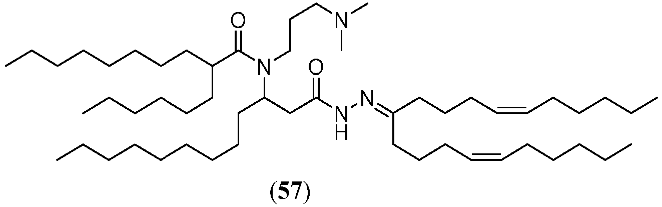 **N-(3-(dimethylamino)propyl)-N-(1-(2-((6Z,15Z)-henicosa-6,15-dien-11-ylidene)hydrazineyl)-1-oxododecan-3-yl)-2-hexyldecanamide (57).** ¹H NMR (400 MHz, CDCl3) δ 5.53 - 5.22 (m, 4H), 4.57 - 4.38 (m, 2H), 4.32 - 4.19 (m, 2H), 3.44 - 3.15 (m, 4H), 3.15 - 2.15 (m, 16H), 2.15 - 1.75 (m, 9H), 1.60 - 1.01 (m, 52H), 0.97 - 0.67 (m, 15H).

### Example 20. Synthesis of N-[3-(dimethylamino)propyl]-N-{1-[N'-(heptadecan-9-yl)hydrazinecarbonyl]undecan-2-yl}decanamide (58)

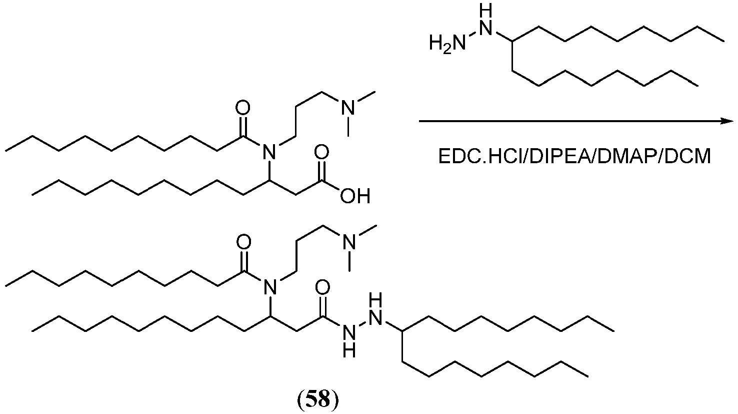

Heptadecan-9-ylhydrazine (500 mg, 1.9 mmol.), 3-{N-[3-(dimethylamino)-propyl]decanamido}dodecanoic acid (925 mg, 2.0 mmol), EDC.HCl (709 mg, 3.7 mmol) and DMAP (23 mg, 0.19 mmol were stirred at RT for 16 hr. The reaction was quenched with sat. NaHCO₃ solution, extracted with DCM, washed with brine, dried (MgSO₄) and concentrated *in-vacuo.* The residue was purified by automated flash chromatography (0-100% EtOAc/Hex and 0-10% MeOH/DCM) to give N-[3-(dimethylamino)propyl]-N-{1-[N'-(heptadecan-9-yl)hydrazinecarbonyl]undecan-2-yl}decanamide **(58)** (598 mg, 45.7 %). ¹H NMR (400 MHz, CDCl3) δ 8.29 - 8.15 (m, 1H), 4.45 (s, 1H), 3.54 - 3.13 (m, 4H), 2.76 - 2.03 (m, 14H), 2.01 - 1.70 (m, 2H), 1.59 - 1.03 (m, 56H), 1.02 - 0.81 (m, 12H).

The following compound was prepared using analogous methodology as described above. 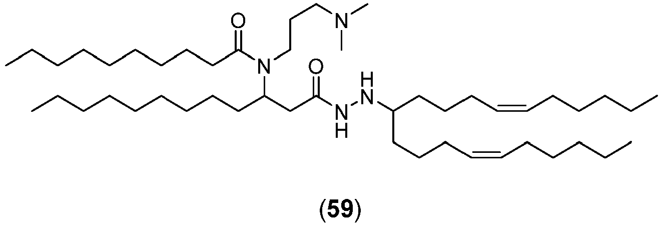 **N-(3-(Dimethylamino)propyl)-N-(1-(2-((6Z,15Z)-henicosa-6,15-dien-11-yl)hydrazineyl)-1-oxododecan-3-yl)decanamide (59).** ¹H NMR (400 MHz, CDCl3) δ 8.20 (d, J = 25.7 Hz, 1H), 5.47 - 5.26 (m, 4H), 3.34 - 3.19 (m, 2H), 3.09 - 2.95 (m, 1H), 2.79 - 2.66 (m, 1H), 2.62 - 2.19 (m, 10H), 2.11 - 1.97 (m, 8H), 1.77 - 1.46 (m, 7H), 1.45 - 1.13 (m, 48H), 0.94 - 0.86 (m, 12H).

### Example 21. Synthesis of (3,5-dihexylphenyl)methyl 6-{N-[3-(dimethylamino)-propyl]decanamido}hexadecanoate (60)

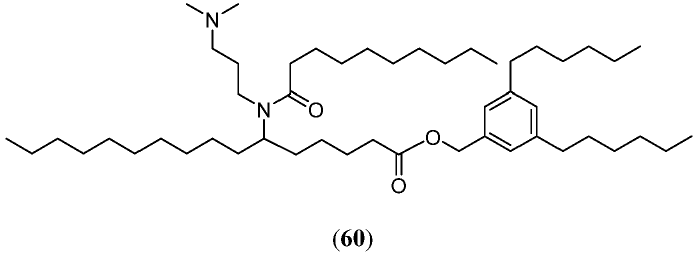

### Step 1. Synthesis of (3,5-dihexylphenyl)methyl 6-oxohexadecanoate

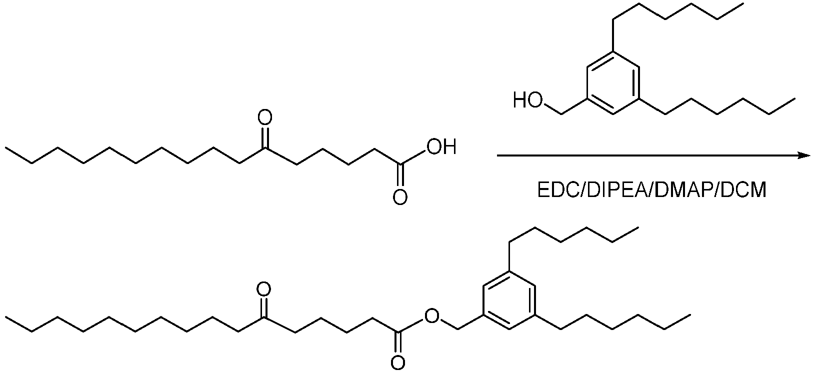

6-Oxohexadecanoic acid (500 mg, 1.8 mmol), (3,5-dihexylphenyl)methanol) (511 mg, 1.8 mmol), DIPEA (1.0 mL, 5.5 mmol), EDC (710 mg, 3.7 mmol) and DMAP (100 mg, 0.19 mmol) were stirred in DCM (10 mL) at 0°C for 16 hr allowing to warm to RT. The reaction was washed with sat NaHCO₃ and brine, dried (MgSO₄) filtered, concentrated *in-vacuo* and the residue purified by automated flash chromatography (0-15% EtOAc/Hex) to give to (3,5-dihexylphenyl)methyl 6-oxohexadecanoate (546 mg, 55.8%).

### Step 2. Synthesis of (3,5-dihexylphenyl)methyl 6-{[3-(dimethylamino)propyl]-amino}hexadecanoate

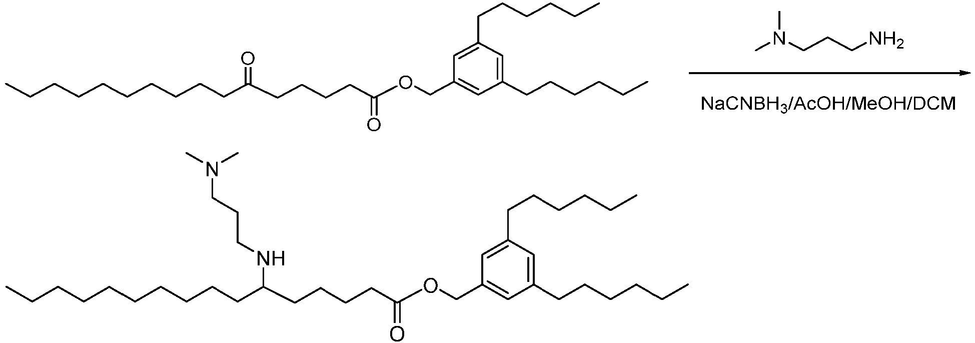

(3,5-Dihexylphenyl)methyl 6-oxohexadecanoate (546 mg, 1.1 mmol), dimethylaminopropane (119 mg, 1.2 mmol), AcOH (0.12 mL, 2.12 mmol) and NaCNBH₃ (200 mg, 3.2 mmol) were stirred in a MeOH (10 mL) and DCM (3 mL) at RT for 15 hr. The reaction was concentrated *in-vacuo* and the residue partitioned between H₂O (50 mL) and EtOAc (50 mL). The aqueous layer was extracted with EtOAc, the combined organics dried (Na₂SO₄), filtered and concentrated *in-vacuo.* The residue was purified by automated flash chromatography (0-15% MEOH/DCM/0.5%TEA) to give (3,5-dihexylphenyl)methyl 6-{[3-(dimethylamino)propyl]amino}hexadecanoate (490 mg, 75.1 %).

### Step 3. (3,5-dihexylphenyl)methyl 6-{N-[3-(dimethylamino)propyl]-decanamido}hexadecanoate (60)

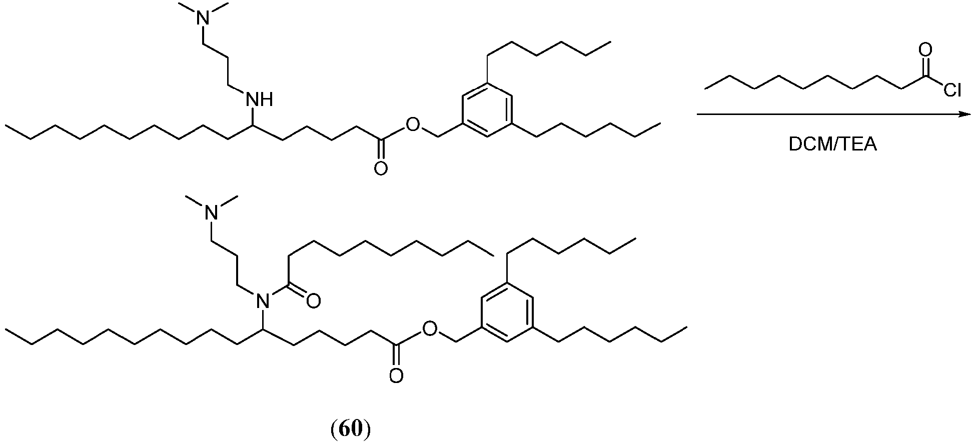

(3,5-Dihexylphenyl)methyl 6-{[3-(dimethylamino)propyl]amino}hexadecanoate (163 mg, 0.27 mmol) and TEA (80 mg, 0.80 mmol) were dissolved in DCM (10 mL) followed by dropwise addition of decanoyl chloride (0.11 mL, 0.53 mmol) in DCM (10 mL). The reaction was stirred at RT for 20 hr, washed with sat. NaHCO₃ and brine, dried (MgSO₄), filtered and concentrated *in-vacuo.* The residue was purified by automated flash chromatography (0-15% MEOH/DCM) to give (3,5-dihexylphenyl)methyl 6-{N-[3-(dimethylamino)propyl]decanamido}hexadecanoate **(60)** (61 mg, 30.0 %). ¹H NMR (400 MHz, CDCl3) δ 6.98 (s, 3H), 5.06 (s, 2H), 3.70 - 3.62 (m, 1H), 3.22 - 3.12 (m, 2H), 2.64 - 2.49 (m, 8H), 2.40 - 2.25 (m, 6H), 1.71 - 1.58 (m, 8H), 1.54 - 1.42 (m, 4H), 1.42 - 1.14 (m, 46H), 0.93 - 0.86 (m, 12H).

The following compounds were prepared using analogous methodology as described above. 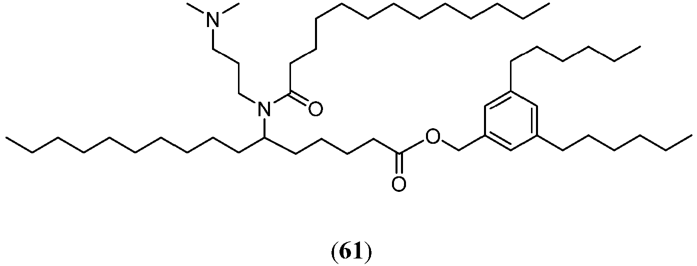 **3,5-Dihexylbenzyl 6-(N-(3-(dimethylamino)propyl)tridecanamido)hexadecanoate (61).** ¹H NMR (400 MHz, CDCl3) δ 6.98 (s, 3H), 5.07 (s, 2H), 3.69 - 3.62 (m, 1H), 3.19 - 3.10 (m, 2H), 2.62 - 2.56 (m, 4H), 2.41 - 2.20 (m, 10H), 1.64 - 1.56 (m, 8H), 1.56 - 1.41 (m, 6H), 1.41 - 1.13 (m, 50H), 0.95 - 0.86 (m, 12H). 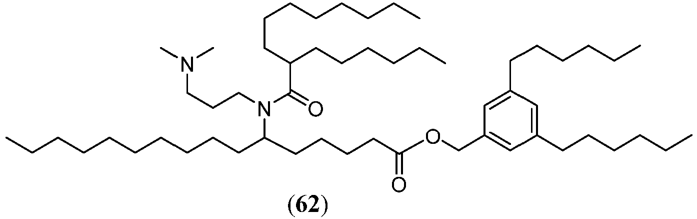 **3,5-Dihexylbenzyl 6-(N-(3-(dimethylamino)propyl)-2-hexyldecanamido)hexadecanoate (62).** ¹H NMR (400 MHz, CDCl3) δ 6.98 (s, 3H), 5.06 (s, 2H), 3.78 - 3.71 (m, 1H), 3.24 - 2.96 (m, 4H), 2.69 - 2.55 (m, 6H), 2.51 - 2.45 (m, 1H), 2.43 - 2.20 (m, 7H), 1.61 - 1.12 (m, 65H), 0.96 - 0.81 (m, 15H).

### Example 22. Synthesis of 2-Hexyldecyl 7-({9-[(2-hexyldecyl)oxy]-9-oxononyl}amino)heptadecanoate (63)

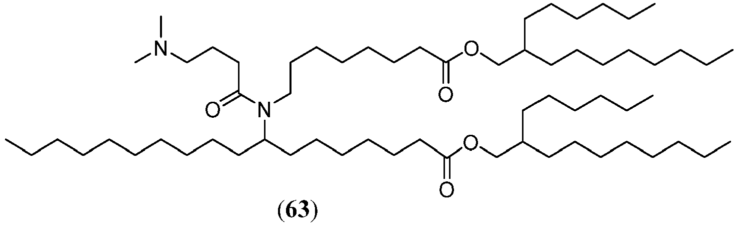

### Step 1. Synthesis of 2-Hexyldecyl 8-({8-[(2-hexyldecyl)oxy]-8-oxooctyl}amino)-octadecenoate

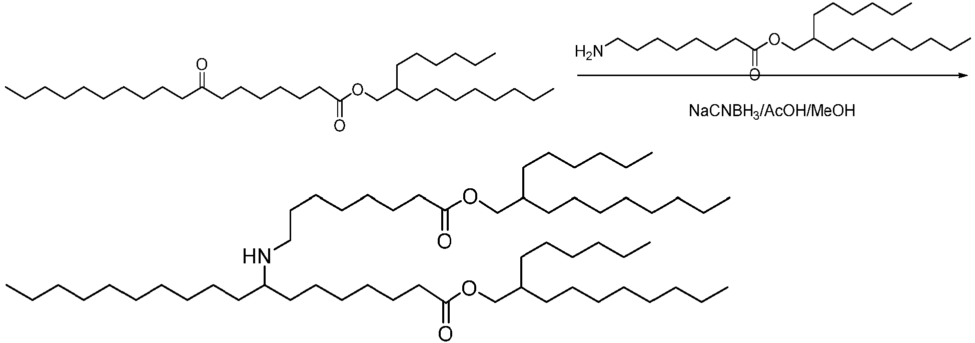

2-Hexyldecyl 8-oxooctadecanoate (743 mg, 1.4 mmol), 2-hexyldecyl 8-aminooctanoate (600 mg, 1.6 mmol) and AcOH (170 mg, 2.8 mmol) were stirred in MeOH at 50°C for 30 mins. NaCNBH₃ (268 mg, 4.3 mmol) was added and stirring continued for 16 hr. The solution was concentrated *in-vacuo,* taken up in EtOAc (75 mL), washed with sat'd NaHCO₃ and brine, dried (MgSO₄), filtered and concentrated *in-vacuo.* The residue was purified by automated flash chromatography (0-10% MeOH/DCM) to give 2-hexyldecyl 8-({8-[(2-hexyldecyl)oxy]-8-oxooctyl}amino)octadecanoate (567 mg, 44.8%).

### Step 2. Synthesis of 2-hexyldecyl 8-[4-(dimethylamino)-N-{8-[(2-hexyldecyl)oxy]-8-oxooctyl}butanamido]octadecenoate (63)

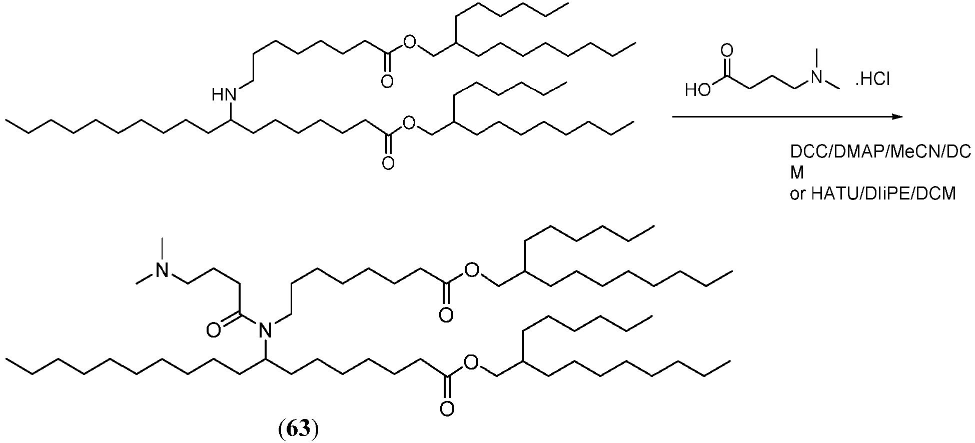

4-Dimethylaminobutanoic acid HCl salt (94 mg, 0.6 mmol), DMAP (103 mg, 0.84 mmol) and DCC (127 mg, 0.62 mmol) were stirred in ACN (1.25 mL) at RT for 2 hr. 2-hexyldecyl 8-({8-[(2-hexyldecyl)oxy]-8-oxooctyl}amino)octadecanoate (250 mg, 0.28 mmol) in DCM (1.25 mL) was added and stirring continued for 18 hr. The reaction was concentrated *in-vacuo,* the residue suspended in hexanes and filtered and the filtrate concentrated *in-vacuo.* The residue was purified by automated flash chromatography (100% EtOAc then 0-10% MeOH/EtOAc) to give 2-hexyldecyl 8-[4-(dimethylamino)-N-{8-[(2-hexyldecyl)oxy]-8-oxooctyl}butanamido]octadecenoate **(63)** (64 mg, 22.7 %). ¹H NMR (400 MHz, CDCl₃) δ 3.99 (d, *J=* 5.9 Hz, 4H), 3.67 (s, 1H), 3.13 - 3.00 (m, 2H), 2.39 - 2.27 (m, 8H), 2.25 (s, 6H), 1.85 (q, *J* = 7.9 Hz, 3H), 1.63 (d, *J* = 10.2 Hz, 15H), 1.46 (s, 4H), 1.30 (s, 74H), 0.91 (d, *J* = 7.2 Hz, 15H). MS (ESI) m/z: [M + H]+ Calcd for C₆₄H₁₂₆N₂O₅ 1003.97; Found 1003.2

Note that HATU/DIPEA/DCM may also be used to produce the final amide coupling

The following compounds were prepared using analogous methodology as described above. 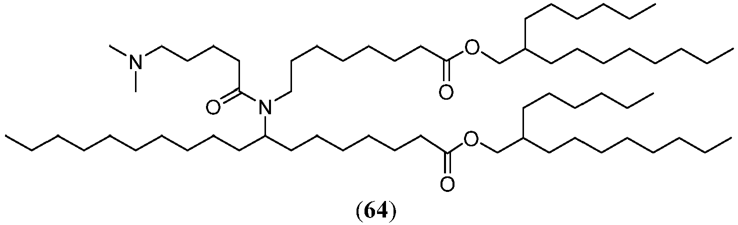 **2-Hexyldecyl 8-(5-(dimethylamino)-N-(8-((2-hexyldecyl)oxy)-8-oxooctyl)pentanamido)-octadecenoate (64).** ¹H NMR (400 MHz, CDCl₃) δ 3.99 (d, *J* = 6.0 Hz, 4H), 3.64 (s, 1H), 3.04 (d, *J =* 8.4 Hz, 2H), 2.41 - 2.26 (m, 8H), 2.24 (d, *J =* 3.1 Hz, 6H), 1.79 - 1.41 (m, 20H), 1.41 - 1.02 (m, 74H), 1.02 - 0.79 (m, 15H). MS (ESI) m/z: [M + H]+ Calcd for C₆₅H₁₂₈N₂O₅ 1017.98; Found 1017.2 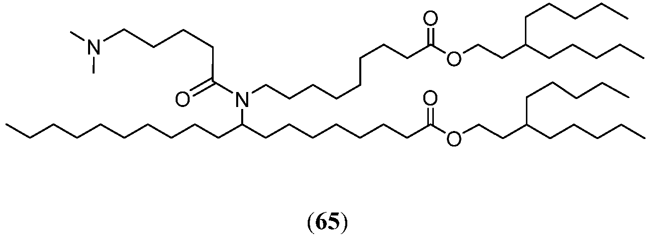 **3-Pentyloctyl 9-(5-(dimethylamino)-N-(9-oxo-9-((3-pentyloctyl)oxy)nonyl)-pentanamido)-nonadecanoate (65) .** MS (ESI) m/z: [M + H]+ Calcd for C₅₉H₁₁₆N₂O₅ 933.89; Found 933. 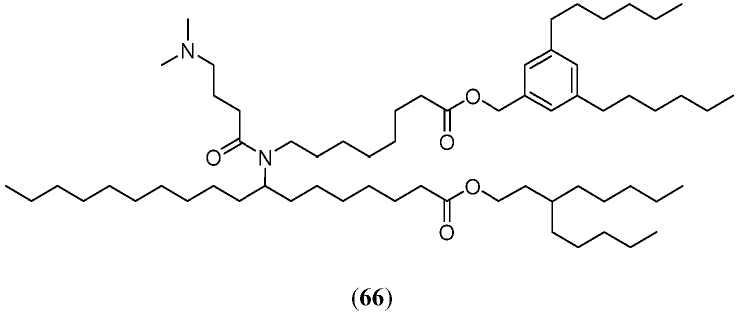 **3-Pentyloctyl 8-(N-(8-((3,5-dihexylbenzyl)oxy)-8-oxooctyl)-4-(dimethylamino)-butanamido)-octadecenoate (66).** ¹H NMR (400 MHz, CDCl₃) δ 7.28 (s, 1H), 6.99 (s, 2H), 5.07 (s, 2H), 4.09 (m, 2H), 3.05 (m, 2H), 2.83 (s, 6H), 2.65 (m, 10H), 2.29-2.37 (m, 5H), 1.28-1.6 (m, 66H), 0.89 (m, 15H) 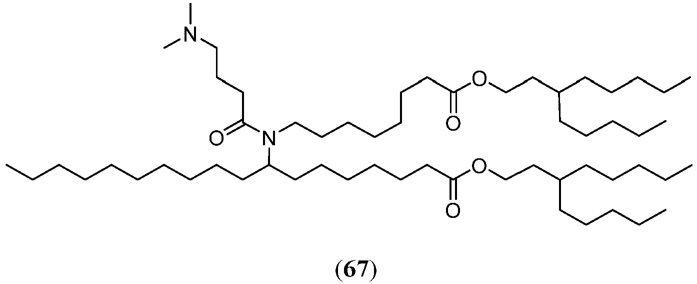 **3-Pentyloctyl 8-(4-(dimethylamino)-N-(8-oxo-8-((3-pentyloctyl)oxy)octyl)-butanamido)-octadecenoate (67).** ¹H NMR (400 MHz, CDCl₃) δ 4.11 (d, *J=* 6.4 Hz, 4H), 3.66 (s, 1H), 3.11 - 3.01 (m, 2H), 2.33 (dq, *J=* 15.0, 6.8 Hz, 8H), 2.24 (s, 6H), 1.85 (h, *J=* 7.6 Hz, 2H), 1.61 (dq, *J* = 14.1, 7.2 Hz, 13H), 1.47 (s, 0H), 1.44 (s, 7H), 1.30 (d, *J =* 16.7 Hz, 60H), 0.91 (t, *J* = 6.9 Hz, 15H). MS (ESI) m/z: [M + H]+ Calcd for C₅₈H₁₁₄N₂O₅ 919.88; Found 920.0 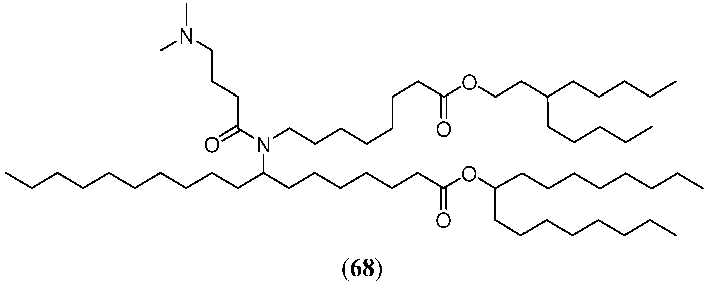 **Heptadecan-9-yl 8-(4-(dimethylamino)-N-(8-oxo-8-((3-pentyloctyl)oxy)-octyl)butanamido)-octadecenoate (68)** MS (ESI) m/z: [M + H]+ Calcd for C₆₂H₁₂₂N₂O₅ 975.94; Found 975.2 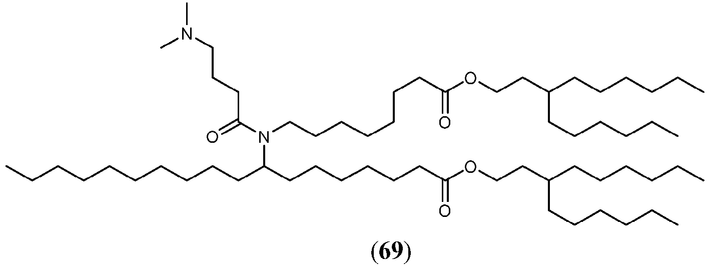 **3-Hexylnonyl 8-(4-(dimethylamino)-N-(8-((3-hexylnonyl)oxy)-8-oxooctyl)-butanamido)-octadecenoate (69).** ¹H NMR (400 MHz, CDCl₃) δ 4.10 (t, 4H), 2.38 - 2.25 (m, 8H), 2.24 (s, 6H), 1.68 (s, 5H), 1.60 (dq, *J =* 15.2, 7.5 Hz, 13H), 1.49 - 1.40 (m, 5H), 1.33 (d, *J=* 7.5 Hz, 8H), 1.30 (s, 9H), 1.27 (s, 45H), 0.94 - 0.86 (m, 15H). 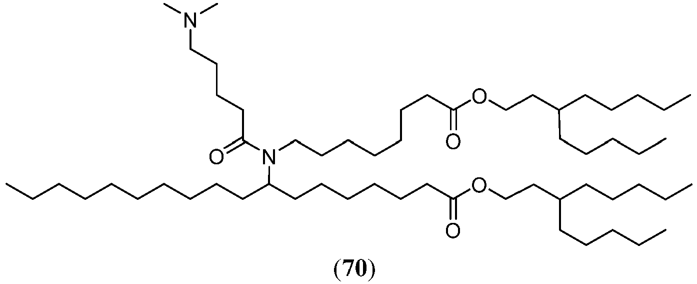 **3-Pentyloctyl 8-(5-(dimethylamino)-N-(8-oxo-8-((3-pentyloctyl)oxy)octyl)-pentanamido)-octadecenoate (70).** ¹H NMR (400 MHz, CDCl₃) δ 4.10 (t, *J =* 7.1, 4H), 2.38 - 2.29 (m, 7H), 2.27 (s, 6H), 1.58 (dt, *J =* 17.0, 8.5 Hz, 19H), 1.43 (s, 7H), 1.29 (d, *J =* 15.8 Hz, 65H), 0.91 (t, *J* = 7.0 Hz, 15H). 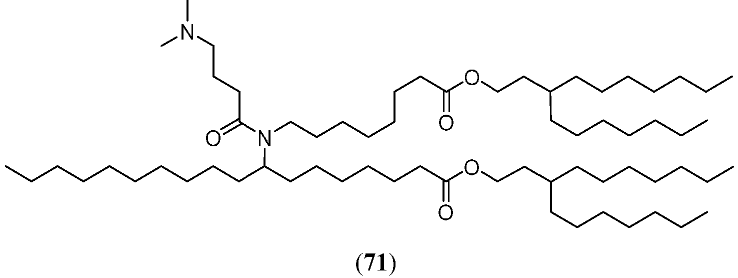 **3-Heptyldecyl 8-(4-(dimethylamino)-N-(8-((3-heptyldecyl)oxy)-8-oxooctyl)butanamido)-octadecenoate (71).** ¹H NMR (400 MHz, CDCl₃) δ 4.10 (td, *J=* 7.2, 2.6 Hz, 4H), 3.11 - 2.99 (m, 2H), 2.41 - 2.29 (m, 7H), 2.28 (s, 7H), 1.85 (dt, *J=* 14.9, 7.5 Hz, 2H), 1.75 (s, 8H), 1.60 (dt, *J=* 13.9, 6.8 Hz, 7H), 1.45 (t, *J*= 7.3 Hz, 6H), 1.37 - 1.18 (m, 76H), 0.94 - 0.87 (m, 15H). 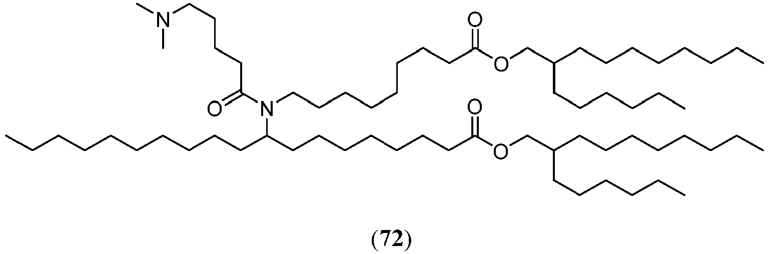 **2-Hexyldecyl 9-(5-(dimethylamino)-N-(9-((2-hexyldecyl)oxy)-9-oxononyl)pentanamido)-nonadecanoate (72)** MS (ESI) m/z: [M + H]+ Calcd for C₆₇H₁₃₂N₂O₅ 1045.01; Found 1046.3 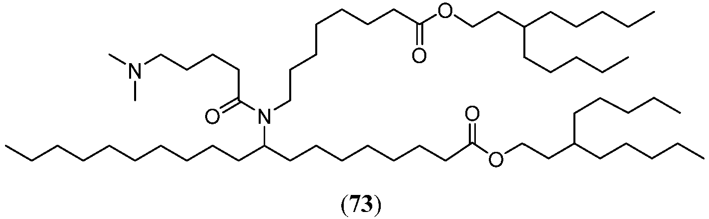 **3-Pentyloctyl 9-(5-(dimethylamino)-N-(8-oxo-8-((3-pentyloctyl)oxy)octyl)-pentanamido)-nonadecanoate (73).** ¹H NMR (400 MHz, CDCl₃) δ 4.10 (t *J* = 7.1, 4H), 2.34 (s, 6H), 2.30 (t, *J* = 8.6 Hz, 5H), 1.62 - 1.56 (m, 17H), 1.49 - 1.41 (m, 6H), 1.30 (d, *J* = 16.9 Hz, 57H), 0.91 (t, *J =* 6.9 Hz, 15H). 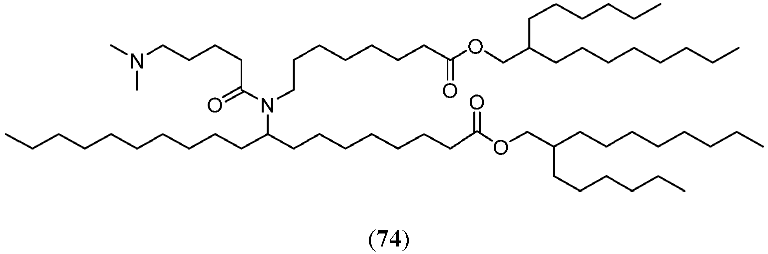 **2-Hexyldecyl 9-(5-(dimethylamino)-N-(8-((2-hexyldecyl)oxy)-8-oxooctyl)-pentanamido)-nonadecanoate (74).** ¹H NMR (400 MHz, CDCl₃) δ 3.98 (d, *J =* 5.4 Hz, 4H), 2.38 - 2.29 (m, 7H), 2.27 (s, 6H), 1.84 - 1.77 (m, 3H), 1.64 (h, *J* = 7.0 Hz, 9H), 1.30 (d, *J =* 7.4 Hz, 78H), 0.90 (t, *J=* 6.6 Hz, 15H). 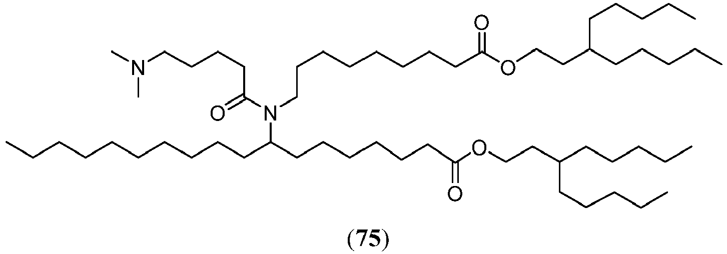 **3-Pentyloctyl 8-(5-(dimethylamino)-N-(9-oxo-9-((3-pentyloctyl)oxy)nonyl)pentanamido)-octadecenoate (75).** ¹H NMR (400 MHz, CDCl₃) δ 4.10 (t, *J=* 7.3 Hz, 4H), 2.34 (dd, *J=* 14.1, 7.2 Hz, 5H), 2.29 (s, 6H), 1.62 (qq, *J =* 17.6, 5.4 Hz, 16H), 1.49 - 1.39 (m, 6H), 1.38 - 1.22 (m, 62H), 0.91 (t, *J =* 6.9 Hz, 15H).

### Example 23. Synthesis of 3-Hexylnonyl 9-[N-decyl-4-(dimethylamino)butanamido]-2-fluorooctadecanoate (76)

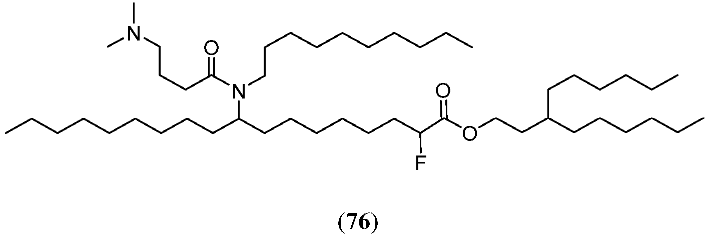

### Step 1. Synthesis of decyl[1-(oxan-2-yloxy)hexadecan-7-yl]amine

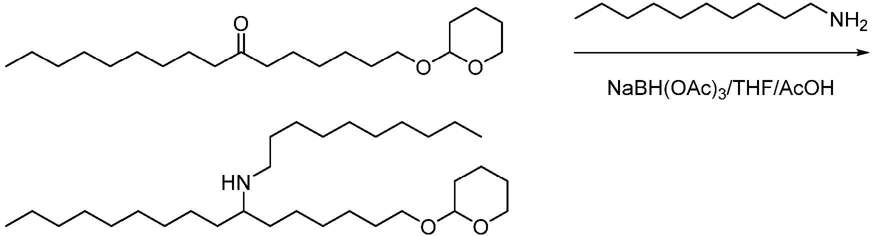

1-(Oxan-2-yloxy)hexadecan-7-one (5 g, 14.7 mmol) and N-decylamine (2.31 g, 14.7 mmol) were stirred in THF (100 mL) at 50°C for 30 mins. NaBH(OAc)₃ (9.3 g, 44.0 mmol) was added and stirring continued for 16 hr. The reaction was concentrated *in-vacuo,* the residue quenched with sat'd NaHCO₃ and the aqueous extracted with EtOAc. The organics were dried (MgSO₄), filtered and concentrated *in-vacuo.* The residue was purified by automated flash chromatography to give decyl[1-(oxan-2-yloxy)hexadecan-7-yl]amine (4.9 g, 69.3 3%).

### Step 2. Synthesis of N-decyl-4-(dimethylamino)-N-[1-(oxan-2-yloxy)hexadecan-7-yl]butanamide

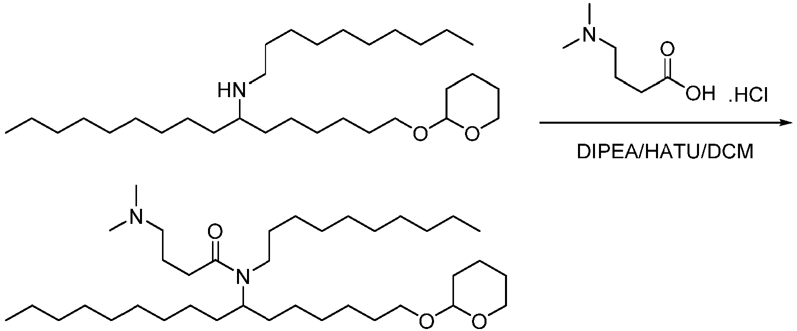

4-(Dimethylamino)butanoic acid HCl salt (4.0 g, 30.5 mmol), HATU (11.6 g, 30.5 mmol) and DIPEA (10.7 mL 61.0 mmol) were stirred in DCM (50 mL) at RT for 30 mins. Decyl[1-(oxan-2-yloxy)hexadecan-7-yl]amine (4.9 g, 10.2 mmol) in DCM was added and the reaction stirred until complete. The reaction was concentrated *in-vacuo,* the residue partitioned between satd NaHCO₃ and EtOAc. The organics were washed with brine, dried (MgSO₄) and concentrated *in-vacuo.* The residue was purified by automated flash chromatography (0-15% MeOH/DCM) to give N-decyl-4-(dimethylamino)-N-[1-(oxan-2-yloxy)hexadecan-7-yl]butanamide (4.7 g, 77.7 %).

### Step 3. Synthesis of N-decyl-4-(dimethylamino)-N-(1-hydroxyhexadecan-7-yl)butanamide

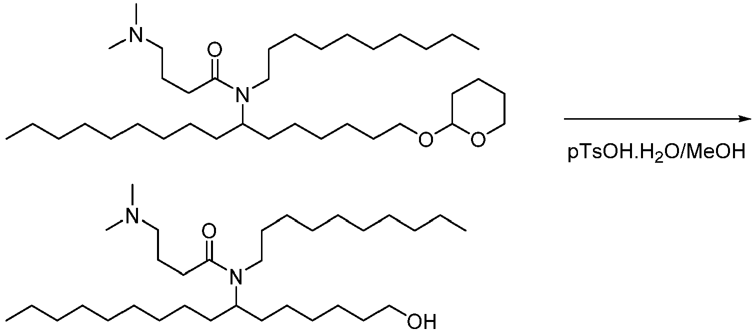

N-Decyl-4-(dimethylamino)-N-[1-(oxan-2-yloxy)hexadecan-7-yl]butanamide (4.6 g, 7.7 mmol) and p-TsOH (67 mg, 0.4 mmol) were stirred in MeOH (150 mL) at RT for 16 hr. The methanol was removed *in-vacuo,* the residue taken up in EtOAc and washed with saturated NaHCO₃. The organics were dried (MgSO₄), filtered and concentrated *in-vacuo* to give N-decyl-4-(dimethylamino)-N-(1-hydroxyhexadecan-7-yl)butanamide (3.5 g, 88.6 %) which was used without purification.

### Step 4. Synthesis of N-decyl-4-(dimethylamino)-N-(1-oxohexadecan-7-yl)butanamide

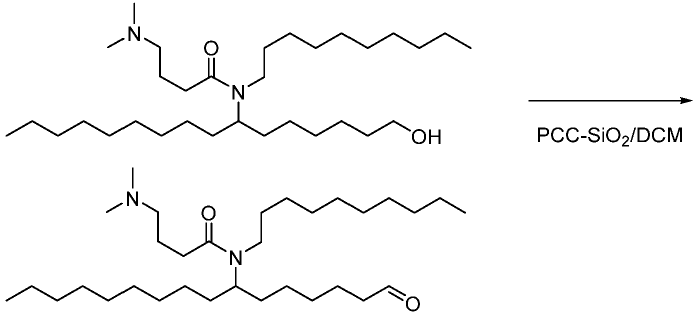

N-Decyl-4-(dimethylamino)-N-(1-hydroxyhexadecan-7-yl)butanamide (3.5 g, 6.9 mmol) and PCC on Silica (1:1) (5.9 g, 13.7 mmol) were stirred in DCM (100 mL) at RT for 16 hr. The mixture was filtered through silica, concentrated *in-vacuo* and residue purified by automated flash chromatography (0-15% MeoH/DCM) to give N-decyl-4-(dimethylamino)-N-(1-oxohexadecan-7-yl)butanamide (1.8 g, 51.6 %).

### Step 5. Synthesis of ethyl (2Z)-9-[N-decyl-4-(dimethylamino)butanamido]-2-fluorooctadec-2-enoate

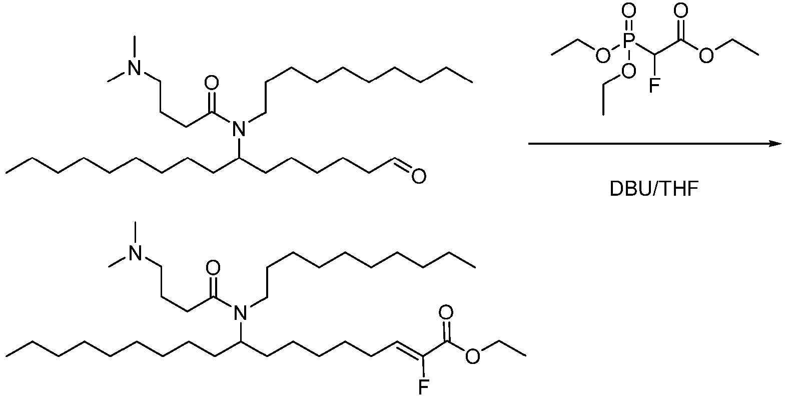

N-Decyl-4-(dimethylamino)-N-(1-oxohexadecan-7-yl)butanamide (1.8 g, 3.5 mmol), ethyl 2-(diethoxyphosphoryl)-2-fluoroacetate (1.7 g, 7.1 mmol) and DBU (1.1 g, 7.1 mmol) were stirred in THF (25 mL) at RT for 16 hr. The reaction was concentrated *in-vacuo* and the residue purified by automated flash chromatography (0-100% EtOAc/hexanes) to give ethyl (2Z)-9-[N-decyl-4-(dimethylamino)butanamido]-2-fluorooctadec-2-enoate (1.54 g, 72.9 %).

### Step 6. Synthesis of ethyl 9-[N-decyl-4-(dimethylamino)butanamido]-2-fluorooctadecanoate

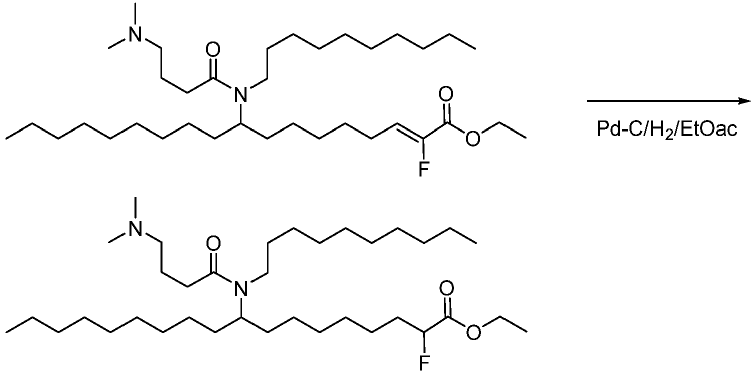

Ethyl (2Z)-9-[N-decyl-4-(dimethylamino)butanamido]-2-fluorooctadec-2-enoate (1.5 g, 2.5 mmol) was subjected to catalytic hydrogenation over Pd-C 10% (150 mg) in EtOAc at RT for 16 hr. The reaction was filtered through celite and concentrated *in-vacuo* to give ethyl 9-[N-decyl-4-(dimethylamino)butanamido]-2-fluorooctadecanoate (1.2 g, 79.7 %).

### Step 7. Synthesis of 9-[N-decyl-4-(dimethylamino)butanamido]-2-fluorooctadecanoic acid

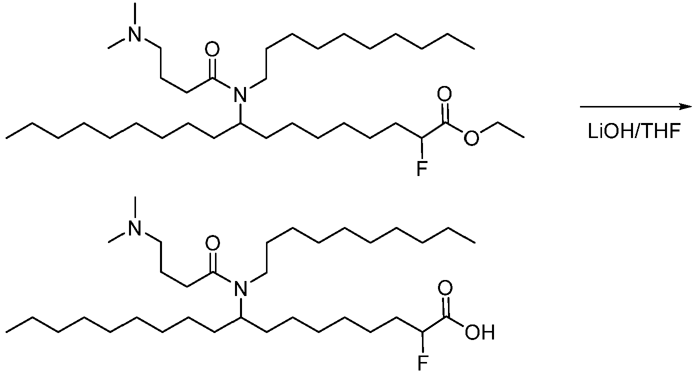

Ethyl 9-[N-decyl-4-(dimethylamino)butanamido]-2-fluorooctadecanoate (1.2 g, 2.0 mmol) and LiOH (144 mg, 6.0 mmol) were stirred in THF (50 mL) at RT for 16 hr. 6M HCl (1.3 mL) was added and the reaction extracted with DCM. The combined extracts were dried (MgSO₄), filtered and concentrated *in-vacuo* to give 9-[N-decyl-4-(dimethylamino)butanamido]-2-fluorooctadecanoic acid (810 mg, 70.8 %) which was used without purification.

### Step 8. Synthesis of 3-hexylnonyl 9-[N-decyl-4-(dimethylamino)butanamido]-2-fluorooctadecanoate (76)

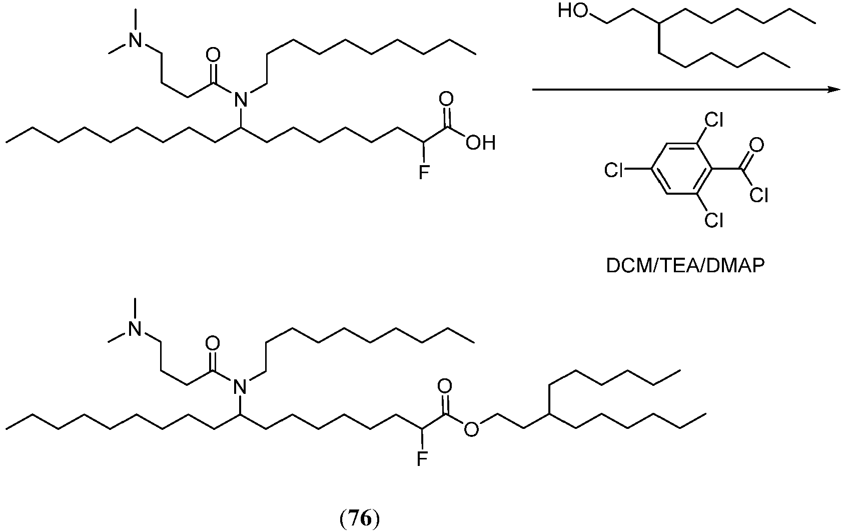

9-[N-Decyl-4-(dimethylamino)butanamido]-2-fluorooctadecanoic acid (800 mg, 1.4 mmol), 2,4,6-trichlorobenzoyl chloride (684 mg, 2.8 mmol), 3-hexylnonan-1-ol (400 mg, 1.75 mmol) and DMAP (514 mg, 4.2 mmol were stirred in DCM (10 mL) at RT for 16 hr. The reaction was concentrated *in-vacuo,* the residue taken up in EtOAc (50mL) and washed with 0.5 M NaOH (2 x 25 mL), water (25mL) and brine (25mL). The organics were dried (MgSO₄), concentrated *in-vacuo* and the residue purified by automated flash chromatography (0-15% MeOH/DCM) to give 3-hexylnonyl 9-[N-decyl-4-(dimethylamino)butanamido]-2-fluorooctadecanoate (**76**) (468 mg, 42.7 %). ¹H NMR (400 MHz, CDCl₃) δ 4.92 (d, *J=* 5.8 Hz, 1H), 4.83 - 4.76 (m, 1H), 4.20 (t, *J=* 7.2 Hz, 2H), 3.62 (s, 1H), 3.04 (q, *J =* 7.2 Hz, 2H), 2.51 - 2.23 (m, 10H), 1.99 - 1.55 (m, 16H), 1.44 (q, *J =* 7.6 Hz, 10H), 1.26 (d, *J =* 8.5 Hz, 55H), 0.93 - 0.84 (m, 12H). ¹⁹F NMR (376 MHz, CDCl₃) δ -191.86 (dtd, *J =* 49.6, 25.4, 19.6 Hz). MS (ESI) m/z: [M + H]+ Calcd for C₄₉H₉₇FN₂O₃ 781.8; Found 781.2

The following compound was prepared using analogous methodology as described above. 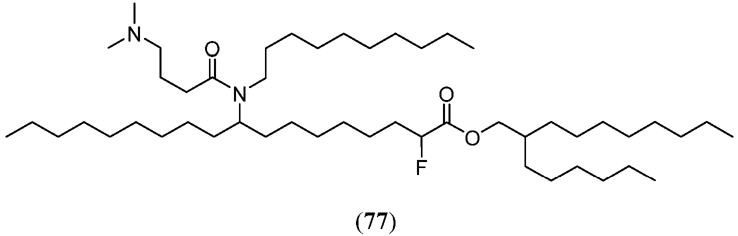 2-Hexyldecyl 9-(N-decyl-4-(dimethylamino)butanamido)-2-fluorooctadecanoate (77). ¹H NMR (400 MHz, CDCl₃) δ 5.01 - 4.74 (m, 1H), 4.08 (d, *J=* 5.7 Hz, 2H), 3.63 - 3.50 (m, 1H), 3.25 (ddt, *J=* 11.1, 5.9, 2.2 Hz, 2H), 3.13 - 3.01 (m, 2H), 2.91 (d, *J =* 8.4 Hz, 6H), 2.79 - 2.72 (m, 1H), 2.68 (t, *J =* 5.6 Hz, 1H), 2.00 (d, *J =* 7.8 Hz, 3H), 1.95 - 1.80 (m, 2H), 1.60 - 1.39 (m, 1H), 1.37 - 1.08 (m, 58H), 0.88 (td, *J=* 6.9, 1.7 Hz, 12H). MS (ESI) m/z: [M + H]+ Calcd for C₅₀H₉₉FN₂O₃ 795.8; Found 795.6.

The following compounds were prepared using analogous methodology as described above. 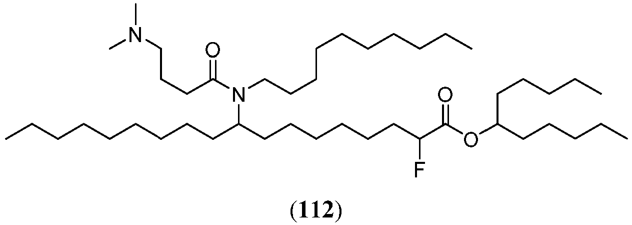 **Undecan-6-yl 9-(N-decyl-4-(dimethylamino)butanamido)-2-fluorooctadecanoate** (**112**)**.** ¹H NMR (400 MHz, CDCl₃) δ 5.13 - 4.90 (m, 2H), 4.82 (q, *J =* 5.4 Hz, 0H), 3.75 - 3.60 (m, 1H), 3.06 (q, *J =* 7.2 Hz, 2H), 2.35 (q, *J =* 6.9 Hz, 4H), 2.26 (s, 6H), 1.86 (dp, *J =* 14.5, 6.3 Hz, 4H), 1.75 - 1.09 (m, 64H), 0.90 (td, *J =* 6.8, 2.7 Hz, 12H). 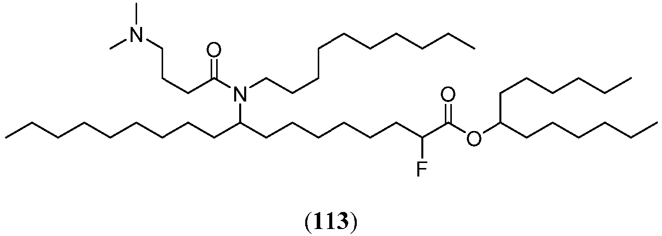 **Tridecan-7-yl 9-(N-decyl-4-(dimethylamino)butanamido)-2-fluorooctadecanoate** (**113**)**.** ¹H NMR (400 MHz, CDCl₃) δ 5.12 - 4.92 (m, 2H), 4.82 (q, *J =* 5.4 Hz, 0H), 3.73 - 3.57 (m, 1H), 3.11 - 3.01 (m, 2H), 2.35 (q, *J =* 6.8 Hz, 4H), 2.27 (s, 6H), 1.87 (td, *J =* 12.5, 5.6 Hz, 4H), 1.77 - 1.39 (m, 17H), 1.30 (dq, *J =* 11.4, 6.4 Hz, 53H), 0.90 (t, *J =* 6.6 Hz, 12H). 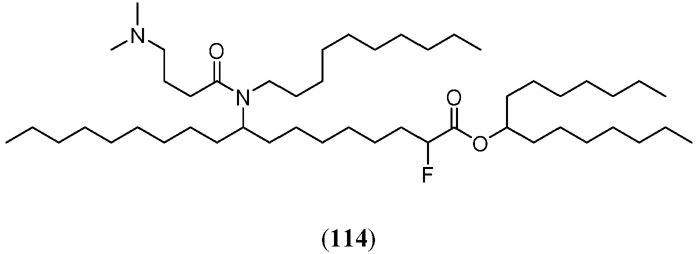 **Pentadecan-8-yl 9-(N-decyl-4-(dimethylamino)butanamido)-2-fluorooctadecanoate** (**114**)**.** ¹H NMR (400 MHz, CDCl₃) δ 5.10 - 4.90 (m, 1H), 4.82 (q, *J =* 5.4 Hz, 0H), 3.74 - 3.57 (m, 1H), 3.06 (q, *J =* 7.3 Hz, 2H), 2.35 (q, *J =* 6.7 Hz, 4H), 2.26 (s, 6H), 1.87 (tt, *J =* 10.7, 6.0 Hz, 4H), 1.81 - 1.16 (m, 73H), 0.90 (t, *J=* 6.7 Hz, 12H). 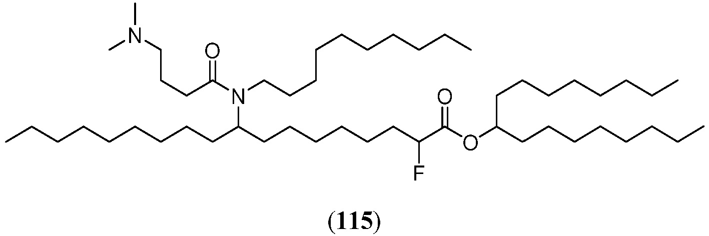 **Heptadecan-9-yl 9-(N-decyl-4-(dimethylamino)butanamido)-2-fluorooctadecanoate** (**115**)**.** ¹H NMR (400 MHz, CDCl₃) δ 5.08 - 4.90 (m, 1H), 4.82 (q, *J =* 5.5 Hz, 0H), 3.79 - 3.58 (m, 1H), 3.15 - 2.95 (m, 2H), 2.35 (q, *J =* 7.0 Hz, 4H), 2.25 (s, 6H), 1.86 (dp, *J =* 14.5, 6.4 Hz, 4H), 1.77 - 1.13 (m, 80H), 0.90 (t, *J =* 6.7 Hz, 12H). 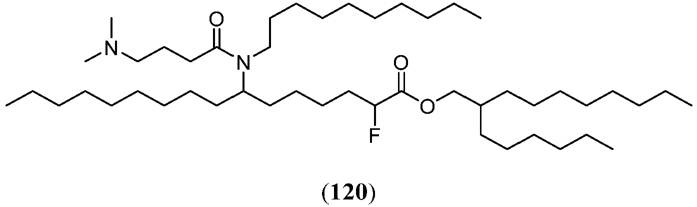 **2-Hexyldecyl 7-(N-decyl-4-(dimethylamino)butanamido)-2-fluorohexadecanoate** (**120**). ¹H NMR (400 MHz, CDCl₃) δ 4.97 (t, *J* = 5.8 Hz, 0H), 4.84 (q, *J =* 5.9 Hz, 0H), 4.11 (t, *J =* 4.9 Hz, 2H), 3.05 (d, *J =* 9.5 Hz, 2H), 2.57 - 2.24 (m, 10H), 2.20 (s, 1H), 1.95 - 1.80 (m, 4H), 1.67 (s, 6H), 1.48 (tt, *J=* 14.2, 8.8 Hz, 7H), 1.28 (dd, *J =* 10.0, 5.6 Hz, 53H), 0.95 - 0.86 (m, 12H). 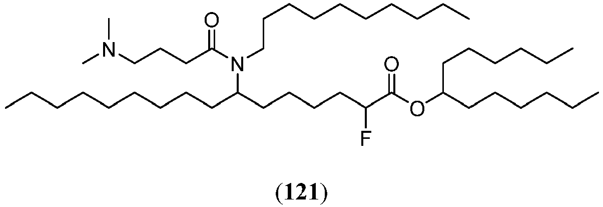 **Tridecan-7-yl 7-(N-decyl-4-(dimethylamino)butanamido)-2-fluorohexadecanoate** (**121**). ¹H NMR (400 MHz, CDCl₃) δ 5.05 - 4.85 (m, 2H), 4.79 (t, *J=* 6.0 Hz, 1H), 3.65 (s, 1H), 3.03 (t, *J=* 8.5 Hz, 2H), 2.32 (dt, *J =* 11.9, 6.9 Hz, 4H), 2.22 (s, 6H), 1.84 (dt, *J =* 15.0, 7.3 Hz, 4H), 1.73 - 1.35 (m, 16H), 1.27 (p, *J =* 6.9 Hz, 47H), 0.88 (t, *J =* 6.6 Hz, 12H). 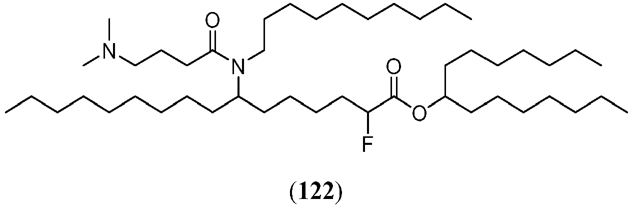 **Pentadecan-8-yl 7-(N-decyl-4-(dimethylamino)butanamido)-2-fluorohexadecanoate** (**122**). ¹H NMR (400 MHz, CDCl₃) δ 5.12 - 4.85 (m, 1H), 4.78 (d, *J =* 5.9 Hz, 1H), 3.64 (s, 1H), 3.02 (d, *J*= 9.0 Hz, 2H), 2.34 (p, *J*= 7.1 Hz, 4H), 2.26 (s, 6H), 1.85 (dd, *J*= 15.6, 8.3 Hz, 6H), 1.67 - 1.36 (m, 12H), 1.26 (q, *J =* 7.3 Hz, 51H), 0.88 (t, *J =* 6.6 Hz, 12H). 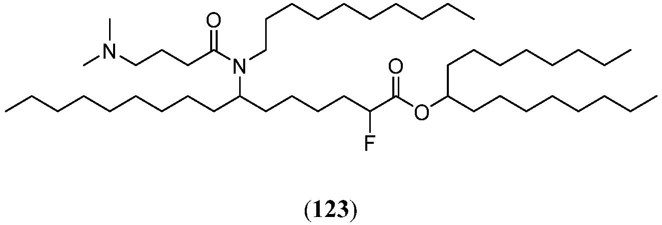 **Heptadecan-9-yl 7-(N-decyl-4-(dimethylamino)butanamido)-2-fluorohexadecanoate** (**123**)**.** ¹H NMR (400 MHz, CDCl₃) δ 5.09 - 4.87 (m, 2H), 4.79 (t, *J* = 5.8 Hz, 1H), 3.04 (q, *J =* 9.7 Hz, 2H), 2.33 (q, *J =* 7.1 Hz, 4H), 2.24 (s, 5H), 1.84 (tt, *J=* 17.5, 8.4 Hz, 6H), 1.65 - 1.40 (m, 12H), 1.27 (d, *J =* 8.5 Hz, 57H), 0.88 (t, *J =* 6.6 Hz, 12H).

### Example 24. Synthesis of 2-hexyldecyl 8-{8-[(2-butyloctyl)oxy]-N-[3-(dimethyl-amino)propyl]-8-oxooctanamido}octadecenoate (78)

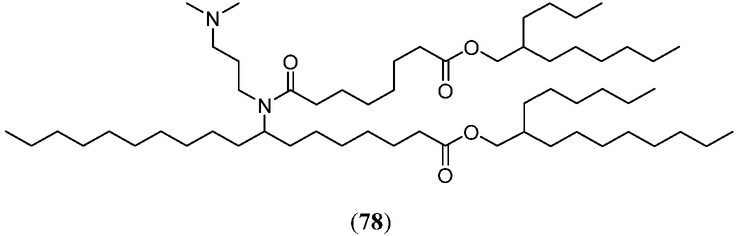

### Step 1. Synthesis of 2-hexyldecyl 8-{[3-(dimethylamino)propyl]amino}octadecenoate

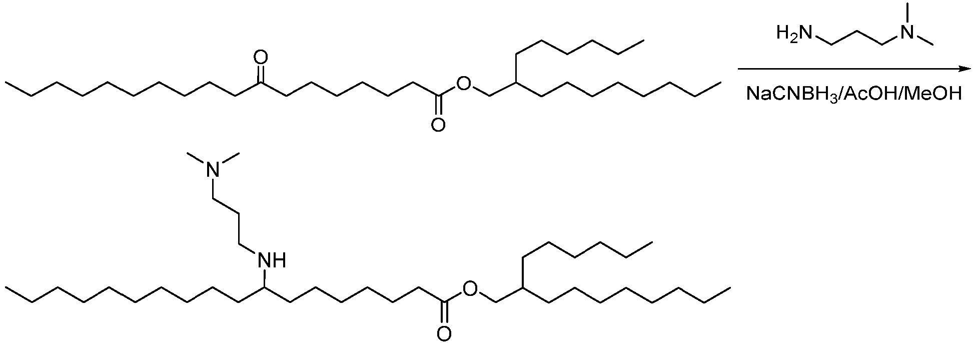

2-Hexyldecyl 8-oxooctadecanoate (500 mg, 1.0 mmol), dimethylaminopropylamine (117 mg, 1.14 mmol) and AcOH (0.11 mL, 1.9 mmol) were stirred in MeOH (15 mL) at 50°C for 1 hr. The reaction was cooled to RT, NaCNBH₃ (180 mg, 2.9 mmol) added and stirring continued for 16 hr. The reaction was concentrated *in-vacuo,* the residue taken up in EtOAc (50 mL) and washed with sat'd NaHCO₃ and brine. The organics were dried (MgSO₄), filtered, concentrated *in-vacuo* and the residue purified by automated flash chromatography (0-20% MeOH/DCM) to give 2-hexyldecyl8-{[3-(dimethylamino)propyl]amino} octadecanoate (430 mg, 73.8 %).

### Step 2. Synthesis of 2-hexyldecyl 8-{8-[(2-butyloctyl)oxy]-N-[3-(dimethylamino)propyl]-8-oxooctanamido}octadecenoate (78)

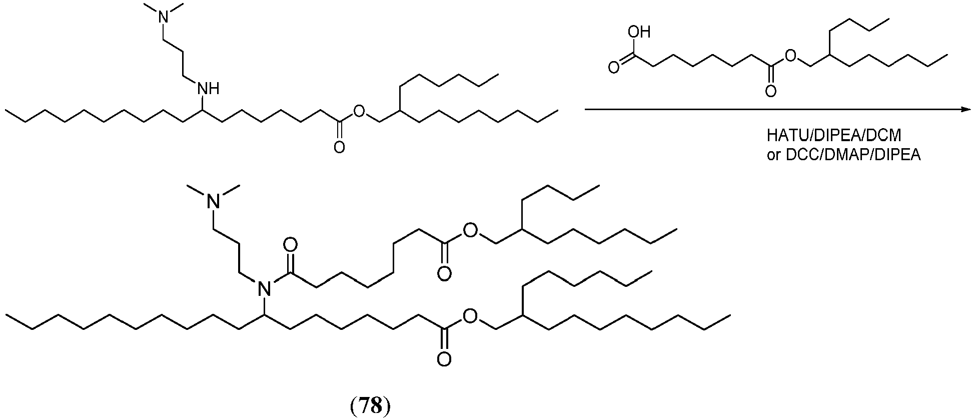

8-[(2-Butyloctyl)oxy]-8-oxooctanoic acid (169 mg, 0.5 mmol), HATU (187 mg, 0.5 mmol) and DIPEA (0.12 mL, 0.66 mmol) were stirred in DCM (5 mL) at RT for 15 mins. 2-Hexyldecyl 8-{[3-(dimethylamino)propyl]amino}octadecanoate (200 mg, 0.33 mmol) was added and stirring continued for 16 hr. The reaction was concentrated *in-vacuo,* The residue taken up in EtOAc (50 mL), washed with NaHCO₃ and brine, dried (MgSO₄), filtered and concentrated *in-vacuo.* The residue was purified by automated flash chromatography (0-5% MeOH/DCM, 100% EtOAc) to give 2-hexyldecyl 8-{8-[(2-butyloctyl)oxy]-N-[3-(dimethylamino)propyl]-8-oxooctanamido}octadecenoate (**78**) (112 mg, 36.5 %). ¹H NMR (400 MHz, CDCl₃) δ 3.96 (dd, *J =* 5.9, 3.0 Hz, 4H), 3.70 (s, 1H), 3.30 (bs, 2H), 3.13 (bs, 2H), 2.94 (s, 6H), 2.38 (t, *J =* 7.6 Hz, 2H), 2.30 (td, *J =* 7.4, 3.8 Hz, 4H), 1.96 (s, 2H), 1.62 (p, *J =* 6.6 Hz, 14H), 1.38 - 1.06 (m, 64H), 0.88 (t, *J =* 6.5 Hz, 15H).

Note that the final amide coupling can be achieved using DCC/DMAP/DIPEA coupling methodology

The following compounds were prepared using analogous methodology as described above. 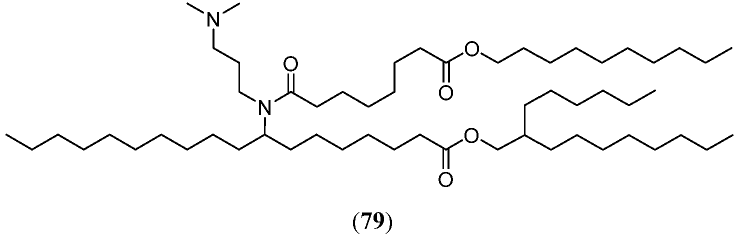 **2-Hexyldecyl 8-(8-(decyloxy)-N-(3-(dimethylamino)propyl)-8-oxooctanamido)-octadecenoate** (**79**). ¹H NMR (400 MHz, CDCl₃) δ 4.08 (t, *J =* 6.8 Hz, 2H), 3.99 (d, *J=* 5.8 Hz, 2H), 3.74 (s, 1H), 3.33 (t, *J =* 6.0 Hz, 2H), 3.17 (s, 2H), 2.97 (s, 5H), 2.41 (t, *J =* 7.7 Hz, 2H), 2.33 (t, *J=* 7.5 Hz, 4H), 1.99 (s, 2H), 1.64 (t, *J =* 7.4 Hz, 12H), 1.29 (s, 57H), 0.91 (t, *J =* 6.7 Hz, 12H). 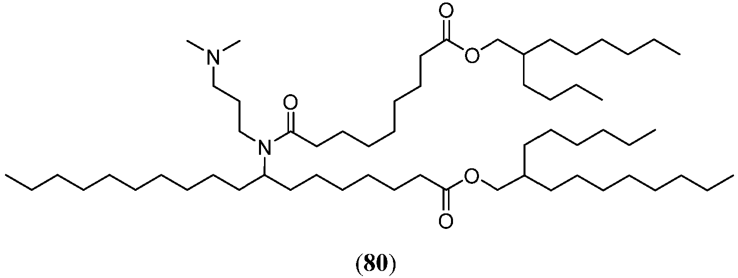 **2-Hexyldecyl 8-(9-((2-butyloctyl)oxy)-N-(3-(dimethylamino)propyl)-9-oxononanamido)-octadecenoate** (**80**). ¹H NMR (400 MHz, CDCl₃) δ 3.98 (dd, *J=* 5.8, 2.3 Hz, 4H), 3.71 (s, 1H), 3.29 (t, *J=* 6.2 Hz, 2H), 3.04 (s, 2H), 2.86 (s, 6H), 2.82 (s, 20H), 2.38 (dd, *J =* 8.6, 6.2 Hz, 2H), 2.32 (t, *J=* 7.5 Hz, 4H), 1.94 (t, *J=* 6.5 Hz, 2H), 1.50 (dd, *J=* 29.4, 6.7 Hz, 13H), 1.38 - 1.24 (m, 68H), 0.94 - 0.88 (m, 15H). 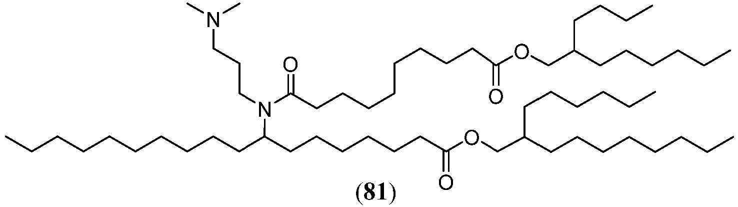 **2-Hexyldecyl 8-(10-((2-butyloctyl)oxy)-N-(3-(dimethylamino)propyl)-10-oxodecanamido)-octadecenoate** (**81**). ¹H NMR (400 MHz, CDCl₃) δ 4.10 (t, *J =* 7.1 Hz, 2H), 3.98 (d, *J=* 5.8 Hz, 2H), 3.29 (t, *J=* 6*.*5 Hz, 2H), 3.06 (d, *J=* 7.0 Hz, 2H), 2.87 (s, 6H), 2.83 (s, 1H), 2.38 (dd, *J =* 8.5, 6.5 Hz, 2H), 2.32 (td, *J=* 7.5, 2.5 Hz, 4H), 2.01 - 1.93 (m, 2H), 1.61 (dq, *J=* 20.5, 6.7 Hz, 10H), 1.41 - 1.17 (m, 68H), 0.91 (t, *J=* 6.7 Hz, 15H). 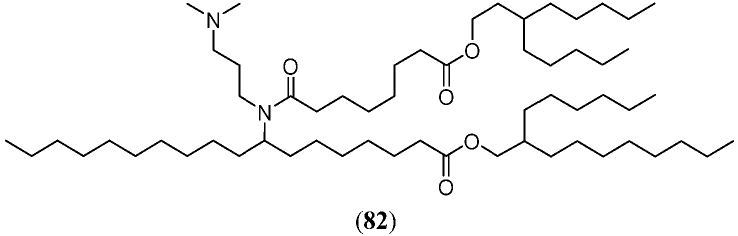 **2-Hexyldecyl 8-(N-(3-(dimethylamino)propyl)-8-oxo-8-((2-pentyloctyl)oxy)-octanamido)-octadecenoate** (**82**)**.** ¹H NMR (400 MHz, CDCl₃) δ 4.10 (t, *J* = 7.1 Hz, 2H), 3.98 (d, *J=* 5.8 Hz, 2H), 3.29 (t, *J=* 6.5 Hz, 2H), 3.06 (d, *J=* 7.0 Hz, 2H), 2.87 (s, 6H), 2.83 (s, 1H), 2.38 (dd, *J =* 8.5, 6.5 Hz, 2H), 2.32 (td, *J =* 7.5, 2.5 Hz, 4H), 2.01 - 1.93 (m, 2H), 1.61 (dq, *J =* 20.5, 6.7 Hz, 10H), 1.41 - 1.17 (m, 68H), 0.91 (t, *J=* 6.7 Hz, 15H). 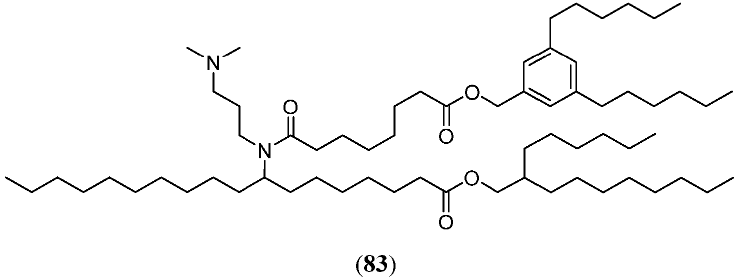 **2-Hexyldecyl 8-(8-((3,5-dihexylbenzyl)oxy)-N-(3-(dimethylamino)propyl)-8-oxooctanamido)-octadecenoate** (**83**)**.** ¹H NMR (400 MHz, CDCl₃) δ 6.98 (m, 3H), 5.07 (s, 2H), 3.98 (dd, *J* = 5.9, 2.8 Hz, 2H), 3.69 - 3.56 (m, 1H), 3.12 (t, *J=* 8.0 Hz, 2H), 2.59 (t, *J=* 7.8 Hz, 4H), 2.42 - 2.20 (m, 13H), 1.79 - 1.56 (m, 21H), 1.46 (t, *J =* 7.2 Hz, 4H), 1.41 - 1.10 (m, 61H), 0.98 - 0.86 (m, 15H). 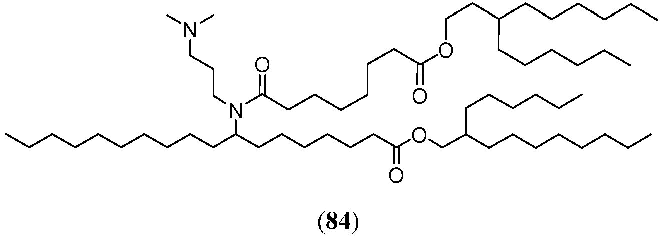 **2-Hexyldecyl 8-(N-(3-(dimethylamino)propyl)-8-((3-hexylnonyl)oxy)-8-oxooctanamido)-octadecenoate** (**84**). ¹H NMR (400 MHz, CDCl₃) δ 4.10 (t, *J=* 7.1 Hz, 2H), 3.98 (dd, *J =* 5.8, 1.9 Hz, 2H), 3.20 (t, *J =* 7.5 Hz, 2H), 2.75 (s, 2H), 2.58 (s, 6H), 2.31 (qd, *J=* 7.5, 2.9 Hz, 6H), 2.26 (s, 2H), 1.88 (t, *J =* 7.4 Hz, 2H), 1.74 - 1.55 (m, 10H), 1.49 (td, *J =* 13.1, 5.6 Hz, 4H), 1.41 - 1.19 (m, 69H), 0.90 (t, *J =* 6.7 Hz, 15H). 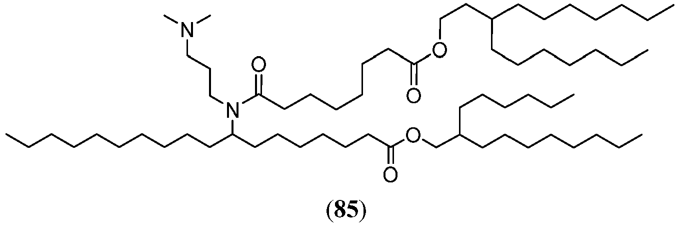 **2-Hexyldecyl 8-(N-(3-(dimethylamino)propyl)-8-((3-heptyldecyl)oxy)-8-oxooctanamido)-octadecenoate** (**85**). ¹H NMR (400 MHz, CDCl₃) δ 4.09 (t, *J=* 7.1 Hz, 2H), 3.98 (dd, *J =* 5.8, 3.2 Hz, 2H), 3.13 (dd, *J =* 10.4, 5.9 Hz, 2H), 2.30 (pd, *J=* 4.9, 3.0 Hz, 8H), 2.24 (s, 6H), 1.77 - 1.55 (m, 13H), 1.47 (q, *J* = 7.5 Hz, 4H), 1.29 (dd, *J =* 8.3, 5.0 Hz, 74H), 0.94 - 0.86 (m, 15H).

### Example 25. Synthesis of 3-pentyloctyl 8-[4-(dimethylamino)-N-{6-fluoro-7-oxo-7-[(3-pentyloctyl)oxy]heptyl}butanamido]octadecenoate (86)

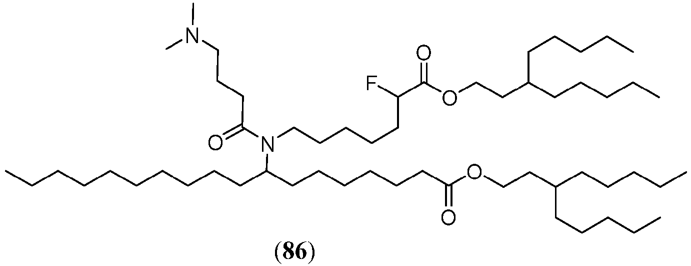

### Step 1. Synthesis of methyl 8-{[5-(oxan-2-yloxy)pentyl]amino}octadecenoate

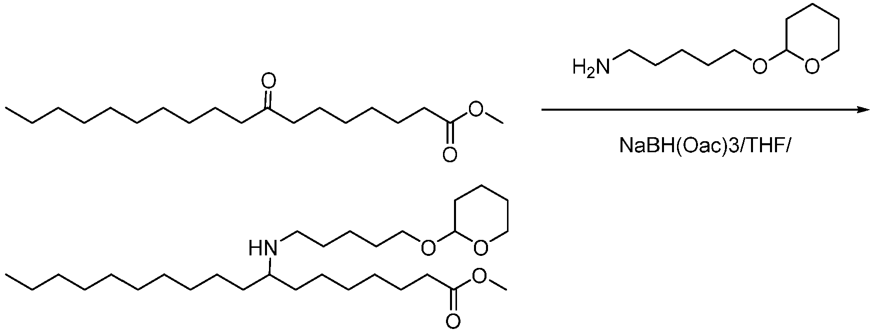

5-(Oxan-2-yloxy)pentan-1-amine (1.8 g, 9.6 mmol) and methyl 8-oxooctadecanoate (3 g, 9.6 mmol) were heated in THF (25 mL) at 50°C for 45 mins. NaBH(OAc)₃ (6.1g, 28.8 mmol) was added and stirring continued for 16 hr. After cooling to RT the reaction was concentrated *in-vacuo,* the residue taken up in EtOAc (100 mL), washed with NaHCO₃ and brine, dried (MgSO₄) and concentrated *in-vacuo.* The crude material was purified by automated flash chromatography (0-100% EtOAc/Hex) to give methyl 8-{[5-(oxan-2-yloxy)pentyl]amino}octadecanoate (1.6 g, 34.5 %).

### Step 2. Synthesis of methyl 8-[4-(dimethylamino)-N-[5-(oxan-2-yloxy)pentyl]-butanamido]octadecenoate

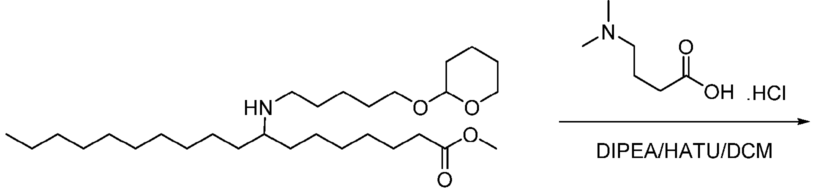

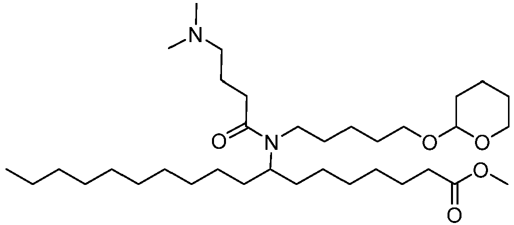

4-(Dimethylamino)butanoic acid HCl (1.30 g, 9.9 mmol), DIPEA (3.5 mL, 20 .0 mmol) and HATU (3.8 g, 9.9 mmol) were stirred in DCM (50 mL) at RT for 30 mins. Methyl 8-{[5-(oxan-2-yloxy)pentyl]amino}octadecanoate (1.6 g, 3.3 mmol) in DCM (10 mL) was added and stirring continued for 16 hr at RT. The reaction was concentrated *in-vacuo* and the residue taken up in EtOAc. The combined organics were washed with 0.5 M NaOH, water and brine (25 mL), dried (MgSO₄) and concentrated *in-vacuo.* The crude material was purified by automated flash chromatography (0-15% MeOH/DCM) to give methyl 8-[4-(dimethylamino)-N-[5-(oxan-2-yloxy)pentyl]butanamido]octadecanoate (1.85 g, 93.7 %).

### Step 3. Synthesis of methyl 8-[4-(dimethylamino)-N-(5-hydroxypentyl)butanamido]-octadecenoate

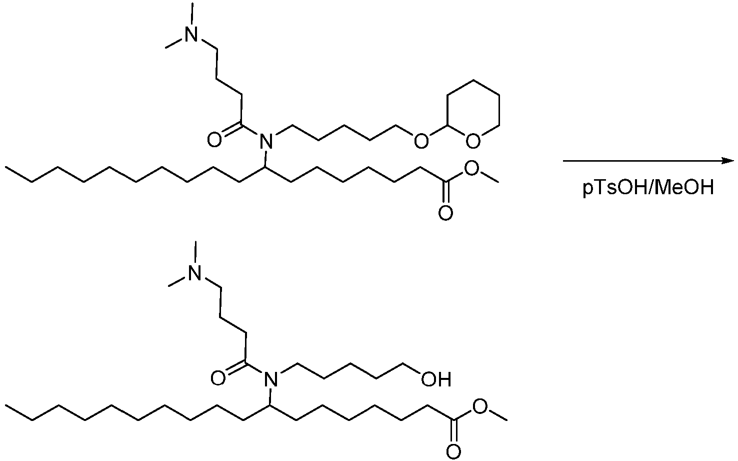

Methyl 8-[4-(dimethylamino)-N-[5-(oxan-2-yloxy)pentyl]butanamido]octadecanoate (1.9 g, 3.2 mmol) and p-ToSH.H₂O (27 mg 0.16 mmol) were stirred in MeOH (25 mL) at RT for 16 hr. The reaction was concentrated *in-vacuo,* the residue taken up in EtOAc (100mL) washed with sat'd NaHCO₃, dried (MgSO₄), filtered and concentrated *in-vacuo* to give methyl 8-[4-(dimethylamino)-N-(5-hydroxypentyl)butanamido]octadecanoate (Quant) which was used without purification.

### Step 4. Synthesis of methyl 8-[4-(dimethylamino)-N-(5-oxopentyl)butanamido]-octadecenoate

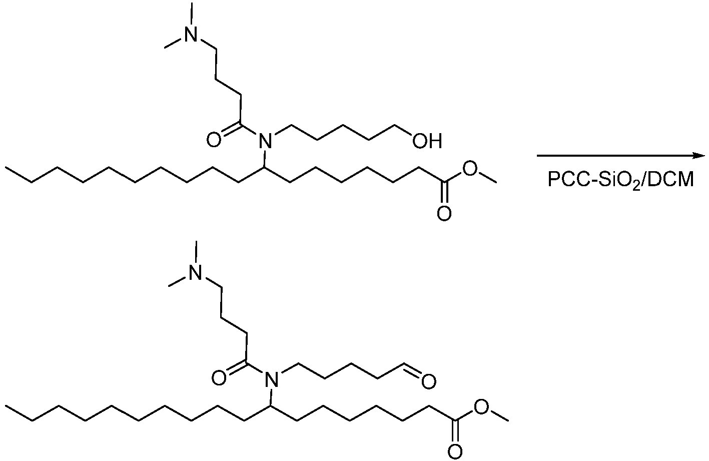

Methyl 8-[4-(dimethylamino)-N-(5-hydroxypentyl)butanamido]octadecanoate (1.6 g, 3.1 mmol) and PCC/Silica 1:1 wt (1.3 g, 3.12 mmol) were stirred in DCM (50 mL) at RT for 16 hr. The reaction was filtered through silica gel eluting with DCM. The filtrate was concentrated *in-vacuo* and the residue purified by automated flash chromatography (0-20% MeOH/DCM) to give methyl 8-[4-(dimethylamino)-N-(5-oxopentyl)butanamido]octadecanoate (1.6 g, Quant).

### Step 5. Synthesis of methyl 8-[4-(dimethylamino)-N-(7-ethoxy-6-fluoro-7-oxohept-5-en-1-yl)butanamido]octadecenoate

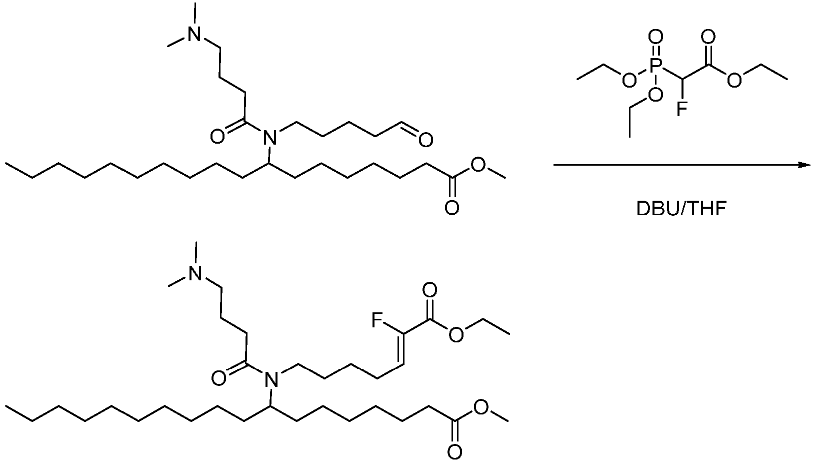

Methyl 8-[4-(dimethylamino)-N-(5-oxopentyl)butanamido]octadecanoate (1.6 g, 3.1 mmol), ethyl 2-(diethoxyphosphoryl)-2-fluoroacetate (1.9 g, 7.8 mmol) and DBU (1.17 mL, 7.8) were stirred in anhydrous THF (25 mL) at RT for 16 hr. Additional ethyl 2-(diethoxyphosphoryl)-2-fluoroacetate (379 mg, 1.6 mmol) and DBU (0.23 mL, 1.6 mmol) were added and stirring continued for another 3 hr. The reaction was concentrated *in-vacuo* and the residue purified by automated flash chromatography (0-15% MeOH/DCM, 0-100% EtOAc/Hex) to give methyl 8-[4-(dimethylamino)-N-(7-ethoxy-6-fluoro-7-oxohept-5-en-1-yl)butanamido]octadecenoate (500 mg, 26.7 %).

### Step 6. Synthesis of methyl 8-[4-(dimethylamino)-N-(7-ethoxy-6-fluoro-7-oxoheptyl)butanamido]octadecenoate

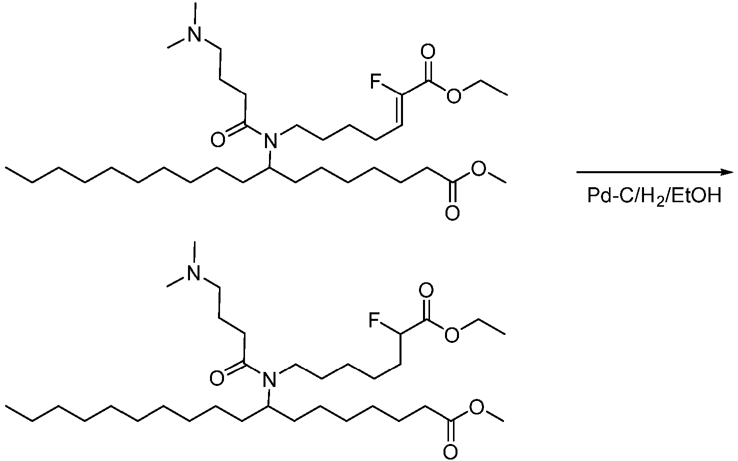

Methyl 8-[4-(dimethylamino)-N-(7-ethoxy-6-fluoro-7-oxohept-5-en-1-yl)butanamido]octadecanoate (500 mg, 0.84 mmol) was subjected to catalytic hydrogenation over Palladium on Carbon (50 mg) in EtOH (25 mL) at RT for 16 hr. The reaction was filtered through celite and the filtrate concentrated *in-vacuo* to give methyl 8-[4-(dimethylamino)-N-(7-ethoxy-6-fluoro-7-oxoheptyl)butanamido]octadecanoate (375 mg, 74.7 %) which was used without purification.

### Step 7. Synthesis of 8-[N-(6-carboxy-6-fluorohexyl)-4-(dimethylamino)butanamido]-octadecanoic acid

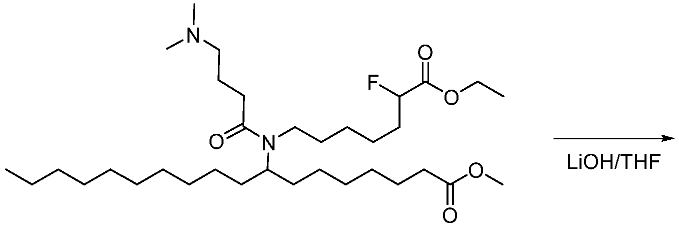

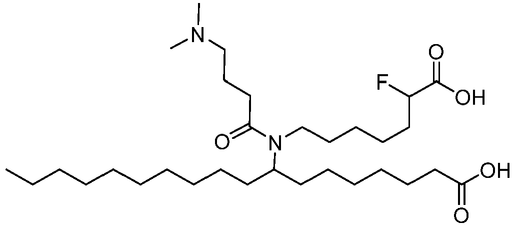

Methyl 8-[4-(dimethylamino)-N-(7-ethoxy-6-fluoro-7-oxoheptyl)butanamido]octadecanoate (375 mg, 0.6 mmol) and LiOH (60.0 mg, 2.5 mmol) were stirred in THF (10 mL) at RT for 16 hr. 6M HCl (0.6 mL) was added, the reaction diluted with H₂O and the reaction extracted with DCM. The combined organics were dried (MgSO₄), filtered and concentrated *in-vacuo* to give 8-[N-(6-carboxy-6-fluorohexyl)-4-(dimethylamino)butanamido]octadecanoic acid (205 mg, 59.0 %) which was used without purification.

### Step 8. Synthesis of 3-pentyloctyl 8-[4-(dimethylamino)-N-{6-fluoro-7-oxo-7-[(3-pentyloctyl)oxy]heptyl}butanamido]octadecenoate (86)

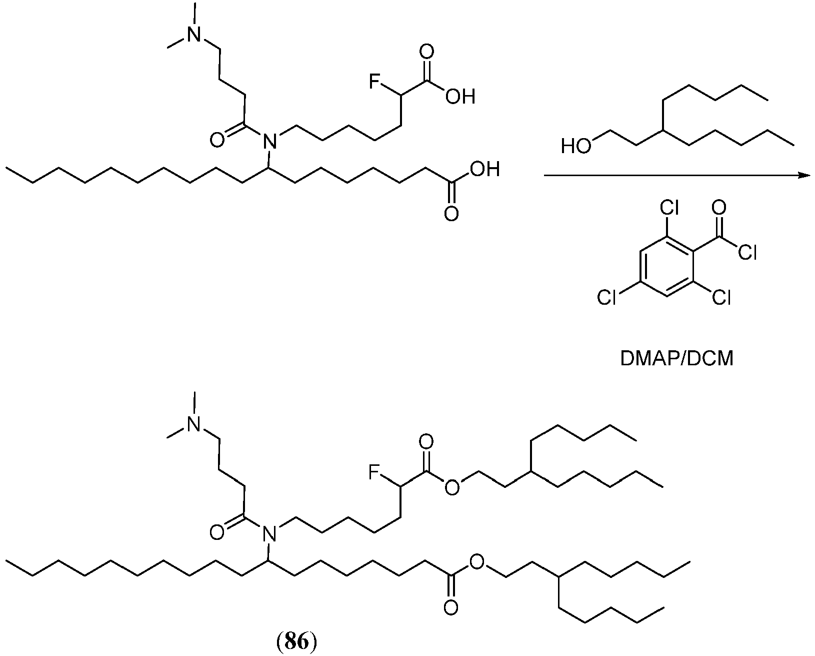

8-[N-(6-Carboxy-6-fluorohexyl)-4-(dimethylamino)butanamido]octadecanoic acid (200 mg, 0.36 mmol), 3-pentyloctan-1-ol (215 mg, 1.0 mmol), DMAP (175 mg, 1.4 mmol) and 2,4,6-trichlorobenzoyl chloride (262 mg, 1.0 mmol) were stirred in DCM (10 mL) at RT for 16 hr. The reaction was concentrated *in-vacuo* the residue taken up in EtOAc and washed with 0.5 M NaOH, H₂O and brine, dried (MgSO₄), filtered and concentrated *in-vacuo.* The residue was purified by automated flash chromatography (0-25% ACN/DCM, 0-15% MeOH/DCM) to give 3-pentyloctyl 8-[4-(dimethylamino)-N-{6-fluoro-7-oxo-7-[(3-pentyloctyl)oxy]heptyl}butanamido]octadecenoate (**86**) (93 mg, 28.14 %). ¹H NMR (400 MHz, CDCl₃) δ 4.97 (ddd, *J=* 6.9, 4.5, 2.3 Hz, 1H), 4.85 (ddd, *J=* 7.1, 5.1, 2.4 Hz, 1H), 4.23 (tt, *J =* 7.3, 2.0 Hz, 2H), 4.09 (t, *J =* 7.1 Hz, 2H), 3.59 (s, 1H), 3.33 - 3.23 (m, 2H), 3.10 (d, *J= 9.1* Hz, 2H), 2.95 (d, *J =* 11.7 Hz, 6H), 2.82 - 2.76 (m, 1H), 2.72 (t, *J =* 5.6 Hz, 1H), 2.30 (td, *J =* 7.5, 3.1 Hz, 2H), 2.06 - 1.84 (m, 4H), 1.71 - 1.38 (m, 22H), 1.38 - 1.16 (m, 55H), 0.91 (tt, *J=* 7.1, 1.5 Hz, 15H). MS (ESI) m/z: [M + H]+ Calcd for C₅₇H₁₁₁FN₂O₅ 923.9; Found 923.2

### Example 26. Synthesis of 3-pentyloctyl 9-[4-(dimethylamino)-N-{8-oxo-8-[(3-pentyloctyl)oxy]octyl}butanamido]-2,2-difluorononadecanoate (87)

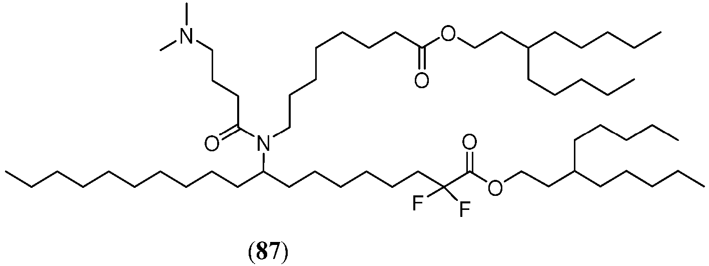

### Step 1. Synthesis of 3-pentyloctyl 8-(heptadec-1-en-7-ylamino)octanoate

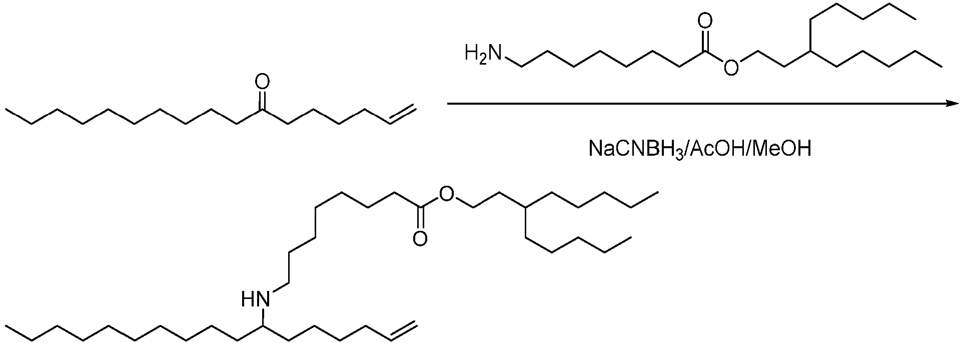

3-Pentyloctyl 8-aminooctanoate (1.2 g, 3.5 mmol), heptadec-1-en-7-one (872 mg, 3.5 mmol) and AcOH acid (200 µL, 3.5 mmol) were stirred in MeOH at 60°C for 30 mins. NaCNBH₃ was added and stirring continued for 16 hr. The reaction was concentrated *in-vacuo,* the residue taken-up in DCM, washed with NaHCO₃ (sat. aq.) and brine, dried (MgSO₄), filtered and concentrated *in-vacuo.* The crude material was purified by automated flash chromatography (0-60% EtOAc/Hex) to give 3-pentyloctyl 8-(heptadec-1-en-7-ylamino)octanoate (1.33 g, 66.7 %).

### Step 2. Synthesis of 3-pentyloctyl 8-[4-(dimethylamino)-N-(heptadec-1-en-7-yl)butanamido]octanoate

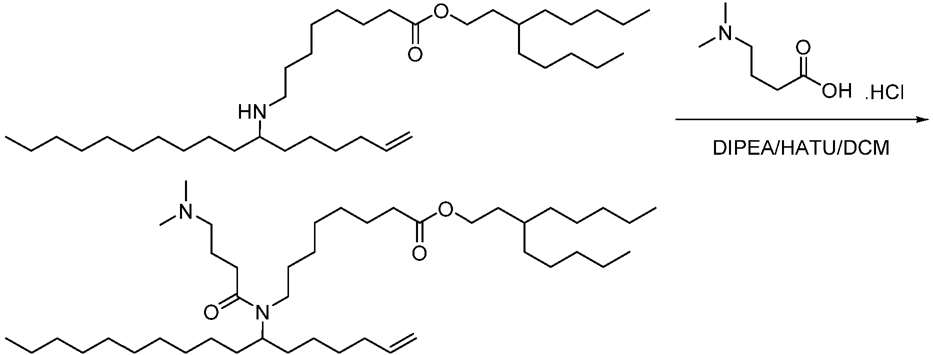

4-(Dimethylamino)butyric acid hydrochloride (1.16 g, 6.9 mmol), HATU (3.15 g, 8.3 mmol) and DIPEA (3.6 mL, 20.7 mmol) were stirred in DCM (40 mL) at RT for 3 hr. 3-Pentyloctyl 8-(heptadec-1-en-7-ylamino)octanoate (1.33 g, 2.3 mmol) in DCM (40 mL) was added and stirring continued for 18 hr. The reaction was concentrated *in-vacuo,* the residue taken up in EtOAc, washed with sat'd NaHCO₃ and the aqueous layer back extracted with EtOAc. The combined organics were dried (MgSO₄), filtered and concentrated *in-vacuo.* The crude material was purified by automated flash chromatography (0-100% EtOAc/Hex and 0-20% MeOH/EtOAc) to give 3-pentyloctyl 8-[4-(dimethylamino)-N-(heptadec-1-en-7-yl)butanamido]octanoate (1.54 g, 97.1 %).

### Step 3. Synthesis of ethyl 9-[4-(dimethylamino)-N-{8-oxo-8-[(3-pentyloctyl)oxy]-octyl}butanamido]-2,2-difluorononadecanoate

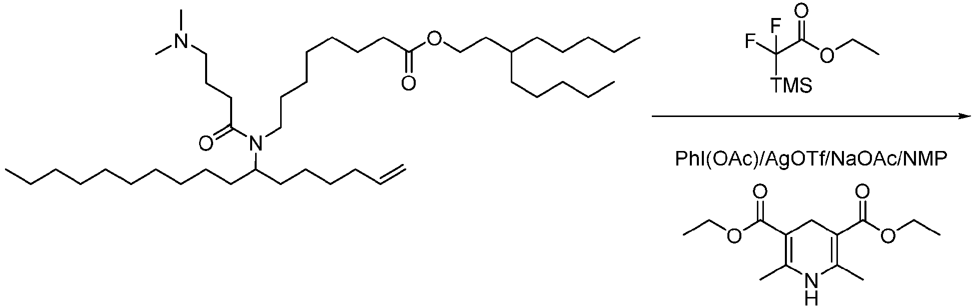

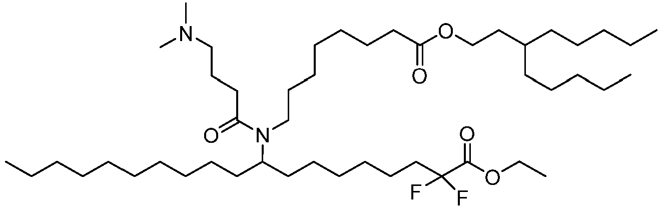

In a heat/vacuum dried flask, Hantsch ester (566 mg, 2.24 mmol), NaOAc (367 mg, 4.5 mmol), AgOTf (1.44 g, 5.6 mmol) and iodobenzene diacetate (1.44 g, 4.471 mmol, 2 equiv.) were stirred in NMP (15mL) under N₂. 3-Pentyloctyl 8-[4-(dimethylamino)-N-(heptadec-1-en-7-yl)butanamido]octanoate (1.55 g, 2.24 mmol) in NMP (7 mL) and ethyl 2,2-difluoro-2-(trimethylsilyl)acetate (2.2 g, 11.2 mmol) were added and the reaction stirred at RT for 18 hr. The reaction was filtered through Celite eluting with Et₂O. The combined organics were washed with brine, dried (MgSO₄), filtered and concentrated *in-vacuo.* The crude material was purified by automated flash chromatography (0-25% ACN/DCM) to give ethyl 9-[4-(dimethylamino)-N-{8-oxo-8-[(3-pentyloctyl)oxy]octyl}butanamido]-2,2-difluorononadecanoate (588 mg, 32.3 %.

### Step 4. Synthesis of 9-[4-(dimethylamino)-N-{8-oxo-8-[(3-pentyloctyl)oxy]octyl}-butanamido]-2,2-difluorononadecanoic acid

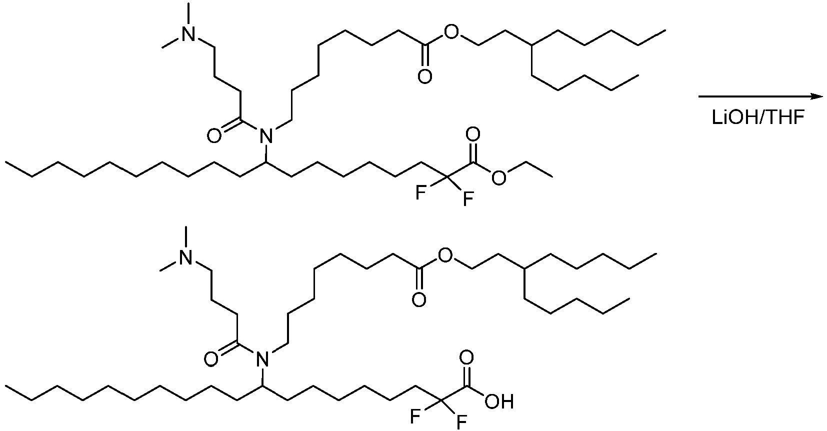

Ethyl 9-[4-(dimethylamino)-N-{8-oxo-8-[(3-pentyloctyl)oxy]octyl}butanamido]-2,2-difluorononadecanoate (588 mg, 0.7 mmol) and LiOH 1M (2.2 mL, 2.2 mmol) were stirred in THF (10 mL) at 0°C until the reaction was completed. 6M HCl (0.36 mL) was added the reaction azeotroped with toluene. The residue was taken up in DCM, dried (MgSO₄), filtered and concentrated *in-vacuo* to give 9-[4-(dimethylamino)-N-{8-oxo-8-[(3-pentyloctyl)oxy]octyl}butanamido]-2,2-difluorononadecanoic acid (600 mg, 98.8 %) which was used without purification.

### Step 5. Synthesis of 3-pentyloctyl 9-[4-(dimethylamino)-N-{8-oxo-8-[(3-pentyloctyl)oxy]-octyl}butanamido]-2,2-difluorononadecanoate (87)

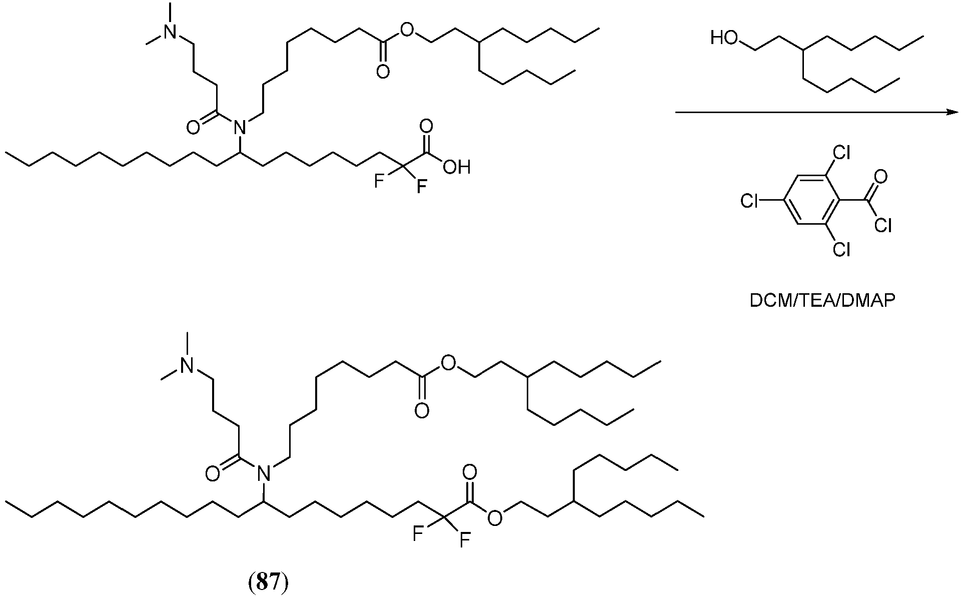

9-[4-(Dimethylamino)-N-{8-oxo-8-[(3-pentyloctyl)oxy]octyl}butanamido]-2,2-difluorononadecanoic acid (561 mg, 0.7 mmol), 3-pentyloctan-1-ol (157 mg, 0.78 mmol), 2,4,6-trichlorobenzoyl chloride (382 mg, 1.6 mmol) and DMAP (261 mg, 2.1 mmol) were combined in DCM (10 mL) at 0°C. The reaction was stirred for 16 hr allowing to warm to RT. Et₂O was added and the resultant PPT removed by filtration. The organics were washed with NaHCO₃, dried (MgSO₄), filtered, concentrated in-vacuo and the residue purified by automated flash chromatography (0-10 % MeOH/DCM and 1:1 ACN/DCM 3CV, 0-20 % MeOH/DCM) to give 3-pentyloctyl 9-[4-(dimethylamino)-N-{8-oxo-8-[(3-pentyloctyl)oxy]octyl}butanamido]-2,2-difluorononadecanoate (**87**) (128 mg, 18.5 %).¹H NMR (400 MHz, MeOD) δ 4.33 (t, *J=* 6.7 Hz, 2H), 4.12 (t, *J=* 6.8 Hz, 2H), 3.19 (d, *J* = 8.3 Hz, 1H), 3.12 (d, *J=* 9.0 Hz, 2H), 2.81 (t, *J =* 7.5 Hz, 3H), 2.62 (s, 6H), 2.52 (dt, *J =* 10.2, 7.0 Hz, 3H), 2.33 (td, *J =* 7.3, 4.3 Hz, 3H), 1.93 (h, *J=* 7.1 Hz, 3H), 1.64 (dq, *J*= 29.9, 6.6 Hz, 9H), 1.55 (s, 3H), 1.46 (s, 3H), 1.39 (d, *J=* 3.8 Hz, 7H), 1.37 (s, 9H), 1.31 (d, *J=* 4.2 Hz, 39H), 0.93 (t, *J=* 6.9 Hz, 15H).

### Example 27. Synthesis of 3-pentyloctyl 9-[4-(dimethylamino)-N-{8-oxo-8-[(3-pentyloctyl)oxy]octyl}butanamido]-2-fluorooctadecanoate (88)

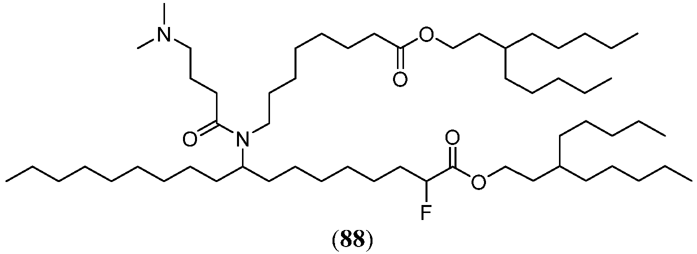

### Step 1. Synthesis of methyl 8-{[1-(oxan-2-yloxy)hexadecan-7-yl]amino}octanoate

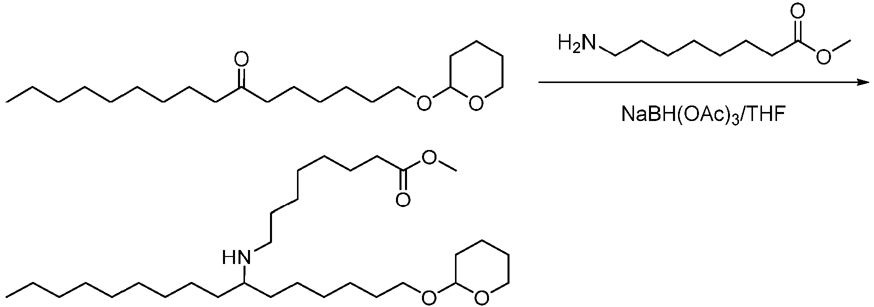

1-(Oxan-2-yloxy)hexadecan-7-one (5.7 g, 16.7 mmol), methyl 8-aminooctanoate (2.9 g, 16.7 mmol) and NaCN(OAc)₃ (10.6 g, 50.2 mmol) were stirred in THF at 50°C for 16 hr. The reaction was filtered, eluting with EtOAc and the organics washed with NaHCO₃ satd soln, water and brine. The organics were dried (MgSO₄) concentrated *in-vacuo* and purified by automated flash chromatography (7%MeOH/DCM) to give methyl 8-{ [1-(oxan-2-yloxy)hexadecan-7-yl]amino}octanoate (2.9 g, 34.2 %).

### Step 2. Synthesis of methyl 8-[4-(dimethylamino)-N-[1-(oxan-2-yloxy)hexadecan-7-yl]butanamido]octanoate

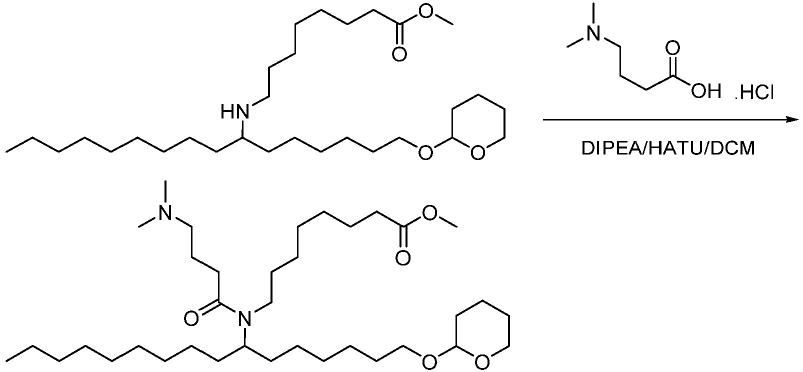

4-Dimethylaminobutanoic acid HCl salt (3.84 g, 22.9 mmol), HATU (8.7 g, 22.9 mmol) and DIPEA ( 8.0 mL, 45.8 mmol) were stirred in DCM (40 mL) for 3 hr at RT. Methyl 8-{[1-(oxan-2-yloxy)hexadecan-7-yl]amino}octanoate (2.85 g, 5.7 mmol) in DCM (40 mL) was added and stirring continued for 18 hr. The reaction was concentrated *in-vacuo,* the residue taken up in EtOAc, washed with sat'd NaHCO₃ and the aqueous layer extracted with additional EtOAc. The combined organics were concentrated *in-vacuo* and the crude material purified by automated flash chromatography (0-100% EtOAc/Hex, and 0-20% MeOH/EtOAc) to give methyl 8-[4-(dimethylamino)-N-[1-(oxan-2-yloxy)hexadecan-7-yl]butanamido]octanoate (Quant).

### Step 3. Synthesis of methyl 8-[4-(dimethylamino)-N-(1-hydroxyhexadecan-7-yl)butanamido]octanoate

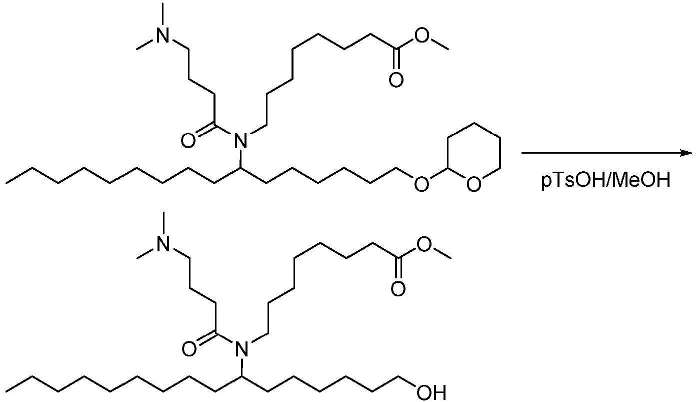

Methyl 8-[4-(dimethylamino)-N-[1-(oxan-2-yloxy)hexadecan-7-yl]butanamido]-octanoate (3.5 g, 5.7 mmol) and pTsOH-H₂O (109 mg, 0.57 mmol) were stirred in MeOH at RT for 2.5 hr. The reaction was concentrated *in-vacuo* to give methyl 8-[4-(dimethylamino)-N-(1-hydroxyhexadecan-7-yl)butanamido]octanoate (5.06 g)which was used without purification.

### Step 4. Synthesis of methyl 8-[4-(dimethylamino)-N-(1-oxohexadecan-7-yl)butanamido]octanoate

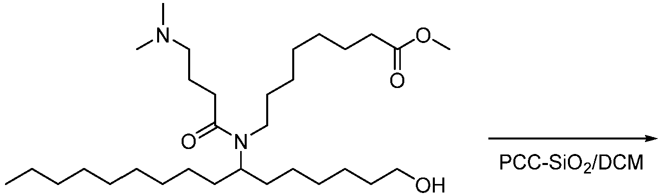

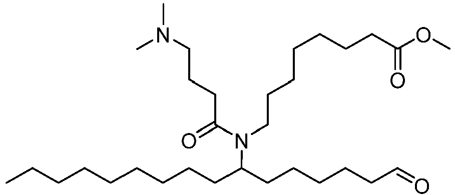

Methyl 8-[4-(dimethylamino)-N-(1-hydroxyhexadecan-7-yl)butanamido]octanoate (3.02 g, 5.73 mmol) and PCC-SiO₂ (1:1) (4.3 g, 10.0 mmol) were stirred in DCM at RT for 16 hr. MeCN was added and the reaction filtered through silica eluting with 1:1 MeCN/DCM. The filtrate was concentrated *in-vacuo* the residue taken up in EtOAc and filtered through celite. The filtrate was concentrated *in-vacuo* and purified by automated flash chromatography (0-50% ACN/DCM) to give methyl 8-[4-(dimethylamino)-N-(1-oxohexadecan-7-yl)butanamido]octanoate (1.79 g, 59.3 %)

### Step 5. Synthesis of ethyl (2Z)-9-[4-(dimethylamino)-N-(8-methoxy-8-oxooctyl)butanamido]-2-fluorooctadec-2-enoate

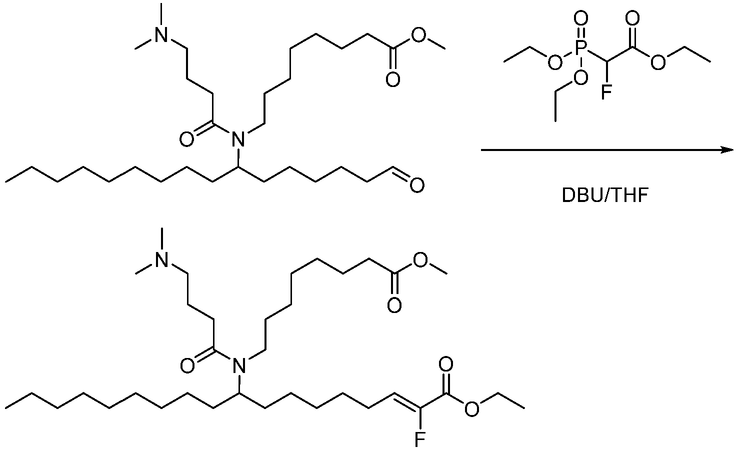

Methyl 8-[4-(dimethylamino)-N-(1-oxohexadecan-7-yl)butanamido]octanoate (1.79 g, 3.4 mmol), ethyl 2-(diethoxyphosphoryl)-2-fluoroacetate (1.4 g, 6.0 mmol) and DBU (0.9 mL, 6.0 mmol) were stirred in THF at RT for 16 hr. The reaction was concentrated *in-vacuo* and the residue taken up in EtOAc. The organics were washed with NaHCO₃ and brine, dried (MgSO₄), filtered and concentrated *in-vacuo.* The crude material was purified by automated flash chromatography (0-100% EtOAc/Hex and 0-15% MeOH/DCM) to give Synthesis of ethyl (2Z)-9-[4-(dimethylamino)-N-(8-methoxy-8-oxooctyl)butanamido]-2-fluorooctadec-2-enoate (626 mg, 30.0 %)

### Step 6. Synthesis of ethyl 9-[4-(dimethylamino)-N-(8-methoxy-8-oxooctyl)butanamido]-2-fluorooctadecanoate

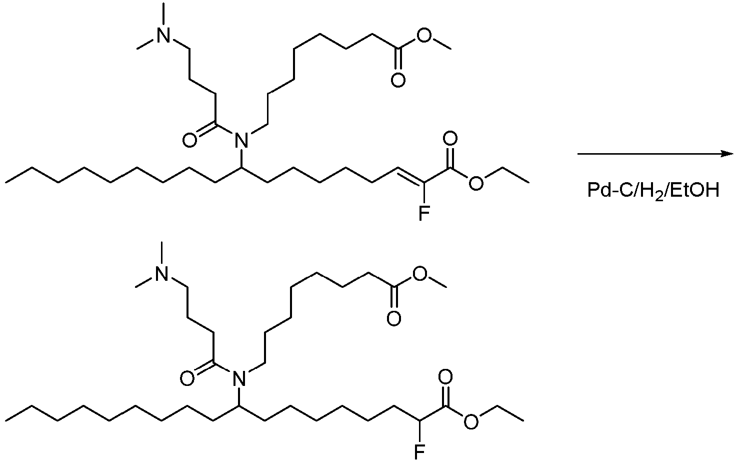

Ethyl (2Z)-9-[4-(dimethylamino)-N-(8-methoxy-8-oxooctyl)butanamido]-2-fluorooctadec-2-enoate (620 mg, 1.0 mmol) was subjected to catalytic hydrogenation over Pd-C in EtOH at RT for 16 hr. The reaction was filtered through celite and concentrated *in-vacuo* to give ethyl 9-[4-(dimethylamino)-N-(8-methoxy-8-oxooctyl)butanamido]-2-fluorooctadecanoate (550 mg, 88.4 %) which was used without purification.

### Step 7. Synthesis of 9-[N-(7-carboxyheptyl)-4-(dimethylamino)butanamido]-2-fluorooctadecanoic acid

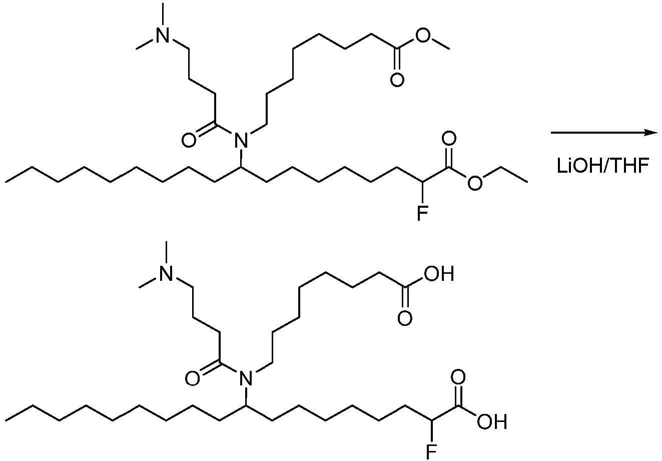

Ethyl 9-[4-(dimethylamino)-N-(8-methoxy-8-oxooctyl)butanamido]-2-fluorooctadecanoate (550 mg, 0.90 mmol) and LiOH (96 mg, 4.0 mmol) were stirred in THF at RT for 16 hr. The reaction was concentrated *in-vacuo,* acidified with 6 M HCl (1.0 mL) and extracted into DCM (50 mL). The organics were washed with brine and the aqueous layer further extracted with DCM. The combined organics were dried (MgSO₄), filtered and concentrated *in-vacuo* to give 9-[N-(7-carboxyheptyl)-4-(dimethylamino)butanamido]-2-fluorooctadecanoic acid (284 mg, 55.4 %) which was used without further purification.

### Step 8. Synthesis of 3-pentyloctyl 9-[4-(dimethylamino)-N-{8-oxo-8-[(3-pentyloctyl)oxy]-octyl}butanamido]-2-fluorooctadecanoate (88)

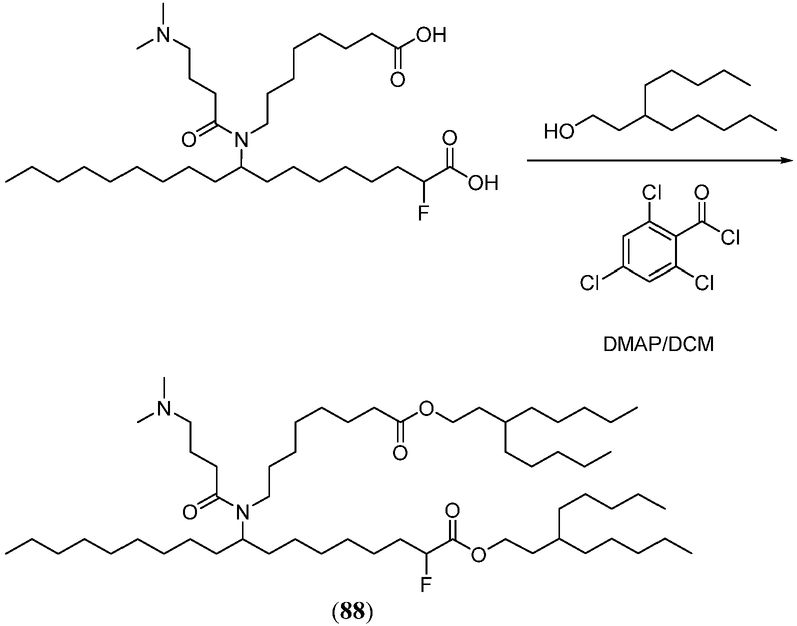

9-[N-(7-Carboxyheptyl)-4-(dimethylamino)butanamido]-2-fluorooctadecanoic acid (275 mg, 0.48 mmol), 3-pentyloctan-1-ol (289 mg, 1.44 mmol), 2,4,6-trichlorobenzoyl chloride (351 mg, 1.44 mmol) and DMAP (235 mg, 1.92 mmol) were combined at 0°C in DCM (10 mL). The reaction was stirred for 16 hr allowing to warm to RT. The reaction was concentrated *in-vacuo* and the residue taken up in EtOAc. The combined organics were washed with sat'd NaHCO₃ and brine, dried (MgSO₄), filtered and concentrated *in-vacuo.* The residue was purified by automated flash chromatography (0-10% MeOH/DCM) to give 3-pentyloctyl 9-[4-(dimethylamino)-N-{8-oxo-8-[(3-pentyloctyl)oxy]octyl}butanamido]-2-fluorooctadecanoate (**88**) (211 mg, 46.9 %). ¹H NMR (400 MHz, CDCl₃) δ 4.23 (td, *J=* 7.1, 2.1 Hz, 2H), 4.10 (td, *J* = 7.1, 4.0 Hz, 2H), 3.09 - 3.01 (m, 2H), 2.33 (dt, *J =* 23.1, 7.3 Hz, 9H), 1.69 - 1.53 (m, 11H), 1.38 - 1.18 (m, 57H), 0.95 - 0.86 (m, 15H). ¹⁹F NMR (376 MHz, CDCl₃) δ -191.67 - -192.07 (m).

### Example 28. Synthesis of 3-hexylnonyl 8-(decyl((3-(dimethylamino)propoxy)carbonyl)-amino) octadecanoate (105)

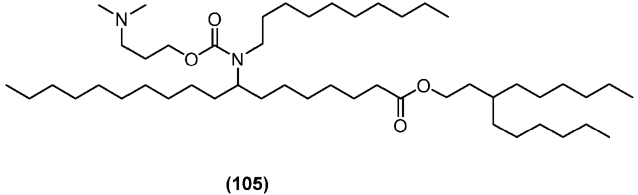

### Step 1: Synthesis of 3-(dimethylamino)propyl (4-nitrophenyl) carbonate hydrochloride

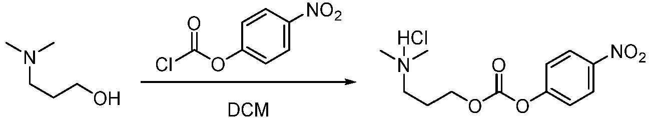

3-(Dimethylamino)propan-1-ol (2.5 g, 24.2 mmol) in DCM (20 mL) was added dropwise to a stirred solution of 4-nitrophenyl chloroformate (4.9 g, 24.2 mmol) in DCM (80 mL) at 0°C. The reaction was stirred for 16 h allowing to warm to RT. The solvent was removed *in-vacuo* to give 3-(dimethylamino)propyl (4-nitrophenyl) carbonate hydrochloride (5.3 g, 70.9%) as a crude mixture which was used without further purification.

### Step 2: Synthesis of 3-hexylnonyl 8-(N-decyl-5-(dimethylamino)pentanamido)octadecenoate (105)

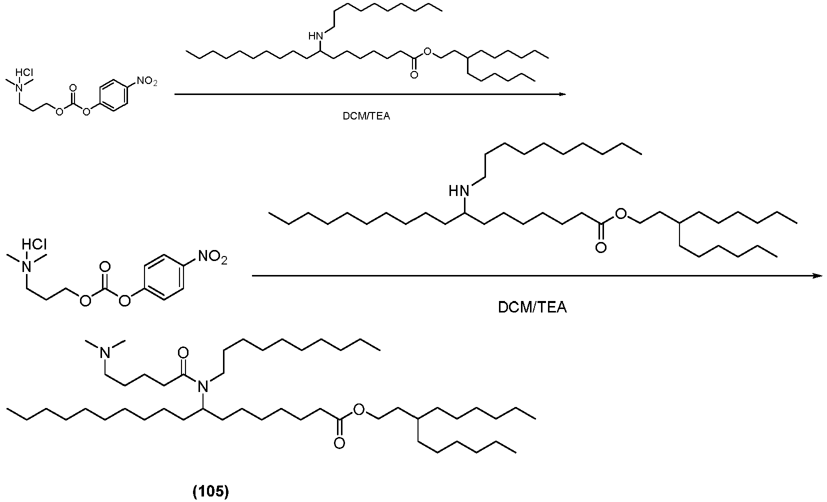

3-Hexylnonyl 8-(decylamino)octadecanoate (0.61 g, 0.94 mmol), triethylamine (0.29 g, 2.82 mmol) and 3-(dimethylammonio)propyl 4-nitrophenyl carbonate hydrochloride (0.36 g, 1.18 mmol) were stirred in DCM at RT for 16 h. An additional 1 eq of 3-(dimethylammino)propyl 4-nitrophenyl carbonate hydrochloride and triethylamine were added wit DMAP (cat) and the reaction heated at reflux for 16 h. The solvent was removed *in-vacuo* and the residue taken up in hexanes, filtered, and concentrated. The resulting oil was taken up in DCM and decanoyl chloride (90 mg, 0.5 equiv.) added and stirred for 15 minutes. The reaction was concentrated *in-vacuo,* diluted with diethyl ether, washed with NaHCO₃ saturated solution and brine, dried (MgSO₄), filtered, and concentrated. The residue was purified with automated flash chromatography (0-10% MeOH/DCM) to give 3-hexylnonyl 8-[decyl({[3-(dimethylamino)propoxy]carbonyl})amino] octadecanoate (0.30 g, 40.5%). ¹H NMR (400 MHz, CDCl₃) δ 4.09 (dt, *J=* 17.8, 6.7 Hz, 4H), 3.02 - 2.89 (m, 2H), 2.39 (d, *J=* 7.6 Hz, 2H), 2.26 (d, *J =* 4.5 Hz, 7H), 1.82 (s, 6H), 1.57 (dp, *J =* 16.8, 8.4 Hz, 4H), 1.41 (d, *J =* 10.5 Hz, 3H), 1.35 - 1.23 (m, 55H), 0.88 (td, *J =* 6.8, 1.9 Hz, 12H).

### General Methods

### Lipid nanoparticle formulations

The lipid solution contains 4 components: a PEG-conjugated lipid, an ionizable lipid, cholesterol, and a phospholipid (e.g., DSPC). Lipid stocks were prepared using the lipid identities and molar ratios as described, to achieve a total concentration of ~7 mg/mL in 100% ethanol. Firefly luciferase mRNA (TriLink Biotechnologies, L-7202) or ornithine transcarbamylase (OTC) mRNA (TriLink Biotechnologies, custom designed) was diluted in acetate, pH 5 buffer and nuclease free water to achieve a target concentration of 0.366 mg/mL mRNA in 100 mM acetate, pH 5. Equal volumes of the lipid and nucleic acid solutions were blended at a flow rate of 400 mL/min through a T-connector, and diluted with ~4 volumes of PBS, pH 7.4. Formulations were placed in Slide-A-Lyzer dialysis units (MWCO 10,000) and dialyzed overnight against 10 mM Tris, 500 mM NaCl, pH 8 buffer. Following dialysis, the formulations were concentrated to ~ 0.6 mg/mL using VivaSpin concentrator units (MWCO 100,000) and dialyzed overnight against 5 mM Tris, 10% sucrose, pH 8 buffer. Formulations were filtered through a 0.2 µm syringe filter (PES membrane). Nucleic acid concentration was determined by the RiboGreen assay. Particle size and polydispersity were determined using a Malvern Nano Series Zetasizer.

### In vivo administration of luciferase LNPs

LNP formulations encapsulating firefly luciferase mRNA were injected intravenously at 0.5 mg/kg to female BALB/c mice (6-8 weeks old). On day of injection, the LNP stocks were filtered and diluted to the required dosing concentration with phosphate buffered saline. At 6 hours post-administration, animals were anaesthetized with a lethal dosage of ketamine/xylazine. Liver samples (left lateral lobe) were collected, weighed, and flash frozen in liquid nitrogen. Liver samples were stored in FastPrep^{®} tubes at -80°C until analyzed for luciferase activity.

### Luciferase activity analysis

Frozen aliquots of liver were thawed and homogenized in 1mL of 1xCCLR (Cell Culture Lysis Reagent) using a FastPrep^{®} homogenizer. The homogenate was then centrifuged at 16,000 RPM for 10 minutes at 4°C. Twenty (20) µL of the supernatant was loaded into a 96-well white plate and luminescence measured following addition of luciferase reagent (Promega Luciferase Assay System). Luciferase activity was determined by comparing luminescence of the homogenized samples to luciferase protein standards. To account for any quenching of luminescence by components in the liver homogenate, luciferase was added to the liver homogenates of untreated animals and the resulting luminescence measured. The resulting quench factor was applied to all samples to obtain the corrected luciferase activity, and normalized per unit mass of tissue analyzed.

### In vivo administration of OTC LNPs

LNP formulations encapsulating ornithine transcarbamylase mRNA were injected intravenously at the specified dose levels to male C57BL/6 mice (6-8 weeks old). On day of injection, the LNP stocks were filtered and diluted to the required dosing concentration with phosphate buffered saline. At 24 hours post-administration, animals were anaesthetized with a lethal dosage of ketamine/xylazine. Liver samples (left lateral lobe) were collected, weighed, and flash frozen in liquid nitrogen. Liver samples were stored in FastPrep^{®} tubes at -80°C until analyzed for OTC expression.

### Human OTC expression analysis

Liver homogenates were prepared with PBS, Halt protease inhibitor cocktail and FastPrep homogenization, standardized to 2 mg/mL protein with a BCA Protein Assay, and then analyzed by ELISA. Briefly, samples were diluted with PBS and plated on Nunc Maxisorp plates. A standard curve using human PROTEIN 1 protein was prepared from the naïve animal controls (vehicle). After 2 h incubation the plates were washed, and the primary antibody was added. After 1 h incubation, the secondary antibody was added and incubated for another hour. After washing, TMB Substrate was added for color development and the reaction was stopped with 2 N sulfuric acid. The plate was read at 450/570 OD.

### In vivo administration of EGFP LNPs

LNP formulations encapsulating enhanced green fluorescent protein mRNA were injected intravenously at 0.5 mg/kg to female BALB/c mice (6-8 weeks old). On day of injection, the LNP stocks were filtered and diluted to the required dosing concentration with phosphate buffered saline. At 24 hours post-administration, animals were anaesthetized with a lethal dosage of ketamine/xylazine. Liver samples (left lateral lobe) were collected, weighed, and flash frozen in liquid nitrogen. Liver samples were stored in FastPrep^{®} tubes at -80°C until analyzed for EGFP expression.

### EGFP expression analysis

Liver homogenates were prepared in cell extraction buffer, diluted, and analyzed for EGFP protein levels using ELISA, according to manufacturer's instructions (Abcam, GFP ELISA Kit).

### Lipid LC-MS analysis

Liver tissues were treated with PBS and homogenized with a FastPrep machine, 3 cycles of 5 m/s x 15 s. Homogenates from the PBS-treated control animals was pooled and used for standard curve preparation. Samples were extracted into a deep well plate with 250 uL of an internal suitability standard. Plates were centrifuged for 15 min at 4°C and 2000 rpm. Analysis was performed using an Agilent 6520 QTOF coupled to an Agilent 1290 UHPLC, using reverse-phase LC separation. Quantitation of the ionizable lipid was by MS/MS against a calibration curve spiked into appropriately matched blank matrix. Calibration used a similar class of synthetic lipid as internal standard.

### Example 29. Particle characteristics of LNP formulations containing novel ionizable lipids

LNP formulations containing novel ionizable lipids all produced stable particles with high encapsulation efficiency (Tables 1-7).

The following compound, identified as 13-B43 is included for comparison below:

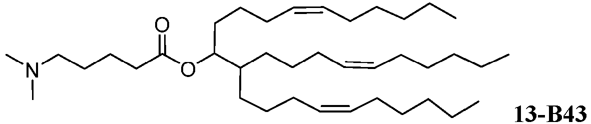

**Table 1. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 70 (0.13) | 94 |
| Compound 48 | 71 (0.04) | 96 |
| Compound 49 | 61 (0.08) | 96 |
| Compound 50 | 61 (0.07) | 93 |
| Compound 51 | 62 (0.09) | 94 |
| Compound 52 | 72 (0.05) | 95 |

**Table 2. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 72 (0.03) | 99 |
| Compound 53 | 106 (0.07) | 97 |
| Compound 21 | 77 (0.03) | 98 |
| Compound 22 | 74 (0.04) | 98 |
| Compound 40 | 87 (0.05) | 98 |
| Compound 55 | 94 (0.17) | 98 |
| Compound 58 | 81 (0.13) | 99 |
| Compound 56 | 97 (0.10) | 96 |
| Compound 25 | 78 (0.03) | 99 |

**Table 3. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 72 (0.06) | 99 |
| Compound 59 | 82 (0.07) | 99 |
| Compound 54 | 82 (0.05) | 97 |
| Compound 57 | 84 (0.03) | 80 |
| Compound 32 | 85 (0.02) | 99 |
| Compound 4 | 82 (0.03) | 99 |
| Compound 3 | 91 (0.05) | 99 |
| Compound 60 | 73 (0.05) | 99 |
| Compound 61 | 76 (0.05) | 99 |
| Compound 62 | 67 (0.08) | 98 |
| Compound 23 | 69 (0.06) | 97 |
| Compound 24 | 72 (0.08) | 96 |

**Table 4. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 72 (0.07) | 98 |
| Compound 35 | 85 (0.06) | 99 |
| Compound 36 | 81 (0.04) | 96 |
| Compound 37 | 73 (0.05) | 82 |
| Compound 26 | 97 (0.03) | 98 |
| Compound 27 | 86 (0.04) | 98 |
| Compound 28 | 90 (0.20) | 95 |
| Compound 78 | 65 (0.03) | 99 |
| Compound 79 | 65 (0.06) | 99 |

**Table 5. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 69 (0.06) | 98 |
| Compound 44 | 68 (0.06) | 98 |
| Compound 63 | 70 (0.03) | 99 |
| Compound 64 | 84 (0.00) | 99 |
| Compound 42 | 65 (0.10) | 97 |
| Compound 2 | 81 (0.08) | 96 |
| Compound 1 | 77 (0.04) | 99 |
| Compound 29 | 80 (0.02) | 98 |
| Compound 67 | 77 (0.04) | 99 |
| Compound 30 | 79 (0.03) | 98 |
| Compound 41 | 70 (0.04) | 94 |
| Compound 31 | 79 (0.04) | 98 |
| Compound 68 | 67 (0.06) | 99 |
| Compound 80 | 75 (0.08) | 99 |
| Compound 81 | 73 (0.06) | 99 |
| Compound 82 | 64 (0.03) | 99 |
| Compound 83 | 75 (0.08) | 96 |

**Table 6. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 77 (0.07) | 99 |
| Compound 84 | 80 (0.04) | 98 |
| Compound 85 | 84 (0.03) | 99 |
| Compound 43 | 73 (0.05) | 99 |
| Compound 45 | 83 (0.01) | 99 |
| Compound 46 | 72 (0.11) | 99 |
| Compound 17 | 68 (0.10) | 99 |
| Compound 16 | 76 (0.10) | 95 |
| Compound 18 | 73 (0.07) | 98 |
| Compound 19 | 71 (0.09) | 99 |
| Compound 69 | 68 (0.07) | 99 |
| Compound 5 | 68 (0.05) | 99 |
| Compound 65 | 83 (0.01) | 98 |
| Compound 71 | 86 (0.02) | 97 |
| Compound 72 | 79 (0.00) | 99 |

**Table 7. Particle characteristics of LNPs containing luciferase mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 74 (0.06) | 98 |
| Compound 73 | 82 (0.00) | 99 |
| Compound 47 | 77 (0.07) | 98 |
| Compound 76 | 69 (0.07) | 98 |
| Compound 74 | 89 (0.05) | 99 |
| Compound 33 | 61 (0.12) | 99 |
| Compound 75 | 84 (0.03) | 99 |
| Compound 20 | 72 (0.08) | 98 |
| Compound 39 | 59 (0.19) | 98 |
| Compound 87 | 78 (0.05) | 98 |
| Compound 66 | 73 (0.04) | 99 |
| Compound 34 | 70 (0.10) | 99 |
| Compound 88 | 72 (0.04) | 99 |
| Compound 86 | 65 (0.23) | 99 |
| Compound 77 | 63 (0.09) | 99 |
| Compound 38 | 65 (0.17) | 98 |

### Example 30. Performance of LNP containing novel ionizable lipids in an intravenous mouse model

LNP formulations bearing a luciferase mRNA payload and different novel ionizable lipids were compared to the benchmark control for activity in a luciferase mouse model following intravenous administration. At 6h post-dose, novel lipid formulations mediated high levels of luciferase activity in the liver. Some novel lipids reported comparable or superior activity to the benchmark, while others are not as active as the benchmark in this model, but may have utility in other LNP applications (Tables 8-14).

**Table 8. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 5.94E+07 | 1.73E+07 |
| Compound 48 | 6.49E+06 | 1.26E+06 |
| Compound 49 | 8.36E+06 | 1.18E+06 |
| Compound 50 | 4.82E+06 | 1.06E+06 |
| Compound 51 | 2.50E+07 | 1.93E+06 |
| Compound 52 | 5.65E+06 | 1.17E+06 |

**Table 9. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 7.29E+07 | 9.59E+06 |
| Compound 53 | 8.02E+06 | 1.36E+06 |
| Compound 21 | 4.58E+07 | 4.10E+06 |
| Compound 22 | 4.71E+07 | 8.19E+06 |
| Compound 40 | 3.57E+07 | 7.78E+06 |
| Compound 55 | 1.22E+06 | 3.01E+05 |
| Compound 58 | 3.11E+05 | 7.64E+04 |
| Compound 56 | 9.00E+06 | 1.82E+06 |
| Compound 25 | 7.76E+06 | 1.42E+06 |

**Table 10. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 7.04E+07 | 2.41E+07 |
| Compound 59 | 1.12E+06 | 9.00E+05 |
| Compound 54 | 3.28E+06 | 8.08E+05 |
| Compound 57 | 2.43E+06 | 7.28E+05 |
| Compound 32 | 3.07E+07 | 7.90E+06 |
| Compound 4 | 7.84E+07 | 1.06E+07 |
| Compound 3 | 5.94E+07 | 1.18E+07 |
| Compound 60 | 8.23E+06 | 1.18E+06 |
| Compound 61 | 2.45E+07 | 6.04E+06 |
| Compound 62 | 7.84E+06 | 1.51E+06 |
| Compound 23 | 4.44E+06 | 5.65E+05 |
| Compound 24 | 3.98E+06 | 6.96E+05 |

**Table 11. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 6.86E+07 | 1.11E+07 |
| Compound 35 | 1.40E+07 | 4.66E+06 |
| Compound 36 | 5.54E+06 | 1.36E+06 |
| Compound 37 | 3.01E+06 | 2.58E+05 |
| Compound 26 | 2.03E+07 | 2.75E+06 |
| Compound 27 | 9.17E+06 | 1.49E+06 |
| Compound 28 | 6.50E+06 | 8.73E+05 |
| Compound 78 | 5.30E+07 | 1.18E+07 |
| Compound 79 | 1.05E+07 | 3.29E+06 |

**Table 12. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 7.83E+07 | 5.16E+06 |
| Compound 44 | 6.17E+07 | 6.12E+07 |
| Compound 63 | 6.23E+07 | 1.45E+07 |
| Compound 64 | 9.76E+07 | 2.26E+07 |
| Compound 42 | 1.14E+07 | 6.81E+05 |
| Compound 2 | 7.11E+07 | 5.10E+06 |
| Compound 1 | 1.70E+08 | 2.64E+07 |
| Compound 29 | 6.74E+07 | 4.00E+07 |
| Compound 67 | 1.31E+08 | 2.94E+07 |
| Compound 30 | 3.76E+07 | 9.34E+06 |
| Compound 41 | 6.69E+06 | 5.40E+05 |
| Compound 31 | 1.47E+07 | 2.93E+06 |
| Compound 68 | 4.36E+07 | 1.00E+06 |
| Compound 80 | 3.28E+07 | 8.20E+06 |
| Compound 81 | 3.47E+07 | 4.92E+06 |
| Compound 82 | 1.23E+07 | 2.69E+06 |
| Compound 83 | 4.59E+07 | 2.41E+06 |

**Table 13. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 7.60E+07 | 8.27E+06 |
| Compound 84 | 6.26E+07 | 2.01E+07 |
| Compound 85 | 4.79E+07 | 1.30E+07 |
| Compound 43 | 1.04E+08 | 4.11E+07 |
| Compound 45 | 8.00E+07 | 1.41E+07 |
| Compound 46 | 1.08E+08 | 2.22E+07 |
| Compound 17 | 5.52E+07 | 5.92E+06 |
| Compound 16 | 4.39E+07 | 1.07E+07 |
| Compound 18 | 4.32E+07 | 4.15E+06 |
| Compound 19 | 1.25E+06 | 2.48E+05 |
| Compound 69 | 4.10E+07 | 7.07E+06 |
| Compound 5 | 9.49E+07 | 1.06E+07 |
| Compound 65 | 3.88E+07 | 1.74E+07 |
| Compound 71 | 4.58E+07 | 4.96E+06 |
| Compound 72 | 2.26E+07 | 9.80E+06 |

**Table 14. Luciferase activity of 0.5 mg/kg LNP containing firefly luciferase mRNA and various ionizable lipids at 6h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 6h post-IV administration | |
|---|---|---|
| | Luc Activity (pg/g liver) | Std Dev (pg/g liver) |
| 13-B43 | 6.02E+07 | 8.72E+06 |
| Compound 73 | 3.64E+07 | 5.95E+06 |
| Compound 47 | 8.09E+07 | 4.67E+06 |
| Compound 76 | 2.09E+07 | 7.52E+06 |
| Compound 74 | 6.77E+07 | 1.21E+07 |
| Compound 75 | 2.58E+07 | 3.61E+06 |
| Compound 20 | 2.71E+07 | 5.48E+06 |
| Compound 39 | 4.30E+06 | 8.30E+05 |
| Compound 87 | 1.18E+06 | 5.56E+05 |
| Compound 66 | 2.00E+07 | 4.53E+06 |
| Compound 34 | 1.91E+06 | 4.04E+05 |
| Compound 88 | 1.64E+07 | 7.91E+06 |
| Compound 86 | 1.21E+07 | 6.99E+06 |
| Compound 77 | 3.26E+07 | 7.41E+06 |
| Compound 38 | 2.13E+07 | 8.50E+06 |

### Example 31. Performance of LNP containing novel ionizable lipids in a dose response study following intravenous administration

LNP formulations bearing an OTC mRNA payload and different novel ionizable lipids were compared to the benchmark control for activity in mice following intravenous administration. At 24h post-dose, the novel lipid formulations reported dose-dependent increases in human OTC expression in the liver (Table 15).

**Table 15. Dose response of LNP containing human OTC mRNA and various ionizable lipids at 24h following intravenous administration in male C57BL/6 mice (n=4)**

| Treatment [LNP mol ratios of 1.6 : 54.6 : 32.8 : 10.9, PEG-lipid : Ionizable lipid : Cholesterol : DSPC] | Human OTC expression (ng hOTC/mg protein in liver) | | | | | |
|---|---|---|---|---|---|---|
| | 0.3 mg/kg | St Dev | 1 mg/kg | St Dev | 3 mg/kg | Std Dev |
| 13-B43 | 908 | 175 | 6651 | 3325 | 12413 | 1946 |
| Compound 43 | 1247 | 404 | 5713 | 775 | 12887 | 1260 |
| Compound 16 | 1155 | 330 | 4127 | 366 | 10316 | 1762 |
| Compound 33 | 660 | 204 | 2954 | 765 | 11080 | 3883 |

### Example 32: Parent ionizable lipid detected in liver at 6 hours following intravenous administration

LNP formulations bearing a luciferase mRNA payload and different novel ionizable lipids were compared to the benchmark for amount of ionizable lipid remaining in the liver following intravenous administration. At 6h post-dose, some treatment groups reported lower levels of novel ionizable lipid in mouse liver compared to the benchmark (Tables 16-18). In aggregate, these novel ionizable lipids either show superior activity and/or clearance compared to the control.

**Table 16. Parent ionizable lipid remaining in liver at 6h following intravenous administration of 0.5 mg/kg Luciferase mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | Average (nmol/g liver) | St Dev (nmol/g liver) |
|---|---|---|
| 13-B43 | 22.64 | 5.62 |
| Compound 59 | 22.67 | 2.58 |
| Compound 54 | 17.78 | 0.68 |
| Compound 57 | 15.88 | 1.68 |
| Compound 32 | 21.66 | 2.21 |
| Compound 4 | 18.97 | 2.38 |
| Compound 3 | 7.72 | 0.77 |
| Compound 60 | 35.39 | 5.02 |
| Compound 61 | 27.21 | 3.49 |
| Compound 62 | 20.17 | 4.14 |
| Compound 23 | 31.85 | 2.88 |
| Compound 24 | 35.16 | 20.53 |

**Table 17. Parent ionizable lipid remaining in liver at 6h following intravenous administration of 0.5 mg/kg Luciferase mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | Average (nmol/g liver) | St Dev (nmol/g liver) |
|---|---|---|
| 13-B43 | 43.87 | 15.72 |
| Compound 84 | 38.96 | 3.46 |
| Compound 85 | 37.66 | 10.20 |
| Compound 43 | 25.97 | 12.36 |
| Compound 45 | 36.39 | 14.81 |
| Compound 46 | 47.63 | 16.10 |
| Compound 17 | 24.00 | 7.00 |
| Compound 16 | 6.32 | 0.66 |
| Compound 18 | 17.02 | 3.14 |
| Compound 19 | 20.22 | 6.27 |
| Compound 69 | 50.57 | 14.28 |
| Compound 5 | 49.53 | 33.93 |
| Compound 65 | 27.84 | 10.74 |
| Compound 71 | 27.52 | 5.53 |
| Compound 72 | 47.56 | 12.24 |

**Table 18. Parent ionizable lipid remaining in liver at 6h following intravenous administration of 0.5 mg/kg Luciferase mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | Average (nmol/g liver) | St Dev (nmol/g liver) |
|---|---|---|
| 13-B43 | 63.32 | 7.62 |
| Compound 67 | 31.32 | 6.01 |
| Compound 17 | 36.58 | 20.51 |
| Compound 16 | 17.06 | 3.48 |
| Compound 43 | 23.83 | 3.74 |
| Compound 73 | 31.41 | 4.58 |
| Compound 47 | 54.78 | 9.98 |
| Compound 76 | 17.86 | 2.26 |
| Compound 74 | 17.92 | 1.40 |
| Compound 75 | 20.44 | 3.29 |
| Compound 20 | 1.82 | 0.66 |
| Compound 39 | 2.91 | 0.72 |
| Compound 87 | 3.23 | 0.64 |
| Compound 66 | 34.35 | 4.06 |
| Compound 34 | 1.78 | 0.65 |
| Compound 88 | 7.88 | 1.49 |
| Compound 86 | 12.79 | 5.26 |
| Compound 77 | 30.63 | 5.83 |
| Compound 38 | 3.14 | 2.44 |

### Example 33. Clearance of LNPs containing novel ionizable lipids following intravenous administration

LNP formulations bearing an OTC mRNA payload and different novel ionizable lipids were compared to the benchmark for lipid clearance in the liver following intravenous administration. At various timepoints post-dose, lipid levels were assessed in the liver. Some novel ionizable lipids reported more rapid clearance rates compared to the benchmark (Table 19).

**Table 19. Parent ionizable lipid remaining in liver following intravenous administration of 0.5 mg/kg human OTC mRNA LNP in male C57BL/6 mice (n=4)**

| Treatment [LNP mol ratios of 1.6 : 54.6 : 32.8 : 10.9, PEG-lipid : Ionizable lipid : Cholesterol : DSPC] | Time Post-Dose (h) | Average (nmol/g liver) | St Dev (nmol/g liver) |
|---|---|---|---|
| 13-B43 | 0.25 | 29.17 | 10.45 |
| | 1 | 46.37 | 7.13 |
| | 3 | 63.61 | 9.80 |
| | 6 | 45.23 | 2.66 |
| | 24 | 40.01 | 2.93 |
| | 168 | 34.42 | 3.91 |
| Compound 67 | 0.25 | 12.37 | 1.61 |
| | 1 | 21.11 | 0.44 |
| | 3 | 16.12 | 1.48 |
| | 6 | 10.82 | 2.69 |
| | 24 | 11.31 | 1.80 |
| | 168 | 0.72 | 0.20 |
| Compound 43 | 0.25 | 22.97 | 4.05 |
| | 1 | 38.91 | 6.47 |
| | 3 | 28.99 | 2.81 |
| | 6 | 24.19 | 4.79 |
| | 24 | 13.50 | 3.24 |
| | 168 | 0.19 | 0.02 |
| Compound 16 | 0.25 | 11.11 | 3.02 |
| | 1 | 15.47 | 2.03 |
| | 3 | 10.91 | 1.84 |
| | 6 | 4.71 | 0.60 |
| | 24 | 1.33 | 0.43 |
| | 168 | 0.04 | 0.03 |
| Compound 33 | 0.25 | 33.97 | 5.84 |
| | 1 | 98.57 | 22.91 |
| | 3 | 76.20 | 14.36 |
| | 6 | 86.57 | 8.83 |
| | 24 | 58.68 | 6.57 |
| | 168 | 6.22 | 0.46 |

### Example 34. Particle characteristics of LNP formulations containing novel ionizable lipids

LNP formulations containing novel ionizable lipids all produced stable particles with high encapsulation efficiency (Tables 20-23).

**Table 20. Particle characteristics of LNPs containing EGFP mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 75 (0.07) | 98 |
| Compound 89 | 83 (0.03) | 99 |
| Compound 90 | 79 (0.07) | 99 |
| Compound 91 | 85 (0.03) | 99 |

**Table 21. Particle characteristics of LNPs containing EGFP mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 75 (0.08) | 97 |
| Compound 92 | 71 (0.09) | 99 |
| Compound 93 | 69 (0.02) | 98 |
| Compound 94 | 83 (0.06) | 98 |
| Compound 95 | 80 (0.08) | 96 |
| Compound 96 | 88 (0.05) | 99 |
| Compound 97 | 85 (0.07) | 99 |
| Compound 98 | 84 (0.06) | 99 |
| Compound 99 | 86 (0.04) | 97 |
| Compound 100 | 72 (0.08) | 98 |
| Compound 101 | 70 (0.10) | 100 |
| Compound 102 | 67 (0.09) | 98 |
| Compound 103 | 67 (0.09) | 98 |

**Table 22. Particle characteristics of LNPs containing EGFP mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 73 (0.08) | 97 |
| Compound 104 | 74 (0.07) | 99 |
| Compound 105 | 82 (0.08) | 97 |
| Compound 106 | 73 (0.13) | 95 |
| Compound 107 | 76 (0.06) | 98 |
| Compound 108 | 80 (0.07) | 95 |
| Compound 109 | 75 (0.10) | 98 |
| Compound 110 | 67 (0.11) | 98 |
| Compound 111 | 69 (0.09) | 97 |
| Compound 112 | 82 (0.08) | 95 |
| Compound 113 | 72 (0.05) | 98 |
| Compound 114 | 73 (0.08) | 98 |
| Compound 115 | 72 (0.04) | 98 |
| Compound 116 | 75 (0.09) | 98 |
| Compound 117 | 80 (0.06) | 99 |
| Compound 118 | 83 (0.06) | 98 |
| Compound 119 | 73 (0.09) | 97 |

**Table 23. Particle characteristics of LNPs containing EGFP mRNA and novel ionizable lipids for in vivo administration**

| LNP mol ratios of 1.6 : 54.6 : 32.8 : 10.9, PEG2000-C-DMA : Ionizable lipid : Cholesterol : DSPC | Final Product | |
|---|---|---|
| | Z-avg (PDI) nm | % EE |
| 13-B43 | 76 (0.15) | 98 |
| Compound 120 | 69 (0.15) | 99 |
| Compound 121 | 77 (0.10) | 99 |
| Compound 122 | 70 (0.13) | 100 |
| Compound 123 | 73 (0.15) | 99 |

### Example 35. Performance of LNP containing novel ionizable lipids in an intravenous mouse model

LNP formulations bearing EGFP mRNA payload and different novel ionizable lipids were compared to the benchmark control for activity in an EGFP mouse model following intravenous administration. At 24 h post-dose, novel lipid formulations mediated high levels of EGFP expression in the liver. Some novel lipids reported comparable or superior activity to the benchmark, while others are not as active as the benchmark in this model, but may have utility in other LNP applications (Tables 24-27).

**Table 24. EGFP expression of 0.5 mg/kg LNP containing EGFP mRNA and various ionizable lipids at 24h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | EGFP (pg/g liver) | Std Dev (pg/g liver) |
| 13-B43 | 3.75E+07 | 1.11E+07 |
| Compound 89 | 2.01E+07 | 4.14E+06 |
| Compound 90 | 2.12E+07 | 5.94E+06 |
| Compound 91 | 3.15E+07 | 1.17E+07 |

**Table 25. EGFP expression of 0.5 mg/kg LNP containing EGFP mRNA and various ionizable lipids at 24h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | EGFP (pg/g liver) | Std Dev (pg/g liver) |
| 13-B43 | 9.74E+04 | 4.62E+04 |
| Compound 92 | 6.34E+05 | 1.69E+05 |
| Compound 93 | 1.77E+05 | 8.03E+04 |
| Compound 94 | 1.20E+06 | 5.66E+05 |
| Compound 95 | 8.76E+05 | 2.75E+05 |
| Compound 96 | 1.50E+06 | 1.60E+05 |
| Compound 97 | 1.48E+06 | 3.22E+05 |
| Compound 98 | 9.76E+05 | 2.33E+05 |
| Compound 99 | 1.05E+06 | 2.26E+05 |
| Compound 100 | 5.98E+05 | 2.47E+05 |
| Compound 101 | 2.25E+05 | 3.52E+04 |
| Compound 102 | 1.94E+05 | 1.26E+05 |
| Compound 103 | 9.54E+04 | 9.11E+04 |

**Table 26. EGFP expression of 0.5 mg/kg LNP containing EGFP mRNA and various ionizable lipids at 24h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | EGFP (pg/g liver) | Std Dev (pg/g liver) |
| 13-B43 | 5.23E+07 | 1.18E+07 |
| Compound 104 | 7.36E+06 | 1.26E+05 |
| Compound 105 | 1.86E+07 | 3.14E+06 |
| Compound 106 | 2.45E+07 | 5.36E+06 |
| Compound 107 | 3.66E+07 | 6.62E+06 |
| Compound 108 | 5.41E+07 | 1.10E+07 |
| Compound 109 | 5.30E+07 | 5.45E+06 |
| Compound 110 | 4.01E+07 | 4.19E+06 |
| Compound 111 | 3.89E+07 | 2.12E+06 |
| Compound 112 | 4.78E+07 | 8.51E+06 |
| Compound 113 | 3.45E+07 | 6.79E+06 |
| Compound 114 | 4.29E+07 | 1.15E+07 |
| Compound 115 | 6.06E+07 | 9.14E+06 |
| Compound 116 | 2.93E+07 | 8.42E+06 |
| Compound 117 | 3.15E+07 | 2.86E+06 |
| Compound 118 | 1.86E+07 | 1.19E+06 |
| Compound 119 | 1.60E+07 | 1.73E+06 |

**Table 27. EGFP expression of 0.5 mg/kg LNP containing EGFP mRNA and various ionizable lipids at 24h following intravenous administration in BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | EGFP (pg/g liver) | Std Dev (pg/g liver) |
| 13-B43 | 6.46E+07 | 1.37E+07 |
| Compound 120 | 1.90E+07 | 5.37E+06 |
| Compound 121 | 3.81E+07 | 6.70E+06 |
| Compound 122 | 2.35E+07 | 4.61E+06 |
| Compound 123 | 3.94E+07 | 4.23E+06 |

### Example 36. Parent ionizable lipid detected in liver at 24 hours following intravenous administration

LNP formulations bearing EGFP mRNA payload and different novel ionizable lipids were compared to the benchmark for amount of ionizable lipid remaining in the liver following intravenous administration. At 24h post-dose, some treatment groups reported lower levels of novel ionizable lipid in mouse liver compared to the benchmark (Tables 28-31). In aggregate, these novel ionizable lipids either show superior activity and/or clearance compared to the control.

**Table 28. Parent ionizable lipid remaining in liver at 24h following intravenous administration of 0.5 mg/kg EGFP mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | Average (nmol/g liver) | St Dev (nmol/g liver) |
| 13-B43 | 38.48 | 6.56 |
| Compound 89 | 4.32 | 0.80 |
| Compound 90 | 13.37 | 0.36 |
| Compound 91 | 18.58 | 0.83 |

**Table 29. Parent ionizable lipid remaining in liver at 24h following intravenous administration of 0.5 mg/kg EGFP mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | Average (nmol/g liver) | St Dev (nmol/g liver) |
| 13-B43 | 29.77 | 4.09 |
| Compound 92 | 8.83 | 1.83 |
| Compound 93 | 24.61 | 6.54 |
| Compound 94 | 6.57 | 1.14 |
| Compound 95 | 9.44 | 2.33 |
| Compound 96 | 17.68 | 1.69 |
| Compound 97 | 19.31 | 1.44 |
| Compound 98 | 30.21 | 4.04 |
| Compound 99 | 19.93 | 2.90 |
| Compound 100 | 5.03 | 1.03 |
| Compound 101 | 16.30 | 1.84 |
| Compound 102 | 5.72 | 1.13 |
| Compound 103 | 0.00 | 0.00 |

**Table 30. Parent ionizable lipid remaining in liver at 24h following intravenous administration of 0.5 mg/kg EGFP mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | Average (nmol/g liver) | St Dev (nmol/g liver) |
| 13-B43 | 50.86 | 2.51 |
| Compound 104 | 51.14 | 15.83 |
| Compound 105 | 18.96 | 2.32 |
| Compound 106 | 56.97 | 24.23 |
| Compound 107 | 28.74 | 4.34 |
| Compound 108 | 3.86 | 1.02 |
| Compound 109 | 7.32 | 1.44 |
| Compound 110 | 33.07 | 3.52 |
| Compound 111 | 17.74 | 3.63 |
| Compound 112 | 1.73 | 0.32 |
| Compound 113 | 4.68 | 1.48 |
| Compound 114 | 12.79 | 1.71 |
| Compound 115 | 27.15 | 7.40 |
| Compound 116 | 60.05 | 7.75 |
| Compound 117 | 49.53 | 8.08 |
| Compound 118 | 62.81 | 6.24 |
| Compound 119 | 25.25 | 4.12 |

**Table 31. Parent ionizable lipid remaining in liver at 24h following intravenous administration of 0.5 mg/kg EGFP mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | 24h post-IV administration | |
|---|---|---|
| | Average (nmol/g liver) | St Dev (nmol/g liver) |
| 13-B43 | 23.9 | 7.13 |
| Compound 120 | 4.8 | 1.33 |
| Compound 121 | 2.1 | 0.68 |
| Compound 122 | 6.3 | 1.25 |
| Compound 123 | 10.5 | 1.23 |

### Example 37. Performance of LNP containing novel ionizable lipids in a dose response study following intravenous administration

LNP formulations bearing EGFP mRNA payload and different novel ionizable lipids were compared to the benchmark control for activity in mice following intravenous administration. At 24h post-dose, the novel lipid formulations reported dose-dependent increases in EGFP expression in the liver (Table 32).

**Table 32. Dose response of LNP containing EGFP mRNA and various ionizable lipids at 24h following intravenous administration in female BALB/c mice (n=4)**

| Treatment [LNP mol ratios of 1.6 : 54.6 : 32.8 : 10.9, PEG-lipid : Ionizable lipid : Cholesterol : DSPC] | EGFP expression (ng EGFP/g liver) | | | | | |
|---|---|---|---|---|---|---|
| | 0.3 mg/kg | St Dev | 1 mg/kg | St Dev | 3 mg/kg | Std Dev |
| 13-B43 | 3.65E+07 | 6.48E+06 | 1.88E+08 | 3.84E+07 | 4.40E+08 | 7.11E+07 |
| Compound 101 | 2.65E+07 | 1.11E+07 | 1.04E+08 | 2.70E+07 | 3.90E+08 | 5.54E+07 |
| Compound 111 | 1.34E+07 | 1.88E+06 | 8.58E+07 | 4.87E+06 | 2.48E+08 | 2.88E+07 |
| Compound 115 | 2.98E+07 | 2.81E+06 | 1.31E+08 | 2.64E+07 | 3.41E+08 | 5.46E+07 |

### Example 38: Clearance of LNPs containing novel ionizable lipids following intravenous administration

LNP formulations bearing EGFP mRNA payload and different novel ionizable lipids were compared to the benchmark for lipid clearance in the liver following intravenous administration. At various timepoints post-dose, lipid levels were assessed in the liver. Some novel ionizable lipids reported more rapid clearance rates compared to the benchmark (Table 33).

**Table 33. Parent ionizable lipid remaining in liver following intravenous administration of 0.5 mg/kg EGFP mRNA LNP in female BALB/c mice (n=4)**

| Treatment [LNP mol ratios of 1.6 : 54.6 : 32.8 : 10.9, PEG-lipid : Ionizable lipid : Cholesterol : DSPC] | Time Post-Dose (h) | Average (nmol/g liver) | St Dev (nmol/g liver) |
|---|---|---|---|
| 13-B43 | 0.25 | 29.8 | 2427 |
| | 1 | 31.2 | 1763 |
| | 3 | 40.2 | 5235 |
| | 6 | 45.2 | 2210 |
| | 24 | 26.4 | 688 |
| | 168 | 25.6 | 1767 |
| Compound 101 | 0.25 | 23.8 | 3753 |
| | 1 | 42.4 | 2078 |
| | 3 | 26.9 | 2103 |
| | 6 | 30.6 | 718 |
| | 24 | 12.6 | 1727 |
| | 168 | 0.6 | 35 |
| Compound 111 | 0.25 | 30.4 | 1174 |
| | 1 | 43.0 | 2145 |
| | 3 | 39.1 | 1934 |
| | 6 | 32.0 | 1151 |
| | 24 | 12.8 | 2732 |
| | 168 | 0.0 | 0 |
| Compound 115 | 0.25 | 36.7 | 4930 |
| | 1 | 51.3 | 7591 |
| | 3 | 44.8 | 4707 |
| | 6 | 45.3 | 630 |
| | 24 | 0.0 | 0 |
| | 168 | 1.3 | 232 |

## Claims

1. A compound of formula (I): 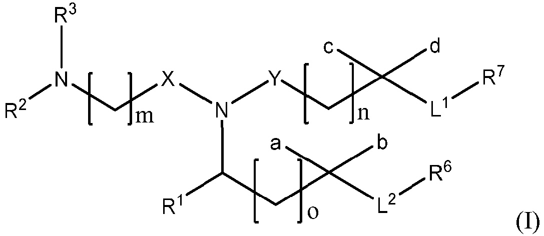 or a salt thereof, wherein:
X is -CH₂-, -O-, -O(C=O); or -C(=O)-
Y is absent, -CH₂-, -O-, -SO₂-, or -C(=O)-;
R¹ is C₂-C₂₅hydrocarbyl;
R² is C₁-C₃alkyl; and R³ is C₁-C₃alkyl; or R² and R³ taken together with the nitrogen to which they are attached form a a aziridino, azetidino, or pyrrolidino ring;
m is 0, 1, 2, 3, 4, 5 or 6;
n is 0-15;
o is 0-15;
L¹ is absent, -(C=O)-O-, -(C=O)N(H)N(R⁴)-, -(C=O)N(H)- N=C(R⁴)-, -(C=O)N(H)N(H)C(R⁴)(R⁵)-, -(C=O)-O-CH₂-R^{z}, or -O(C=O)-R^{w}-;
L² is absent, -(C=O)-O-, -(C=O)N(H)N(R^{4'})-, -(C=O)N(H)- N=C(R^{4'})-, -(C=O)N(H)N(H)C(R^{4'})(R^{5'})-, -(C=O)-O-CH₂-R^{z'}, or -O(C=O)-R^{x}-
a is H or F;
b is H or F;
c is H or F; and d is H or F; or c and d taken together with the carbon to which they are attached form -C(=O)-;
R⁴ is H or C₂-C₂₅hydrocarbyl;
R⁵ is H or C₂-C₂₅hydrocarbyl;
R^{4'} is H or C₂-C₂₅hydrocarbyl;
R^{5'} is H or C₂-C₂₅hydrocarbyl;
R⁶ is C₂-C₂₅hydrocarbyl;
R⁷ is C₂-C₂₅hydrocarbyl;
R^{w} is absent, -C(F)H-, or -C(F)₂-;
R^{x} is absent, -C(F)H-, or -C(F)₂-;
R^{z} is phenyl that is substituted with 1, 2, or 3 groups independently selected from C₂-C₁₀hydrocarbyl; and
R^{z'} is phenyl that is substituted with 1, 2, or 3 groups independently selected from C₂-C₁₀hydrocarbyl.

2. The compound or salt of claim 1, wherein:
(i) R¹ is C₅-C₂₅hydrocarbyl, C₅-C₂₅alkyl, C₅-C₂₅alkenyl, 1-nonyl or 1-decyl; and/or
(ii) X is -CH₂-, -C(=O)- or -O-; and/or
(iii) R² is methyl, ethyl or propyl; and/or
(iv) R³ is methyl, ethyl or propyl; and/or
(v) m is 1, 2 or 3; and/or
(vi) o is 2-15, 10-15, 2-10 or 2-5; and/or
(vii) L² is -(C=O)-O-, -(C=O)N(H)N(R⁴)(R⁵)-, -(C=O)N(H)-N=C(R⁴)-, -(C=O)N(H)N(H)C(R⁴)(R⁵)-, or -(C=O)-O-CH₂-R^{z}, and optionally wherein L² is - (C=O)-O-, -(C=O)N(H)N(R⁴)(R⁵)- or -(C=O)-O-CH₂-R^{z}; and/or
(viii) a is H or F; and/or
(ix) b is H or F; and/or
(x) R⁶ is C₂-C₂₅hydrocarbyl, C₅-C₂₅hydrocarbyl, C₂-C₂₅alkyl, C₅-C₂₅alkyl or C₅-C₂₅alkenyl.

3. The compound or salt of claim 1 or 2 wherein:
(i) 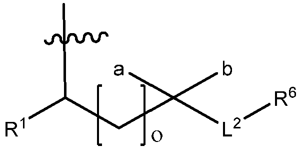 is selected from the group consisting of: 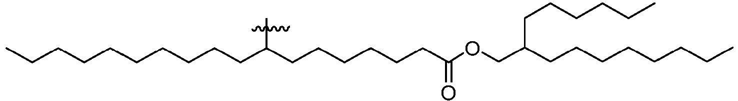 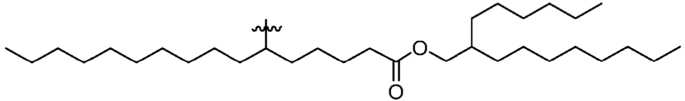 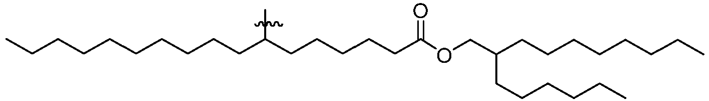 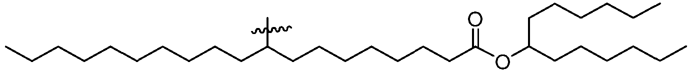 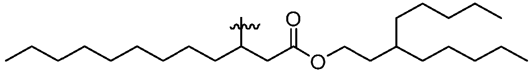 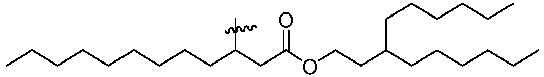 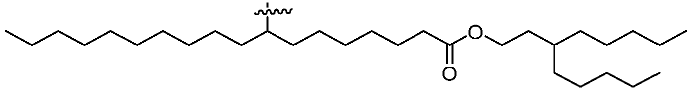 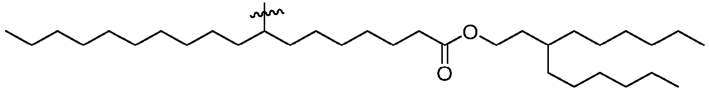 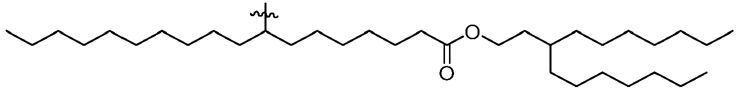 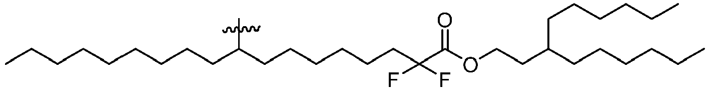 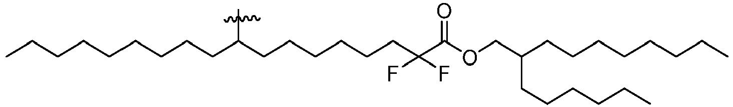 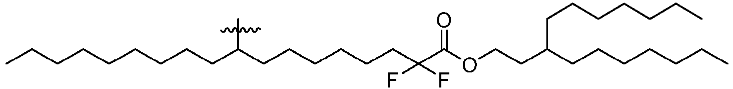 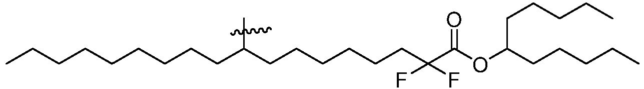 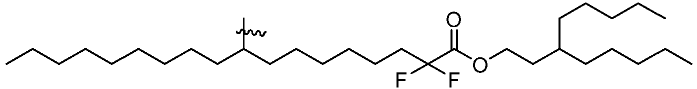 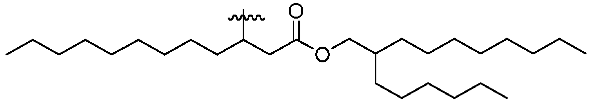 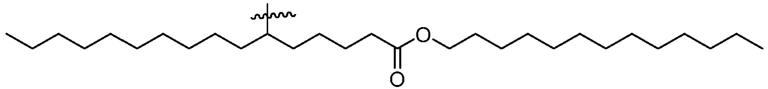 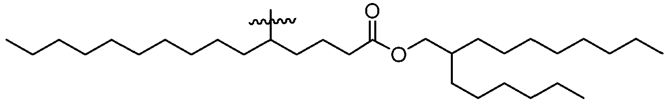 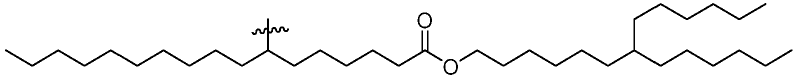 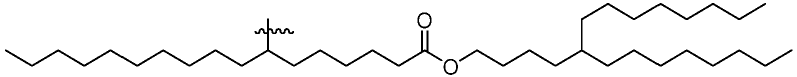 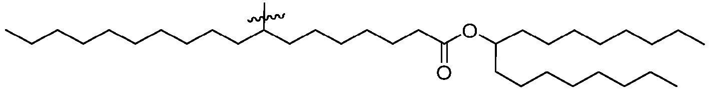 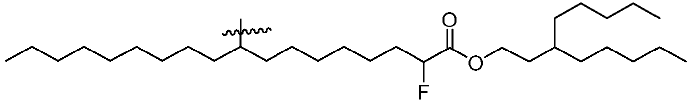 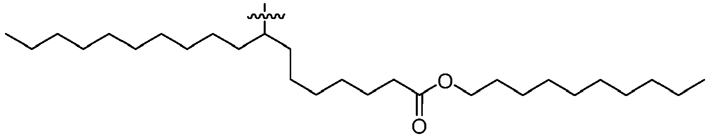 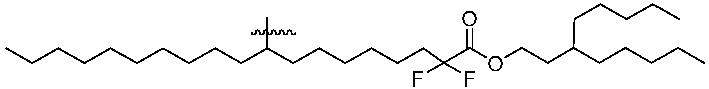 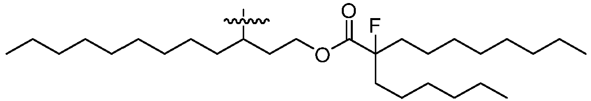 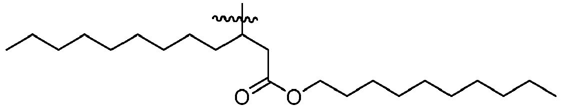 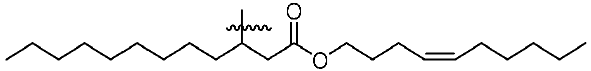 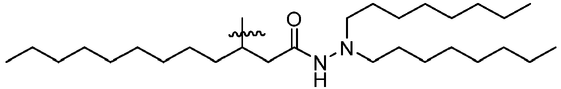 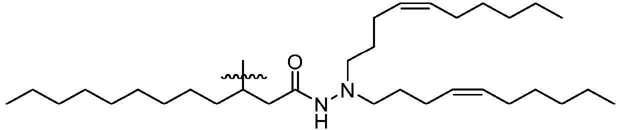 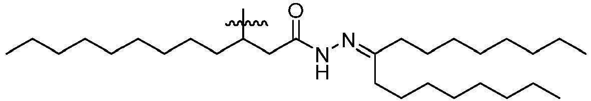 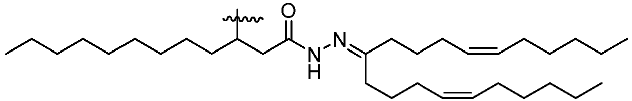 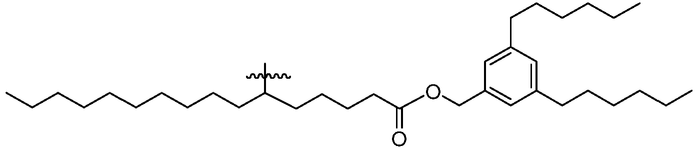 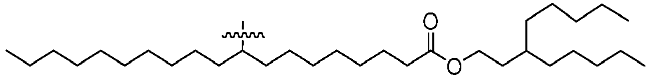 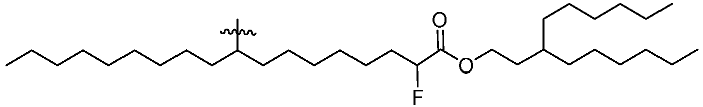 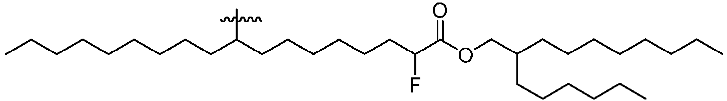 and 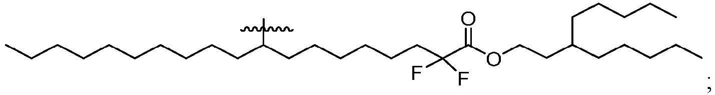 or
(ii) 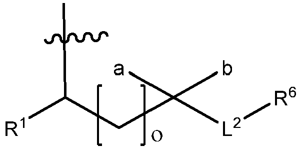 is selected from the group consisting of: 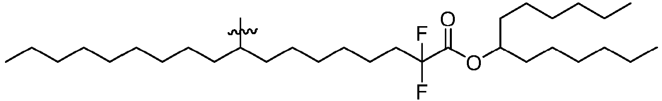 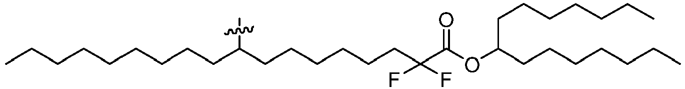 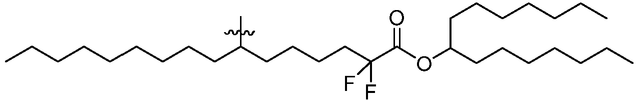 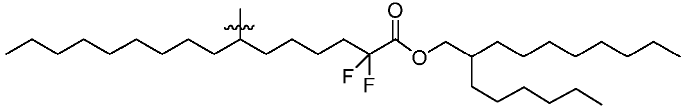 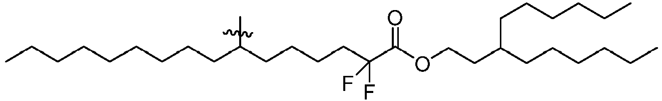 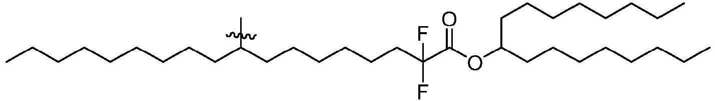 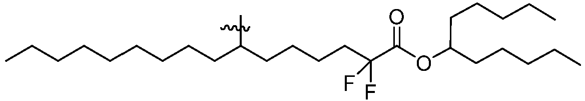 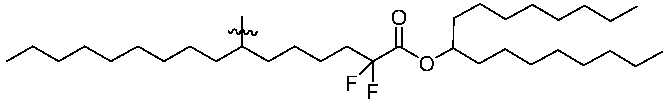 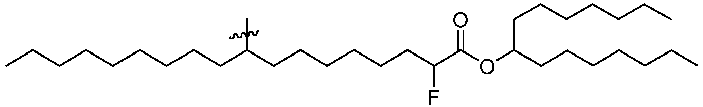 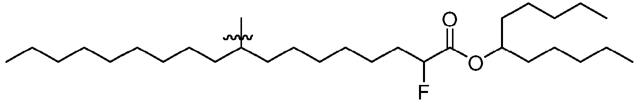 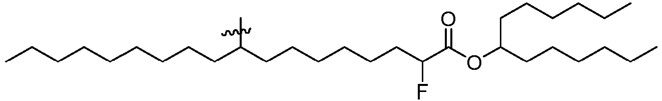 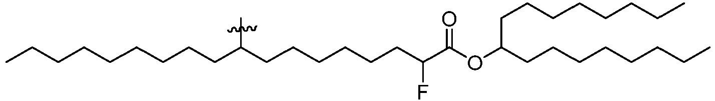 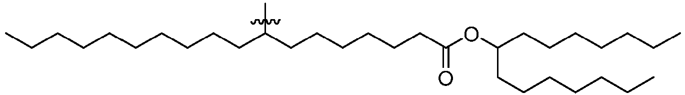 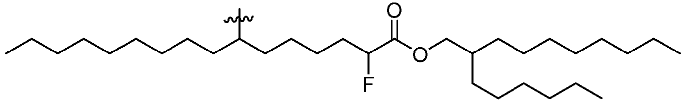 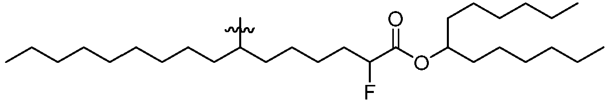 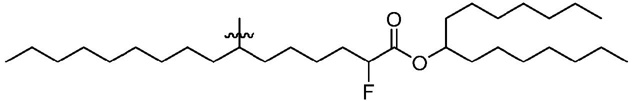 and 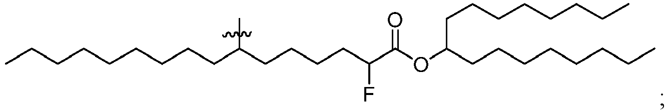 or
(iii) 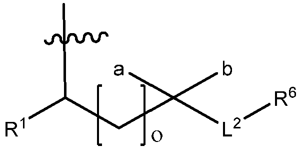 is selected from the group consisting of: 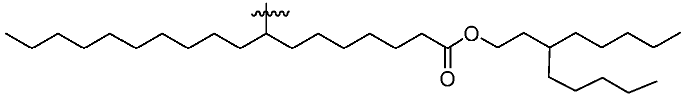 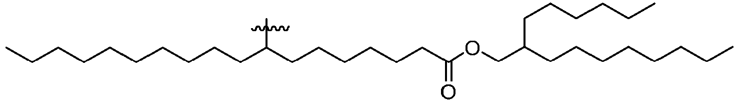 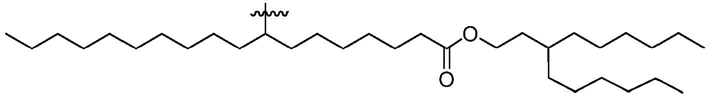 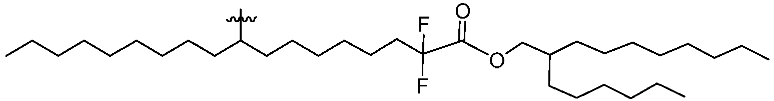 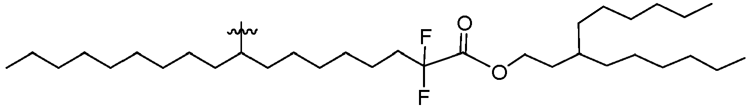 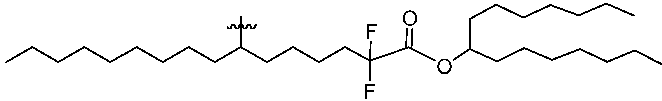 and

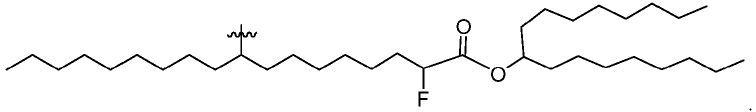

4. The compound or salt of any one of claims 1-3, wherein:
(i) Y is -CH₂-, -C(=O)- or -O-; and/or
(ii) n is 2-15, 10-15, 2-10 or 2-5; and/or
(iii)(a) L¹ is absent; and L² is -(C=O)-O-, -(C=O)N(H)N(R⁴)(R⁵)-, -(C=O)N(H)-N=C(R⁴)-, -(C=O)N(H)N(H)C(R⁴)(R⁵)-, or -(C=O)-O-CH₂-R^{z}, or (b) L¹ is -(C=O)-O-, -(C=O)N(H)N(R⁴)(R⁵)-, -(C=O)N(H)-N=C(R⁴)-, -(C=O)N(H)N(H)C(R⁴)(R⁵)-, or -(C=O)-O-CH₂-R^{a}; and L² is absent, or (c) L¹ is -(C=O)-O-, -(C=O)N(H)N(R⁴)(R⁵)-, -(C=O)N(H)-N=C(R⁴)-, -(C=O)N(H)N(H)C(R⁴)(R⁵)-, or -(C=O)-O-CH₂-R^{a}, or (d) L¹ is -(C=O)-O, L¹ is -(C=O)N(H)N(R⁴)(R⁵)- or L¹ is -(C=O)-O-CH₂-R^{z}; and/or
(iv) c is H or F; and/or
(v) d is H or F; and/or
(vi) R⁷ is C₂-C₂₅hydrocarbyl, C₅-C₂₅hydrocarbyl, C₂-C₂₅alkyl, C₅-C₂₅alkyl or C₅-C₂₅alkenyl.

5. The compound or salt of any one of claims 1-3, wherein:
(i) 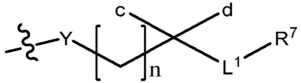 is selected from the group consisting of: 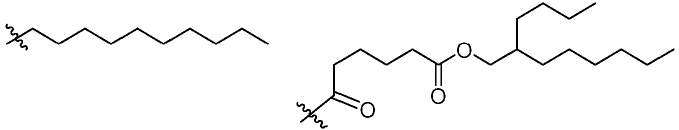 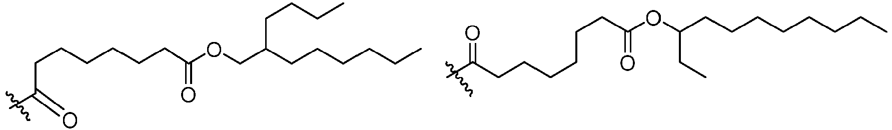 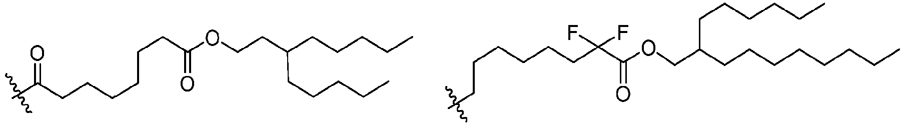 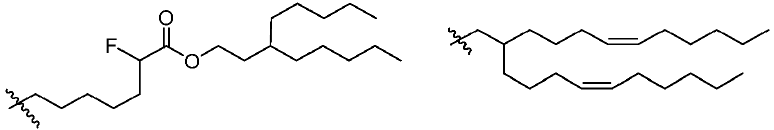 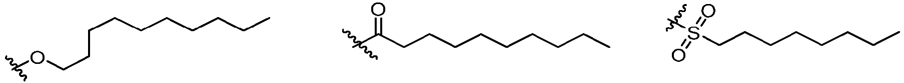 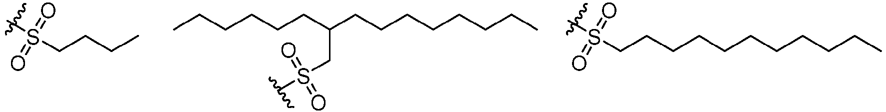 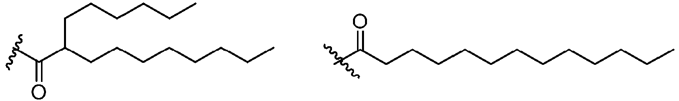 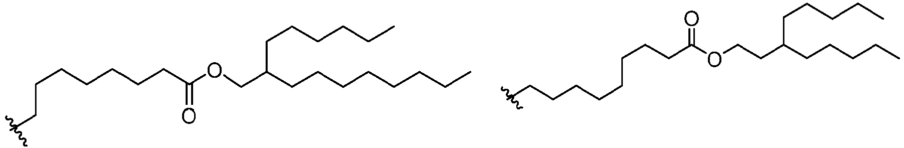 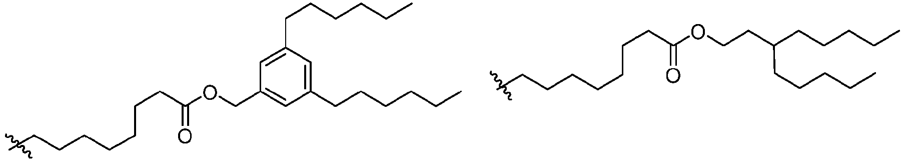 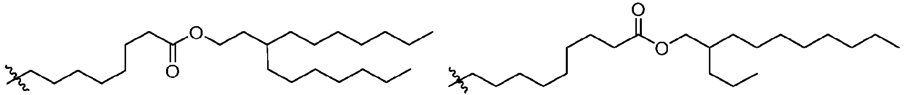 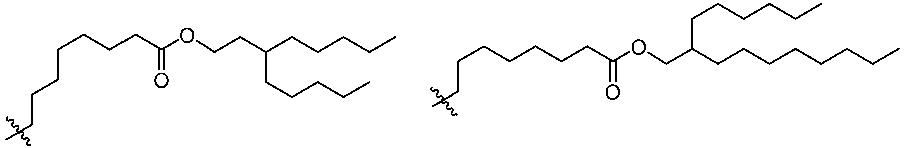 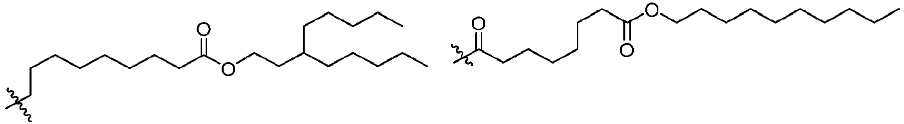 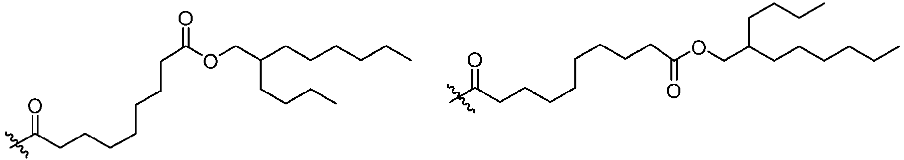 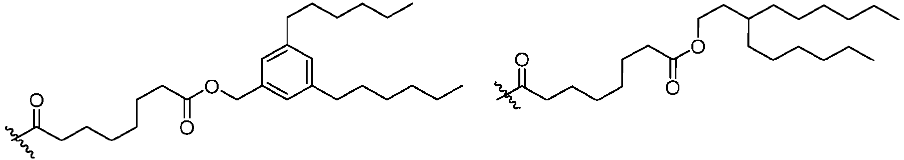 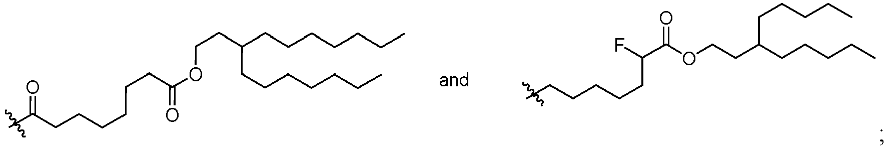 or
(ii) 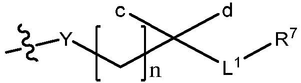 is selected from the group consisting of: 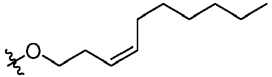 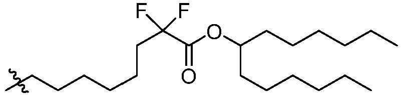 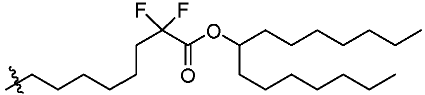 and 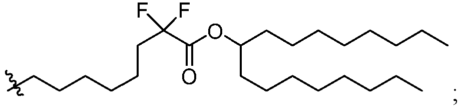 or
(iii) 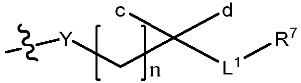 is selected from the group consisting of:

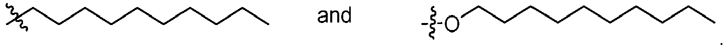

6. The compound or salt of any one of claims 1-4, wherein:
(i) R⁴ is C₂-C₂₅hydrocarbyl, C₅-C₂₅hydrocarbyl, C₂-C₂₅alkyl, C₅-C₂₅alkyl or C₅-C₂₅alkenyl; or
(ii) R*⁵* is C₂-C₂₅hydrocarbyl, C₅-C₂₅hydrocarbyl, C₂-C₂₅alkyl, C₅-C₂₅alkyl or C₅-C₂₅alkenyl; or
(iii) R^{z} is phenyl that is substituted with 1 or 2 groups independently selected from C₂-C₁₀hydrocarbyl, and optionally wherein each C₂-C₁₀hydrocarbyl is independently selected from the group consisting of C₅-C₁₀hydrocarbyl, C₂-C₁₀alkyl, C₅-C₁₀alkyl and C₅-C₁₀alkenyl.

7. The compound or salt of claim 1 or 2, wherein:
(i) R^{4'} is C₂-C₂₅hydrocarbyl, C₅-C₂₅hydrocarbyl, C₂-C₂₅alkyl, C₅-C₂₅alkyl or C₅-C₂₅alkenyl; or
(ii) R^{5'} is C₂-C₂₅hydrocarbyl, C₅-C₂₅hydrocarbyl, C₂-C₂₅alkyl, C₅-C₂₅alkyl or C₅-C₂₅alkenyl or
(iii) R^{z'} is phenyl that is substituted with 1 or 2 groups independently selected from C₂-C₁₀hydrocarbyl, and optionally wherein each C₂-C₁₀hydrocarbyl is independently selected from the group consisting of C₅-C₁₀hydrocarbyl, C₂-C₁₀alkyl, C₅-C₁₀alkyl and C₅-C₁₀alkenyl.

8. The compound or salt of claim 1, which is selected from the group consisting of: 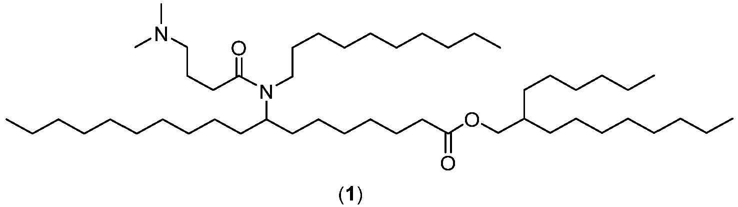 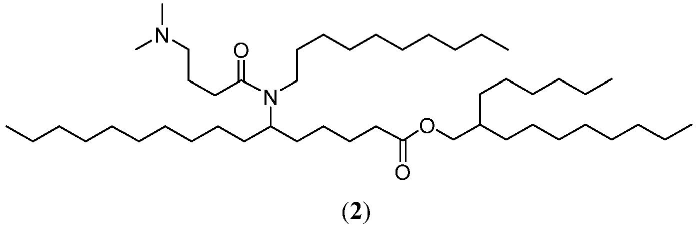 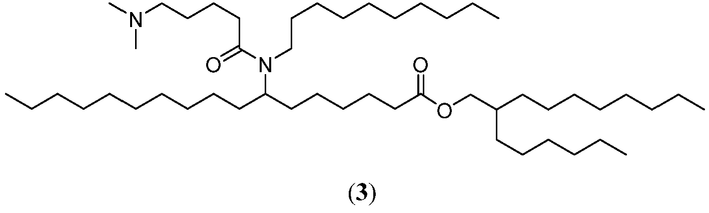 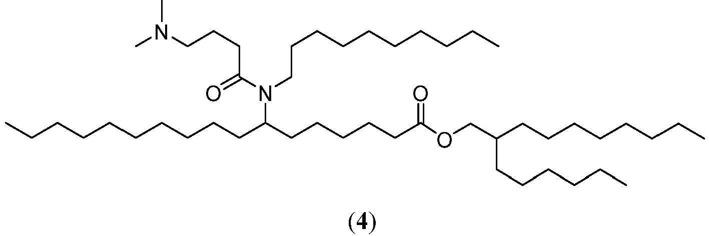 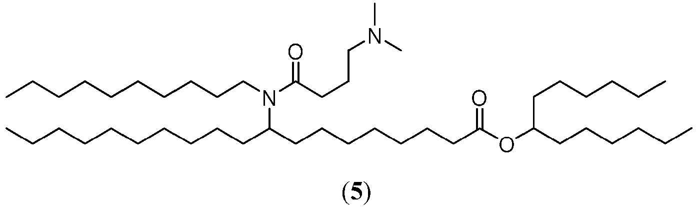 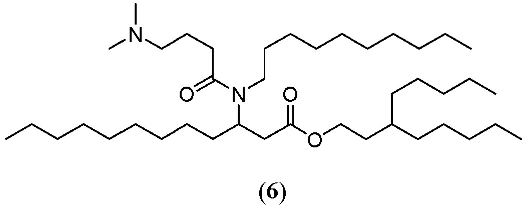 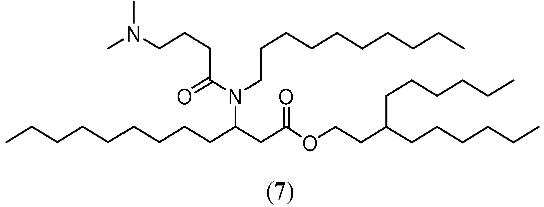 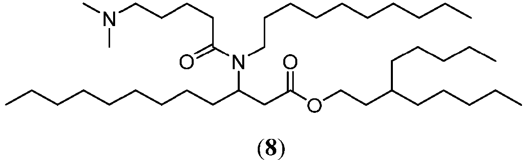 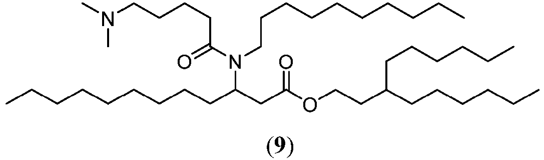 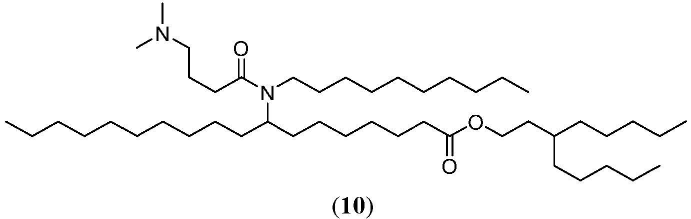 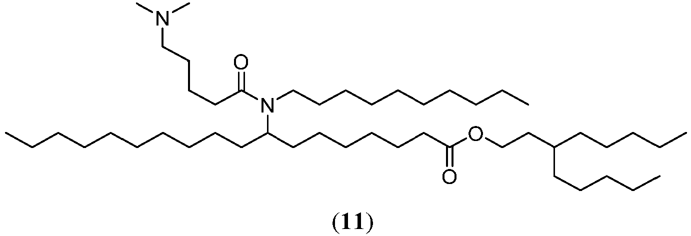 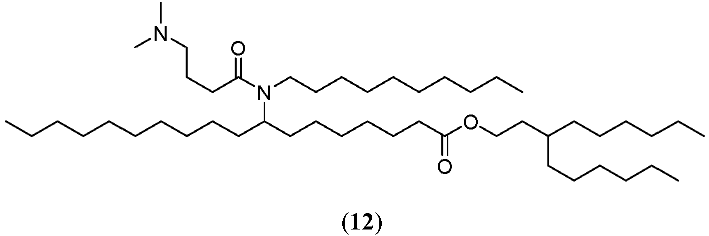 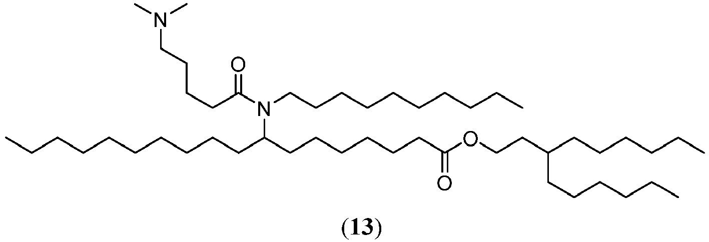 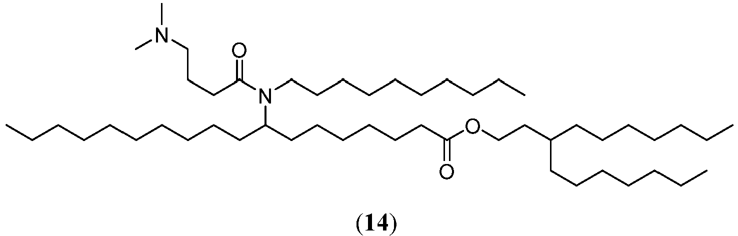 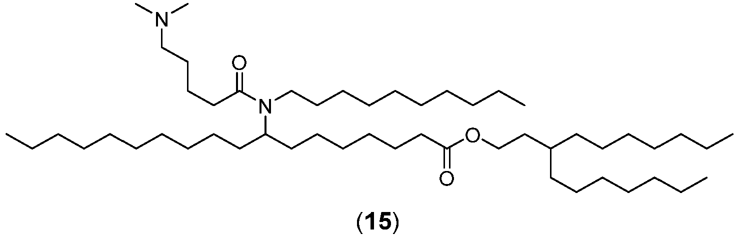 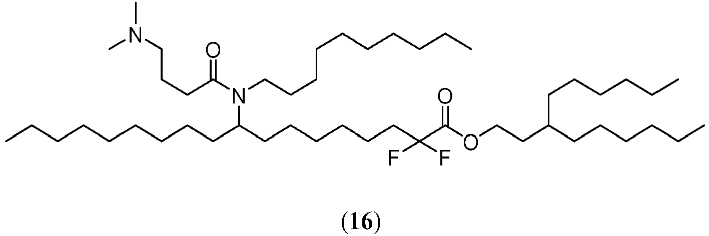 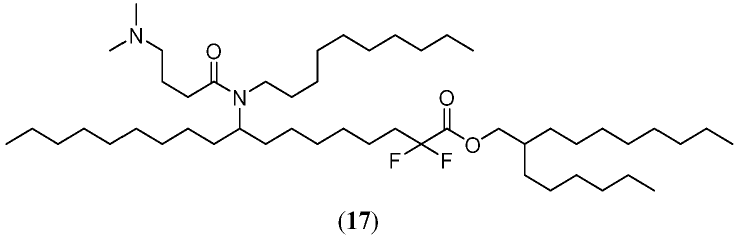 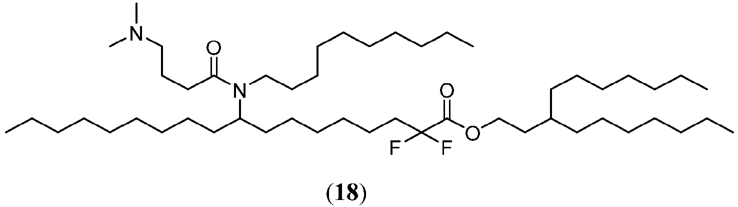 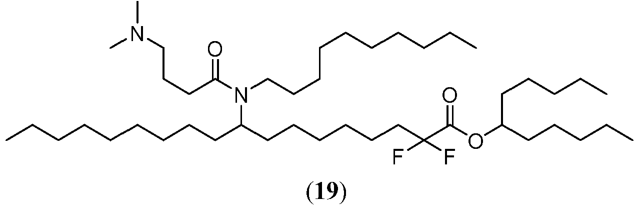 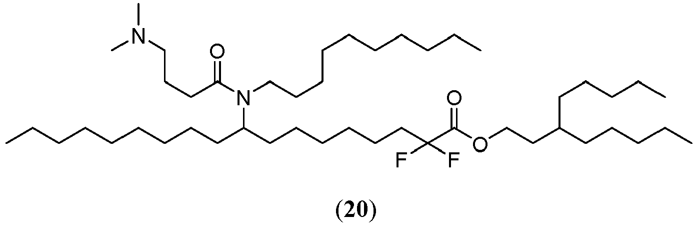 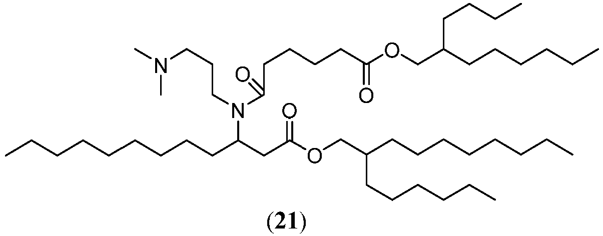 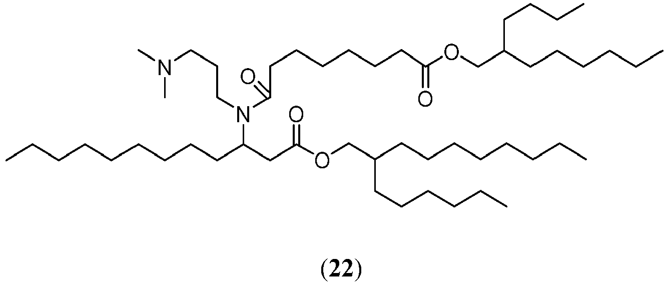 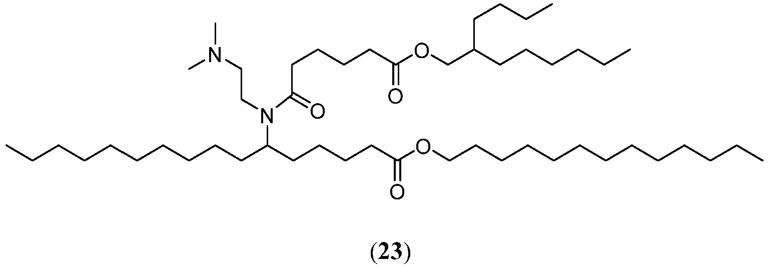 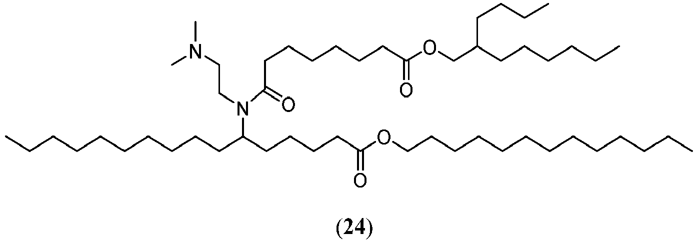 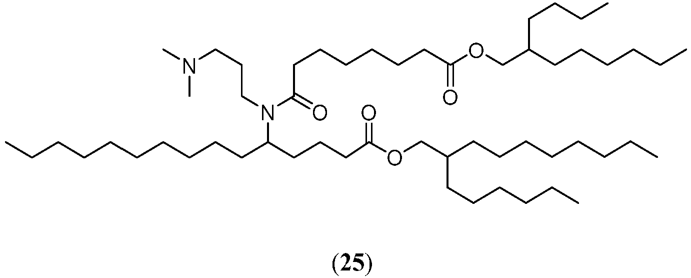 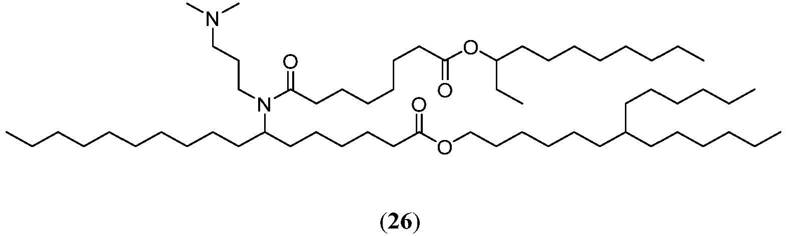 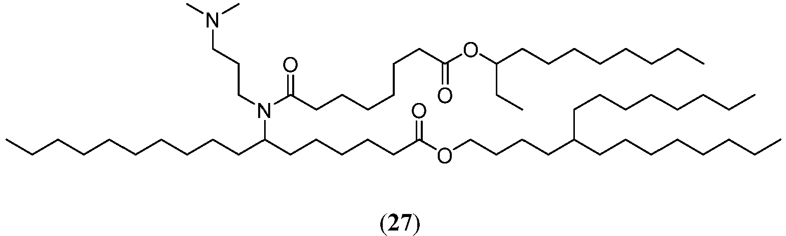 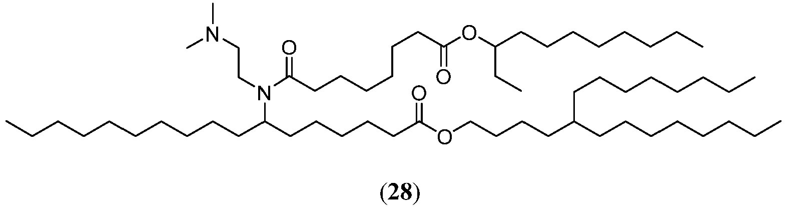 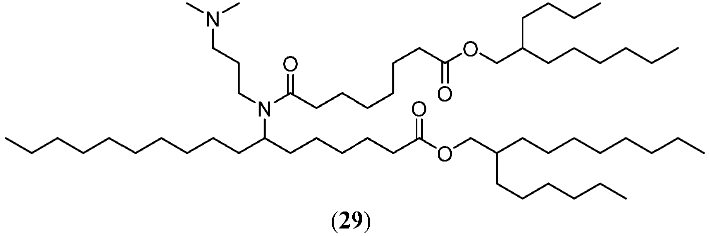 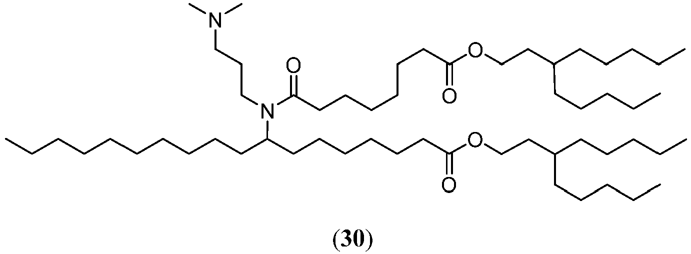 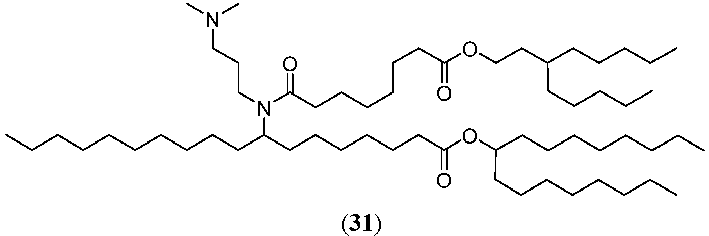 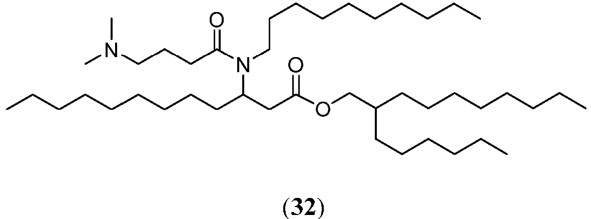 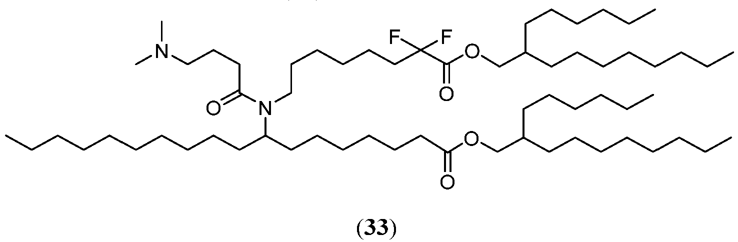 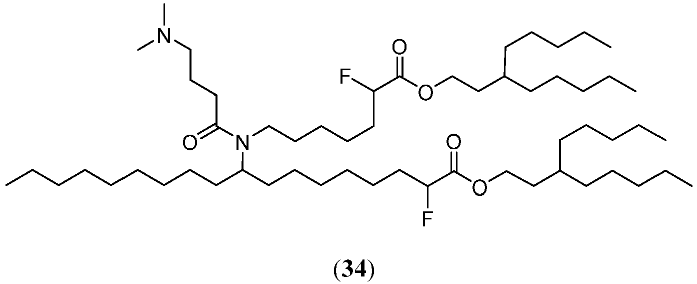 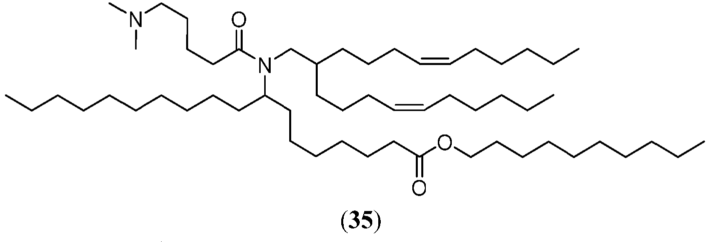 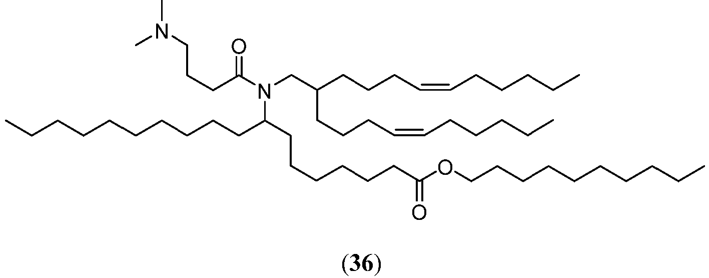 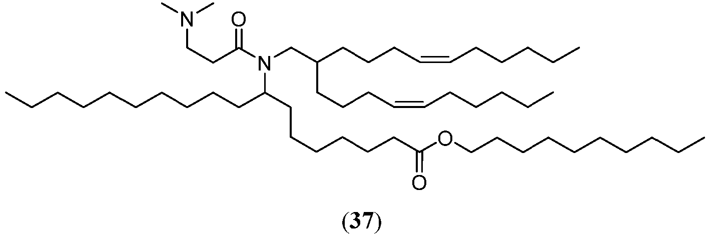 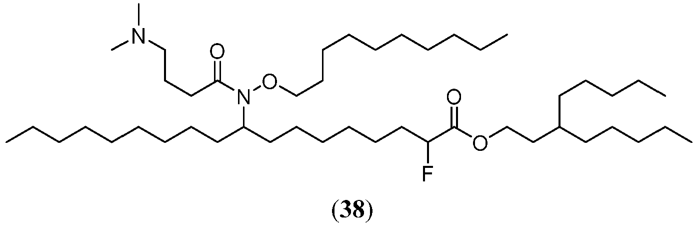 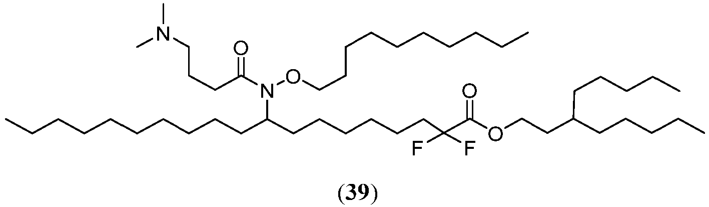 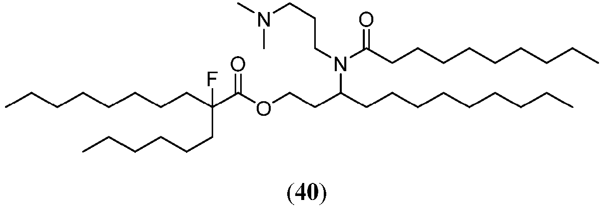 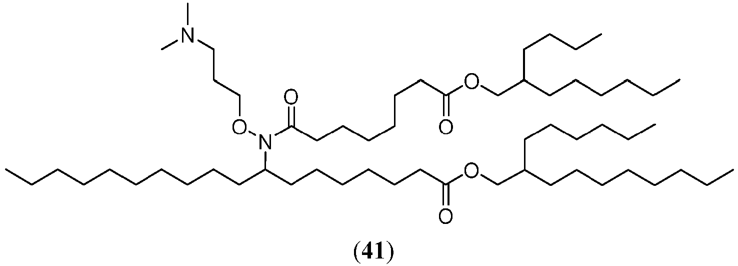 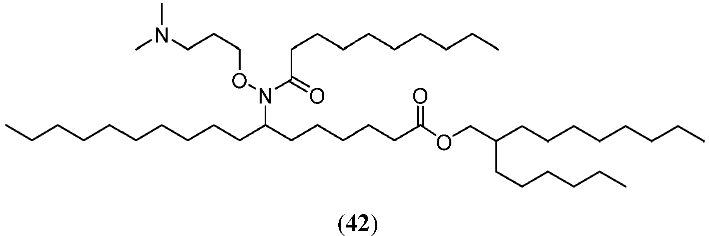 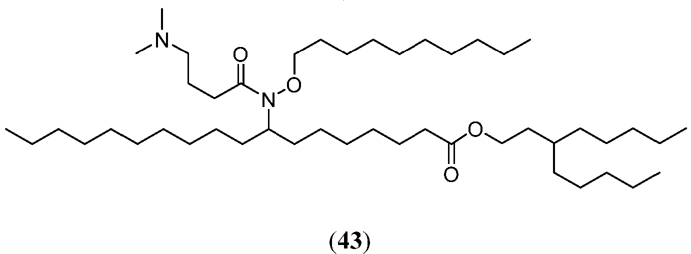 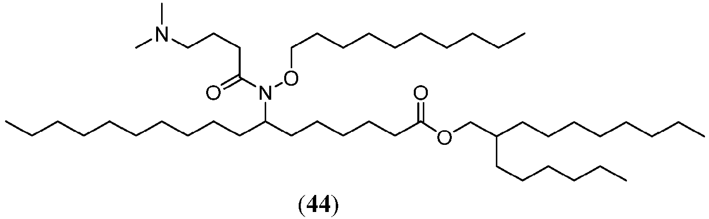 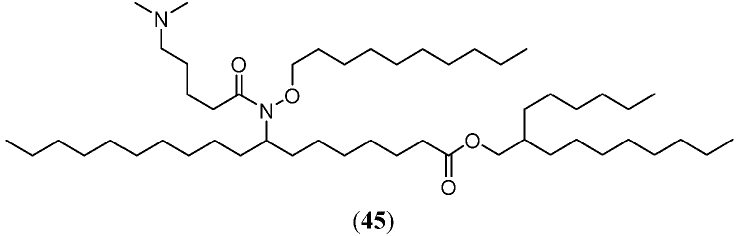 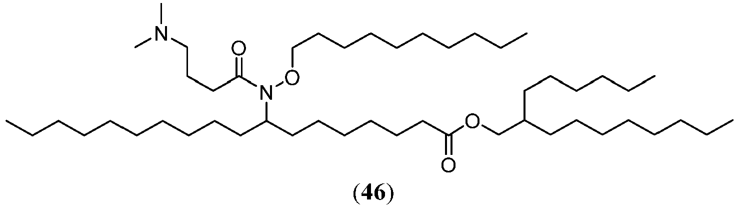 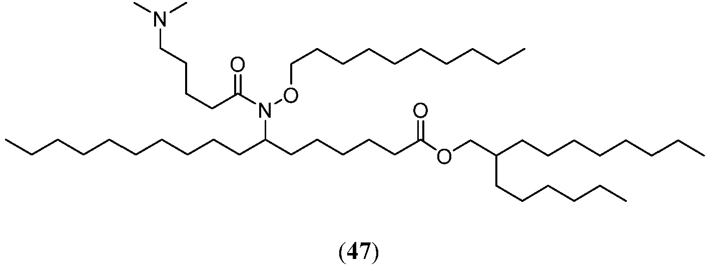 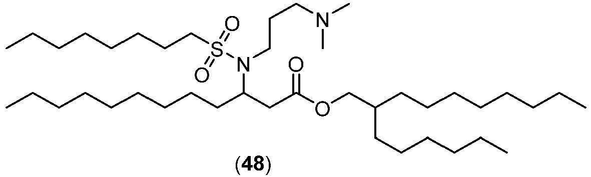 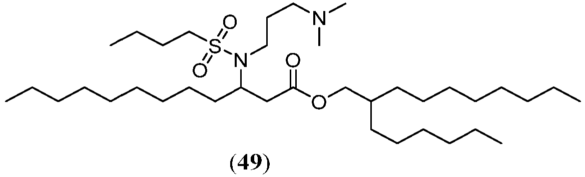 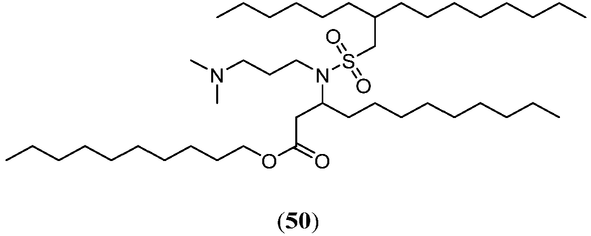 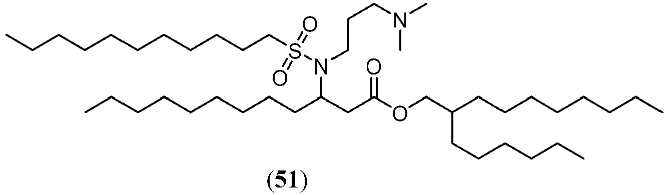 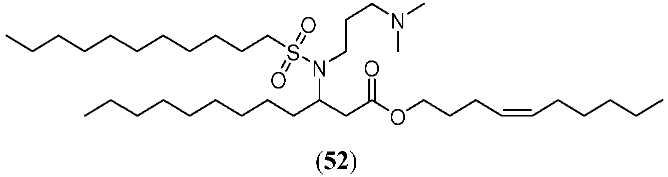 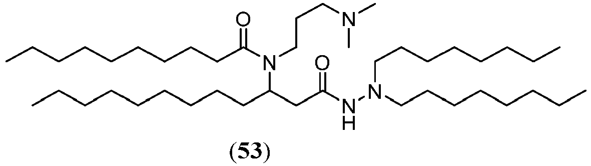 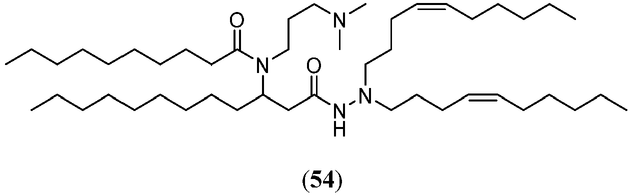 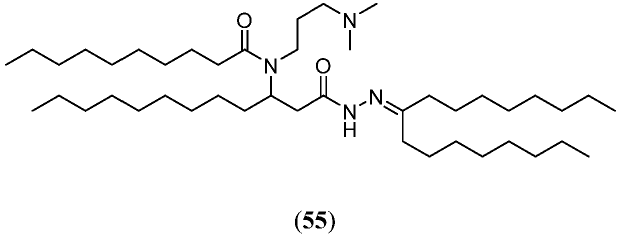 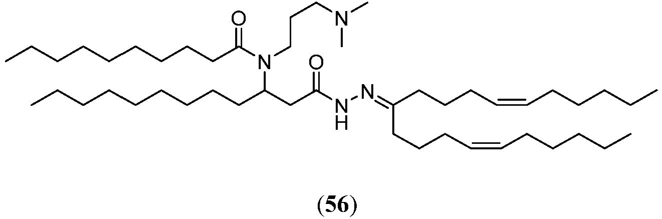 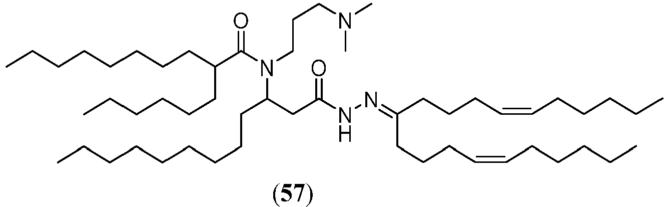 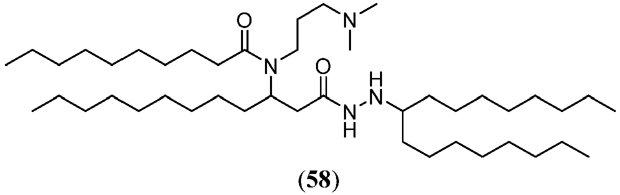 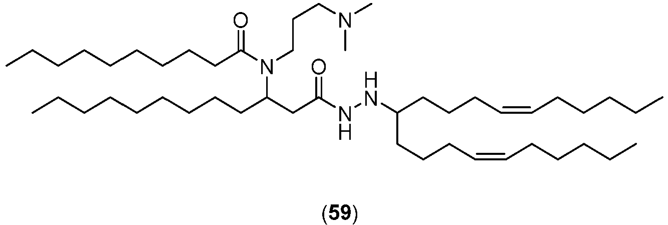 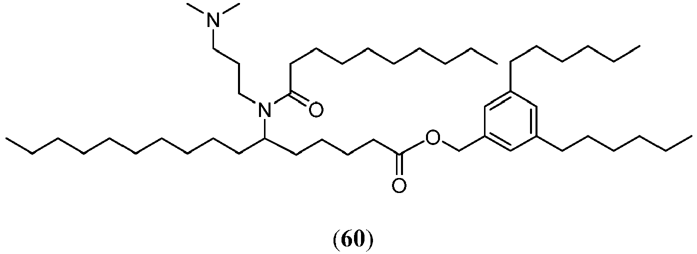 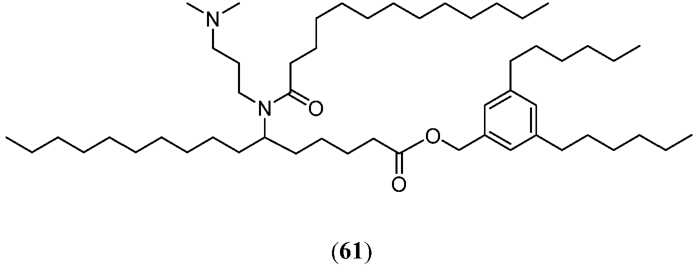 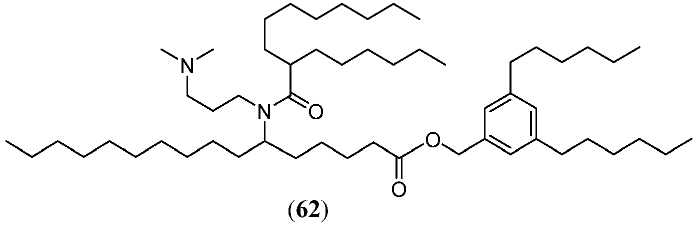 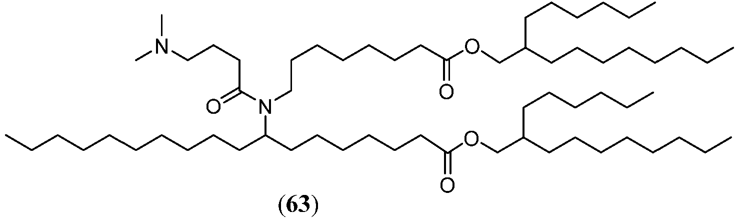 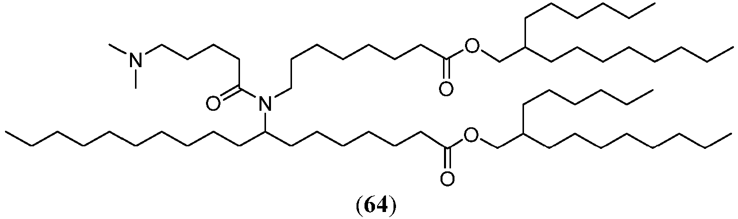 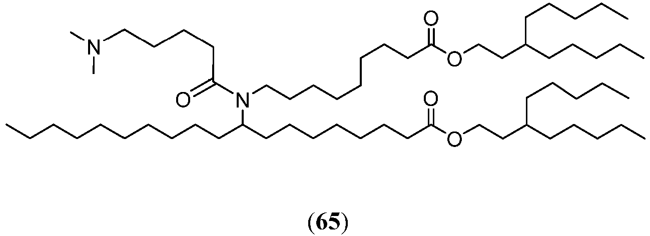 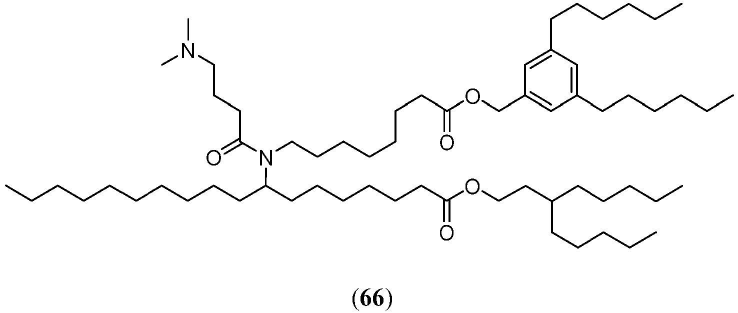 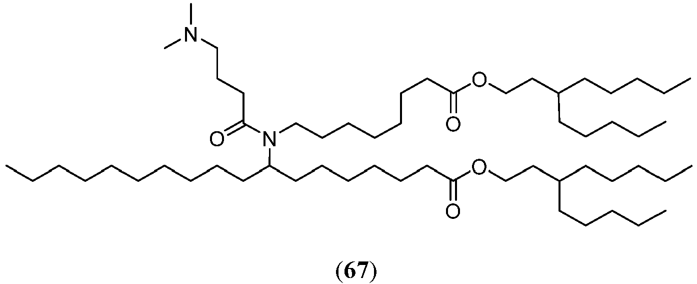 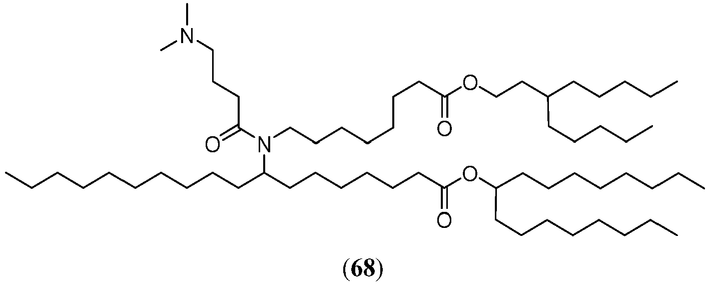 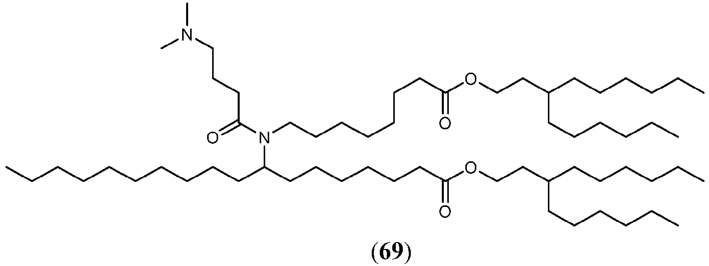 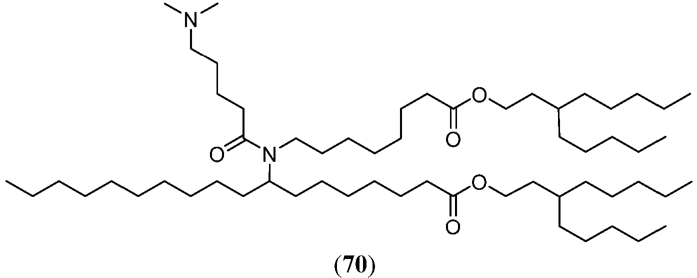 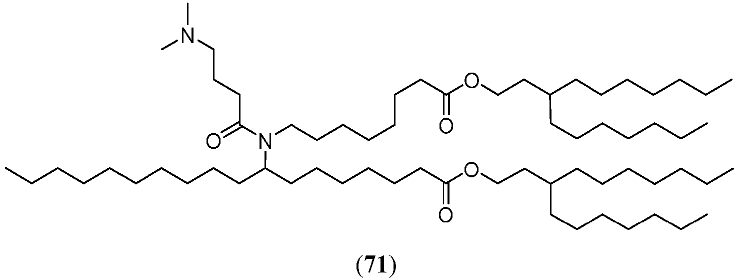 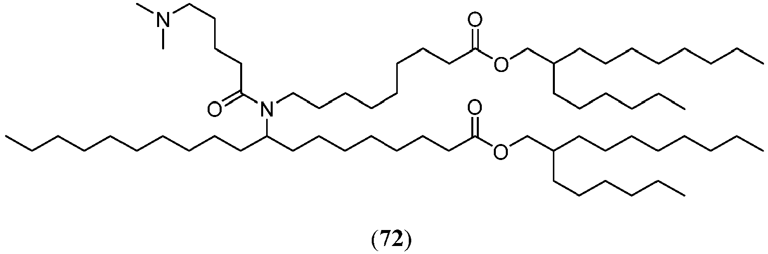 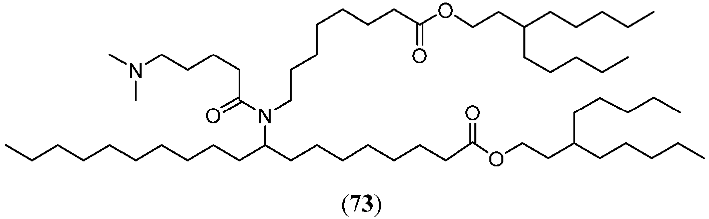 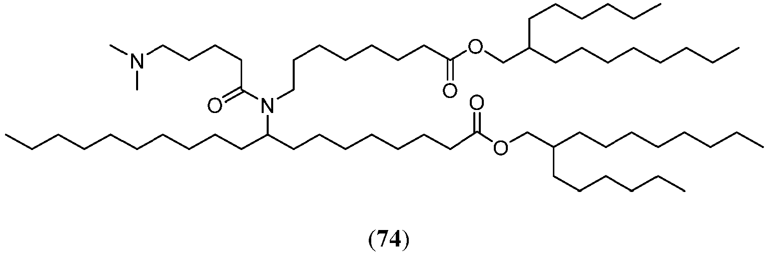 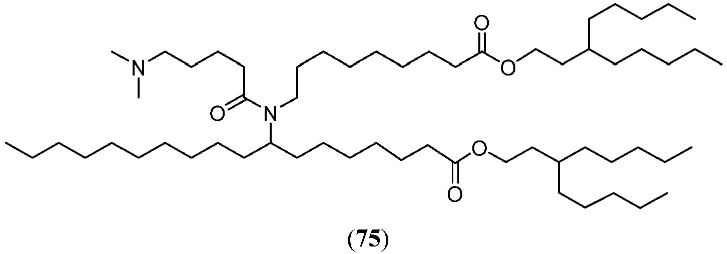 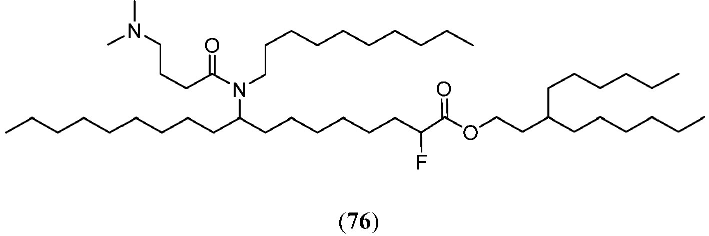 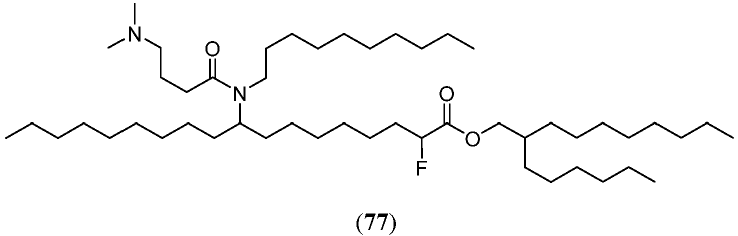 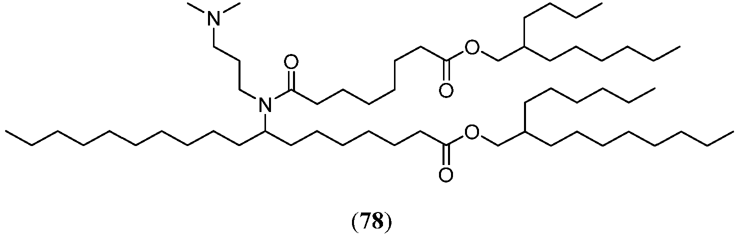 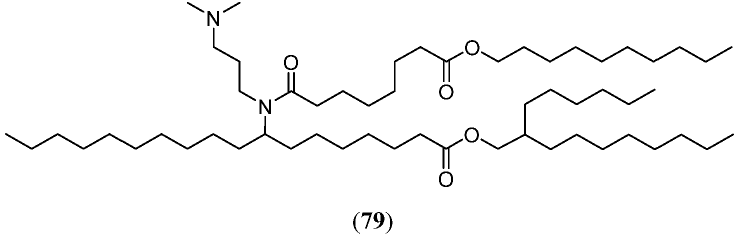 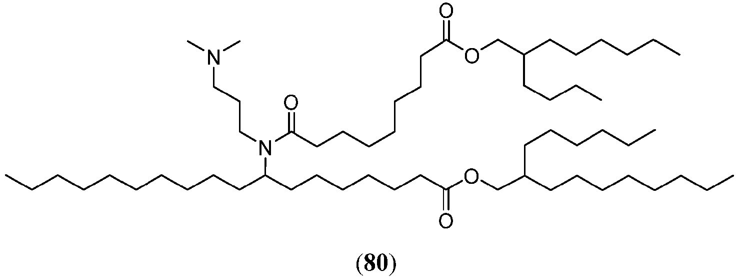 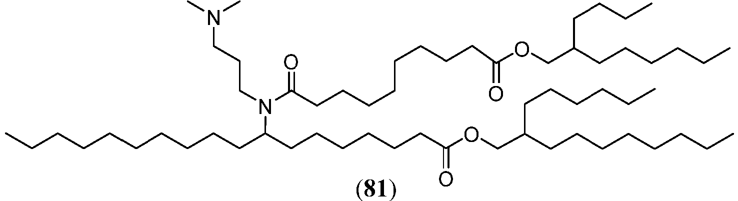 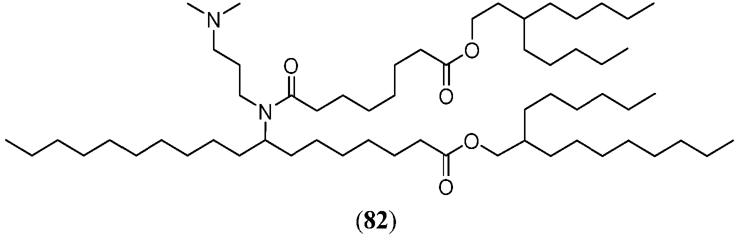 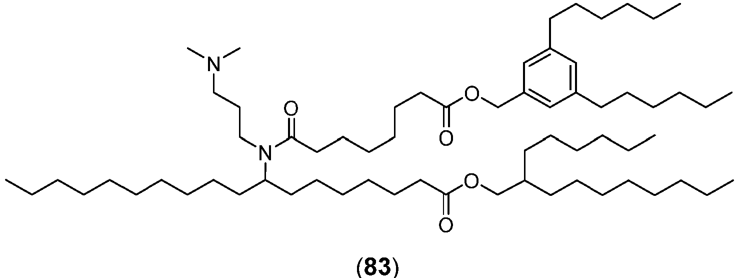 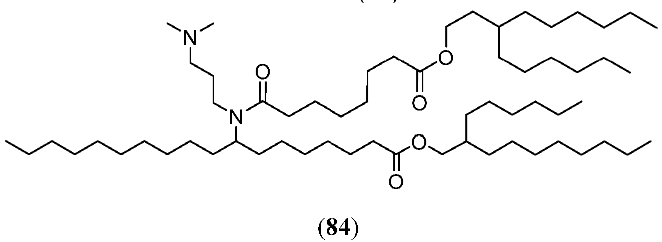 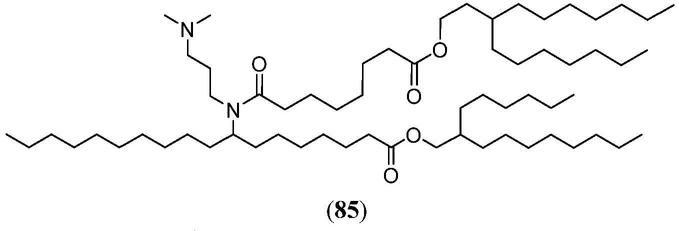 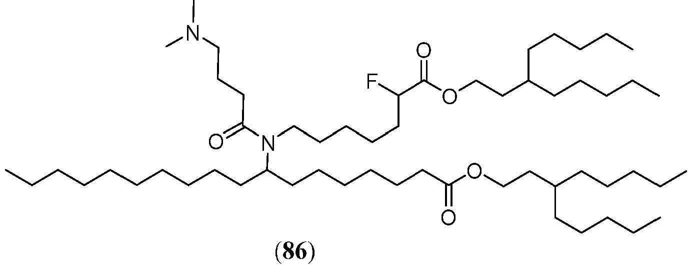 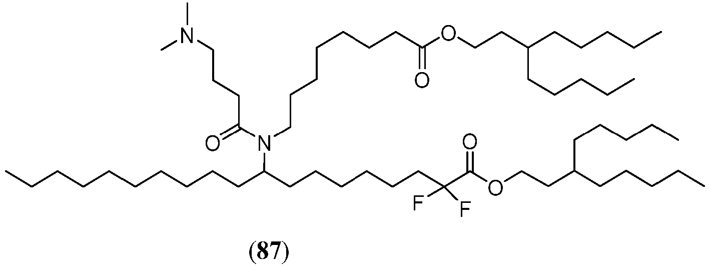 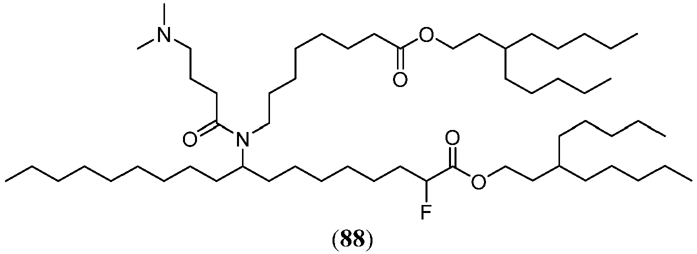 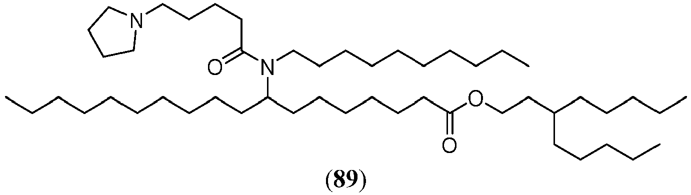 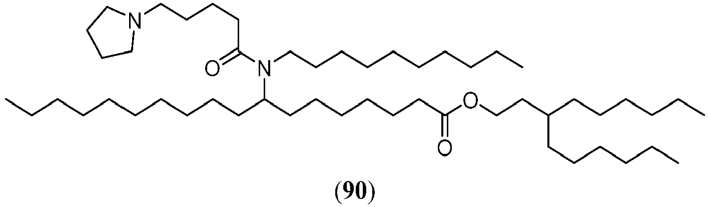 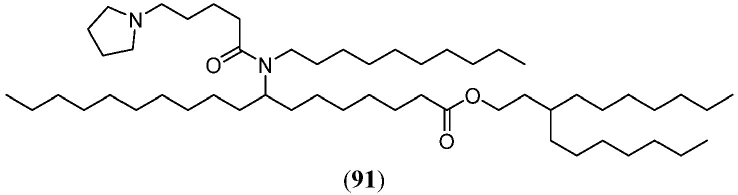 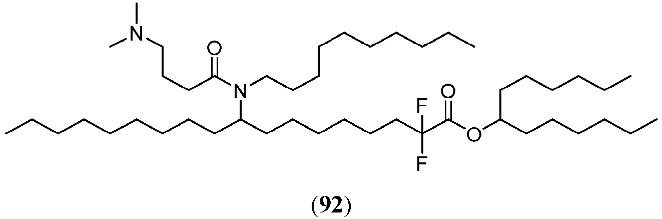 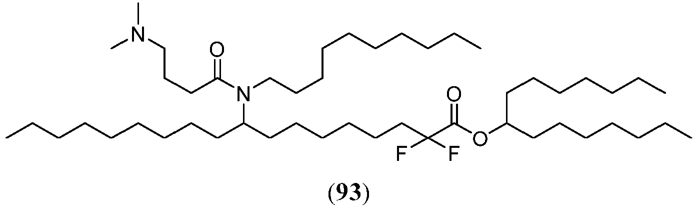 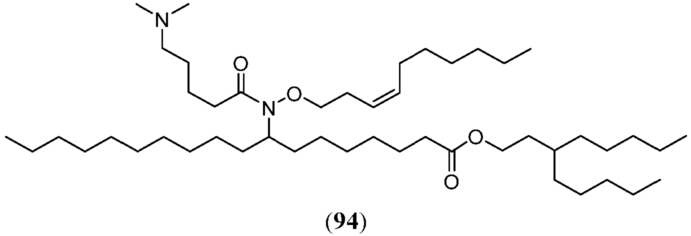 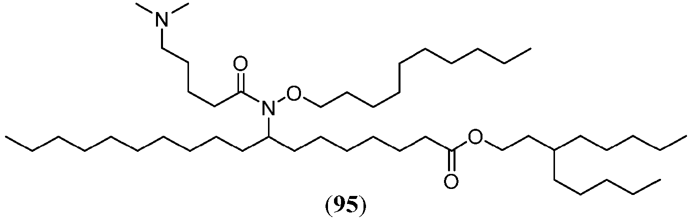 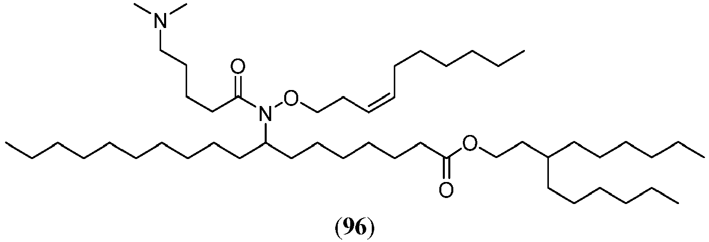 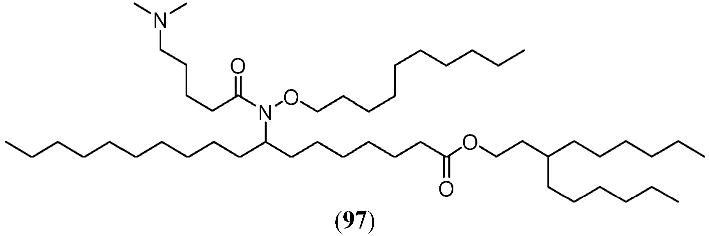 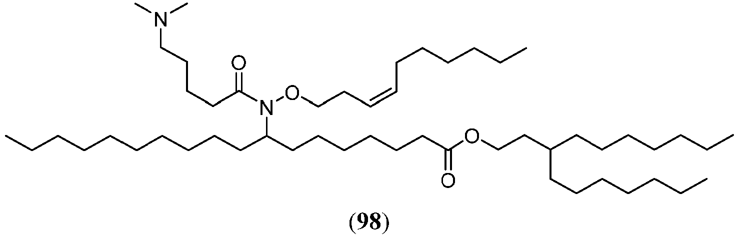 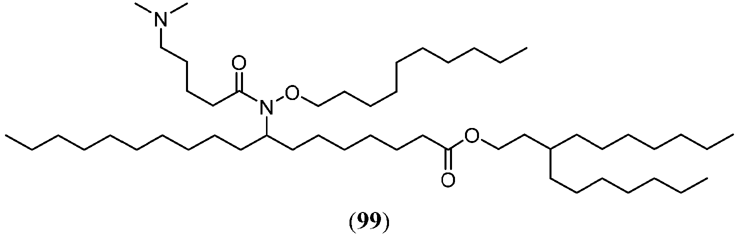 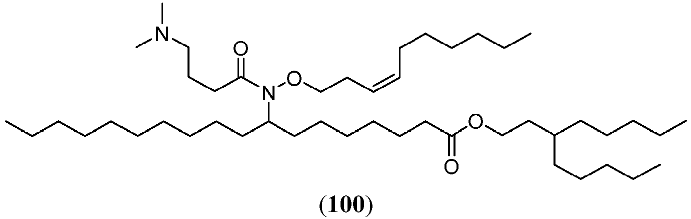 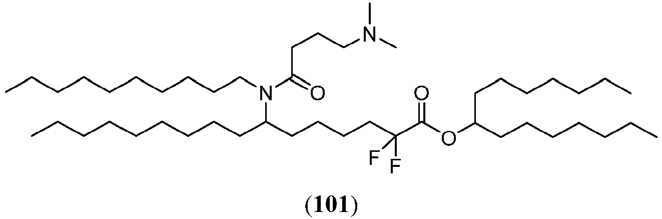 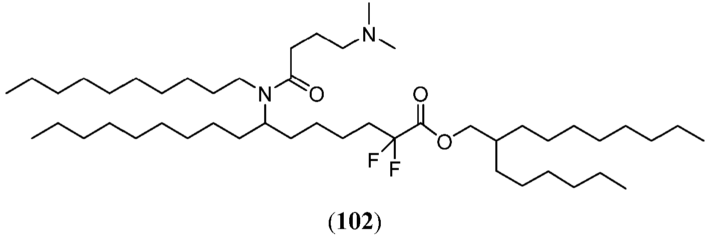 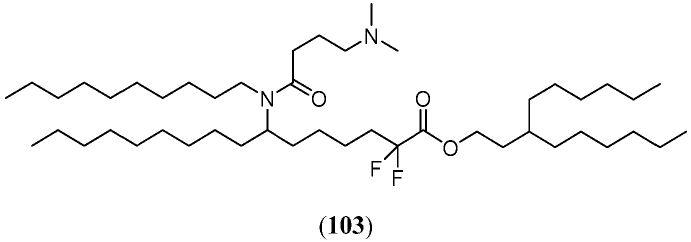 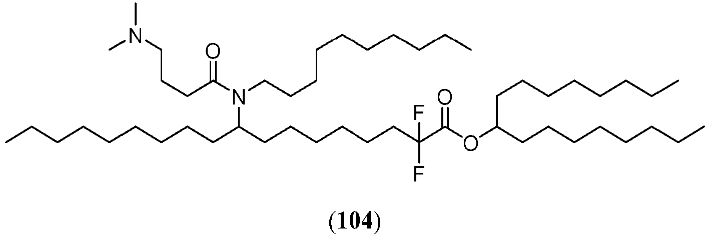 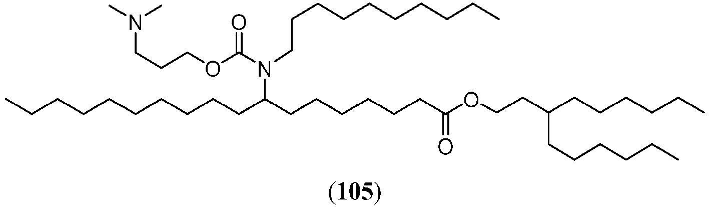 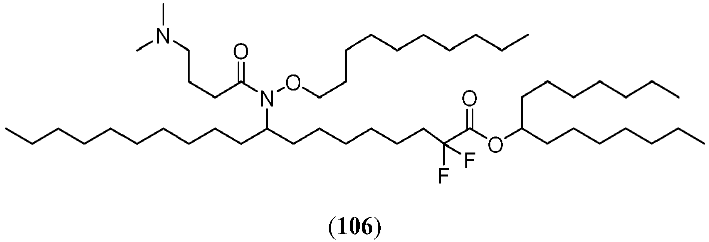 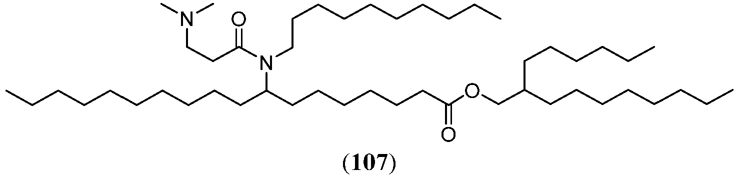 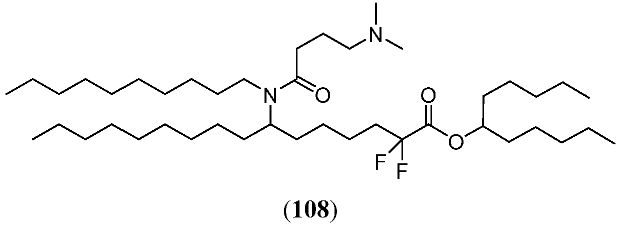 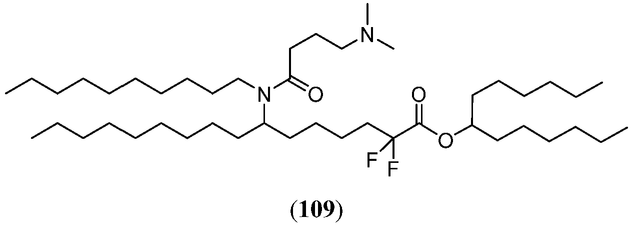 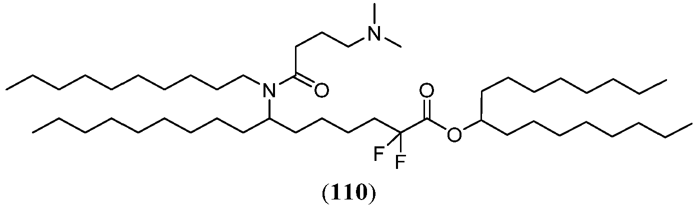 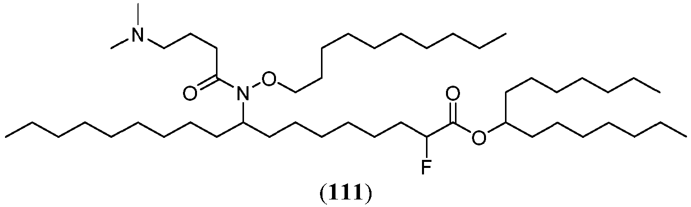 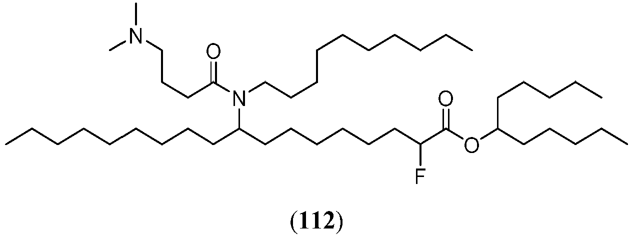 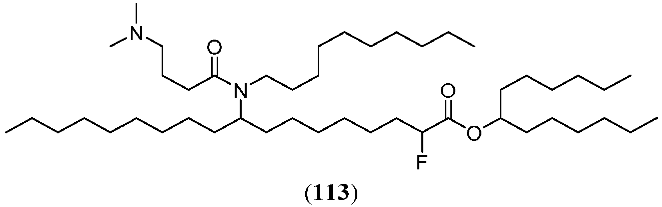 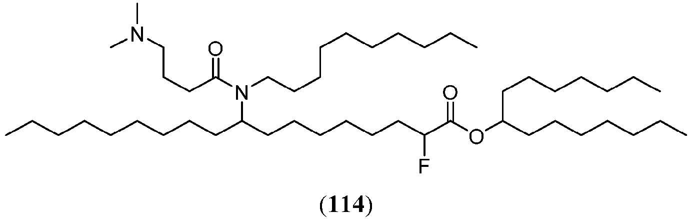 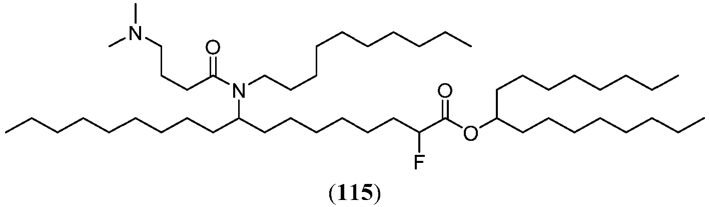 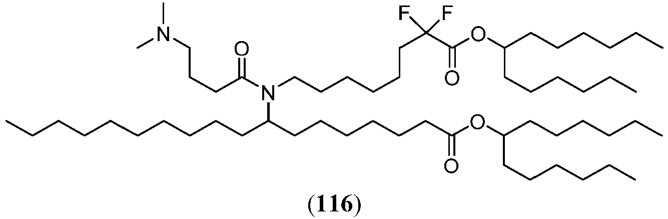 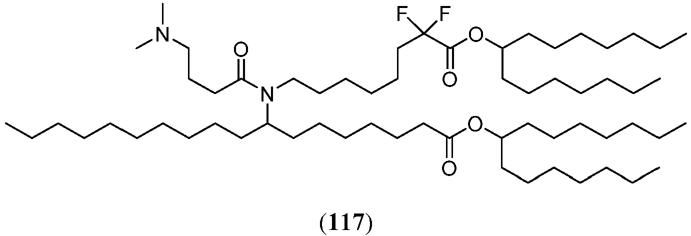 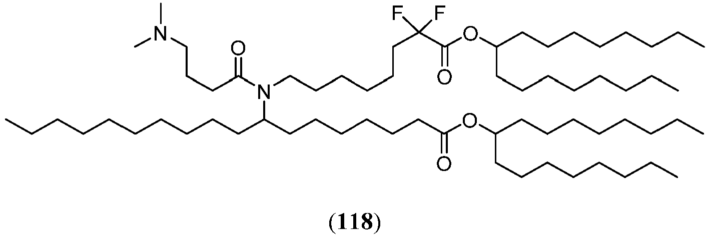 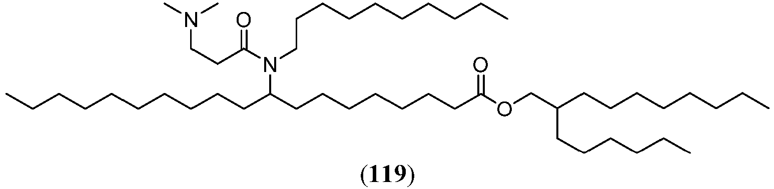 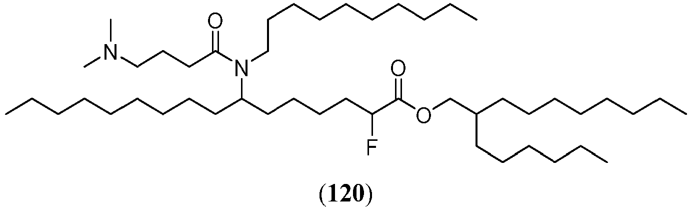 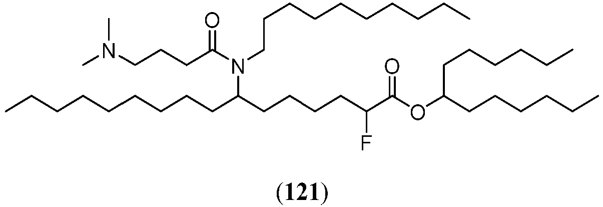 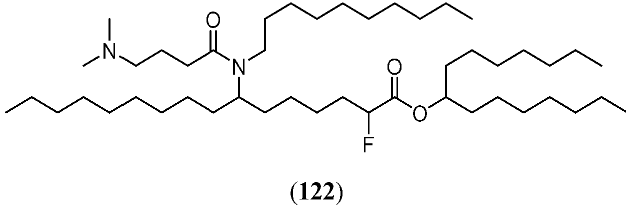 and 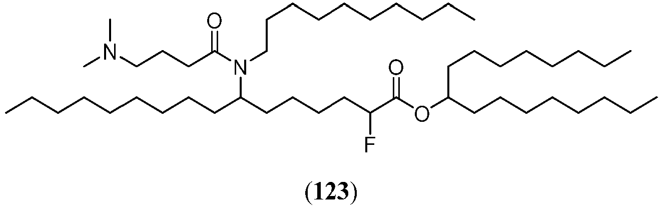 or a salt thereof.

9. The compound or salt of claim 1, wherein:
(i) the compound or salt is: 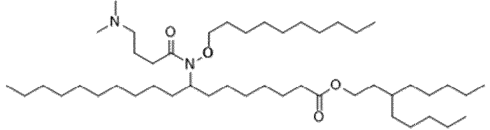 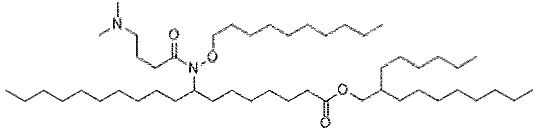 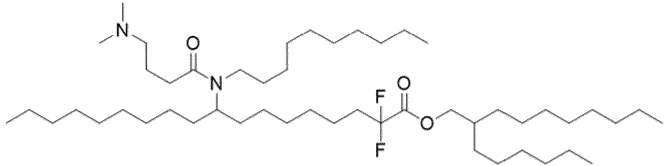 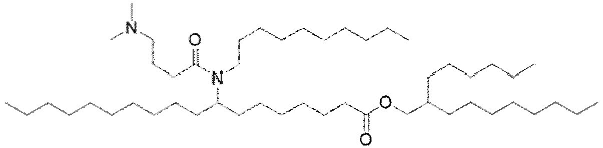 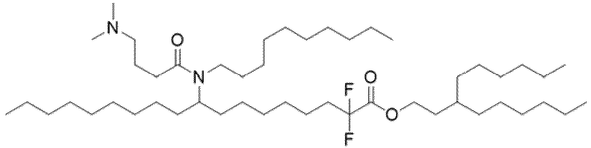 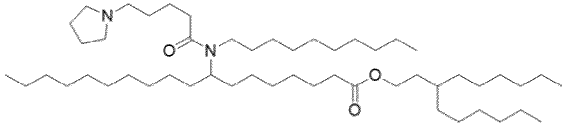 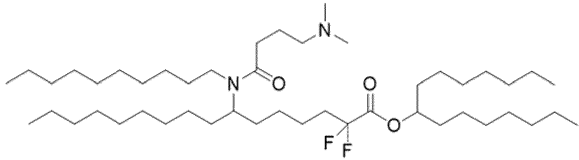 or 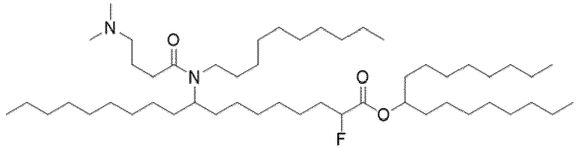 or a salt thereof; or
(ii) 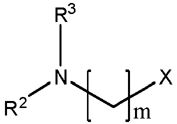 is selected from the group consisting of: (CH₃)₂NCH₂CH₂C(=O)-, (CH₃)₂NCH₂CH₂CH₂C(=O)-, (CH₃)₂NCH₂CH₂CH₂CH₂C(=O)-, (CH₃)₂NCH₂CH₂-, (CH₃)₂NCH₂CH₂CH-, 5-pyrolidin-1-ylpentanoyl, and (CH₃)₂NCH₂CH₂CH₂O-.

10. A nucleic acid-lipid particle comprising:
(a) one or more nucleic acid molecules;
(b) a non-cationic lipid;
(c) a conjugated lipid; and
(d) a compound or salt as described in any one of claims 1-9,
wherein the one or more nucleic acid molecules are encapsulated within the lipid particle.

11. The nucleic acid-lipid particle of claim 10, wherein:
(i)(a) the compound or salt comprises from 30 mol % to 85 mol % of the total lipid present in the particle; the non-cationic lipid comprises from 13 mol % to 49.5 mol % of the total lipid present in the particle; and the conjugated lipid comprises from 0.1 mol % to 10 mol % of the total lipid present in the particle, or (b) the compound or salt comprises from 30 mol % to 85 mol % of the total lipid present in the particle; the non-cationic lipid comprises from 13 mol % to 49.5 mol % of the total lipid present in the particle; and the conjugated lipid comprises from 0.1 mol % to 10 mol % of the total lipid present in the particle; and/or
(ii) the nucleic acid is selected from the group consisting of small interfering RNA (siRNA), Dicer-substrate dsRNA, small hairpin RNA (shRNA), asymmetrical interfering RNA (aiRNA), microRNA (miRNA), mRNA, tRNA, rRNA, viral RNA (vRNA), self-amplifying RNA, and combinations thereof; or the nucleic acid is an mRNA molecule; or the nucleic acid comprises a double stranded siRNA molecule, optionally wherein the double stranded siRNA molecule comprises at least one modified nucleotide or the siRNA comprises at least one 2'-O-methyl (2'OMe) nucleotide; and/or
(iii) the non-cationic lipid comprises: (a) cholesterol or a derivative thereof, optionally wherein the cholesterol or derivative thereof comprises from 31.5 mol % to 42.5 mol % of the total lipid present in the particle; (b) a phospholipid; or (c) a mixture of a phospholipid and cholesterol or a derivative thereof, optionally wherein the phospholipid comprises dipalmitoylphosphatidylcholine (DPPC), distearoylphosphatidylcholine (DSPC), or a mixture thereof, the phospholipid comprises from 4 mol % to 10 mol % of the total lipid present in the particle and the cholesterol comprises from 30 mol % to 40 mol % of the total lipid present in the particle, or the phospholipid comprises from 10 mol % to 30 mol % of the total lipid present in the particle and the cholesterol comprises from 10 mol % to 30 mol % of the total lipid present in the particle; and/or
(iv) the conjugated lipid comprises a polyethyleneglycol (PEG)-lipid conjugate, optionally wherein: (a) the PEG-lipid conjugate comprises a PEG-diacylglycerol (PEG-DAG) conjugate, a PEG-dialkyloxypropyl (PEG-DAA) conjugate, or a mixture thereof, optionally wherein the PEG-DAA conjugate comprises a PEG-dimyristyloxypropyl (PEG-DMA) conjugate, a PEG-distearyloxypropyl (PEG-DSA) conjugate, or a mixture thereof, or a PEG-dimyristyloxypropyl (PEG-DMA) conjugate; (b) the polyethyleneglycol (PEG)-lipid conjugate is a compound of formula:
A-B-C
or a salt thereof, wherein:
A is (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl , (C₁-C₆)alkylthio , or (C₂-C₆)alkanoyloxy, wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is substituted with one or more anionic precursor groups, and wherein any (C₁-C₆)alkyl, (C₃-C₈)cycloalkyl, (C₃-C₈)cycloalkyl(C₁-C₆)alkyl, (C₁-C₆)alkoxy, (C₂-C₆)alkenyl, (C₂-C₆)alkynyl, (C₁-C₆)alkanoyl, (C₁-C₆)alkoxycarbonyl, (C₁-C₆)alkylthio, and (C₂-C₆)alkanoyloxy is optionally substituted with one or more groups independently selected from the group consisting of halo, hydroxyl, (C₁-C₃)alkoxy, (C₁-C₆)alkanoyl, (C₁-C₃)alkoxycarbonyl , (C₁-C₃)alkylthio , or (C₂-C₃)alkanoyloxy;
B is a polyethylene glycol chain having a molecular weight of from 550 daltons to 10,000 daltons;
C is -L-R^{a}
L is selected from the group consisting of a direct bond, -C(O)O-, -C(O)NR^{b}-, -NR^{b}-, - C(O)-, -NR^{b}C(O)O-, -NR^{b}C(O)NR^{b}-, -S-S-, -O-, -(O)CCH₂CH₂C(O)-,
and -NHC(O)CH₂CH₂C(O)NH-;
R^{a} is a branched (C₁₀-C₅₀)alkyl or branched (C₁₀-C₅₀)alkenyl wherein one or more carbon atoms of the branched (C₁₀-C₅₀)alkyl or branched (C₁₀-C₅₀)alkenyl have been replaced with -O-; and
each R^{b} is independently H or (C₁-C₆)alkyl; or (c) the PEG has an average molecular weight of 2,000 daltons; and/or
(v) the conjugated lipid comprises a PEG-lipid conjugate selected from: 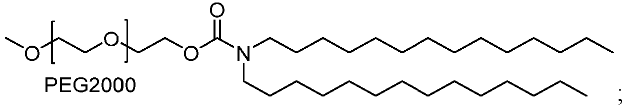 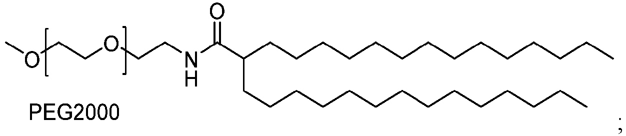 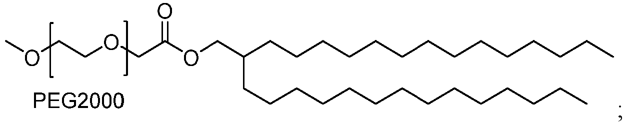 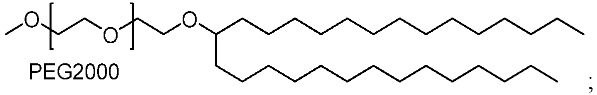 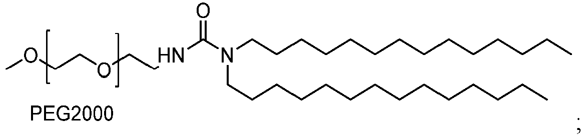 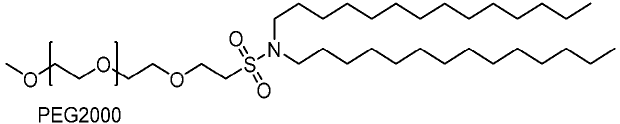 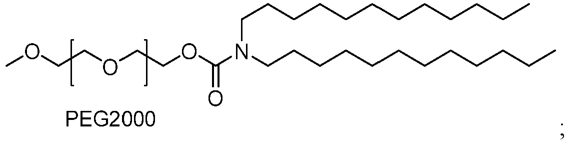 and 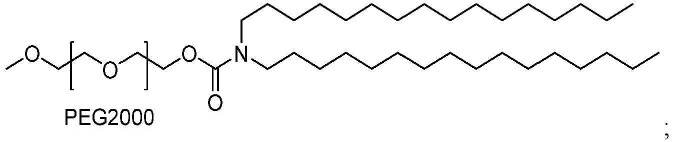 or a salt thereof; and/or
(vii) the conjugated lipid comprises a compound of the following formula: 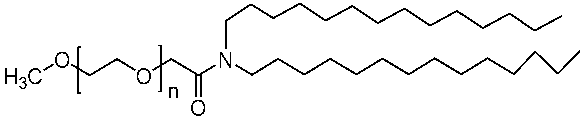 wherein n is 45-50; and/or
(viii) the conjugated lipid comprises from 1 mol % to 2 mol % of the total lipid present in the particle; and/or
(ix) the nucleic acid in the nucleic acid-lipid particle is not substantially degraded after incubation of the particle in serum at 37°C for 30 minutes and/or
(x) the nucleic acid is fully encapsulated in the nucleic acid-lipid particle, optionally wherein the nucleic acid-lipid particle has a lipid:nucleic acid mass ratio of from 5 to 15 or from 5 to 30; and/or
(xi) the nucleic acid-lipid particle has a median diameter of from 40 nm to 150 nm.

12. The nucleic acid-lipid particle of claim 10, wherein:
(i) the compound or salt comprises from 56.5 mol % to 66.5 mol % of the total lipid present in the particle; the non-cationic lipid comprises cholesterol or a derivative thereof comprising from 31.5 mol % to 42.5 mol % of the total lipid present in the particle; and a PEG-lipid conjugate comprises from 1 mol % to 2 mol % of the total lipid present in the particle, optionally wherein the nucleic acid-lipid particle comprises 61.5 mol % compound or salt, 36.9% cholesterol or a derivative thereof, and 1.5 mol % PEG-lipid conjugate; or
(ii) the compound or salt comprises from 52 mol % to 62 mol % of the total lipid present in the particle; the non-cationic lipid comprises mixture of a phospholipid and cholesterol or a derivative thereof comprising from 36 mol % to 47 mol % of the total lipid present in the particle; and the PEG-lipid conjugate comprising from 1 mol % to 2 mol % of the total lipid present in the particle, optionally wherein: (a) the nucleic acid-lipid particle comprises 57.1 mol % compound or salt, 7.1 mol % phospholipid, 34.3 mol % cholesterol or a derivative thereof, and 1.4 mol % PEG-lipid conjugate; or (b) the nucleic acid-lipid particle comprises 57.1 mol % compound or salt, 20 mol % phospholipid, 20 mol % cholesterol or a derivative thereof, and 1.4 mol % PEG-lipid conjugate; or
(iii) the compound or salt comprises from 50 mol % to 65 mol % of the total lipid present in the particle; the non-cationic lipid comprises a mixture of a phospholipid and cholesterol or a derivative thereof, wherein the phospholipid comprises from 4 mol % to 10 mol % of the total lipid present in the particle and the cholesterol or derivative thereof comprises from 30 mol % to 40 mol % of the total lipid present in the particle; and the conjugated lipid comprises from 0.5 mol % to 2 mol % of the total lipid present in the particle; or
(iv) the compound or salt comprises from 50 mol % to 65 mol % of the total lipid present in the particle; the non-cationic lipid comprising a mixture of a phospholipid and cholesterol or a derivative thereof, wherein the phospholipid comprises from 3 mol % to 15 mol % of the total lipid present in the particle and the cholesterol or derivative thereof comprises from 30 mol % to 40 mol % of the total lipid present in the particle; and the conjugated lipid comprises from 0.5 mol % to 2 mol % of the total lipid present in the particle; or
(v) the compound or salt comprises from 50 mol % to 65 mol % of the total lipid present in the particle; the non-cationic lipid comprises up to 49.5 mol % of the total lipid present in the particle; the cholesterol or derivative thereof comprises from 30 mol % to 40 mol % of the total lipid present in the particle; and the conjugated lipid comprises from 0.5 mol % to 2 mol % of the total lipid present in the particle; or
(vi) the compound or salt comprises from 50 mol % to 85 mol % of the total lipid present in the particle; the non-cationic lipid comprises from 13 mol % to 49.5 mol % of the total lipid present in the particle; and the conjugated lipid comprises from 0.5 mol % to 2 mol % of the total lipid present in the particle; or
(vii) the compound or salt comprises from 30 mol % to 50 mol % of the total lipid present in the particle; the non-cationic lipid comprises mixture of a phospholipid and cholesterol or a derivative thereof comprising from 47 mol % to 69 mol % of the total lipid present in the particle; and the conjugated lipid comprises from 1 mol % to 3 mol % of the total lipid present in the particle, optionally wherein the lipid to nucleic acid ratio is 24; or
(viii) the non-cationic lipid comprises a mixture of cholesterol and DSPC; the conjugated lipid is: 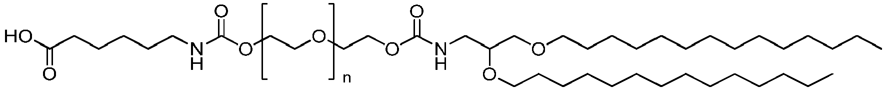 wherein n is selected so that the resulting polymer chain has a molecular weight of 2000 daltons
wherein the conjugated lipid comprises 2 mol % of the total lipid present in the particle; DSPC comprises 10 mol % of the total lipid present in the particle; cholesterol comprises 48 mol % of the total lipid present in the particle; and the compound or salt comprises 40 mol % of the total lipid present in the particle, optionally wherein the lipid to nucleic acid ratio is 24; or
(ix) the non-cationic lipid comprises a mixture of cholesterol and DSPC; the conjugated lipid is: 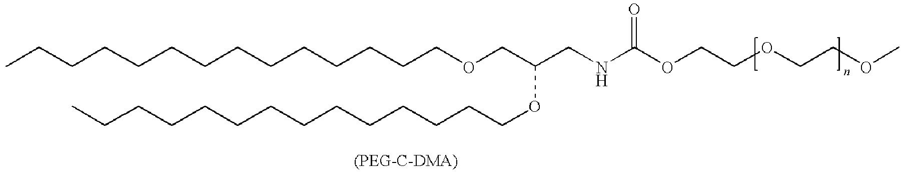 wherein n is selected so that the resulting polymer chain has a molecular weight of 2000 daltons;
wherein the conjugated lipid comprises 2 mol % of the total lipid present in the particle; DSPC comprises 10 mol % of the total lipid present in the particle; cholesterol comprises 48 mol % of the total lipid present in the particle; and the compound or salt comprises 40 mol % of the total lipid present in the particle, optionally wherein the lipid to nucleic acid ratio is 24; or
(x) the non-cationic lipid comprises a mixture of cholesterol and DSPC; the conjugated lipid is: 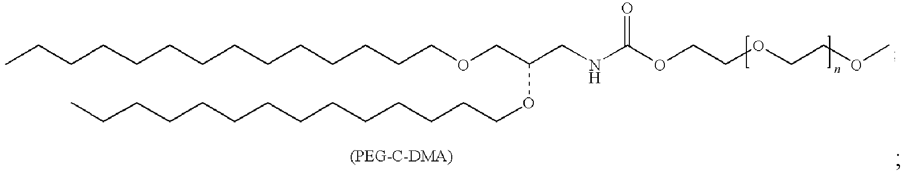 wherein n is selected so that the resulting polymer chain has a molecular weight of 2000;
wherein the conjugated lipid comprises 1.6 mol % of the total lipid present in the particle; DSPC comprises 10.9 mol % of the total lipid present in the particle; cholesterol comprises 32.8 mol % of the total lipid present in the particle; and the compound or salt comprises 54.9 mol % of the total lipid present in the particle, optionally wherein the lipid to nucleic acid ratio is 24; or
(xi) the nucleic acid-lipid particle comprises two or more compounds or salts as described in any one of claims 1-9,

13. A pharmaceutical composition comprising a nucleic acid-lipid particle of any one of claims 10-12, and a pharmaceutically acceptable carrier.

14. A nucleic acid-lipid particle of any one of claims 10-12 or the composition of claim 13, for use in treating a disease or disorder in a mammal.

15. The nucleic acid-lipid particle or the composition for use according to claim 14, wherein the disease or disorder is selected from the group consisting of a viral infection, a liver disease or disorder, and cancer.

## Patentansprüche

1. Verbindung der Formel (I): 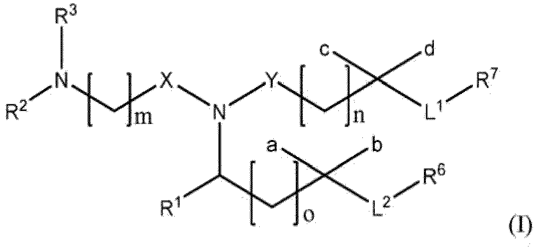 oder ein Salz davon, wobei:
X -CH₂-, -O-, -O(C=O); oder -C(=O)- ist
Y abwesend, -CH₂-, -O-, -SO₂- oder -C(=O)- ist;
R¹ ein C₂-C₂₅-Kohlenwasserstoffrest ist;
R² C₁-C₃-Alkyl ist; und R³ C₁-C₃-Alkyl ist; oder R² und R³ zusammen mit dem Stickstoff, an den sie angelagert sind, einen Aziridino-, Azetidino- oder Pyrrolidinoring ausbilden;
m 0, 1, 2, 3, 4, 5 oder 6 ist;
n 0-15 ist;
o 0-15 ist;
L¹ abwesend, -(C=O)-O-, -(C=O)N(H)N(R⁴)-, -(C=O)N(H)-N=C(R⁴)-, - (C=O)N(H)N(H)C(R⁴)(R⁵)-, -(C=O)-O-CH₂-R^{z}, oder -O(C=O)-R^{w}- ist;
L² abwesend, -(C=O)-O-, -(C=O)N(H)N(R^{4'}), -(C=O)N(H)-N=C(R^{4'}), - (C=O)N(H)N(H)C(R^{4'})(R^{5'}), -(C=O)-O-CH₂-R^{2'}, oder -O(C=O)-R^{x}- ist
a H oder F ist;
b H oder F ist;
c H oder F ist; und d H oder F ist; oder c und d zusammen mit dem Kohlenstoff, an den sie angelagert sind, -C(=O)- ausbilden;
R⁴ H oder C₂-C₂₅-Kohlenwasserstoffrest ist;
R⁵ H oder C₂-C₂₅-Kohlenwasserstoffrest ist;
R^{4'} H oder C₂-C₂₅-Kohlenwasserstoffrest ist;
R^{5'} H oder C₂-C₂₅-Kohlenwasserstoffrest ist
R⁶ C₂-C₂₅-Kohlenwasserstoffrest ist;
R⁷ C₂-C₂₅-Kohlenwasserstoffrest ist;
R^{w} abwesend, -C(F)H- oder -C(F)₂- ist;
R^{x} abwesend, -C(F)H- oder -C(F)₂- ist;
R^{z} Phenyl ist, das mit 1, 2 oder 3 Gruppen substituiert ist, die unabhängig voneinander aus C₂-C₁₀-Kohlenwasserstoffrest ausgewählt sind; und
R^{z'} Phenyl ist, das mit 1, 2 oder 3 Gruppen substituiert ist, die unabhängig voneinander aus C₂-C₁₀-Kohlenwasserstoffrest ausgewählt sind.

2. Verbindung oder Salz nach Anspruch 1, wobei:
(i) R¹ C₅-C₂₅-Kohlenwasserstoffrest, C₅-C₂₅-Alkyl, C₅-C₂₅-Alkenyl, 1-Nonyl oder 1-Decyl ist; und/oder
(ii) X -CH₂-, -C(=O)- oder -O- ist; und/oder
(iii) R² Methyl, Ethyl oder Propyl ist; und/oder
(iv) R³ Methyl, Ethyl oder Propyl ist; und/oder
(v) m 1, 2 oder 3 ist; und/oder
(vi) o 2-15, 10-15, 2-10 oder 2-5 ist; und/oder
(vii) L² -(C=O)-O-, -(C=O)N(H)N(R⁴)(R⁵)-, -(C=O)N(H)-N=C(R⁴)-, - (C=O)N(H)N(H)C(R⁴)(R⁵) oder -(C=O)-O-CH₂-R^{z} ist, und wobei optional L² -(C=O)-O-, - (C=O)N(H)N(R⁴)(R⁵) oder -(C=O)-O-CH₂-R^{z} ist; und/oder
(viii) a H oder F ist; und/oder
(ix) b H oder F ist; und/oder
(x) R⁶ C₂-C₂₅-Kohlenwasserstoffrest, C₅-C₂₅-Kohlenwasserstoffrest, C₂-C₂₅-Alkyl, C₅-C₂₅-Alkyl oder C₂-C₂₅-Alkenyl ist.

3. Verbindung oder Salz nach Anspruch 1 oder 2, wobei:
(i) 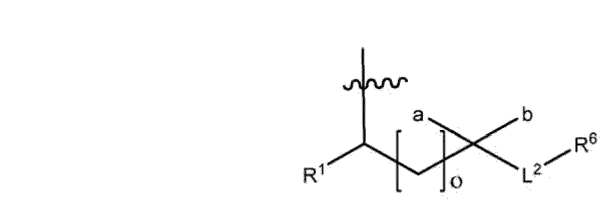 aus der Gruppe ausgewählt ist, bestehend aus: 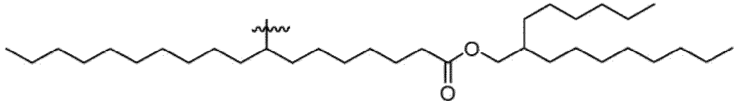 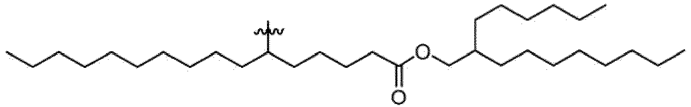 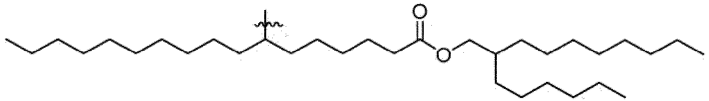 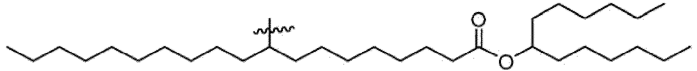 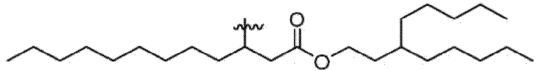 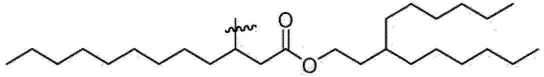 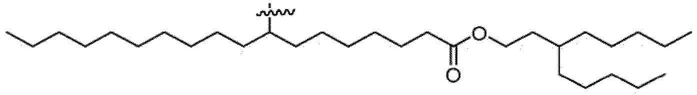 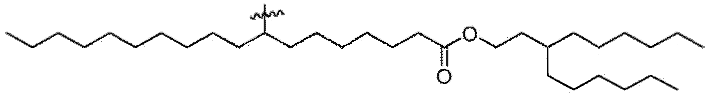 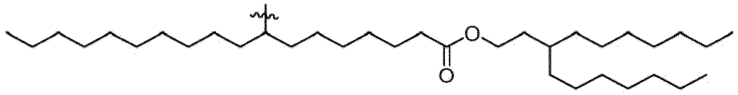 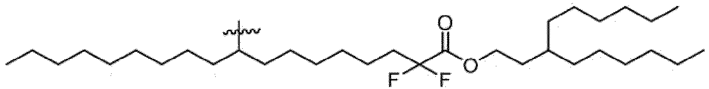 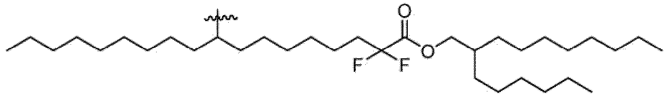 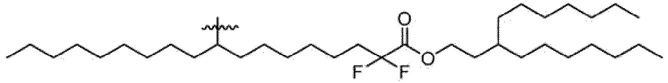 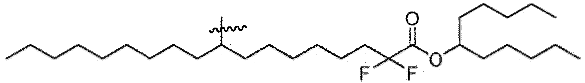 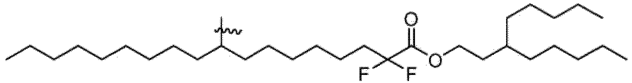 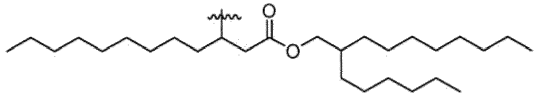 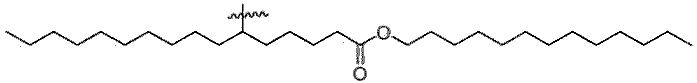 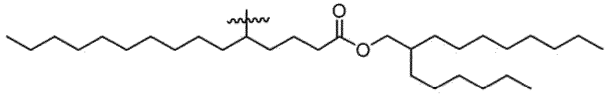 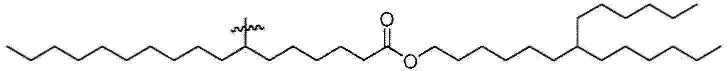 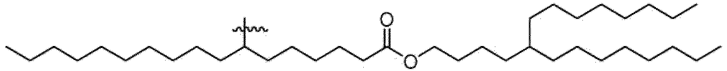 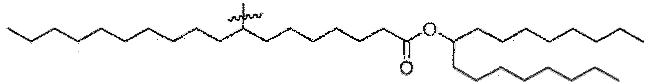 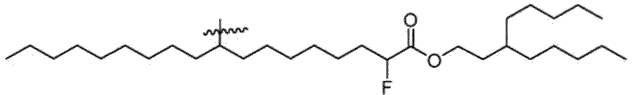 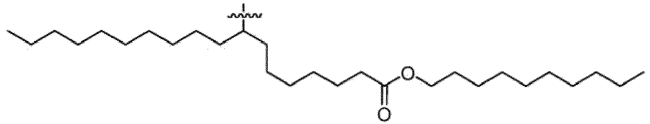 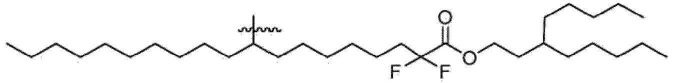 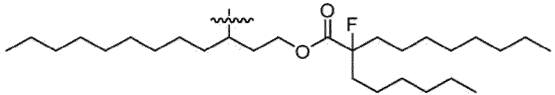 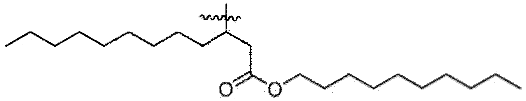 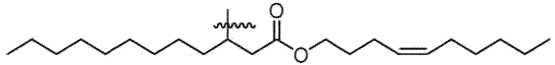 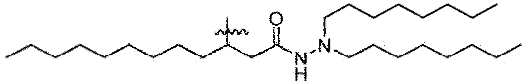 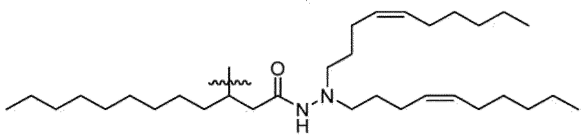 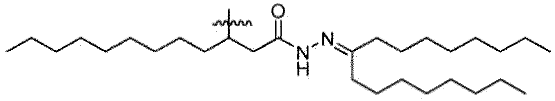 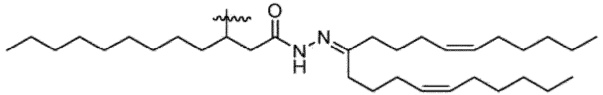 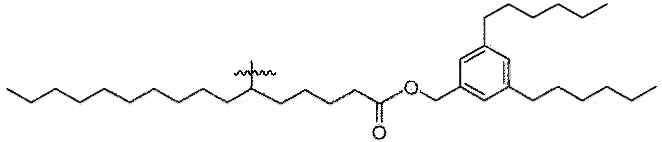 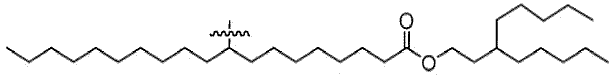 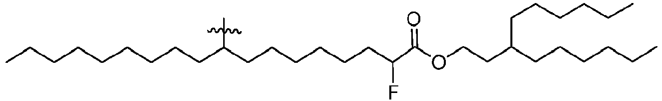 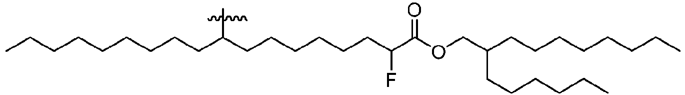 und 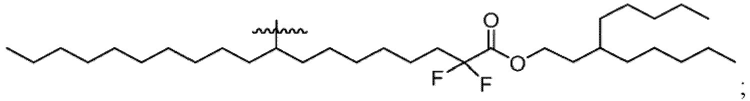 oder
(ii) 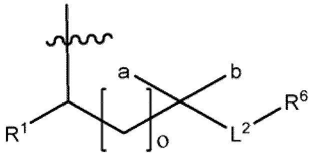 aus der Gruppe ausgewählt ist, bestehend aus: 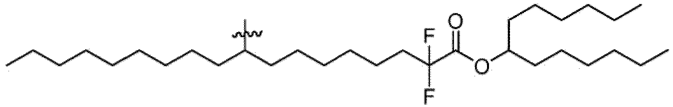 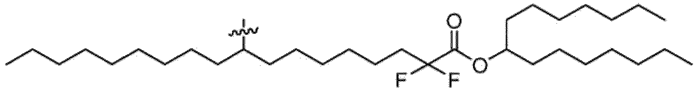 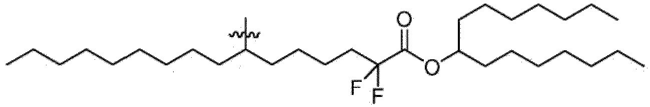 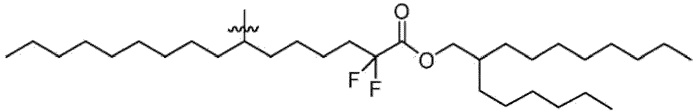 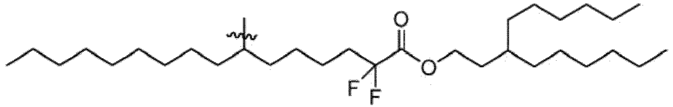 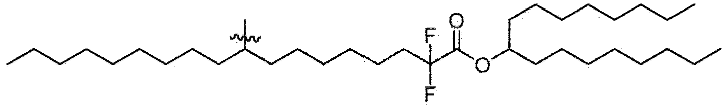 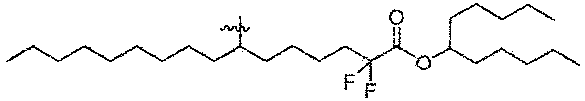 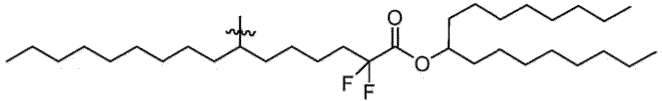 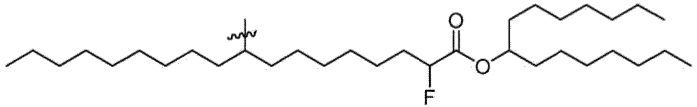 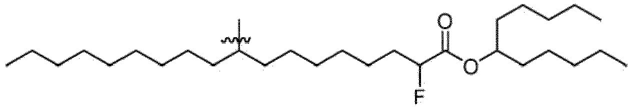 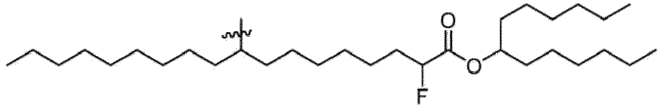 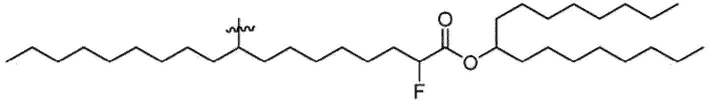 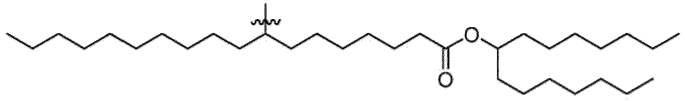 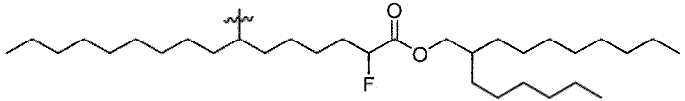 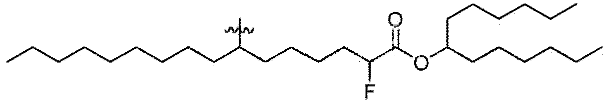 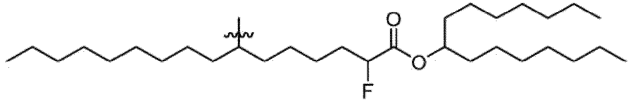 und 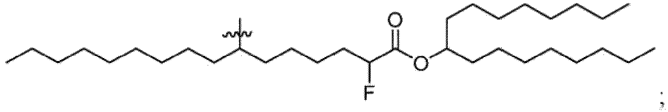 oder
(iii) 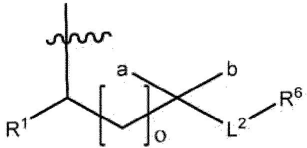 aus der Gruppe ausgewählt ist, bestehend aus: 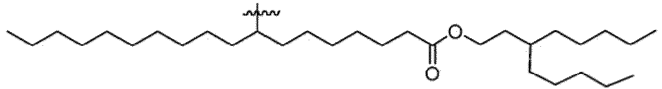 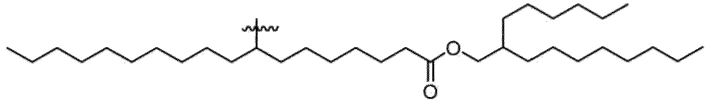 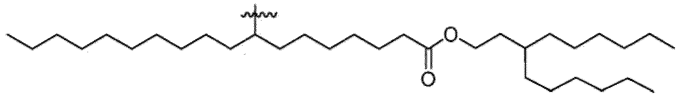 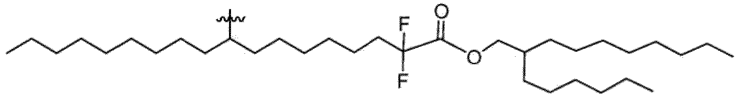 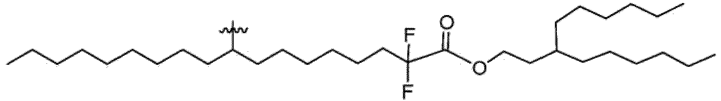 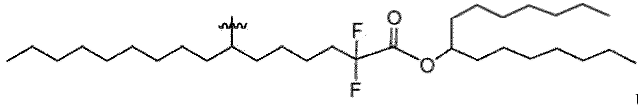
und

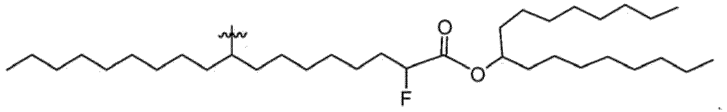

4. Verbindung oder Salz nach einem der Ansprüche 1-3, wobei:
(i) Y -CH₂-, -C(=O)- oder -O- ist; und/oder
(ii) n 2-15, 10-15, 2-10 oder 2-5 ist; und/oder
(iii)(a) L¹ abwesend ist; und L² -(C=O)-O-, -(C=O)N(H)N(R⁴)(R⁵)-, -(C=O)N(H)-N=C(R⁴)-, -(C=O)N(H)N(H)C(R⁴)(R⁵) oder -(C=O)-O-CH₂-R^{z} ist, oder (b) L¹ -(C=O)-O-, - (C=O)N(H)N(R⁴)(R⁵)-, -(C=O)N(H)-N=C(R⁴)-, -(C=O)N(H)N(H)C(R⁴)(R⁵) oder -(C=O)-O-CH₂-R^{a} ist; und L² abwesend ist, oder (c) L¹ -(C=O)-O-, -(C=O)N(H)N(R⁴)(R⁵)-, - (C=O)N(H)-N=C(R⁴), -(C=O)N(H)N(H)C(R⁴)(R⁵)- oder -(C=O)-O-CH₂-R^{a} ist oder (d) L¹ - (C=O)-O ist, L¹ -(C=O)N(H)N(R⁴)(R⁵)- ist oder L¹ -(C=O)-O-CH₂-R^{z} ist; und/oder
(iv) c H oder F ist; und/oder
(v) d H oder F ist; und/oder
(vi) R⁷ C₂-C₂₅-Kohlenwasserstoffrest, C₅-C₂₅-Kohlenwasserstoffrest, C₂-C₂₅-Alkyl, C₅-C₂₅-Alkyl oder C₅-C₂₅-Alkenyl ist.

5. Verbindung oder Salz nach einem der Ansprüche 1-3, wobei:
(i) 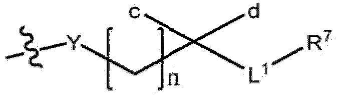 aus der Gruppe ausgewählt ist, bestehend aus: 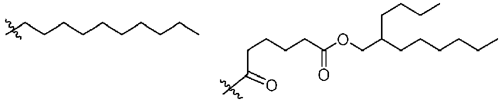 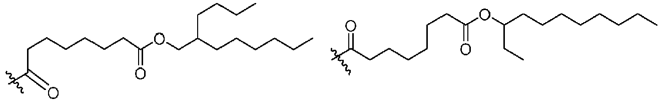 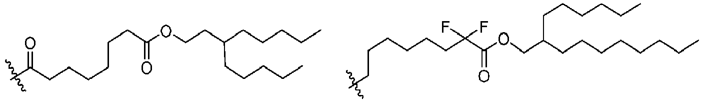 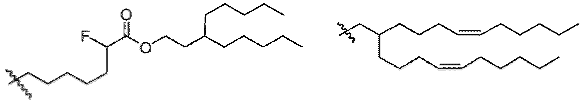 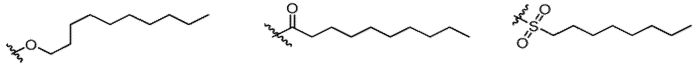 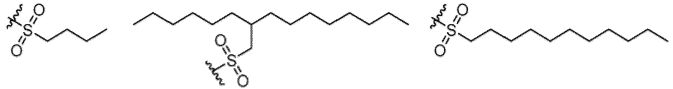 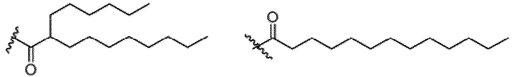 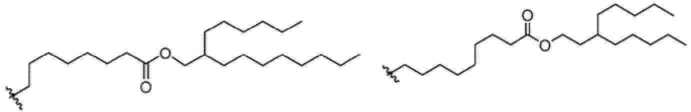 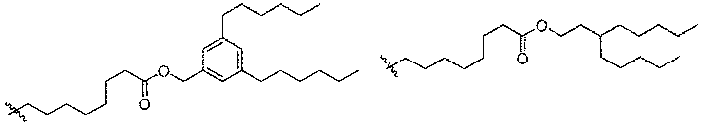 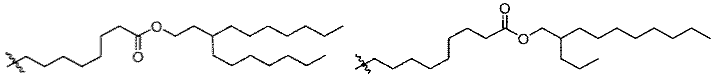 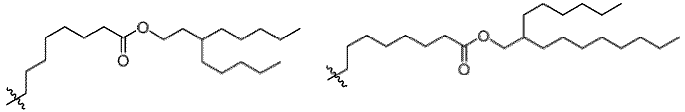 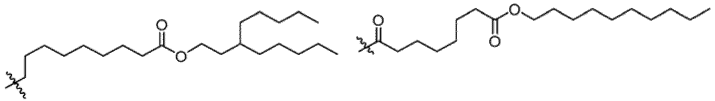 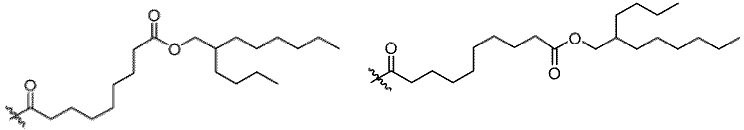 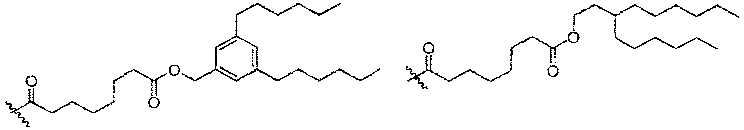 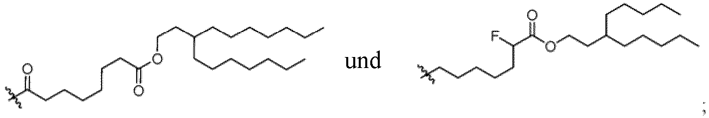 oder
(ii) 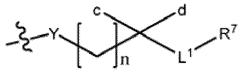 aus der Gruppe ausgewählt ist, bestehend aus: 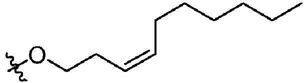 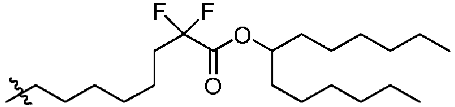 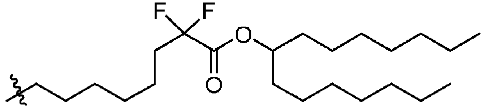 und 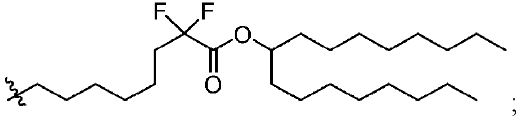 oder
(iii) 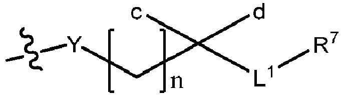 aus der Gruppe ausgewählt ist, bestehend aus:

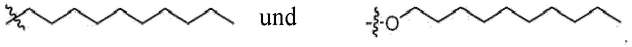

6. Verbindung oder Salz nach einem der Ansprüche 1-4, wobei:
(i) R⁴ C₂-C₂₅-Kohlenwasserstoffrest, C₅-C₂₅-Kohlenwasserstoffrest, C₂-C₂₅-Alkyl, C₅-C₂₅-Alkyl oder C₅-C₂₅-Alkenyl ist; oder
(ii) R*⁵* C₂-C₂₅-Kohlenwasserstoffrest, C₅-C₂₅-Kohlenwasserstoffrest, C₂-C₂₅-Alkyl, C₅-C₂₅-Alkyl oder C₅-C₂₅-Alkenyl ist; oder
(iii) R² Phenyl ist, das mit 1 oder 2 Gruppen substituiert ist, die unabhängig voneinander aus C₂-C₁₀-Kohlenwasserstoffrest ausgewählt sind, wobei optional jeder C₂-C₁₀-Kohlenwasserstoffrest unabhängig aus der Gruppe ausgewählt ist, bestehend aus C₅-C₁₀-Kohlenwasserstoffrest, C₂-C₁₀-Alkyl, C₅-C₁₀-Alkyl und C₅-C₁₀-Alkenyl.

7. Verbindung oder Salz nach Anspruch 1 oder 2, wobei:
(i) R^{4'} C₂-C₂₅-Kohlenwasserstoffrest, C₅-C₂₅-Kohlenwasserstoffrest, C₂-C₂₅-Alkyl, C₅-C₂₅-Alkyl oder C₅-C₂₅-Alkenyl ist; oder
(ii) R^{5'} C₂-C₂₅-Kohlenwasserstoffrest, C₅-C₂₅-Kohlenwasserstoffrest, C₂-C₂₅-Alkyl, C₅-C₂₅-Alkyl oder C₅-C₂₅-Alkenyl ist; oder
(iii) R^{z'} Phenyl ist, das mit 1 oder 2 Gruppen substituiert ist, die unabhängig voneinander aus C₂-C₁₀-Kohlenwasserstoffrest ausgewählt sind, wobei optional jeder C₂-C₁₀-Kohlenwasserstoffrest unabhängig aus der Gruppe ausgewählt ist, bestehend aus C₅-C₁₀-Kohlenwasserstoffrest, C₂-C₁₀-Alkyl, C₅-C₁₀-Alkyl und C₅-C₁₀-Alkenyl.

8. Verbindung oder Salz nach Anspruch 1, ausgewählt aus der Gruppe bestehend aus: 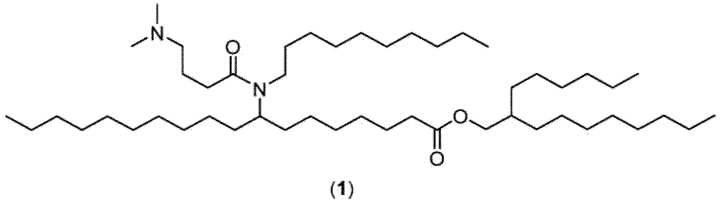 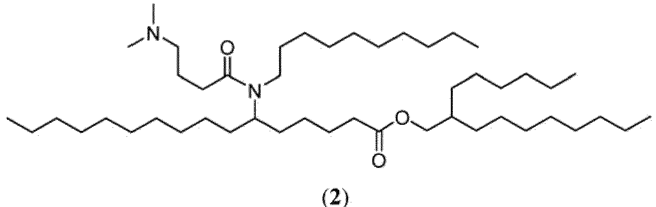 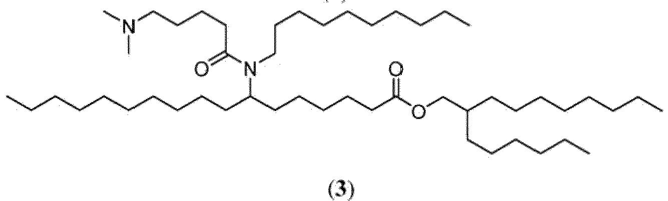 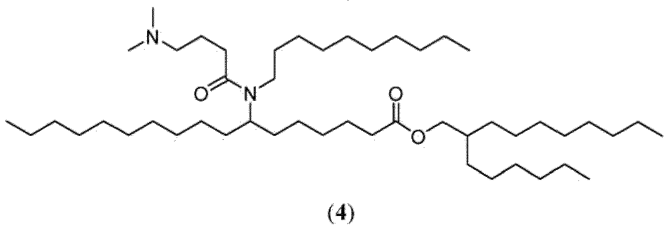 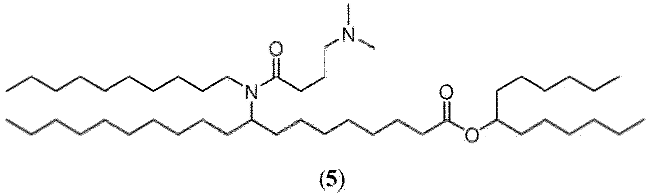 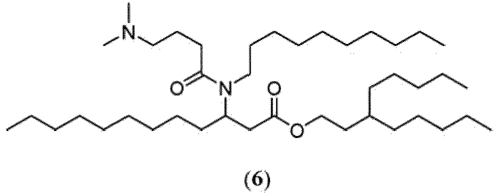 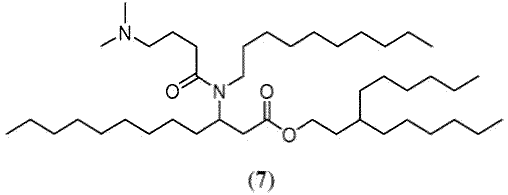 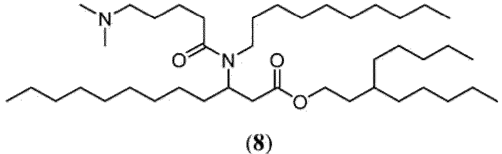 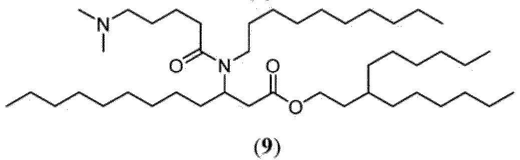 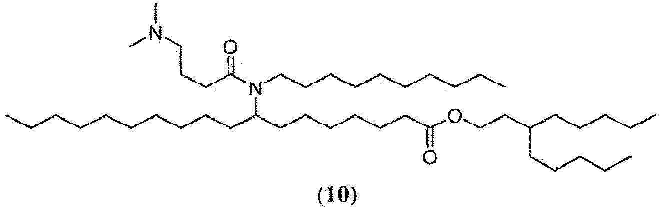 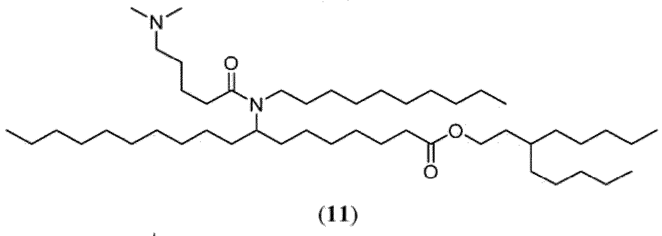 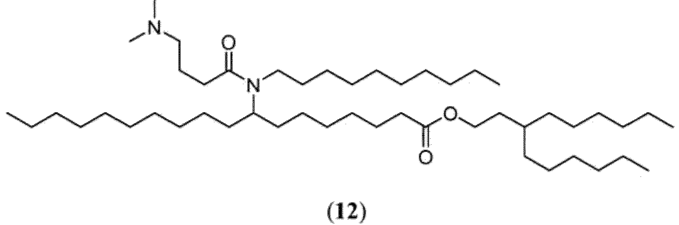 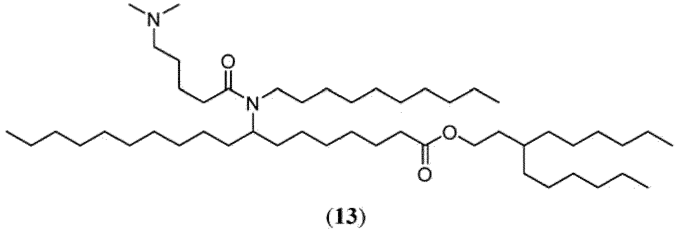 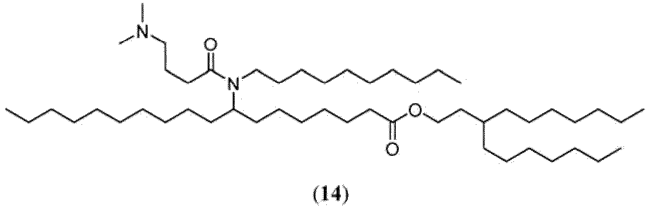 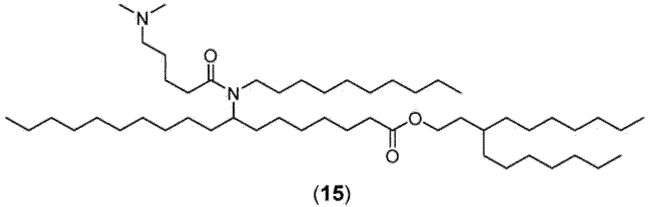 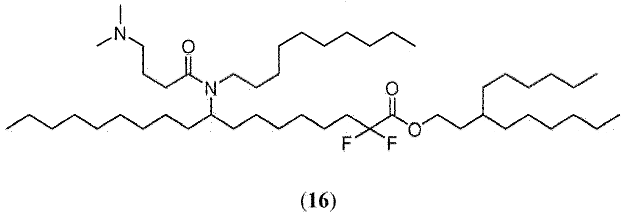 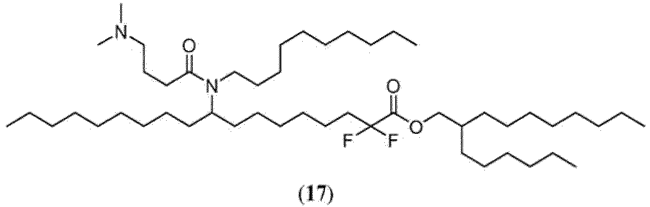 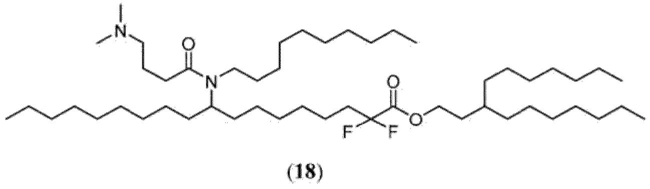 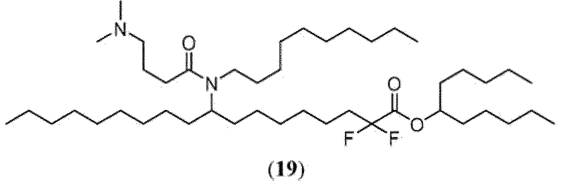 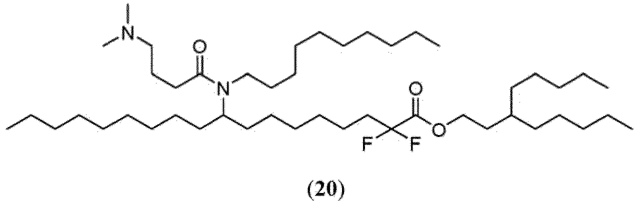 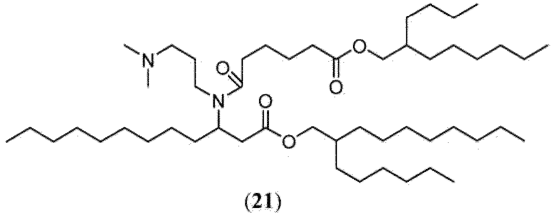 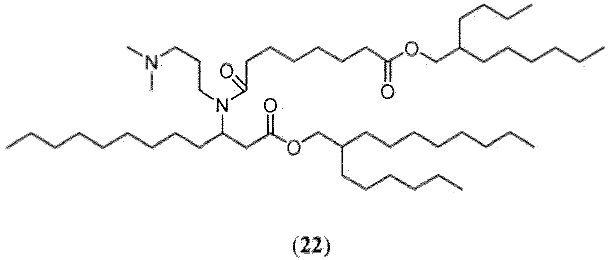 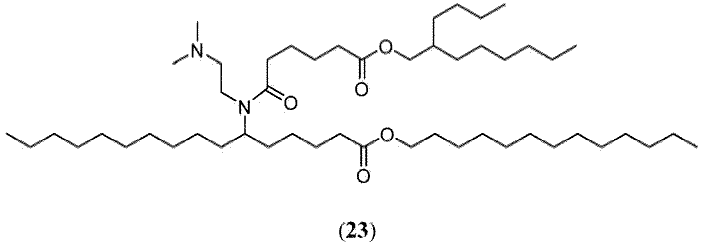 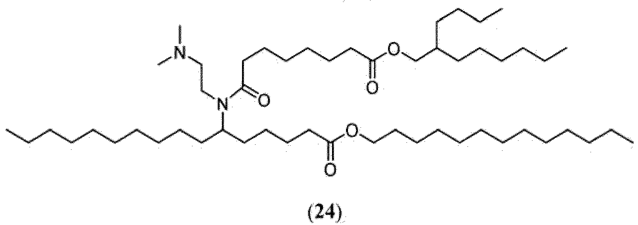 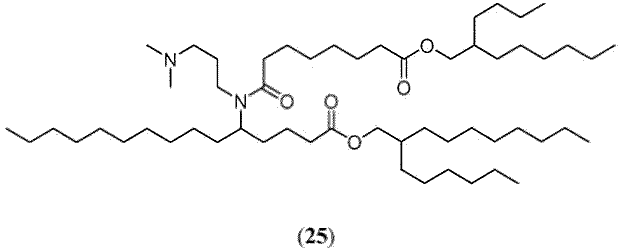 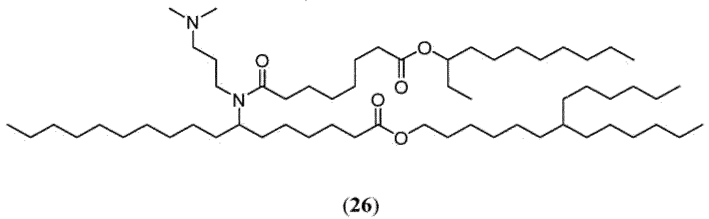 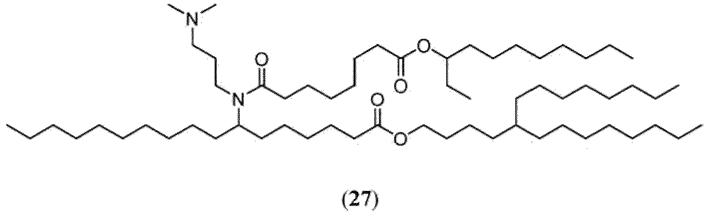 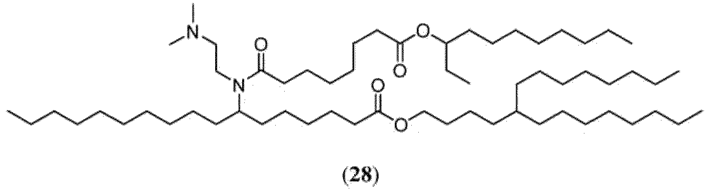 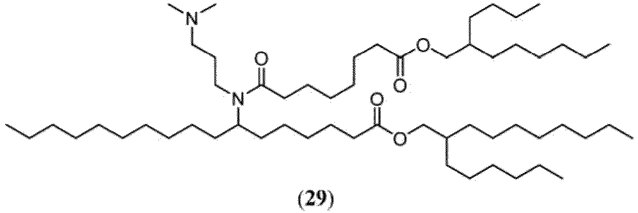 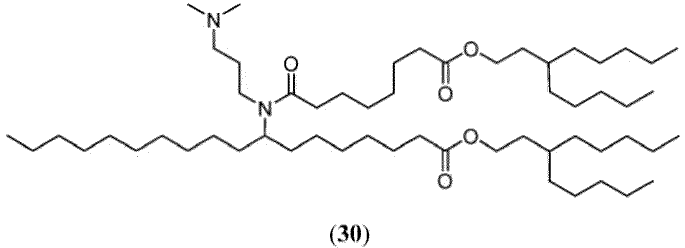 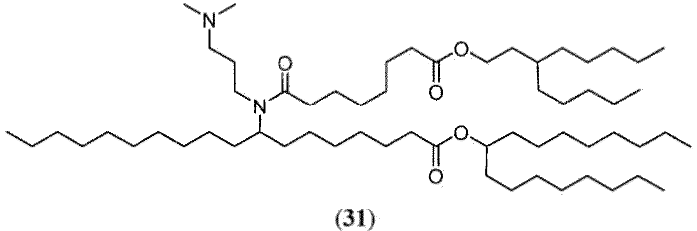 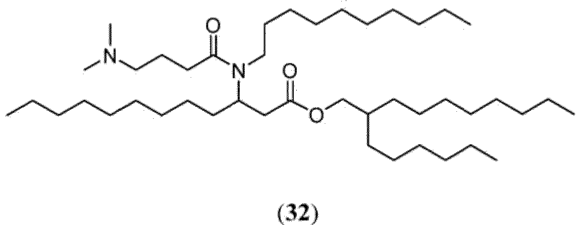 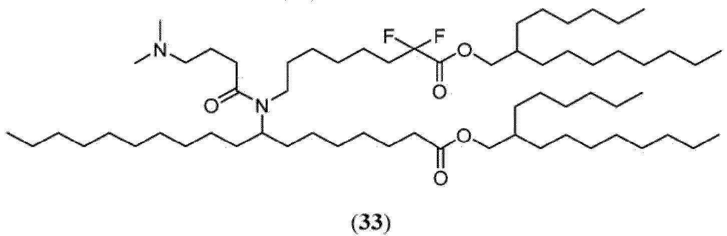 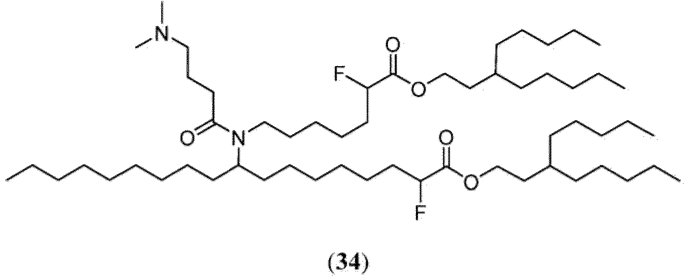 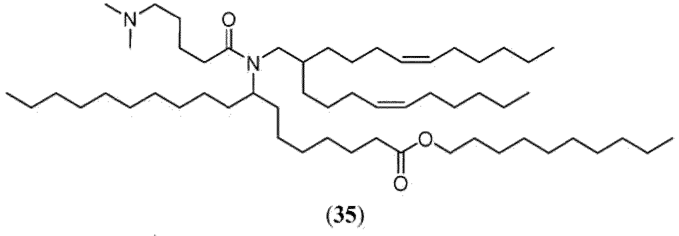 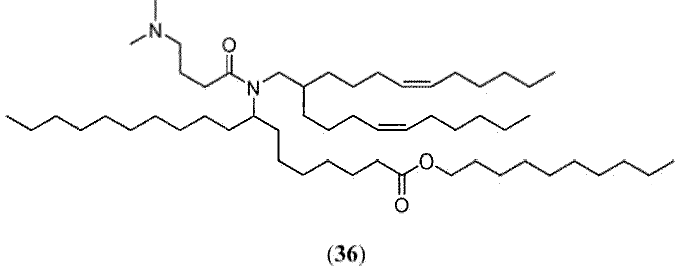 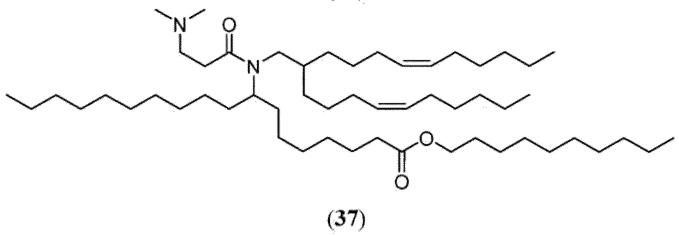 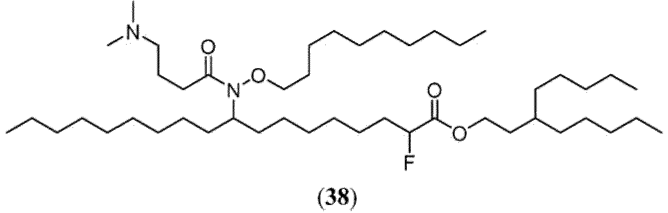 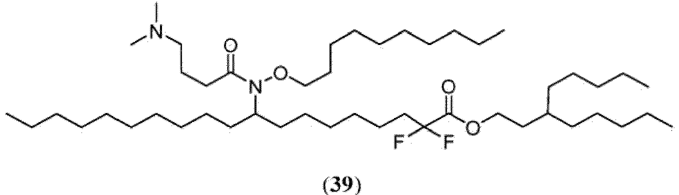 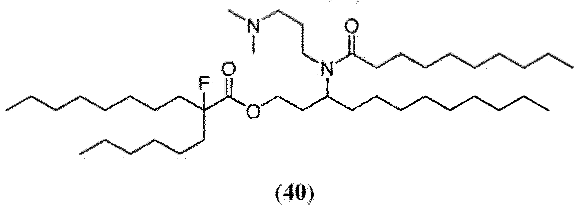 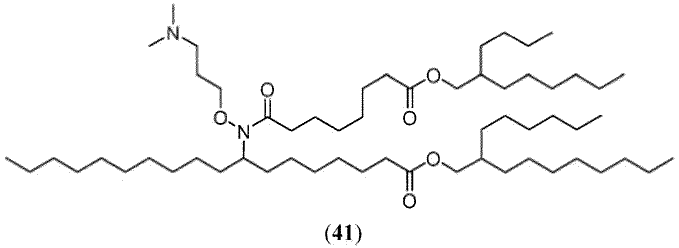 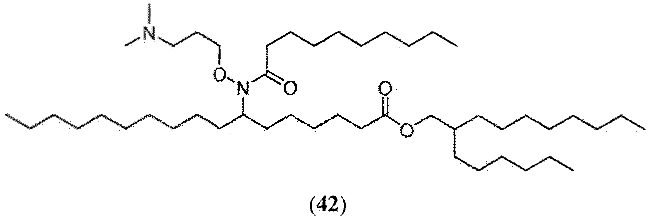 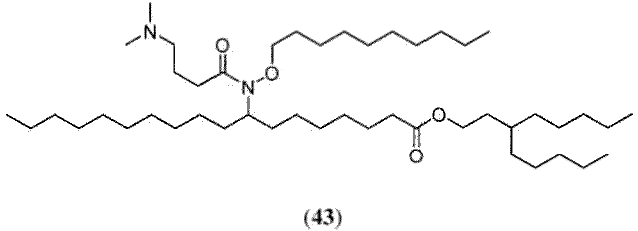 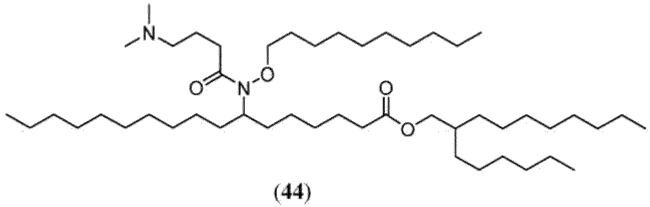 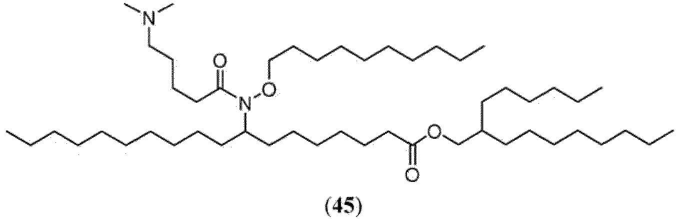 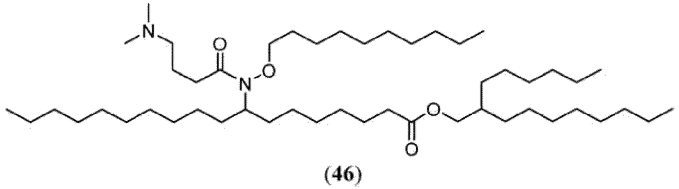 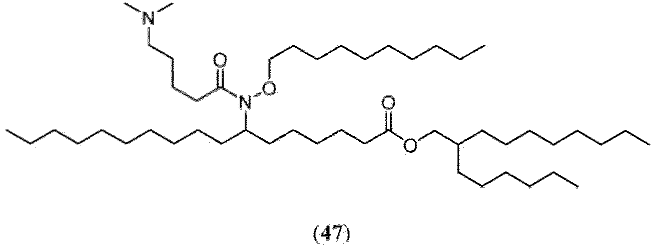 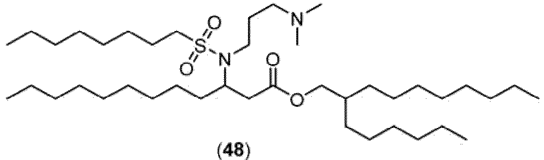 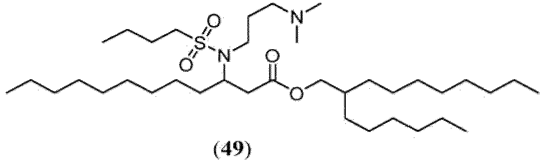 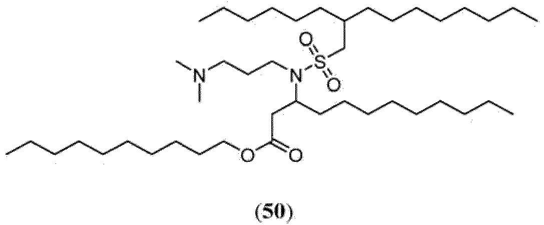 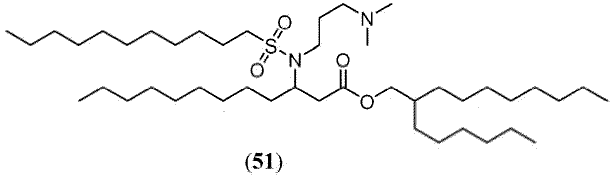 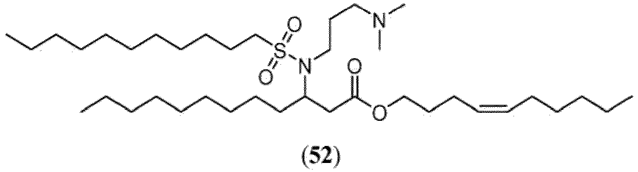 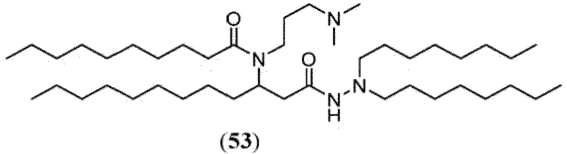 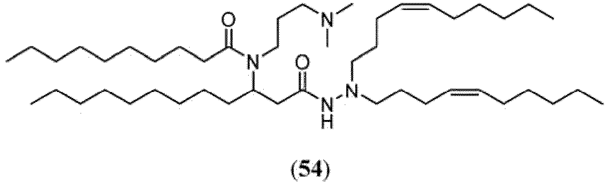 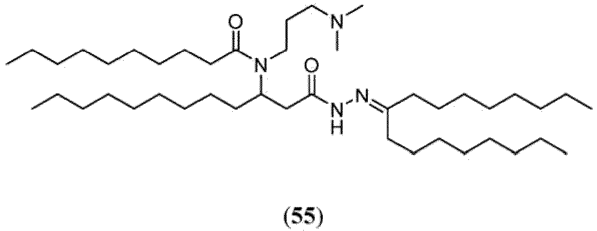 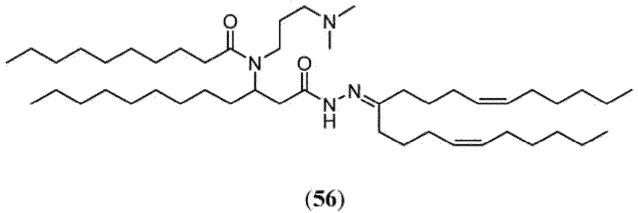 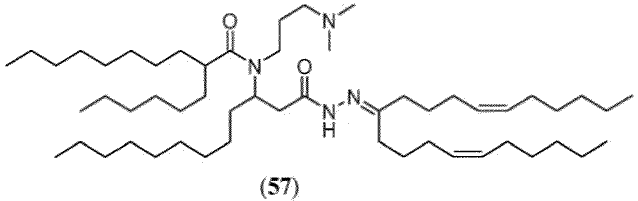 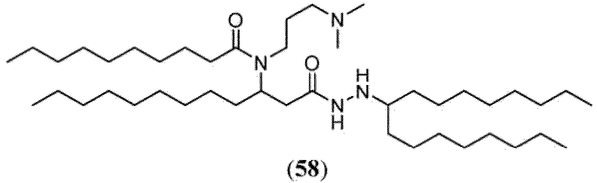 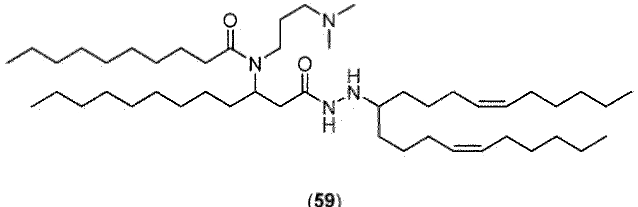 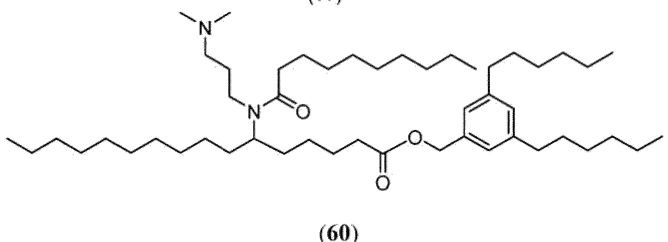 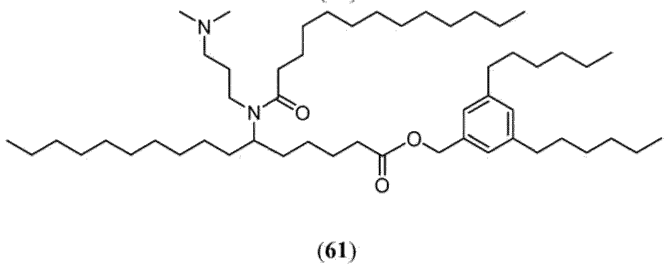 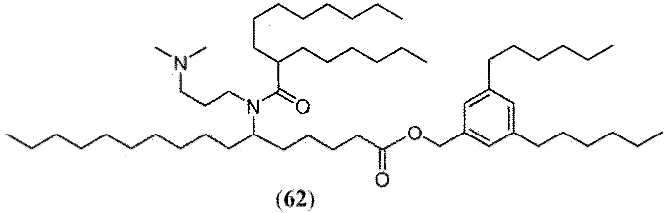 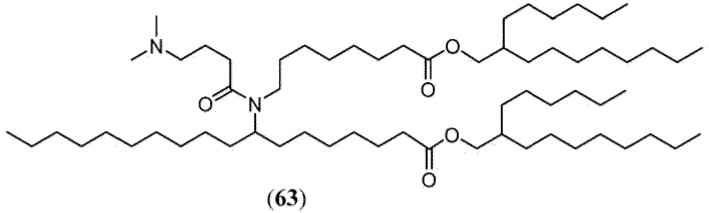 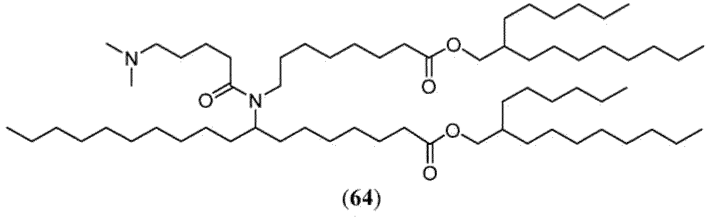 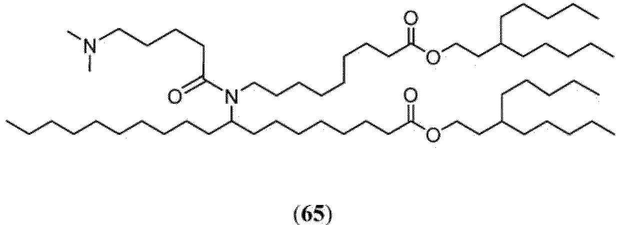 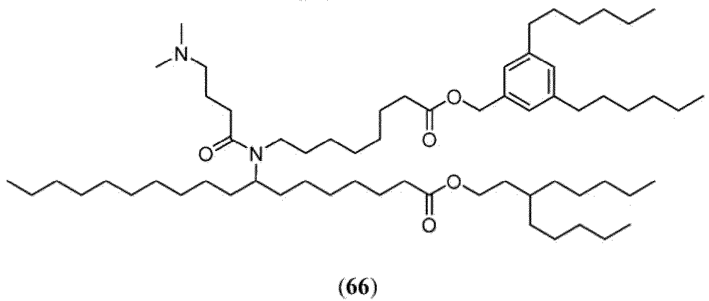 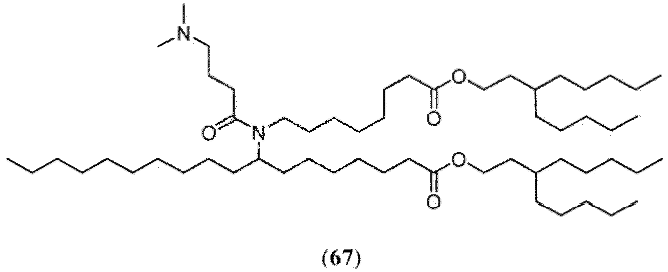 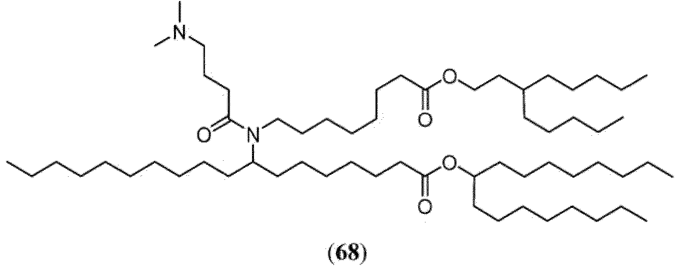 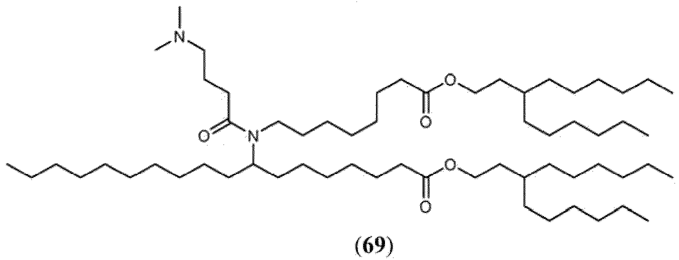 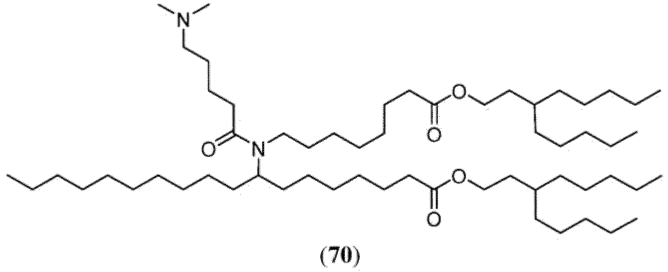 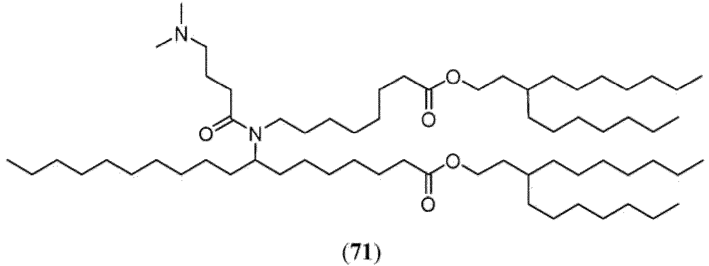 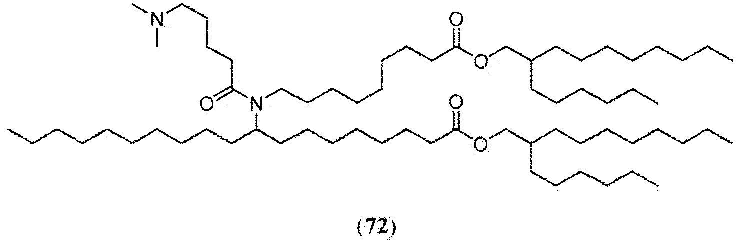 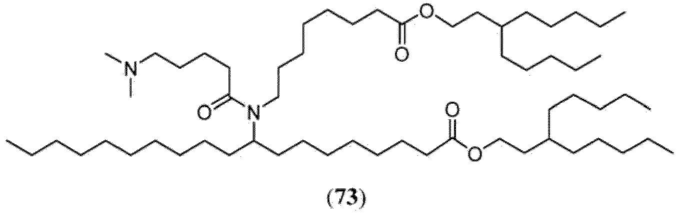 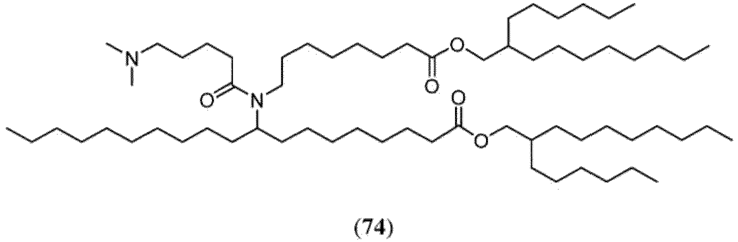 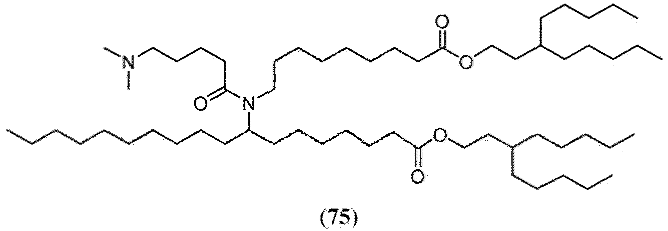 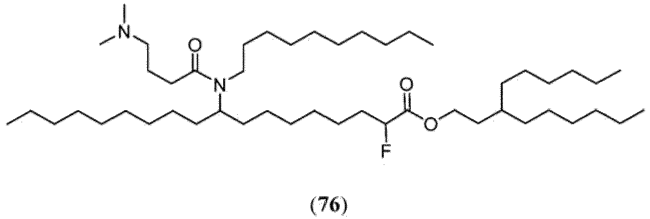 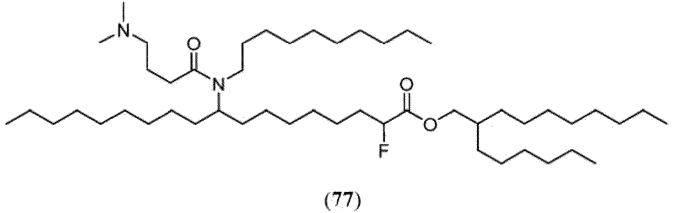 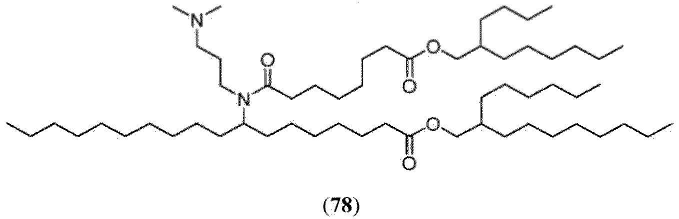 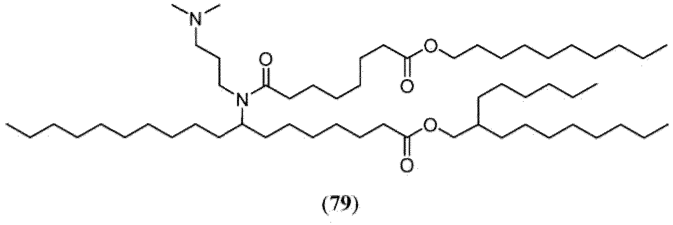 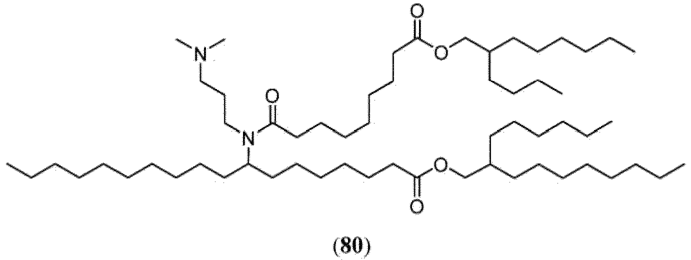 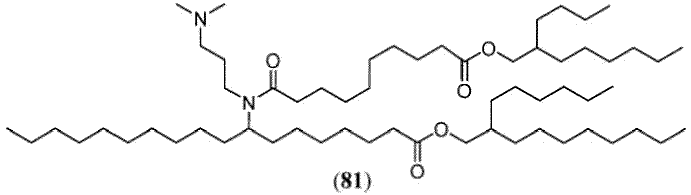 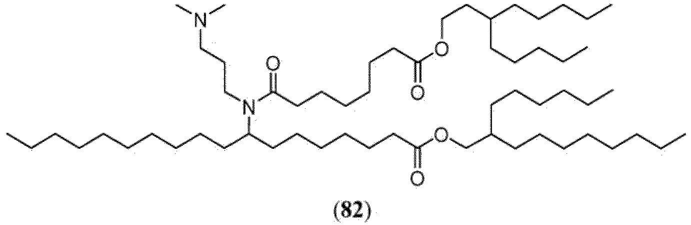 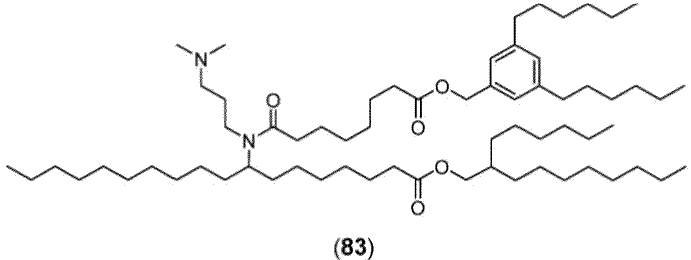 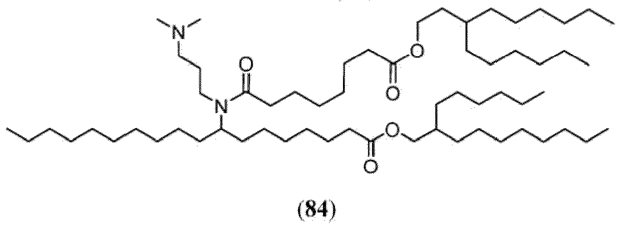 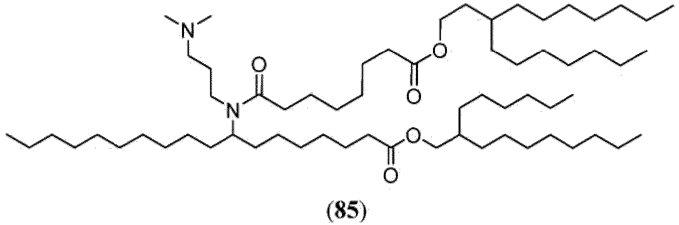 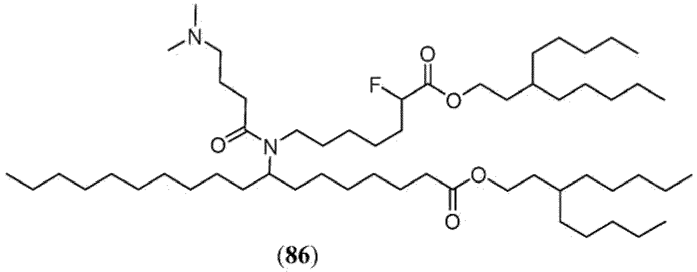 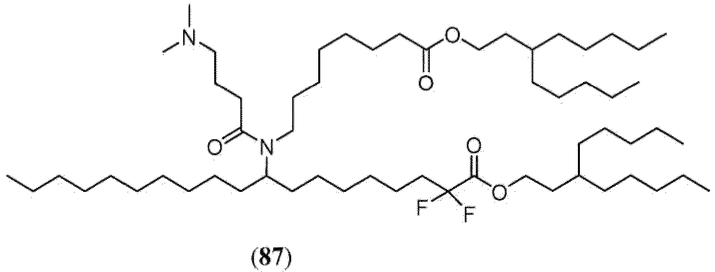 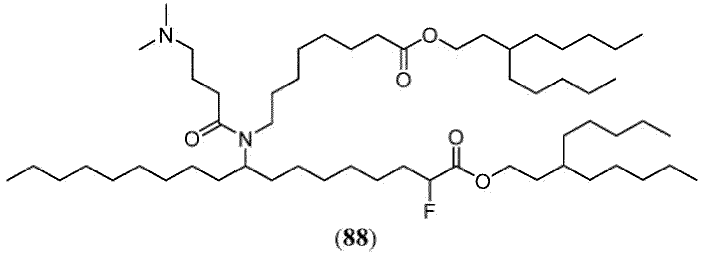 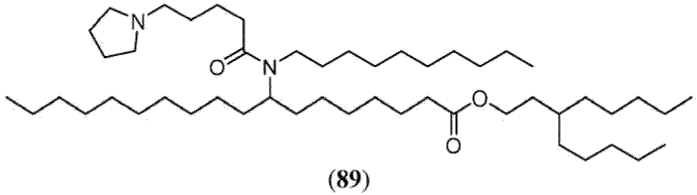 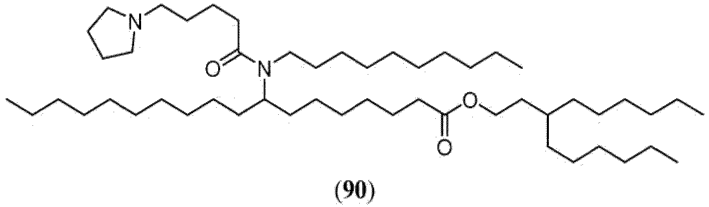 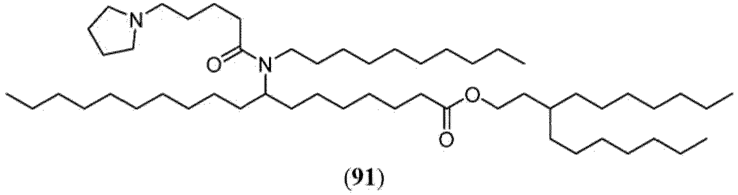 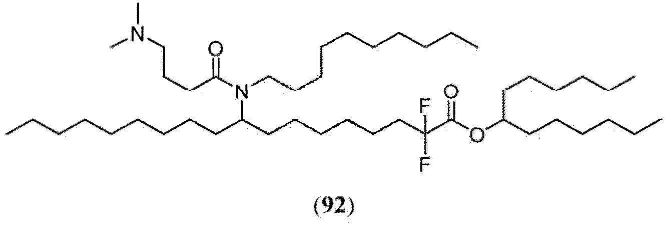 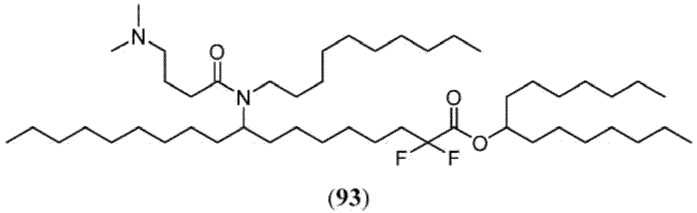 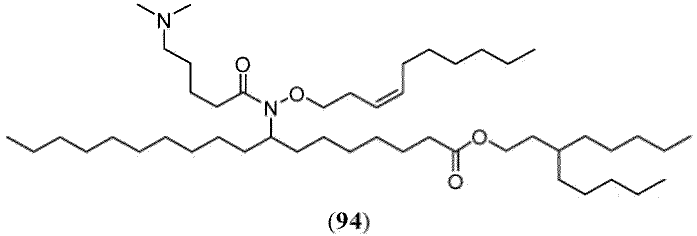 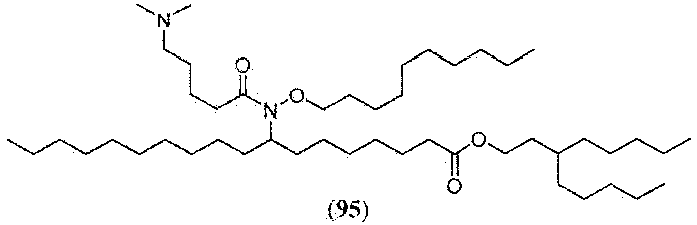 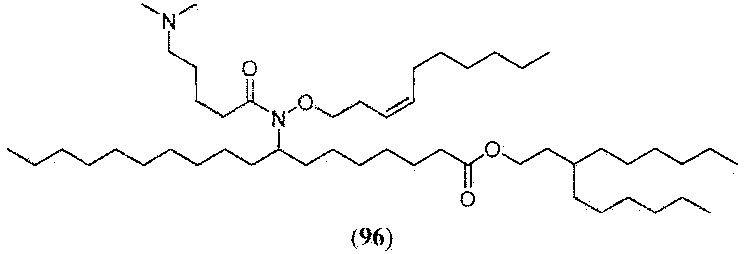 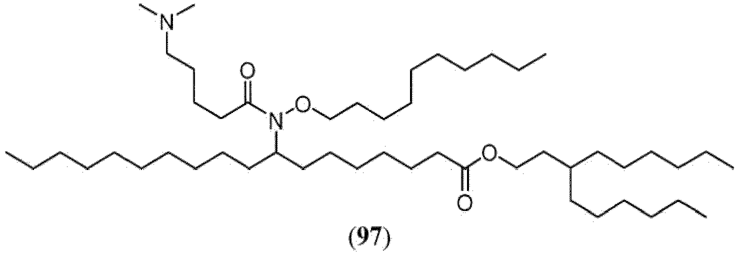 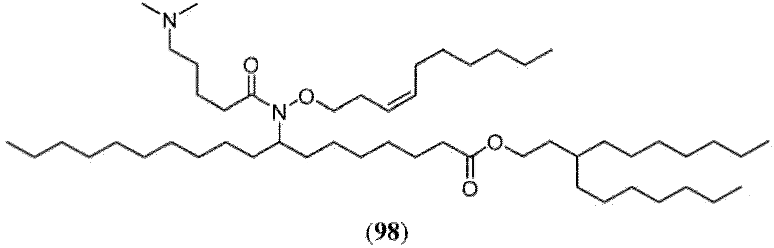 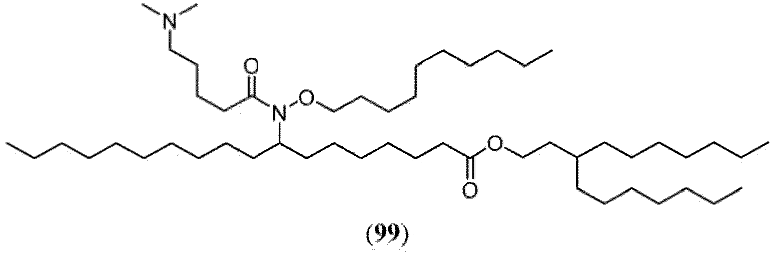 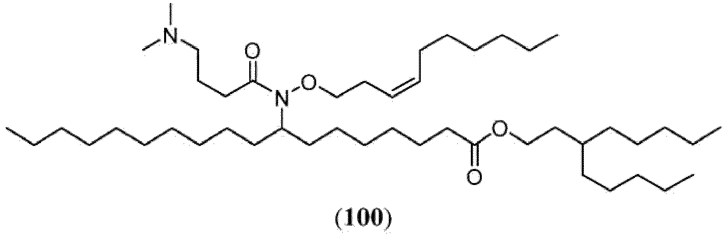 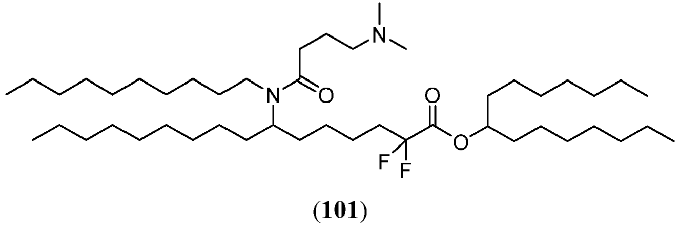 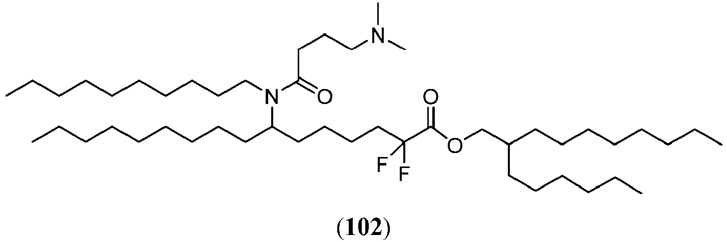 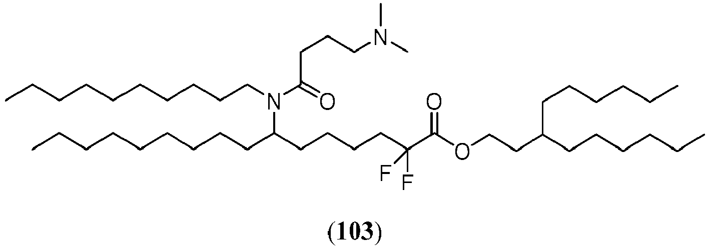 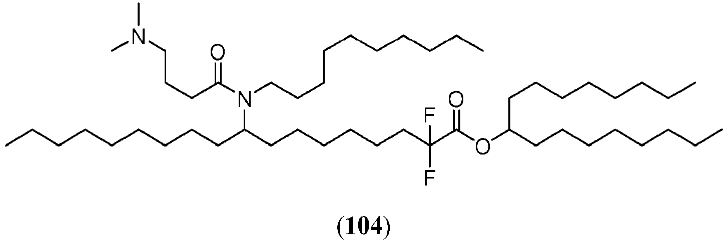 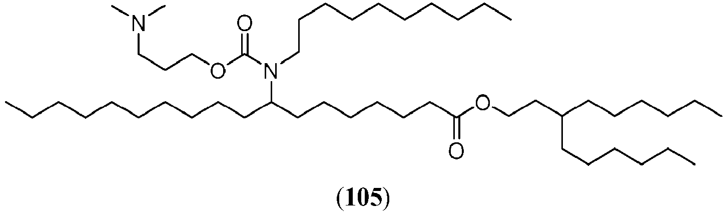 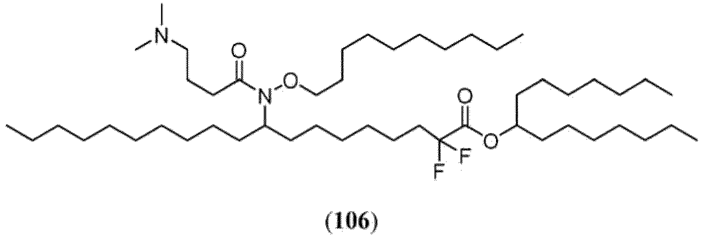 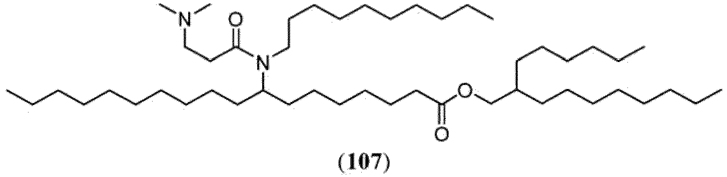 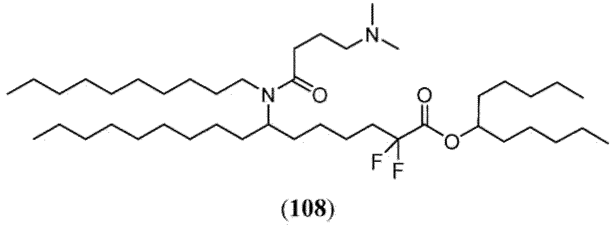 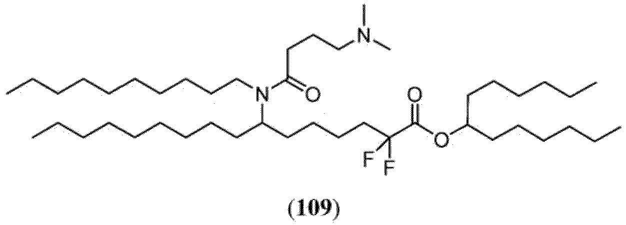 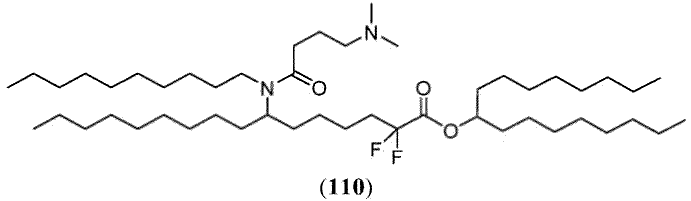 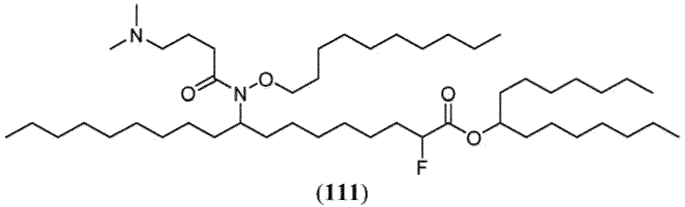 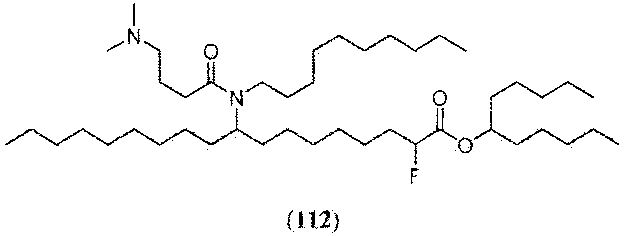 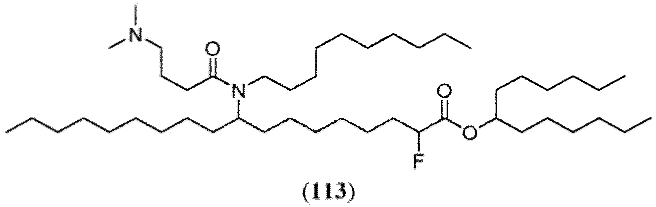 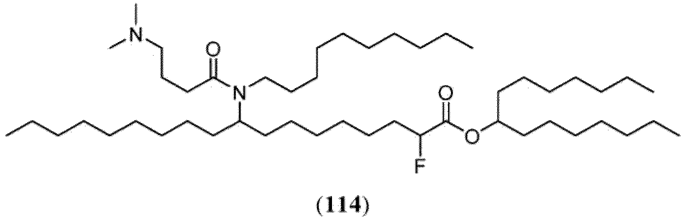 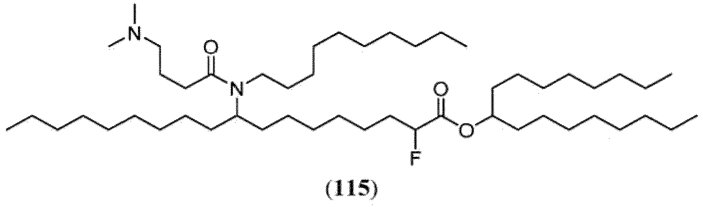 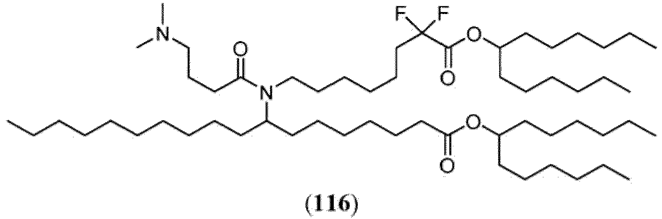 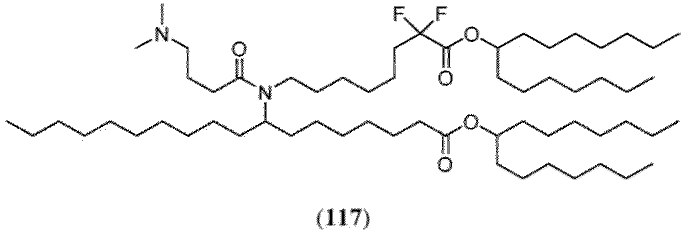 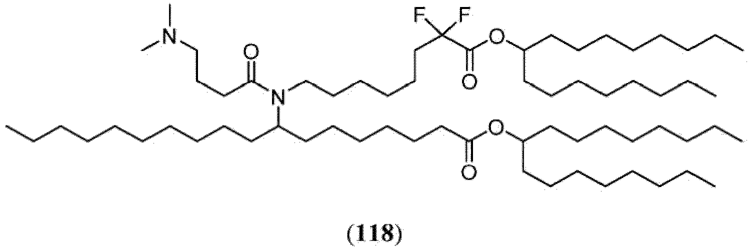 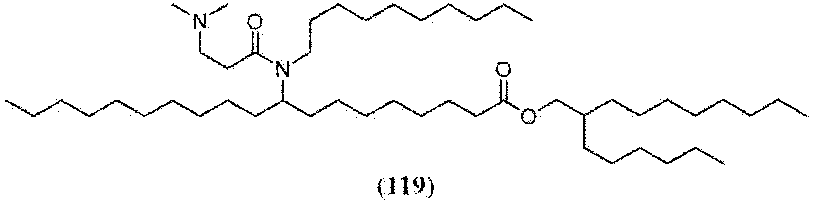 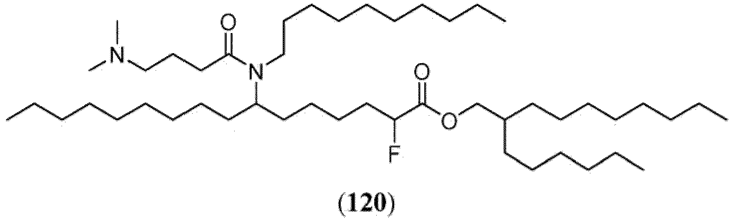 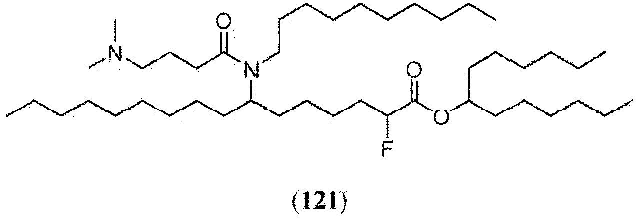 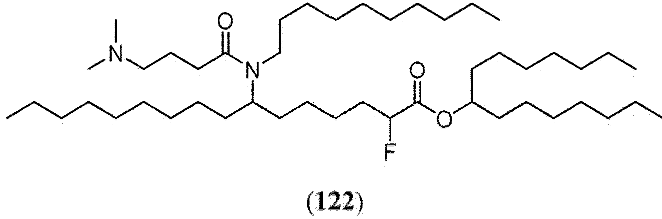 und 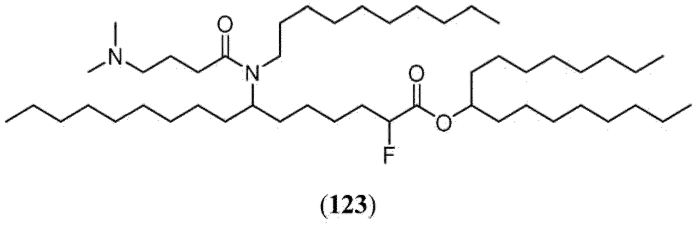 oder einem Salz davon.

9. Verbindung oder Salz nach Anspruch 1, wobei:
(i) die Verbindung oder das Salz ist: 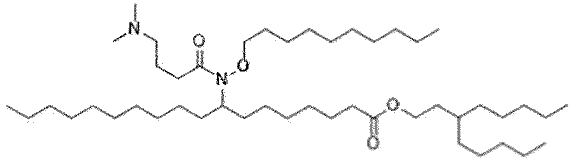 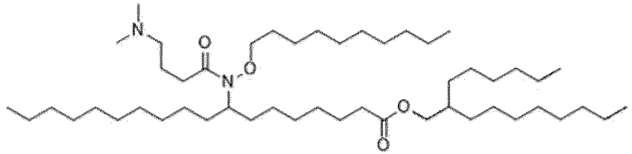 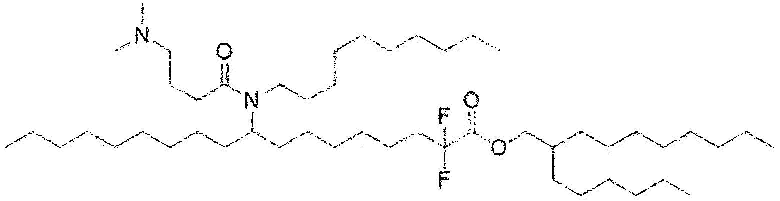 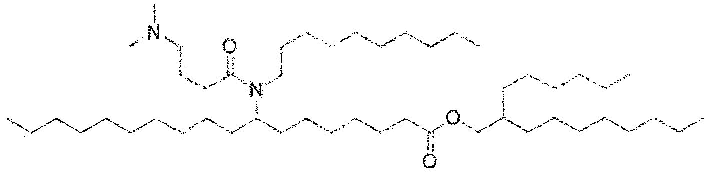 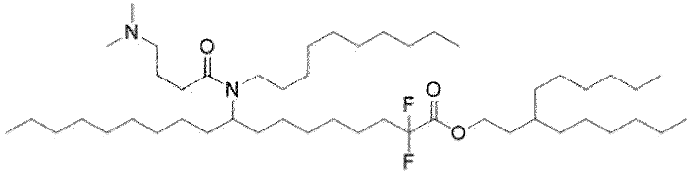 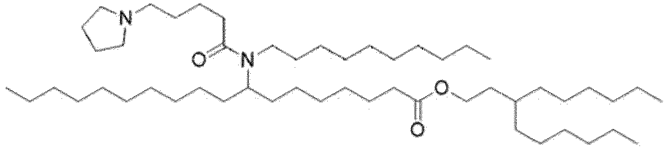 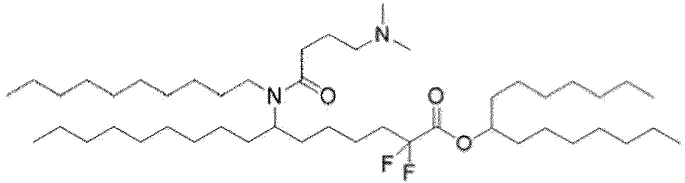 oder 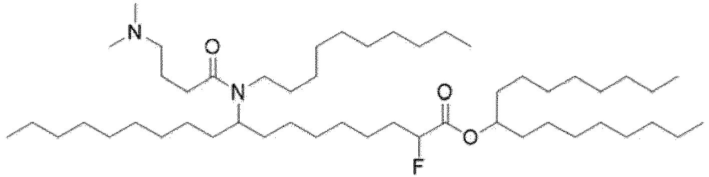 oder ein Salz davon; oder
(ii) 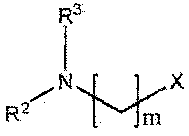 aus der Gruppe ausgewählt ist, bestehend aus: (CH₃)₂NCH₂CH₂C(=O)-, (CH₃)₂NCH₂CH₂CH₂C(=O)-, (CH₃)₂NCH₂CH₂CH₂CH₂C(=O)-, (CH₃)₂NCH₂CH₂-, (CH₃)₂NCH₂CH₂CH-, 5-Pyrolidin-1-ylpentanoyl und (CH₃)₂NCH₂CH₂CH₂O-.

10. Nukleinsäure-Lipid-Partikel, umfassend
(a) ein oder mehrere Nukleinsäuremoleküle;
(b) ein nicht-kationisches Lipid;
(c) ein konjugiertes Lipid; und
(d) eine Verbindung oder ein Salz, wie in einem der Ansprüche 1-9 beschrieben, wobei das eine oder die mehreren Nukleinsäuremoleküle in dem Lipid-Partikel eingekapselt sind.

11. Nukleinsäure-Lipid-Partikel nach Anspruch 10, wobei:
(i)(a) die Verbindung oder das Salz 30 Mol-% bis 85 Mol-% des in dem Partikel vorhandenen Gesamtlipids umfasst; das nicht-kationische Lipid 13 Mol-% bis 49,5 Mol-% des in dem Partikel vorhandenen Gesamtlipids umfasst; und das konjugierte Lipid 0,1 Mol-% bis 10 Mol-% des in dem Partikel vorhandenen Gesamtlipids umfasst, oder (b) die Verbindung oder das Salz 30 Mol-% bis 85 Mol-% des in dem Partikel vorhandenen Gesamtlipids umfasst; das nicht-kationische Lipid 13 Mol-% bis 49,5 Mol-% des in dem Partikel vorhandenen Gesamtlipids umfasst; und das konjugierte Lipid 0,1 Mol-% bis 10 Mol-% des in dem Partikel vorhandenen Gesamtlipids umfasst; und/oder
(ii) die Nukleinsäure aus der Gruppe ausgewählt ist, bestehend aus kleiner interferierender RNA (siRNA), Dicer-Substrat-dsRNA, kleiner Haarnadel-RNA (shRNA), asymmetrischer interferierender RNA (aiRNA), microRNA (miRNA), mRNA, tRNA, rRNA, viraler RNA (vRNA), selbst-amplifizierender RNA und Kombinationen davon; oder die Nukleinsäure ein mRNA-Molekül ist; oder die Nukleinsäure ein doppelsträngiges siRNA-Molekül umfasst, optional wobei das doppelsträngige siRNA-Molekül wenigstens ein modifiziertes Nukleotid umfasst oder die siRNA wenigstens ein 2'-O-Methyl- (2'OMe-) Nukleotid umfasst; und/oder
(iii) das nicht-kationische Lipid umfasst: (a) Cholesterin oder ein Derivat davon, wobei optional das Cholesterin oder ein Derivat davon 31,5 Mol-% bis 42,5 Mol-% des in dem Partikel vorhandenen Gesamtlipids umfasst; (b) ein Phospholipid; oder (c) eine Mischung aus einem Phospholipid und Cholesterin oder einem Derivat davon, wobei das Phospholipid optional Dipalmitoylphosphatidylcholin (DPPC), Distearoylphosphatidylcholin (DSPC) oder eine Mischung davon umfasst, wobei das Phospholipid von 4 Mol-% bis 10 Mol-% des in dem Partikel vorhandenen Gesamtlipids umfasst und das Cholesterin von 30 Mol-% bis 40 Mol-% des in dem Partikel vorhandenen Gesamtlipids umfasst, oder das Phospholipid von 10 Mol-% bis 30 Mol-% des in dem Partikel vorhandenen Gesamtlipids umfasst und das Cholesterin von 10 Mol-% bis 30 Mol-% des in dem Partikel vorhandenen Gesamtlipids umfasst; und/oder
(iv) das konjugierte Lipid ein Polyethylenglykol- (PEG-) Lipid-Konjugat umfasst, wobei optional: (a) das PEG-Lipid-Konjugat ein PEG-Diacylglycerol (PEG-DAG-) Konjugat, ein PEG-Dialkyloxypropyl (PEG-DAA-) Konjugat oder eine Mischung davon umfasst, wobei optional das PEG-DAA-Konjugat ein PEG-Dimyristyloxypropyl- (PEG-DMA-) Konjugat, ein PEG-Distearyloxypropyl- (PEG-DAS-) Konjugat oder eine Mischung davon oder ein PEG-Dimyristyloxypropyl- (PEG-DMA-) Konjugat umfasst; (b) das Polyethylenglykol-(PEG-) Lipid-Konjugat eine Verbindung der Formel ist:
A-B-C
oder ein Salz davon, wobei:
A (C₁-C₆)-Alkyl, (C₃-C₈)-Cycloalkyl, (C₃-C₈)-Cycloalkyl(C₁-C₆)-Alkyl, (C₁-C₆)-Alkoxy, (C₂-C₆)-Alkenyl, (C₂-C₆)-Alkenyl, (C₁-C₆)-Alkinyl, (C₁-C₆)-Alkanoyl, (C₁-C₆)-Alkoxycarbonyl, (C₁-C₆)-Alkylthio, oder (C₂-C₆)-Alkanoyloxy ist, wobei jedes (C₁-C₆)-Alkyl, (C₃-C₈)-Cycloalkyl, (C₃-C₈)-Cycloalkyl(C₁-C₆)-Alkyl, (C₁-C₆)-Alkoxy, (C₂-C₆)-Alkenyl, (C₂-C₆)-Alkenyl, (C₁-C₆)-Alkinyl, (C₁-C₆)-Alkanoyl, (C₁-C₆)-Alkoxycarbonyl, (C₁-C₆)-Alkylthio und (C₂-C₆)-Alkanoyloxy mit einer oder mehreren anionischen Vorläufergruppen substituiert ist, und wobei jedes (C₁-C₆)-Alkyl, (C₃-C₈)-Cycloalkyl, (C₃-C₈)-Cycloalkyl(C₁-C₆)-Alkyl, (C₁-C₆)-Alkoxy, (C₂-C₆)-Alkenyl, (C₂-C₆)-Alkenyl, (C₁-C₆)-Alkinyl, (C₁-C₆)-Alkanoyl, (C₁-C₆)-Alkoxycarbonyl, (C₁-C₆)-Alkylthio und (C₂-C₆)-Alkanoyloxy optional mit einer oder mehreren Gruppen substituiert ist, die unabhängig aus der Gruppe ausgewählt sind, bestehend aus Halo, Hydroxyl, (C₁-C₃)-Alkoxy, (C₁-C₆)-Alkanoyl, (C₁-C₃)-Alkoxycarbonyl, (C₁-C₃)-Alkylthio oder (C₂-C₃)-Alkanoyloxy;
B eine Polyethylenglykolkette mit einem Molekulargewicht von 550 Dalton bis 10.000 Dalton ist;
C -L-R^{a} ist
L der Gruppe ausgewählt ist, bestehend aus einer direkten Bindung, -C(O)O-, - C(O)NR^{b}-, -NR^{b}-, - C(O)-, -NR^{b}C(O)O-, -NR^{b}C(O)NR^{b}-, -S-S-, -O-, -(O)CCH₂CH₂C(O)-, und -NHC(O)CH₂CH₂C(O)NH-;
R^{a} ein verzweigtes (C₁₀-C₅₀)-Alkyl oder verzweigtes (C₁₀-C₅₀)-Alkenyl ist, wobei ein oder mehrere Kohlenstoffatome des verzweigten (C₁₀-C₅₀)-Alkyls oder des verzweigten (C₁₀-C₅₀)-Alkenyls durch -O- ersetzt worden sind; und
jedes R^{b} unabhängig H oder (C₁-C₆)-Alkyl ist; oder (c) das PEG ein durchschnittliches Molekulargewicht von 2.000 Dalton aufweist; und/oder
(v) das konjugierte Lipid ein PEG-Lipid-Konjugat umfasst, ausgewählt aus: 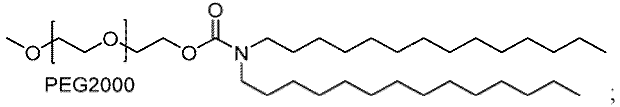 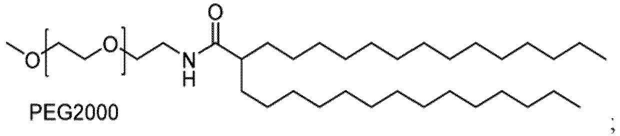 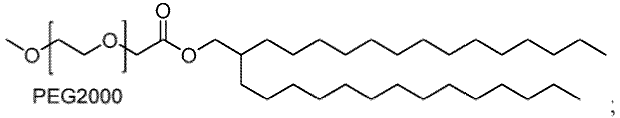 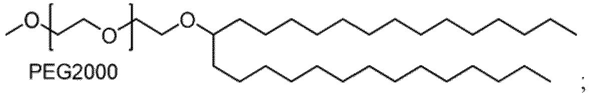 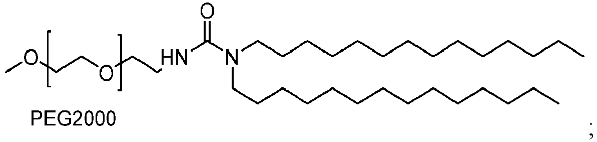 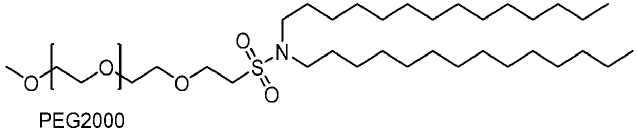 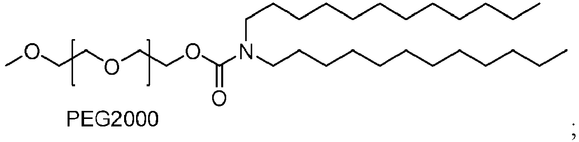 und 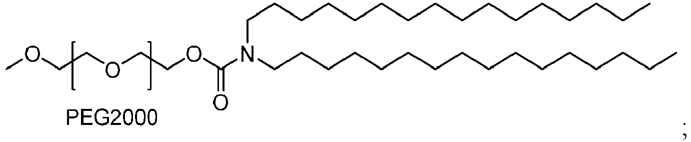 oder ein Salz davon; und/oder
(vii) das konjugierte Lipid eine Verbindung der folgenden Formel umfasst: 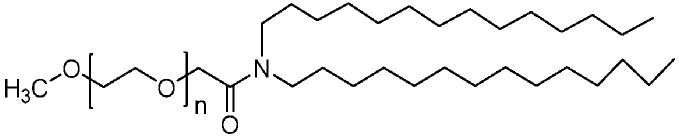 wobei n 45-50 ist; und/oder
(viii) das konjugierte Lipid 1 Mol-% bis 2 Mol-% des in dem Partikel vorhandenen Gesamtlipids umfasst; und/oder
(ix) die Nukleinsäure in dem Nukleinsäure-Lipid-Partikel nach Inkubation des Partikels in Serum bei 37 °C für 30 Minuten nicht wesentlich abgebaut wird und/oder
(x) die Nukleinsäure vollständig in dem Nukleinsäure-Lipid-Partikel eingekapselt ist, wobei optional das Nukleinsäure-Lipid-Partikel ein Lipid : Nukleinsäure-Massenverhältnis von 5 bis 15 oder von 5 bis 30 aufweist; und/oder
(xi) das Nukleinsäure-Lipid-Partikel einen mittleren Durchmesser von 40 nm bis 150 nm aufweist.

12. Nukleinsäure-Lipid-Partikel nach Anspruch 10, wobei:
(i) die Verbindung oder das Salz 56,5 Mol-% bis 66,5 Mol-% des in dem Partikel vorhandenen Gesamtlipids umfasst; das nicht-kationische Lipid Cholesterin oder ein Derivat davon umfasst, das von 31,5 Mol-% bis 42,5 Mol-% des in dem Partikel vorhandenen Gesamtlipids umfasst; und ein PEG-Lipid-Konjugat 1 Mol-% bis 2 Mol-% des in dem Partikel vorhandenen Gesamtlipids umfasst, wobei optional das Nukleinsäure-Lipid-Partikel 61,5 Mol-% Verbindung oder Salz, 36,9 % Cholesterin oder ein Derivat davon und 1,5 Mol-% PEG-Lipid-Konjugat umfasst; oder
(ii) die Verbindung oder das Salz 52 Mol-% bis 62 Mol-% des in dem Partikel vorhandenen Gesamtlipids umfasst; das nicht-kationische Lipid eine Mischung aus einem Phospholipid und Cholesterin oder einem Derivat davon umfasst, das 36 Mol-% bis 47 Mol-% des in dem Partikel vorhandenen Gesamtlipids umfasst; und das PEG-Lipid-Konjugat 1 Mol-% bis 2 Mol-% des in dem Partikel vorhandenen Gesamtlipids umfasst, wobei optional: (a) das Nukleinsäure-Lipid-Partikel 57,1 Mol-% Verbindung oder Salz, 7,1 Mol-% Phospholipid, 34,3 Mol-% Cholesterin oder ein Derivat davon und 1,4 Mol-% PEG-Lipid-Konjugat umfasst; oder (b) das Nukleinsäure-Lipid-Partikel 57,1 Mol-% Verbindung oder Salz, 20 Mol-% Phospholipid, 20 Mol-% Cholesterin oder ein Derivat davon, und 1,4 Mol-% PEG-Lipid-Konjugat umfasst; oder
(iii) die Verbindung oder das Salz 50 Mol-% bis 65 Mol-% des in dem Partikel vorhandenen Gesamtlipids umfasst; das nicht-kationische Lipid eine Mischung aus einem Phospholipid und Cholesterin oder einem Derivat davon umfasst, wobei das Phospholipid 4 Mol-% bis 10 Mol-% des in dem Partikel vorhandenen Gesamtlipids umfasst und das Cholesterin oder ein Derivat davon 30 Mol-% bis 40 Mol-% des in dem Partikel vorhandenen Gesamtlipids umfasst; und das konjugierte Lipid 0,5 Mol-% bis 2 Mol-% des in dem Partikel vorhandenen Gesamtlipids umfasst; oder
(iv) die Verbindung oder das Salz 50 Mol-% bis 65 Mol-% des in dem Partikel vorhandenen Gesamtlipids umfasst; wobei das nicht-kationische Lipid eine Mischung aus einem Phospholipid und Cholesterin oder einem Derivat davon umfasst, wobei das Phospholipid 3 Mol-% bis 15 Mol-% des in dem Partikel vorhandenen Gesamtlipids umfasst und das Cholesterin oder ein Derivat davon 30 Mol-% bis 40 Mol-% des in dem Partikel vorhandenen Gesamtlipids umfasst; und das konjugierte Lipid 0,5 Mol-% bis 2 Mol-% des in dem Partikel vorhandenen Gesamtlipids umfasst; oder
(v) die Verbindung oder das Salz 50 Mol-% bis 65 Mol-% des in dem Partikel vorhandenen Gesamtlipids umfasst; das nicht-kationische Lipid bis zu 49,5 Mol-% des in dem Partikel vorhandenen Gesamtlipids umfasst; das Cholesterin oder ein Derivat davon 30 Mol-% bis 40 Mol-% des in dem Partikel vorhandenen Gesamtlipids umfasst; und das konjugierte Lipid von 0,5 Mol-% bis 2 Mol-% des in dem Partikel vorhandenen Gesamtlipids umfasst; oder
(vi) die Verbindung oder das Salz 50 Mol-% bis 85 Mol-% des in dem Partikel vorhandenen Gesamtlipids umfasst; das nicht-kationische Lipid 13 Mol-% bis 49,5 Mol-% des in dem Partikel vorhandenen Gesamtlipids umfasst; und das konjugierte Lipid 0,5 Mol-% bis 2 Mol-% des in dem Partikel vorhandenen Gesamtlipids umfasst; oder
(vii) die Verbindung oder das Salz 30 Mol-% bis 50 Mol-% des in dem Partikel vorhandenen Gesamtlipids umfasst; das nicht-kationische Lipid eine Mischung aus einem Phospholipid und Cholesterin oder einem Derivat davon umfasst, das 47 Mol-% bis 69 Mol-% des in dem Partikel vorhandenen Gesamtlipids umfasst; und das konjugierte Lipid von 1 Mol-% bis 3 Mol-% des in dem Partikel vorhandenen Gesamtlipids umfasst, wobei optional das Verhältnis von Lipid zu Nukleinsäure 24 beträgt; oder
(viii) das nicht-kationische Lipid eine Mischung aus Cholesterin und DSPC umfasst; das konjugierte Lipid ist: 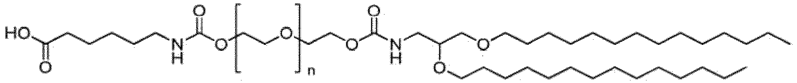 wobei n derart ausgewählt ist, dass die resultierende Polymerkette ein Molekulargewicht von 2000 Dalton aufweist
wobei das konjugierte Lipid 2 Mol-% des in dem Partikel vorhandenen Gesamtlipids umfasst; DSPC 10 Mol-% des in dem Partikel vorhandenen Gesamtlipids umfasst; Cholesterin 48 Mol-% des in dem Partikel vorhandenen Gesamtlipids umfasst; und die Verbindung oder das Salz 40 Mol-% des in dem Partikel vorhandenen Gesamtlipids umfasst, wobei optional das Verhältnis von Lipid zu Nukleinsäure 24 beträgt; oder
(ix) das nicht-kationische Lipid eine Mischung aus Cholesterin und DSPC umfasst; das konjugierte Lipid ist: 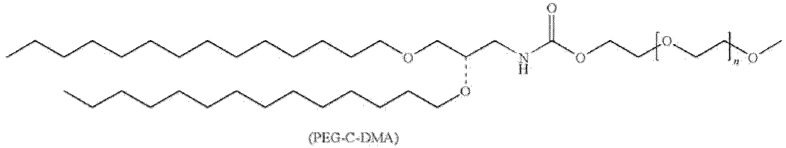 wobei n derart ausgewählt ist, dass die resultierende Polymerkette ein Molekulargewicht von 2000 Dalton aufweist;
wobei das konjugierte Lipid 2 Mol-% des in dem Partikel vorhandenen Gesamtlipids umfasst; DSPC 10 Mol-% des in dem Partikel vorhandenen Gesamtlipids umfasst; Cholesterin 48 Mol-% des in dem Partikel vorhandenen Gesamtlipids umfasst; und die Verbindung oder das Salz 40 Mol-% des in dem Partikel vorhandenen Gesamtlipids umfasst, wobei optional das Verhältnis von Lipid zu Nukleinsäure 24 beträgt; oder
(x) das nicht-ionische Lipid eine Mischung aus Cholesterin und DSPC umfasst; das konjugierte Lipid ist: 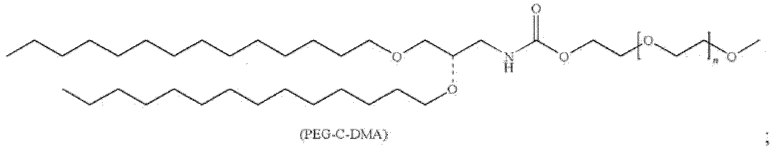 wobei n derart ausgewählt ist, dass die resultierende Polymerkette ein Molekulargewicht von 2000 Dalton aufweist;
wobei das konjugierte Lipid 1,6 Mol-% des in dem Partikel vorhandenen Gesamtlipids umfasst; DSPC 10,9 Mol-% des in dem Partikel vorhandenen Gesamtlipids umfasst; Cholesterin 32,8 Mol-% des in dem Partikel vorhandenen Gesamtlipids umfasst; und die Verbindung oder das Salz 54,9 Mol-% des in dem Partikel vorhandenen Gesamtlipids umfasst, wobei optional das Verhältnis von Lipid zu Nukleinsäure 24 beträgt; oder
(xi) das Nukleinsäure-Lipid-Partikel zwei oder mehr Verbindungen oder Salze wie in einem der Ansprüche 1-9 beschrieben umfasst.

13. Pharmazeutische Zusammensetzung, umfassend ein Nukleinsäure-Lipid-Partikel nach einem der Ansprüche 10-12 und einen pharmazeutisch unbedenklichen Träger.

14. Nukleinsäure-Lipid-Partikel nach einem der Ansprüche 10-12 oder Zusammensetzung nach Anspruch 13 für die Verwendung bei der Behandlung einer Krankheit oder Erkrankung bei einem Säugetier.

15. Nukleinsäure-Lipid-Partikel oder Zusammensetzung für die Verwendung nach Anspruch 14, wobei die Krankheit oder Erkrankung aus der Gruppe ausgewählt ist, bestehend aus einer Virusinfektion, einer Leberkrankheit oder -erkrankung und Krebs.

## Revendications

1. Composé de formule (I) : 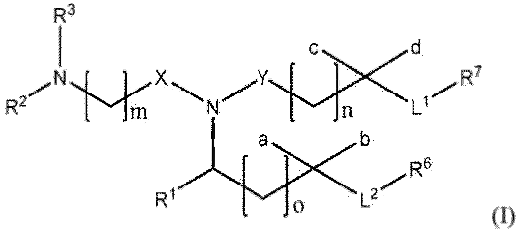 ou sel de celui-ci, où :
X est -CH₂-, -O-, -O(C=O) ; ou -C(=O)-
Y est absent, -CH₂-, -O-, -SO₂-, ou -C(=O)- ;
R¹ est hydrocarbyle en C₂-C₂₅ ;
R² est alkyle en C₁-C₃ ; et R³ est alkyle en C₁-C₃ ; ou R² et R³ pris conjointement avec l'atome d'azote auquel ils sont attachés forment un cycle aziridino, azétidino, ou pyrrolidino ;
m vaut 0, 1, 2, 3, 4, 5 ou 6 ;
n vaut 0 à 15 ;
o vaut 0 à 15 ;
L¹ est absent, -(C=O)-O-, -(C=O)N(H)N(R⁴)-, -C(=O)N(H)-N=C(R⁴)-, - (C=O)N(H)C(R⁴)(R⁵)-, -(C=O)-O-CH₂-R^{z}, ou -O(C=O)-R^{w}- ;
L² est absent, -(C=O)-O-, -(C=O)N(H)N(R^{4'})-, -(C=O)N(H)-N=C(R^{4'})-, - (C=O)N(H)N(H)C(R^{4'})(R^{5'})-, -(C=O)-O-CH₂-R^{z'}, ou -O(C=O)-R^{x}-
a est H ou F ;
b est H ou F ;
c est H ou F ; et d est H ou F ; ou c et d pris conjointement avec l'atome de carbone auquel ils sont attachés forment -C(=O) ;
R⁴ est H ou hydrocarbyle en C₂-C₂₅ ;
R⁵est H ou hydrocarbyle en C₂-C₂₅ ;
R^{4'} est H ou hydrocarbyle en C₂-C₂₅ ;
R⁵*^{'}* est H ou hydrocarbyle en C₂-C₂₅ ;
R⁶ est hydrocarbyle en C₂-C₂₅ ;
R⁷ est hydrocarbyle en C₂-C₂₅ ;
R^{w} est absent, -C(F)H-, ou -C(F)₂- ;
R^{x} est absent, -C(F)H-, ou -C(F)₂- ;
R² est phényle qui est substitué par 1, 2, ou 3 groupes indépendamment sélectionnés parmi hydrocarbyle en C₂-C₁₀ ; et
R^{z'} est phényle qui est substitué par 1, 2, ou 3 groupes indépendamment sélectionnés parmi hydrocarbyle en C₂-C₁₀.

2. Composé ou sel selon la revendication 1, dans lequel :
(i) R¹ est hydrocarbyle en C₅-C₂₅, alkyle en C₅-C₂₅, alcényle en C₅-C₂₅, 1-nonyle ou 1-décyle ; et/ou
(ii) X est -CH₂-, -C(=O)- ou -O- ; et/ou
(iii) R² est méthyle, éthyle ou propyle ; et/ou
(iv) R³ est méthyle, éthyle ou propyle ; et/ou
(v) m vaut 1, 2 ou 3 ; et/ou
(vi) o vaut 2 à 15, 10 à 15, 2 à 10 ou 2 à 5 ; et/ou
(vii) L² est -C(=O)-O-, -(C=O)N(H)N(R⁴)(R⁵)-, -(C=O)N(H)-N=C(R⁴)-, - (C=O)N(H)N(H)C(R⁴)(R⁵)-, ou -(C=O)-O-CH₂-R^{z}, et éventuellement où L² est -(C=O)-O-, - (C=O)N(H)N(R⁴)(R⁵)- ou -(C=O)-O-CH₂-R^{z} ; et/ou
(viii) a est H ou F ; et/ou
(ix) b est H ou F ; et/ou
(x) R⁶ est hydrocarbyle en C₂-C₂₅, hydrocarbyle en C₅-C₂₅, alkyle en C₂-C₂₅, alkyle en C₅-C₂₅ ou alcényle en C₅-C₂₅.

3. Composé ou sel selon la revendication 1 ou 2 dans lequel :
(i) 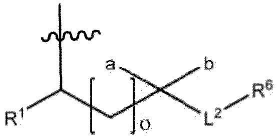 est sélectionné dans le groupe constitué par : 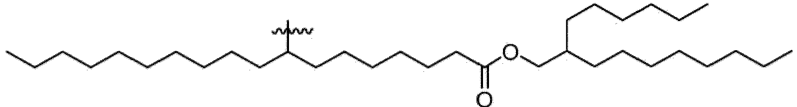 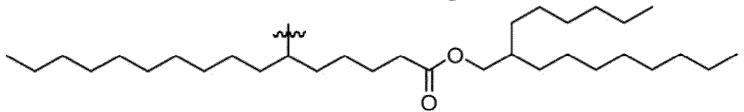 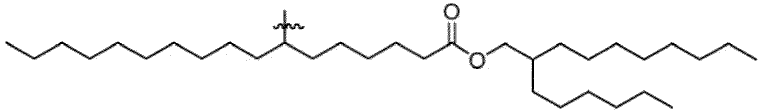 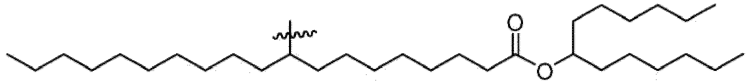 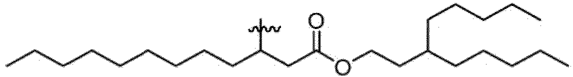 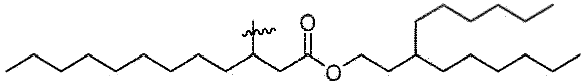 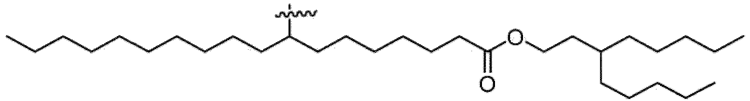 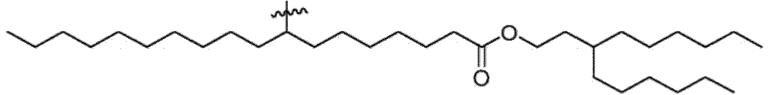 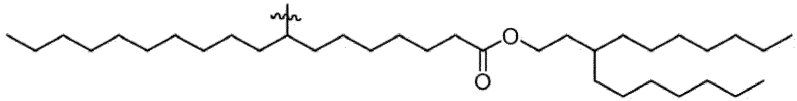 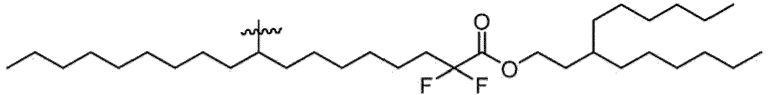 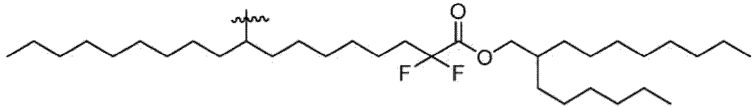 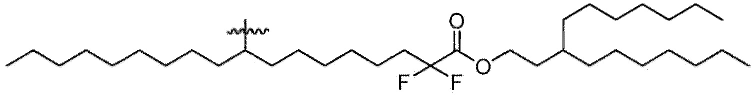 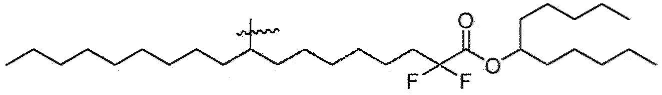 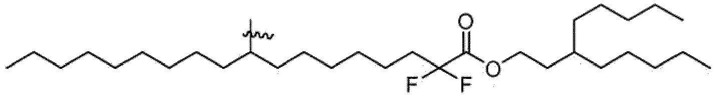 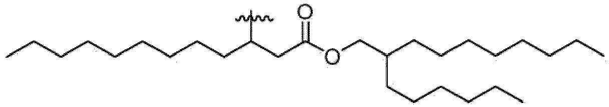 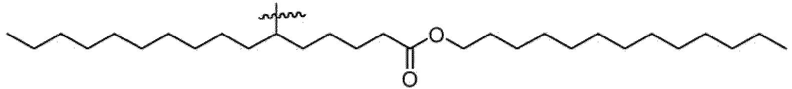 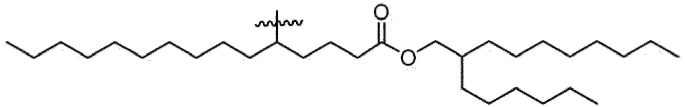 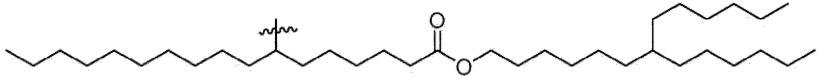 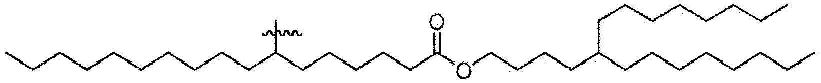 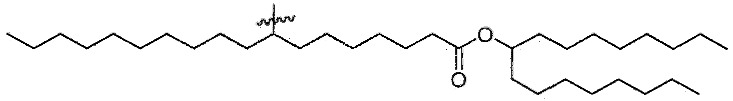 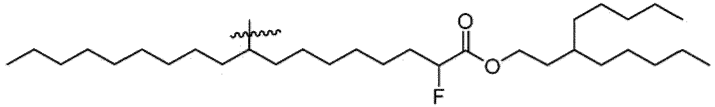 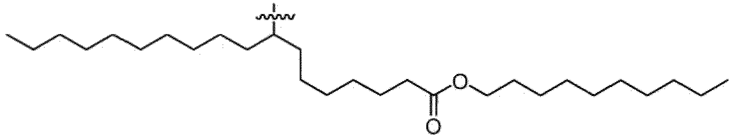 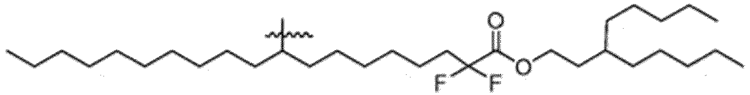 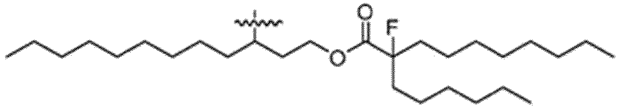 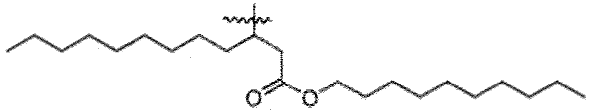 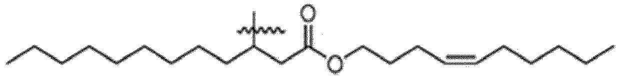 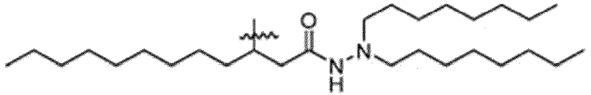 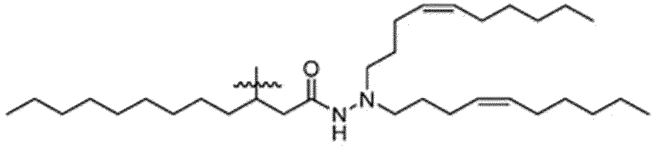 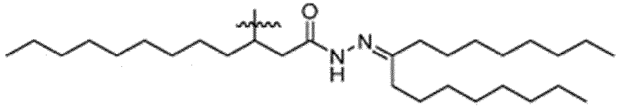 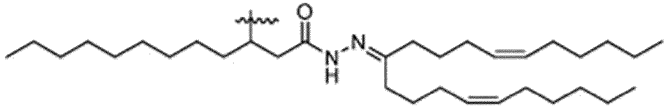 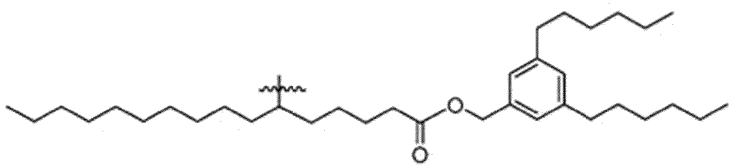 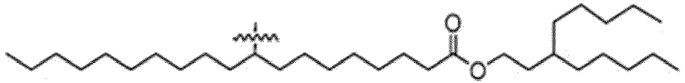 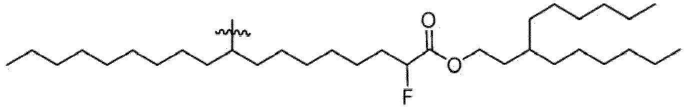 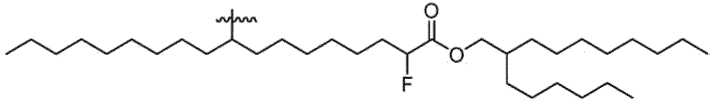 et 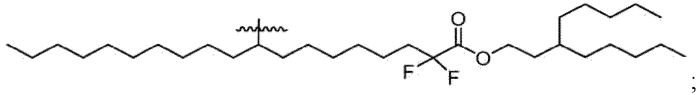 ou
(ii) 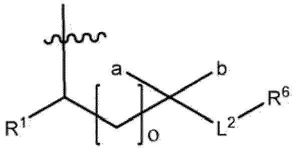 est sélectionné dans le groupe constitué par : 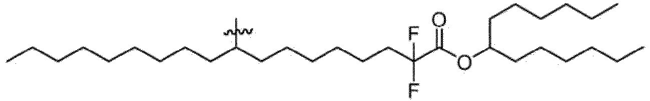 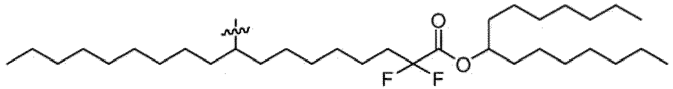 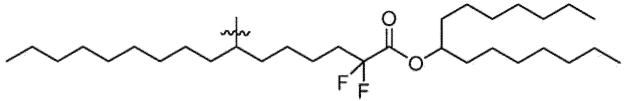 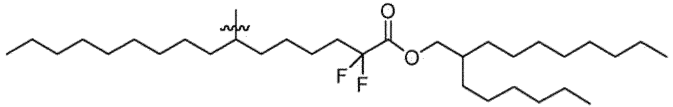 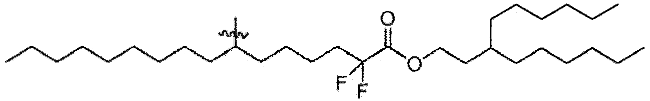 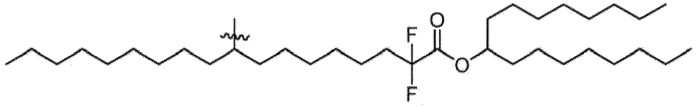 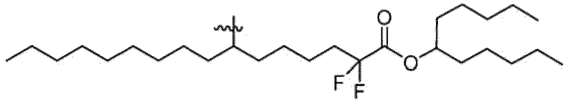 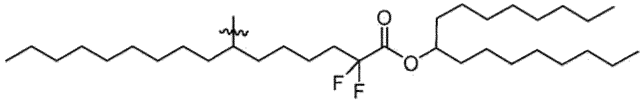 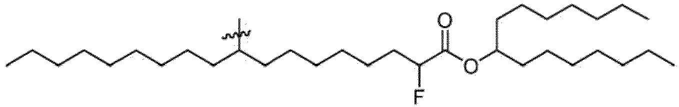 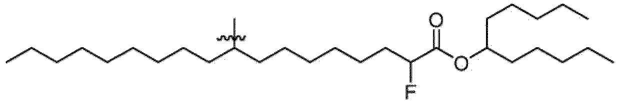 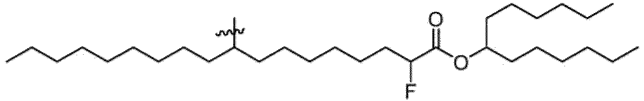 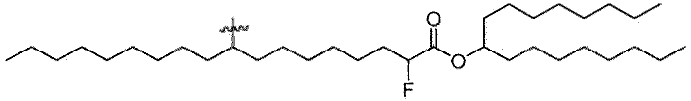 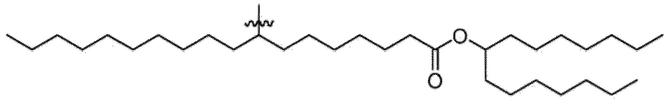 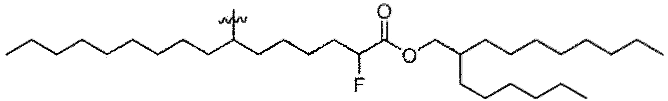 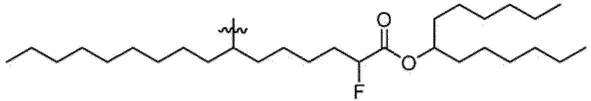 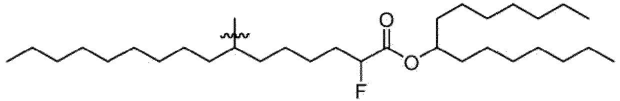 et 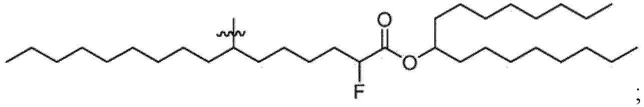 ou
(iii) 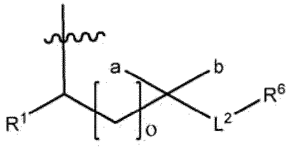 est sélectionné dans le groupe constitué par : 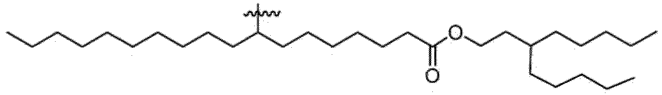 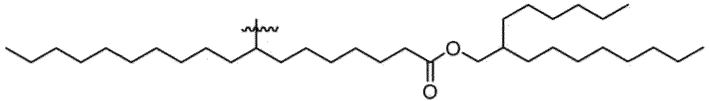 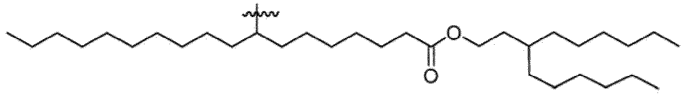 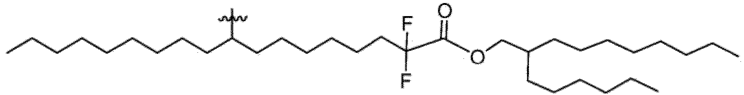 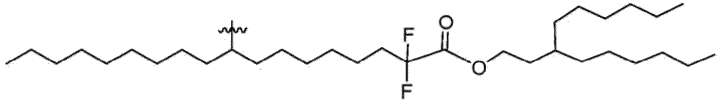 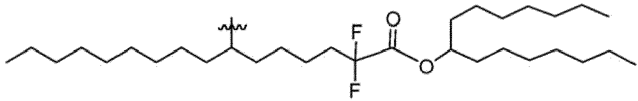 et

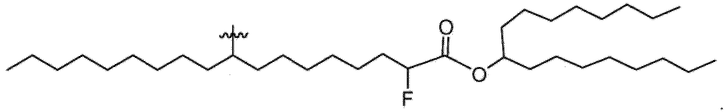

4. Composé ou sel selon l'une quelconque des revendications 1 à 3, dans lequel :
(i) Y est -CH₂-, -C(=O)- ou -O- ; et/ou
(ii) n vaut 2 à 15, 10 à 15, 2 à 10 ou 2 à 5 ; et/ou
(iii)(a) L¹ est absent ; et L² est -(C=O)-O-, -(C=O)N(H)N(R⁴)(R⁵)-, -(C=O)N(H)-N=C(R⁴)-, -(C=O)N(H)N(H)C(R⁴)(R⁵)-, ou -(C=O)-O-CH₂-R^{z}, ou (b) L¹ est -(C=O)-O-, - (C=O)N(H)N(R⁴)(R⁵)-, -(C=O)N(H)-N=C(R⁴)-, -(C=O)N(H)N(H)C(R⁴)(R⁵)-, ou -(C=O)-O-CH₂-R^{a} ; et L² est absent, ou (c) L¹ est -(C=O)-O-, -(C=O)N(H)N(R⁴)(R⁵)-, -(C=O)N(H)-N=C(R⁴)-, -(C=O)N(H)N(H)C(R⁴)(R⁵)-, ou -(C=O)-O-CH₂-R^{a}, ou (d) L¹ est -(C=O)-O, L¹ est -(C=O)N(H)N(R⁴)(R⁵)- ou L¹ est -(C=O)-O-CH₂-R^{z} ; et/ou
(iv) c est H ou F ; et/ou
(v) d est H ou F ; et/ou
(vi) R⁷ est hydrocarbyle en C₂-C₂₅, hydrocarbyle en C₅-C₂₅, alkyle en C₂-C₂₅, alkyle en C₅-C₂₅ ou alcényle en C₅-C₂₅.

5. Composé ou sel selon l'une quelconque des revendications 1 à 3, dans lequel :
(i) 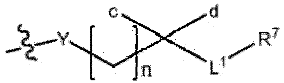 est sélectionné dans le groupe constitué par : 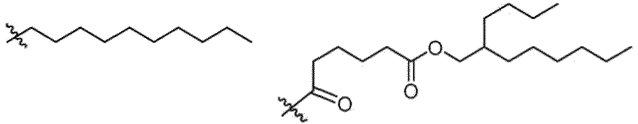 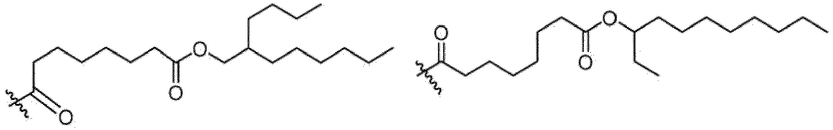 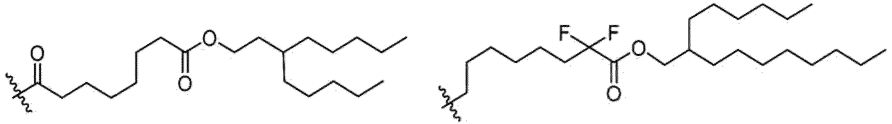 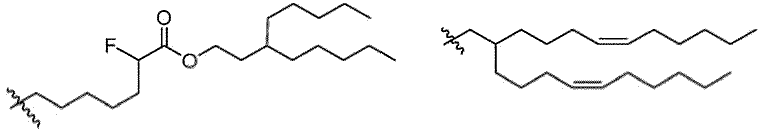 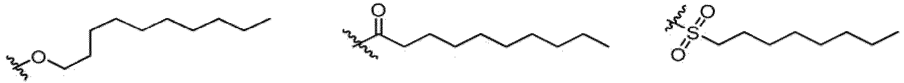 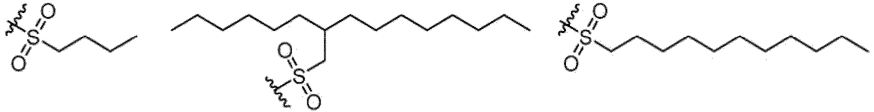 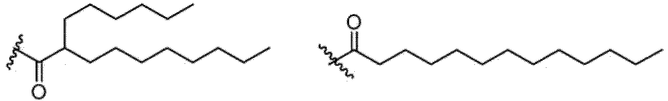 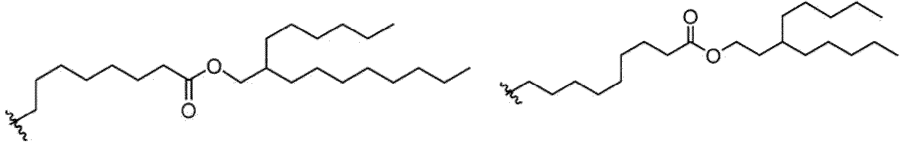 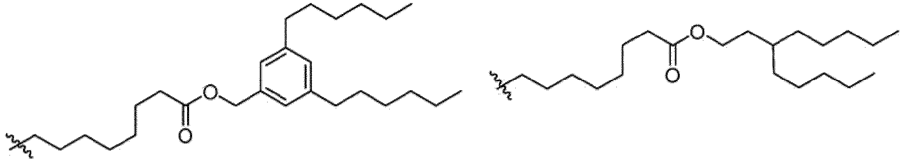 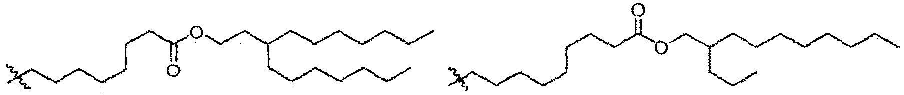 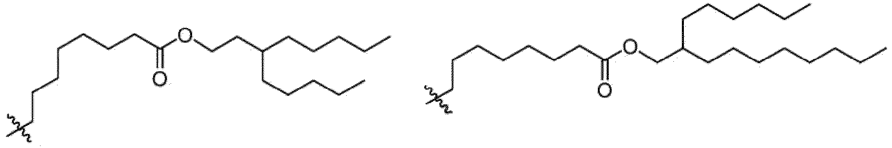 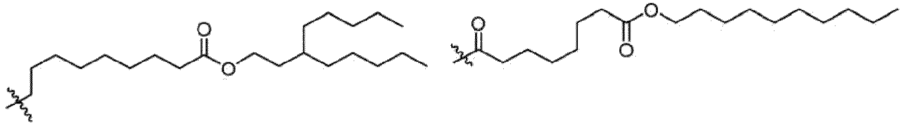 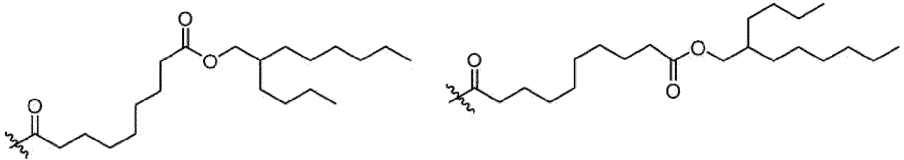 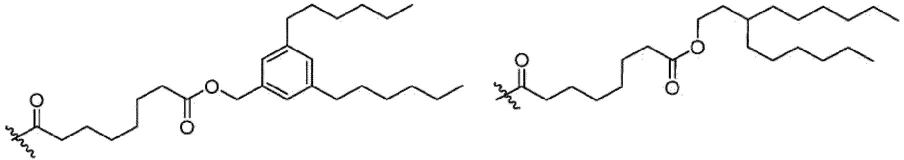 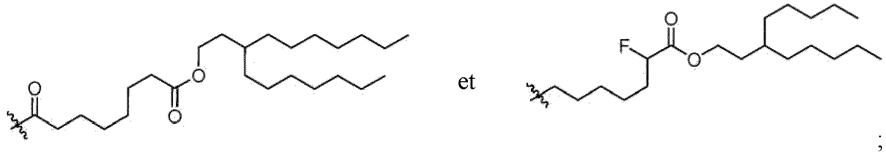 ou
(ii) 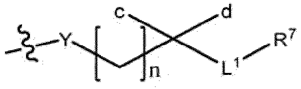 est sélectionné dans le groupe constitué par : 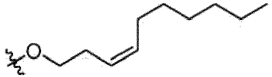 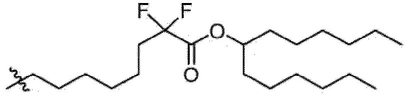 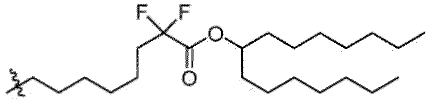 et 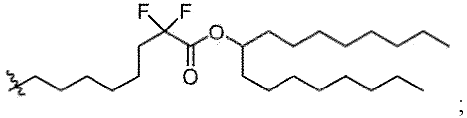 ou
(iii) 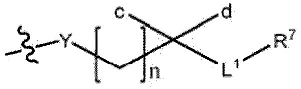 est sélectionné dans le groupe constitué par :

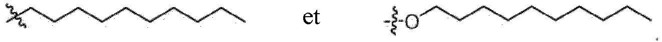

6. Composé ou sel selon l'une quelconque des revendications 1 à 4, dans lequel :
(i) R⁴ est hydrocarbyle en C₂-C₂₅, hydrocarbyle en C₅-C₂₅, alkyle en C₂-C₂₅, alkyle en C₅-C₂₅ ou alcényle en C₅-C₂₅ ; ou
(ii) R*⁵* est hydrocarbyle en C₂-C₂₅, hydrocarbyle en C₅-C₂₅, alkyle en C₂-C₂₅, alkyle en C₅-C₂₅ ou alcényle en C₅-C₂₅ ; ou
(iii) R^{z} est phényle qui est substitué par 1 ou 2 groupes indépendamment sélectionnés parmi hydrocarbyle en C₂-C₁₀, et éventuellement où chaque hydrocarbyle en C₂-C₁₀ est indépendamment sélectionné dans le groupe constitué par hydrocarbyle en C₅-C₁₀, alkyle en C₂-C₁₀, alkyle en C₅-C₁₀ et alcényle en C₅-C₁₀.

7. Composé ou sel selon la revendication 1 ou 2, dans lequel :
(i) R^{4'} est hydrocarbyle en C₂-C₂₅, hydrocarbyle en C₅-C₂₅, alkyle en C₂-C₂₅, alkyle en C₅-C₂₅ ou alcényle en C₅-C₂₅ ; ou
(ii) R^{5'} est hydrocarbyle en C₂-C₂₅, hydrocarbyle en C₅-C₂₅, alkyle en C₂-C₂₅, alkyle en C₅-C₂₅ ou alcényle en C₅-C₂₅ ou
(iii) R^{z'} est phényle qui est substitué par 1 ou 2 groupes indépendamment sélectionnés parmi hydrocarbyle en C₂-C₁₀, et éventuellement où chaque hydrocarbyle en C₂-C₁₀ est indépendamment sélectionné dans le groupe constitué par hydrocarbyle en C₅-C₁₀, alkyle en C₂-C₁₀, alkyle en C₅-C₁₀ et alcényle en C₅-C₁₀.

8. Composé ou sel selon la revendication 1, qui est sélectionné dans le groupe constitué par : 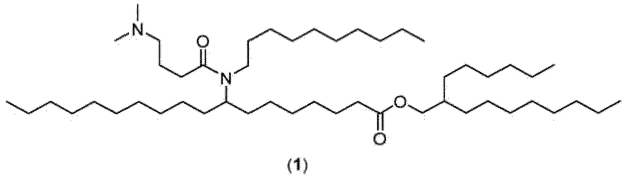 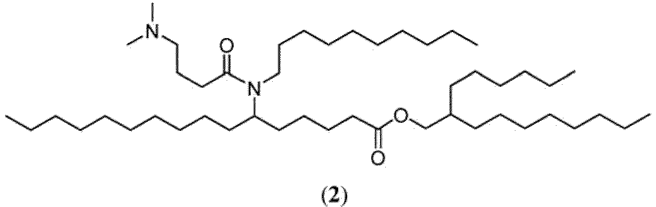 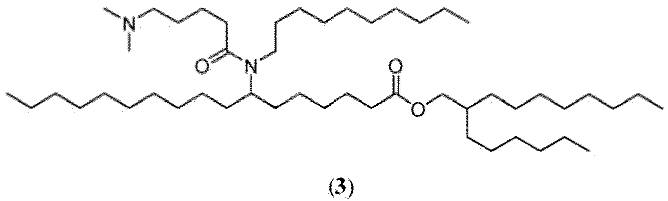 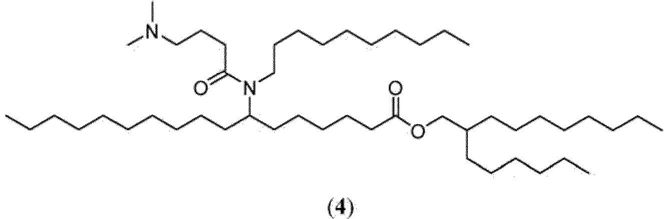 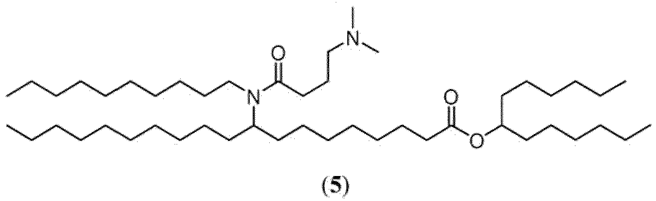 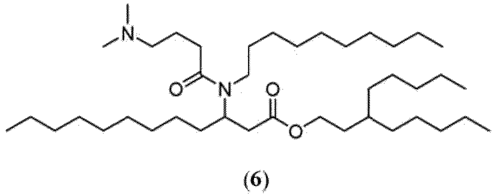 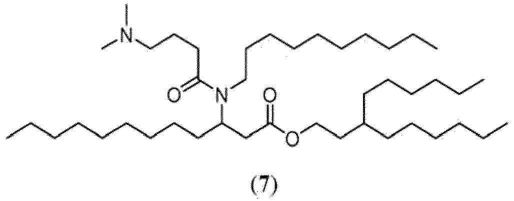 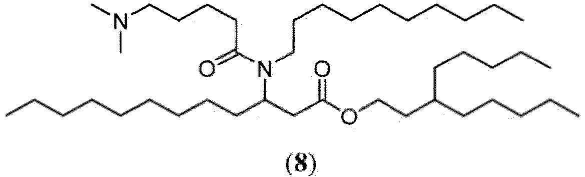 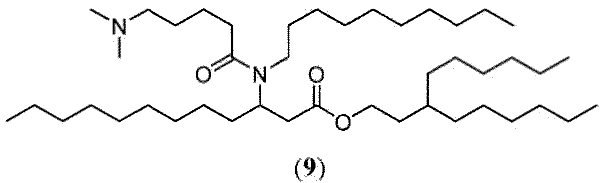 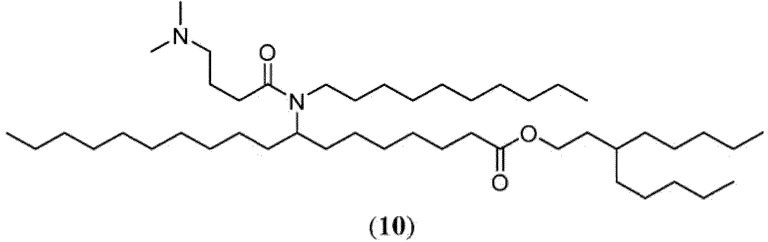 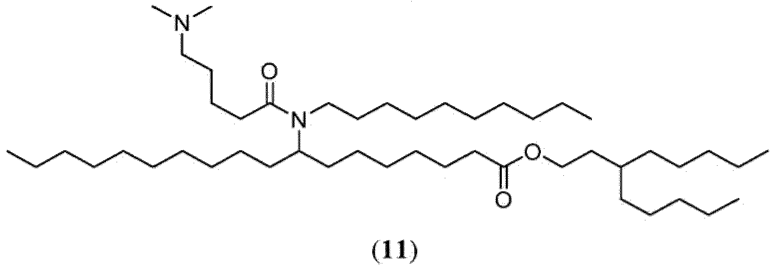 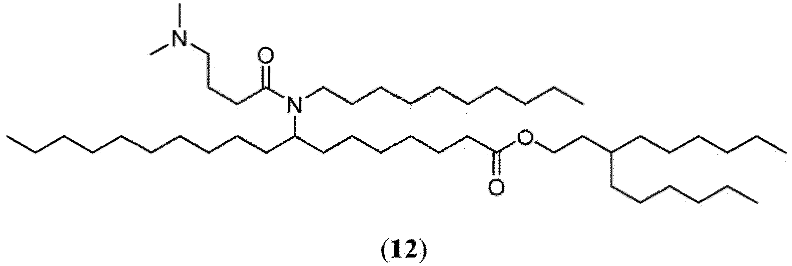 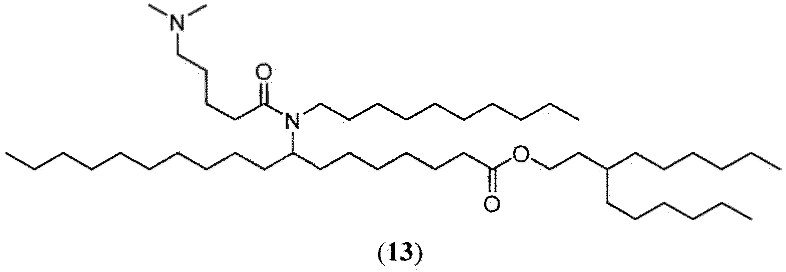 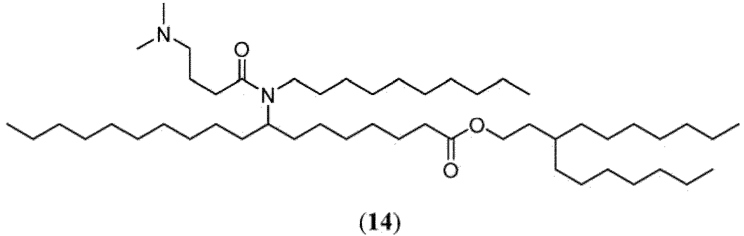 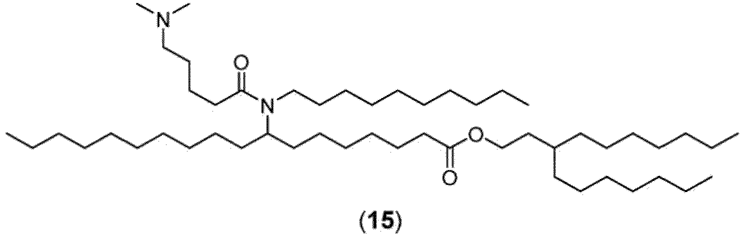 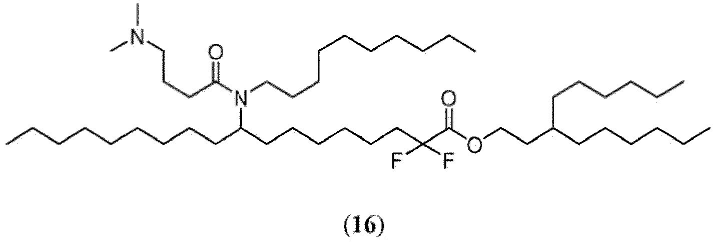 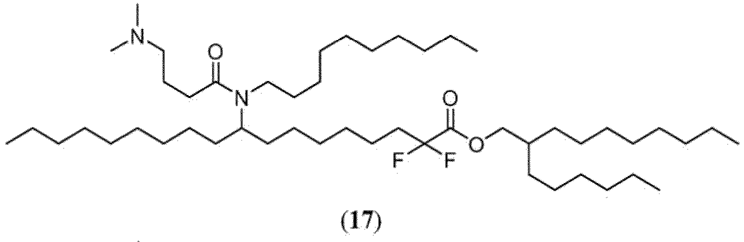 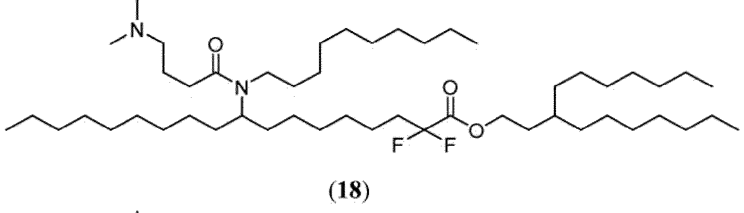 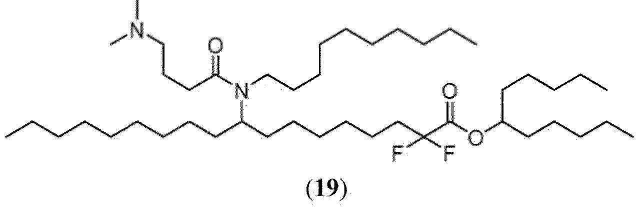 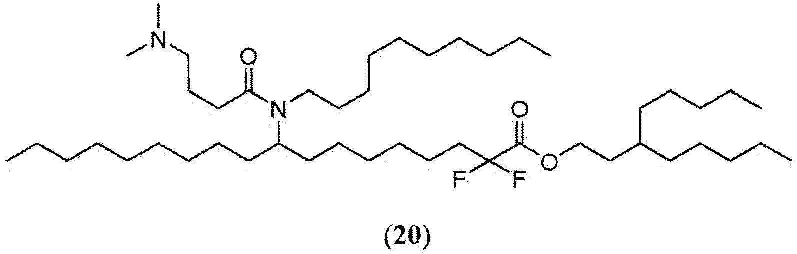 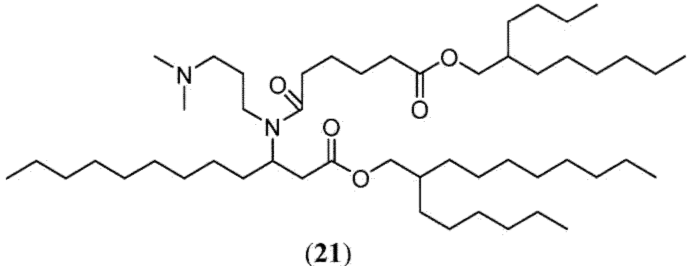 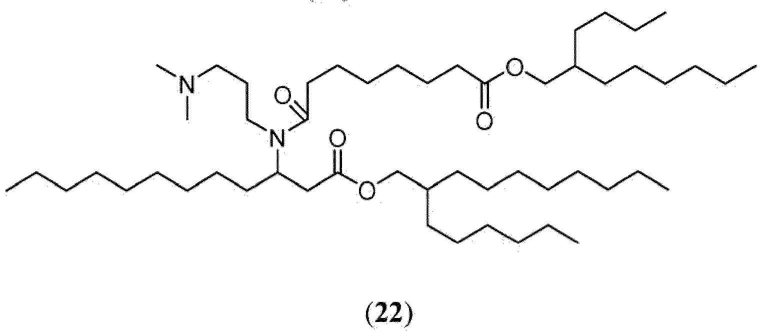 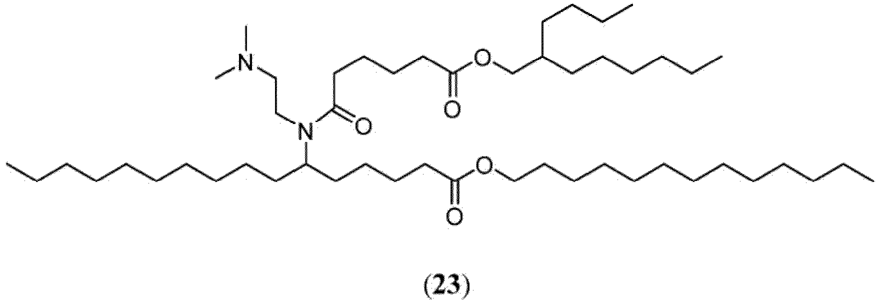 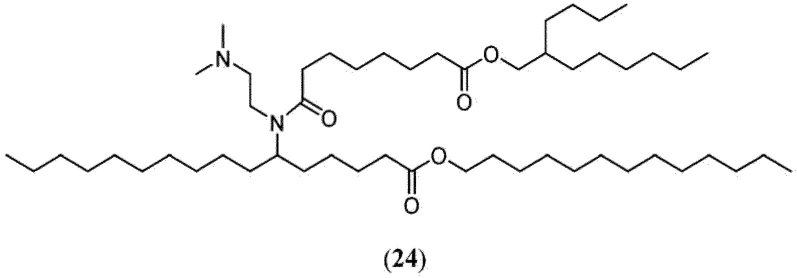 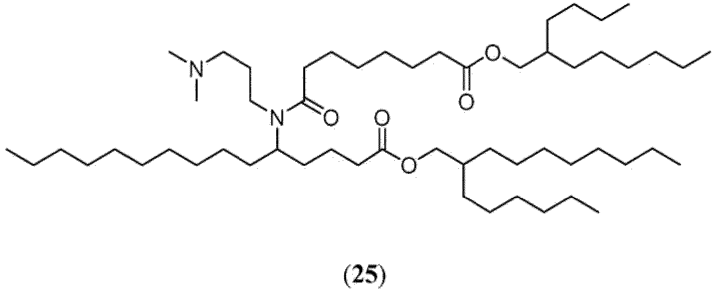 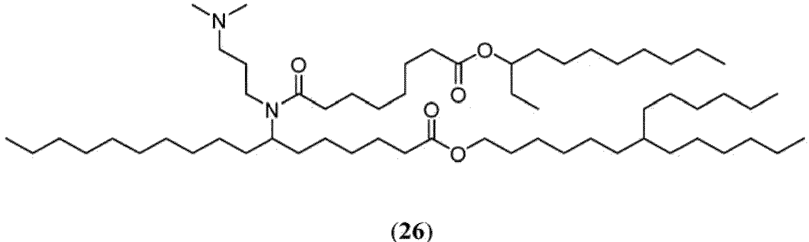 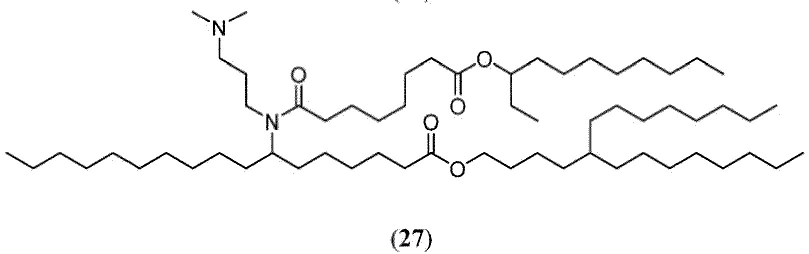 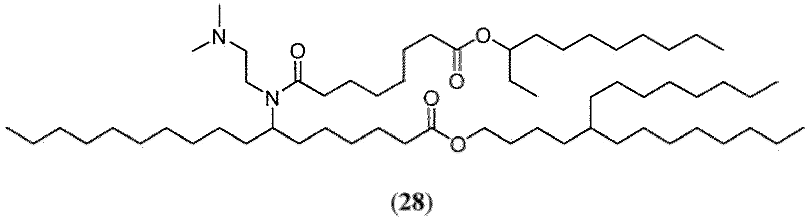 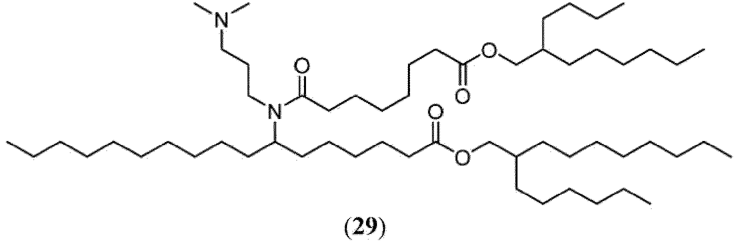 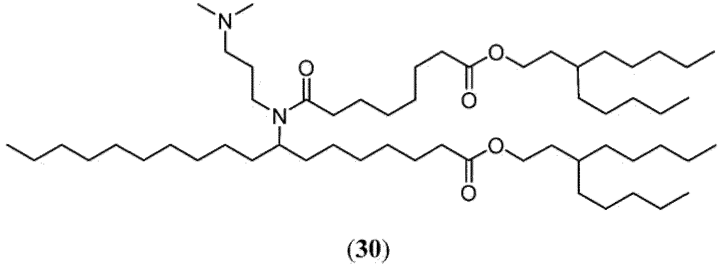 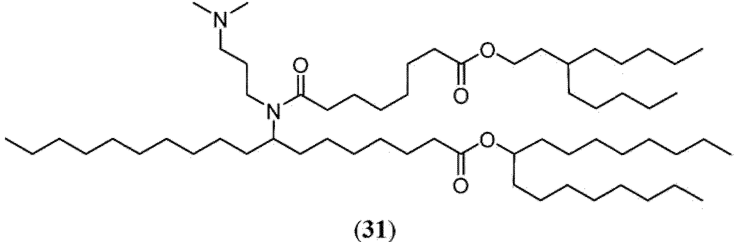 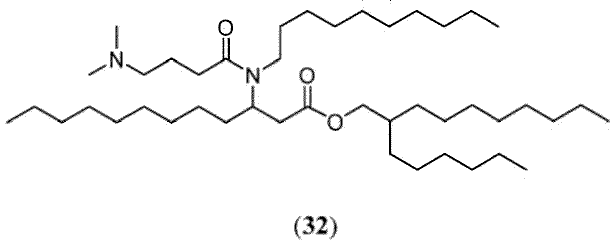 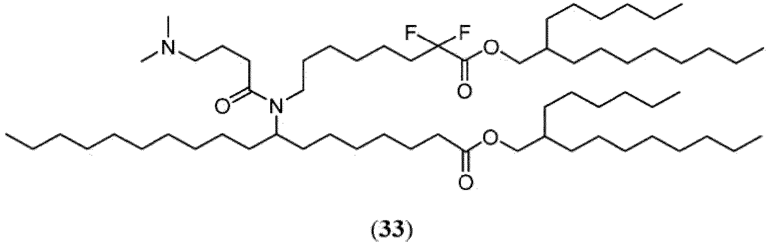 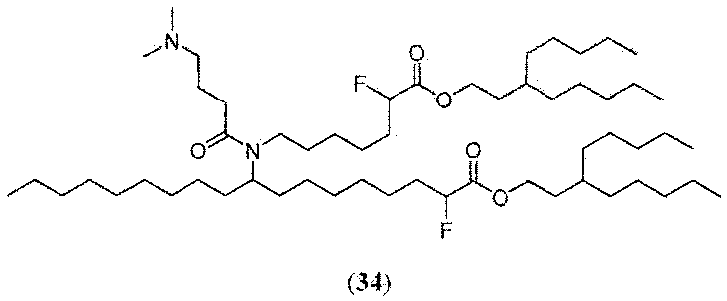 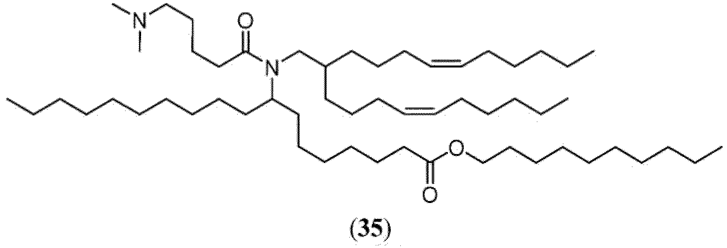 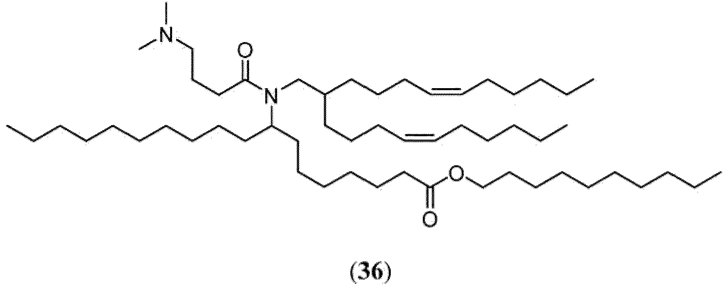 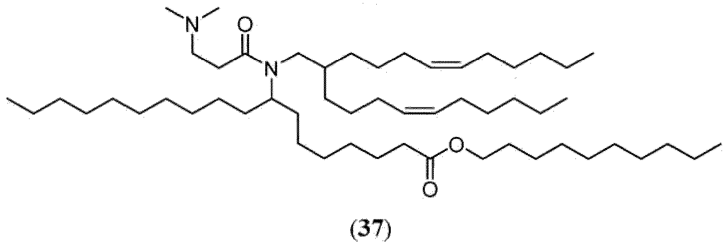 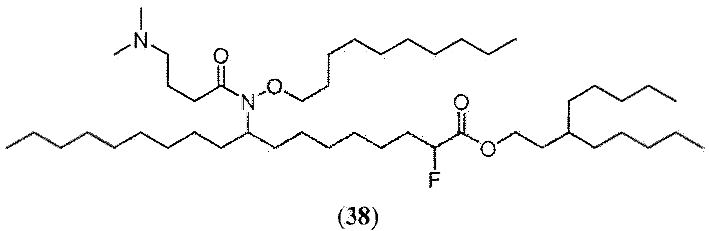 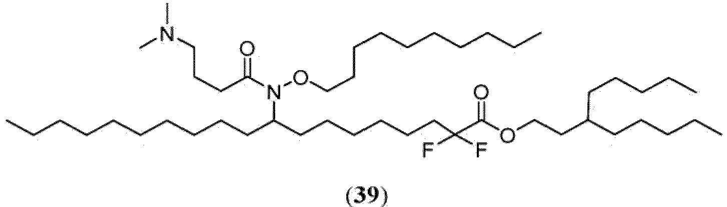 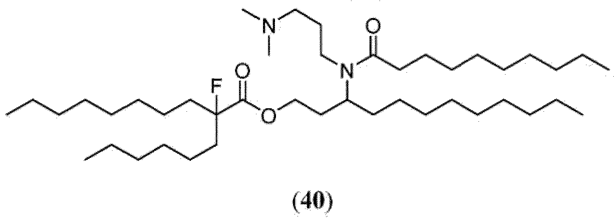 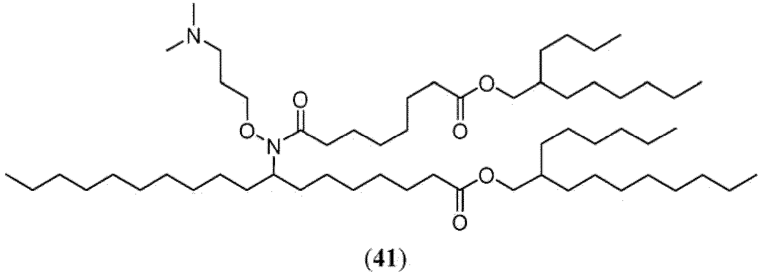 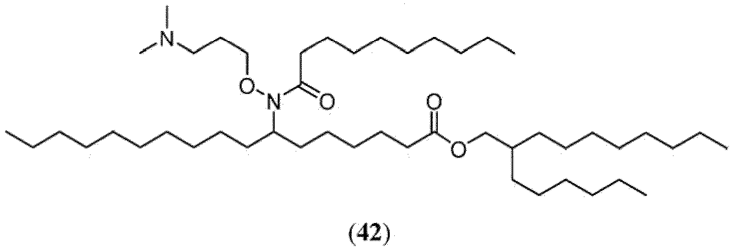 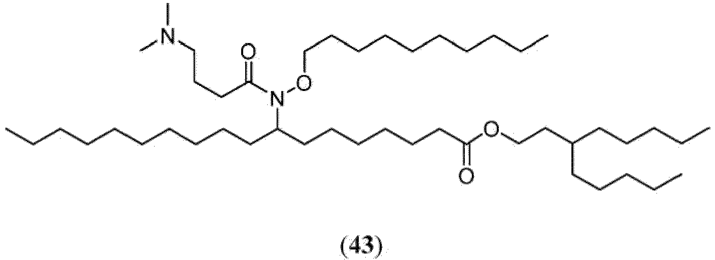 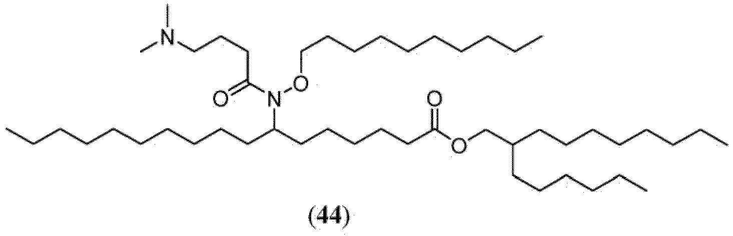 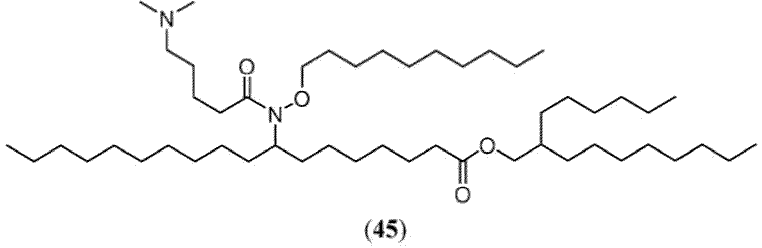 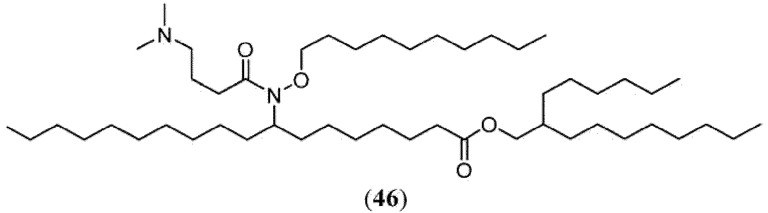 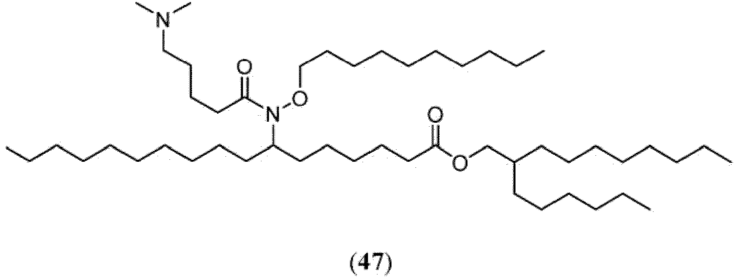 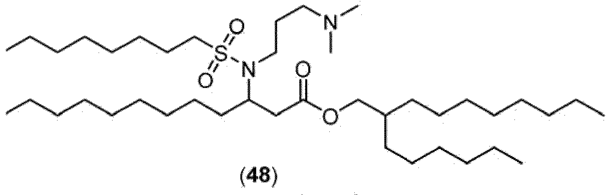 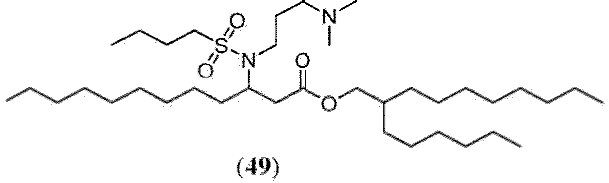 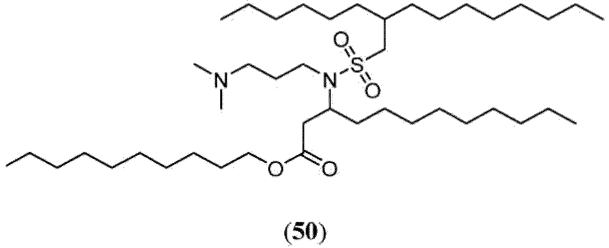 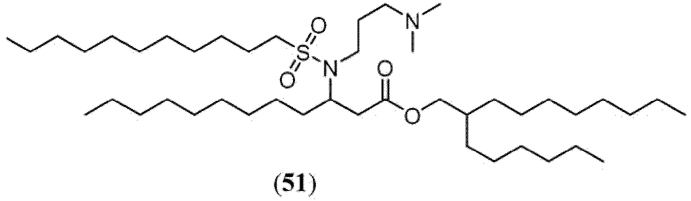 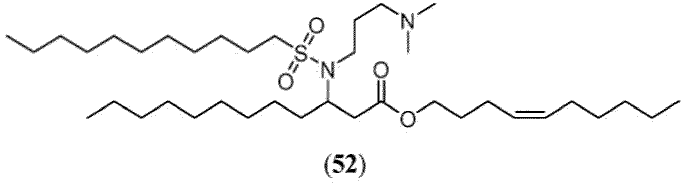 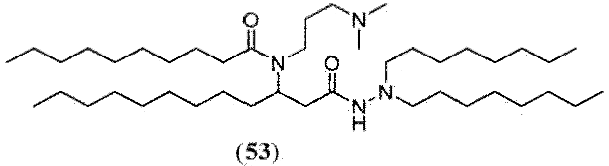 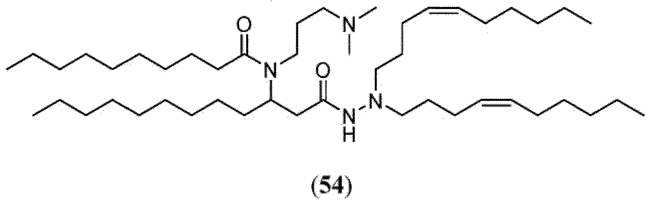 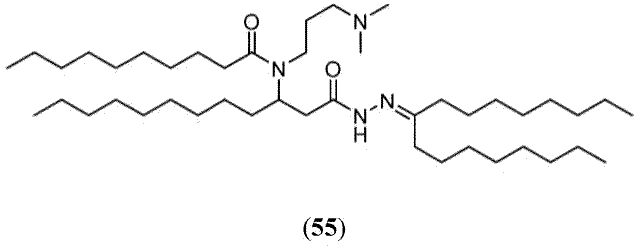 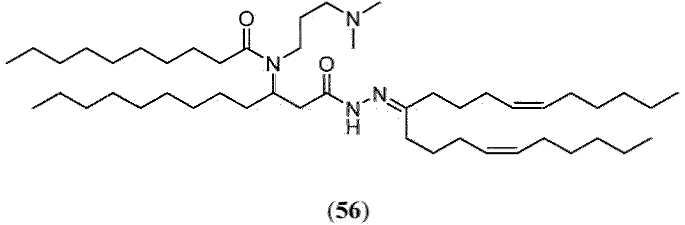 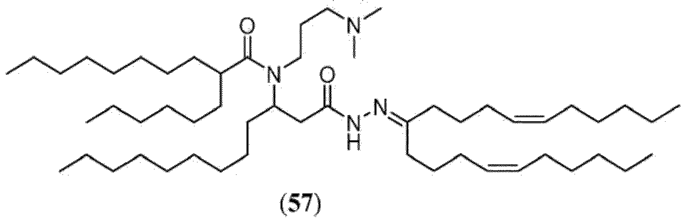 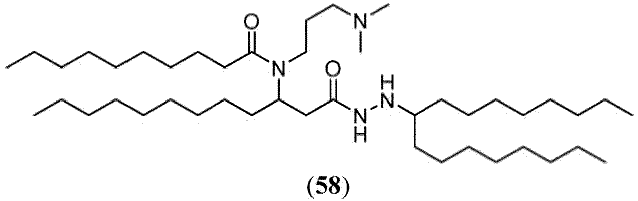 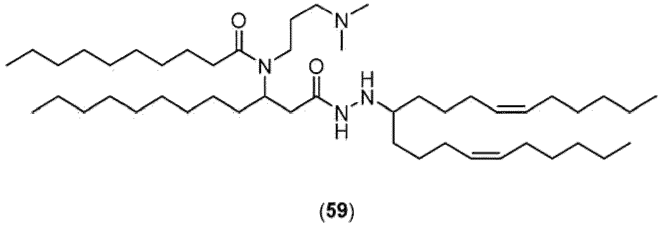 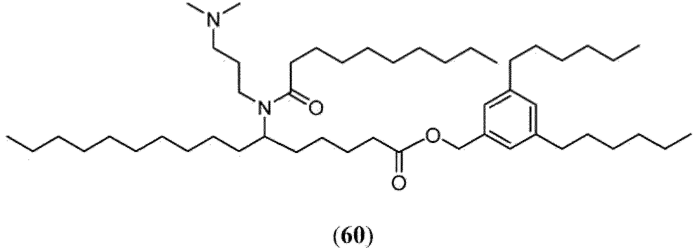 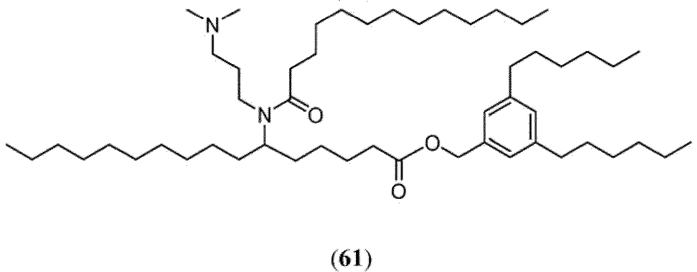 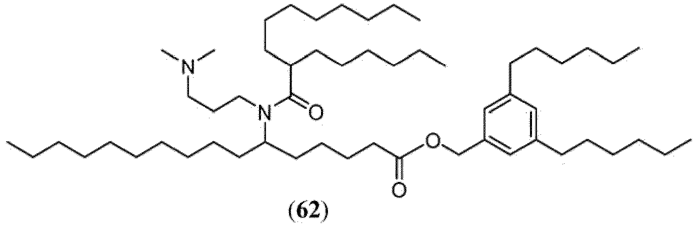 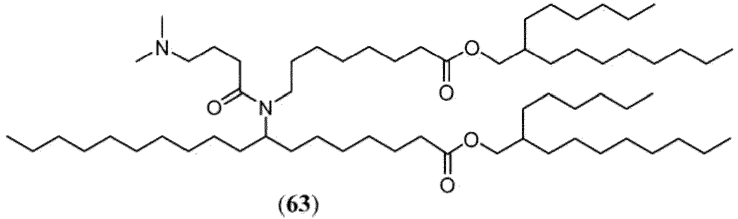 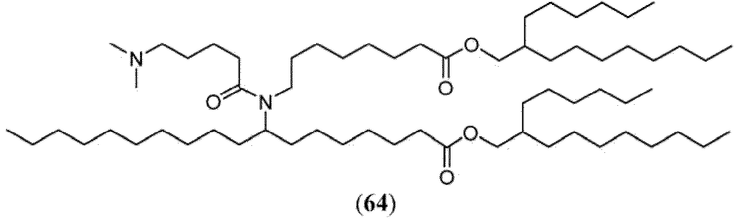 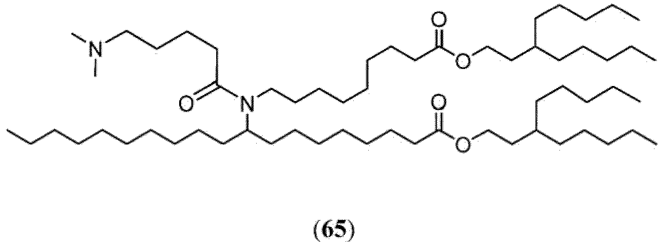 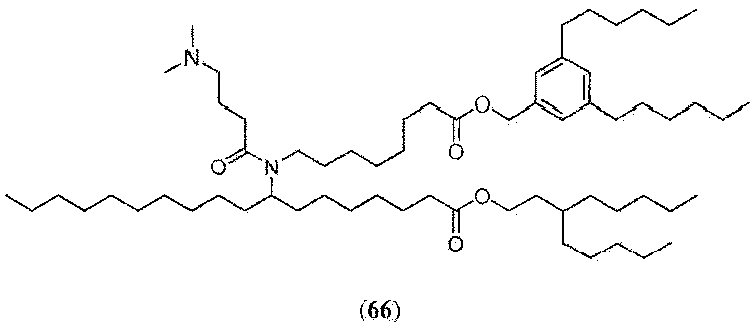 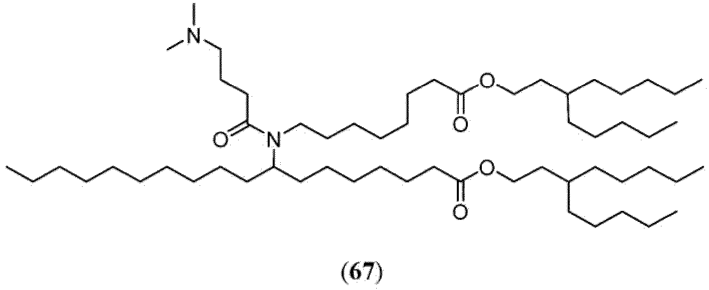 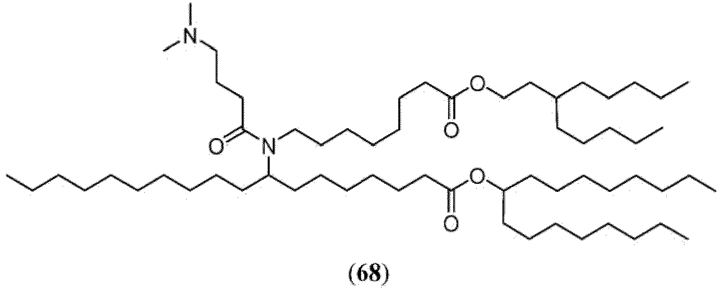 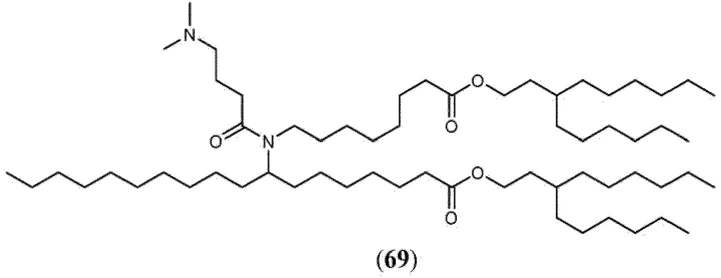 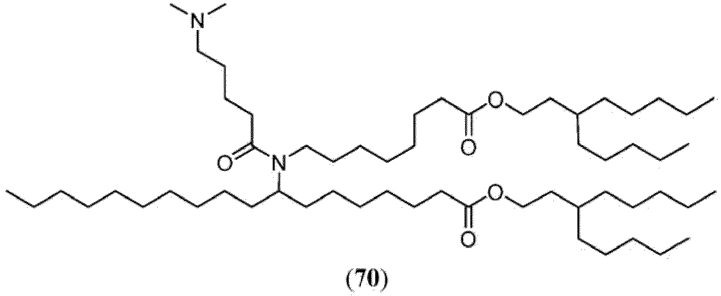 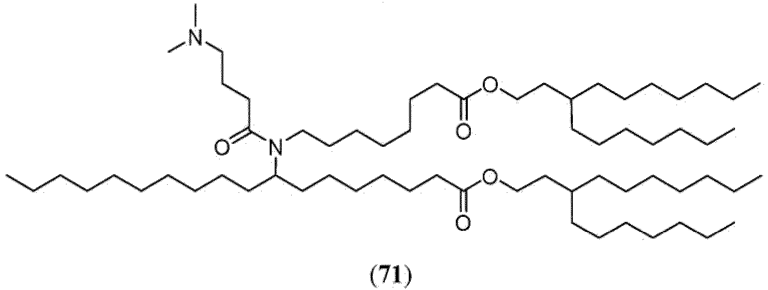 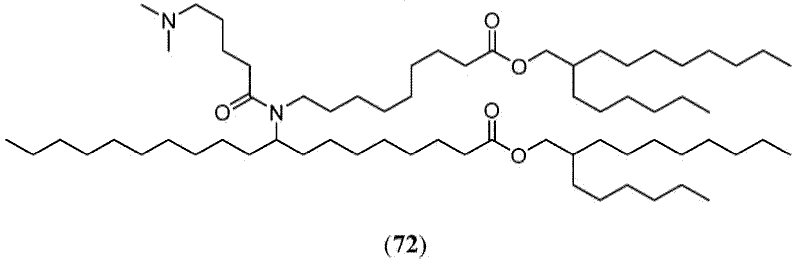 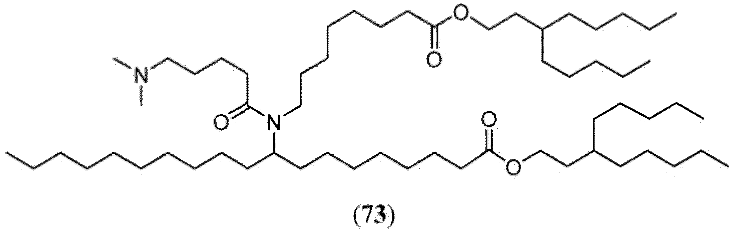 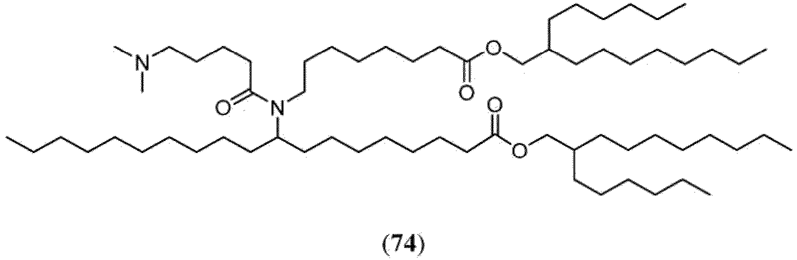 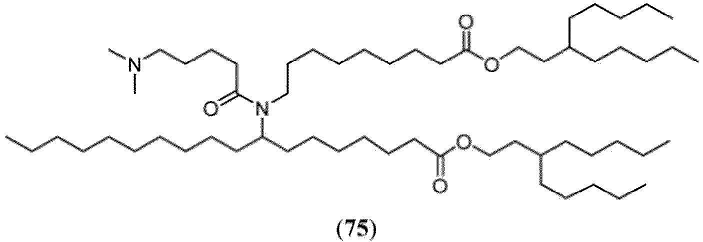 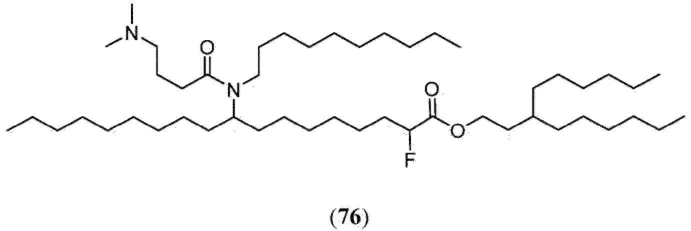 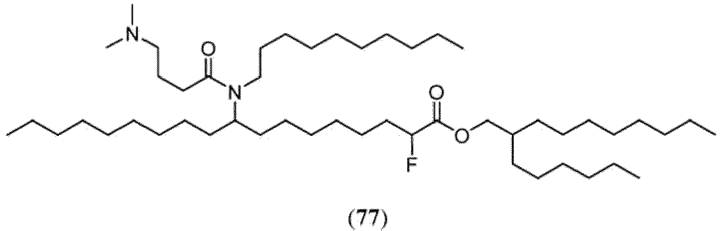 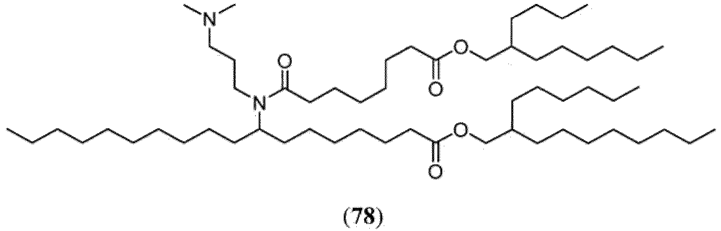 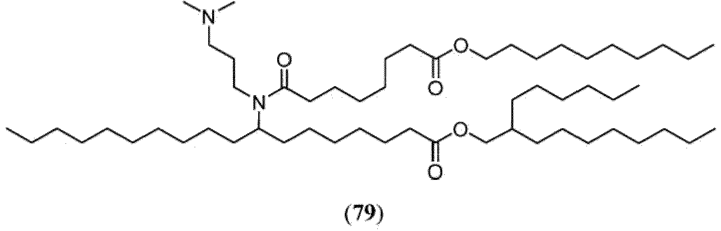 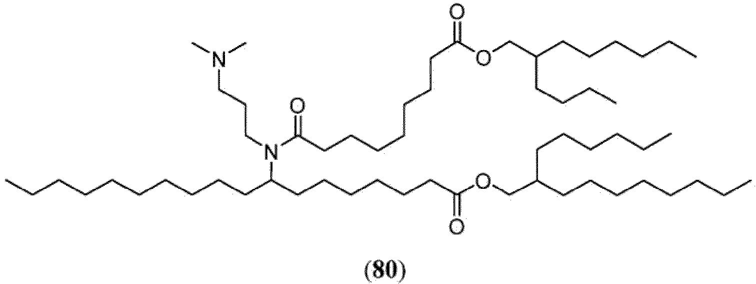 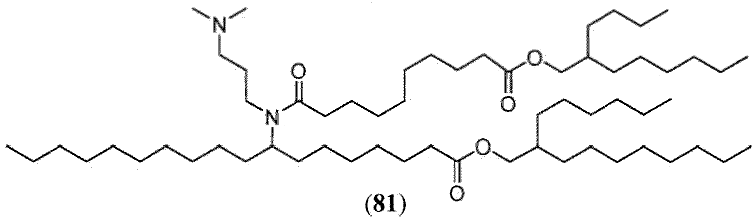 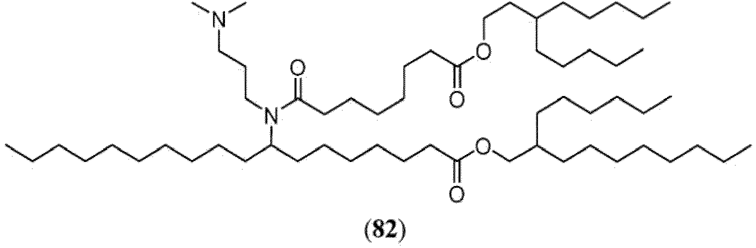 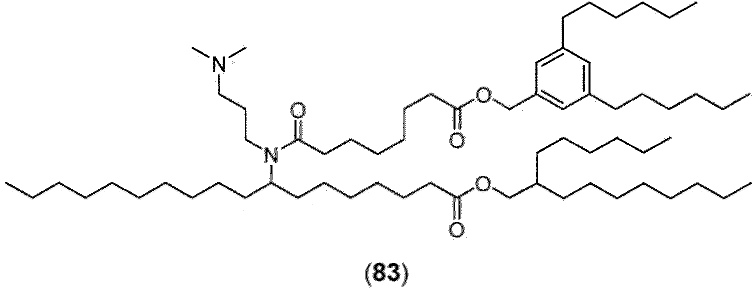 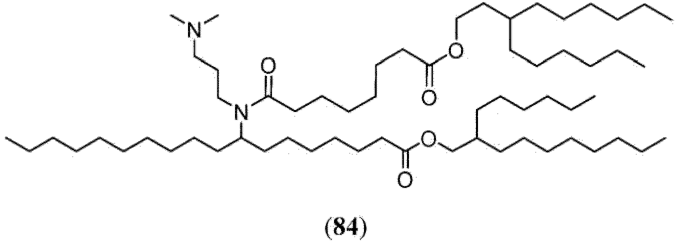 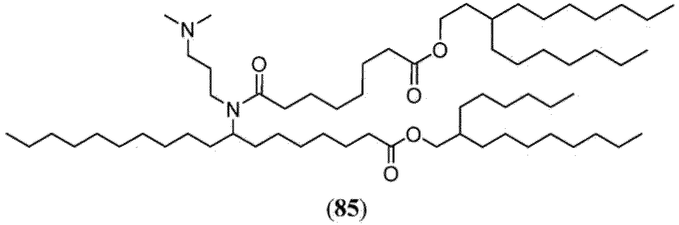 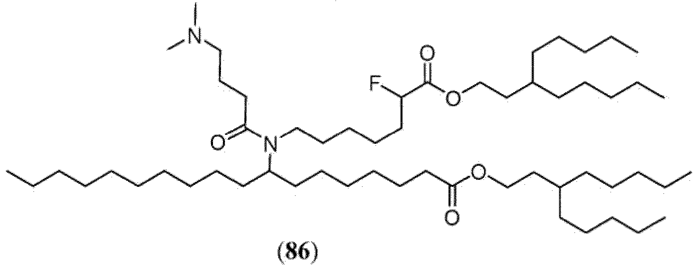 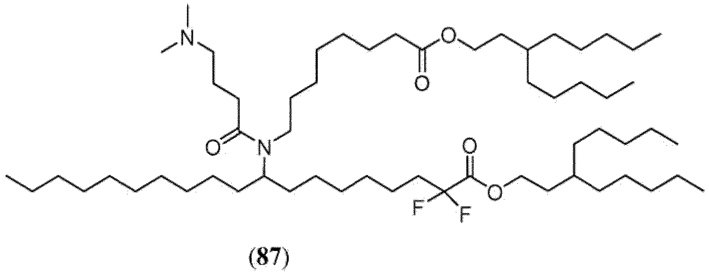 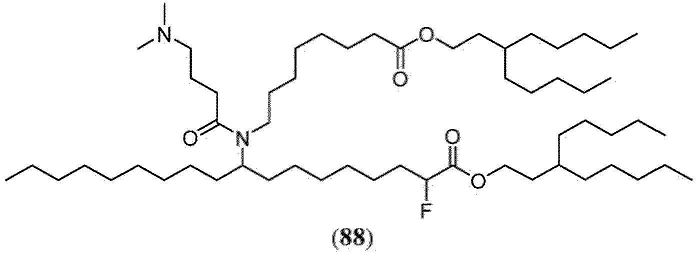 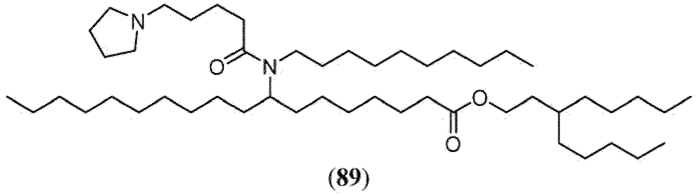 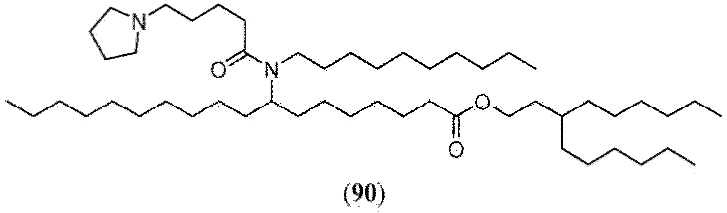 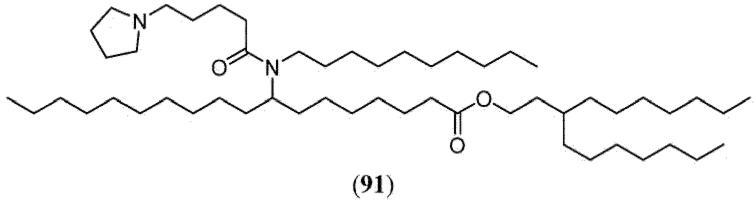 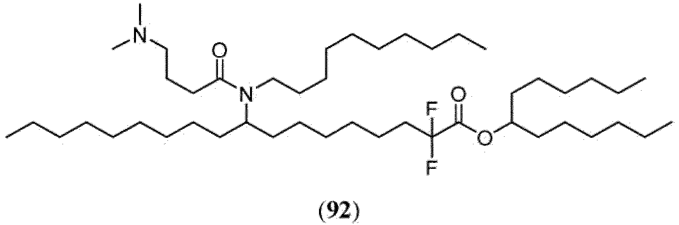 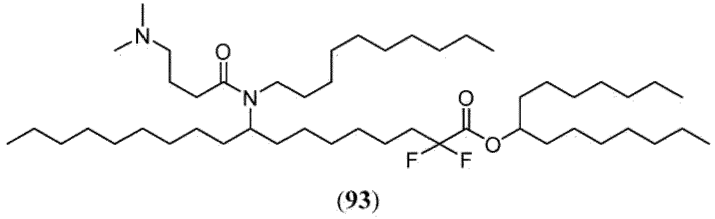 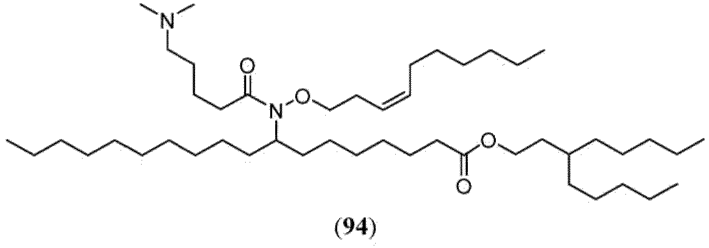 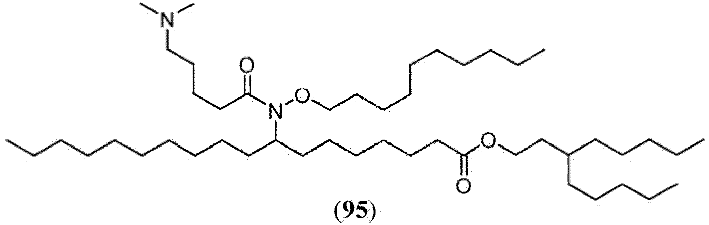 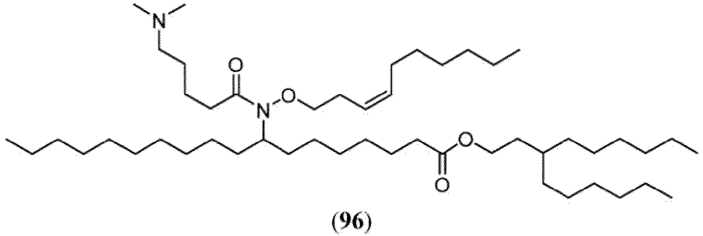 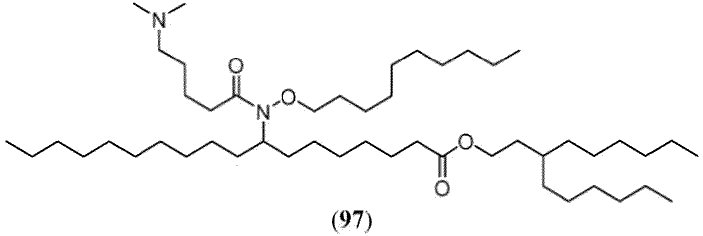 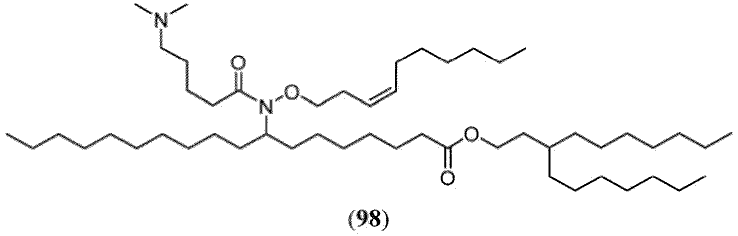 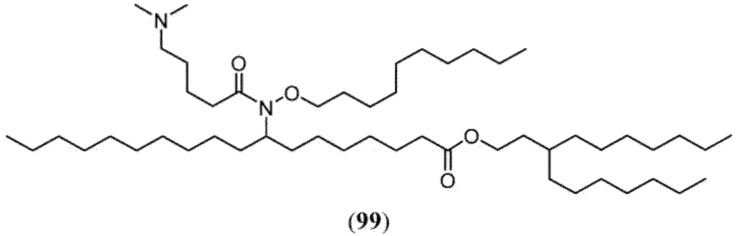 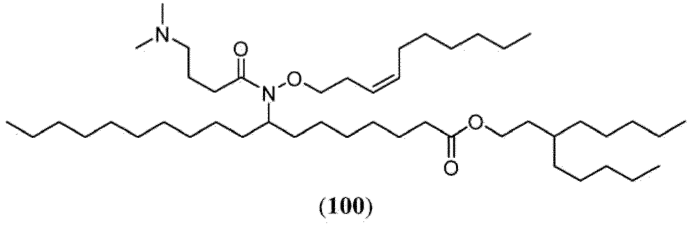 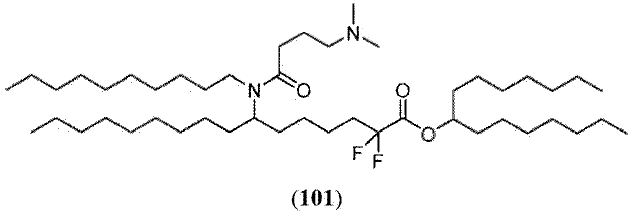 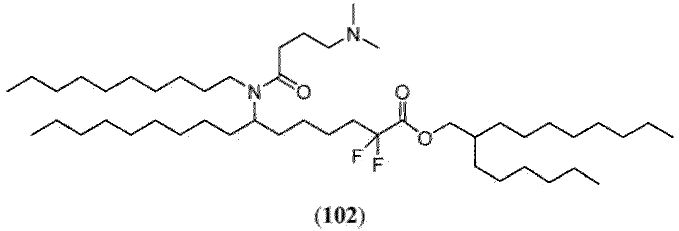 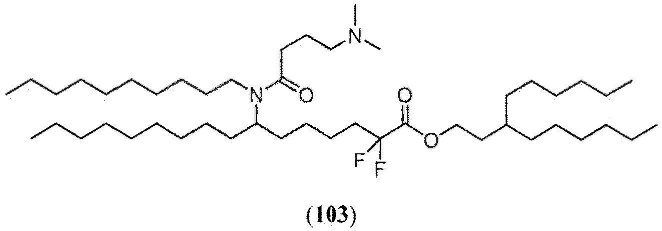 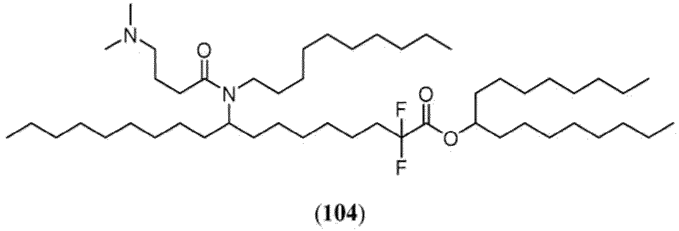 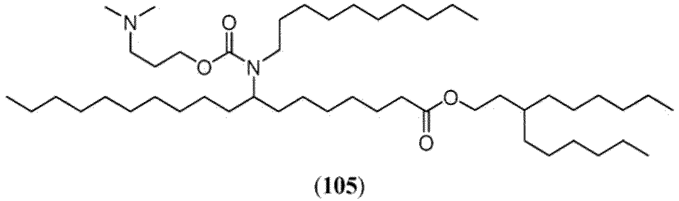 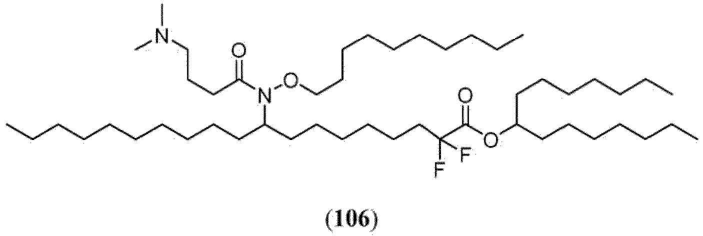 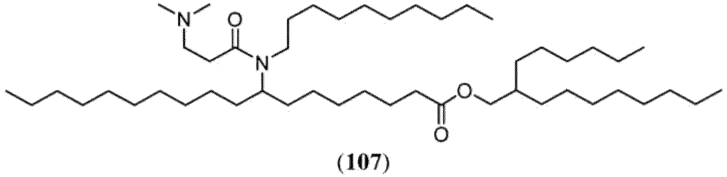 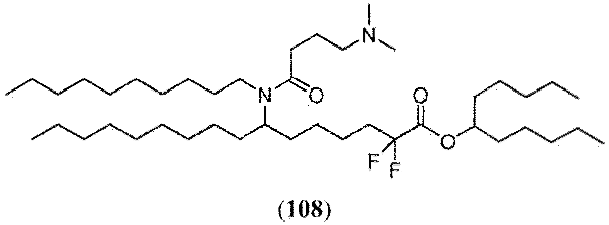 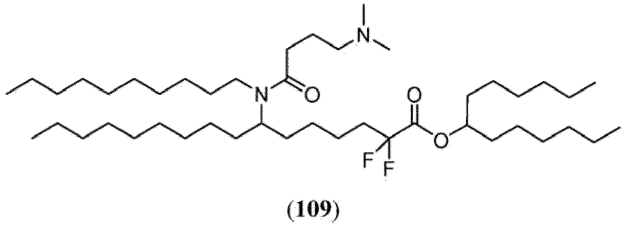 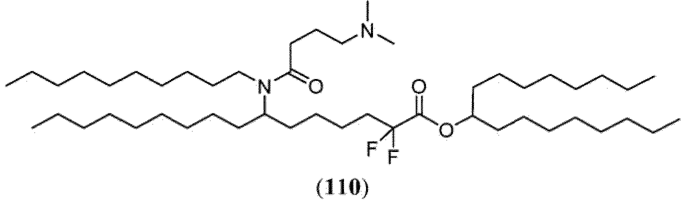 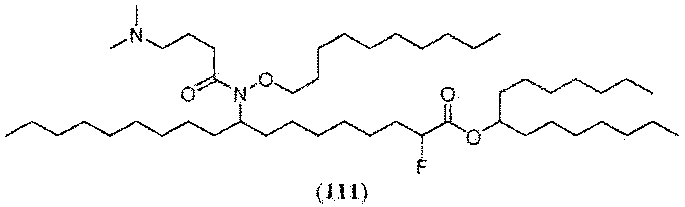 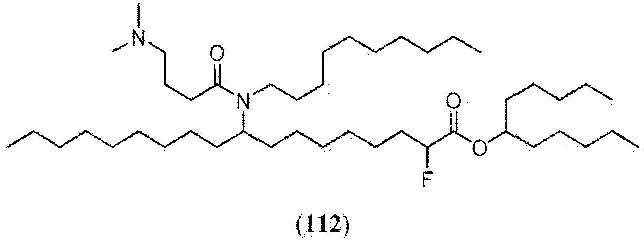 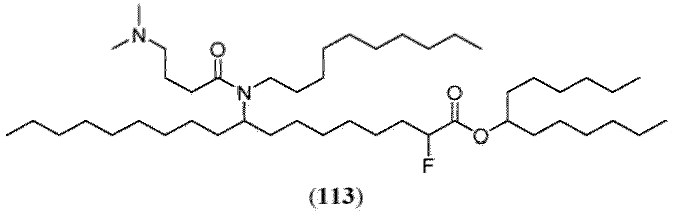 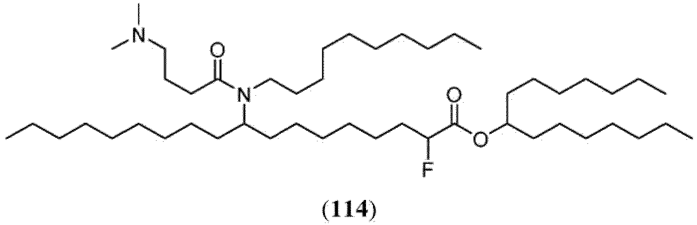 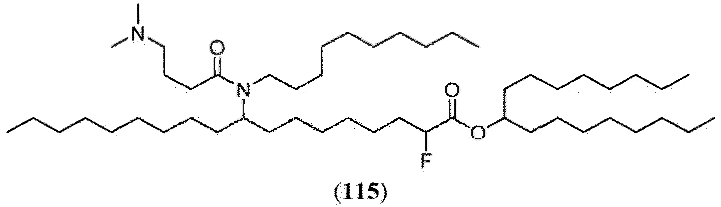 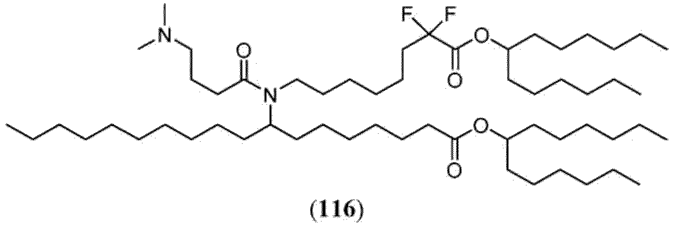 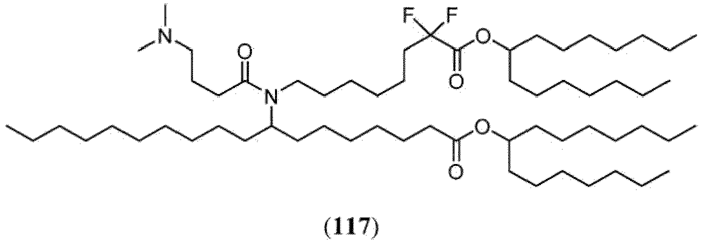 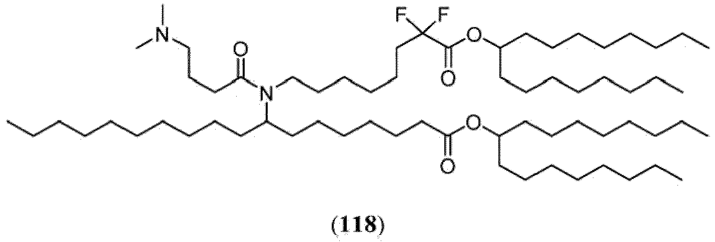 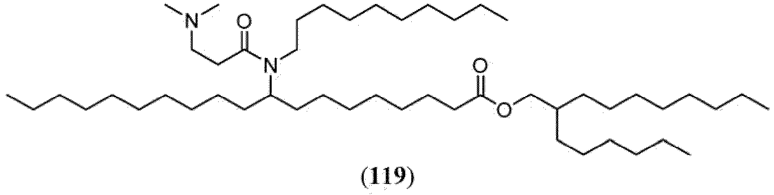 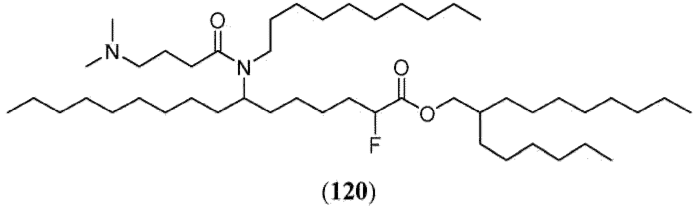 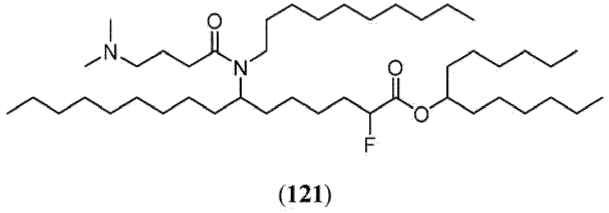 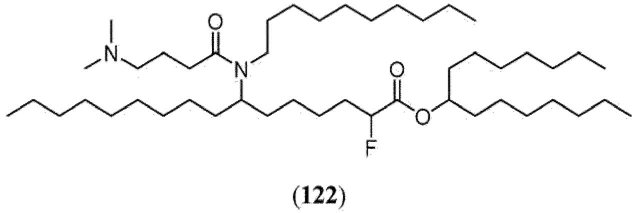 et 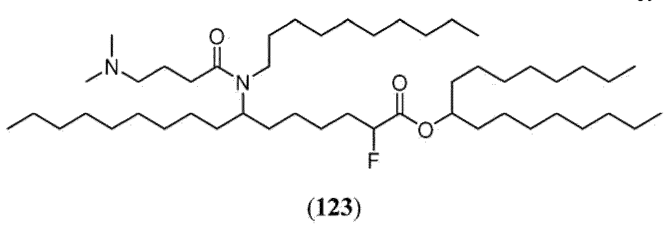 ou un sel de ceux-ci.

9. Composé ou sel selon la revendication 1, dans lequel :
(i) le composé ou sel est : 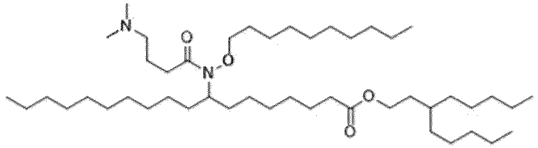 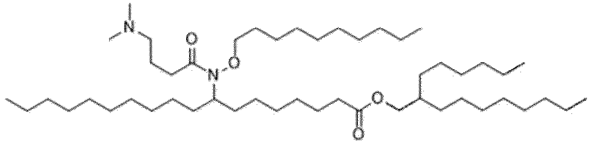 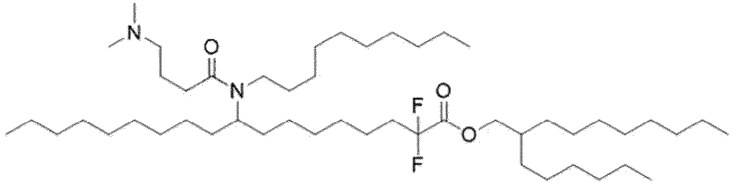 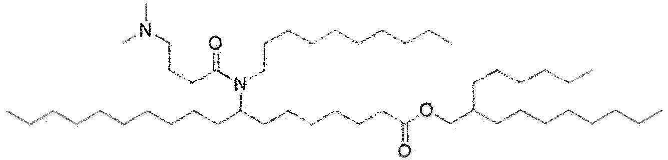 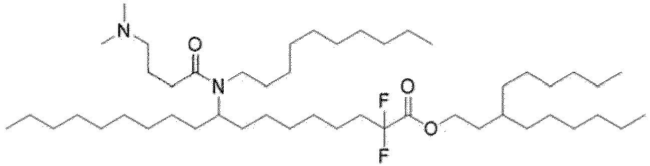 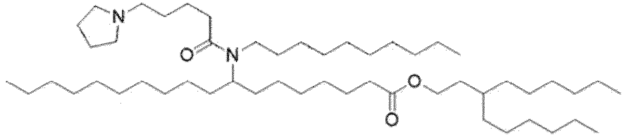 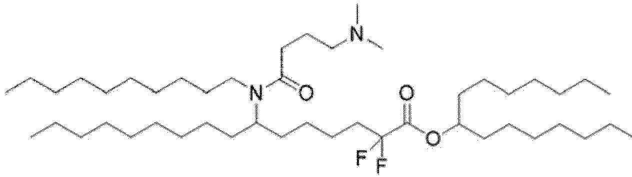 ou 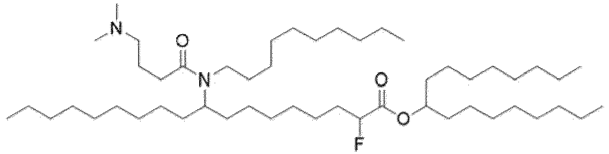 ou un sel de ceux-ci ; ou
(ii) 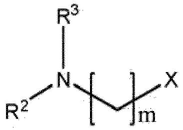 est sélectionné dans le groupe constitué par : (CH₃)₂NCH₂CH₂C(=O)-, (CH₃)₂NCH₂CH₂CH₂C(=O)-, (CH₃)₂NCH₂CH₂CH₂CH₂C(=O)-, (CH₃)₂NCH₂CH₂-, (CH₃)₂NCH₂CH₂CH-, 5-pyrolidin-1-ylpentanoyle, et (CH₃)₂NCH₂CH₂CH₂O-.

10. Particule acide nucléique-lipide comprenant :
(a) une ou plusieurs molécules d'acide nucléique ;
(b) un lipide non cationique ;
(c) un lipide conjugué ; et
(d) un composé ou sel tel que décrit dans l'une quelconque des revendications 1 à 9, dans laquelle les une ou plusieurs molécules d'acide nucléique sont encapsulées au sein de la particule lipidique.

11. Particule acide nucléique-lipide selon la revendication 10, dans laquelle :
(i)(a) le composé ou sel représente 30 % en moles à 85 % en moles du lipide total présent dans la particule ; le lipide non cationique représente 13 % en moles à 49,5 % en moles du lipide total présent dans la particule ; et le lipide conjugué représente 0,1 % en moles à 10 % en moles du lipide total présent dans la particule, ou (b) le composé ou sel représente 30 % en moles à 85 % en moles du lipide total présent dans la particule ; le lipide non cationique représente 13 % en moles à 49,5 % en moles du lipide total présent dans la particule ; et le lipide conjugué représente 0,1 % en moles à 10 % en moles du lipide total présent dans la particule ; et/ou
(ii) l'acide nucléique est sélectionné dans le groupe constitué par un petit ARN interférent (ARNsi), un ARN substrat de Dicer ARNds, un petit ARN en épingle à cheveux (ARNsh), un ARN interférent asymétrique (ARNai), un microARN (miARN), un ARNm, un ARNt, un ARNr, un ARN viral (ARNv), un ARN auto-amplifié, et des combinaisons de ceux-ci ; ou l'acide nucléique est une molécule d'ARNm ; ou l'acide nucléique comprend une molécule d'ARNsi double brin, éventuellement dans laquelle la molécule d'ARNsi double brin comprend au moins un nucléotide modifié ou l'ARNsi comprend au moins un nucléotide 2'-O-méthyle (2'OMe) ; et/ou
(iii) le lipide non cationique comprend : (a) du cholestérol ou un dérivé de celui-ci, éventuellement dans laquelle le cholestérol ou dérivé de celui-ci représente 31,5 % en moles à 42,5 % en moles du lipide total présent dans la particule ; (b) un phospholipide ; ou (c) un mélange d'un phospholipide et de cholestérol ou d'un dérivé de celui-ci, éventuellement dans laquelle le phospholipide comprend de la dipalmitoylphosphatidylcholine (DPPC), de la distéaroylphosphatidylcholine (DSPC), ou un mélange de celles-ci, le phospholipide représente 4 % en moles à 10 % en moles du lipide total présent dans la particule et le cholestérol représente 30 % en moles à 40 % en moles du lipide total présent dans la particule, ou le phospholipide représente 10 % en moles à 30 % en moles du lipide total présent dans la particule et le cholestérol représente 10% en moles à 30 % en moles du lipide total présent dans la particule ; et/ou
(iv) le lipide conjugué comprend un conjugué polyéthylèneglycol (PEG)-lipide, éventuellement dans laquelle : (a) le conjugué PEG-lipide comprend un conjugué PEG-diacylglycérol (PEG-DAG), un conjugué PEG-dialkyloxypropyle (PEG-DAA), ou un mélange de ceux-ci, éventuellement dans laquelle le conjugué PEG-DAA comprend un conjugué PEG-dimyristyloxypropyle (PEG-DMA), un conjugué PEG-distéaryloxypropyle (PEG-DSA), ou un mélange de ceux-ci, ou un conjugué PEG-dimyristyloxypropyle (PEG-DMA) ; (b) le conjugué polyéthylèneglycol (PEG)-lipide est un composé de formule :
A-B-C
ou un sel de celui-ci, où :
A est alkyle(en C₁-C₆), cycloalkyle(en C₃-C₈), cycloalkyl(en C₃-C₈)alkyle(en C₁-C₆), alcoxy(en C₁-C₆), alcényle(en C₂-C₆), alcynyle(en C₂-C₆), alcanoyle(en C₁-C₆), alcoxycarbonyle(en C₁-C₆), alkylthio(en C₁-C₆), ou alcanoyloxy(en C₂-C₆), où un quelconque alkyle(en C₁-C₆), cycloalkyle(en C₃-C₈), cycloalkyl(en C₃-C₈)alkyle(en C₁-C₆), alcoxy(en C₁-C₆), alcényle(en C₂-C₆), alcynyle(en C₂-C₆), alcanoyle(en C₁-C₆), alcoxycarbonyle(en C₁-C₆), alkylthio(en C₁-C₆), et alcanoyloxy(en C₂-C₆) est substitué par un ou plusieurs groupes précurseurs anioniques, et où un quelconque alkyle(en C₁-C₆), cycloalkyle(en C₃-C₈), cycloalkyl(en C₃-C₈)alkyle(en C₁-C₆), alcoxy(en C₁-C₆), alcényle(en C₂-C₆), alcynyle(en C₂-C₆), alcanoyle(en C₁-C₆), alcoxycarbonyle(en C₁-C₆), alkylthio(en C₁-C₆), et alcanoyloxy(en C₂-C₆) est éventuellement substitué par un ou plusieurs groupes indépendamment sélectionnés dans le groupe constitué par halogéno, hydroxyle, alcoxy(en C₁-C₃), alcanoyle(en C₁-C₆), alcoxycarbonyle(en C₁-C₃), alkylthio(en C₁-C₃), ou alcanoyloxy(en C₂-C₃) ;
B est une chaîne de polyéthylène glycol ayant un poids moléculaire de 550 daltons à 10 000 daltons ;
C est -L-R^{a}
L est sélectionné dans le groupe constitué par une liaison directe, -C(O)O-, -C(O)NR^{b}-, -NR^{b}-, -C(O)-, -NR^{b}C(O)O-, -NR^{b}C(O)NR^{b}-, -S-S-, -O-, -(O)CCH₂CH₂C(O)-, et - NHC(O)CH₂CH₂C(O)NH- ;
R^{a} est alkyle(en C₁₀-C₅₀) ramifié ou alcényle(en C₁₀-C₅₀) ramifié où un ou plusieurs atomes de carbone de l'alkyle(en C₁₀-C₅₀) ramifié ou de l'alcényle(en C₁₀-C₅₀) ramifié ont été remplacés par -O- ; et
chaque R^{b} est indépendamment H ou alkyle(en C₁-C₆) ; ou (c) le PEG a un poids moléculaire moyen de 2000 daltons ; et/ou
(v) le lipide conjugué comprend un conjugué PEG-lipide sélectionné parmi : 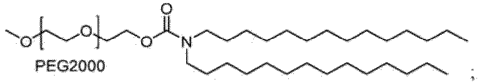 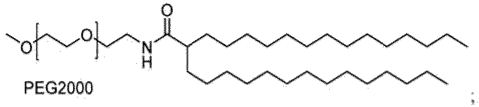 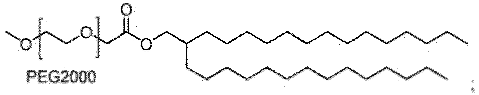 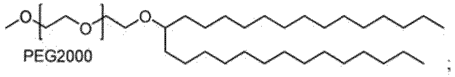 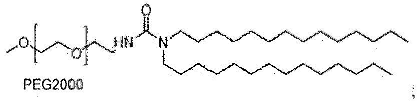 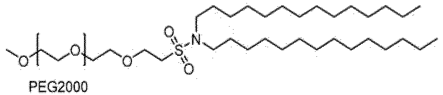 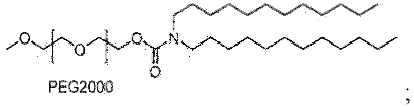 et 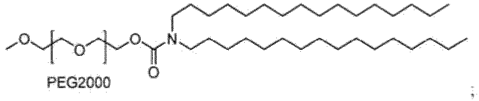 ou un sel de ceux-ci ; et/ou
(vii) le lipide conjugué comprend un composé de formule suivante : 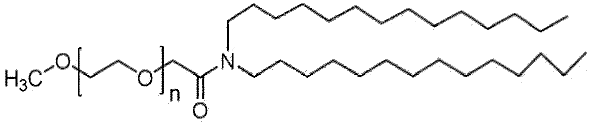 où n vaut 45 à 50 ; et/ou
(viii) le lipide conjugué représente 1 % en moles à 2 % en moles du lipide total présent dans la particule ; et/ou
(ix) l'acide nucléique dans la particule acide nucléique-lipide n'est pas sensiblement dégradé après une incubation de la particule dans du sérum à 37 °C pendant 30 minutes et/ou
(x) l'acide nucléique est pleinement encapsulé dans la particule acide nucléique-lipide, éventuellement dans laquelle la particule acide nucléique-lipide a un rapport en masse lipide:acide nucléique de 5 à 15 ou de 5 à 30 ; et/ou
(xi) la particule acide nucléique-lipide a un diamètre médian de 40 nm à 150 nm.

12. Particule acide nucléique-lipide selon la revendication 10, dans laquelle :
(i) le composé ou sel représente 56,5 % en moles à 66,5 % en moles du lipide total présent dans la particule ; le lipide non cationique comprend du cholestérol ou un dérivé de celui-ci représentant 31,5 % en moles à 42,5 % en moles du lipide total présent dans la particule ; et un conjugué PEG-lipide représente 1 % en moles à 2 % en moles du lipide total présent dans la particule, éventuellement dans laquelle la particule acide nucléique-lipide comprend 61,5 % en moles de composé ou sel, 36,9 % de cholestérol ou d'un dérivé de celui-ci, et 1,5 % en moles de conjugué PEG-lipide ; ou
(ii) le composé ou sel représente 52 % en moles à 62 % en moles du lipide total présent dans la particule ; le lipide non cationique comprend un mélange de phospholipide et de cholestérol ou d'un dérivé de celui-ci représentant 36 % en moles à 47 % en moles du lipide total présent dans la particule ; et le conjugué PEG-lipide représentant 1 % en moles à 2 % en moles du lipide total présent dans la particule, éventuellement dans laquelle : (a) la particule acide nucléique-lipide comprend 57,1 % en moles de composé ou sel, 7,1 % en moles de phospholipide, 34,3 % de cholestérol ou d'un dérivé de celui-ci, et 1,4 % en moles de conjugué PEG-lipide ; ou (b) la particule acide nucléique-lipide comprend 57,1 % en moles de composé ou sel, 20 % en moles de phospholipide, 20 % en moles de cholestérol ou d'un dérivé de celui-ci, et 1,4 % en moles de conjugué PEG-lipide ; ou
(iii) le composé ou sel représente 50 % en moles à 65 % en moles du lipide total présent dans la particule ; le lipide non cationique comprend un mélange de phospholipide et de cholestérol ou d'un dérivé de celui-ci, dans laquelle le phospholipide représente 4 % en moles à 10 % en moles du lipide total présent dans la particule et le cholestérol ou dérivé de celui-ci représente 30 % en moles à 40 % en moles du lipide total présent dans la particule ; et le lipide conjugué représente 0,5 % en moles à 2 % en moles du lipide total présent dans la particule ; ou
(iv) le composé ou sel représente 50 % en moles à 65 % en moles du lipide total présent dans la particule ; le lipide non cationique comprenant un mélange de phospholipide et de cholestérol ou d'un dérivé de celui-ci, dans laquelle le phospholipide représente 3 % en moles à 15 % en moles du lipide total présent dans la particule et le cholestérol ou dérivé de celui-ci représente 30 % en moles à 40 % en moles du lipide total présent dans la particule ; et le lipide conjugué représente 0,5 % en moles à 2 % en moles du lipide total présent dans la particule ; ou
(v) le composé ou sel représente 50 % en moles à 65 % en moles du lipide total présent dans la particule ; le lipide non cationique représente jusqu'à 49,5 % en moles du lipide total présent dans la particule ; le cholestérol ou dérivé de celui-ci représente 30 % en moles à 40 % en moles du lipide total présent dans la particule ; et le lipide conjugué représente 0,5 % en moles à 2 % en moles du lipide total présent dans la particule ; ou
(vi) le composé ou sel représente 50 % en moles à 85 % en moles du lipide total présent dans la particule ; le lipide non cationique représente 13 % en moles à 49,5 % en moles du lipide total présent dans la particule ; et le lipide conjugué représente 0,5 % en moles à 2 % en moles du lipide total présent dans la particule ; ou
(vii) le composé ou sel représente 30 % en moles à 50 % en moles du lipide total présent dans la particule ; le lipide non cationique comprend un mélange de phospholipide et de cholestérol ou d'un dérivé de celui-ci représentant 47 % en moles à 69 % en moles du lipide total présent dans la particule ; et le lipide conjugué représente 1 % en moles à 3 % en moles du lipide total présent dans la particule, éventuellement dans laquelle le rapport entre lipide et acide nucléique est de 24 ; ou
(viii) le lipide non cationique comprend un mélange de cholestérol et de DSPC ; le lipide conjugué est : 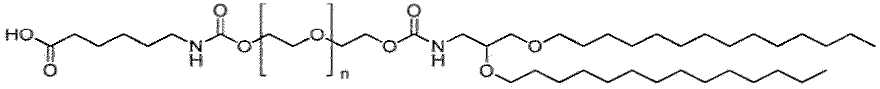 où n est sélectionné de sorte que la chaîne de polymère résultante a un poids moléculaire de 2000 daltons
dans laquelle le lipide conjugué représente 2 % en moles du lipide total présent dans la particule ; la DSPC représente 10 % en moles du lipide total présent dans la particule ; le cholestérol représente 48 % en moles du lipide total présent dans la particule ; et le composé ou sel représente 40 % en moles du lipide total présent dans la particule, éventuellement dans laquelle le rapport entre lipide et acide nucléique est de 24 ; ou
(ix) le lipide non cationique comprend un mélange de cholestérol et de DSPC ; le lipide conjugué est : 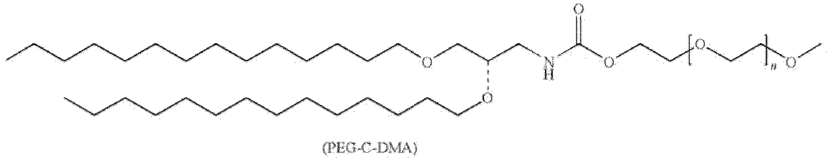 où n est sélectionné de sorte que la chaîne de polymère résultante a un poids moléculaire de 2000 daltons ;
dans laquelle le lipide conjugué représente 2 % en moles du lipide total présent dans la particule ; la DSPC représente 10 % en moles du lipide total présent dans la particule ; le cholestérol représente 48 % en moles du lipide total présent dans la particule ; et le composé ou sel représente 40 % en moles du lipide total présent dans la particule, éventuellement dans laquelle le rapport entre lipide et acide nucléique est de 24 ; ou
(x) le lipide non cationique comprend un mélange de cholestérol et de DSPC ; le lipide conjugué est : 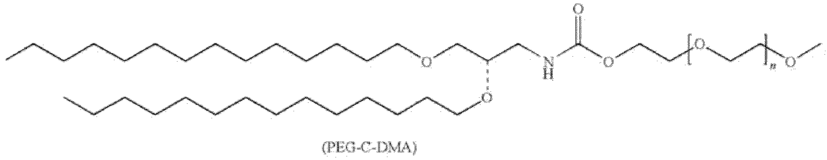 où n est sélectionné de sorte que la chaîne de polymère résultante a un poids moléculaire de 2000 ;
dans laquelle le lipide conjugué représente 1,6 % en moles du lipide total présent dans la particule ; la DSPC représente 10,9 % en moles du lipide total présent dans la particule ; le cholestérol représente 32,8 % en moles du lipide total présent dans la particule ; et le composé ou sel représente 54,9 % en moles du lipide total présent dans la particule, éventuellement dans laquelle le rapport entre lipide et acide nucléique est de 24 ; ou
(xi) la particule acide nucléique-lipide comprend au moins deux composés ou sels tels que décrits dans l'une quelconque des revendications 1 à 9.

13. Composition pharmaceutique comprenant une particule acide nucléique-lipide de l'une quelconque des revendications 10 à 12, et un support pharmaceutiquement acceptable.

14. Particule acide nucléique-lipide selon l'une quelconque des revendications 10 à 12 ou composition selon la revendication 13, pour une utilisation dans le traitement d'une maladie ou d'un trouble chez un mammifère.

15. Particule acide nucléique-lipide ou composition pour une utilisation selon la revendication 14, dans laquelle la maladie ou le trouble est sélectionné dans le groupe constitué par une infection virale, une maladie ou un trouble hépatique, et le cancer.
